# EUROPEAN PATENT SPECIFICATION

(11) **EP 2 519 543 B1**
(45) Date of publication and mention of the grant of the patent: **29.06.2016**
(21) Application number: 10803713.6
(22) Date of filing: 29.12.2010
(51) Int. Cl.: C07K 16/46, A61K 39/395, A61P 35/00, A61P 37/00

(54) **HETERODIMER BINDING PROTEINS AND USES THEREOF**
HETERODIMER-BINDEPROTEINE UND IHRE VERWENDUNGEN
PROTÉINES DE LIAISON HÉTÉRODIMÈRES ET UTILISATIONS DE CELLES-CI

(30) Priority: 22.07.2010 US 366743 P; 16.07.2010 US 365266 P; 29.12.2009 US 290840 P
(43) Date of publication of application: 07.11.2012
(60) Divisional application: 16169947.5
(73) Proprietor: Emergent Product Development Seattle, LLC, Seattle, Washington 98121 (US)
(72) Inventor: BLANKENSHIP, John, W., Seattle, Washington 98117 (US); TAN, Philip, Edmonds, Washington 98026 (US)
(74) Representative: Walker, Ross Thomson
(86) International application number: PCT/US2010/062436
(87) International publication number: WO 2011/090762

(56) References cited:
- WO-A1-02/02781
- WO-A1-2005/017148
- WO-A1-2009/080251
- WO-A1-2009/080254
- WO-A1-2010/014629
- WO-A1-2010/034441
- WO-A1-2010/040508
- WO-A1-2010/136172
- WO-A2-2006/074399
- WO-A2-2007/048037
- WO-A2-2007/146968
- WO-A2-2009/023386
- WO-A2-2010/042904
- US-A1- 2007 014 794
- US-B1- 6 809 185

### SEQUENCE LISTING

<110> Emergent Product Development Seattle, LLC Blankenship, John W. Tan, Philip
<120> HETERODIMER BINDING PROTEINS AND USES THEREOF
<130> 910180.422PC
<140> PCT
   <141> 2010-12-29
<150> US 61/290,840
   <151> 2009-12-29
<150> US 61/365,266
   <151> 2010-07-16
<160> 882
<170> FastSEQ for Windows Version 4.0
<210> 1
   <211> 2088
   <212> DNA
   <213> Artificial Sequence
<220>
   <223> nucleotide sequence of 2E12CH1CH2CH3Ck (X0130)
<400> 1
   caggtgcagc tgaaggagtc aggacctggc agcgtggcgc cctcacagag cctgtccatc 60
   acatgcaccg tctcagggtt ctcattaacc ggctatggtg taaactgggt tcgccagcct 120
   ccaggaaagg gtctggagtg gctgggaatg atatggggtg atggaagcac agactataat 180
   tcagctctca aatccagact atcgatcacc aaggacaact ccaagagcca agttttctta 240
   aaaatgaaca gtctgcaaac tgatgacaca gccagatact actgtgctcg ggatggttat 300
   agtaactttc attactatgt tatggactac tggggtcaag gaacctcagt caccgtctcc 360
   tctgggggtg gaggctctgg tggcggtgga tccggcggag gtgggtcggg tggcggcgga 420
   tctgacattg tgctcaccca atctccagct tctttggctg tgtctctagg tcagagagcc 480
   accatctcct gcagagccag tgaaagtgtt gaatattatg tcacaagttt aatgcagtgg 540
   taccaacaga aaccaggaca gccacccaaa ctcctcatct ctgctgctag caacgtagaa 600
   tctggggtcc ctgccaggtt tagtggcagt gggtctggga cagactttag cctcaacatc 660
   catcctgtgg aggaggatga tattgcaatg tatttctgtc agcaaagtag gaaggttcca 720
   tggacgttcg gtggaggcac caagctggaa atcaagtcct cgagcgcctc caccaagggc 780
   ccatcggtct tccccctggc accctcctcc aagagcacct ctgggggcac agcggccctg 840
   ggctgcctgg tcaaggacta cttccccgag ccggtgacgg tgtcgtggaa ctcaggcgcc 900
   ctgaccagcg gcgtgcacac cttcccggct gtcctacagt cctcaggact ctactccctc 960
   agcagcgtgg tgaccgtgcc ctccagcagc ttgggcaccc agacctacat ctgcaacgtg 1020
   aatcacaagc ccagcaacac caaggtggac aagaaagttc gtacggagcc caaatcttct 1080
   gacaaaactc acacatgccc accgtgccca tccggagcac ctgaactcct gggtggaccg 1140
   tcagtcttcc tcttcccccc aaaacccaag gacaccctca tgatctcccg gacccctgag 1200
   gtcacatgcg tggtggtgga cgtgagccac gaagaccctg aggtcaagtt caactggtac 1260
   gtggacggcg tggaggtgca taatgccaag acaaagccgc gggaggagca gtacaacagc 1320
   acgtaccgtg tggtcagcgt cctcaccgtc ctgcaccagg actggctgaa tggcaaggag 1380
   tacaagtgca aggtctccaa caaagccctc ccagccccca tcgagaaaac catctccaaa 1440
   gccaaagggc agccccgaga accacaggtg tacaccctgc ccccatcccg ggatgagctg 1500
   accaagaacc aggtcagcct gacctgcctg gtcaaaggct tctatcccag cgacatcgcc 1560
   gtggagtggg agagcaatgg gcagccggag aacaactaca agaccacgcc tcccgtgctg 1620
   gactccgacg gctccttctt cctctacagc aagctcaccg tggacaagag caggtggcag 1680
   caggggaacg tcttctcatg ctccgtgatg catgaggctc tgcacaacca ctacacgcag 1740
   aagagcctct ccctgtctcc gggtaaatct agaactgtgg ctgcaccatc tgtcttcatc 1800
   ttcccgccat ctgatgagca gttgaaatct ggaactgcct ctgttgtgtg cctgctgaat 1860
   aacttctatc ccagagaggc caaagtacag tggaaggtgg ataacgccct ccaatcgggt 1920
   aactcccagg agagtgtcac agagcaggac agcaaggaca gcacctacag cctcagcagc 1980
   accctgacgc tgagcaaagc agactacgag aaacacaaag tctacgcctg cgaagtcacc 2040
   catcagggcc tgagctcgcc cgtcacaaag agcttcaaca ggggagag 2088
<210> 2
   <211> 696
   <212> PRT
   <213> Artificial Sequence
<220>
   <223> amino acid sequence of 2E12CH1CH2CH3Ck (X0130)
<400> 2
   Gln Val Gln Leu Lys Glu Ser Gly Pro Gly Ser Val Ala Pro Ser Gln
   1 5 10 15
   Ser Leu Ser Ile Thr Cys Thr Val Ser Gly Phe Ser Leu Thr Gly Tyr
   20 25 30
   Gly Val Asn Trp Val Arg Gln Pro Pro Gly Lys Gly Leu Glu Trp Leu
   35 40 45
   Gly Met Ile Trp Gly Asp Gly Ser Thr Asp Tyr Asn Ser Ala Leu Lys
   50 55 60
   Ser Arg Leu Ser Ile Thr Lys Asp Asn Ser Lys Ser Gln Val Phe Leu
   65 70 75 80
   Lys Met Asn Ser Leu Gln Thr Asp Asp Thr Ala Arg Tyr Tyr Cys Ala
   85 90 95
   Arg Asp Gly Tyr Ser Asn Phe His Tyr Tyr Val Met Asp Tyr Trp Gly
   100 105 110
   Gln Gly Thr Ser Val Thr Val Ser Ser Gly Gly Gly Gly Ser Gly Gly
   115 120 125
   Gly Gly Ser Gly Gly Gly Gly Ser Gly Gly Gly Gly Ser Asp Ile Val
   130 135 140
   Leu Thr Gln Ser Pro Ala Ser Leu Ala Val Ser Leu Gly Gln Arg Ala
   145 150 155 160
   Thr Ile Ser Cys Arg Ala Ser Glu Ser Val Glu Tyr Tyr Val Thr Ser
   165 170 175
   Leu Met Gln Trp Tyr Gln Gln Lys Pro Gly Gln Pro Pro Lys Leu Leu
   180 185 190
   Ile Ser Ala Ala Ser Asn Val Glu Ser Gly Val Pro Ala Arg Phe Ser
   195 200 205
   Gly Ser Gly Ser Gly Thr Asp Phe Ser Leu Asn Ile His Pro Val Glu
   210 215 220
   Glu Asp Asp Ile Ala Met Tyr Phe Cys Gln Gln Ser Arg Lys Val Pro
   225 230 235 240
   Trp Thr Phe Gly Gly Gly Thr Lys Leu Glu Ile Lys Ser Ser Ser Ala
   245 250 255
   Ser Thr Lys Gly Pro Ser Val Phe Pro Leu Ala Pro Ser Ser Lys Ser
   260 265 270
   Thr Ser Gly Gly Thr Ala Ala Leu Gly Cys Leu Val Lys Asp Tyr Phe
   275 280 285
   Pro Glu Pro Val Thr Val Ser Trp Asn Ser Gly Ala Leu Thr Ser Gly
   290 295 300
   Val His Thr Phe Pro Ala Val Leu Gln Ser Ser Gly Leu Tyr Ser Leu
   305 310 315 320
   Ser Ser Val Val Thr Val Pro Ser Ser Ser Leu Gly Thr Gln Thr Tyr
   325 330 335
   Ile Cys Asn Val Asn His Lys Pro Ser Asn Thr Lys Val Asp Lys Lys
   340 345 350
   Val Arg Thr Glu Pro Lys Ser Ser Asp Lys Thr His Thr Cys Pro Pro
   355 360 365
   Cys Pro Ser Gly Ala Pro Glu Leu Leu Gly Gly Pro Ser Val Phe Leu
   370 375 380
   Phe Pro Pro Lys Pro Lys Asp Thr Leu Met Ile Ser Arg Thr Pro Glu
   385 390 395 400
   Val Thr Cys Val Val Val Asp Val Ser His Glu Asp Pro Glu Val Lys
                   405                 410                 415
   Phe Asn Trp Tyr Val Asp Gly Val Glu Val His Asn Ala Lys Thr Lys
               420                 425                 430
   Pro Arg Glu Glu Gln Tyr Asn Ser Thr Tyr Arg Val Val Ser Val Leu
           435                 440                 445
   Thr Val Leu His Gln Asp Trp Leu Asn Gly Lys Glu Tyr Lys Cys Lys
       450                 455                 460
   Val Ser Asn Lys Ala Leu Pro Ala Pro Ile Glu Lys Thr Ile Ser Lys
   465                 470                 475                 480
   Ala Lys Gly Gln Pro Arg Glu Pro Gln Val Tyr Thr Leu Pro Pro Ser
                   485                 490                 495
   Arg Asp Glu Leu Thr Lys Asn Gln Val Ser Leu Thr Cys Leu Val Lys
               500                 505                 510
   Gly Phe Tyr Pro Ser Asp Ile Ala Val Glu Trp Glu Ser Asn Gly Gln
           515                 520                 525
   Pro Glu Asn Asn Tyr Lys Thr Thr Pro Pro Val Leu Asp Ser Asp Gly
       530                 535                 540
   Ser Phe Phe Leu Tyr Ser Lys Leu Thr Val Asp Lys Ser Arg Trp Gln
   545                 550                 555                 560
   Gln Gly Asn Val Phe Ser Cys Ser Val Met His Glu Ala Leu His Asn
                   565                 570                 575
   His Tyr Thr Gln Lys Ser Leu Ser Leu Ser Pro Gly Lys Ser Arg Thr
               580                 585                 590
   Val Ala Ala Pro Ser Val Phe Ile Phe Pro Pro Ser Asp Glu Gln Leu
           595                 600                 605
   Lys Ser Gly Thr Ala Ser Val Val Cys Leu Leu Asn Asn Phe Tyr Pro
       610                 615                 620
   Arg Glu Ala Lys Val Gln Trp Lys Val Asp Asn Ala Leu Gln Ser Gly
   625                 630                 635                 640
   Asn Ser Gln Glu Ser Val Thr Glu Gln Asp Ser Lys Asp Ser Thr Tyr
                   645                 650                 655
   Ser Leu Ser Ser Thr Leu Thr Leu Ser Lys Ala Asp Tyr Glu Lys His
               660                 665                 670
   Lys Val Tyr Ala Cys Glu Val Thr His Gln Gly Leu Ser Ser Pro Val
           675                 680                 685
   Thr Lys Ser Phe Asn Arg Gly Glu
       690                 695
<210> 3
   <211> 2142
   <212> DNA
   <213> Artificial Sequence
<220>
   <223> Nucleotide sequence of CKCH2CH3CH1-H68-2E12 (X0168)
<400> 3
   actgtggctg caccatctgt cttcatcttc ccgccatctg atgagcagtt gaaatctgga 60
   actgcctctg ttgtgtgcct gctgaataac ttctatccca gagaggccaa agtacagtgg 120
   aaggtggata acgccctcca atcgggtaac tcccaggaga gtgtcacaga gcaggacagc 180
   aaggacagca cctacagcct cagcagcacc ctgacgctga gcaaagcaga ctacgagaaa 240
   cacaaagtct acgcctgcga agtcacccat cagggcctga gctcgcccgt cacaaagagc 300
   ttcaacaggg gagagcgtac ggagcccaaa tcttctgaca aaactcacac atgcccaccg 360
   tgcccatccg gagcacctga actcctgggt ggaccgtcag tcttcctctt ccccccaaaa 420
   cccaaggaca ccctcatgat ctcccggacc cctgaggtca catgcgtggt ggtggacgtg 480
   agccacgaag accctgaggt caagttcaac tggtacgtgg acggcgtgga ggtgcataat 540
   gccaagacaa agccgcggga ggagcagtac aacagcacgt accgtgtggt cagcgtcctc 600
   accgtcctgc accaggactg gctgaatggc aaggagtaca agtgcaaggt ctccaacaaa 660
   gccctcccag cccccatcga gaaaaccatc tccaaagcca aagggcagcc ccgagaacca 720
   caggtgtaca ccctgccccc atcccgggat gagctgacca agaaccaggt cagcctgacc 780
   tgcctggtca aaggcttcta tccaagcgac atcgccgtgg agtgggagag caatgggcag 840
   ccggagaaca actacaagac cacgcctccc gtgctggact ccgacggctc cttcttcctc 900
   tacagcaagc tcaccgtgga caagagcagg tggcagcagg ggaacgtctt ctcatgctcc 960
   gtgatgcatg aggctctgca caaccactac acgcagaaga gcctctccct gtctccgggt 1020
   aaatctagag cctccaccaa gggcccatcg gtcttccccc tggcaccctc ctccaagagc 1080
   acctctgggg gcacagcggc cctgggctgc ctggtcaagg actacttccc cgagccggtg 1140
   acggtgtcgt ggaactcagg cgccctgacc agcggcgtgc acaccttccc ggctgtccta 1200
   cagtcctcag gactctactc cctcagcagc gtggtgaccg tgccctccag cagcttgggc 1260
   acccagacct acatctgcaa cgtgaatcac aagcccagca acaccaaggt ggacaagaaa 1320
   gttaactcat tattcaacca agaagttcaa attcccttga ccgaaagtta cagcccgaat 1380
   tctcaggtgc agctgaagga gtcaggacct ggcagcgtgg cgccctcaca gagcctgtcc 1440
   atcacatgca ccgtctcagg gttctcatta accggctatg gtgtaaactg ggttcgccag 1500
   cctccaggaa agggtctgga gtggctggga atgatatggg gtgatggaag cacagactat 1560
   aattcagctc tcaaatccag actatcgatc accaaggaca actccaagag ccaagttttc 1620
   ttaaaaatga acagtctgca aactgatgac acagccagat actactgtgc tcgggatggt 1680
   tatagtaact ttcattacta tgttatggac tactggggtc aaggaacctc agtcaccgtc 1740
   tcctctgggg gtggaggctc tggtggcggt ggatccggcg gaggtgggtc gggtggcggc 1800
   ggatctgaca ttgtgctcac ccaatctcca gcttctttgg ctgtgtctct aggtcagaga 1860
   gccaccatct cctgcagagc cagtgaaagt gttgaatatt atgtcacaag tttaatgcag 1920
   tggtaccaac agaaaccagg acagccaccc aaactcctca tctctgctgc tagcaacgta 1980
   gaatctgggg tccctgccag gtttagtggc agtgggtctg ggacagactt tagcctcaac 2040
   atccatcctg tggaggagga tgatattgca atgtatttct gtcagcaaag taggaaggtt 2100
   ccatggacgt tcggtggagg caccaagctg gaaatcaagt ga 2142
<210> 4
   <211> 713
   <212> PRT
   <213> Artificial Sequence
<220>
   <223> Amino acid sequence of CKCH2CH3CH1-H68-2E12 (X0168)
<400> 4
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Arg Thr Glu Pro Lys Ser Ser
               100                 105                 110
   Asp Lys Thr His Thr Cys Pro Pro Cys Pro Ser Gly Ala Pro Glu Leu
           115                 120                 125
   Leu Gly Gly Pro Ser Val Phe Leu Phe Pro Pro Lys Pro Lys Asp Thr
       130                 135                 140
   Leu Met Ile Ser Arg Thr Pro Glu Val Thr Cys Val Val Val Asp Val
   145                 150                 155                 160
   Ser His Glu Asp Pro Glu Val Lys Phe Asn Trp Tyr Val Asp Gly Val
                   165                 170                 175
   Glu Val His Asn Ala Lys Thr Lys Pro Arg Glu Glu Gln Tyr Asn Ser
               180                 185                 190
   Thr Tyr Arg Val Val Ser Val Leu Thr Val Leu His Gln Asp Trp Leu
           195                 200                 205
   Asn Gly Lys Glu Tyr Lys Cys Lys Val Ser Asn Lys Ala Leu Pro Ala
       210                 215                 220
   Pro Ile Glu Lys Thr Ile Ser Lys Ala Lys Gly Gln Pro Arg Glu Pro
   225                 230                 235                 240
   Gln Val Tyr Thr Leu Pro Pro Ser Arg Asp Glu Leu Thr Lys Asn Gln
                   245                 250                 255
   Val Ser Leu Thr Cys Leu Val Lys Gly Phe Tyr Pro Ser Asp Ile Ala
               260                 265                 270
   Val Glu Trp Glu Ser Asn Gly Gln Pro Glu Asn Asn Tyr Lys Thr Thr
           275                 280                 285
   Pro Pro Val Leu Asp Ser Asp Gly Ser Phe Phe Leu Tyr Ser Lys Leu
       290                 295                 300
   Thr Val Asp Lys Ser Arg Trp Gln Gln Gly Asn Val Phe Ser Cys Ser
   305                 310                 315                 320
   Val Met His Glu Ala Leu His Asn His Tyr Thr Gln Lys Ser Leu Ser
                   325                 330                 335
   Leu Ser Pro Gly Lys Ser Arg Ala Ser Thr Lys Gly Pro Ser Val Phe
               340                 345                 350
   Pro Leu Ala Pro Ser Ser Lys Ser Thr Ser Gly Gly Thr Ala Ala Leu
           355                 360                 365
   Gly Cys Leu Val Lys Asp Tyr Phe Pro Glu Pro Val Thr Val Ser Trp
       370                 375                 380
   Asn Ser Gly Ala Leu Thr Ser Gly Val His Thr Phe Pro Ala Val Leu
   385                 390                 395                 400
   Gln Ser Ser Gly Leu Tyr Ser Leu Ser Ser Val Val Thr Val Pro Ser
                   405                 410                 415
   Ser Ser Leu Gly Thr Gln Thr Tyr Ile Cys Asn Val Asn His Lys Pro
               420                 425                 430
   Ser Asn Thr Lys Val Asp Lys Lys Val Asn Ser Leu Phe Asn Gln Glu
           435                 440                 445
   Val Gln Ile Pro Leu Thr Glu Ser Tyr Ser Pro Asn Ser Gln Val Gln
       450                 455                 460
   Leu Lys Glu Ser Gly Pro Gly Ser Val Ala Pro Ser Gln Ser Leu Ser
   465                 470                 475                 480
   Ile Thr Cys Thr Val Ser Gly Phe Ser Leu Thr Gly Tyr Gly Val Asn
                   485                 490                 495
   Trp Val Arg Gln Pro Pro Gly Lys Gly Leu Glu Trp Leu Gly Met Ile
               500                 505                 510
   Trp Gly Asp Gly Ser Thr Asp Tyr Asn Ser Ala Leu Lys Ser Arg Leu
           515                 520                 525
   Ser Ile Thr Lys Asp Asn Ser Lys Ser Gln Val Phe Leu Lys Met Asn
       530                 535                 540
   Ser Leu Gln Thr Asp Asp Thr Ala Arg Tyr Tyr Cys Ala Arg Asp Gly
   545                 550                 555                 560
   Tyr Ser Asn Phe His Tyr Tyr Val Met Asp Tyr Trp Gly Gln Gly Thr
                   565                 570                 575
   Ser Val Thr Val Ser Ser Gly Gly Gly Gly Ser Gly Gly Gly Gly Ser
               580                 585                 590
   Gly Gly Gly Gly Ser Gly Gly Gly Gly Ser Asp Ile Val Leu Thr Gln
           595                 600                 605
   Ser Pro Ala Ser Leu Ala Val Ser Leu Gly Gln Arg Ala Thr Ile Ser
       610                 615                 620
   Cys Arg Ala Ser Glu Ser Val Glu Tyr Tyr Val Thr Ser Leu Met Gln
   625                 630                 635                 640
   Trp Tyr Gln Gln Lys Pro Gly Gln Pro Pro Lys Leu Leu Ile Ser Ala
                   645                 650                 655
   Ala Ser Asn Val Glu Ser Gly Val Pro Ala Arg Phe Ser Gly Ser Gly
               660                 665                 670
   Ser Gly Thr Asp Phe Ser Leu Asn Ile His Pro Val Glu Glu Asp Asp
           675                 680                 685
   Ile Ala Met Tyr Phe Cys Gln Gln Ser Arg Lys Val Pro Trp Thr Phe
       690                 695                 700
   Gly Gly Gly Thr Lys Leu Glu Ile Lys
   705                 710
<210> 5
   <211> 1770
   <212> DNA
   <213> Artificial Sequence
<220>
   <223> Nucleotide sequence of 2E12CH1CH2CH3 (X0112)
<400> 5
   caggtgcagc tgaaggagtc aggacctggc agcgtggcgc cctcacagag cctgtccatc 60
   acatgcaccg tctcagggtt ctcattaacc ggctatggtg taaactgggt tcgccagcct 120
   ccaggaaagg gtctggagtg gctgggaatg atatggggtg atggaagcac agactataat 180
   tcagctctca aatccagact atcgatcacc aaggacaact ccaagagcca agttttctta 240
   aaaatgaaca gtctgcaaac tgatgacaca gccagatact actgtgctcg ggatggttat 300
   agtaactttc attactatgt tatggactac tggggtcaag gaacctcagt caccgtctcc 360
   tctgggggtg gaggctctgg tggcggtgga tccggcggag gtgggtcggg tggcggcgga 420
   tctgacattg tgctcaccca atctccagct tctttggctg tgtctctagg tcagagagcc 480
   accatctcct gcagagccag tgaaagtgtt gaatattatg tcacaagttt aatgcagtgg 540
   taccaacaga aaccaggaca gccacccaaa ctcctcatct ctgctgctag caacgtagaa 600
   tctggggtcc ctgccaggtt tagtggcagt gggtctggga cagactttag cctcaacatc 660
   catcctgtgg aggaggatga tattgcaatg tatttctgtc agcaaagtag gaaggttcca 720
   tggacgttcg gtggaggcac caagctggaa atcaagtcct cgagcgcctc caccaagggc 780
   ccatcggtct tccccctggc accctcctcc aagagcacct ctgggggcac agcggccctg 840
   ggctgcctgg tcaaggacta cttccccgag ccggtgacgg tgtcgtggaa ctcaggcgcc 900
   ctgaccagcg gcgtgcacac cttcccggct gtcctacagt cctcaggact ctactccctc 960
   agcagcgtgg tgaccgtgcc ctccagcagc ttgggcaccc agacctacat ctgcaacgtg 1020
   aatcacaagc ccagcaacac caaggtggac aagaaagttc gtacggagcc caaatcttct 1080
   gacaaaactc acacatgccc accgtgccca tccggagcac ctgaactcct gggtggaccg 1140
   tcagtcttcc tcttcccccc aaaacccaag gacaccctca tgatctcccg gacccctgag 1200
   gtcacatgcg tggtggtgga cgtgagccac gaagaccctg aggtcaagtt caactggtac 1260
   gtggacggcg tggaggtgca taatgccaag acaaagccgc gggaggagca gtacaacagc 1320
   acgtaccgtg tggtcagcgt cctcaccgtc ctgcaccagg actggctgaa tggcaaggag 1380
   tacaagtgca aggtctccaa caaagccctc ccagccccca tcgagaaaac catctccaaa 1440
   gccaaagggc agccccgaga accacaggtg tacaccctgc ccccatcccg ggatgagctg 1500
   accaagaacc aggtcagcct gacctgcctg gtcaaaggct tctatccaag cgacatcgcc 1560
   gtggagtggg agagcaatgg gcagccggag aacaactaca agaccacgcc tcccgtgctg 1620
   gactccgacg gctccttctt cctctacagc aagctcaccg tggacaagag caggtggcag 1680
   caggggaacg tcttctcatg ctccgtgatg catgaggctc tgcacaacca ctacacgcag 1740
   aagagcctct ccctgtctcc gggtaaatga                                  1770
<210> 6
   <211> 589
   <212> PRT
   <213> Artificial Sequence
<220>
   <223> Amino acid sequence of 2E12CH1CH2CH3 (X0112)
<400> 6
   Gln Val Gln Leu Lys Glu Ser Gly Pro Gly Ser Val Ala Pro Ser Gln
    1              5                   10                  15
   Ser Leu Ser Ile Thr Cys Thr Val Ser Gly Phe Ser Leu Thr Gly Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Ser Thr Asp Tyr Asn Ser Ala Leu Lys
       50                  55                  60
   Ser Arg Leu Ser Ile Thr Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Gly Tyr Ser Asn Phe His Tyr Tyr Val Met Asp Tyr Trp Gly
               100                 105                 110
   Gln Gly Thr Ser Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ala Ser Leu Ala Val Ser Leu Gly Gln Arg Ala
   145                 150                 155                 160
   Thr Ile Ser Cys Arg Ala Ser Glu Ser Val Glu Tyr Tyr Val Thr Ser
                   165                 170                 175
   Leu Met Gln Trp Tyr Gln Gln Lys Pro Gly Gln Pro Pro Lys Leu Leu
                   180                 185                 190
   Ile Ser Ala Ala Ser Asn Val Glu Ser Gly Val Pro Ala Arg Phe Ser
           195                 200                 205
   Gly Ser Gly Ser Gly Thr Asp Phe Ser Leu Asn Ile His Pro Val Glu
       210                 215                 220
   Glu Asp Asp Ile Ala Met Tyr Phe Cys Gln Gln Ser Arg Lys Val Pro
   225                 230                 235                 240
   Trp Thr Phe Gly Gly Gly Thr Lys Leu Glu Ile Lys Ser Ser Ser Ala
                   245                 250                 255
   Ser Thr Lys Gly Pro Ser Val Phe Pro Leu Ala Pro Ser Ser Lys Ser
               260                 265                 270
   Thr Ser Gly Gly Thr Ala Ala Leu Gly Cys Leu Val Lys Asp Tyr Phe
           275                 280                 285
   Pro Glu Pro Val Thr Val Ser Trp Asn Ser Gly Ala Leu Thr Ser Gly
       290                 295                 300
   Val His Thr Phe Pro Ala Val Leu Gln Ser Ser Gly Leu Tyr Ser Leu
   305                 310                 315                 320
   Ser Ser Val Val Thr Val Pro Ser Ser Ser Leu Gly Thr Gln Thr Tyr
                   325                 330                 335
   Ile Cys Asn Val Asn His Lys Pro Ser Asn Thr Lys Val Asp Lys Lys
               340                 345                 350
   Val Arg Thr Glu Pro Lys Ser Ser Asp Lys Thr His Thr Cys Pro Pro
           355                 360                 365
   Cys Pro Ser Gly Ala Pro Glu Leu Leu Gly Gly Pro Ser Val Phe Leu
       370                 375                 380
   Phe Pro Pro Lys Pro Lys Asp Thr Leu Met Ile Ser Arg Thr Pro Glu
   385                 390                 395                 400
   Val Thr Cys Val Val Val Asp Val Ser His Glu Asp Pro Glu Val Lys
                   405                 410                 415
   Phe Asn Trp Tyr Val Asp Gly Val Glu Val His Asn Ala Lys Thr Lys
               420                 425                 430
   Pro Arg Glu Glu Gln Tyr Asn Ser Thr Tyr Arg Val Val Ser Val Leu
           435                 440                 445
   Thr Val Leu His Gln Asp Trp Leu Asn Gly Lys Glu Tyr Lys Cys Lys
       450                 455                 460
   Val Ser Asn Lys Ala Leu Pro Ala Pro Ile Glu Lys Thr Ile Ser Lys
   465                 470                 475                 480
   Ala Lys Gly Gln Pro Arg Glu Pro Gln Val Tyr Thr Leu Pro Pro Ser
                   485                 490                 495
   Arg Asp Glu Leu Thr Lys Asn Gln Val Ser Leu Thr Cys Leu Val Lys
               500                 505                 510
   Gly Phe Tyr Pro Ser Asp Ile Ala Val Glu Trp Glu Ser Asn Gly Gln
           515                 520                 525
   Pro Glu Asn Asn Tyr Lys Thr Thr Pro Pro Val Leu Asp Ser Asp Gly
       530                 535                 540
   Ser Phe Phe Leu Tyr Ser Lys Leu Thr Val Asp Lys Ser Arg Trp Gln
   545                 550                 555                 560
   Gln Gly Asn Val Phe Ser Cys Ser Val Met His Glu Ala Leu His Asn
                   565                 570                 575
   His Tyr Thr Gln Lys Ser Leu Ser Leu Ser Pro Gly Lys
               580                 585
<210> 7
   <211> 1026
   <212> DNA
   <213> Artificial Sequence
<220>
   <223> Nucleotide sequence of CkCH2CH3 (X0113)
<400> 7
   actgtggctg caccatctgt cttcatcttc ccgccatctg atgagcagtt gaaatctgga 60
   actgcctctg ttgtgtgcct gctgaataac ttctatccca gagaggccaa agtacagtgg 120
   aaggtggata acgccctcca atcgggtaac tcccaggaga gtgtcacaga gcaggacagc 180
   aaggacagca cctacagcct cagcagcacc ctgacgctga gcaaagcaga ctacgagaaa 240
   cacaaagtct acgcctgcga agtcacccat cagggcctga gctcgcccgt cacaaagagc 300
   ttcaacaggg gagagcgtac ggagcccaaa tcttctgaca aaactcacac atgcccaccg 360
   tgcccatccg gagcacctga actcctgggt ggaccgtcag tcttcctctt ccccccaaaa 420
   cccaaggaca ccctcatgat ctcccggacc cctgaggtca catgcgtggt ggtggacgtg 480
   agccacgaag accctgaggt caagttcaac tggtacgtgg acggcgtgga ggtgcataat 540
   gccaagacaa agccgcggga ggagcagtac aacagcacgt accgtgtggt cagcgtcctc 600
   accgtcctgc accaggactg gctgaatggc aaggagtaca agtgcaaggt ctccaacaaa 660
   gccctcccag cccccatcga gaaaaccatc tccaaagcca aagggcagcc ccgagaacca 720
   caggtgtaca ccctgccccc atcccgggat gagctgacca agaaccaggt cagcctgacc 780
   tgcctggtca aaggcttcta tccaagcgac atcgccgtgg agtgggagag caatgggcag 840
   ccggagaaca actacaagac cacgcctccc gtgctggact ccgacggctc cttcttcctc 900
   tacagcaagc tcaccgtgga caagagcagg tggcagcagg ggaacgtctt ctcatgctcc 960
   gtgatgcatg aggctctgca caaccactac acgcagaaga gcctctccct gtctccgggt 1020
   aaatga                                                            1026
<210> 8
   <211> 341
   <212> PRT
   <213> Artificial Sequence
<220>
   <223> Amino acid sequence of CkCH2CH3 (X0113)
<400> 8
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Arg Thr Glu Pro Lys Ser Ser
               100                 105                 110
   Asp Lys Thr His Thr Cys Pro Pro Cys Pro Ser Gly Ala Pro Glu Leu
           115                 120                 125
   Leu Gly Gly Pro Ser Val Phe Leu Phe Pro Pro Lys Pro Lys Asp Thr
       130                 135                 140
   Leu Met Ile Ser Arg Thr Pro Glu Val Thr Cys Val Val Val Asp Val
   145                 150                 155                 160
   Ser His Glu Asp Pro Glu Val Lys Phe Asn Trp Tyr Val Asp Gly Val
                   165                 170                 175
   Glu Val His Asn Ala Lys Thr Lys Pro Arg Glu Glu Gln Tyr Asn Ser
               180                 185                 190
   Thr Tyr Arg Val Val Ser Val Leu Thr Val Leu His Gln Asp Trp Leu
           195                 200                 205
   Asn Gly Lys Glu Tyr Lys Cys Lys Val Ser Asn Lys Ala Leu Pro Ala
       210                 215                 220
   Pro Ile Glu Lys Thr Ile Ser Lys Ala Lys Gly Gln Pro Arg Glu Pro
   225                 230                 235                 240
   Gln Val Tyr Thr Leu Pro Pro Ser Arg Asp Glu Leu Thr Lys Asn Gln
                   245                 250                 255
   Val Ser Leu Thr Cys Leu Val Lys Gly Phe Tyr Pro Ser Asp Ile Ala
               260                 265                 270
   Val Glu Trp Glu Ser Asn Gly Gln Pro Glu Asn Asn Tyr Lys Thr Thr
           275                 280                 285
   Pro Pro Val Leu Asp Ser Asp Gly Ser Phe Phe Leu Tyr Ser Lys Leu
       290                 295                 300
   Thr Val Asp Lys Ser Arg Trp Gln Gln Gly Asn Val Phe Ser Cys Ser
   305                 310                 315                 320
   Val Met His Glu Ala Leu His Asn His Tyr Thr Gln Lys Ser Leu Ser
                   325                 330                 335
   Leu Ser Pro Gly Lys
               340
<210> 9
   <211> 1839
   <212> DNA
   <213> Artificial Sequence
<220>
   <223> Nucleotide sequence of Ck(YAE) Fc (N297A) 2E12 (X0244)
<400> 9
   actgtggctg caccatctgt cttcatcttc ccgccatctg atgagcagtt gaaatctgga 60
   actgcctctg ttgtgtgcct gctgaattac ttctatccca gagaggccaa agtacagtgg 120
   aaggtggata acgccctcca atcgggtaac tcccaggaga gtgccacaga gcaggacagc 180
   aaggacagca cctacagcct cagcagcgag ctgacgctga gcaaagcaga ctacgagaaa 240
   cacaaagtct acgcctgcga agtcacccat cagggcctga gctcgcccgt cacaaagagc 300
   ttcaacaggg gagagcgtac ggagcccaaa tcttctgaca aaactcacac atgcccaccg 360
   tgcccatccg gagcacctga actcctgggt ggaccgtcag tcttcctctt ccccccaaaa 420
   cccaaggaca ccctcatgat ctcccggacc cctgaggtca catgcgtggt ggtggacgtg 480
   agccacgaag accctgaggt caagttcaac tggtacgtgg acggcgtgga ggtgcataat 540
   gccaagacaa agccgcggga ggagcagtac gccagcacgt accgtgtggt cagcgtcctc 600
   accgtcctgc accaggactg gctgaatggc aaggagtaca agtgcaaggt ctccaacaaa 660
   gccctcccag cccccatcga gaaaaccatc tccaaagcca aagggcagcc ccgagaacca 720
   caggtgtaca ccctgccccc atcccgggat gagctgacca agaaccaggt cagcctgacc 780
   tgcctggtca aaggcttcta tccaagcgac atcgccgtgg agtgggagag caatgggcag 840
   ccggagaaca actacaagac cacgcctccc gtgctggact ccgacggctc cttcttcctc 900
   tacagcaagc tcaccgtgga caagagcagg tggcagcagg ggaacgtctt ctcatgctcc 960
   gtgatgcatg aggctctgca caaccactac acgcagaaga gcctctccct gtctccgggt 1020
   aactcattat tcaaccaaga agttcaaatt cccttgaccg aaagttacag cccgaattct 1080
   caggtgcagc tgaaggagtc aggacctggc agcgtggcgc cctcacagag cctgtccatc 1140
   acatgcaccg tctcagggtt ctcattaacc ggctatggtg taaactgggt tcgccagcct 1200
   ccaggaaagg gtctggagtg gctgggaatg atatggggtg atggaagcac agactataat 1260
   tcagctctca aatccagact atcgatcacc aaggacaact ccaagagcca agttttctta 1320
   aaaatgaaca gtctgcaaac tgatgacaca gccagatact actgtgctcg ggatggttat 1380
   agtaactttc attactatgt tatggactac tggggtcaag gaacctcagt caccgtctcc 1440
   tctgggggtg gaggctctgg tggcggtgga tccggcggag gtgggtcggg tggcggcgga 1500
   tctgacattg tgctcaccca atctccagct tctttggctg tgtctctagg tcagagagcc 1560
   accatctcct gcagagccag tgaaagtgtt gaatattatg tcacaagttt aatgcagtgg 1620
   taccaacaga aaccaggaca gccacccaaa ctcctcatct ctgctgctag caacgtagaa 1680
   tctggggtcc ctgccaggtt tagtggcagt gggtctggga cagactttag cctcaacatc 1740
   catcctgtgg aggaggatga tattgcaatg tatttctgtc agcaaagtag gaaggttcca 1800
   tggacgttcg gtggaggcac caagctggaa atcaagtga                        1839
<210> 10
   <211> 612
   <212> PRT
   <213> Artificial Sequence
<220>
   <223> Amino acid sequence of Ck(YAE) Fc (N297A) 2E12 (X0244)
<400> 10
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Tyr Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Arg Thr Glu Pro Lys Ser Ser
               100                 105                 110
   Asp Lys Thr His Thr Cys Pro Pro Cys Pro Ser Gly Ala Pro Glu Leu
           115                 120                 125
   Leu Gly Gly Pro Ser Val Phe Leu Phe Pro Pro Lys Pro Lys Asp Thr
       130                 135                 140
   Leu Met Ile Ser Arg Thr Pro Glu Val Thr Cys Val Val Val Asp Val
   145                 150                 155                 160
   Ser His Glu Asp Pro Glu Val Lys Phe Asn Trp Tyr Val Asp Gly Val
                   165                 170                 175
   Glu Val His Asn Ala Lys Thr Lys Pro Arg Glu Glu Gln Tyr Ala Ser
               180                 185                 190
   Thr Tyr Arg Val Val Ser Val Leu Thr Val Leu His Gln Asp Trp Leu
           195                 200                 205
   Asn Gly Lys Glu Tyr Lys Cys Lys Val Ser Asn Lys Ala Leu Pro Ala
       210                 215                 220
   Pro Ile Glu Lys Thr Ile Ser Lys Ala Lys Gly Gln Pro Arg Glu Pro
   225                 230                 235                 240
   Gln Val Tyr Thr Leu Pro Pro Ser Arg Asp Glu Leu Thr Lys Asn Gln
                   245                 250                 255
   Val Ser Leu Thr Cys Leu Val Lys Gly Phe Tyr Pro Ser Asp Ile Ala
               260                 265                 270
   Val Glu Trp Glu Ser Asn Gly Gln Pro Glu Asn Asn Tyr Lys Thr Thr
           275                 280                 285
   Pro Pro Val Leu Asp Ser Asp Gly Ser Phe Phe Leu Tyr Ser Lys Leu
       290                 295                 300
   Thr Val Asp Lys Ser Arg Trp Gln Gln Gly Asn Val Phe Ser Cys Ser
   305                 310                 315                 320
   Val Met His Glu Ala Leu His Asn His Tyr Thr Gln Lys Ser Leu Ser
                   325                 330                 335
   Leu Ser Pro Gly Asn Ser Leu Phe Asn Gln Glu Val Gln Ile Pro Leu
               340                 345                 350
   Thr Glu Ser Tyr Ser Pro Asn Ser Gln Val Gln Leu Lys Glu Ser Gly
           355                 360                 365
   Pro Gly Ser Val Ala Pro Ser Gln Ser Leu Ser Ile Thr Cys Thr Val
       370                 375                 380
   Ser Gly Phe Ser Leu Thr Gly Tyr Gly Val Asn Trp Val Arg Gln Pro
   385                 390                 395                 400
   Pro Gly Lys Gly Leu Glu Trp Leu Gly Met Ile Trp Gly Asp Gly Ser
                   405                 410                 415
   Thr Asp Tyr Asn Ser Ala Leu Lys Ser Arg Leu Ser Ile Thr Lys Asp
               420                 425                 430
   Asn Ser Lys Ser Gln Val Phe Leu Lys Met Asn Ser Leu Gln Thr Asp
           435                 440                 445
   Asp Thr Ala Arg Tyr Tyr Cys Ala Arg Asp Gly Tyr Ser Asn Phe His
       450                 455                 460
   Tyr Tyr Val Met Asp Tyr Trp Gly Gln Gly Thr Ser Val Thr Val Ser
   465                 470                 475                 480
   Ser Gly Gly Gly Gly Ser Gly Gly Gly Gly Ser Gly Gly Gly Gly Ser
                   485                 490                 495
   Gly Gly Gly Gly Ser Asp Ile Val Leu Thr Gln Ser Pro Ala Ser Leu
               500                 505                 510
   Ala Val Ser Leu Gly Gln Arg Ala Thr Ile Ser Cys Arg Ala Ser Glu
           515                 520                 525
   Ser Val Glu Tyr Tyr Val Thr Ser Leu Met Gln Trp Tyr Gln Gln Lys
       530                 535                 540
   Pro Gly Gln Pro Pro Lys Leu Leu Ile Ser Ala Ala Ser Asn Val Glu
   545                 550                 555                 560
   Ser Gly Val Pro Ala Arg Phe Ser Gly Ser Gly Ser Gly Thr Asp Phe
                   565                 570                 575
   Ser Leu Asn Ile His Pro Val Glu Glu Asp Asp Ile Ala Met Tyr Phe
               580                 585                 590
   Cys Gln Gln Ser Arg Lys Val Pro Trp Thr Phe Gly Gly Gly Thr Lys
           595                 600                 605
   Leu Glu Ile Lys
       610
<210> 11
   <211> 1770
   <212> DNA
   <213> Artificial Sequence
<220>
   <223> Nucleotide sequence of 2E12 CH1 Fc (N297A) (X0245)
<400> 11
   caggtgcagc tgaaggagtc aggacctggc agcgtggcgc cctcacagag cctgtccatc 60
   acatgcaccg tctcagggtt ctcattaacc ggctatggtg taaactgggt tcgccagcct 120
   ccaggaaagg gtctggagtg gctgggaatg atatggggtg atggaagcac agactataat 180
   tcagctctca aatccagact atcgatcacc aaggacaact ccaagagcca agttttctta 240
   aaaatgaaca gtctgcaaac tgatgacaca gccagatact actgtgctcg ggatggttat 300
   agtaactttc attactatgt tatggactac tggggtcaag gaacctcagt caccgtctcc 360
   tctgggggtg gaggctctgg tggcggtgga tccggcggag gtgggtcggg tggcggcgga 420
   tctgacattg tgctcaccca atctccagct tctttggctg tgtctctagg tcagagagcc 480
   accatctcct gcagagccag tgaaagtgtt gaatattatg tcacaagttt aatgcagtgg 540
   taccaacaga aaccaggaca gccacccaaa ctcctcatct ctgctgctag caacgtagaa 600
   tctggggtcc ctgccaggtt tagtggcagt gggtctggga cagactttag cctcaacatc 660
   catcctgtgg aggaggatga tattgcaatg tatttctgtc agcaaagtag gaaggttcca 720
   tggacgttcg gtggaggcac caagctggaa atcaagtcct cgagcgcctc caccaagggc 780
   ccatcggtct tccccctggc accctcctcc aagagcacct ctgggggcac agcggccctg 840
   ggctgcctgg tcaaggacta cttccccgag ccggtgacgg tgtcgtggaa ctcaggcgcc 900
   ctgaccagcg gcgtgcacac cttcccggct gtcctacagt cctcaggact ctactccctc 960
   agcagcgtgg tgaccgtgcc ctccagcagc ttgggcaccc agacctacat ctgcaacgtg 1020
   aatcacaagc ccagcaacac caaggtggac aagaaagttc gtacggagcc caaatcttct 1080
   gacaaaactc acacatgccc accgtgccca tccggagcac ctgaactcct gggtggaccg 1140
   tcagtcttcc tcttcccccc aaaacccaag gacaccctca tgatctcccg gacccctgag 1200
   gtcacatgcg tggtggtgga cgtgagccac gaagaccctg aggtcaagtt caactggtac 1260
   gtggacggcg tggaggtgca taatgccaag acaaagccgc gggaggagca gtacgccagc 1320
   acgtaccgtg tggtcagcgt cctcaccgtc ctgcaccagg actggctgaa tggcaaggag 1380
   tacaagtgca aggtctccaa caaagccctc ccagccccca tcgagaaaac catctccaaa 1440
   gccaaagggc agccccgaga accacaggtg tacaccctgc ccccatcccg ggatgagctg 1500
   accaagaacc aggtcagcct gacctgcctg gtcaaaggct tctatccaag cgacatcgcc 1560
   gtggagtggg agagcaatgg gcagccggag aacaactaca agaccacgcc tcccgtgctg 1620
   gactccgacg gctccttctt cctctacagc aagctcaccg tggacaagag caggtggcag 1680
   caggggaacg tcttctcatg ctccgtgatg catgaggctc tgcacaacca ctacacgcag 1740
   aagagcctct ccctgtctcc gggtaaatga                                  1770
<210> 12
   <211> 589
   <212> PRT
   <213> Artificial Sequence
<220>
   <223> Amino acid sequence of 2E12 CH1 Fc (N297A) (X0245)
<400> 12
   Gln Val Gln Leu Lys Glu Ser Gly Pro Gly Ser Val Ala Pro Ser Gln
    1               5                  10                  15
   Ser Leu Ser Ile Thr Cys Thr Val Ser Gly Phe Ser Leu Thr Gly Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Ser Thr Asp Tyr Asn Ser Ala Leu Lys
       50                  55                  60
   Ser Arg Leu Ser Ile Thr Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Gly Tyr Ser Asn Phe His Tyr Tyr Val Met Asp Tyr Trp Gly
               100                 105                 110
   Gln Gly Thr Ser Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ala Ser Leu Ala Val Ser Leu Gly Gln Arg Ala
   145                 150                 155                 160
   Thr Ile Ser Cys Arg Ala Ser Glu Ser Val Glu Tyr Tyr Val Thr Ser
                   165                 170                 175
   Leu Met Gln Trp Tyr Gln Gln Lys Pro Gly Gln Pro Pro Lys Leu Leu
               180                 185                 190
   Ile Ser Ala Ala Ser Asn Val Glu Ser Gly Val Pro Ala Arg Phe Ser
           195                 200                 205
   Gly Ser Gly Ser Gly Thr Asp Phe Ser Leu Asn Ile His Pro Val Glu
       210                 215                 220
   Glu Asp Asp Ile Ala Met Tyr Phe Cys Gln Gln Ser Arg Lys Val Pro
   225                 230                 235                 240
   Trp Thr Phe Gly Gly Gly Thr Lys Leu Glu Ile Lys Ser Ser Ser Ala
                   245                 250                 255
   Ser Thr Lys Gly Pro Ser Val Phe Pro Leu Ala Pro Ser Ser Lys Ser
               260                 265                 270
   Thr Ser Gly Gly Thr Ala Ala Leu Gly Cys Leu Val Lys Asp Tyr Phe
           275                 280                 285
   Pro Glu Pro Val Thr Val Ser Trp Asn Ser Gly Ala Leu Thr Ser Gly
       290                 295                 300
   Val His Thr Phe Pro Ala Val Leu Gln Ser Ser Gly Leu Tyr Ser Leu
   305                 310                 315                 320
   Ser Ser Val Val Thr Val Pro Ser Ser Ser Leu Gly Thr Gln Thr Tyr
                   325                 330                 335
   Ile Cys Asn Val Asn His Lys Pro Ser Asn Thr Lys Val Asp Lys Lys
               340                 345                 350
   Val Arg Thr Glu Pro Lys Ser Ser Asp Lys Thr His Thr Cys Pro Pro
           355                 360                 365
   Cys Pro Ser Gly Ala Pro Glu Leu Leu Gly Gly Pro Ser Val Phe Leu
       370                 375                 380
   Phe Pro Pro Lys Pro Lys Asp Thr Leu Met Ile Ser Arg Thr Pro Glu
   385                 390                 395                 400
   Val Thr Cys Val Val Val Asp Val Ser His Glu Asp Pro Glu Val Lys
                   405                 410                 415
   Phe Asn Trp Tyr Val Asp Gly Val Glu Val His Asn Ala Lys Thr Lys
               420                 425                 430
   Pro Arg Glu Glu Gln Tyr Ala Ser Thr Tyr Arg Val Val Ser Val Leu
           435                 440                 445
   Thr Val Leu His Gln Asp Trp Leu Asn Gly Lys Glu Tyr Lys Cys Lys
       450                 455                 460
   Val Ser Asn Lys Ala Leu Pro Ala Pro Ile Glu Lys Thr Ile Ser Lys
   465                 470                 475                 480
   Ala Lys Gly Gln Pro Arg Glu Pro Gln Val Tyr Thr Leu Pro Pro Ser
                   485                 490                 495
   Arg Asp Glu Leu Thr Lys Asn Gln Val Ser Leu Thr Cys Leu Val Lys
               500                 505                 510
   Gly Phe Tyr Pro Ser Asp Ile Ala Val Glu Trp Glu Ser Asn Gly Gln
           515                 520                 525
   Pro Glu Asn Asn Tyr Lys Thr Thr Pro Pro Val Leu Asp Ser Asp Gly
       530                 535                 540
   Ser Phe Phe Leu Tyr Ser Lys Leu Thr Val Asp Lys Ser Arg Trp Gln
   545                 550                 555                 560
   Gln Gly Asn Val Phe Ser Cys Ser Val Met His Glu Ala Leu His Asn
                       565                 570                 575
   His Tyr Thr Gln Lys Ser Leu Ser Leu Ser Pro Gly Lys
                   580                 585
<210> 13
   <211> 1749
   <212> DNA
   <213> Artificial Sequence
<220>
   <223> Nucleotide sequence of P2C2 Ck(YAE) Fc (N297A) (X0246)
<400> 13
   gatattgtga tgatccagga tgaactctcc aatcctgtca gttctggaga atcagtttcc 60
   atctcctgta ggtctagtaa gagtctccta gataaggaag ggaagacata cttgaattgg 120
   tttctgcaga gaccaggaca atctcctcag ctcctgatct atctgatgtc catgcgtgaa 180
   tcaggagtct cagaccggtt tagtggcagt gggtcaggaa cagatttcac cctggaaatc 240
   agtagagtga aggctgagga tgtgggtgtg tattactgtc aacaacttgt agagtatccg 300
   tggacgttcg gtggaggcac caagctggaa atcaaaggtg gcggtggctc tggcggaggt 360
   ggatccggtg gcggcggctc tcagatccag ttggtgcagt ctggacctga gctgaagaag 420
   cctggagaga cagtcaagat ctcctgcaag gcttctggtt ataccttcac agacttttca 480
   atgcactggg tgaggcaggc tccaggaaag ggtttaaagt ggatgggctg gataaacact 540
   gagactggtg agccaacata tgcagatgac ttcaggggac ggtttgcctt ctctttggaa 600
   acttctgcca gcactgccta tttgcagatc aacaacctca aaaatgagga cacggctaca 660
   tatttttgta cctggtctgc ttactggggc caagggactc tggtcactgt ctctgcctcg 720
   agcactgtgg ctgcaccatc tgtcttcatc ttcccgccat ctgatgagca gttgaaatct 780
   ggaactgcct ctgttgtgtg cctgctgaat tacttctatc ccagagaggc caaagtacag 840
   tggaaggtgg ataacgccct ccaatcgggt aactcccagg agagtgccac agagcaggac 900
   agcaaggaca gcacctacag cctcagcagc gagctgacgc tgagcaaagc agactacgag 960
   aaacacaaag tctacgcctg cgaagtcacc catcagggcc tgagctcgcc cgtcacaaag 1020
   agcttcaaca ggggagagcg tacggagccc aaatcttctg acaaaactca cacatgccca 1080
   ccgtgcccat ccggagcacc tgaactcctg ggtggaccgt cagtcttcct cttcccccca 1140
   aaacccaagg acaccctcat gatctcccgg acccctgagg tcacatgcgt ggtggtggac 1200
   gtgagccacg aagaccctga ggtcaagttc aactggtacg tggacggcgt ggaggtgcat 1260
   aatgccaaga caaagccgcg ggaggagcag tacgccagca cgtaccgtgt ggtcagcgtc 1320
   ctcaccgtcc tgcaccagga ctggctgaat ggcaaggagt acaagtgcaa ggtctccaac 1380
   aaagccctcc cagcccccat cgagaaaacc atctccaaag ccaaagggca gccccgagaa 1440
   ccacaggtgt acaccctgcc cccatcccgg gatgagctga ccaagaacca ggtcagcctg 1500
   acctgcctgg tcaaaggctt ctatccaagc gacatcgccg tggagtggga gagcaatggg 1560
   cagccggaga acaactacaa gaccacgcct cccgtgctgg actccgacgg ctccttcttc 1620
   ctctacagca agctcaccgt ggacaagagc aggtggcagc aggggaacgt cttctcatgc 1680
   tccgtgatgc atgaggctct gcacaaccac tacacgcaga agagcctctc cctgtctccg 1740
   ggtaaatga                                                         1749
<210> 14
   <211> 582
   <212> PRT
   <213> Artificial Sequence
<220>
   <223> amino acid sequence of P2C2 Ck(YAE) Fc (N297A) (X0246)
<400> 14
   Asp Ile Val Met Ile Gln Asp Glu Leu Ser Asn Pro Val Ser Ser Gly
    1               5                  10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
               100                 105                 110
   Gly Gly Gly Gly Ser Gly Gly Gly Gly Ser Gly Gly Gly Gly Ser Gln
           115                 120                 125
   Ile Gln Leu Val Gln Ser Gly Pro Glu Leu Lys Lys Pro Gly Glu Thr
       130                 135                 140
   Val Lys Ile Ser Cys Lys Ala Ser Gly Tyr Thr Phe Thr Asp Phe Ser
   145                 150                 155                 160
   Met His Trp Val Arg Gln Ala Pro Gly Lys Gly Leu Lys Trp Met Gly
                   165                 170                 175
   Trp Ile Asn Thr Glu Thr Gly Glu Pro Thr Tyr Ala Asp Asp Phe Arg
               180                 185                 190
   Gly Arg Phe Ala Phe Ser Leu Glu Thr Ser Ala Ser Thr Ala Tyr Leu
           195                 200                 205
   Gln Ile Asn Asn Leu Lys Asn Glu Asp Thr Ala Thr Tyr Phe Cys Thr
       210                 215                 220
   Trp Ser Ala Tyr Trp Gly Gln Gly Thr Leu Val Thr Val Ser Ala Ser
   225                 230                 235                 240
   Ser Thr Val Ala Ala Pro Ser Val Phe Ile Phe Pro Pro Ser Asp Glu
                   245                 250                 255
   Gln Leu Lys Ser Gly Thr Ala Ser Val Val Cys Leu Leu Asn Tyr Phe
               260                 265                 270
   Tyr Pro Arg Glu Ala Lys Val Gln Trp Lys Val Asp Asn Ala Leu Gln
           275                 280                 285
   Ser Gly Asn Ser Gln Glu Ser Ala Thr Glu Gln Asp Ser Lys Asp Ser
       290                 295                 300
   Thr Tyr Ser Leu Ser Ser Glu Leu Thr Leu Ser Lys Ala Asp Tyr Glu
   305                 310                 315                 320
   Lys His Lys Val Tyr Ala Cys Glu Val Thr His Gln Gly Leu Ser Ser
                   325                 330                 335
   Pro Val Thr Lys Ser Phe Asn Arg Gly Glu Arg Thr Glu Pro Lys Ser
               340                 345                 350
   Ser Asp Lys Thr His Thr Cys Pro Pro Cys Pro Ser Gly Ala Pro Glu
           355                 360                 365
   Leu Leu Gly Gly Pro Ser Val Phe Leu Phe Pro Pro Lys Pro Lys Asp
       370                 375                 380
   Thr Leu Met Ile Ser Arg Thr Pro Glu Val Thr Cys Val Val Val Asp
   385                 390                 395                 400
   Val Ser His Glu Asp Pro Glu Val Lys Phe Asn Trp Tyr Val Asp Gly
                   405                 410                 415
   Val Glu Val His Asn Ala Lys Thr Lys Pro Arg Glu Glu Gln Tyr Ala
               420                 425                 430
   Ser Thr Tyr Arg Val Val Ser Val Leu Thr Val Leu His Gln Asp Trp
           435                 440                 445
   Leu Asn Gly Lys Glu Tyr Lys Cys Lys Val Ser Asn Lys Ala Leu Pro
       450                 455                 460
   Ala Pro Ile Glu Lys Thr Ile Ser Lys Ala Lys Gly Gln Pro Arg Glu
   465                 470                 475                 480
   Pro Gln Val Tyr Thr Leu Pro Pro Ser Arg Asp Glu Leu Thr Lys Asn
                   485                 490                 495
   Gln Val Ser Leu Thr Cys Leu Val Lys Gly Phe Tyr Pro Ser Asp Ile
               500                 505                 510
   Ala Val Glu Trp Glu Ser Asn Gly Gln Pro Glu Asn Asn Tyr Lys Thr
           515                 520                 525
   Thr Pro Pro Val Leu Asp Ser Asp Gly Ser Phe Phe Leu Tyr Ser Lys
       530                 535                 540
   Leu Thr Val Asp Lys Ser Arg Trp Gln Gln Gly Asn Val Phe Ser Cys
   545                 550                 555                 560
   Ser Val Met His Glu Ala Leu His Asn His Tyr Thr Gln Lys Ser Leu
                   565                 570                 575
   Ser Leu Ser Pro Gly Lys
               580
<210> 15
   <211> 2583
   <212> DNA
   <213> Artificial Sequence
<220>
   <223> Nucleotide sequence of 2E12 CH1 Fc (N297A) H68 2E12 (X0247)
<400> 15
   caggtgcagc tgaaggagtc aggacctggc agcgtggcgc cctcacagag cctgtccatc 60
   acatgcaccg tctcagggtt ctcattaacc ggctatggtg taaactgggt tcgccagcct 120
   ccaggaaagg gtctggagtg gctgggaatg atatggggtg atggaagcac agactataat 180
   tcagctctca aatccagact atcgatcacc aaggacaact ccaagagcca agttttctta 240
   aaaatgaaca gtctgcaaac tgatgacaca gccagatact actgtgctcg ggatggttat 300
   agtaactttc attactatgt tatggactac tggggtcaag gaacctcagt caccgtctcc 360
   tctgggggtg gaggctctgg tggcggtgga tccggcggag gtgggtcggg tggcggcgga 420
   tctgacattg tgctcaccca atctccagct tctttggctg tgtctctagg tcagagagcc 480
   accatctcct gcagagccag tgaaagtgtt gaatattatg tcacaagttt aatgcagtgg 540
   taccaacaga aaccaggaca gccacccaaa ctcctcatct ctgctgctag caacgtagaa 600
   tctggggtcc ctgccaggtt tagtggcagt gggtctggga cagactttag cctcaacatc 660
   catcctgtgg aggaggatga tattgcaatg tatttctgtc agcaaagtag gaaggttcca 720
   tggacgttcg gtggaggcac caagctggaa atcaagtcct cgagcgcctc caccaagggc 780
   ccatcggtct tccccctggc accctcctcc aagagcacct ctgggggcac agcggccctg 840
   ggctgcctgg tcaaggacta cttccccgag ccggtgacgg tgtcgtggaa ctcaggcgcc 900
   ctgaccagcg gcgtgcacac cttcccggct gtcctacagt cctcaggact ctactccctc 960
   agcagcgtgg tgaccgtgcc ctccagcagc ttgggcaccc agacctacat ctgcaacgtg 1020
   aatcacaagc ccagcaacac caaggtggac aagaaagttc gtacggagcc caaatcttct 1080
   gacaaaactc acacatgccc accgtgccca tccggagcac ctgaactcct gggtggaccg 1140
   tcagtcttcc tcttcccccc aaaacccaag gacaccctca tgatctcccg gacccctgag 1200
   gtcacatgcg tggtggtgga cgtgagccac gaagaccctg aggtcaagtt caactggtac 1260
   gtggacggcg tggaggtgca taatgccaag acaaagccgc gggaggagca gtacgccagc 1320
   acgtaccgtg tggtcagcgt cctcaccgtc ctgcaccagg actggctgaa tggcaaggag 1380
   tacaagtgca aggtctccaa caaagccctc ccagccccca tcgagaaaac catctccaaa 1440
   gccaaagggc agccccgaga accacaggtg tacaccctgc ccccatcccg ggatgagctg 1500
   accaagaacc aggtcagcct gacctgcctg gtcaaaggct tctatccaag cgacatcgcc 1560
   gtggagtggg agagcaatgg gcagccggag aacaactaca agaccacgcc tcccgtgctg 1620
   gactccgacg gctccttctt cctctacagc aagctcaccg tggacaagag caggtggcag 1680
   caggggaacg tcttctcatg ctccgtgatg catgaggctc tgcacaacca ctacacgcag 1740
   aagagcctct ccctgtctcc gggtaactca ttattcaacc aagaagttca aattcccttg 1800
   accgaaagtt acagcccgaa ttctcaggtg cagctgaagg agtcaggacc tggcagcgtg 1860
   gcgccctcac agagcctgtc catcacatgc accgtctcag ggttctcatt aaccggctat 1920
   ggtgtaaact gggttcgcca gcctccagga aagggtctgg agtggctggg aatgatatgg 1980
   ggtgatggaa gcacagacta taattcagct ctcaaatcca gactatcgat caccaaggac 2040
   aactccaaga gccaagtttt cttaaaaatg aacagtctgc aaactgatga cacagccaga 2100
   tactactgtg ctcgggatgg ttatagtaac tttcattact atgttatgga ctactggggt 2160
   caaggaacct cagtcaccgt ctcctctggg ggtggaggct ctggtggcgg tggatccggc 2220
   ggaggtgggt cgggtggcgg cggatctgac attgtgctca cccaatctcc agcttctttg 2280
   gctgtgtctc taggtcagag agccaccatc tcctgcagag ccagtgaaag tgttgaatat 2340
   tatgtcacaa gtttaatgca gtggtaccaa cagaaaccag gacagccacc caaactcctc 2400
   atctctgctg ctagcaacgt agaatctggg gtccctgcca ggtttagtgg cagtgggtct 2460
   gggacagact ttagcctcaa catccatcct gtggaggagg atgatattgc aatgtatttc 2520
   tgtcagcaaa gtaggaaggt tccatggacg ttcggtggag gcaccaagct ggaaatcaag 2580
   tga                                                               2583
<210> 16
   <211> 860
   <212> PRT
   <213> Artificial Sequence
<220>
   <223> Amino acid sequence of 2E12 CH1 Fc (N297A) H68 2E12 (X0247)
<400> 16
   Gln Val Gln Leu Lys Glu Ser Gly Pro Gly Ser Val Ala Pro Ser Gln
    1               5                  10                  15
   Ser Leu Ser Ile Thr Cys Thr Val Ser Gly Phe Ser Leu Thr Gly Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Ser Thr Asp Tyr Asn Ser Ala Leu Lys
       50                  55                  60
   Ser Arg Leu Ser Ile Thr Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Gly Tyr Ser Asn Phe His Tyr Tyr Val Met Asp Tyr Trp Gly
               100                 105                 110
   Gln Gly Thr Ser Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ala Ser Leu Ala Val Ser Leu Gly Gln Arg Ala
   145                 150                 155                 160
   Thr Ile Ser Cys Arg Ala Ser Glu Ser Val Glu Tyr Tyr Val Thr Ser
                   165                 170                 175
   Leu Met Gln Trp Tyr Gln Gln Lys Pro Gly Gln Pro Pro Lys Leu Leu
               180                 185                 190
   Ile Ser Ala Ala Ser Asn Val Glu Ser Gly Val Pro Ala Arg Phe Ser
           195                 200                 205
   Gly Ser Gly Ser Gly Thr Asp Phe Ser Leu Asn Ile His Pro Val Glu
       210                 215                 220
   Glu Asp Asp Ile Ala Met Tyr Phe Cys Gln Gln Ser Arg Lys Val Pro
   225                 230                 235                 240
   Trp Thr Phe Gly Gly Gly Thr Lys Leu Glu Ile Lys Ser Ser Ser Ala
                   245                 250                 255
   Ser Thr Lys Gly Pro Ser Val Phe Pro Leu Ala Pro Ser Ser Lys Ser
               260                 265                 270
   Thr Ser Gly Gly Thr Ala Ala Leu Gly Cys Leu Val Lys Asp Tyr Phe
           275                 280                 285
   Pro Glu Pro Val Thr Val Ser Trp Asn Ser Gly Ala Leu Thr Ser Gly
       290                 295                 300
   Val His Thr Phe Pro Ala Val Leu Gln Ser Ser Gly Leu Tyr Ser Leu
   305                 310                 315                 320
   Ser Ser Val Val Thr Val Pro Ser Ser Ser Leu Gly Thr Gln Thr Tyr
                   325                 330                 335
   Ile Cys Asn Val Asn His Lys Pro Ser Asn Thr Lys Val Asp Lys Lys
               340                 345                 350
   Val Arg Thr Glu Pro Lys Ser Ser Asp Lys Thr His Thr Cys Pro Pro
           355                 360                 365
   Cys Pro Ser Gly Ala Pro Glu Leu Leu Gly Gly Pro Ser Val Phe Leu
       370                 375                 380
   Phe Pro Pro Lys Pro Lys Asp Thr Leu Met Ile Ser Arg Thr Pro Glu
   385                 390                 395                 400
   Val Thr Cys Val Val Val Asp Val Ser His Glu Asp Pro Glu Val Lys
                   405                 410                 415
   Phe Asn Trp Tyr Val Asp Gly Val Glu Val His Asn Ala Lys Thr Lys
               420                 425                 430
   Pro Arg Glu Glu Gln Tyr Ala Ser Thr Tyr Arg Val Val Ser Val Leu
           435                 440                 445
   Thr Val Leu His Gln Asp Trp Leu Asn Gly Lys Glu Tyr Lys Cys Lys
       450                 455                 460
   Val Ser Asn Lys Ala Leu Pro Ala Pro Ile Glu Lys Thr Ile Ser Lys
   465                 470                 475                 480
   Ala Lys Gly Gln Pro Arg Glu Pro Gln Val Tyr Thr Leu Pro Pro Ser
                   485                 490                 495
   Arg Asp Glu Leu Thr Lys Asn Gln Val Ser Leu Thr Cys Leu Val Lys
                   500                 505                 510
   Gly Phe Tyr Pro Ser Asp Ile Ala Val Glu Trp Glu Ser Asn Gly Gln
           515                 520                 525
   Pro Glu Asn Asn Tyr Lys Thr Thr Pro Pro Val Leu Asp Ser Asp Gly
       530                 535                 540
   Ser Phe Phe Leu Tyr Ser Lys Leu Thr Val Asp Lys Ser Arg Trp Gln
   545                 550                 555                 560
   Gln Gly Asn Val Phe Ser Cys Ser Val Met His Glu Ala Leu His Asn
                   565                 570                 575
   His Tyr Thr Gln Lys Ser Leu Ser Leu Ser Pro Gly Asn Ser Leu Phe
               580                 585                 590
   Asn Gln Glu Val Gln Ile Pro Leu Thr Glu Ser Tyr Ser Pro Asn Ser
           595                 600                 605
   Gln Val Gln Leu Lys Glu Ser Gly Pro Gly Ser Val Ala Pro Ser Gln
       610                 615                 620
   Ser Leu Ser Ile Thr Cys Thr Val Ser Gly Phe Ser Leu Thr Gly Tyr
   625                 630                 635                 640
   Gly Val Asn Trp Val Arg Gln Pro Pro Gly Lys Gly Leu Glu Trp Leu
                   645                 650                 655
   Gly Met Ile Trp Gly Asp Gly Ser Thr Asp Tyr Asn Ser Ala Leu Lys
               660                 665                 670
   Ser Arg Leu Ser Ile Thr Lys Asp Asn Ser Lys Ser Gln Val Phe Leu
           675                 680                 685
   Lys Met Asn Ser Leu Gln Thr Asp Asp Thr Ala Arg Tyr Tyr Cys Ala
       690                 695                 700
   Arg Asp Gly Tyr Ser Asn Phe His Tyr Tyr Val Met Asp Tyr Trp Gly
   705                 710                 715                 720
   Gln Gly Thr Ser Val Thr Val Ser Ser Gly Gly Gly Gly Ser Gly Gly
                   725                 730                 735
   Gly Gly Ser Gly Gly Gly Gly Ser Gly Gly Gly Gly Ser Asp Ile Val
               740                 745                 750
   Leu Thr Gln Ser Pro Ala Ser Leu Ala Val Ser Leu Gly Gln Arg Ala
           755                 760                 765
   Thr Ile Ser Cys Arg Ala Ser Glu Ser Val Glu Tyr Tyr Val Thr Ser
       770                 775                 780
   Leu Met Gln Trp Tyr Gln Gln Lys Pro Gly Gln Pro Pro Lys Leu Leu
   785                 790                 795                 800
   Ile Ser Ala Ala Ser Asn Val Glu Ser Gly Val Pro Ala Arg Phe Ser
                   805                 810                 815
   Gly Ser Gly Ser Gly Thr Asp Phe Ser Leu Asn Ile His Pro Val Glu
               820                 825                 830
   Glu Asp Asp Ile Ala Met Tyr Phe Cys Gln Gln Ser Arg Lys Val Pro
           835                 840                 845
   Trp Thr Phe Gly Gly Gly Thr Lys Leu Glu Ile Lys
       850                 855                 860
<210> 17
   <211> 2547
   <212> DNA
   <213> Artificial Sequence
<220>
   <223> Nucleotide sequence of P2C2 CK(YAE) Fc (N297A) H68 A2 (X0248)
<400> 17
   gatattgtga tgatccagga tgaactctcc aatcctgtca gttctggaga atcagtttcc 60
   atctcctgta ggtctagtaa gagtctccta gataaggaag ggaagacata cttgaattgg 120
   tttctgcaga gaccaggaca atctcctcag ctcctgatct atctgatgtc catgcgtgaa 180
   tcaggagtct cagaccggtt tagtggcagt gggtcaggaa cagatttcac cctggaaatc 240
   agtagagtga aggctgagga tgtgggtgtg tattactgtc aacaacttgt agagtatccg 300
   tggacgttcg gtggaggcac caagctggaa atcaaaggtg gcggtggctc tggcggaggt 360
   ggatccggtg gcggcggctc tcagatccag ttggtgcagt ctggacctga gctgaagaag 420
   cctggagaga cagtcaagat ctcctgcaag gcttctggtt ataccttcac agacttttca 480
   atgcactggg tgaggcaggc tccaggaaag ggtttaaagt ggatgggctg gataaacact 540
   gagactggtg agccaacata tgcagatgac ttcaggggac ggtttgcctt ctctttggaa 600
   acttctgcca gcactgccta tttgcagatc aacaacctca aaaatgagga cacggctaca 660
   tatttttgta cctggtctgc ttactggggc caagggactc tggtcactgt ctctgcctcg 720
   agcactgtgg ctgcaccatc tgtcttcatc ttcccgccat ctgatgagca gttgaaatct 780
   ggaactgcct ctgttgtgtg cctgctgaat tacttctatc ccagagaggc caaagtacag 840
   tggaaggtgg ataacgccct ccaatcgggt aactcccagg agagtgccac agagcaggac 900
   agcaaggaca gcacctacag cctcagcagc gagctgacgc tgagcaaagc agactacgag 960
   aaacacaaag tctacgcctg cgaagtcacc catcagggcc tgagctcgcc cgtcacaaag 1020
   agcttcaaca ggggagagcg tacggagccc aaatcttctg acaaaactca cacatgccca 1080
   ccgtgcccat ccggagcacc tgaactcctg ggtggaccgt cagtcttcct cttcccccca 1140
   aaacccaagg acaccctcat gatctcccgg acccctgagg tcacatgcgt ggtggtggac 1200
   gtgagccacg aagaccctga ggtcaagttc aactggtacg tggacggcgt ggaggtgcat 1260
   aatgccaaga caaagccgcg ggaggagcag tacgccagca cgtaccgtgt ggtcagcgtc 1320
   ctcaccgtcc tgcaccagga ctggctgaat ggcaaggagt acaagtgcaa ggtctccaac 1380
   aaagccctcc cagcccccat cgagaaaacc atctccaaag ccaaagggca gccccgagaa 1440
   ccacaggtgt acaccctgcc cccatcccgg gatgagctga ccaagaacca ggtcagcctg 1500
   acctgcctgg tcaaaggctt ctatccaagc gacatcgccg tggagtggga gagcaatggg 1560
   cagccggaga acaactacaa gaccacgcct cccgtgctgg actccgacgg ctccttcttc 1620
   ctctacagca agctcaccgt ggacaagagc aggtggcagc aggggaacgt cttctcatgc 1680
   tccgtgatgc atgaggctct gcacaaccac tacacgcaga agagcctctc cctgtctccg 1740
   ggtaactcat tattcaacca agaagttcaa attcccttga ccgaaagtta cagcccgaat 1800
   tctgaagttc aattgttaga gtctggtggc ggtcttgttc agcctggtgg ttctttacgt 1860
   ctttcttgcg ctgcttccgg attcactttc tctttttacc agatgcattg ggttcgccaa 1920
   gctcctggta aaggtttgga gtgggtttct ggtatctatc cttctggtgg ctatactaag 1980
   tatgctgact ccgttaaagg tcgcttcact atctctagag acaactctaa gaatactctc 2040
   tacttgcaga tgaacagctt aagggctgag gacactgcag tctactattg tgcgagagat 2100
   ctagggtacg gcagtagctg gtactacttt gactactggg gccagggaac cctggtcacc 2160
   gtctcaagcg gtggcggcgg ttcggggggt ggcggaagtg gaggtggagg gagtcagagc 2220
   gctttgactc agcctgcctc cgtgtctggg tctcctggac agtcgatcac catctcctgc 2280
   agtggaaccg acagtgacgt tggtggttat aaccatgtct cctggtacca acaacaccca 2340
   ggcaaggccc ccaaactcat aatttatgat gtcgatcatc ggccctcagg gatctctaac 2400
   cgtttctctg gctccaagtc tggcaacacg gcctccctga ccatctctgg gctccaggct 2460
   gaggacgagg ctgattatta ctgcagctca tatagaagcg gtagcactta tgtcttcgga 2520
   actgggacca aggtcaccgt cctatga                                    2547
<210> 18
   <211> 848
   <212> PRT
   <213> Artificial Sequence
<220>
   <223> Amino acid sequence of P2C2 CK(YAE) Fc (N297A) H68 A2 (X0248)
<400> 18
   Asp Ile Val Met Ile Gln Asp Glu Leu Ser Asn Pro Val Ser Ser Gly
    1               5                  10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
               100                 105                 110
   Gly Gly Gly Gly Ser Gly Gly Gly Gly Ser Gly Gly Gly Gly Ser Gln
           115                 120                 125
   Ile Gln Leu Val Gln Ser Gly Pro Glu Leu Lys Lys Pro Gly Glu Thr
       130                 135                 140
   Val Lys Ile Ser Cys Lys Ala Ser Gly Tyr Thr Phe Thr Asp Phe Ser
   145                 150                 155                 160
   Met His Trp Val Arg Gln Ala Pro Gly Lys Gly Leu Lys Trp Met Gly
                   165                 170                 175
   Trp Ile Asn Thr Glu Thr Gly Glu Pro Thr Tyr Ala Asp Asp Phe Arg
               180                 185                 190
   Gly Arg Phe Ala Phe Ser Leu Glu Thr Ser Ala Ser Thr Ala Tyr Leu
           195                 200                 205
   Gln Ile Asn Asn Leu Lys Asn Glu Asp Thr Ala Thr Tyr Phe Cys Thr
       210                 215                 220
   Trp Ser Ala Tyr Trp Gly Gln Gly Thr Leu Val Thr Val Ser Ala Ser
   225                 230                 235                 240
   Ser Thr Val Ala Ala Pro Ser Val Phe Ile Phe Pro Pro Ser Asp Glu
                   245                 250                 255
   Gln Leu Lys Ser Gly Thr Ala Ser Val Val Cys Leu Leu Asn Tyr Phe
               260                 265                 270
   Tyr Pro Arg Glu Ala Lys Val Gln Trp Lys Val Asp Asn Ala Leu Gln
           275                 280                 285
   Ser Gly Asn Ser Gln Glu Ser Ala Thr Glu Gln Asp Ser Lys Asp Ser
       290                 295                 300
   Thr Tyr Ser Leu Ser Ser Glu Leu Thr Leu Ser Lys Ala Asp Tyr Glu
   305                 310                 315                 320
   Lys His Lys Val Tyr Ala Cys Glu Val Thr His Gln Gly Leu Ser Ser
                   325                 330                 335
   Pro Val Thr Lys Ser Phe Asn Arg Gly Glu Arg Thr Glu Pro Lys Ser
               340                 345                 350
   Ser Asp Lys Thr His Thr Cys Pro Pro Cys Pro Ser Gly Ala Pro Glu
           355                 360                 365
   Leu Leu Gly Gly Pro Ser Val Phe Leu Phe Pro Pro Lys Pro Lys Asp
       370                 375                 380
   Thr Leu Met Ile Ser Arg Thr Pro Glu Val Thr Cys Val Val Val Asp
   385                 390                 395                 400
   Val Ser His Glu Asp Pro Glu Val Lys Phe Asn Trp Tyr Val Asp Gly
                   405                 410                 415
   Val Glu Val His Asn Ala Lys Thr Lys Pro Arg Glu Glu Gln Tyr Ala
               420                 425                 430
   Ser Thr Tyr Arg Val Val Ser Val Leu Thr Val Leu His Gln Asp Trp
           435                 440                 445
   Leu Asn Gly Lys Glu Tyr Lys Cys Lys Val Ser Asn Lys Ala Leu Pro
       450                 455                 460
   Ala Pro Ile Glu Lys Thr Ile Ser Lys Ala Lys Gly Gln Pro Arg Glu
   465                 470                 475                 480
   Pro Gln Val Tyr Thr Leu Pro Pro Ser Arg Asp Glu Leu Thr Lys Asn
                   485                 490                 495
   Gln Val Ser Leu Thr Cys Leu Val Lys Gly Phe Tyr Pro Ser Asp Ile
               500                 505                 510
   Ala Val Glu Trp Glu Ser Asn Gly Gln Pro Glu Asn Asn Tyr Lys Thr
           515                 520                 525
   Thr Pro Pro Val Leu Asp Ser Asp Gly Ser Phe Phe Leu Tyr Ser Lys
       530                 535                 540
   Leu Thr Val Asp Lys Ser Arg Trp Gln Gln Gly Asn Val Phe Ser Cys
   545                 550                 555                 560
   Ser Val Met His Glu Ala Leu His Asn His Tyr Thr Gln Lys Ser Leu
                   565                 570                 575
   Ser Leu Ser Pro Gly Asn Ser Leu Phe Asn Gln Glu Val Gln Ile Pro
               580                 585                 590
   Leu Thr Glu Ser Tyr Ser Pro Asn Ser Glu Val Gln Leu Leu Glu Ser
           595                 600                 605
   Gly Gly Gly Leu Val Gln Pro Gly Gly Ser Leu Arg Leu Ser Cys Ala
       610                 615                 620
   Ala Ser Gly Phe Thr Phe Ser Phe Tyr Gln Met His Trp Val Arg Gln
   625                 630                 635                 640
   Ala Pro Gly Lys Gly Leu Glu Trp Val Ser Gly Ile Tyr Pro Ser Gly
                   645                 650                 655
   Gly Tyr Thr Lys Tyr Ala Asp Ser Val Lys Gly Arg Phe Thr Ile Ser
               660                 665                 670
   Arg Asp Asn Ser Lys Asn Thr Leu Tyr Leu Gln Met Asn Ser Leu Arg
           675                 680                 685
   Ala Glu Asp Thr Ala Val Tyr Tyr Cys Ala Arg Asp Leu Gly Tyr Gly
       690                 695                 700
   Ser Ser Trp Tyr Tyr Phe Asp Tyr Trp Gly Gln Gly Thr Leu Val Thr
   705                 710                 715                 720
   Val Ser Ser Gly Gly Gly Gly Ser Gly Gly Gly Gly Ser Gly Gly Gly
                   725                 730                 735
   Gly Ser Gln Ser Ala Leu Thr Gln Pro Ala Ser Val Ser Gly Ser Pro
               740                 745                 750
   Gly Gln Ser Ile Thr Ile Ser Cys Ser Gly Thr Asp Ser Asp Val Gly
           755                 760                 765
   Gly Tyr Asn His Val Ser Trp Tyr Gln Gln His Pro Gly Lys Ala Pro
       770                 775                 780
   Lys Leu Ile Ile Tyr Asp Val Asp His Arg Pro Ser Gly Ile Ser Asn
   785                 790                 795                 800
   Arg Phe Ser Gly Ser Lys Ser Gly Asn Thr Ala Ser Leu Thr Ile Ser
                   805                 810                 815
   Gly Leu Gln Ala Glu Asp Glu Ala Asp Tyr Tyr Cys Ser Ser Tyr Arg
               820                 825                 830
   Ser Gly Ser Thr Tyr Val Phe Gly Thr Gly Thr Lys Val Thr Val Leu
           835                 840                 845
<210> 19
   <211> 2427
   <212> DNA
   <213> Artificial Sequence
<220>
   <223> Nucleotide sequence of PDL2-CH1 Fc(N297A) H68 2E12 (X0249)
<400> 19
   ttattcacag tgacagtccc taaggaactg tacataatag agcatggcag caatgtgacc 60
   ctggaatgca actttgacac tggaagtcat gtgaaccttg gagcaataac agccagtttg 120
   caaaaggtgg aaaatgatac atccccacac cgtgaaagag ccactttgct ggaggagcag 180
   ctgcccttag ggaaggcctc gttccacata cctcaagtcc aagtgaggga cgaaggacag 240
   taccaatgca taatcatcta tggggtcgcc tgggactaca agtacctgac tctgaaagtc 300
   aaagcttcct acaggaaaat aaacactcac atcctaaagg ttccagaaac agatgaggta 360
   gagctcacct gccaggctac aggttatcct ctggcagaag tatcctggcc aaacgtcagc 420
   gttcctgcca acaccagcca ctccaggacc cctgaaggcc tctaccaggt caccagtgtt 480
   ctgcgcctaa agccaccccc tggcagaaac ttcagctgtg tgttctggaa tactcacgtg 540
   agggaactta ctttggccag cattgacctt caaagtcaga tggaacccag gacccatcca 600
   acctcgagcg cctccaccaa gggcccatcg gtcttccccc tggcaccctc ctccaagagc 660
   acctctgggg gcacagcggc cctgggctgc ctggtcaagg actacttccc cgagccggtg 720
   acggtgtcgt ggaactcagg cgccctgacc agcggcgtgc acaccttccc ggctgtccta 780
   cagtcctcag gactctactc cctcagcagc gtggtgaccg tgccctccag cagcttgggc 840
   acccagacct acatctgcaa cgtgaatcac aagcccagca acaccaaggt ggacaagaaa 900
   gttcgtacgg agcccaaatc ttctgacaaa actcacacat gcccaccgtg cccatccgga 960
   gcacctgaac tcctgggtgg accgtcagtc ttcctcttcc ccccaaaacc caaggacacc 1020
   ctcatgatct cccggacccc tgaggtcaca tgcgtggtgg tggacgtgag ccacgaagac 1080
   cctgaggtca agttcaactg gtacgtggac ggcgtggagg tgcataatgc caagacaaag 1140
   ccgcgggagg agcagtacgc cagcacgtac cgtgtggtca gcgtcctcac cgtcctgcac 1200
   caggactggc tgaatggcaa ggagtacaag tgcaaggtct ccaacaaagc cctcccagcc 1260
   cccatcgaga aaaccatctc caaagccaaa gggcagcccc gagaaccaca ggtgtacacc 1320
   ctgcccccat cccgggatga gctgaccaag aaccaggtca gcctgacctg cctggtcaaa 1380
   ggcttctatc caagcgacat cgccgtggag tgggagagca atgggcagcc ggagaacaac 1440
   tacaagacca cgcctcccgt gctggactcc gacggctcct tcttcctcta cagcaagctc 1500
   accgtggaca agagcaggtg gcagcagggg aacgtcttct catgctccgt gatgcatgag 1560
   gctctgcaca accactacac gcagaagagc ctctccctgt ctccgggtaa ctcattattc 1620
   aaccaagaag ttcaaattcc cttgaccgaa agttacagcc cgaattctca ggtgcagctg 1680
   aaggagtcag gacctggcag cgtggcgccc tcacagagcc tgtccatcac atgcaccgtc 1740
   tcagggttct cattaaccgg ctatggtgta aactgggttc gccagcctcc aggaaagggt 1800
   ctggagtggc tgggaatgat atggggtgat ggaagcacag actataattc agctctcaaa 1860
   tccagactat cgatcaccaa ggacaactcc aagagccaag ttttcttaaa aatgaacagt 1920
   ctgcaaactg atgacacagc cagatactac tgtgctcggg atggttatag taactttcat 1980
   tactatgtta tggactactg gggtcaagga acctcagtca ccgtctcctc tgggggtgga 2040
   ggctctggtg gcggtggatc cggcggaggt gggtcgggtg gcggcggatc tgacattgtg 2100
   ctcacccaat ctccagcttc tttggctgtg tctctaggtc agagagccac catctcctgc 2160
   agagccagtg aaagtgttga atattatgtc acaagtttaa tgcagtggta ccaacagaaa 2220
   ccaggacagc cacccaaact cctcatctct gctgctagca acgtagaatc tggggtccct 2280
   gccaggttta gtggcagtgg gtctgggaca gactttagcc tcaacatcca tcctgtggag 2340
   gaggatgata ttgcaatgta tttctgtcag caaagtagga aggttccatg gacgttcggt 2400
   ggaggcacca agctggaaat caagtga                                     2427
<210> 20
   <211> 808
   <212> PRT
   <213> Artificial Sequence
<220>
   <223> Amino acid sequence of PDL2-CH1 Fc(N297A) H68 2E12 (X0249)
<400> 20
<210> 21
   <211> 2331
   <212> DNA
   <213> Artificial Sequence
<220>
   <223> Nucleotide sequence of monoIL10 Ck(YAE) Fc(N297A) H68 3D1 (X0281)
<400> 21
   agcccaggcc agggcaccca gtctgagaac agctgcaccc acttcccagg caacctgcct 60
   aacatgcttc gagatctccg agatgccttc agcagagtga agactttctt tcaaatgaag 120
   gatcagctgg acaacttgtt gttaaaggag tccttgctgg aggactttaa gggttacctg 180
   ggttgccaag ccttgtctga gatgatccag ttttacctgg aggaggtgat gccccaagct 240
   gagaaccaag acccagacat caaggcgcat gtgaactccc tgggggagaa cctgaagacc 300
   ctcaggctga ggctacggcg ctgtcatcga tttcttccct gtgaaaacgg tggtggatcc 360
   ggcggtaaga gcaaggccgt ggagcaggtg aagaatgcct ttaataagct ccaagagaaa 420
   ggcatctaca aagccatgag tgagtttgac atcttcatca actacataga agcctacatt 480
   acaatgaaga tacgaaactc gagcactgtg gctgcaccat ctgtcttcat cttcccgcca 540
   tctgatgagc agttgaaatc tggaactgcc tctgttgtgt gcctgctgaa ttacttctat 600
   cccagagagg ccaaagtaca gtggaaggtg gataacgccc tccaatcggg taactcccag 660
   gagagtgcca cagagcagga cagcaaggac agcacctaca gcctcagcag cgagctgacg 720
   ctgagcaaag cagactacga gaaacacaaa gtctacgcct gcgaagtcac ccatcagggc 780
   ctgagctcgc ccgtcacaaa gagcttcaac aggggagagc gtacggagcc caaatcttct 840
   gacaaaactc acacatgccc accgtgccca tccggagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacgccagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaactca ttattcaacc aagaagttca aattcccttg 1560
   accgaaagtt acagcccgaa ttctcaggtc cagctgcagc agtctgggcc tgagctggtg 1620
   aggcctgggg aatcagtgaa gatttcctgc aagggttccg gctacacatt cactgattat 1680
   gctatacagt gggtgaagca gagtcatgca aagagtctag agtggattgg agttattaat 1740
   atttactatg ataatacaaa ctacaaccag aagtttaagg gcaaggccac aatgactgta 1800
   gacaaatcct ccagcacagc ctatatggaa cttgccagat tgacatctga ggattctgcc 1860
   atctattact gtgcaagagc ggcctggtat atggactact ggggtcaagg aacctcagtc 1920
   accgtctcct cagggggtgg aggctctggt ggcggtggct ctggcggagg tggatccggt 1980
   ggcggcggat ctgacattgt gctgtcacag tctccatcct ccctggctgt gtcagcagga 2040
   gagaaggtca ctatgagctg caaatccagt cagagtctgc tcaacagtag aacccgagag 2100
   aactacttgg cttggtacca gcagaaacca gggcagtctc ctaaactgct gatctactgg 2160
   gcatccacta gggaatctgg ggtccctgat cgcttcacag gcagtggatc tgggacagat 2220
   ttcactctca ccatcagcag tgtgcaggct gaagacctgg cagtttatta ctgcacgcaa 2280
   tcttataatc tttacacgtt cggagggggg accaagctgg aaataaaata a          2331
<210> 22
   <211> 776
   <212> PRT
   <213> Artificial Sequence
<220>
   <223> Amino acid sequence of monoIL10 Ck(YAE) Fc(N297A) H68 3D1 (X0281)
<400> 22
   Ser Pro Gly Gln Gly Thr Gln Ser Glu Asn Ser Cys Thr His Phe Pro
    1               5                  10                  15
   Gly Asn Leu Pro Asn Met Leu Arg Asp Leu Arg Asp Ala Phe Ser Arg
               20                  25                  30
   Val Lys Thr Phe Phe Gln Met Lys Asp Gln Leu Asp Asn Leu Leu Leu
           35                  40                  45
   Lys Glu Ser Leu Leu Glu Asp Phe Lys Gly Tyr Leu Gly Cys Gln Ala
       50                  55                  60
   Leu Ser Glu Met Ile Gln Phe Tyr Leu Glu Glu Val Met Pro Gln Ala
   65                  70                  75                  80
   Glu Asn Gln Asp Pro Asp Ile Lys Ala His Val Asn Ser Leu Gly Glu
                   85                  90                  95
   Asn Leu Lys Thr Leu Arg Leu Arg Leu Arg Arg Cys His Arg Phe Leu
               100                 105                 110
   Pro Cys Glu Asn Gly Gly Gly Ser Gly Gly Lys Ser Lys Ala Val Glu
           115                 120                 125
   Gln Val Lys Asn Ala Phe Asn Lys Leu Gln Glu Lys Gly Ile Tyr Lys
       130                 135                 140
   Ala Met Ser Glu Phe Asp Ile Phe Ile Asn Tyr Ile Glu Ala Tyr Ile
   145                 150                 155                 160
   Thr Met Lys Ile Arg Asn Ser Ser Thr Val Ala Ala Pro Ser Val Phe
                   165                 170                 175
   Ile Phe Pro Pro Ser Asp Glu Gln Leu Lys Ser Gly Thr Ala Ser Val
               180                 185                 190
   Val Cys Leu Leu Asn Tyr Phe Tyr Pro Arg Glu Ala Lys Val Gln Trp
           195                 200                 205
   Lys Val Asp Asn Ala Leu Gln Ser Gly Asn Ser Gln Glu Ser Ala Thr
       210                 215                 220
   Glu Gln Asp Ser Lys Asp Ser Thr Tyr Ser Leu Ser Ser Glu Leu Thr
   225                 230                 235                 240
   Leu Ser Lys Ala Asp Tyr Glu Lys His Lys Val Tyr Ala Cys Glu Val
                   245                 250                 255
   Thr His Gln Gly Leu Ser Ser Pro Val Thr Lys Ser Phe Asn Arg Gly
               260                 265                 270
   Glu Arg Thr Glu Pro Lys Ser Ser Asp Lys Thr His Thr Cys Pro Pro
           275                 280                 285
   Cys Pro Ser Gly Ala Pro Glu Leu Leu Gly Gly Pro Ser Val Phe Leu
       290                 295                 300
   Phe Pro Pro Lys Pro Lys Asp Thr Leu Met Ile Ser Arg Thr Pro Glu
   305                 310                 315                 320
   Val Thr Cys Val Val Val Asp Val Ser His Glu Asp Pro Glu Val Lys
                   325                 330                 335
   Phe Asn Trp Tyr Val Asp Gly Val Glu Val His Asn Ala Lys Thr Lys
               340                 345                 350
   Pro Arg Glu Glu Gln Tyr Ala Ser Thr Tyr Arg Val Val Ser Val Leu
           355                 360                 365
   Thr Val Leu His Gln Asp Trp Leu Asn Gly Lys Glu Tyr Lys Cys Lys
       370                 375                 380
   Val Ser Asn Lys Ala Leu Pro Ala Pro Ile Glu Lys Thr Ile Ser Lys
   385                 390                 395                 400
   Ala Lys Gly Gln Pro Arg Glu Pro Gln Val Tyr Thr Leu Pro Pro Ser
                   405                 410                 415
   Arg Asp Glu Leu Thr Lys Asn Gln Val Ser Leu Thr Cys Leu Val Lys
               420                 425                 430
   Gly Phe Tyr Pro Ser Asp Ile Ala Val Glu Trp Glu Ser Asn Gly Gln
           435                 440                 445
   Pro Glu Asn Asn Tyr Lys Thr Thr Pro Pro Val Leu Asp Ser Asp Gly
       450                 455                 460
   Ser Phe Phe Leu Tyr Ser Lys Leu Thr Val Asp Lys Ser Arg Trp Gln
   465                 470                 475                 480
   Gln Gly Asn Val Phe Ser Cys Ser Val Met His Glu Ala Leu His Asn
                   485                 490                 495
   His Tyr Thr Gln Lys Ser Leu Ser Leu Ser Pro Gly Asn Ser Leu Phe
               500                 505                 510
   Asn Gln Glu Val Gln Ile Pro Leu Thr Glu Ser Tyr Ser Pro Asn Ser
           515                 520                 525
   Gln Val Gln Leu Gln Gln Ser Gly Pro Glu Leu Val Arg Pro Gly Glu
       530                 535                 540
   Ser Val Lys Ile Ser Cys Lys Gly Ser Gly Tyr Thr Phe Thr Asp Tyr
   545                 550                 555                 560
   Ala Ile Gln Trp Val Lys Gln Ser His Ala Lys Ser Leu Glu Trp Ile
                   565                 570                 575
   Gly Val Ile Asn Ile Tyr Tyr Asp Asn Thr Asn Tyr Asn Gln Lys Phe
               580                 585                 590
   Lys Gly Lys Ala Thr Met Thr Val Asp Lys Ser Ser Ser Thr Ala Tyr
           595                 600                 605
   Met Glu Leu Ala Arg Leu Thr Ser Glu Asp Ser Ala Ile Tyr Tyr Cys
       610                 615                 620
   Ala Arg Ala Ala Trp Tyr Met Asp Tyr Trp Gly Gln Gly Thr Ser Val
   625                 630                 635                 640
   Thr Val Ser Ser Gly Gly Gly Gly Ser Gly Gly Gly Gly Ser Gly Gly
                   645                 650                 655
   Gly Gly Ser Gly Gly Gly Gly Ser Asp Ile Val Leu Ser Gln Ser Pro
               660                 665                 670
   Ser Ser Leu Ala Val Ser Ala Gly Glu Lys Val Thr Met Ser Cys Lys
           675                 680                 685
   Ser Ser Gln Ser Leu Leu Asn Ser Arg Thr Arg Glu Asn Tyr Leu Ala
       690                 695                 700
   Trp Tyr Gln Gln Lys Pro Gly Gln Ser Pro Lys Leu Leu Ile Tyr Trp
   705                 710                 715                 720
   Ala Ser Thr Arg Glu Ser Gly Val Pro Asp Arg Phe Thr Gly Ser Gly
                   725                 730                 735
   Ser Gly Thr Asp Phe Thr Leu Thr Ile Ser Ser Val Gln Ala Glu Asp
               740                 745                 750
   Leu Ala Val Tyr Tyr Cys Thr Gln Ser Tyr Asn Leu Tyr Thr Phe Gly
           755                 760                 765
   Gly Gly Thr Lys Leu Glu Ile Lys
       770                 775
<210> 23
   <211> 2330
   <212> DNA
   <213> Artificial Sequence
<220>
   <223> Nucleotide sequence of monoIl10CkwtFc (N297A) H68 3D1 (X0282)
<400> 23
   agcccaggcc agggcaccca gtctgagaac agctgcaccc acttcccagg caacctgcct 60
   aacatgcttc gagatctccg agatgccttc agcagagtga agactttctt tcaaatgaag 120
   gatcagctgg acaacttgtt gttaaaggag tccttgctgg aggactttaa gggttacctg 180
   ggttgccaag ccttgtctga gatgatccag ttttacctgg aggaggtgat gccccaagct 240
   gagaaccaag acccagacat caaggcgcat gtgaactccc tgggggagaa cctgaagacc 300
   ctcaggctga ggctacggcg ctgtcatcga tttcttccct gtgaaaacgg tggtggatcc 360
   ggcggtaaga gcaaggccgt ggagcaggtg aagaatgcct ttaataagct ccaagagaaa 420
   ggcatctaca aagccatgag tgagtttgac atcttcatca actacataga agcctacatt 480
   acaatgaaga tacgaaactc gagcactgtg gctgcaccat ctgtcttcat cttcccgcca 540
   tctgatgagc agttgaaatc tggaactgcc tctgttgtgt gcctgctgaa taacttctat 600
   cccagagagg ccaaagtaca gtggaaggtg gataacgccc tccaatcggg taactcccag 660
   gagagtgtca cagagcagga cagcaaggac agcacctaca gcctcagcag caccctgacg 720
   ctgagcaaag cagactacga gaaacacaaa gtctacgcct gcgaagtcac ccatcagggc 780
   ctgagctcgc ccgtcacaaa gagcttcaac aggggagagc gtacggagcc caaatcttct 840
   gacaaaactc acacatgccc accgtgccca tccggagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacgccagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaactca ttattcaacc aagaagttca aattcccttg 1560
   accgaaagtt acagcccgaa ttctcaggtc cagctgcagc agtctgggcc tgagctggtg 1620
   aggcctgggg aatcagtgaa gatttcctgc aagggttccg gctacacatt cactgattat 1680
   gctatacagt gggtgaagca gagtcatgca aagagtctag agtggattgg agttattaat 1740
   atttactatg ataatacaaa ctacaaccag aagtttaagg gcaaggccac aatgactgta 1800
   gacaaatcct ccagcacagc ctatatggaa cttgccagat tgacatctga ggattctgcc 1860
   atctattact gtgcaagagc ggcctggtat atggactact ggggtcaagg aacctcagtc 1920
   accgtctcct cagggggtgg aggctctggt ggcggtggct ctggcggagg tggatccggt 1980
   ggcggcggat ctgacattgt gctgtcacag tctccatcct ccctggctgt gtcagcagga 2040
   gagaaggtca ctatgagctg caaatccagt cagagtctgc tcaacagtag aacccgagag 2100
   aactacttgg cttggtacca gcagaaacca gggcagtctc ctaaactgct gatctactgg 2160
   gcatccacta gggaatctgg ggtccctgat cgcttcacag gcagtggatc tgggacagat 2220
   ttcactctca ccatcagcag tgtgcaggct gaagacctgg cagtttatta ctgcacgcaa 2280
   tcttataatc tttacacgtt cggagggggg accaagctgg aaataaaata           2330
<210> 24
   <211> 776
   <212> PRT
   <213> Artificial Sequence
<220>
   <223> Amino acid sequence of monoIl10CkwtFc (N297A) H68 3D1 (X0282)
<400> 24
<210> 25
   <211> 1806
   <212> DNA
   <213> Artificial Sequence
<220>
   <223> Nucleotide sequence for ORN116 comprising MET021 (anti-c-Met) scFv, human IgG1 SCC-P hinge, human IgG1 CH2, human IgG1 CH3 and human CH1
<400> 25
   atggaagcac cagcgcagct tctcttcctc ctgctactct ggctcccaga taccaccggt 60
   gacatccaga tgacccagtc tccatcctcc ctgtctgcat ctgtaggaga cagagtcacc 120
   atcacttgtc gggcaagtca gagcattagg aactatttaa attggtatca gcaggaacca 180
   gggaaagccc ctaagctcct gatctatgct gcatccagtt tgcaaagtgg ggtcccatca 240
   aggttcagtg gcagtggatc tgggacagac ttcattctca ccatcagcag tctgcagcct 300
   gaagattttg caacttacta ctgtcaacag agttacgtta ccccgctcac tttcggcgga 360
   gggaccaagg tggagatcaa agggggtgga ggctctggtg gcggtggctc tggcggaggt 420
   ggatccggtg gcggcggctc tgaagttcaa ttgttagagt ctggtggcgg tcttgttcag 480
   cctggtggtt ctttacgtct ttcttgcgct gcttccggat tcactttctc taagtacgat 540
   atgctttggg ttcgccaagc tcctggtaaa ggtttggagt gggtttctta tatctatcct 600
   tctggtggcc ttactgagta tgctgactcc gttaaaggtc gcttcactat ctctagagac 660
   aactctaaga atactctcta cttgcagatg aacagcttaa gggctgagga cacggccgtg 720
   tattactgtg cgagacgggc tccccggtcc ctttcttttg atatctgggg ccaagggaca 780
   atggtcaccg tctcctcgag cgagcccaaa tcttctgaca aaactcacac atgcccaccg 840
   tgcccatccg gagcacctga actcctgggt ggaccgtcag tcttcctctt ccccccaaaa 900
   cccaaggaca ccctcatgat ctcccggacc cctgaggtca catgcgtggt ggtggacgtg 960
   agccacgaag accctgaggt caagttcaac tggtacgtgg acggcgtgga ggtgcataat 1020
   gccaagacaa agccgcggga ggagcagtac aacagcacgt accgtgtggt cagcgtcctc 1080
   accgtcctgc accaggactg gctgaatggc aaggagtaca agtgcaaggt ctccaacaaa 1140
   gccctcccag cccccatcga gaaaaccatc tccaaagcca aagggcagcc ccgagaacca 1200
   caggtgtaca ccctgccccc atcccgggat gagctgacca agaaccaggt cagcctgacc 1260
   tgcctggtca aaggcttcta tccaagcgac atcgccgtgg agtgggagag caatgggcag 1320
   ccggagaaca actacaagac cacgcctccc gtgctggact ccgacggctc cttcttcctc 1380
   tacagcaagc tcaccgtgga caagagcagg tggcagcagg ggaacgtctt ctcatgctcc 1440
   gtgatgcatg aggctctgca caaccactac acgcagaaga gcctctccct gtctccgggt 1500
   aaatctagag cctccaccaa gggcccatcg gtcttccccc tggcaccctc ctccaagagc 1560
   acctctgggg gcacagcggc cctgggctgc ctggtcaagg actacttccc cgagccggtg 1620
   acggtgtcgt ggaactcagg cgccctgacc agcggcgtgc acaccttccc ggctgtccta 1680
   cagtcctcag gactctactc cctcagcagc gtggtgaccg tgccctccag cagcttgggc 1740
   acccagacct acatctgcaa cgtgaatcac aagcccagca acaccaaggt ggacaagaaa 1800
   gtttga                                                            1806
<210> 26
   <211> 1797
   <212> DNA
   <213> Artificial Sequence
<220>
   <223> Nucleotide sequence for ORN145 comprising : 4C04 (anti-RON) scFv, human IgG1 SCC-P hinge, human IgG1 CH2, human IgG1 CH3 and human CH1
<400> 26
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gcgagcccaa atcttctgac aaaactcaca catgcccacc gtgcccatcc 840
   ggagcacctg aactcctggg tggaccgtca gtcttcctct tccccccaaa acccaaggac 900
   accctcatga tctcccggac ccctgaggtc acatgcgtgg tggtggacgt gagccacgaa 960
   gaccctgagg tcaagttcaa ctggtacgtg gacggcgtgg aggtgcataa tgccaagaca 1020
   aagccgcggg aggagcagta caacagcacg taccgtgtgg tcagcgtcct caccgtcctg 1080
   caccaggact ggctgaatgg caaggagtac aagtgcaagg tctccaacaa agccctccca 1140
   gcccccatcg agaaaaccat ctccaaagcc aaagggcagc cccgagaacc acaggtgtac 1200
   accctgcccc catcccggga tgagctgacc aagaaccagg tcagcctgac ctgcctggtc 1260
   aaaggcttct atccaagcga catcgccgtg gagtgggaga gcaatgggca gccggagaac 1320
   aactacaaga ccacgcctcc cgtgctggac tccgacggct ccttcttcct ctacagcaag 1380
   ctcaccgtgg acaagagcag gtggcagcag gggaacgtct tctcatgctc cgtgatgcat 1440
   gaggctctgc acaaccacta cacgcagaag agcctctccc tgtctccggg taaatctaga 1500
   gcctccacca agggcccatc ggtcttcccc ctggcaccct cctccaagag cacctctggg 1560
   ggcacagcgg ccctgggctg cctggtcaag gactacttcc ccgagccggt gacggtgtcg 1620
   tggaactcag gcgccctgac cagcggcgtg cacaccttcc cggctgtcct acagtcctca 1680
   ggactctact ccctcagcag cgtggtgacc gtgccctcca gcagcttggg cacccagacc 1740
   tacatctgca acgtgaatca caagcccagc aacaccaagg tggacaagaa agtttga    1797
<210> 27
   <211> 1818
   <212> DNA
   <213> Artificial Sequence
<220>
   <223> Nucleotide sequence for ORN146 comprising : 4C04 (anti-RON) scFv, human IgG1 SCC-P hinge, human CH2, human CH3, and human Ck (YAE)
<400> 27
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gcgagcccaa atcttctgac aaaactcaca catgcccacc gtgcccatcc 840
   ggagcacctg aactcctggg tggaccgtca gtcttcctct tccccccaaa acccaaggac 900
   accctcatga tctcccggac ccctgaggtc acatgcgtgg tggtggacgt gagccacgaa 960
   gaccctgagg tcaagttcaa ctggtacgtg gacggcgtgg aggtgcataa tgccaagaca 1020
   aagccgcggg aggagcagta caacagcacg taccgtgtgg tcagcgtcct caccgtcctg 1080
   caccaggact ggctgaatgg caaggagtac aagtgcaagg tctccaacaa agccctccca 1140
   gcccccatcg agaaaaccat ctccaaagcc aaagggcagc cccgagaacc acaggtgtac 1200
   accctgcccc catcccggga tgagctgacc aagaaccagg tcagcctgac ctgcctggtc 1260
   aaaggcttct atccaagcga catcgccgtg gagtgggaga gcaatgggca gccggagaac 1320
   aactacaaga ccacgcctcc cgtgctggac tccgacggct ccttcttcct ctacagcaag 1380
   ctcaccgtgg acaagagcag gtggcagcag gggaacgtct tctcatgctc cgtgatgcat 1440
   gaggctctgc acaaccacta cacgcagaag agcctctccc tgtctccggg taaatctaga 1500
   actgtggctg caccatctgt cttcatcttc ccgccatctg atgagcagtt gaaatctgga 1560
   actgcctctg ttgtgtgcct gctgaattac ttctatccca gagaggccaa agtacagtgg 1620
   aaggtggata acgccctcca atcgggtaac tcccaggaga gtgccacaga gcaggacagc 1680
   aaggacagca cctacagcct cagcagcgag ctgacgctga gcaaagcaga ctacgagaaa 1740
   cacaaagtct acgcctgcga agtcacccat cagggcctga gctcgcccgt cacaaagagc 1800
   ttcaacaggg gagagtga                                               1818
<210> 28
   <211> 1806
   <212> DNA
   <213> Artificial Sequence
<220>
   <223> Nucleotide sequence for ORN148 comprising : 11H09 (anti-RON) scFv, human IgG1 SCC-P hinge, human CH2, human CH3, and human Ck(YAE)
<400> 28
   atggaagcac cagcgcagct tctcttcctc ctgctactct ggctcccaga taccaccggt 60
   gacatccaga tgacccagtc tccatcctcc ttatctgcct ctctgggaga aagagtcagt 120
   ctcacttgtc gggcaagtca ggacattggt agtagcttaa gctggcttca gcaggaacca 180
   gatggtacta ttaaacgcct gatctactcc acatccagtt tagattctgg tgtccccaaa 240
   aggttcagtg gcagtaggtc ggggtcagat tattctctca ccatcagcag ccttgagtct 300
   gaagattttg tagactatta ctgtctacaa tatgctagtt ctccgtggac gttcggtgga 360
   ggcaccaagc tggaaatcaa aggtggcggt ggctctggcg gaggtggatc cggtggcggc 420
   ggctctcagg tccagcttca gcagtctggg gctgaactgg caaaacctgg ggcctcagtg 480
   aagatgtcct gcaagtcttc tggctacacc tttactagct actggataca ctgggtaaaa 540
   gagaggcctg gacagggtct ggaatggatt ggatacattt atcctaccac tggttatact 600
   gagtccaatc agaagttcaa ggacaaggcc acattgactg cagacaaatc ctccagcaca 660
   gcctacatgc aactgagcag cctgacatct gaggactctg cagtctatta ctgtttccta 720
   ctacggccct ggtacttcga tgtctggggc gcagggacca cggtcaccgt ctcctcgagc 780
   gagcccaaat cttctgacaa aactcacaca tgcccaccgt gcccatccgg agcacctgaa 840
   ctcctgggtg gaccgtcagt cttcctcttc cccccaaaac ccaaggacac cctcatgatc 900
   tcccggaccc ctgaggtcac atgcgtggtg gtggacgtga gccacgaaga ccctgaggtc 960
   aagttcaact ggtacgtgga cggcgtggag gtgcataatg ccaagacaaa gccgcgggag 1020
   gagcagtaca acagcacgta ccgtgtggtc agcgtcctca ccgtcctgca ccaggactgg 1080
   ctgaatggca aggagtacaa gtgcaaggtc tccaacaaag ccctcccagc ccccatcgag 1140
   aaaaccatct ccaaagccaa agggcagccc cgagaaccac aggtgtacac cctgccccca 1200
   tcccgggatg agctgaccaa gaaccaggtc agcctgacct gcctggtcaa aggcttctat 1260
   cccagcgaca tcgccgtgga gtgggagagc aatgggcagc cggagaacaa ctacaagacc 1320
   acgcctcccg tgctggactc cgacggctcc ttcttcctct acagcaagct caccgtggac 1380
   aagagcaggt ggcagcaggg gaacgtcttc tcatgctccg tgatgcatga ggctctgcac 1440
   aaccactaca cgcagaagag cctctccctg tctccgggta aatctagaac tgtggctgca 1500
   ccatctgtct tcatcttccc gccatctgat gagcagttga aatctggaac tgcctctgtt 1560
   gtgtgcctgc tgaattactt ctatcccaga gaggccaaag tacagtggaa ggtggataac 1620
   gccctccaat cgggtaactc ccaggagagt gccacagagc aggacagcaa ggacagcacc 1680
   tacagcctca gcagcgagct gacgctgagc aaagcagact acgagaaaca caaagtctac 1740
   gcctgcgaag tcacccatca gggcctgagc tcgcccgtca caaagagctt caacagggga 1800
   gagtga                                                            1806
<210> 29
   <211> 581
   <212> PRT
   <213> Artificial Sequence
<220>
   <223> ORN116 mature protein comprising MET021 (anti-c-Met) scFv, human IgG1 SCC-P hinge, human IgG1 CH2, human IgG1 CH3 and human CH1
<400> 29
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ser Ile Arg Asn Tyr
               20                  25                  30
   Leu Asn Trp Tyr Gln Gln Glu Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Ile Leu Thr Ile Ser Ser Leu Gln Pro
   65                  70                  75                  80
   Glu Asp Phe Ala Thr Tyr Tyr Cys Gln Gln Ser Tyr Val Thr Pro Leu
                   85                  90                  95
   Thr Phe Gly Gly Gly Thr Lys Val Glu Ile Lys Gly Gly Gly Gly Ser
               100                 105                 110
   Gly Gly Gly Gly Ser Gly Gly Gly Gly Ser Gly Gly Gly Gly Ser Glu
           115                 120                 125
   Val Gln Leu Leu Glu Ser Gly Gly Gly Leu Val Gln Pro Gly Gly Ser
       130                 135                 140
   Leu Arg Leu Ser Cys Ala Ala Ser Gly Phe Thr Phe Ser Lys Tyr Asp
   145                 150                 155                 160
   Met Leu Trp Val Arg Gln Ala Pro Gly Lys Gly Leu Glu Trp Val Ser
                   165                 170                 175
   Tyr Ile Tyr Pro Ser Gly Gly Leu Thr Glu Tyr Ala Asp Ser Val Lys
   180 185 190
   Gly Arg Phe Thr Ile Ser Arg Asp Asn Ser Lys Asn Thr Leu Tyr Leu
   195 200 205
   Gln Met Asn Ser Leu Arg Ala Glu Asp Thr Ala Val Tyr Tyr Cys Ala
   210 215 220
   Arg Arg Ala Pro Arg Ser Leu Ser Phe Asp Ile Trp Gly Gln Gly Thr
   225 230 235 240
   Met Val Thr Val Ser Ser Ser Glu Pro Lys Ser Ser Asp Lys Thr His
   245 250 255
   Thr Cys Pro Pro Cys Pro Ser Gly Ala Pro Glu Leu Leu Gly Gly Pro
   260 265 270
   Ser Val Phe Leu Phe Pro Pro Lys Pro Lys Asp Thr Leu Met Ile Ser
   275 280 285
   Arg Thr Pro Glu Val Thr Cys Val Val Val Asp Val Ser His Glu Asp
   290 295 300
   Pro Glu Val Lys Phe Asn Trp Tyr Val Asp Gly Val Glu Val His Asn
   305 310 315 320
   Ala Lys Thr Lys Pro Arg Glu Glu Gln Tyr Asn Ser Thr Tyr Arg Val
   325 330 335
   Val Ser Val Leu Thr Val Leu His Gln Asp Trp Leu Asn Gly Lys Glu
   340 345 350
   Tyr Lys Cys Lys Val Ser Asn Lys Ala Leu Pro Ala Pro Ile Glu Lys
   355 360 365
   Thr Ile Ser Lys Ala Lys Gly Gln Pro Arg Glu Pro Gln Val Tyr Thr
   370 375 380
   Leu Pro Pro Ser Arg Asp Glu Leu Thr Lys Asn Gln Val Ser Leu Thr
   385 390 395 400
   Cys Leu Val Lys Gly Phe Tyr Pro Ser Asp Ile Ala Val Glu Trp Glu
   405 410 415
   Ser Asn Gly Gln Pro Glu Asn Asn Tyr Lys Thr Thr Pro Pro Val Leu
   420 425 430
   Asp Ser Asp Gly Ser Phe Phe Leu Tyr Ser Lys Leu Thr Val Asp Lys
   435 440 445
   Ser Arg Trp Gln Gln Gly Asn Val Phe Ser Cys Ser Val Met His Glu
   450 455 460
   Ala Leu His Asn His Tyr Thr Gln Lys Ser Leu Ser Leu Ser Pro Gly
   465 470 475 480
   Lys Ser Arg Ala Ser Thr Lys Gly Pro Ser Val Phe Pro Leu Ala Pro
   485 490 495
   Ser Ser Lys Ser Thr Ser Gly Gly Thr Ala Ala Leu Gly Cys Leu Val
   500 505 510
   Lys Asp Tyr Phe Pro Glu Pro Val Thr Val Ser Trp Asn Ser Gly Ala
   515 520 525
   Leu Thr Ser Gly Val His Thr Phe Pro Ala Val Leu Gln Ser Ser Gly
   530 535 540
   Leu Tyr Ser Leu Ser Ser Val Val Thr Val Pro Ser Ser Ser Leu Gly
   545 550 555 560
   Thr Gln Thr Tyr Ile Cys Asn Val Asn His Lys Pro Ser Asn Thr Lys
   565 570 575
   Val Asp Lys Lys Val
   580
<210> 30
   <211> 578
   <212> PRT
   <213> Artificial Sequence
<220>
   <223> ORN145 mature protein comprising : 4C04 (anti-RON) scFv, human IgG1 SCC-P hinge, human IgG1 CH2, human IgG1 CH3 and human CH1
<400> 30
   Asp Ile Gln Met Thr Gln Ser Pro Ala Ser Gln Ser Ala Ser Leu Gly
   1               5                   10                  15
   Glu Ser Val Thr Ile Thr Cys Leu Ala Ser Gln Thr Ile Gly Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Ser Leu Ala Asp Gly Val Pro Ser Arg Phe Ser Ala
       50                  55                  60
   Ser Gly Ser Gly Thr Lys Phe Ser Phe Lys Ile Asp Ser Leu Gln Ser
   65                  70                  75                  80
   Glu Asp Phe Val Ser Tyr Tyr Cys Gln Gln Leu Tyr Asn Thr Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Ile Gln Leu Gln Gln
           115                 120                 125
   Ser Gly Pro Glu Leu Val Lys Pro Gly Thr Ser Val Lys Ile Ser Cys
       130                 135                 140
   Lys Ala Ser Asp Tyr Thr Leu Thr Asp Phe Tyr Met Asn Trp Val Arg
   145                 150                 155                 160
   Gln Lys Pro Gly Gln Gly Leu Glu Trp Ile Gly Arg Ile Tyr Pro Gly
                   165                 170                 175
   Thr Asp Lys Thr Arg Tyr Asn Glu Lys Phe Arg Asp Lys Ala Ile Leu
               180                 185                 190
   Thr Val Asp Thr Ser Ser Ser Thr Ala Tyr Met Gln Leu Ser Ser Leu
           195                 200                 205
   Thr Ser Glu Asp Thr Ala Val Tyr Phe Cys Ala Arg Ser Ala Tyr Tyr
       210                 215                 220
   Gly Asn Tyr Val Ala Met Asp Tyr Trp Gly Gln Gly Thr Ser Val Thr
   225                 230                 235                 240
   Val Ser Ser Ser Glu Pro Lys Ser Ser Asp Lys Thr His Thr Cys Pro
                   245                 250                 255
   Pro Cys Pro Ser Gly Ala Pro Glu Leu Leu Gly Gly Pro Ser Val Phe
               260                 265                 270
   Leu Phe Pro Pro Lys Pro Lys Asp Thr Leu Met Ile Ser Arg Thr Pro
           275                 280                 285
   Glu Val Thr Cys Val Val Val Asp Val Ser His Glu Asp Pro Glu Val
       290                 295                 300
   Lys Phe Asn Trp Tyr Val Asp Gly Val Glu Val His Asn Ala Lys Thr
   305                 310                 315                 320
   Lys Pro Arg Glu Glu Gln Tyr Asn Ser Thr Tyr Arg Val Val Ser Val
                   325                 330                 335
   Leu Thr Val Leu His Gln Asp Trp Leu Asn Gly Lys Glu Tyr Lys Cys
               340                 345                 350
   Lys Val Ser Asn Lys Ala Leu Pro Ala Pro Ile Glu Lys Thr Ile Ser
           355                 360                 365
   Lys Ala Lys Gly Gln Pro Arg Glu Pro Gln Val Tyr Thr Leu Pro Pro
       370                 375                 380
   Ser Arg Asp Glu Leu Thr Lys Asn Gln Val Ser Leu Thr Cys Leu Val
   385                 390                 395                 400
   Lys Gly Phe Tyr Pro Ser Asp Ile Ala Val Glu Trp Glu Ser Asn Gly
                   405                 410                 415
   Gln Pro Glu Asn Asn Tyr Lys Thr Thr Pro Pro Val Leu Asp Ser Asp
               420                 425                 430
   Gly Ser Phe Phe Leu Tyr Ser Lys Leu Thr Val Asp Lys Ser Arg Trp
           435                 440                 445
   Gln Gln Gly Asn Val Phe Ser Cys Ser Val Met His Glu Ala Leu His
       450                 455                 460
   Asn His Tyr Thr Gln Lys Ser Leu Ser Leu Ser Pro Gly Lys Ser Arg
   465                 470                 475                 480
   Ala Ser Thr Lys Gly Pro Ser Val Phe Pro Leu Ala Pro Ser Ser Lys
                   485                 490                 495
   Ser Thr Ser Gly Gly Thr Ala Ala Leu Gly Cys Leu Val Lys Asp Tyr
               500                 505                 510
   Phe Pro Glu Pro Val Thr Val Ser Trp Asn Ser Gly Ala Leu Thr Ser
           515                 520                 525
   Gly Val His Thr Phe Pro Ala Val Leu Gln Ser Ser Gly Leu Tyr Ser
       530                 535                 540
   Leu Ser Ser Val Val Thr Val Pro Ser Ser Ser Leu Gly Thr Gln Thr
   545                 550                 555                 560
   Tyr Ile Cys Asn Val Asn His Lys Pro Ser Asn Thr Lys Val Asp Lys
                   565                 570                 575
   Lys Val
<210> 31
   <211> 585
   <212> PRT
   <213> Artificial Sequence
<220>
   <223> ORN146 mature protein comprising : 4C04 (anti-RON) scFv, human IgG1 SCC-P hinge, human CH2, human CH3, and human Ck(YAE)
<400> 31
   Asp Ile Gln Met Thr Gln Ser Pro Ala Ser Gln Ser Ala Ser Leu Gly
    1               5                  10                  15
   Glu Ser Val Thr Ile Thr Cys Leu Ala Ser Gln Thr Ile Gly Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Ser Leu Ala Asp Gly Val Pro Ser Arg Phe Ser Ala
       50                  55                  60
   Ser Gly Ser Gly Thr Lys Phe Ser Phe Lys Ile Asp Ser Leu Gln Ser
   65                  70                  75                  80
   Glu Asp Phe Val Ser Tyr Tyr Cys Gln Gln Leu Tyr Asn Thr Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Ile Gln Leu Gln Gln
           115                 120                 125
   Ser Gly Pro Glu Leu Val Lys Pro Gly Thr Ser Val Lys Ile Ser Cys
       130                 135                 140
   Lys Ala Ser Asp Tyr Thr Leu Thr Asp Phe Tyr Met Asn Trp Val Arg
   145                 150                 155                 160
   Gln Lys Pro Gly Gln Gly Leu Glu Trp Ile Gly Arg Ile Tyr Pro Gly
                   165                 170                 175
   Thr Asp Lys Thr Arg Tyr Asn Glu Lys Phe Arg Asp Lys Ala Ile Leu
               180                 185                 190
   Thr Val Asp Thr Ser Ser Ser Thr Ala Tyr Met Gln Leu Ser Ser Leu
           195                 200                 205
   Thr Ser Glu Asp Thr Ala Val Tyr Phe Cys Ala Arg Ser Ala Tyr Tyr
       210                 215                 220
   Gly Asn Tyr Val Ala Met Asp Tyr Trp Gly Gln Gly Thr Ser Val Thr
   225                 230                 235                 240
   Val Ser Ser Ser Glu Pro Lys Ser Ser Asp Lys Thr His Thr Cys Pro
                   245                 250                 255
   Pro Cys Pro Ser Gly Ala Pro Glu Leu Leu Gly Gly Pro Ser Val Phe
               260                 265                 270
   Leu Phe Pro Pro Lys Pro Lys Asp Thr Leu Met Ile Ser Arg Thr Pro
           275                 280                 285
   Glu Val Thr Cys Val Val Val Asp Val Ser His Glu Asp Pro Glu Val
       290                 295                 300
   Lys Phe Asn Trp Tyr Val Asp Gly Val Glu Val His Asn Ala Lys Thr
   305                 310                 315                 320
   Lys Pro Arg Glu Glu Gln Tyr Asn Ser Thr Tyr Arg Val Val Ser Val
                   325                 330                 335
   Leu Thr Val Leu His Gln Asp Trp Leu Asn Gly Lys Glu Tyr Lys Cys
               340                 345                 350
   Lys Val Ser Asn Lys Ala Leu Pro Ala Pro Ile Glu Lys Thr Ile Ser
           355                 360                 365
   Lys Ala Lys Gly Gln Pro Arg Glu Pro Gln Val Tyr Thr Leu Pro Pro
       370                 375                 380
   Ser Arg Asp Glu Leu Thr Lys Asn Gln Val Ser Leu Thr Cys Leu Val
   385                 390                 395                 400
   Lys Gly Phe Tyr Pro Ser Asp Ile Ala Val Glu Trp Glu Ser Asn Gly
                   405                 410                 415
   Gln Pro Glu Asn Asn Tyr Lys Thr Thr Pro Pro Val Leu Asp Ser Asp
               420                 425                 430
   Gly Ser Phe Phe Leu Tyr Ser Lys Leu Thr Val Asp Lys Ser Arg Trp
           435                 440                 445
   Gln Gln Gly Asn Val Phe Ser Cys Ser Val Met His Glu Ala Leu His
       450                 455                 460
   Asn His Tyr Thr Gln Lys Ser Leu Ser Leu Ser Pro Gly Lys Ser Arg
   465                 470                 475                 480
   Thr Val Ala Ala Pro Ser Val Phe Ile Phe Pro Pro Ser Asp Glu Gln
                   485                 490                 495
   Leu Lys Ser Gly Thr Ala Ser Val Val Cys Leu Leu Asn Tyr Phe Tyr
               500                 505                 510
   Pro Arg Glu Ala Lys Val Gln Trp Lys Val Asp Asn Ala Leu Gln Ser
           515                 520                 525
   Gly Asn Ser Gln Glu Ser Ala Thr Glu Gln Asp Ser Lys Asp Ser Thr
       530                 535                 540
   Tyr Ser Leu Ser Ser Glu Leu Thr Leu Ser Lys Ala Asp Tyr Glu Lys
   545                 550                 555                 560
   His Lys Val Tyr Ala Cys Glu Val Thr His Gln Gly Leu Ser Ser Pro
                   565                 570                 575
   Val Thr Lys Ser Phe Asn Arg Gly Glu
               580                 585
<210> 32
   <211> 581
   <212> PRT
   <213> Artificial Sequence
<220>
   <223> ORN148 mature protein comprising : 11H09 (anti-RON) scFv, human IgG1 SCC-P hinge, human CH2, human CH3, and human Ck(YAE)
<400> 32
   Asp Ile Gln Met Thr Gln Ser Pro Ser Ser Leu Ser Ala Ser Leu Gly
    1               5                  10                  15
   Glu Arg Val Ser Leu Thr Cys Arg Ala Ser Gln Asp Ile Gly Ser Ser
               20                  25                  30
   Leu Ser Trp Leu Gln Gln Glu Pro Asp Gly Thr Ile Lys Arg Leu Ile
           35                  40                  45
   Tyr Ser Thr Ser Ser Leu Asp Ser Gly Val Pro Lys Arg Phe Ser Gly
       50                  55                  60
   Ser Arg Ser Gly Ser Asp Tyr Ser Leu Thr Ile Ser Ser Leu Glu Ser
   65                  70                  75                  80
   Glu Asp Phe Val Asp Tyr Tyr Cys Leu Gln Tyr Ala Ser Ser Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Gln
           115                 120                 125
   Ser Gly Ala Glu Leu Ala Lys Pro Gly Ala Ser Val Lys Met Ser Cys
       130                 135                 140
   Lys Ser Ser Gly Tyr Thr Phe Thr Ser Tyr Trp Ile His Trp Val Lys
   145                 150                 155                 160
   Glu Arg Pro Gly Gln Gly Leu Glu Trp Ile Gly Tyr Ile Tyr Pro Thr
                   165                 170                 175
   Thr Gly Tyr Thr Glu Ser Asn Gln Lys Phe Lys Asp Lys Ala Thr Leu
               180                 185                 190
   Thr Ala Asp Lys Ser Ser Ser Thr Ala Tyr Met Gln Leu Ser Ser Leu
           195                 200                 205
   Thr Ser Glu Asp Ser Ala Val Tyr Tyr Cys Phe Leu Leu Arg Pro Trp
       210                 215                 220
   Tyr Phe Asp Val Trp Gly Ala Gly Thr Thr Val Thr Val Ser Ser Ser
   225                 230                 235                 240
   Glu Pro Lys Ser Ser Asp Lys Thr His Thr Cys Pro Pro Cys Pro Ser
                   245                 250                 255
   Gly Ala Pro Glu Leu Leu Gly Gly Pro Ser Val Phe Leu Phe Pro Pro
               260                 265                 270
   Lys Pro Lys Asp Thr Leu Met Ile Ser Arg Thr Pro Glu Val Thr Cys
           275                 280                 285
   Val Val Val Asp Val Ser His Glu Asp Pro Glu Val Lys Phe Asn Trp
       290                 295                 300
   Tyr Val Asp Gly Val Glu Val His Asn Ala Lys Thr Lys Pro Arg Glu
   305                 310                 315                 320
   Glu Gln Tyr Asn Ser Thr Tyr Arg Val Val Ser Val Leu Thr Val Leu
                   325                 330                 335
   His Gln Asp Trp Leu Asn Gly Lys Glu Tyr Lys Cys Lys Val Ser Asn
               340                 345                 350
   Lys Ala Leu Pro Ala Pro Ile Glu Lys Thr Ile Ser Lys Ala Lys Gly
           355                 360                 365
   Gln Pro Arg Glu Pro Gln Val Tyr Thr Leu Pro Pro Ser Arg Asp Glu
       370                 375                 380
   Leu Thr Lys Asn Gln Val Ser Leu Thr Cys Leu Val Lys Gly Phe Tyr
   385                 390                 395                 400
   Pro Ser Asp Ile Ala Val Glu Trp Glu Ser Asn Gly Gln Pro Glu Asn
                   405                 410                 415
   Asn Tyr Lys Thr Thr Pro Pro Val Leu Asp Ser Asp Gly Ser Phe Phe
               420                 425                 430
   Leu Tyr Ser Lys Leu Thr Val Asp Lys Ser Arg Trp Gln Gln Gly Asn
           435                 440                 445
   Val Phe Ser Cys Ser Val Met His Glu Ala Leu His Asn His Tyr Thr
       450                 455                 460
   Gln Lys Ser Leu Ser Leu Ser Pro Gly Lys Ser Arg Thr Val Ala Ala
   465                 470                 475                 480
   Pro Ser Val Phe Ile Phe Pro Pro Ser Asp Glu Gln Leu Lys Ser Gly
                   485                 490                 495
   Thr Ala Ser Val Val Cys Leu Leu Asn Tyr Phe Tyr Pro Arg Glu Ala
               500                 505                 510
   Lys Val Gln Trp Lys Val Asp Asn Ala Leu Gln Ser Gly Asn Ser Gln
           515                 520                 525
   Glu Ser Ala Thr Glu Gln Asp Ser Lys Asp Ser Thr Tyr Ser Leu Ser
       530                 535                 540
   Ser Glu Leu Thr Leu Ser Lys Ala Asp Tyr Glu Lys His Lys Val Tyr
   545                 550                 555                 560
   Ala Cys Glu Val Thr His Gln Gly Leu Ser Ser Pro Val Thr Lys Ser
                   565                 570                 575
   Phe Asn Arg Gly Glu
               580
<210> 33
   <211> 1809
   <212> DNA
   <213> Artificial Sequence
<220>
   <223> Nucleotide sequence for ORN160 comprising : 5D5 (anti-c-Met) scFv, human IgG1 SCC-P hinge, human IgG1 CH2, human IgG1 CH3, and human CH1
<400> 33
   atggaagcac cagcgcagct tctcttcctc ctgctactct ggctcccaga taccaccggt 60
   gactttatga tgtcacagtc tccatcctcc ctaactgtgt cagttggaga gaaggttact 120
   gtgagctgca agtccagtca gtccctttta tatactagca gtcagaagaa ctacttggcc 180
   tggtaccagc agaaaccagg tcagtctcct aaactgctga tttactgggc atccactagg 240
   gaatctgggg tccctgatcg cttcacaggc agtggatctg ggacagattt cactctcacc 300
   atcaccagtg tgaaggctga cgacctggca gtttattact gtcagcaata ttatgcctat 360
   ccgtggacgt tcggtggagg caccaagttg gagctcaaag gtggcggtgg ctctggcgga 420
   ggtggatccg gtggcggcgg ctctcaggtc caactgcagc agtctgggcc tgagctggtg 480
   aggcctgggg cttcagtgaa gatgtcctgc agggcttcgg gctatacctt caccagctac 540
   tggttgcact gggttaaaca gaggcctgga caaggccttg agtggattgg catgattgat 600
   ccttccaata gtgacactag gtttaatccg aacttcaagg acaaggccac attgaatgta 660
   gacagatctt ccaacacagc ctacatgctg ctcagcagcc tgacatctgc tgactctgca 720
   gtctattact gtgccacata tggtagctac gtttcccctc tggactactg gggtcaagga 780
   acctcagtca ccgtctcctc gagcgagccc aaatcttctg acaaaactca cacatgccca 840
   ccgtgcccat ccggagcacc tgaactcctg ggtggaccgt cagtcttcct cttcccccca 900
   aaacccaagg acaccctcat gatctcccgg acccctgagg tcacatgcgt ggtggtggac 960
   gtgagccacg aagaccctga ggtcaagttc aactggtacg tggacggcgt ggaggtgcat 1020
   aatgccaaga caaagccgcg ggaggagcag tacaacagca cgtaccgtgt ggtcagcgtc 1080
   ctcaccgtcc tgcaccagga ctggctgaat ggcaaggagt acaagtgcaa ggtctccaac 1140
   aaagccctcc cagcccccat cgagaaaacc atctccaaag ccaaagggca gccccgagaa 1200
   ccacaggtgt acaccctgcc cccatcccgg gatgagctga ccaagaacca ggtcagcctg 1260
   acctgcctgg tcaaaggctt ctatccaagc gacatcgccg tggagtggga gagcaatggg 1320
   cagccggaga acaactacaa gaccacgcct cccgtgctgg actccgacgg ctccttcttc 1380
   ctctacagca agctcaccgt ggacaagagc aggtggcagc aggggaacgt cttctcatgc 1440
   tccgtgatgc atgaggctct gcacaaccac tacacgcaga agagcctctc cctgtctccg 1500
   ggtaaatcta gagcctccac caagggccca tcggtcttcc ccctggcacc ctcctccaag 1560
   agcacctctg ggggcacagc ggccctgggc tgcctggtca aggactactt ccccgagccg 1620
   gtgacggtgt cgtggaactc aggcgccctg accagcggcg tgcacacctt cccggctgtc 1680
   ctacagtcct caggactcta ctccctcagc agcgtggtga ccgtgccctc cagcagcttg 1740
   ggcacccaga cctacatctg caacgtgaat cacaagccca gcaacaccaa ggtggacaag 1800
   aaagtttga                                                         1809
<210> 34
   <211> 1821
   <212> DNA
   <213> Artificial Sequence
<220>
   <223> Nucleotide sequence for TSC001 comprising : HD37 (anti-CD39), human IgG1 SCC-P hinge, human IgG1 CH2, human IgG1 CH3 and human CH1
<400> 34
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcgagc ccaaatcttc tgacaaaact 840
   cacacatgcc caccgtgccc atccggagca cctgaactcc tgggtggacc gtcagtcttc 900
   ctcttccccc caaaacccaa ggacaccctc atgatctccc ggacccctga ggtcacatgc 960
   gtggtggtgg acgtgagcca cgaagaccct gaggtcaagt tcaactggta cgtggacggc 1020
   gtggaggtgc ataatgccaa gacaaagccg cgggaggagc agtacaacag cacgtaccgt 1080
   gtggtcagcg tcctcaccgt cctgcaccag gactggctga atggcaagga gtacaagtgc 1140
   aaggtctcca acaaagccct cccagccccc atcgagaaaa ccatctccaa agccaaaggg 1200
   cagccccgag aaccacaggt gtacaccctg cccccatccc gggatgagct gaccaagaac 1260
   caggtcagcc tgacctgcct ggtcaaaggc ttctatccaa gcgacatcgc cgtggagtgg 1320
   gagagcaatg ggcagccgga gaacaactac aagaccacgc ctcccgtgct ggactccgac 1380
   ggctccttct tcctctacag caagctcacc gtggacaaga gcaggtggca gcaggggaac 1440
   gtcttctcat gctccgtgat gcatgaggct ctgcacaacc actacacgca gaagagcctc 1500
   tccctgtctc cgggtaaatc tagagcctcc accaagggcc catcggtctt ccccctggca 1560
   ccctcctcca agagcacctc tgggggcaca gcggccctgg gctgcctggt caaggactac 1620
   ttccccgagc cggtgacggt gtcgtggaac tcaggcgccc tgaccagcgg cgtgcacacc 1680
   ttcccggctg tcctacagtc ctcaggactc tactccctca gcagcgtggt gaccgtgccc 1740
   tccagcagct tgggcaccca gacctacatc tgcaacgtga atcacaagcc cagcaacacc 1800
   aaggtggaca agaaagtttg a 1821
<210> 35
   <211> 1800
   <212> DNA
   <213> Artificial Sequence
<220>
   <223> Nucleotide sequence for TSC002 comprising : 2H7 (anti-CD20) scFv, human IgG1 SCC-P hinge, human IgG1 CH2, human IgG1 CH3 and human CH1
<400> 35
   atggaagcac cagcgcagct tctcttcctc ctgctactct ggctcccaga taccaccggt 60
   caaattgttc tctcccagtc tccagcaatc ctgtctgcat ctccagggga gaaggtcaca 120
   atgacttgca gggccagctc aagtgtaagt tacatgcact ggtaccagca gaagccagga 180
   tcctccccca aaccctggat ttatgcccca tccaacctgg cttctggagt ccctgctcgc 240
   ttcagtggca gtgggtctgg gacctcttac tctctcacaa tcagcagagt ggaggctgaa 300
   gatgctgcca cttattactg ccagcagtgg agttttaacc cacccacgtt cggtgctggg 360
   accaagctgg agctgaaaga tggcggtggc tcgggcggtg gtggatctgg aggaggtggg 420
   gctagccagg cttatctaca gcagtctggg gctgagtcgg tgaggcctgg ggcctcagtg 480
   aagatgtcct gcaaggcttc tggctacaca tttaccagtt acaatatgca ctgggtaaag 540
   cagacaccta gacagggcct ggaatggatt ggagctattt atccaggaaa tggtgatact 600
   tcctacaatc agaagttcaa gggcaaggcc acactgactg tagacaaatc ctccagcaca 660
   gcctacatgc agctcagcag cctgacatct gaagactctg cggtctattt ctgtgcaaga 720
   gtggtgtact atagtaactc ttactggtac ttcgatgtct ggggcacagg gaccacggtc 780
   accgtctcct cgagcgagcc caaatcttct gacaaaactc acacatgccc accgtgccca 840
   tccggagcac ctgaactcct gggtggaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggag tacaagtgca aggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agagcctcca ccaagggccc atcggtcttc cccctggcac cctcctccaa gagcacctct 1560
   gggggcacag cggccctggg ctgcctggtc aaggactact tccccgagcc ggtgacggtg 1620
   tcgtggaact caggcgccct gaccagcggc gtgcacacct tcccggctgt cctacagtcc 1680
   tcaggactct actccctcag cagcgtggtg accgtgccct ccagcagctt gggcacccag 1740
   acctacatct gcaacgtgaa tcacaagccc agcaacacca aggtggacaa gaaagtttga 1800
<210> 36
   <211> 1851
   <212> DNA
   <213> Artificial Sequence
<220>
   <223> Nucleotide sequence for TSC003 comprising : G19-4 (anti-CD3) scFv, altered human IgA1 hinge, human IgG1 CH2, human IgG1 CH3, and human Ck(YAE)
<400> 36
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcccc tcaactccac ctaccccatc tccctcaact 840
   ccacctaccc catctccctc atgcccaccg tgcccagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacaacagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaaatct agaactgtgg ctgcaccatc tgtcttcatc 1560
   ttcccgccat ctgatgagca gttgaaatct ggaactgcct ctgttgtgtg cctgctgaat 1620
   tacttctatc ccagagaggc caaagtacag tggaaggtgg ataacgccct ccaatcgggt 1680
   aactcccagg agagtgccac agagcaggac agcaaggaca gcacctacag cctcagcagc 1740
   gagctgacgc tgagcaaagc agactacgag aaacacaaag tctacgcctg cgaagtcacc 1800
   catcagggcc tgagctcgcc cgtcacaaag agcttcaaca ggggagagtg a          1851
<210> 37
   <211> 1812
   <212> DNA
   <213> Artificial Sequence
<220>
   <223> Nucleotide sequence for TSC004 comprising : G19-4 (anti-CD3) scFv, altered human IgA2 hinge, human IgG1 CH2, human IgG1 CH3, and human Ck(YAE)
<400> 37
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcccc ccacctcccc catgcccacc gtgcccagca 840
   cctgaactcc tgggtggacc gtcagtcttc ctcttccccc caaaacccaa ggacaccctc 900
   atgatctccc ggacccctga ggtcacatgc gtggtggtgg acgtgagcca cgaagaccct 960
   gaggtcaagt tcaactggta cgtggacggc gtggaggtgc ataatgccaa gacaaagccg 1020
   cgggaggagc agtacaacag cacgtaccgt gtggtcagcg tcctcaccgt cctgcaccag 1080
   gactggctga atggcaagga gtacaagtgc aaggtctcca acaaagccct cccagccccc 1140
   atcgagaaaa ccatctccaa agccaaaggg cagccccgag aaccacaggt gtacaccctg 1200
   cccccatccc gggatgagct gaccaagaac caggtcagcc tgacctgcct ggtcaaaggc 1260
   ttctatccaa gcgacatcgc cgtggagtgg gagagcaatg ggcagccgga gaacaactac 1320
   aagaccacgc ctcccgtgct ggactccgac ggctccttct tcctctacag caagctcacc 1380
   gtggacaaga gcaggtggca gcaggggaac gtcttctcat gctccgtgat gcatgaggct 1440
   ctgcacaacc actacacgca gaagagcctc tccctgtctc cgggtaaatc tagaactgtg 1500
   gctgcaccat ctgtcttcat cttcccgcca tctgatgagc agttgaaatc tggaactgcc 1560
   tctgttgtgt gcctgctgaa ttacttctat cccagagagg ccaaagtaca gtggaaggtg 1620
   gataacgccc tccaatcggg taactcccag gagagtgcca cagagcagga cagcaaggac 1680
   agcacctaca gcctcagcag cgagctgacg ctgagcaaag cagactacga gaaacacaaa 1740
   gtctacgcct gcgaagtcac ccatcagggc ctgagctcgc ccgtcacaaa gagcttcaac 1800
   aggggagagt ga                                                    1812
<210> 38
   <211> 1842
   <212> DNA
   <213> Artificial Sequence
<220>
   <223> Nucleotide sequence for TSC005 comprising : G19-4 (anti-CD3) scFv, altered human IgG3 hinge, human IgG1 CH2, human IgG1 CH3, and human Ck(YAE)
<400> 38
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcgag cccaaatcta gcgacacacc tcccccaagc 840
   ccacggtccc catgcccacc gtgcccagca cctgaactcc tgggtggacc gtcagtcttc 900
   ctcttccccc caaaacccaa ggacaccctc atgatctccc ggacccctga ggtcacatgc 960
   gtggtggtgg acgtgagcca cgaagaccct gaggtcaagt tcaactggta cgtggacggc 1020
   gtggaggtgc ataatgccaa gacaaagccg cgggaggagc agtacaacag cacgtaccgt 1080
   gtggtcagcg tcctcaccgt cctgcaccag gactggctga atggcaagga gtacaagtgc 1140
   aaggtctcca acaaagccct cccagccccc atcgagaaaa ccatctccaa agccaaaggg 1200
   cagccccgag aaccacaggt gtacaccctg cccccatccc gggatgagct gaccaagaac 1260
   caggtcagcc tgacctgcct ggtcaaaggc ttctatccaa gcgacatcgc cgtggagtgg 1320
   gagagcaatg ggcagccgga gaacaactac aagaccacgc ctcccgtgct ggactccgac 1380
   ggctccttct tcctctacag caagctcacc gtggacaaga gcaggtggca gcaggggaac 1440
   gtcttctcat gctccgtgat gcatgaggct ctgcacaacc actacacgca gaagagcctc 1500
   tccctgtctc cgggtaaatc tagaactgtg gctgcaccat ctgtcttcat cttcccgcca 1560
   tctgatgagc agttgaaatc tggaactgcc tctgttgtgt gcctgctgaa ttacttctat 1620
   cccagagagg ccaaagtaca gtggaaggtg gataacgccc tccaatcggg taactcccag 1680
   gagagtgcca cagagcagga cagcaaggac agcacctaca gcctcagcag cgagctgacg 1740
   ctgagcaaag cagactacga gaaacacaaa gtctacgcct gcgaagtcac ccatcagggc 1800
   ctgagctcgc ccgtcacaaa gagcttcaac aggggagagt ga                   1842
<210> 39
   <211> 1899
   <212> DNA
   <213> Artificial Sequence
<220>
   <223> Nucleotide sequence for TSC006 comprising : G19-4 (anti-CD3) scFv, altered human IgD hinge, human IgG1 CH2, human IgG1 CH3, and human Ck(YAE)
<400> 39
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcgag tctccaaagg cacaggcctc ctccgtgccc 840
   actgcacaac cccaagcaga gggcagcctc gccaaggcaa ccacagcccc agccaccacc 900
   cgtaacacat gcccaccgtg cccagcacct gaactcctgg gtggaccgtc agtcttcctc 960
   ttccccccaa aacccaagga caccctcatg atctcccgga cccctgaggt cacatgcgtg 1020
   gtggtggacg tgagccacga agaccctgag gtcaagttca actggtacgt ggacggcgtg 1080
   gaggtgcata atgccaagac aaagccgcgg gaggagcagt acaacagcac gtaccgtgtg 1140
   gtcagcgtcc tcaccgtcct gcaccaggac tggctgaatg gcaaggagta caagtgcaag 1200
   gtctccaaca aagccctccc agcccccatc gagaaaacca tctccaaagc caaagggcag 1260
   ccccgagaac cacaggtgta caccctgccc ccatcccggg atgagctgac caagaaccag 1320
   gtcagcctga cctgcctggt caaaggcttc tatccaagcg acatcgccgt ggagtgggag 1380
   agcaatgggc agccggagaa caactacaag accacgcctc ccgtgctgga ctccgacggc 1440
   tccttcttcc tctacagcaa gctcaccgtg gacaagagca ggtggcagca ggggaacgtc 1500
   ttctcatgct ccgtgatgca tgaggctctg cacaaccact acacgcagaa gagcctctcc 1560
   ctgtctccgg gtaaatctag aactgtggct gcaccatctg tcttcatctt cccgccatct 1620
   gatgagcagt tgaaatctgg aactgcctct gttgtgtgcc tgctgaatta cttctatccc 1680
   agagaggcca aagtacagtg gaaggtggat aacgccctcc aatcgggtaa ctcccaggag 1740
   agtgccacag agcaggacag caaggacagc acctacagcc tcagcagcga gctgacgctg 1800
   agcaaagcag actacgagaa acacaaagtc tacgcctgcg aagtcaccca tcagggcctg 1860
   agctcgcccg tcacaaagag cttcaacagg ggagagtga 1899
<210> 40
   <211> 1842
   <212> DNA
   <213> Artificial Sequence
<220>
   <223> Nucleotide sequence for TSC007 comprising : G19-4 (anti-CD3) scFv, altered human IgG1 hinge, human IgG1 CH2, human IgG1 CH3, and human Ck (YAE)
<400> 40
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcgag cccaaatctt ctgacaaaac tcacacaagc 840
   ccaccgagcc catgcccacc gtgcccagca cctgaactcc tgggtggacc gtcagtcttc 900
   ctcttccccc caaaacccaa ggacaccctc atgatctccc ggacccctga ggtcacatgc 960
   gtggtggtgg acgtgagcca cgaagaccct gaggtcaagt tcaactggta cgtggacggc 1020
   gtggaggtgc ataatgccaa gacaaagccg cgggaggagc agtacaacag cacgtaccgt 1080
   gtggtcagcg tcctcaccgt cctgcaccag gactggctga atggcaagga gtacaagtgc 1140
   aaggtctcca acaaagccct cccagccccc atcgagaaaa ccatctccaa agccaaaggg 1200
   cagccccgag aaccacaggt gtacaccctg cccccatccc gggatgagct gaccaagaac 1260
   caggtcagcc tgacctgcct ggtcaaaggc ttctatccaa gcgacatcgc cgtggagtgg 1320
   gagagcaatg ggcagccgga gaacaactac aagaccacgc ctcccgtgct ggactccgac 1380
   ggctccttct tcctctacag caagctcacc gtggacaaga gcaggtggca gcaggggaac 1440
   gtcttctcat gctccgtgat gcatgaggct ctgcacaacc actacacgca gaagagcctc 1500
   tccctgtctc cgggtaaatc tagaactgtg gctgcaccat ctgtcttcat cttcccgcca 1560
   tctgatgagc agttgaaatc tggaactgcc tctgttgtgt gcctgctgaa ttacttctat 1620
   cccagagagg ccaaagtaca gtggaaggtg gataacgccc tccaatcggg taactcccag 1680
   gagagtgcca cagagcagga cagcaaggac agcacctaca gcctcagcag cgagctgacg 1740
   ctgagcaaag cagactacga gaaacacaaa gtctacgcct gcgaagtcac ccatcagggc 1800
   ctgagctcgc ccgtcacaaa gagcttcaac aggggagagt ga 1842
<210> 41
   <211> 2115
   <212> DNA
   <213> Artificial Sequence
<220>
   <223> Nucleotide sequence for TSC008 comprising : G19-4 (anti-CD3) scFv, altered human IgE CH2 hinge, human IgG1 CH2, human IgG1 CH3, and human Ck (YAE)
<400> 41
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagctcc agggacttca ccccgcccac cgtgaagatc 840
   ttacagtcgt ccagcgacgg cggcgggcac ttccccccga ccatccagct cctgtgcctc 900
   gtctctgggt acaccccagg gactatcaac atcacctggc tggaggacgg gcaggtcatg 960
   gacgtggact tgtccaccgc ctctaccacg caggagggtg agctggcctc cacacaaagc 1020
   gagctcaccc tcagccagaa gcactggctg tcagaccgca cctacacctg ccaggtcacc 1080
   tatcaaggtc acacctttga ggacagcacc aagaagtctg catgcccacc gtgctccgga 1140
   gcacctgaac tcctgggtgg accgtcagtc ttcctcttcc ccccaaaacc caaggacacc 1200
   ctcatgatct cccggacccc tgaggtcaca tgcgtggtgg tggacgtgag ccacgaagac 1260
   cctgaggtca agttcaactg gtacgtggac ggcgtggagg tgcataatgc caagacaaag 1320
   ccgcgggagg agcagtacaa cagcacgtac cgtgtggtca gcgtcctcac cgtcctgcac 1380
   caggactggc tgaatggcaa ggagtacaag tgcaaggtct ccaacaaagc cctcccagcc 1440
   cccatcgaga aaaccatctc caaagccaaa gggcagcccc gagaaccaca ggtgtacacc 1500
   ctgcccccat cccgggatga gctgaccaag aaccaggtca gcctgacctg cctggtcaaa 1560
   ggcttctatc caagcgacat cgccgtggag tgggagagca atgggcagcc ggagaacaac 1620
   tacaagacca cgcctcccgt gctggactcc gacggctcct tcttcctcta cagcaagctc 1680
   accgtggaca agagcaggtg gcagcagggg aacgtcttct catgctccgt gatgcatgag 1740
   gctctgcaca accactacac gcagaagagc ctctccctgt ctccgggtaa atctagaact 1800
   gtggctgcac catctgtctt catcttcccg ccatctgatg agcagttgaa atctggaact 1860
   gcctctgttg tgtgcctgct gaattacttc tatcccagag aggccaaagt acagtggaag 1920
   gtggataacg ccctccaatc gggtaactcc caggagagtg ccacagagca ggacagcaag 1980
   gacagcacct acagcctcag cagcgagctg acgctgagca aagcagacta cgagaaacac 2040
   aaagtctacg cctgcgaagt cacccatcag ggcctgagct cgcccgtcac aaagagcttc 2100
   aacaggggag agtga                                                  2115
<210> 42
   <211> 2136
   <212> DNA
   <213> Artificial Sequence
<220>
   <223> Nucleotide sequence for TSC009 comprising : G19-4 (anti-CD3) scFv, altered human IgM CH2 hinge, human IgG1 CH2, human IgG1 CH3, and human Ck (YAE)
<400> 42
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcatt gccgagctgc ctcccaaagt gagcgtcttc 840
   gtcccacccc gcgacggctt cttcggcaac ccccgcaagt ccaagctcat ctgccaggcc 900
   acgggtttca gtccccggca gattcaggtg tcctggctgc gcgaggggaa gcaggtgggg 960
   tctggcgtca ccacggacca ggtgcaggct gaggccaaag agtctgggcc cacgacctac 1020
   aaggtgacca gcacactgac catcaaagag agcgactggc tcagccagag catgttcacc 1080
   tgccgcgtgg atcacagggg cctgaccttc cagcagaatg cgtcctccat gagtgtcccc 1140
   tgcccaccgt gcccatccgg agcacctgaa ctcctgggtg gaccgtcagt cttcctcttc 1200
   cccccaaaac ccaaggacac cctcatgatc tcccggaccc ctgaggtcac atgcgtggtg 1260
   gtggacgtga gccacgaaga ccctgaggtc aagttcaact ggtacgtgga cggcgtggag 1320
   gtgcataatg ccaagacaaa gccgcgggag gagcagtaca acagcacgta ccgtgtggtc 1380
   agcgtcctca ccgtcctgca ccaggactgg ctgaatggca aggagtacaa gtgcaaggtc 1440
   tccaacaaag ccctcccagc ccccatcgag aaaaccatct ccaaagccaa agggcagccc 1500
   cgagaaccac aggtgtacac cctgccccca tcccgggatg agctgaccaa gaaccaggtc 1560
   agcctgacct gcctggtcaa aggcttctat ccaagcgaca tcgccgtgga gtgggagagc 1620
   aatgggcagc cggagaacaa ctacaagacc acgcctcccg tgctggactc cgacggctcc 1680
   ttcttcctct acagcaagct caccgtggac aagagcaggt ggcagcaggg gaacgtcttc 1740
   tcatgctccg tgatgcatga ggctctgcac aaccactaca cgcagaagag cctctccctg 1800
   tctccgggta aatctagaac tgtggctgca ccatctgtct tcatcttccc gccatctgat 1860
   gagcagttga aatctggaac tgcctctgtt gtgtgcctgc tgaattactt ctatcccaga 1920
   gaggccaaag tacagtggaa ggtggataac gccctccaat cgggtaactc ccaggagagt 1980
   gccacagagc aggacagcaa ggacagcacc tacagcctca gcagcgagct gacgctgagc 2040
   aaagcagact acgagaaaca caaagtctac gcctgcgaag tcacccatca gggcctgagc 2100
   tcgcccgtca caaagagctt caacagggga gagtga                           2136
<210> 43
   <211> 1839
   <212> DNA
   <213> Artificial Sequence
<220>
   <223> Nucleotide sequence for TSC010 comprising : HD37 (anti-CD19) scFv, altered human IgA1 hinge, human IgG1 CH2, human IgG1 CH3, and human CH1
<400> 43
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcccct caactccacc taccccatct 840
   ccctcaactc cacctacccc atctccctca tgcccaccgt gcccagcacc tgaactcctg 900
   ggtggaccgt cagtcttcct cttcccccca aaacccaagg acaccctcat gatctcccgg 960
   acccctgagg tcacatgcgt ggtggtggac gtgagccacg aagaccctga ggtcaagttc 1020
   aactggtacg tggacggcgt ggaggtgcat aatgccaaga caaagccgcg ggaggagcag 1080
   tacaacagca cgtaccgtgt ggtcagcgtc ctcaccgtcc tgcaccagga ctggctgaat 1140
   ggcaaggagt acaagtgcaa ggtctccaac aaagccctcc cagcccccat cgagaaaacc 1200
   atctccaaag ccaaagggca gccccgagaa ccacaggtgt acaccctgcc cccatcccgg 1260
   gatgagctga ccaagaacca ggtcagcctg acctgcctgg tcaaaggctt ctatccaagc 1320
   gacatcgccg tggagtggga gagcaatggg cagccggaga acaactacaa gaccacgcct 1380
   cccgtgctgg actccgacgg ctccttcttc ctctacagca agctcaccgt ggacaagagc 1440
   aggtggcagc aggggaacgt cttctcatgc tccgtgatgc atgaggctct gcacaaccac 1500
   tacacgcaga agagcctctc cctgtctccg ggtaaatcta gagcctccac caagggccca 1560
   tcggtcttcc ccctggcacc ctcctccaag agcacctctg ggggcacagc ggccctgggc 1620
   tgcctggtca aggactactt ccccgagccg gtgacggtgt cgtggaactc aggcgccctg 1680
   accagcggcg tgcacacctt cccggctgtc ctacagtcct caggactcta ctccctcagc 1740
   agcgtggtga ccgtgccctc cagcagcttg ggcacccaga cctacatctg caacgtgaat 1800
   cacaagccca gcaacaccaa ggtggacaag aaagtttga 1839
<210> 44
   <211> 1800
   <212> DNA
   <213> Artificial Sequence
<220>
   <223> Nucleotide sequence for TSC011 comprising : HD37 (anti-CD19) scFv, altered human IgA2 hinge, human IgG1 CH2, human IgG1 CH3, and human CH1
<400> 44
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagccccc cacctccccc atgcccaccg 840
   tgcccagcac ctgaactcct gggtggaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggag tacaagtgca aggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agagcctcca ccaagggccc atcggtcttc cccctggcac cctcctccaa gagcacctct 1560
   gggggcacag cggccctggg ctgcctggtc aaggactact tccccgagcc ggtgacggtg 1620
   tcgtggaact caggcgccct gaccagcggc gtgcacacct tcccggctgt cctacagtcc 1680
   tcaggactct actccctcag cagcgtggtg accgtgccct ccagcagctt gggcacccag 1740
   acctacatct gcaacgtgaa tcacaagccc agcaacacca aggtggacaa gaaagtttga 1800
<210> 45
   <211> 1830
   <212> DNA
   <213> Artificial Sequence
<220>
   <223> Nucleotide sequence for TSC012 comprising : HD37 (anti-CD19) scFv, altered human IgG1 hinge, human IgG1 CH2, human IgG1 CH3, and human CH1
<400> 45
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcgagc ccaaatcttc tgacaaaact 840
   cacacaagcc caccgagccc atgcccaccg tgcccagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacaacagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaaatct agagcctcca ccaagggccc atcggtcttc 1560
   cccctggcac cctcctccaa gagcacctct gggggcacag cggccctggg ctgcctggtc 1620
   aaggactact tccccgagcc ggtgacggtg tcgtggaact caggcgccct gaccagcggc 1680
   gtgcacacct tcccggctgt cctacagtcc tcaggactct actccctcag cagcgtggtg 1740
   accgtgccct ccagcagctt gggcacccag acctacatct gcaacgtgaa tcacaagccc 1800
   agcaacacca aggtggacaa gaaagtttga 1830
<210> 46
   <211> 1830
   <212> DNA
   <213> Artificial Sequence
<220>
   <223> Nucleotide sequence for TSC013 comprising : HD37 (anti-CD19) scFv, altered human IgG3 hinge, human IgG1 CH2, human IgG1 CH3, and human CH1
<400> 46
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcgagc ccaaatctag cgacacacct 840
   cccccaagcc cacggtcccc atgcccaccg tgcccagcac ctgaactcct gggtggaccg 900
   tcagtcttcc tcttcccccc aaaacccaag gacaccctca tgatctcccg gacccctgag 960
   gtcacatgcg tggtggtgga cgtgagccac gaagaccctg aggtcaagtt caactggtac 1020
   gtggacggcg tggaggtgca taatgccaag acaaagccgc gggaggagca gtacaacagc 1080
   acgtaccgtg tggtcagcgt cctcaccgtc ctgcaccagg actggctgaa tggcaaggag 1140
   tacaagtgca aggtctccaa caaagccctc ccagccccca tcgagaaaac catctccaaa 1200
   gccaaagggc agccccgaga accacaggtg tacaccctgc ccccatcccg ggatgagctg 1260
   accaagaacc aggtcagcct gacctgcctg gtcaaaggct tctatccaag cgacatcgcc 1320
   gtggagtggg agagcaatgg gcagccggag aacaactaca agaccacgcc tcccgtgctg 1380
   gactccgacg gctccttctt cctctacagc aagctcaccg tggacaagag caggtggcag 1440
   caggggaacg tcttctcatg ctccgtgatg catgaggctc tgcacaacca ctacacgcag 1500
   aagagcctct ccctgtctcc gggtaaatct agagcctcca ccaagggccc atcggtcttc 1560
   cccctggcac cctcctccaa gagcacctct gggggcacag cggccctggg ctgcctggtc 1620
   aaggactact tccccgagcc ggtgacggtg tcgtggaact caggcgccct gaccagcggc 1680
   gtgcacacct tcccggctgt cctacagtcc tcaggactct actccctcag cagcgtggtg 1740
   accgtgccct ccagcagctt gggcacccag acctacatct gcaacgtgaa tcacaagccc 1800
   agcaacacca aggtggacaa gaaagtttga                                  1830
<210> 47
   <211> 1887
   <212> DNA
   <213> Artificial Sequence
<220>
   <223> Nucleotide sequence for TSC014 comprising : HD37 (anti-CD19) scFv, altered human IgD hinge, human IgG1 CH2, human IgG1 CH3, and human CH1
<400> 47
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcgagt ctccaaaggc acaggcctcc 840
   tccgtgccca ctgcacaacc ccaagcagag ggcagcctcg ccaaggcaac cacagcccca 900
   gccaccaccc gtaacacatg cccaccgtgc ccagcacctg aactcctggg tggaccgtca 960
   gtcttcctct tccccccaaa acccaaggac accctcatga tctcccggac ccctgaggtc 1020
   acatgcgtgg tggtggacgt gagccacgaa gaccctgagg tcaagttcaa ctggtacgtg 1080
   gacggcgtgg aggtgcataa tgccaagaca aagccgcggg aggagcagta caacagcacg 1140
   taccgtgtgg tcagcgtcct caccgtcctg caccaggact ggctgaatgg caaggagtac 1200
   aagtgcaagg tctccaacaa agccctccca gcccccatcg agaaaaccat ctccaaagcc 1260
   aaagggcagc cccgagaacc acaggtgtac accctgcccc catcccggga tgagctgacc 1320
   aagaaccagg tcagcctgac ctgcctggtc aaaggcttct atccaagcga catcgccgtg 1380
   gagtgggaga gcaatgggca gccggagaac aactacaaga ccacgcctcc cgtgctggac 1440
   tccgacggct ccttcttcct ctacagcaag ctcaccgtgg acaagagcag gtggcagcag 1500
   gggaacgtct tctcatgctc cgtgatgcat gaggctctgc acaaccacta cacgcagaag 1560
   agcctctccc tgtctccggg taaatctaga gcctccacca agggcccatc ggtcttcccc 1620
   ctggcaccct cctccaagag cacctctggg ggcacagcgg ccctgggctg cctggtcaag 1680
   gactacttcc ccgagccggt gacggtgtcg tggaactcag gcgccctgac cagcggcgtg 1740
   cacaccttcc cggctgtcct acagtcctca ggactctact ccctcagcag cgtggtgacc 1800
   gtgccctcca gcagcttggg cacccagacc tacatctgca acgtgaatca caagcccagc 1860
   aacaccaagg tggacaagaa agtttga                                     1887
<210> 48
   <211> 2103
   <212> DNA
   <213> Artificial Sequence
<220>
   <223> Nucleotide sequence for TSC015 comprising : HD37 (anti-CD19) scFv, altered human IgE CH2 hinge, human IgG1 CH2, human IgG1 CH3, and human CH1
<400> 48
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagctcca gggacttcac cccgcccacc 840
   gtgaagatct tacagtcgtc cagcgacggc ggcgggcact tccccccgac catccagctc 900
   ctgtgcctcg tctctgggta caccccaggg actatcaaca tcacctggct ggaggacggg 960
   caggtcatgg acgtggactt gtccaccgcc tctaccacgc aggagggtga gctggcctcc 1020
   acacaaagcg agctcaccct cagccagaag cactggctgt cagaccgcac ctacacctgc 1080
   caggtcacct atcaaggtca cacctttgag gacagcacca agaagtctgc atgcccaccg 1140
   tgctccggag cacctgaact cctgggtgga ccgtcagtct tcctcttccc cccaaaaccc 1200
   aaggacaccc tcatgatctc ccggacccct gaggtcacat gcgtggtggt ggacgtgagc 1260
   cacgaagacc ctgaggtcaa gttcaactgg tacgtggacg gcgtggaggt gcataatgcc 1320
   aagacaaagc cgcgggagga gcagtacaac agcacgtacc gtgtggtcag cgtcctcacc 1380
   gtcctgcacc aggactggct gaatggcaag gagtacaagt gcaaggtctc caacaaagcc 1440
   ctcccagccc ccatcgagaa aaccatctcc aaagccaaag ggcagccccg agaaccacag 1500
   gtgtacaccc tgcccccatc ccgggatgag ctgaccaaga accaggtcag cctgacctgc 1560
   ctggtcaaag gcttctatcc aagcgacatc gccgtggagt gggagagcaa tgggcagccg 1620
   gagaacaact acaagaccac gcctcccgtg ctggactccg acggctcctt cttcctctac 1680
   agcaagctca ccgtggacaa gagcaggtgg cagcagggga acgtcttctc atgctccgtg 1740
   atgcatgagg ctctgcacaa ccactacacg cagaagagcc tctccctgtc tccgggtaaa 1800
   tctagagcct ccaccaaggg cccatcggtc ttccccctgg caccctcctc caagagcacc 1860
   tctgggggca cagcggccct gggctgcctg gtcaaggact acttccccga gccggtgacg 1920
   gtgtcgtgga actcaggcgc cctgaccagc ggcgtgcaca ccttcccggc tgtcctacag 1980
   tcctcaggac tctactccct cagcagcgtg gtgaccgtgc cctccagcag cttgggcacc 2040
   cagacctaca tctgcaacgt gaatcacaag cccagcaaca ccaaggtgga caagaaagtt 2100
   tga                                                               2103
<210> 49
   <211> 2124
   <212> DNA
   <213> Artificial Sequence
<220>
   <223> Nucleotide sequence for TSC016 comprising : HD37 (anti-CD19) scFv, altered human IgM CH2 hinge, human IgG1 CH2, human IgG1 CH3, and human CH1
<400> 49
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcattg ccgagctgcc tcccaaagtg 840
   agcgtcttcg tcccaccccg cgacggcttc ttcggcaacc cccgcaagtc caagctcatc 900
   tgccaggcca cgggtttcag tccccggcag attcaggtgt cctggctgcg cgaggggaag 960
   caggtggggt ctggcgtcac cacggaccag gtgcaggctg aggccaaaga gtctgggccc 1020
   acgacctaca aggtgaccag cacactgacc atcaaagaga gcgactggct cagccagagc 1080
   atgttcacct gccgcgtgga tcacaggggc ctgaccttcc agcagaatgc gtcctccatg 1140
   agtgtcccct gcccaccgtg cccatccgga gcacctgaac tcctgggtgg accgtcagtc 1200
   ttcctcttcc ccccaaaacc caaggacacc ctcatgatct cccggacccc tgaggtcaca 1260
   tgcgtggtgg tggacgtgag ccacgaagac cctgaggtca agttcaactg gtacgtggac 1320
   ggcgtggagg tgcataatgc caagacaaag ccgcgggagg agcagtacaa cagcacgtac 1380
   cgtgtggtca gcgtcctcac cgtcctgcac caggactggc tgaatggcaa ggagtacaag 1440
   tgcaaggtct ccaacaaagc cctcccagcc cccatcgaga aaaccatctc caaagccaaa 1500
   gggcagcccc gagaaccaca ggtgtacacc ctgcccccat cccgggatga gctgaccaag 1560
   aaccaggtca gcctgacctg cctggtcaaa ggcttctatc caagcgacat cgccgtggag 1620
   tgggagagca atgggcagcc ggagaacaac tacaagacca cgcctcccgt gctggactcc 1680
   gacggctcct tcttcctcta cagcaagctc accgtggaca agagcaggtg gcagcagggg 1740
   aacgtcttct catgctccgt gatgcatgag gctctgcaca accactacac gcagaagagc 1800
   ctctccctgt ctccgggtaa atctagagcc tccaccaagg gcccatcggt cttccccctg 1860
   gcaccctcct ccaagagcac ctctgggggc acagcggccc tgggctgcct ggtcaaggac 1920
   tacttccccg agccggtgac ggtgtcgtgg aactcaggcg ccctgaccag cggcgtgcac 1980
   accttcccgg ctgtcctaca gtcctcagga ctctactccc tcagcagcgt ggtgaccgtg 2040
   ccctccagca gcttgggcac ccagacctac atctgcaacg tgaatcacaa gcccagcaac 2100
   accaaggtgg acaagaaagt ttga                                        2124
<210> 50
   <211> 1788
   <212> DNA
   <213> Artificial Sequence
<220>
   <223> Nucleotide sequence for TSC017 comprising : P2C2 (anti-CD79b) scFv, human IgG1 SCC-P hinge, human IgG1 CH2, human IgG1 CH3 and human CH1
<400> 50
   atggaagcac cagcgcagct tctcttcctc ctgctactct ggctcccaga taccaccggt 60
   gatattgtga tgatccagga tgaactctcc aatcctgtca gttctggaga atcagtttcc 120
   atctcctgta ggtctagtaa gagtctccta gataaggaag ggaagacata cttgaattgg 180
   tttctgcaga gaccaggaca atctcctcag ctcctgatct atctgatgtc catgcgtgaa 240
   tcaggagtct cagaccggtt tagtggcagt gggtcaggaa cagatttcac cctggaaatc 300
   agtagagtga aggctgagga tgtgggtgtg tattactgtc aacaacttgt agagtatccg 360
   tggacgttcg gtggaggcac caagctggaa atcaaaggtg gcggtggctc tggcggaggt 420
   ggatccggtg gcggcggctc tcagatccag ttggtgcagt ctggacctga gctgaagaag 480
   cctggagaga cagtcaagat ctcctgcaag gcttctggtt ataccttcac agacttttca 540
   atgcactggg tgaggcaggc tccaggaaag ggtttaaagt ggatgggctg gataaacact 600
   gagactggtg agccaacata tgcagatgac ttcaggggac ggtttgcctt ctctttggaa 660
   acttctgcca gcactgccta tttgcagatc aacaacctca aaaatgagga cacggctaca 720
   tatttttgta cctggtctgc ttactggggc caagggactc tggtcactgt ctctgcctcg 780
   agcgagccca aatcttctga caaaactcac acatgcccac cgtgcccatc cggagcacct 840
   gaactcctgg gtggaccgtc agtcttcctc ttccccccaa aacccaagga caccctcatg 900
   atctcccgga cccctgaggt cacatgcgtg gtggtggacg tgagccacga agaccctgag 960
   gtcaagttca actggtacgt ggacggcgtg gaggtgcata atgccaagac aaagccgcgg 1020
   gaggagcagt acaacagcac gtaccgtgtg gtcagcgtcc tcaccgtcct gcaccaggac 1080
   tggctgaatg gcaaggagta caagtgcaag gtctccaaca aagccctccc agcccccatc 1140
   gagaaaacca tctccaaagc caaagggcag ccccgagaac cacaggtgta caccctgccc 1200
   ccatcccggg atgagctgac caagaaccag gtcagcctga cctgcctggt caaaggcttc 1260
   tatccaagcg acatcgccgt ggagtgggag agcaatgggc agccggagaa caactacaag 1320
   accacgcctc ccgtgctgga ctccgacggc tccttcttcc tctacagcaa gctcaccgtg 1380
   gacaagagca ggtggcagca ggggaacgtc ttctcatgct ccgtgatgca tgaggctctg 1440
   cacaaccact acacgcagaa gagcctctcc ctgtctccgg gtaaatctag agcctccacc 1500
   aagggcccat cggtcttccc cctggcaccc tcctccaaga gcacctctgg gggcacagcg 1560
   gccctgggct gcctggtcaa ggactacttc cccgagccgg tgacggtgtc gtggaactca 1620
   ggcgccctga ccagcggcgt gcacaccttc ccggctgtcc tacagtcctc aggactctac 1680
   tccctcagca gcgtggtgac cgtgccctcc agcagcttgg gcacccagac ctacatctgc 1740
   aacgtgaatc acaagcccag caacaccaag gtggacaaga aagtttga              1788
<210> 51
   <211> 1812
   <212> DNA
   <213> Artificial Sequence
<220>
   <223> Nucleotide sequence for TSC018 comprising : M0042 (anti-HLA-DR) scFv, human IgG1 SCC-P hinge, human IgG1 CH2, human IgG1 CH3, and human CH1
<400> 51
   atggaagcac cagcgcagct tctcttcctc ctgctactct ggctcccaga taccaccggt 60
   gacatccaga tgacccagtc tccttccacc ctgtctgcat ctgtaggaga cagagtcacc 120
   atcacttgcc gggccagtca gagtattagt agctggttgg cctggtatca gcagaaacca 180
   gggaaagccc ctaagctcct ggtctattct gtttccagtt tgcaaagtgg ggtcccatca 240
   aggttcagtg gcagtggatc cgggacagat ttcactctca ccatcaacag tctgcaacct 300
   gaagattttg ccacttacta ctgtcaacag agttacagta ccccattcac tttcggccct 360
   gggaccaaag tggatatcaa agggggtgga ggctctggtg gcggtggctc tggcggaggt 420
   ggatccggtg gcggcggctc tgaagttcaa ttgttagagt ctggtggcgg tcttgttcag 480
   cctggtggtt ctttacgtct ttcttgcgct gcttccggat tcactttctc tcattactat 540
   atgtcttggg ttcgccaagc tcctggtaaa ggtttggagt gggtttctta tatcggtcct 600
   tctggtggct ttactgctta tgctgactcc gttaaaggtc gcttcactat ctctagagac 660
   aactctaaga atactctcta cttgcagatg aacagcttaa gggctgagga cacggccgtg 720
   tattactgtg cgagagacgg gtgggttggt agcgagaact actccggtat ggacgtctgg 780
   ggccaaggga ccacggtctc ctcgagcgag cccaaatctt ctgacaaaac tcacacatgc 840
   ccaccgtgcc catccggagc acctgaactc ctgggtggac cgtcagtctt cctcttcccc 900
   ccaaaaccca aggacaccct catgatctcc cggacccctg aggtcacatg cgtggtggtg 960
   gacgtgagcc acgaagaccc tgaggtcaag ttcaactggt acgtggacgg cgtggaggtg 1020
   cataatgcca agacaaagcc gcgggaggag cagtacaaca gcacgtaccg tgtggtcagc 1080
   gtcctcaccg tcctgcacca ggactggctg aatggcaagg agtacaagtg caaggtctcc 1140
   aacaaagccc tcccagcccc catcgagaaa accatctcca aagccaaagg gcagccccga 1200
   gaaccacagg tgtacaccct gcccccatcc cgggatgagc tgaccaagaa ccaggtcagc 1260
   ctgacctgcc tggtcaaagg cttctatcca agcgacatcg ccgtggagtg ggagagcaat 1320
   gggcagccgg agaacaacta caagaccacg cctcccgtgc tggactccga cggctccttc 1380
   ttcctctaca gcaagctcac cgtggacaag agcaggtggc agcaggggaa cgtcttctca 1440
   tgctccgtga tgcatgaggc tctgcacaac cactacacgc agaagagcct ctccctgtct 1500
   ccgggtaaat ctagagcctc caccaagggc ccatcggtct tccccctggc accctcctcc 1560
   aagagcacct ctgggggcac agcggccctg ggctgcctgg tcaaggacta cttccccgag 1620
   ccggtgacgg tgtcgtggaa ctcaggcgcc ctgaccagcg gcgtgcacac cttcccggct 1680
   gtcctacagt cctcaggact ctactccctc agcagcgtgg tgaccgtgcc ctccagcagc 1740
   ttgggcaccc agacctacat ctgcaacgtg aatcacaagc ccagcaacac caaggtggac 1800
   aagaaagttt ga                                                     1812
<210> 52
   <211> 1833
   <212> DNA
   <213> Artificial Sequence
<220>
   <223> Nucleotide sequence for TSC019 comprising : G19-4 (anti-CD3) scFv, human IgG1 SCC-P hinge, human CH2, human CH3, and human Ck (YAE)
<400> 52
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcgag cccaaatctt ctgacaaaac tcacacatgc 840
   ccaccgtgcc catccggagc acctgaactc ctgggtggac cgtcagtctt cctcttcccc 900
   ccaaaaccca aggacaccct catgatctcc cggacccctg aggtcacatg cgtggtggtg 960
   gacgtgagcc acgaagaccc tgaggtcaag ttcaactggt acgtggacgg cgtggaggtg 1020
   cataatgcca agacaaagcc gcgggaggag cagtacaaca gcacgtaccg tgtggtcagc 1080
   gtcctcaccg tcctgcacca ggactggctg aatggcaagg agtacaagtg caaggtctcc 1140
   aacaaagccc tcccagcccc catcgagaaa accatctcca aagccaaagg gcagccccga 1200
   gaaccacagg tgtacaccct gcccccatcc cgggatgagc tgaccaagaa ccaggtcagc 1260
   ctgacctgcc tggtcaaagg cttctatcca agcgacatcg ccgtggagtg ggagagcaat 1320
   gggcagccgg agaacaacta caagaccacg cctcccgtgc tggactccga cggctccttc 1380
   ttcctctaca gcaagctcac cgtggacaag agcaggtggc agcaggggaa cgtcttctca 1440
   tgctccgtga tgcatgaggc tctgcacaac cactacacgc agaagagcct ctccctgtct 1500
   ccgggtaaat ctagaactgt ggctgcacca tctgtcttca tcttcccgcc atctgatgag 1560
   cagttgaaat ctggaactgc ctctgttgtg tgcctgctga attacttcta tcccagagag 1620
   gccaaagtac agtggaaggt ggataacgcc ctccaatcgg gtaactccca ggagagtgcc 1680
   acagagcagg acagcaagga cagcacctac agcctcagca gcgagctgac gctgagcaaa 1740
   gcagactacg agaaacacaa agtctacgcc tgcgaagtca cccatcaggg cctgagctcg 1800
   cccgtcacaa agagcttcaa caggggagag tga                               1833
<210> 53
   <211> 582
   <212> PRT
   <213> Artificial Sequence
<220>
   <223> ORN160 mature protein comprising : 5D5 (anti-c-Met) scFv, human IgG1 SCC-P hinge, human IgG1 CH2, human IgG1 CH3, and human CH1
<400> 53
   Asp Phe Met Met Ser Gln Ser Pro Ser Ser Leu Thr Val Ser Val Gly
    1               5                  10                  15
   Glu Lys Val Thr Val Ser Cys Lys Ser Ser Gln Ser Leu Leu Tyr Thr
               20                  25                  30
   Ser Ser Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Thr Ser Val Lys Ala Asp Asp Leu Ala Val Tyr Tyr Cys Gln Gln
                   85                  90                  95
   Tyr Tyr Ala Tyr Pro Trp Thr Phe Gly Gly Gly Thr Lys Leu Glu Leu
               100                 105                 110
   Lys Gly Gly Gly Gly Ser Gly Gly Gly Gly Ser Gly Gly Gly Gly Ser
           115                 120                 125
   Gln Val Gln Leu Gln Gln Ser Gly Pro Glu Leu Val Arg Pro Gly Ala
       130                 135                 140
   Ser Val Lys Met Ser Cys Arg Ala Ser Gly Tyr Thr Phe Thr Ser Tyr
   145                 150                 155                 160
   Trp Leu His Trp Val Lys Gln Arg Pro Gly Gln Gly Leu Glu Trp Ile
                   165                 170                 175
   Gly Met Ile Asp Pro Ser Asn Ser Asp Thr Arg Phe Asn Pro Asn Phe
               180                 185                 190
   Lys Asp Lys Ala Thr Leu Asn Val Asp Arg Ser Ser Asn Thr Ala Tyr
           195                 200                 205
   Met Leu Leu Ser Ser Leu Thr Ser Ala Asp Ser Ala Val Tyr Tyr Cys
       210                 215                 220
   Ala Thr Tyr Gly Ser Tyr Val Ser Pro Leu Asp Tyr Trp Gly Gln Gly
   225                 230                 235                 240
   Thr Ser Val Thr Val Ser Ser Ser Glu Pro Lys Ser Ser Asp Lys Thr
                   245                 250                 255
   His Thr Cys Pro Pro Cys Pro Ser Gly Ala Pro Glu Leu Leu Gly Gly
               260                 265                 270
   Pro Ser Val Phe Leu Phe Pro Pro Lys Pro Lys Asp Thr Leu Met Ile
           275                 280                 285
   Ser Arg Thr Pro Glu Val Thr Cys Val Val Val Asp Val Ser His Glu
       290                 295                 300
   Asp Pro Glu Val Lys Phe Asn Trp Tyr Val Asp Gly Val Glu Val His
   305                 310                 315                 320
   Asn Ala Lys Thr Lys Pro Arg Glu Glu Gln Tyr Asn Ser Thr Tyr Arg
                   325                 330                 335
   Val Val Ser Val Leu Thr Val Leu His Gln Asp Trp Leu Asn Gly Lys
               340                 345                 350
   Glu Tyr Lys Cys Lys Val Ser Asn Lys Ala Leu Pro Ala Pro Ile Glu
           355                 360                 365
   Lys Thr Ile Ser Lys Ala Lys Gly Gln Pro Arg Glu Pro Gln Val Tyr
       370                 375                 380
   Thr Leu Pro Pro Ser Arg Asp Glu Leu Thr Lys Asn Gln Val Ser Leu
   385                 390                 395                 400
   Thr Cys Leu Val Lys Gly Phe Tyr Pro Ser Asp Ile Ala Val Glu Trp
                   405                 410                 415
   Glu Ser Asn Gly Gln Pro Glu Asn Asn Tyr Lys Thr Thr Pro Pro Val
               420                 425                 430
   Leu Asp Ser Asp Gly Ser Phe Phe Leu Tyr Ser Lys Leu Thr Val Asp
           435                 440                 445
   Lys Ser Arg Trp Gln Gln Gly Asn Val Phe Ser Cys Ser Val Met His
       450                 455                 460
   Glu Ala Leu His Asn His Tyr Thr Gln Lys Ser Leu Ser Leu Ser Pro
   465                 470                 475                 480
   Gly Lys Ser Arg Ala Ser Thr Lys Gly Pro Ser Val Phe Pro Leu Ala
                   485                 490                 495
   Pro Ser Ser Lys Ser Thr Ser Gly Gly Thr Ala Ala Leu Gly Cys Leu
               500                 505                 510
   Val Lys Asp Tyr Phe Pro Glu Pro Val Thr Val Ser Trp Asn Ser Gly
           515                 520                 525
   Ala Leu Thr Ser Gly Val His Thr Phe Pro Ala Val Leu Gln Ser Ser
       530                 535                 540
   Gly Leu Tyr Ser Leu Ser Ser Val Val Thr Val Pro Ser Ser Ser Leu
   545                 550                 555                 560
   Gly Thr Gln Thr Tyr Ile Cys Asn Val Asn His Lys Pro Ser Asn Thr
                   565                 570                 575
   Lys Val Asp Lys Lys Val
               580
<210> 54
   <211> 586
   <212> PRT
   <213> Artificial Sequence
<220>
   <223> TSC001 mature protein comprising : HD37 (anti-CD39), human IgG1 SCC-P hinge, human IgG1 CH2, human IgG1 CH3 and human CH1
<400> 54
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Cys Pro Pro Cys Pro Ser Gly Ala Pro Glu
               260                 265                 270
   Leu Leu Gly Gly Pro Ser Val Phe Leu Phe Pro Pro Lys Pro Lys Asp
           275                 280                 285
   Thr Leu Met Ile Ser Arg Thr Pro Glu Val Thr Cys Val Val Val Asp
       290                 295                 300
   Val Ser His Glu Asp Pro Glu Val Lys Phe Asn Trp Tyr Val Asp Gly
   305                 310                 315                 320
   Val Glu Val His Asn Ala Lys Thr Lys Pro Arg Glu Glu Gln Tyr Asn
                   325                 330                 335
   Ser Thr Tyr Arg Val Val Ser Val Leu Thr Val Leu His Gln Asp Trp
               340                 345                 350
   Leu Asn Gly Lys Glu Tyr Lys Cys Lys Val Ser Asn Lys Ala Leu Pro
           355                 360                 365
   Ala Pro Ile Glu Lys Thr Ile Ser Lys Ala Lys Gly Gln Pro Arg Glu
       370                 375                 380
   Pro Gln Val Tyr Thr Leu Pro Pro Ser Arg Asp Glu Leu Thr Lys Asn
   385                 390                 395                 400
   Gln Val Ser Leu Thr Cys Leu Val Lys Gly Phe Tyr Pro Ser Asp Ile
                   405                 410                 415
   Ala Val Glu Trp Glu Ser Asn Gly Gln Pro Glu Asn Asn Tyr Lys Thr
               420                 425                 430
   Thr Pro Pro Val Leu Asp Ser Asp Gly Ser Phe Phe Leu Tyr Ser Lys
           435                 440                 445
   Leu Thr Val Asp Lys Ser Arg Trp Gln Gln Gly Asn Val Phe Ser Cys
       450                 455                 460
   Ser Val Met His Glu Ala Leu His Asn His Tyr Thr Gln Lys Ser Leu
   465                 470                 475                 480
   Ser Leu Ser Pro Gly Lys Ser Arg Ala Ser Thr Lys Gly Pro Ser Val
                   485                 490                 495
   Phe Pro Leu Ala Pro Ser Ser Lys Ser Thr Ser Gly Gly Thr Ala Ala
               500                 505                 510
   Leu Gly Cys Leu Val Lys Asp Tyr Phe Pro Glu Pro Val Thr Val Ser
           515                 520                 525
   Trp Asn Ser Gly Ala Leu Thr Ser Gly Val His Thr Phe Pro Ala Val
       530                 535                 540
   Leu Gln Ser Ser Gly Leu Tyr Ser Leu Ser Ser Val Val Thr Val Pro
   545                 550                 555                 560
   Ser Ser Ser Leu Gly Thr Gln Thr Tyr Ile Cys Asn Val Asn His Lys
                   565                 570                 575
   Pro Ser Asn Thr Lys Val Asp Lys Lys Val
   580 585
<210> 55
   <211> 579
   <212> PRT
   <213> Artificial Sequence
<220>
   <223> TSC002 mature protein comprising : 2H7 (anti-CD20) scFv, human IgG1 SCC-P hinge, human IgG1 CH2, human IgG1 CH3 and human CH1
<400> 55
   Gln Ile Val Leu Ser Gln Ser Pro Ala Ile Leu Ser Ala Ser Pro Gly
    1               5                  10                  15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Pro Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Phe Asn Pro Pro Thr
                   85                  90                  95
   Phe Gly Ala Gly Thr Lys Leu Glu Leu Lys Asp Gly Gly Gly Ser Gly
               100                 105                 110
   Gly Gly Gly Ser Gly Gly Gly Gly Ala Ser Gln Ala Tyr Leu Gln Gln
           115                 120                 125
   Ser Gly Ala Glu Ser Val Arg Pro Gly Ala Ser Val Lys Met Ser Cys
       130                 135                 140
   Lys Ala Ser Gly Tyr Thr Phe Thr Ser Tyr Asn Met His Trp Val Lys
   145                 150                 155                 160
   Gln Thr Pro Arg Gln Gly Leu Glu Trp Ile Gly Ala Ile Tyr Pro Gly
                   165                 170                 175
   Asn Gly Asp Thr Ser Tyr Asn Gln Lys Phe Lys Gly Lys Ala Thr Leu
               180                 185                 190
   Thr Val Asp Lys Ser Ser Ser Thr Ala Tyr Met Gln Leu Ser Ser Leu
           195                 200                 205
   Thr Ser Glu Asp Ser Ala Val Tyr Phe Cys Ala Arg Val Val Tyr Tyr
       210                 215                 220
   Ser Asn Ser Tyr Trp Tyr Phe Asp Val Trp Gly Thr Gly Thr Thr Val
   225                 230                 235                 240
   Thr Val Ser Ser Ser Glu Pro Lys Ser Ser Asp Lys Thr His Thr Cys
                   245                 250                 255
   Pro Pro Cys Pro Ser Gly Ala Pro Glu Leu Leu Gly Gly Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Glu Tyr Lys
               340                 345                 350
   Cys Lys Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Ala Ser Thr Lys Gly Pro Ser Val Phe Pro Leu Ala Pro Ser Ser
                   485                 490                 495
   Lys Ser Thr Ser Gly Gly Thr Ala Ala Leu Gly Cys Leu Val Lys Asp
               500                 505                 510
   Tyr Phe Pro Glu Pro Val Thr Val Ser Trp Asn Ser Gly Ala Leu Thr
           515                 520                 525
   Ser Gly Val His Thr Phe Pro Ala Val Leu Gln Ser Ser Gly Leu Tyr
       530                 535                 540
   Ser Leu Ser Ser Val Val Thr Val Pro Ser Ser Ser Leu Gly Thr Gln
   545                 550                 555                 560
   Thr Tyr Ile Cys Asn Val Asn His Lys Pro Ser Asn Thr Lys Val Asp
                   565                 570                 575
   Lys Lys Val
<210> 56
   <211> 596
   <212> PRT
   <213> Artificial Sequence
<220>
   <223> TSC003 mature protein comprising : G19-4 (anti-CD3) scFv, altered human IgA1 hinge, human IgG1 CH2, human IgG1 CH3, and human Ck (YAE)
<400> 56
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Pro Ser Thr Pro Pro Thr Pro
                   245                 250                 255
   Ser Pro Ser Thr Pro Pro Thr Pro Ser Pro Ser Cys Pro Pro Cys Pro
               260                 265                 270
   Ala Pro Glu Leu Leu Gly Gly Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Asp Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Glu Tyr Lys Cys Lys Val Ser Asn Lys
           355                 360                 365
   Ala Leu Pro Ala Pro Ile Glu Lys Thr Ile Ser Lys Ala Lys Gly Gln
       370                 375                 380
   Pro Arg Glu Pro Gln Val Tyr Thr Leu Pro Pro Ser Arg Asp Glu Leu
   385                 390                 395                 400
   Thr Lys Asn Gln Val Ser Leu Thr Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys Ser Arg Thr Val Ala Ala Pro
                   485                 490                 495
   Ser Val Phe Ile Phe Pro Pro Ser Asp Glu Gln Leu Lys Ser Gly Thr
               500                 505                 510
   Ala Ser Val Val Cys Leu Leu Asn Tyr Phe Tyr Pro Arg Glu Ala Lys
           515                 520                 525
   Val Gln Trp Lys Val Asp Asn Ala Leu Gln Ser Gly Asn Ser Gln Glu
       530                 535                 540
   Ser Ala Thr Glu Gln Asp Ser Lys Asp Ser Thr Tyr Ser Leu Ser Ser
   545                 550                 555                 560
   Glu Leu Thr Leu Ser Lys Ala Asp Tyr Glu Lys His Lys Val Tyr Ala
                   565                 570                 575
   Cys Glu Val Thr His Gln Gly Leu Ser Ser Pro Val Thr Lys Ser Phe
               580                 585                 590
   Asn Arg Gly Glu
           595
<210> 57
   <211> 583
   <212> PRT
   <213> Artificial Sequence
<220>
   <223> TSC004 mature protein comprising : G19-4 (anti-CD3) scFv, altered human IgA2 hinge, human IgG1 CH2, human IgG1 CH3, and human Ck (YAE)
<400> 57
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Pro Pro Pro Pro Pro Cys Pro
                   245                 250                 255
   Pro Cys Pro Ala Pro Glu Leu Leu Gly Gly Pro Ser Val Phe Leu Phe
               260                 265                 270
   Pro Pro Lys Pro Lys Asp Thr Leu Met Ile Ser Arg Thr Pro Glu Val
           275                 280                 285
   Thr Cys Val Val Val Asp Val Ser His Glu Asp Pro Glu Val Lys Phe
       290                 295                 300
   Asn Trp Tyr Val Asp Gly Val Glu Val His Asn Ala Lys Thr Lys Pro
   305                 310                 315                 320
   Arg Glu Glu Gln Tyr Asn Ser Thr Tyr Arg Val Val Ser Val Leu Thr
                   325                 330                 335
   Val Leu His Gln Asp Trp Leu Asn Gly Lys Glu Tyr Lys Cys Lys Val
               340                 345                 350
   Ser Asn Lys Ala Leu Pro Ala Pro Ile Glu Lys Thr Ile Ser Lys Ala
           355                 360                 365
   Lys Gly Gln Pro Arg Glu Pro Gln Val Tyr Thr Leu Pro Pro Ser Arg
       370                 375                 380
   Asp Glu Leu Thr Lys Asn Gln Val Ser Leu Thr Cys Leu Val Lys Gly
   385                 390                 395                 400
   Phe Tyr Pro Ser Asp Ile Ala Val Glu Trp Glu Ser Asn Gly Gln Pro
                   405                 410                 415
   Glu Asn Asn Tyr Lys Thr Thr Pro Pro Val Leu Asp Ser Asp Gly Ser
               420                 425                 430
   Phe Phe Leu Tyr Ser Lys Leu Thr Val Asp Lys Ser Arg Trp Gln Gln
           435                 440                 445
   Gly Asn Val Phe Ser Cys Ser Val Met His Glu Ala Leu His Asn His
       450                 455                 460
   Tyr Thr Gln Lys Ser Leu Ser Leu Ser Pro Gly Lys Ser Arg Thr Val
   465                 470                 475                 480
   Ala Ala Pro Ser Val Phe Ile Phe Pro Pro Ser Asp Glu Gln Leu Lys
                   485                 490                 495
   Ser Gly Thr Ala Ser Val Val Cys Leu Leu Asn Tyr Phe Tyr Pro Arg
               500                 505                 510
   Glu Ala Lys Val Gln Trp Lys Val Asp Asn Ala Leu Gln Ser Gly Asn
           515                 520                 525
   Ser Gln Glu Ser Ala Thr Glu Gln Asp Ser Lys Asp Ser Thr Tyr Ser
       530                 535                 540
   Leu Ser Ser Glu Leu Thr Leu Ser Lys Ala Asp Tyr Glu Lys His Lys
   545                 550                 555                 560
   Val Tyr Ala Cys Glu Val Thr His Gln Gly Leu Ser Ser Pro Val Thr
                   565                 570                 575
   Lys Ser Phe Asn Arg Gly Glu
               580
<210> 58
   <211> 593
   <212> PRT
   <213> Artificial Sequence
<220>
   <223> TSC005 mature protein comprising : G19-4 (anti-CD3) scFv, altered human IgG3 hinge, human IgG1 CH2, human IgG1 CH3, and human Ck (YAE)
<400> 58
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Glu Pro Lys Ser Ser Asp Thr
                   245                 250                 255
   Pro Pro Pro Ser Pro Arg Ser Pro Cys Pro Pro Cys Pro Ala Pro Glu
               260                 265                 270
   Leu Leu Gly Gly Pro Ser Val Phe Leu Phe Pro Pro Lys Pro Lys Asp
           275                 280                 285
   Thr Leu Met Ile Ser Arg Thr Pro Glu Val Thr Cys Val Val Val Asp
       290                 295                 300
   Val Ser His Glu Asp Pro Glu Val Lys Phe Asn Trp Tyr Val Asp Gly
   305                 310                 315                 320
   Val Glu Val His Asn Ala Lys Thr Lys Pro Arg Glu Glu Gln Tyr Asn
                   325                 330                 335
   Ser Thr Tyr Arg Val Val Ser Val Leu Thr Val Leu His Gln Asp Trp
               340                 345                 350
   Leu Asn Gly Lys Glu Tyr Lys Cys Lys Val Ser Asn Lys Ala Leu Pro
           355                 360                 365
   Ala Pro Ile Glu Lys Thr Ile Ser Lys Ala Lys Gly Gln Pro Arg Glu
       370                 375                 380
   Pro Gln Val Tyr Thr Leu Pro Pro Ser Arg Asp Glu Leu Thr Lys Asn
   385                 390                 395                 400
   Gln Val Ser Leu Thr Cys Leu Val Lys Gly Phe Tyr Pro Ser Asp Ile
                   405                 410                 415
   Ala Val Glu Trp Glu Ser Asn Gly Gln Pro Glu Asn Asn Tyr Lys Thr
               420                 425                 430
   Thr Pro Pro Val Leu Asp Ser Asp Gly Ser Phe Phe Leu Tyr Ser Lys
           435                 440                 445
   Leu Thr Val Asp Lys Ser Arg Trp Gln Gln Gly Asn Val Phe Ser Cys
       450                 455                 460
   Ser Val Met His Glu Ala Leu His Asn His Tyr Thr Gln Lys Ser Leu
   465                 470                 475                 480
   Ser Leu Ser Pro Gly Lys Ser Arg Thr Val Ala Ala Pro Ser Val Phe
                   485                 490                 495
   Ile Phe Pro Pro Ser Asp Glu Gln Leu Lys Ser Gly Thr Ala Ser Val
               500                 505                 510
   Val Cys Leu Leu Asn Tyr Phe Tyr Pro Arg Glu Ala Lys Val Gln Trp
           515                 520                 525
   Lys Val Asp Asn Ala Leu Gln Ser Gly Asn Ser Gln Glu Ser Ala Thr
       530                 535                 540
   Glu Gln Asp Ser Lys Asp Ser Thr Tyr Ser Leu Ser Ser Glu Leu Thr
   545                 550                 555                 560
   Leu Ser Lys Ala Asp Tyr Glu Lys His Lys Val Tyr Ala Cys Glu Val
                   565                 570                 575
   Thr His Gln Gly Leu Ser Ser Pro Val Thr Lys Ser Phe Asn Arg Gly
               580                 585                 590
   Glu
<210> 59
   <211> 612
   <212> PRT
   <213> Artificial Sequence
<220>
   <223> TSC006 mature protein comprising : G19-4 (anti-CD3) scFv, altered human IgD hinge, human IgG1 CH2, human IgG1 CH3, and human Ck (YAE)
<220>
   <221> VARIANT
   <222> 375
   <223> Xaa = Any Amino Acid
<400> 59
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Glu Ser Pro Lys Ala Gln Ala
                   245                 250                 255
   Ser Ser Val Pro Thr Ala Gln Pro Gln Ala Glu Gly Ser Leu Ala Lys
               260                 265                 270
   Ala Thr Thr Ala Pro Ala Thr Thr Arg Asn Thr Cys Pro Pro Cys Pro
           275                 280                 285
   Ala Pro Glu Leu Leu Gly Gly Pro Ser Val Phe Leu Phe Pro Pro Lys
       290                 295                 300
   Pro Lys Asp Thr Leu Met Ile Ser Arg Thr Pro Glu Val Thr Cys Val
   305                 310                 315                 320
   Val Val Asp Val Ser His Glu Asp Pro Glu Val Lys Phe Asn Trp Tyr
                   325                 330                 335
   Val Asp Gly Val Glu Val His Asn Ala Lys Thr Lys Pro Arg Glu Glu
               340                 345                 350
   Gln Tyr Asn Ser Thr Tyr Arg Val Val Ser Val Leu Thr Val Leu His
           355                 360                 365
   Gln Asp Trp Leu Asn Gly Xaa Glu Tyr Lys Cys Lys Val Ser Asn Lys
       370                 375                 380
   Ala Leu Pro Ala Pro Ile Glu Lys Thr Ile Ser Lys Ala Lys Gly Gln
   385                 390                 395                 400
   Pro Arg Glu Pro Gln Val Tyr Thr Leu Pro Pro Ser Arg Asp Glu Leu
                   405                 410                 415
   Thr Lys Asn Gln Val Ser Leu Thr Cys Leu Val Lys Gly Phe Tyr Pro
               420                 425                 430
   Ser Asp Ile Ala Val Glu Trp Glu Ser Asn Gly Gln Pro Glu Asn Asn
           435                 440                 445
   Tyr Lys Thr Thr Pro Pro Val Leu Asp Ser Asp Gly Ser Phe Phe Leu
       450                 455                 460
   Tyr Ser Lys Leu Thr Val Asp Lys Ser Arg Trp Gln Gln Gly Asn Val
   465                 470                 475                 480
   Phe Ser Cys Ser Val Met His Glu Ala Leu His Asn His Tyr Thr Gln
                   485                 490                 495
   Lys Ser Leu Ser Leu Ser Pro Gly Lys Ser Arg Thr Val Ala Ala Pro
               500                 505                 510
   Ser Val Phe Ile Phe Pro Pro Ser Asp Glu Gln Leu Lys Ser Gly Thr
           515                 520                 525
   Ala Ser Val Val Cys Leu Leu Asn Tyr Phe Tyr Pro Arg Glu Ala Lys
       530                 535                 540
   Val Gln Trp Lys Val Asp Asn Ala Leu Gln Ser Gly Asn Ser Gln Glu
   545                 550                 555                 560
   Ser Ala Thr Glu Gln Asp Ser Lys Asp Ser Thr Tyr Ser Leu Ser Ser
                   565                 570                 575
   Glu Leu Thr Leu Ser Lys Ala Asp Tyr Glu Lys His Lys Val Tyr Ala
               580                 585                 590
   Cys Glu Val Thr His Gln Gly Leu Ser Ser Pro Val Thr Lys Ser Phe
           595                 600                 605
   Asn Arg Gly Glu
       610
<210> 60
   <211> 593
   <212> PRT
   <213> Artificial Sequence
<220>
   <223> TSC007 mature protein comprising : G19-4 (anti-CD3) scFv, altered human IgG1 hinge, human IgG1 CH2, human IgG1 CH3, and human Ck (YAE)
<400> 60
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Glu Pro Lys Ser Ser Asp Lys
                   245                 250                 255
   Thr His Thr Ser Pro Pro Ser Pro Cys Pro Pro Cys Pro Ala Pro Glu
               260                 265                 270
   Leu Leu Gly Gly Pro Ser Val Phe Leu Phe Pro Pro Lys Pro Lys Asp
           275                 280                 285
   Thr Leu Met Ile Ser Arg Thr Pro Glu Val Thr Cys Val Val Val Asp
       290                 295                 300
   Val Ser His Glu Asp Pro Glu Val Lys Phe Asn Trp Tyr Val Asp Gly
   305                 310                 315                 320
   Val Glu Val His Asn Ala Lys Thr Lys Pro Arg Glu Glu Gln Tyr Asn
                   325                 330                 335
   Ser Thr Tyr Arg Val Val Ser Val Leu Thr Val Leu His Gln Asp Trp
               340                 345                 350
   Leu Asn Gly Lys Glu Tyr Lys Cys Lys Val Ser Asn Lys Ala Leu Pro
           355                 360                 365
   Ala Pro Ile Glu Lys Thr Ile Ser Lys Ala Lys Gly Gln Pro Arg Glu
       370                 375                 380
   Pro Gln Val Tyr Thr Leu Pro Pro Ser Arg Asp Glu Leu Thr Lys Asn
   385                 390                 395                 400
   Gln Val Ser Leu Thr Cys Leu Val Lys Gly Phe Tyr Pro Ser Asp Ile
                   405                 410                 415
   Ala Val Glu Trp Glu Ser Asn Gly Gln Pro Glu Asn Asn Tyr Lys Thr
               420                 425                 430
   Thr Pro Pro Val Leu Asp Ser Asp Gly Ser Phe Phe Leu Tyr Ser Lys
           435                 440                 445
   Leu Thr Val Asp Lys Ser Arg Trp Gln Gln Gly Asn Val Phe Ser Cys
       450                 455                 460
   Ser Val Met His Glu Ala Leu His Asn His Tyr Thr Gln Lys Ser Leu
   465                 470                 475                 480
   Ser Leu Ser Pro Gly Lys Ser Arg Thr Val Ala Ala Pro Ser Val Phe
                   485                 490                 495
   Ile Phe Pro Pro Ser Asp Glu Gln Leu Lys Ser Gly Thr Ala Ser Val
               500                 505                 510
   Val Cys Leu Leu Asn Tyr Phe Tyr Pro Arg Glu Ala Lys Val Gln Trp
           515                 520                 525
   Lys Val Asp Asn Ala Leu Gln Ser Gly Asn Ser Gln Glu Ser Ala Thr
       530                 535                 540
   Glu Gln Asp Ser Lys Asp Ser Thr Tyr Ser Leu Ser Ser Glu Leu Thr
   545                 550                 555                 560
   Leu Ser Lys Ala Asp Tyr Glu Lys His Lys Val Tyr Ala Cys Glu Val
                   565                 570                 575
   Thr His Gln Gly Leu Ser Ser Pro Val Thr Lys Ser Phe Asn Arg Gly
               580                 585                 590
   Glu
<210> 61
   <211> 684
   <212> PRT
   <213> Artificial Sequence
<220>
   <223> TSC008 mature protein comprising : G19-4 (anti-CD3) scFv, altered human IgE CH2 hinge (SEQ ID NO:757), human IgG1 CH2, human IgG1 CH3, and human Ck (YAE)
<400> 61
   Glu Val Gln Leu Gln Gln Ser Gly Pro Glu Leu Val Lys Pro Gly Ala
   1 5 10 15
   Ser Met Lys Ile Ser Cys Lys Ala Ser Gly Tyr Ser Phe Thr Gly Tyr
   20 25 30
   Ile Val Asn Trp Leu Lys Gln Ser His Gly Lys Asn Leu Glu Trp Ile
   35 40 45
   Gly Leu Ile Asn Pro Tyr Lys Gly Leu Thr Thr Tyr Asn Gln Lys Phe
   50 55 60
   Lys Gly Lys Ala Thr Leu Thr Val Asp Lys Ser Ser Ser Thr Ala Tyr
   65 70 75 80
   Met Glu Leu Leu Ser Leu Thr Ser Glu Asp Ser Ala Val Tyr Tyr Cys
   85 90 95
   Ala Arg Ser Gly Tyr Tyr Gly Asp Ser Asp Trp Tyr Phe Asp Val Trp
   100 105 110
   Gly Ala Gly Thr Thr Val Thr Val Ser Ser Gly Gly Gly Gly Ser Gly
   115 120 125
   Gly Gly Gly Ser Gly Gly Gly Gly Ser Ala Gln Asp Ile Gln Met Thr
   130 135 140
   Gln Thr Thr Ser Ser Leu Ser Ala Ser Leu Gly Asp Arg Val Thr Ile
   145 150 155 160
   Ser Cys Arg Ala Ser Gln Asp Ile Arg Asn Tyr Leu Asn Trp Tyr Gln
   165 170 175
   Gln Lys Pro Asp Gly Thr Val Lys Leu Leu Ile Tyr Tyr Thr Ser Arg
   180 185 190
   Leu His Ser Gly Val Pro Ser Arg Phe Ser Gly Ser Gly Ser Gly Thr
   195 200 205
   Asp Tyr Ser Leu Thr Ile Ala Asn Leu Gln Pro Glu Asp Ile Ala Thr
   210 215 220
   Tyr Phe Cys Gln Gln Gly Asn Thr Leu Pro Trp Thr Phe Gly Gly Gly
   225 230 235 240
   Thr Lys Leu Val Thr Lys Arg Ser Ser Ser Arg Asp Phe Thr Pro Pro
   245 250 255
   Thr Val Lys Ile Leu Gln Ser Ser Ser Asp Gly Gly Gly His Phe Pro
               260                 265                 270
   Pro Thr Ile Gln Leu Leu Cys Leu Val Ser Gly Tyr Thr Pro Gly Thr
           275                 280                 285
   Ile Asn Ile Thr Trp Leu Glu Asp Gly Gln Val Met Asp Val Asp Leu
       290                 295                 300
   Ser Thr Ala Ser Thr Thr Gln Glu Gly Glu Leu Ala Ser Thr Gln Ser
   305                 310                 315                 320
   Glu Leu Thr Leu Ser Gln Lys His Trp Leu Ser Asp Arg Thr Tyr Thr
                   325                 330                 335
   Cys Gln Val Thr Tyr Gln Gly His Thr Phe Glu Asp Ser Thr Lys Lys
               340                 345                 350
   Ser Ala Cys Pro Pro Cys Ser Gly Ala Pro Glu Leu Leu Gly Gly Pro
           355                 360                 365
   Ser Val Phe Leu Phe Pro Pro Lys Pro Lys Asp Thr Leu Met Ile Ser
       370                 375                 380
   Arg Thr Pro Glu Val Thr Cys Val Val Val Asp Val Ser His Glu Asp
   385                 390                 395                 400
   Pro Glu Val Lys Phe Asn Trp Tyr Val Asp Gly Val Glu Val His Asn
                   405                 410                 415
   Ala Lys Thr Lys Pro Arg Glu Glu Gln Tyr Asn Ser Thr Tyr Arg Val
               420                 425                 430
   Val Ser Val Leu Thr Val Leu His Gln Asp Trp Leu Asn Gly Lys Glu
           435                 440                 445
   Tyr Lys Cys Lys Val Ser Asn Lys Ala Leu Pro Ala Pro Ile Glu Lys
       450                 455                 460
   Thr Ile Ser Lys Ala Lys Gly Gln Pro Arg Glu Pro Gln Val Tyr Thr
   465                 470                 475                 480
   Leu Pro Pro Ser Arg Asp Glu Leu Thr Lys Asn Gln Val Ser Leu Thr
                   485                 490                 495
   Cys Leu Val Lys Gly Phe Tyr Pro Ser Asp Ile Ala Val Glu Trp Glu
               500                 505                 510
   Ser Asn Gly Gln Pro Glu Asn Asn Tyr Lys Thr Thr Pro Pro Val Leu
           515                 520                 525
   Asp Ser Asp Gly Ser Phe Phe Leu Tyr Ser Lys Leu Thr Val Asp Lys
       530                 535                 540
   Ser Arg Trp Gln Gln Gly Asn Val Phe Ser Cys Ser Val Met His Glu
   545                 550                 555                 560
   Ala Leu His Asn His Tyr Thr Gln Lys Ser Leu Ser Leu Ser Pro Gly
                   565                 570                 575
   Lys Ser Arg Thr Val Ala Ala Pro Ser Val Phe Ile Phe Pro Pro Ser
               580                 585                 590
   Asp Glu Gln Leu Lys Ser Gly Thr Ala Ser Val Val Cys Leu Leu Asn
           595                 600                 605
   Tyr Phe Tyr Pro Arg Glu Ala Lys Val Gln Trp Lys Val Asp Asn Ala
       610                 615                 620
   Leu Gln Ser Gly Asn Ser Gln Glu Ser Ala Thr Glu Gln Asp Ser Lys
   625                 630                 635                 640
   Asp Ser Thr Tyr Ser Leu Ser Ser Glu Leu Thr Leu Ser Lys Ala Asp
                   645                 650                 655
   Tyr Glu Lys His Lys Val Tyr Ala Cys Glu Val Thr His Gln Gly Leu
               660                 665                 670
   Ser Ser Pro Val Thr Lys Ser Phe Asn Arg Gly Glu
           675                 680
<210> 62
   <211> 691
   <212> PRT
   <213> Artificial Sequence
<220>
   <223> TSC009 mature protein comprising : G19-4 (anti-CD3) scFv, altered human IgM CH2 hinge, human IgG1 CH2, human IgG1 CH3, and human Ck (YAE)
<400> 62
<210> 63
   <211> 592
   <212> PRT
   <213> Artificial Sequence
<220>
   <223> TSC010 mature protein comprising : HD37 (anti-CD19) scFv, altered human IgA1 hinge, human IgG1 CH2, human IgG1 CH3, and human CH1
<400> 63
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
           210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Pro Ser Thr Pro
                   245                 250                 255
   Pro Thr Pro Ser Pro Ser Thr Pro Pro Thr Pro Ser Pro Ser Cys Pro
               260                 265                 270
   Pro Cys Pro Ala Pro Glu Leu Leu Gly Gly Pro Ser Val Phe Leu Phe
           275                 280                 285
   Pro Pro Lys Pro Lys Asp Thr Leu Met Ile Ser Arg Thr Pro Glu Val
       290                 295                 300
   Thr Cys Val Val Val Asp Val Ser His Glu Asp Pro Glu Val Lys Phe
   305                 310                 315                 320
   Asn Trp Tyr Val Asp Gly Val Glu Val His Asn Ala Lys Thr Lys Pro
                   325                 330                 335
   Arg Glu Glu Gln Tyr Asn Ser Thr Tyr Arg Val Val Ser Val Leu Thr
               340                 345                 350
   Val Leu His Gln Asp Trp Leu Asn Gly Lys Glu Tyr Lys Cys Lys Val
           355                 360                 365
   Ser Asn Lys Ala Leu Pro Ala Pro Ile Glu Lys Thr Ile Ser Lys Ala
       370                 375                 380
   Lys Gly Gln Pro Arg Glu Pro Gln Val Tyr Thr Leu Pro Pro Ser Arg
   385                 390                 395                 400
   Asp Glu Leu Thr Lys Asn Gln Val Ser Leu Thr Cys Leu Val Lys Gly
                   405                 410                 415
   Phe Tyr Pro Ser Asp Ile Ala Val Glu Trp Glu Ser Asn Gly Gln Pro
               420                 425                 430
   Glu Asn Asn Tyr Lys Thr Thr Pro Pro Val Leu Asp Ser Asp Gly Ser
           435                 440                 445
   Phe Phe Leu Tyr Ser Lys Leu Thr Val Asp Lys Ser Arg Trp Gln Gln
       450                 455                 460
   Gly Asn Val Phe Ser Cys Ser Val Met His Glu Ala Leu His Asn His
   465                 470                 475                 480
   Tyr Thr Gln Lys Ser Leu Ser Leu Ser Pro Gly Lys Ser Arg Ala Ser
                   485                 490                 495
   Thr Lys Gly Pro Ser Val Phe Pro Leu Ala Pro Ser Ser Lys Ser Thr
               500                 505                 510
   Ser Gly Gly Thr Ala Ala Leu Gly Cys Leu Val Lys Asp Tyr Phe Pro
           515                 520                 525
   Glu Pro Val Thr Val Ser Trp Asn Ser Gly Ala Leu Thr Ser Gly Val
       530                 535                 540
   His Thr Phe Pro Ala Val Leu Gln Ser Ser Gly Leu Tyr Ser Leu Ser
   545                 550                 555                 560
   Ser Val Val Thr Val Pro Ser Ser Ser Leu Gly Thr Gln Thr Tyr Ile
                   565                 570                 575
   Cys Asn Val Asn His Lys Pro Ser Asn Thr Lys Val Asp Lys Lys Val
               580                 585                 590
<210> 64
   <211> 579
   <212> PRT
   <213> Artificial Sequence
<220>
   <223> TSC011 mature protein comprising : HD37 (anti-CD19) scFv, altered human IgA2 hinge, human IgG1 CH2, human IgG1 CH3, and human CH1
<400> 64
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Pro Pro Pro Pro
                   245                 250                 255
   Pro Cys Pro Pro Cys Pro Ala Pro Glu Leu Leu Gly Gly Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Glu Tyr Lys
               340                 345                 350
   Cys Lys Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Ala Ser Thr Lys Gly Pro Ser Val Phe Pro Leu Ala Pro Ser Ser
                   485                 490                 495
   Lys Ser Thr Ser Gly Gly Thr Ala Ala Leu Gly Cys Leu Val Lys Asp
               500                 505                 510
   Tyr Phe Pro Glu Pro Val Thr Val Ser Trp Asn Ser Gly Ala Leu Thr
           515                 520                 525
   Ser Gly Val His Thr Phe Pro Ala Val Leu Gln Ser Ser Gly Leu Tyr
       530                 535                 540
   Ser Leu Ser Ser Val Val Thr Val Pro Ser Ser Ser Leu Gly Thr Gln
   545                 550                 555                 560
   Thr Tyr Ile Cys Asn Val Asn His Lys Pro Ser Asn Thr Lys Val Asp
                   565                 570                 575
   Lys Lys Val
<210> 65
   <211> 589
   <212> PRT
   <213> Artificial Sequence
<220>
   <223> TSC012 mature protein comprising : HD37 (anti-CD19) scFv, altered human IgG1 hinge, human IgG1 CH2, human IgG1 CH3, and human CH1
<400> 65
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Ser Pro Pro Ser Pro Cys Pro Pro Cys Pro
               260                 265                 270
   Ala Pro Glu Leu Leu Gly Gly Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Asp Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Glu Tyr Lys Cys Lys Val Ser Asn Lys
           355                 360                 365
   Ala Leu Pro Ala Pro Ile Glu Lys Thr Ile Ser Lys Ala Lys Gly Gln
       370                 375                 380
   Pro Arg Glu Pro Gln Val Tyr Thr Leu Pro Pro Ser Arg Asp Glu Leu
   385                 390                 395                 400
   Thr Lys Asn Gln Val Ser Leu Thr Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys Ser Arg Ala Ser Thr Lys Gly
                   485                 490                 495
   Pro Ser Val Phe Pro Leu Ala Pro Ser Ser Lys Ser Thr Ser Gly Gly
               500                 505                 510
   Thr Ala Ala Leu Gly Cys Leu Val Lys Asp Tyr Phe Pro Glu Pro Val
           515                 520                 525
   Thr Val Ser Trp Asn Ser Gly Ala Leu Thr Ser Gly Val His Thr Phe
       530                 535                 540
   Pro Ala Val Leu Gln Ser Ser Gly Leu Tyr Ser Leu Ser Ser Val Val
   545                 550                 555                 560
   Thr Val Pro Ser Ser Ser Leu Gly Thr Gln Thr Tyr Ile Cys Asn Val
                   565                 570                 575
   Asn His Lys Pro Ser Asn Thr Lys Val Asp Lys Lys Val
               580                 585
<210> 66
   <211> 589
   <212> PRT
   <213> Artificial Sequence
<220>
   <223> TSC013 mature protein comprising : HD37 (anti-CD19) scFv, altered human IgG3 hinge, human IgG1 CH2, human IgG1 CH3, and human CH1
<400> 66
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Thr Pro Pro Pro Ser Pro Arg Ser Pro Cys Pro Pro Cys Pro
               260                 265                 270
   Ala Pro Glu Leu Leu Gly Gly Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Asp Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Glu Tyr Lys Cys Lys Val Ser Asn Lys
           355                 360                 365
   Ala Leu Pro Ala Pro Ile Glu Lys Thr Ile Ser Lys Ala Lys Gly Gln
       370                 375                 380
   Pro Arg Glu Pro Gln Val Tyr Thr Leu Pro Pro Ser Arg Asp Glu Leu
   385                 390                 395                 400
   Thr Lys Asn Gln Val Ser Leu Thr Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys Ser Arg Ala Ser Thr Lys Gly
                   485                 490                 495
   Pro Ser Val Phe Pro Leu Ala Pro Ser Ser Lys Ser Thr Ser Gly Gly
               500                 505                 510
   Thr Ala Ala Leu Gly Cys Leu Val Lys Asp Tyr Phe Pro Glu Pro Val
           515                 520                 525
   Thr Val Ser Trp Asn Ser Gly Ala Leu Thr Ser Gly Val His Thr Phe
       530                 535                 540
   Pro Ala Val Leu Gln Ser Ser Gly Leu Tyr Ser Leu Ser Ser Val Val
   545                 550                 555                 560
   Thr Val Pro Ser Ser Ser Leu Gly Thr Gln Thr Tyr Ile Cys Asn Val
                   565                 570                 575
   Asn His Lys Pro Ser Asn Thr Lys Val Asp Lys Lys Val
               580                 585
<210> 67
   <211> 608
   <212> PRT
   <213> Artificial Sequence
<220>
   <223> TSC014 mature protein comprising : HD37 (anti-CD19) scFv, altered human IgD hinge, human IgG1 CH2, human IgG1 CH3, and human CH1
<400> 67
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
       35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Ser Pro Lys
                   245                 250                 255
   Ala Gln Ala Ser Ser Val Pro Thr Ala Gln Pro Gln Ala Glu Gly Ser
               260                 265                 270
   Leu Ala Lys Ala Thr Thr Ala Pro Ala Thr Thr Arg Asn Thr Cys Pro
           275                 280                 285
   Pro Cys Pro Ala Pro Glu Leu Leu Gly Gly Pro Ser Val Phe Leu Phe
       290                 295                 300
   Pro Pro Lys Pro Lys Asp Thr Leu Met Ile Ser Arg Thr Pro Glu Val
   305                 310                 315                 320
   Thr Cys Val Val Val Asp Val Ser His Glu Asp Pro Glu Val Lys Phe
                   325                 330                 335
   Asn Trp Tyr Val Asp Gly Val Glu Val His Asn Ala Lys Thr Lys Pro
               340                 345                 350
   Arg Glu Glu Gln Tyr Asn Ser Thr Tyr Arg Val Val Ser Val Leu Thr
           355                 360                 365
   Val Leu His Gln Asp Trp Leu Asn Gly Lys Glu Tyr Lys Cys Lys Val
       370                 375                 380
   Ser Asn Lys Ala Leu Pro Ala Pro Ile Glu Lys Thr Ile Ser Lys Ala
   385                 390                 395                 400
   Lys Gly Gln Pro Arg Glu Pro Gln Val Tyr Thr Leu Pro Pro Ser Arg
                   405                 410                 415
   Asp Glu Leu Thr Lys Asn Gln Val Ser Leu Thr Cys Leu Val Lys Gly
               420                 425                 430
   Phe Tyr Pro Ser Asp Ile Ala Val Glu Trp Glu Ser Asn Gly Gln Pro
           435                 440                 445
   Glu Asn Asn Tyr Lys Thr Thr Pro Pro Val Leu Asp Ser Asp Gly Ser
       450                 455                 460
   Phe Phe Leu Tyr Ser Lys Leu Thr Val Asp Lys Ser Arg Trp Gln Gln
   465                 470                 475                 480
   Gly Asn Val Phe Ser Cys Ser Val Met His Glu Ala Leu His Asn His
                   485                 490                 495
   Tyr Thr Gln Lys Ser Leu Ser Leu Ser Pro Gly Lys Ser Arg Ala Ser
               500                 505                 510
   Thr Lys Gly Pro Ser Val Phe Pro Leu Ala Pro Ser Ser Lys Ser Thr
           515                 520                 525
   Ser Gly Gly Thr Ala Ala Leu Gly Cys Leu Val Lys Asp Tyr Phe Pro
       530                 535                 540
   Glu Pro Val Thr Val Ser Trp Asn Ser Gly Ala Leu Thr Ser Gly Val
   545                 550                 555                 560
   His Thr Phe Pro Ala Val Leu Gln Ser Ser Gly Leu Tyr Ser Leu Ser
                   565                 570                 575
   Ser Val Val Thr Val Pro Ser Ser Ser Leu Gly Thr Gln Thr Tyr Ile
               580                 585                 590
   Cys Asn Val Asn His Lys Pro Ser Asn Thr Lys Val Asp Lys Lys Val
           595                 600                 605
<210> 68
   <211> 680
   <212> PRT
   <213> Artificial Sequence
<220>
   <223> TSC015 mature protein comprising : HD37 (anti-CD19) scFv, altered human IgE CH2 hinge, human IgG1 CH2, human IgG1 CH3, and human CH1
<400> 68
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Ser Arg Asp Phe
                   245                 250                 255
   Thr Pro Pro Thr Val Lys Ile Leu Gln Ser Ser Ser Asp Gly Gly Gly
               260                 265                 270
   His Phe Pro Pro Thr Ile Gln Leu Leu Cys Leu Val Ser Gly Tyr Thr
           275                 280                 285
   Pro Gly Thr Ile Asn Ile Thr Trp Leu Glu Asp Gly Gln Val Met Asp
       290                 295                 300
   Val Asp Leu Ser Thr Ala Ser Thr Thr Gln Glu Gly Glu Leu Ala Ser
   305                 310                 315                 320
   Thr Gln Ser Glu Leu Thr Leu Ser Gln Lys His Trp Leu Ser Asp Arg
                   325                 330                 335
   Thr Tyr Thr Cys Gln Val Thr Tyr Gln Gly His Thr Phe Glu Asp Ser
               340                 345                 350
   Thr Lys Lys Ser Ala Cys Pro Pro Cys Ser Gly Ala Pro Glu Leu Leu
   355 360 365
   Gly Gly Pro Ser Val Phe Leu Phe Pro Pro Lys Pro Lys Asp Thr Leu
   370 375 380
   Met Ile Ser Arg Thr Pro Glu Val Thr Cys Val Val Val Asp Val Ser
   385 390 395 400
   His Glu Asp Pro Glu Val Lys Phe Asn Trp Tyr Val Asp Gly Val Glu
   405 410 415
   Val His Asn Ala Lys Thr Lys Pro Arg Glu Glu Gln Tyr Asn Ser Thr
   420 425 430
   Tyr Arg Val Val Ser Val Leu Thr Val Leu His Gln Asp Trp Leu Asn
   435 440 445
   Gly Lys Glu Tyr Lys Cys Lys Val Ser Asn Lys Ala Leu Pro Ala Pro
   450 455 460
   Ile Glu Lys Thr Ile Ser Lys Ala Lys Gly Gln Pro Arg Glu Pro Gln
   465 470 475 480
   Val Tyr Thr Leu Pro Pro Ser Arg Asp Glu Leu Thr Lys Asn Gln Val
   485 490 495
   Ser Leu Thr Cys Leu Val Lys Gly Phe Tyr Pro Ser Asp Ile Ala Val
   500 505 510
   Glu Trp Glu Ser Asn Gly Gln Pro Glu Asn Asn Tyr Lys Thr Thr Pro
   515 520 525
   Pro Val Leu Asp Ser Asp Gly Ser Phe Phe Leu Tyr Ser Lys Leu Thr
   530 535 540
   Val Asp Lys Ser Arg Trp Gln Gln Gly Asn Val Phe Ser Cys Ser Val
   545 550 555 560
   Met His Glu Ala Leu His Asn His Tyr Thr Gln Lys Ser Leu Ser Leu
   565 570 575
   Ser Pro Gly Lys Ser Arg Ala Ser Thr Lys Gly Pro Ser Val Phe Pro
   580 585 590
   Leu Ala Pro Ser Ser Lys Ser Thr Ser Gly Gly Thr Ala Ala Leu Gly
   595 600 605
   Cys Leu Val Lys Asp Tyr Phe Pro Glu Pro Val Thr Val Ser Trp Asn
   610 615 620
   Ser Gly Ala Leu Thr Ser Gly Val His Thr Phe Pro Ala Val Leu Gln
   625 630 635 640
   Ser Ser Gly Leu Tyr Ser Leu Ser Ser Val Val Thr Val Pro Ser Ser
   645 650 655
   Ser Leu Gly Thr Gln Thr Tyr Ile Cys Asn Val Asn His Lys Pro Ser
   660 665 670
   Asn Thr Lys Val Asp Lys Lys Val
   675 680
<210> 69
   <211> 687
   <212> PRT
   <213> Artificial Sequence
<220>
   <223> TSC016 mature protein comprising : HD37 (anti-CD19) scFv, altered human IgM CH2 hinge, human IgG1 CH2, human IgG1 CH3, and human CH1
<400> 69
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Ile Ala Glu Leu
                   245                 250                 255
   Pro Pro Lys Val Ser Val Phe Val Pro Pro Arg Asp Gly Phe Phe Gly
               260                 265                 270
   Asn Pro Arg Lys Ser Lys Leu Ile Cys Gln Ala Thr Gly Phe Ser Pro
           275                 280                 285
   Arg Gln Ile Gln Val Ser Trp Leu Arg Glu Gly Lys Gln Val Gly Ser
       290                 295                 300
   Gly Val Thr Thr Asp Gln Val Gln Ala Glu Ala Lys Glu Ser Gly Pro
   305                 310                 315                 320
   Thr Thr Tyr Lys Val Thr Ser Thr Leu Thr Ile Lys Glu Ser Asp Trp
                   325                 330                 335
   Leu Ser Gln Ser Met Phe Thr Cys Arg Val Asp His Arg Gly Leu Thr
               340                 345                 350
   Phe Gln Gln Asn Ala Ser Ser Met Ser Val Pro Cys Pro Pro Cys Pro
           355                 360                 365
   Ser Gly Ala Pro Glu Leu Leu Gly Gly Pro Ser Val Phe Leu Phe Pro
       370                 375                 380
   Pro Lys Pro Lys Asp Thr Leu Met Ile Ser Arg Thr Pro Glu Val Thr
   385                 390                 395                 400
   Cys Val Val Val Asp Val Ser His Glu Asp Pro Glu Val Lys Phe Asn
                   405                 410                 415
   Trp Tyr Val Asp Gly Val Glu Val His Asn Ala Lys Thr Lys Pro Arg
               420                 425                 430
   Glu Glu Gln Tyr Asn Ser Thr Tyr Arg Val Val Ser Val Leu Thr Val
           435                 440                 445
   Leu His Gln Asp Trp Leu Asn Gly Lys Glu Tyr Lys Cys Lys Val Ser
       450                 455                 460
   Asn Lys Ala Leu Pro Ala Pro Ile Glu Lys Thr Ile Ser Lys Ala Lys
   465                 470                 475                 480
   Gly Gln Pro Arg Glu Pro Gln Val Tyr Thr Leu Pro Pro Ser Arg Asp
                   485                 490                 495
   Glu Leu Thr Lys Asn Gln Val Ser Leu Thr Cys Leu Val Lys Gly Phe
               500                 505                 510
   Tyr Pro Ser Asp Ile Ala Val Glu Trp Glu Ser Asn Gly Gln Pro Glu
           515                 520                 525
   Asn Asn Tyr Lys Thr Thr Pro Pro Val Leu Asp Ser Asp Gly Ser Phe
       530                 535                 540
   Phe Leu Tyr Ser Lys Leu Thr Val Asp Lys Ser Arg Trp Gln Gln Gly
   545                 550                 555                 560
   Asn Val Phe Ser Cys Ser Val Met His Glu Ala Leu His Asn His Tyr
                   565                 570                 575
   Thr Gln Lys Ser Leu Ser Leu Ser Pro Gly Lys Ser Arg Ala Ser Thr
               580                 585                 590
   Lys Gly Pro Ser Val Phe Pro Leu Ala Pro Ser Ser Lys Ser Thr Ser
           595                 600                 605
   Gly Gly Thr Ala Ala Leu Gly Cys Leu Val Lys Asp Tyr Phe Pro Glu
       610                 615                 620
   Pro Val Thr Val Ser Trp Asn Ser Gly Ala Leu Thr Ser Gly Val His
   625                 630                 635                 640
   Thr Phe Pro Ala Val Leu Gln Ser Ser Gly Leu Tyr Ser Leu Ser Ser
                   645                 650                 655
   Val Val Thr Val Pro Ser Ser Ser Leu Gly Thr Gln Thr Tyr Ile Cys
               660                 665                 670
   Asn Val Asn His Lys Pro Ser Asn Thr Lys Val Asp Lys Lys Val
           675                 680                 685
<210> 70
   <211> 575
   <212> PRT
   <213> Artificial Sequence
<220>
   <223> TSC017 mature protein comprising : P2C2 (anti-CD79b) scFv, human IgG1 SCC-P hinge, human IgG1 CH2, human IgG1 CH3 and human CH1
<400> 70
   Asp Ile Val Met Ile Gln Asp Glu Leu Ser Asn Pro Val Ser Ser Gly
    1               5                  10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
               100                 105                 110
   Gly Gly Gly Gly Ser Gly Gly Gly Gly Ser Gly Gly Gly Gly Ser Gln
           115                 120                 125
   Ile Gln Leu Val Gln Ser Gly Pro Glu Leu Lys Lys Pro Gly Glu Thr
       130                 135                 140
   Val Lys Ile Ser Cys Lys Ala Ser Gly Tyr Thr Phe Thr Asp Phe Ser
   145                 150                 155                 160
   Met His Trp Val Arg Gln Ala Pro Gly Lys Gly Leu Lys Trp Met Gly
                   165                 170                 175
   Trp Ile Asn Thr Glu Thr Gly Glu Pro Thr Tyr Ala Asp Asp Phe Arg
               180                 185                 190
   Gly Arg Phe Ala Phe Ser Leu Glu Thr Ser Ala Ser Thr Ala Tyr Leu
           195                 200                 205
   Gln Ile Asn Asn Leu Lys Asn Glu Asp Thr Ala Thr Tyr Phe Cys Thr
       210                 215                 220
   Trp Ser Ala Tyr Trp Gly Gln Gly Thr Leu Val Thr Val Ser Ala Ser
   225                 230                 235                 240
   Ser Glu Pro Lys Ser Ser Asp Lys Thr His Thr Cys Pro Pro Cys Pro
                   245                 250                 255
   Ser Gly Ala Pro Glu Leu Leu Gly Gly Pro Ser Val Phe Leu Phe Pro
               260                 265                 270
   Pro Lys Pro Lys Asp Thr Leu Met Ile Ser Arg Thr Pro Glu Val Thr
           275                 280                 285
   Cys Val Val Val Asp Val Ser His Glu Asp Pro Glu Val Lys Phe Asn
       290                 295                 300
   Trp Tyr Val Asp Gly Val Glu Val His Asn Ala Lys Thr Lys Pro Arg
   305                 310                 315                 320
   Glu Glu Gln Tyr Asn Ser Thr Tyr Arg Val Val Ser Val Leu Thr Val
                   325                 330                 335
   Leu His Gln Asp Trp Leu Asn Gly Lys Glu Tyr Lys Cys Lys Val Ser
               340                 345                 350
   Asn Lys Ala Leu Pro Ala Pro Ile Glu Lys Thr Ile Ser Lys Ala Lys
           355                 360                 365
   Gly Gln Pro Arg Glu Pro Gln Val Tyr Thr Leu Pro Pro Ser Arg Asp
       370                 375                 380
   Glu Leu Thr Lys Asn Gln Val Ser Leu Thr Cys Leu Val Lys Gly Phe
   385                 390                 395                 400
   Tyr Pro Ser Asp Ile Ala Val Glu Trp Glu Ser Asn Gly Gln Pro Glu
                   405                 410                 415
   Asn Asn Tyr Lys Thr Thr Pro Pro Val Leu Asp Ser Asp Gly Ser Phe
               420                 425                 430
   Phe Leu Tyr Ser Lys Leu Thr Val Asp Lys Ser Arg Trp Gln Gln Gly
           435                 440                 445
   Asn Val Phe Ser Cys Ser Val Met His Glu Ala Leu His Asn His Tyr
       450                 455                 460
   Thr Gln Lys Ser Leu Ser Leu Ser Pro Gly Lys Ser Arg Ala Ser Thr
   465                 470                 475                 480
   Lys Gly Pro Ser Val Phe Pro Leu Ala Pro Ser Ser Lys Ser Thr Ser
                   485                 490                 495
   Gly Gly Thr Ala Ala Leu Gly Cys Leu Val Lys Asp Tyr Phe Pro Glu
               500                 505                 510
   Pro Val Thr Val Ser Trp Asn Ser Gly Ala Leu Thr Ser Gly Val His
           515                 520                 525
   Thr Phe Pro Ala Val Leu Gln Ser Ser Gly Leu Tyr Ser Leu Ser Ser
       530                 535                 540
   Val Val Thr Val Pro Ser Ser Ser Leu Gly Thr Gln Thr Tyr Ile Cys
   545                 550                 555                 560
   Asn Val Asn His Lys Pro Ser Asn Thr Lys Val Asp Lys Lys Val
                   565                 570                 575
<210> 71
   <211> 583
   <212> PRT
   <213> Artificial Sequence
<220>
   <223> TSC018 mature protein comprising : M0042 (anti-HLA-DR) scFv, human IgG1 SCC-P hinge, human IgG1 CH2, human IgG1 CH3, and human CH1
<400> 71
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Val
           35                  40                  45
   Tyr Ser Val Ser Ser Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Ala Thr Tyr Tyr Cys Gln Gln Ser Tyr Ser Thr Pro Phe
                   85                  90                  95
   Thr Phe Gly Pro Gly Thr Lys Val Asp Ile Lys Gly Gly Gly Gly Ser
               100                 105                 110
   Gly Gly Gly Gly Ser Gly Gly Gly Gly Ser Gly Gly Gly Gly Ser Glu
           115                 120                 125
   Val Gln Leu Leu Glu Ser Gly Gly Gly Leu Val Gln Pro Gly Gly Ser
       130                 135                 140
   Leu Arg Leu Ser Cys Ala Ala Ser Gly Phe Thr Phe Ser His Tyr Tyr
   145                 150                 155                 160
   Met Ser Trp Val Arg Gln Ala Pro Gly Lys Gly Leu Glu Trp Val Ser
                   165                 170                 175
   Tyr Ile Gly Pro Ser Gly Gly Phe Thr Ala Tyr Ala Asp Ser Val Lys
               180                 185                 190
   Gly Arg Phe Thr Ile Ser Arg Asp Asn Ser Lys Asn Thr Leu Tyr Leu
           195                 200                 205
   Gln Met Asn Ser Leu Arg Ala Glu Asp Thr Ala Val Tyr Tyr Cys Ala
       210                 215                 220
   Arg Asp Gly Trp Val Gly Ser Glu Asn Tyr Ser Gly Met Asp Val Trp
   225                 230                 235                 240
   Gly Gln Gly Thr Thr Val Ser Ser Ser Glu Pro Lys Ser Ser Asp Lys
                   245                 250                 255
   Thr His Thr Cys Pro Pro Cys Pro Ser Gly Ala Pro Glu Leu Leu Gly
               260                 265                 270
   Gly Pro Ser Val Phe Leu Phe Pro Pro Lys Pro Lys Asp Thr Leu Met
           275                 280                 285
   Ile Ser Arg Thr Pro Glu Val Thr Cys Val Val Val Asp Val Ser His
       290                 295                 300
   Glu Asp Pro Glu Val Lys Phe Asn Trp Tyr Val Asp Gly Val Glu Val
   305                 310                 315                 320
   His Asn Ala Lys Thr Lys Pro Arg Glu Glu Gln Tyr Asn Ser Thr Tyr
                   325                 330                 335
   Arg Val Val Ser Val Leu Thr Val Leu His Gln Asp Trp Leu Asn Gly
               340                 345                 350
   Lys Glu Tyr Lys Cys Lys Val Ser Asn Lys Ala Leu Pro Ala Pro Ile
           355                 360                 365
   Glu Lys Thr Ile Ser Lys Ala Lys Gly Gln Pro Arg Glu Pro Gln Val
       370                 375                 380
   Tyr Thr Leu Pro Pro Ser Arg Asp Glu Leu Thr Lys Asn Gln Val Ser
   385                 390                 395                 400
   Leu Thr Cys Leu Val Lys Gly Phe Tyr Pro Ser Asp Ile Ala Val Glu
                   405                 410                 415
   Trp Glu Ser Asn Gly Gln Pro Glu Asn Asn Tyr Lys Thr Thr Pro Pro
               420                 425                 430
   Val Leu Asp Ser Asp Gly Ser Phe Phe Leu Tyr Ser Lys Leu Thr Val
           435                 440                 445
   Asp Lys Ser Arg Trp Gln Gln Gly Asn Val Phe Ser Cys Ser Val Met
       450                 455                 460
   His Glu Ala Leu His Asn His Tyr Thr Gln Lys Ser Leu Ser Leu Ser
   465                 470                 475                 480
   Pro Gly Lys Ser Arg Ala Ser Thr Lys Gly Pro Ser Val Phe Pro Leu
                   485                 490                 495
   Ala Pro Ser Ser Lys Ser Thr Ser Gly Gly Thr Ala Ala Leu Gly Cys
               500                 505                 510
   Leu Val Lys Asp Tyr Phe Pro Glu Pro Val Thr Val Ser Trp Asn Ser
           515                 520                 525
   Gly Ala Leu Thr Ser Gly Val His Thr Phe Pro Ala Val Leu Gln Ser
       530                 535                 540
   Ser Gly Leu Tyr Ser Leu Ser Ser Val Val Thr Val Pro Ser Ser Ser
   545                 550                 555                 560
   Leu Gly Thr Gln Thr Tyr Ile Cys Asn Val Asn His Lys Pro Ser Asn
                   565                 570                 575
   Thr Lys Val Asp Lys Lys Val
               580
<210> 72
   <211> 590
   <212> PRT
   <213> Artificial Sequence
<220>
   <223> TSC019 mature protein comprising : G19-4 (anti-CD3) scFv, human IgG1 SCC-P hinge, human CH2, human CH3, and human Ck (YAE)
<400> 72
   Glu Val Gln Leu Gln Gln Ser Gly Pro Glu Leu Val Lys Pro Gly Ala
   1 5 10 15
   Ser Met Lys Ile Ser Cys Lys Ala Ser Gly Tyr Ser Phe Thr Gly Tyr
   20 25 30
   Ile Val Asn Trp Leu Lys Gln Ser His Gly Lys Asn Leu Glu Trp Ile
   35 40 45
   Gly Leu Ile Asn Pro Tyr Lys Gly Leu Thr Thr Tyr Asn Gln Lys Phe
   50 55 60
   Lys Gly Lys Ala Thr Leu Thr Val Asp Lys Ser Ser Ser Thr Ala Tyr
   65 70 75 80
   Met Glu Leu Leu Ser Leu Thr Ser Glu Asp Ser Ala Val Tyr Tyr Cys
   85 90 95
   Ala Arg Ser Gly Tyr Tyr Gly Asp Ser Asp Trp Tyr Phe Asp Val Trp
   100 105 110
   Gly Ala Gly Thr Thr Val Thr Val Ser Ser Gly Gly Gly Gly Ser Gly
   115 120 125
   Gly Gly Gly Ser Gly Gly Gly Gly Ser Ala Gln Asp Ile Gln Met Thr
   130 135 140
   Gln Thr Thr Ser Ser Leu Ser Ala Ser Leu Gly Asp Arg Val Thr Ile
   145 150 155 160
   Ser Cys Arg Ala Ser Gln Asp Ile Arg Asn Tyr Leu Asn Trp Tyr Gln
   165 170 175
   Gln Lys Pro Asp Gly Thr Val Lys Leu Leu Ile Tyr Tyr Thr Ser Arg
   180 185 190
   Leu His Ser Gly Val Pro Ser Arg Phe Ser Gly Ser Gly Ser Gly Thr
   195 200 205
   Asp Tyr Ser Leu Thr Ile Ala Asn Leu Gln Pro Glu Asp Ile Ala Thr
   210 215 220
   Tyr Phe Cys Gln Gln Gly Asn Thr Leu Pro Trp Thr Phe Gly Gly Gly
   225 230 235 240
   Thr Lys Leu Val Thr Lys Arg Ser Ser Glu Pro Lys Ser Ser Asp Lys
   245 250 255
   Thr His Thr Cys Pro Pro Cys Pro Ser Gly Ala Pro Glu Leu Leu Gly
   260 265 270
   Gly Pro Ser Val Phe Leu Phe Pro Pro Lys Pro Lys Asp Thr Leu Met
   275 280 285
   Ile Ser Arg Thr Pro Glu Val Thr Cys Val Val Val Asp Val Ser His
   290 295 300
   Glu Asp Pro Glu Val Lys Phe Asn Trp Tyr Val Asp Gly Val Glu Val
   305 310 315 320
   His Asn Ala Lys Thr Lys Pro Arg Glu Glu Gln Tyr Asn Ser Thr Tyr
   325 330 335
   Arg Val Val Ser Val Leu Thr Val Leu His Gln Asp Trp Leu Asn Gly
   340 345 350
   Lys Glu Tyr Lys Cys Lys Val Ser Asn Lys Ala Leu Pro Ala Pro Ile
   355 360 365
   Glu Lys Thr Ile Ser Lys Ala Lys Gly Gln Pro Arg Glu Pro Gln Val
   370 375 380
   Tyr Thr Leu Pro Pro Ser Arg Asp Glu Leu Thr Lys Asn Gln Val Ser
   385 390 395 400
   Leu Thr Cys Leu Val Lys Gly Phe Tyr Pro Ser Asp Ile Ala Val Glu
   405 410 415
   Trp Glu Ser Asn Gly Gln Pro Glu Asn Asn Tyr Lys Thr Thr Pro Pro
   420 425 430
   Val Leu Asp Ser Asp Gly Ser Phe Phe Leu Tyr Ser Lys Leu Thr Val
   435 440 445
   Asp Lys Ser Arg Trp Gln Gln Gly Asn Val Phe Ser Cys Ser Val Met
   450 455 460
   His Glu Ala Leu His Asn His Tyr Thr Gln Lys Ser Leu Ser Leu Ser
   465 470 475 480
   Pro Gly Lys Ser Arg Thr Val Ala Ala Pro Ser Val Phe Ile Phe Pro
   485 490 495
   Pro Ser Asp Glu Gln Leu Lys Ser Gly Thr Ala Ser Val Val Cys Leu
   500 505 510
   Leu Asn Tyr Phe Tyr Pro Arg Glu Ala Lys Val Gln Trp Lys Val Asp
           515                 520                 525
   Asn Ala Leu Gln Ser Gly Asn Ser Gln Glu Ser Ala Thr Glu Gln Asp
       530                 535                 540
   Ser Lys Asp Ser Thr Tyr Ser Leu Ser Ser Glu Leu Thr Leu Ser Lys
   545                 550                 555                 560
   Ala Asp Tyr Glu Lys His Lys Val Tyr Ala Cys Glu Val Thr His Gln
                   565                 570                 575
   Gly Leu Ser Ser Pro Val Thr Lys Ser Phe Asn Arg Gly Glu
               580                 585                 590
<210> 73
   <211> 2292
   <212> DNA
   <213> Artificial Sequence
<220>
   <223> 4C04 (anti-RON) x G19-4 (anti-CD3) Scorpion DNA sequence (SO266)
<400> 73
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gcgagcccaa atcttctgac aaaactcaca catgcccacc gtgcccagca 840
   cctgaactcc tgggtggacc gtcagtcttc ctcttccccc caaaacccaa ggacaccctc 900
   atgatctccc ggacccctga ggtcacatgc gtggtggtgg acgtgagcca cgaagaccct 960
   gaggtcaagt tcaactggta cgtggacggc gtggaggtgc ataatgccaa gacaaagccg 1020
   cgggaggagc agtacaacag cacgtaccgt gtggtcagcg tcctcaccgt cctgcaccag 1080
   gactggctga atggcaagga gtacaagtgc aaggtctcca acaaagccct cccagccccc 1140
   atcgagaaaa ccatctccaa agccaaaggg cagccccgag aaccacaggt gtacaccctg 1200
   cccccatccc gggatgagct gaccaagaac caggtcagcc tgacctgcct ggtcaaaggc 1260
   ttctatccaa gcgacatcgc cgtggagtgg gagagcaatg ggcagccgga gaacaactac 1320
   aagaccacgc ctcccgtgct ggactccgac ggctccttct tcctctacag caagctcacc 1380
   gtggacaaga gcaggtggca gcaggggaac gtcttctcat gctccgtgat gcatgaggct 1440
   ctgcacaacc actacacgca gaagagcctc tccctgtctc cgggtcagag gcacaacaat 1500
   tcttccctga atacaggaac tcagatggca ggtcattctc cgaattctga ggtccagctg 1560
   caacagtctg gacctgaact ggtgaagcct ggagcttcaa tgaagatttc ctgcaaggcc 1620
   tctggttact cattcactgg ctacatcgtg aactggctga agcagagcca tggaaagaac 1680
   cttgagtgga ttggacttat taatccatac aaaggtctta ctacctacaa ccagaaattc 1740
   aagggcaagg ccacattaac tgtagacaag tcatccagca cagcctacat ggagctcctc 1800
   agtctgacat ctgaagactc tgcagtctat tactgtgcaa gatctgggta ctatggtgac 1860
   tcggactggt acttcgatgt ctggggcgca gggaccacgg tcaccgtctc aagcggtggc 1920
   ggagggtctg ggggtggcgg atccggaggt ggtggctctg cacaagacat ccagatgaca 1980
   cagactacat cctccctgtc tgcctctctg ggagacagag tcaccatcag ttgcagggca 2040
   agtcaggaca ttcgcaatta tttaaactgg tatcagcaga aaccagatgg aactgttaaa 2100
   ctcctgatct actacacatc aagattacac tcaggagtcc catcaaggtt cagtggcagt 2160
   gggtctggaa cagattattc tctcaccatt gccaacctgc aaccagaaga tattgccact 2220
   tacttttgcc aacagggtaa tacgcttccg tggacgttcg gtggaggcac caaactggta 2280
   accaaacgat aa                                                     2292
<210> 74
   <211> 743
   <212> PRT
   <213> Artificial Sequence
<220>
   <223> 4C04 (anti-RON) x G19-4 (anti-CD3) Scorpion mature protein sequence (SO266)
<400> 74
   Asp Ile Gln Met Thr Gln Ser Pro Ala Ser Gln Ser Ala Ser Leu Gly
    1               5                   10                  15
   Glu Ser Val Thr Ile Thr Cys Leu Ala Ser Gln Thr Ile Gly Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Ser Leu Ala Asp Gly Val Pro Ser Arg Phe Ser Ala
       50                  55                  60
   Ser Gly Ser Gly Thr Lys Phe Ser Phe Lys Ile Asp Ser Leu Gln Ser
   65                  70                  75                  80
   Glu Asp Phe Val Ser Tyr Tyr Cys Gln Gln Leu Tyr Asn Thr Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Ile Gln Leu Gln Gln
           115                 120                 125
   Ser Gly Pro Glu Leu Val Lys Pro Gly Thr Ser Val Lys Ile Ser Cys
       130                 135                 140
   Lys Ala Ser Asp Tyr Thr Leu Thr Asp Phe Tyr Met Asn Trp Val Arg
   145                 150                 155                 160
   Gln Lys Pro Gly Gln Gly Leu Glu Trp Ile Gly Arg Ile Tyr Pro Gly
                   165                 170                 175
   Thr Asp Lys Thr Arg Tyr Asn Glu Lys Phe Arg Asp Lys Ala Ile Leu
               180                 185                 190
   Thr Val Asp Thr Ser Ser Ser Thr Ala Tyr Met Gln Leu Ser Ser Leu
           195                 200                 205
   Thr Ser Glu Asp Thr Ala Val Tyr Phe Cys Ala Arg Ser Ala Tyr Tyr
       210                 215                 220
   Gly Asn Tyr Val Ala Met Asp Tyr Trp Gly Gln Gly Thr Ser Val Thr
   225                 230                 235                 240
   Val Ser Ser Ser Glu Pro Lys Ser Ser Asp Lys Thr His Thr Cys Pro
                   245                 250                 255
   Pro Cys Pro Ala Pro Glu Leu Leu Gly Gly Pro Ser Val Phe Leu Phe
               260                 265                 270
   Pro Pro Lys Pro Lys Asp Thr Leu Met Ile Ser Arg Thr Pro Glu Val
           275                 280                 285
   Thr Cys Val Val Val Asp Val Ser His Glu Asp Pro Glu Val Lys Phe
       290                 295                 300
   Asn Trp Tyr Val Asp Gly Val Glu Val His Asn Ala Lys Thr Lys Pro
   305                 310                 315                 320
   Arg Glu Glu Gln Tyr Asn Ser Thr Tyr Arg Val Val Ser Val Leu Thr
                   325                 330                 335
   Val Leu His Gln Asp Trp Leu Asn Gly Lys Glu Tyr Lys Cys Lys Val
               340                 345                 350
   Ser Asn Lys Ala Leu Pro Ala Pro Ile Glu Lys Thr Ile Ser Lys Ala
           355                 360                 365
   Lys Gly Gln Pro Arg Glu Pro Gln Val Tyr Thr Leu Pro Pro Ser Arg
       370                 375                 380
   Asp Glu Leu Thr Lys Asn Gln Val Ser Leu Thr Cys Leu Val Lys Gly
   385                 390                 395                 400
   Phe Tyr Pro Ser Asp Ile Ala Val Glu Trp Glu Ser Asn Gly Gln Pro
                   405                 410                 415
   Glu Asn Asn Tyr Lys Thr Thr Pro Pro Val Leu Asp Ser Asp Gly Ser
               420                 425                 430
   Phe Phe Leu Tyr Ser Lys Leu Thr Val Asp Lys Ser Arg Trp Gln Gln
           435                 440                 445
   Gly Asn Val Phe Ser Cys Ser Val Met His Glu Ala Leu His Asn His
       450                 455                 460
   Tyr Thr Gln Lys Ser Leu Ser Leu Ser Pro Gly Gln Arg His Asn Asn
   465                 470                 475                 480
   Ser Ser Leu Asn Thr Gly Thr Gln Met Ala Gly His Ser Pro Asn Ser
                   485                 490                 495
   Glu Val Gln Leu Gln Gln Ser Gly Pro Glu Leu Val Lys Pro Gly Ala
               500                 505                 510
   Ser Met Lys Ile Ser Cys Lys Ala Ser Gly Tyr Ser Phe Thr Gly Tyr
           515                 520                 525
   Ile Val Asn Trp Leu Lys Gln Ser His Gly Lys Asn Leu Glu Trp Ile
       530                 535                 540
   Gly Leu Ile Asn Pro Tyr Lys Gly Leu Thr Thr Tyr Asn Gln Lys Phe
   545                 550                 555                 560
   Lys Gly Lys Ala Thr Leu Thr Val Asp Lys Ser Ser Ser Thr Ala Tyr
                   565                 570                 575
   Met Glu Leu Leu Ser Leu Thr Ser Glu Asp Ser Ala Val Tyr Tyr Cys
               580                 585                 590
   Ala Arg Ser Gly Tyr Tyr Gly Asp Ser Asp Trp Tyr Phe Asp Val Trp
           595                 600                 605
   Gly Ala Gly Thr Thr Val Thr Val Ser Ser Gly Gly Gly Gly Ser Gly
       610                 615                 620
   Gly Gly Gly Ser Gly Gly Gly Gly Ser Ala Gln Asp Ile Gln Met Thr
   625                 630                 635                 640
   Gln Thr Thr Ser Ser Leu Ser Ala Ser Leu Gly Asp Arg Val Thr Ile
                   645                 650                 655
   Ser Cys Arg Ala Ser Gln Asp Ile Arg Asn Tyr Leu Asn Trp Tyr Gln
               660                 665                 670
   Gln Lys Pro Asp Gly Thr Val Lys Leu Leu Ile Tyr Tyr Thr Ser Arg
           675                 680                 685
   Leu His Ser Gly Val Pro Ser Arg Phe Ser Gly Ser Gly Ser Gly Thr
       690                 695                 700
   Asp Tyr Ser Leu Thr Ile Ala Asn Leu Gln Pro Glu Asp Ile Ala Thr
   705                 710                 715                 720
   Tyr Phe Cys Gln Gln Gly Asn Thr Leu Pro Trp Thr Phe Gly Gly Gly
                   725                 730                 735
   Thr Lys Leu Val Thr Lys Arg
               740
<210> 75
<400> 75
   000
<210> 76
<400> 76
   000
<210> 77
   <211> 510
   <212> PRT
   <213> Artificial Sequence
<220>
   <223> Mutated M0039
<220>
   <221> SIGNAL
   <222> (1)...(22)
<400> 77
   Met Asp Phe Gln Val Gln Ile Phe Ser Phe Leu Leu Ile Ser Ala Ser
           -20                 -15                 -10
   Val Ile Met Ser Arg Gly Val Gln Val Gln Leu Lys Glu Ser Gly Pro
       -5                  1               5                   10
   Gly Leu Val Ala Pro Ser Gln Ser Leu Ser Ile Thr Cys Thr Val Ser
                   15                  20                  25
   Gly Phe Ser Leu Thr Gly Tyr Gly Val Asn Trp Val Arg Gln Pro Pro
               30                  35                  40
   Gly Lys Gly Leu Glu Trp Leu Gly Met Ile Trp Gly Asp Gly Ser Thr
           45                  50                  55
   Asp Tyr Asn Ser Ala Leu Lys Ser Arg Leu Ser Ile Thr Lys Asp Asn
       60                  65                  70
   Ser Lys Ser Gln Val Phe Leu Lys Met Asn Ser Leu Gln Thr Asp Asp
   75                  80                  85                  90
   Thr Ala Arg Tyr Tyr Cys Ala Arg Asp Gly Tyr Ser Asn Phe His Tyr
                   95                  100                 105
   Tyr Val Met Asp Tyr Trp Gly Gln Gly Thr Ser Val Thr Val Ser Ser
               110                 115                 120
   Gly Gly Gly Gly Ser Gly Gly Gly Gly Ser Gly Gly Gly Gly Ser Gly
           125                 130                 135
   Gly Gly Gly Ser Asp Ile Val Leu Thr Gln Ser Pro Ala Ser Leu Ala
       140                 145                 150
   Val Ser Leu Gly Gln Arg Ala Thr Ile Ser Cys Arg Ala Ser Glu Ser
   155                 160                 165                 170
   Val Glu Tyr Tyr Val Thr Ser Leu Met Gln Trp Tyr Gln Gln Lys Pro
                   175                 180                 185
   Gly Gln Pro Pro Lys Leu Leu Ile Ser Ala Ala Ser Asn Val Glu Ser
                   190                 195                 200
   Gly Val Pro Ala Arg Phe Ser Gly Ser Gly Ser Gly Thr Asp Phe Ser
           205                 210                 215
   Leu Asn Ile His Pro Val Glu Glu Asp Asp Ile Ala Met Tyr Phe Cys
       220                 225                 230
   Gln Gln Ser Arg Lys Val Pro Trp Thr Phe Gly Gly Gly Thr Lys Leu
   235                 240                 245                 250
   Glu Ile Lys Arg Asp Gln Glu Pro Lys Ser Ser Asp Lys Thr His Thr
                   255                 260                 265
   Ser Pro Pro Ser Pro Ala Pro Glu Leu Leu Gly Gly Pro Ser Val Phe
               270                 275                 280
   Leu Phe Pro Pro Lys Pro Lys Asp Thr Leu Met Ile Ser Arg Thr Pro
           285                 290                 295
   Glu Val Thr Cys Val Val Val Asp Val Ser His Glu Asp Pro Glu Val
       300                 305                 310
   Lys Phe Asn Trp Tyr Val Asp Gly Val Glu Val His Asn Ala Lys Thr
   315                 320                 325                 330
   Lys Pro Arg Glu Glu Gln Tyr Asn Ser Thr Tyr Arg Val Val Ser Val
                   335                 340                 345
   Leu Thr Val Leu His Gln Asp Trp Leu Asn Gly Lys Glu Tyr Lys Cys
                   350                 355                 360
   Lys Val Ser Asn Lys Ala Leu Pro Ala Pro Ile Glu Lys Thr Ile Ser
           365                 370                 375
   Lys Ala Lys Gly Gln Pro Arg Glu Pro Gln Val Tyr Thr Leu Pro Pro
       380                 385                 390
   Ser Arg Asp Glu Leu Thr Lys Asn Gln Val Ser Leu Thr Cys Leu Val
   395                 400                 405                 410
   Lys Gly Phe Tyr Pro Ser Asp Ile Ala Val Glu Trp Glu Ser Asn Gly
                   415                 420                 425
   Gln Pro Glu Asn Asn Tyr Lys Thr Thr Pro Pro Val Leu Asp Ser Asp
                   430                 435                 440
   Gly Ser Phe Phe Leu Tyr Ser Lys Leu Thr Val Asp Lys Ser Arg Trp
           445                 450                 455
   Gln Gln Gly Asn Val Phe Ser Cys Ser Val Met His Glu Ala Leu His
       460                 465                 470
   Asn His Tyr Thr Gln Lys Ser Leu Ser Leu Ser Pro Gly Lys
   475                 480                 485
<210> 78
   <211> 20
   <212> PRT
   <213> Artificial Sequence
<220>
   <223> H68 linker (scorpion linker derived from NKG2D)
<400> 78
   Asn Ser Leu Phe Asn Gln Glu Val Gln Ile Pro Leu Thr Glu Ser Tyr
    1               5                  10                  15
   Ser Pro Asn Ser
               20
<210> 79
   <211> 366
   <212> DNA
   <213> Artificial Sequence
<220>
   <223> VH of A2
<400> 79
   gaagttcaat tgttagagtc tggtggcggt cttgttcagc ctggtggttc tttacgtctt 60
   tcttgcgctg cttccggatt cactttctct ttttaccaga tgcattgggt tcgccaagct 120
   cctggtaaag gtttggagtg ggtttctggt atctatcctt ctggtggcta tactaagtat 180
   gctgactccg ttaaaggtcg cttcactatc tctagagaca actctaagaa tactctctac 240
   ttgcagatga acagcttaag ggctgaggac actgcagtct actattgtgc gagagatcta 300
   gggtacggca gtagctggta ctactttgac tactggggcc agggaaccct ggtcaccgtc 360
   tcaagc                                                            366
<210> 80
   <211> 122
   <212> PRT
   <213> Artificial Sequence
<220>
   <223> VH of A2
<400> 80
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Phe Tyr
               20                  25                  30
   Gln Met His Trp Val Arg Gln Ala Pro Gly Lys Gly Leu Glu Trp Val
           35                  40                  45
   Ser Gly Ile Tyr Pro Ser Gly Gly Tyr Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Gly Tyr Gly Ser Ser Trp Tyr Tyr Phe Asp Tyr Trp
               100                 105                 110
   Gly Gln Gly Thr Leu Val Thr Val Ser Ser
           115                 120
<210> 81
<400> 81
   000
<210> 82
<400> 82
   000
<210> 83
   <211> 330
   <212> DNA
   <213> Artificial Sequence
<220>
   <223> VL of A2
<400> 83
   cagagcgctt tgactcagcc tgcctccgtg tctgggtctc ctggacagtc gatcaccatc 60
   tcctgcagtg gaaccgacag tgacgttggt ggttataacc atgtctcctg gtaccaacaa 120
   cacccaggca aggcccccaa actcataatt tatgatgtcg atcatcggcc ctcagggatc 180
   tctaaccgtt tctctggctc caagtctggc aacacggcct ccctgaccat ctctgggctc 240
   caggctgagg acgaggctga ttattactgc agctcatata gaagcggtag cacttatgtc 300
   ttcggaactg ggaccaaggt caccgtccta                                  330
<210> 84
   <211> 110
   <212> PRT
   <213> Artificial Sequence
<220>
   <223> VL of A2
<400> 84
   Gln Ser Ala Leu Thr Gln Pro Ala Ser Val Ser Gly Ser Pro Gly Gln
    1               5                   10                  15
   Ser Ile Thr Ile Ser Cys Ser Gly Thr Asp Ser Asp Val Gly Gly Tyr
               20                  25                  30
   Asn His Val Ser Trp Tyr Gln Gln His Pro Gly Lys Ala Pro Lys Leu
           35                  40                  45
   Ile Ile Tyr Asp Val Asp His Arg Pro Ser Gly Ile Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Arg Ser Gly
                   85                  90                  95
   Ser Thr Tyr Val Phe Gly Thr Gly Thr Lys Val Thr Val Leu
               100                 105                 110
<210> 85
   <211> 741
   <212> DNA
   <213> Artificial Sequence
<220>
   <223> A2 scFv
<400> 85
   gaagttcaat tgttagagtc tggtggcggt cttgttcagc ctggtggttc tttacgtctt 60
   tcttgcgctg cttccggatt cactttctct ttttaccaga tgcattgggt tcgccaagct 120
   cctggtaaag gtttggagtg ggtttctggt atctatcctt ctggtggcta tactaagtat 180
   gctgactccg ttaaaggtcg cttcactatc tctagagaca actctaagaa tactctctac 240
   ttgcagatga acagcttaag ggctgaggac actgcagtct actattgtgc gagagatcta 300
   gggtacggca gtagctggta ctactttgac tactggggcc agggaaccct ggtcaccgtc 360
   tcaagcggtg gcggcggttc ggggggtggc ggaagtggag gtggagggag tcagagcgct 420
   ttgactcagc ctgcctccgt gtctgggtct cctggacagt cgatcaccat ctcctgcagt 480
   ggaaccgaca gtgacgttgg tggttataac catgtctcct ggtaccaaca acacccaggc 540
   aaggccccca aactcataat ttatgatgtc gatcatcggc cctcagggat ctctaaccgt 600
   ttctctggct ccaagtctgg caacacggcc tccctgacca tctctgggct ccaggctgag 660
   gacgaggctg attattactg cagctcatat agaagcggta gcacttatgt cttcggaact 720
   gggaccaagg tcaccgtcct a                                           741
<210> 86
   <211> 247
   <212> PRT
   <213> Artificial Sequence
<220>
   <223> A2 scFv
<400> 86
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Phe Tyr
               20                  25                  30
   Gln Met His Trp Val Arg Gln Ala Pro Gly Lys Gly Leu Glu Trp Val
           35                  40                  45
   Ser Gly Ile Tyr Pro Ser Gly Gly Tyr Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Gly Tyr Gly Ser Ser Trp Tyr Tyr Phe Asp Tyr Trp
               100                 105                 110
   Gly Gln Gly Thr Leu Val Thr Val Ser Ser Gly Gly Gly Gly Ser Gly
           115                 120                 125
   Gly Gly Gly Ser Gly Gly Gly Gly Ser Gln Ser Ala Leu Thr Gln Pro
       130                 135                 140
   Ala Ser Val Ser Gly Ser Pro Gly Gln Ser Ile Thr Ile Ser Cys Ser
   145                 150                 155                 160
   Gly Thr Asp Ser Asp Val Gly Gly Tyr Asn His Val Ser Trp Tyr Gln
                   165                 170                 175
   Gln His Pro Gly Lys Ala Pro Lys Leu Ile Ile Tyr Asp Val Asp His
               180                 185                 190
   Arg Pro Ser Gly Ile Ser Asn Arg Phe Ser Gly Ser Lys Ser Gly Asn
           195                 200                 205
   Thr Ala Ser Leu Thr Ile Ser Gly Leu Gln Ala Glu Asp Glu Ala Asp
       210                 215                 220
   Tyr Tyr Cys Ser Ser Tyr Arg Ser Gly Ser Thr Tyr Val Phe Gly Thr
   225                 230                 235                 240
   Gly Thr Lys Val Thr Val Leu
                   245
<210> 87
   <211> 603
   <212> DNA
   <213> Artificial Sequence
<220>
   <223> PLD ECD
<400> 87
   ttattcacag tgacagtccc taaggaactg tacataatag agcatggcag caatgtgacc 60
   ctggaatgca actttgacac tggaagtcat gtgaaccttg gagcaataac agccagtttg 120
   caaaaggtgg aaaatgatac atccccacac cgtgaaagag ccactttgct ggaggagcag 180
   ctgcccttag ggaaggcctc gttccacata cctcaagtcc aagtgaggga cgaaggacag 240
   taccaatgca taatcatcta tggggtcgcc tgggactaca agtacctgac tctgaaagtc 300
   aaagcttcct acaggaaaat aaacactcac atcctaaagg ttccagaaac agatgaggta 360
   gagctcacct gccaggctac aggttatcct ctggcagaag tatcctggcc aaacgtcagc 420
   gttcctgcca acaccagcca ctccaggacc cctgaaggcc tctaccaggt caccagtgtt 480
   ctgcgcctaa agccaccccc tggcagaaac ttcagctgtg tgttctggaa tactcacgtg 540
   agggaactta ctttggccag cattgacctt caaagtcaga tggaacccag gacccatcca 600
   acc                                                               603
<210> 88
   <211> 201
   <212> PRT
   <213> Artificial Sequence
<220>
   <223> PLD ECD
<400> 88
   Leu Phe Thr Val Thr Val Pro Lys Glu Leu Tyr Ile Ile Glu His Gly
    1               5                  10                  15
   Ser Asn Val Thr Leu Glu Cys Asn Phe Asp Thr Gly Ser His Val Asn
               20                  25                  30
   Leu Gly Ala Ile Thr Ala Ser Leu Gln Lys Val Glu Asn Asp Thr Ser
           35                  40                  45
   Pro His Arg Glu Arg Ala Thr Leu Leu Glu Glu Gln Leu Pro Leu Gly
       50                  55                  60
   Lys Ala Ser Phe His Ile Pro Gln Val Gln Val Arg Asp Glu Gly Gln
   65                  70                  75                  80
   Tyr Gln Cys Ile Ile Ile Tyr Gly Val Ala Trp Asp Tyr Lys Tyr Leu
                   85                  90                  95
   Thr Leu Lys Val Lys Ala Ser Tyr Arg Lys Ile Asn Thr His Ile Leu
               100                 105                 110
   Lys Val Pro Glu Thr Asp Glu Val Glu Leu Thr Cys Gln Ala Thr Gly
           115                 120                 125
   Tyr Pro Leu Ala Glu Val Ser Trp Pro Asn Val Ser Val Pro Ala Asn
       130                 135                 140
   Thr Ser His Ser Arg Thr Pro Glu Gly Leu Tyr Gln Val Thr Ser Val
   145                 150                 155                 160
   Leu Arg Leu Lys Pro Pro Pro Gly Arg Asn Phe Ser Cys Val Phe Trp
                   165                 170                 175
   Asn Thr His Val Arg Glu Leu Thr Leu Ala Ser Ile Asp Leu Gln Ser
               180                 185                 190
   Gln Met Glu Pro Arg Thr His Pro Thr
           195                 200
<210> 89
   <211> 498
   <212> DNA
   <213> Artificial Sequence
<220>
   <223> monoIL10
<400> 89
   agcccaggcc agggcaccca gtctgagaac agctgcaccc acttcccagg caacctgcct 60
   aacatgcttc gagatctccg agatgccttc agcagagtga agactttctt tcaaatgaag 120
   gatcagctgg acaacttgtt gttaaaggag tccttgctgg aggactttaa gggttacctg 180
   ggttgccaag ccttgtctga gatgatccag ttttacctgg aggaggtgat gccccaagct 240
   gagaaccaag acccagacat caaggcgcat gtgaactccc tgggggagaa cctgaagacc 300
   ctcaggctga ggctacggcg ctgtcatcga tttcttccct gtgaaaacgg tggtggatcc 360
   ggcggtaaga gcaaggccgt ggagcaggtg aagaatgcct ttaataagct ccaagagaaa 420
   ggcatctaca aagccatgag tgagtttgac atcttcatca actacataga agcctacatt 480
   acaatgaaga tacgaaac                                               498
<210> 90
   <211> 166
   <212> PRT
   <213> Artificial Sequence
<220>
   <223> monoIL10
<400> 90
   Ser Pro Gly Gln Gly Thr Gln Ser Glu Asn Ser Cys Thr His Phe Pro
    1               5                  10                  15
   Gly Asn Leu Pro Asn Met Leu Arg Asp Leu Arg Asp Ala Phe Ser Arg
               20                  25                  30
   Val Lys Thr Phe Phe Gln Met Lys Asp Gln Leu Asp Asn Leu Leu Leu
           35                  40                  45
   Lys Glu Ser Leu Leu Glu Asp Phe Lys Gly Tyr Leu Gly Cys Gln Ala
       50                  55                  60
   Leu Ser Glu Met Ile Gln Phe Tyr Leu Glu Glu Val Met Pro Gln Ala
   65                  70                  75                  80
   Glu Asn Gln Asp Pro Asp Ile Lys Ala His Val Asn Ser Leu Gly Glu
                   85                  90                  95
   Asn Leu Lys Thr Leu Arg Leu Arg Leu Arg Arg Cys His Arg Phe Leu
               100                 105                 110
   Pro Cys Glu Asn Gly Gly Gly Ser Gly Gly Lys Ser Lys Ala Val Glu
           115                 120                 125
   Gln Val Lys Asn Ala Phe Asn Lys Leu Gln Glu Lys Gly Ile Tyr Lys
       130                 135                 140
   Ala Met Ser Glu Phe Asp Ile Phe Ile Asn Tyr Ile Glu Ala Tyr Ile
   145                 150                 155                 160
   Thr Met Lys Ile Arg Asn
                   165
<210> 91
   <211> 348
   <212> DNA
   <213> Artificial Sequence
<220>
   <223> VH of 3D1
<400> 91
   caggtccagc tgcagcagtc tgggcctgag ctggtgaggc ctggggaatc agtgaagatt 60
   tcctgcaagg gttccggcta cacattcact gattatgcta tacagtgggt gaagcagagt 120
   catgcaaaga gtctagagtg gattggagtt attaatattt actatgataa tacaaactac 180
   aaccagaagt ttaagggcaa ggccacaatg actgtagaca aatcctccag cacagcctat 240
   atggaacttg ccagattgac atctgaggat tctgccatct attactgtgc aagagcggcc 300
   tggtatatgg actactgggg tcaaggaacc tcagtcaccg tctcctca              348
<210> 92
   <211> 116
   <212> PRT
   <213> Artificial Sequence
<220>
   <223> VH of 3D1
<400> 92
   Gln Val Gln Leu Gln Gln Ser Gly Pro Glu Leu Val Arg Pro Gly Glu
    1               5                  10                  15
   Ser Val Lys Ile Ser Cys Lys Gly Ser Gly Tyr Thr Phe Thr Asp Tyr
               20                  25                  30
   Ala Ile Gln Trp Val Lys Gln Ser His Ala Lys Ser Leu Glu Trp Ile
           35                  40                  45
   Gly Val Ile Asn Ile Tyr Tyr Asp Asn Thr Asn Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Arg Ala Ala Trp Tyr Met Asp Tyr Trp Gly Gln Gly Thr Ser Val
               100                 105                 110
   Thr Val Ser Ser
           115
<210> 93
<400> 93
   000
<210> 94
<400> 94
   000
<210> 95
   <211> 336
   <212> DNA
   <213> Artificial Sequence
<220>
   <223> VL of 3D1
<400> 95
   gacattgtgc tgtcacagtc tccatcctcc ctggctgtgt cagcaggaga gaaggtcact 60
   atgagctgca aatccagtca gagtctgctc aacagtagaa cccgagagaa ctacttggct 120
   tggtaccagc agaaaccagg gcagtctcct aaactgctga tctactgggc atccactagg 180
   gaatctgggg tccctgatcg cttcacaggc agtggatctg ggacagattt cactctcacc 240
   atcagcagtg tgcaggctga agacctggca gtttattact gcacgcaatc ttataatctt 300
   tacacgttcg gaggggggac caagctggaa ataaaa                           336
<210> 96
   <211> 112
   <212> PRT
   <213> Artificial Sequence
<220>
   <223> VL of 3D1
<400> 96
   Asp Ile Val Leu Ser Gln Ser Pro Ser Ser Leu Ala Val Ser Ala Gly
    1               5                   10                  15
   Glu Lys Val Thr Met Ser Cys Lys Ser Ser Gln Ser Leu Leu Asn Ser
               20                  25                  30
   Arg Thr Arg Glu Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Thr Gln
                   85                  90                  95
   Ser Tyr Asn Leu Tyr Thr Phe Gly Gly Gly Thr Lys Leu Glu Ile Lys
               100                 105                 110
<210> 97
   <211> 744
   <212> DNA
   <213> Artificial Sequence
<220>
   <223> 3D1 scFv
<400> 97
   caggtccagc tgcagcagtc tgggcctgag ctggtgaggc ctggggaatc agtgaagatt 60
   tcctgcaagg gttccggcta cacattcact gattatgcta tacagtgggt gaagcagagt 120
   catgcaaaga gtctagagtg gattggagtt attaatattt actatgataa tacaaactac 180
   aaccagaagt ttaagggcaa ggccacaatg actgtagaca aatcctccag cacagcctat 240
   atggaacttg ccagattgac atctgaggat tctgccatct attactgtgc aagagcggcc 300
   tggtatatgg actactgggg tcaaggaacc tcagtcaccg tctcctcagg gggtggaggc 360
   tctggtggcg gtggctctgg cggaggtgga tccggtggcg gcggatctga cattgtgctg 420
   tcacagtctc catcctccct ggctgtgtca gcaggagaga aggtcactat gagctgcaaa 480
   tccagtcaga gtctgctcaa cagtagaacc cgagagaact acttggcttg gtaccagcag 540
   aaaccagggc agtctcctaa actgctgatc tactgggcat ccactaggga atctggggtc 600
   cctgatcgct tcacaggcag tggatctggg acagatttca ctctcaccat cagcagtgtg 660
   caggctgaag acctggcagt ttattactgc acgcaatctt ataatcttta cacgttcgga 720
   ggggggacca agctggaaat aaaa                                        744
<210> 98
   <211> 248
   <212> PRT
   <213> Artificial Sequence
<220>
   <223> 3D1 scFv
<400> 98
   Gln Val Gln Leu Gln Gln Ser Gly Pro Glu Leu Val Arg Pro Gly Glu
    1               5                  10                  15
   Ser Val Lys Ile Ser Cys Lys Gly Ser Gly Tyr Thr Phe Thr Asp Tyr
               20                  25                  30
   Ala Ile Gln Trp Val Lys Gln Ser His Ala Lys Ser Leu Glu Trp Ile
           35                  40                  45
   Gly Val Ile Asn Ile Tyr Tyr Asp Asn Thr Asn Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Arg Ala Ala Trp Tyr Met Asp Tyr Trp Gly Gln Gly Thr Ser Val
               100                 105                 110
   Thr Val Ser Ser Gly Gly Gly Gly Ser Gly Gly Gly Gly Ser Gly Gly
           115                 120                 125
   Gly Gly Ser Gly Gly Gly Gly Ser Asp Ile Val Leu Ser Gln Ser Pro
       130                 135                 140
   Ser Ser Leu Ala Val Ser Ala Gly Glu Lys Val Thr Met Ser Cys Lys
   145                 150                 155                 160
   Ser Ser Gln Ser Leu Leu Asn Ser Arg Thr Arg Glu Asn Tyr Leu Ala
                   165                 170                 175
   Trp Tyr Gln Gln Lys Pro Gly Gln Ser Pro Lys Leu Leu Ile Tyr Trp
               180                 185                 190
   Ala Ser Thr Arg Glu Ser Gly Val Pro Asp Arg Phe Thr Gly Ser Gly
           195                 200                 205
   Ser Gly Thr Asp Phe Thr Leu Thr Ile Ser Ser Val Gln Ala Glu Asp
       210                 215                 220
   Leu Ala Val Tyr Tyr Cys Thr Gln Ser Tyr Asn Leu Tyr Thr Phe Gly
   225                 230                 235                 240
   Gly Gly Thr Lys Leu Glu Ile Lys
                   245
<210> 99
   <211> 247
   <212> PRT
   <213> Artificial Sequence
<220>
   <223> MET021 scFv
<400> 99
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ser Ile Arg Asn Tyr
               20                  25                  30
   Leu Asn Trp Tyr Gln Gln Glu Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Ile Leu Thr Ile Ser Ser Leu Gln Pro
   65 70 75 80
   Glu Asp Phe Ala Thr Tyr Tyr Cys Gln Gln Ser Tyr Val Thr Pro Leu
   85 90 95
   Thr Phe Gly Gly Gly Thr Lys Val Glu Ile Lys Gly Gly Gly Gly Ser
   100 105 110
   Gly Gly Gly Gly Ser Gly Gly Gly Gly Ser Gly Gly Gly Gly Ser Glu
   115 120 125
   Val Gln Leu Leu Glu Ser Gly Gly Gly Leu Val Gln Pro Gly Gly Ser
   130 135 140
   Leu Arg Leu Ser Cys Ala Ala Ser Gly Phe Thr Phe Ser Lys Tyr Asp
   145 150 155 160
   Met Leu Trp Val Arg Gln Ala Pro Gly Lys Gly Leu Glu Trp Val Ser
   165 170 175
   Tyr Ile Tyr Pro Ser Gly Gly Leu Thr Glu Tyr Ala Asp Ser Val Lys
   180 185 190
   Gly Arg Phe Thr Ile Ser Arg Asp Asn Ser Lys Asn Thr Leu Tyr Leu
   195 200 205
   Gln Met Asn Ser Leu Arg Ala Glu Asp Thr Ala Val Tyr Tyr Cys Ala
   210 215 220
   Arg Arg Ala Pro Arg Ser Leu Ser Phe Asp Ile Trp Gly Gln Gly Thr
   225 230 235 240
   Met Val Thr Val Ser Ser Ser
   245
<210> 100
   <211> 120
   <212> PRT
   <213> Artificial Sequence
<220>
   <223> VH of MET021 scFv
<400> 100
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Lys Tyr
   20 25 30
   Asp Met Leu Trp Val Arg Gln Ala Pro Gly Lys Gly Leu Glu Trp Val
   35 40 45
   Ser Tyr Ile Tyr Pro Ser Gly Gly Leu Thr Glu Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Ala Pro Arg Ser Leu Ser Phe Asp Ile Trp Gly Gln Gly
   100 105 110
   Thr Met Val Thr Val Ser Ser Ser
   115 120
<210> 101
   <211> 107
   <212> PRT
   <213> Artificial Sequence
<220>
   <223> VL of MET021 scFv
<400> 101
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ser Ile Arg Asn Tyr
               20                  25                  30
   Leu Asn Trp Tyr Gln Gln Glu Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Ile Leu Thr Ile Ser Ser Leu Gln Pro
   65                  70                  75                  80
   Glu Asp Phe Ala Thr Tyr Tyr Cys Gln Gln Ser Tyr Val Thr Pro Leu
                   85                  90                  95
   Thr Phe Gly Gly Gly Thr Lys Val Glu Ile Lys
               100                 105
<210> 102
   <211> 249
   <212> PRT
   <213> Artificial Sequence
<220>
   <223> G19-4 scFv
<400> 102
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser
                   245
<210> 103
   <211> 122
   <212> PRT
   <213> Artificial Sequence
<220>
   <223> VH of G19-4
<400> 103
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser
           115                 120
<210> 104
   <211> 112
   <212> PRT
   <213> Artificial Sequence
<220>
   <223> VL of G19-4
<400> 104
   Ala Gln Asp Ile Gln Met Thr Gln Thr Thr Ser Ser Leu Ser Ala Ser
    1               5                  10                  15
   Leu Gly Asp Arg Val Thr Ile Ser Cys Arg Ala Ser Gln Asp Ile Arg
               20                  25                  30
   Asn Tyr Leu Asn Trp Tyr Gln Gln Lys Pro Asp Gly Thr Val Lys Leu
           35                  40                  45
   Leu Ile Tyr Tyr Thr Ser Arg Leu His Ser Gly Val Pro Ser Arg Phe
       50                  55                  60
   Ser Gly Ser Gly Ser Gly Thr Asp Tyr Ser Leu Thr Ile Ala Asn Leu
   65                  70                  75                  80
   Gln Pro Glu Asp Ile Ala Thr Tyr Phe Cys Gln Gln Gly Asn Thr Leu
                   85                  90                  95
   Pro Trp Thr Phe Gly Gly Gly Thr Lys Leu Val Thr Lys Arg Ser Ser
               100                 105                 110
<210> 105
   <211> 252
   <212> PRT
   <213> Artificial Sequence
<220>
   <223> HD37 scFv
<400> 105
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
                       100                 105                 110
   Gly Gly Gly Ser Gly Gly Gly Gly Ser Gly Gly Gly Gly Ala Ser Gln
               115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser
                   245                 250
<210> 106
   <211> 121
   <212> PRT
   <213> Artificial Sequence
<220>
   <223> VH of 2E12 scFv
<400> 106
   Gln Val Gln Leu Lys Glu Ser Gly Pro Gly Ser Val Ala Pro Ser Gln
    1               5                  10                  15
   Ser Leu Ser Ile Thr Cys Thr Val Ser Gly Phe Ser Leu Thr Gly Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Ser Thr Asp Tyr Asn Ser Ala Leu Lys
       50                  55                  60
   Ser Arg Leu Ser Ile Thr Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Gly Tyr Ser Asn Phe His Tyr Tyr Val Met Asp Tyr Trp Gly
               100                 105                 110
   Gln Gly Thr Ser Val Thr Val Ser Ser
           115                 120
<210> 107
   <211> 111
   <212> PRT
   <213> Artificial Sequence
<220>
   <223> VL of 2E12 scFv
<400> 107
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Glu Ser Val Glu Tyr Tyr
               20                  25                  30
   Val Thr Ser Leu Met Gln Trp Tyr Gln Gln Lys Pro Gly Gln Pro Pro
           35                  40                  45
   Lys Leu Leu Ile Ser Ala Ala Ser Asn Val Glu Ser Gly Val Pro Ala
       50                  55                  60
   Arg Phe Ser Gly Ser Gly Ser Gly Thr Asp Phe Ser Leu Asn Ile His
   65                  70                  75                  80
   Pro Val Glu Glu Asp Asp Ile Ala Met Tyr Phe Cys Gln Gln Ser Arg
                   85                  90                  95
   Lys Val Pro Trp Thr Phe Gly Gly Gly Thr Lys Leu Glu Ile Lys
               100                 105                 110
<210> 108
   <211> 20
   <212> PRT
   <213> Artificial Sequence
<220>
   <223> Linker of 2E12 scFv
<400> 108
   Gly Gly Gly Gly Ser Gly Gly Gly Gly Ser Gly Gly Gly Gly Ser Gly
   1               5                   10                  15
   Gly Gly Gly Ser
               20
<210> 109
   <211> 252
   <212> PRT
   <213> Artificial Sequence
<220>
   <223> full length 2E12 scFv
<400> 109
   Gln Val Gln Leu Lys Glu Ser Gly Pro Gly Ser Val Ala Pro Ser Gln
    1               5                  10                  15
   Ser Leu Ser Ile Thr Cys Thr Val Ser Gly Phe Ser Leu Thr Gly Tyr
               20                  25                  30
   Gly Val Asn Trp Val Arg Gln Pro Pro Gly Lys Gly Leu Glu Trp Leu
           35                  40                  45
   Gly Met Ile Trp Gly Asp Gly Ser Thr Asp Tyr Asn Ser Ala Leu Lys
       50                  55                  60
   Ser Arg Leu Ser Ile Thr Lys Asp Asn Ser Lys Ser Gln Val Phe Leu
   65                  70                  75                  80
   Lys Met Asn Ser Leu Gln Thr Asp Asp Thr Ala Arg Tyr Tyr Cys Ala
                   85                  90                  95
   Arg Asp Gly Tyr Ser Asn Phe His Tyr Tyr Val Met Asp Tyr Trp Gly
               100                 105                 110
   Gln Gly Thr Ser Val Thr Val Ser Ser Gly Gly Gly Gly Ser Gly Gly
           115                 120                 125
   Gly Gly Ser Gly Gly Gly Gly Ser Gly Gly Gly Gly Ser Asp Ile Val
       130                 135                 140
   Leu Thr Gln Ser Pro Ala Ser Leu Ala Val Ser Leu Gly Gln Arg Ala
   145                 150                 155                 160
   Thr Ile Ser Cys Arg Ala Ser Glu Ser Val Glu Tyr Tyr Val Thr Ser
                   165                 170                 175
   Leu Met Gln Trp Tyr Gln Gln Lys Pro Gly Gln Pro Pro Lys Leu Leu
               180                 185                 190
   Ile Ser Ala Ala Ser Asn Val Glu Ser Gly Val Pro Ala Arg Phe Ser
           195                 200                 205
   Gly Ser Gly Ser Gly Thr Asp Phe Ser Leu Asn Ile His Pro Val Glu
       210                 215                 220
   Glu Asp Asp Ile Ala Met Tyr Phe Cys Gln Gln Ser Arg Lys Val Pro
   225                 230                 235                 240
   Trp Thr Phe Gly Gly Gly Thr Lys Leu Glu Ile Lys
                   245                 250
<210> 110
   <211> 22
   <212> PRT
   <213> Artificial Sequence
<220>
   <223> Signal peptide of 2E12 scFv
<400> 110
   Met Asp Phe Gln Val Gln Ile Phe Ser Phe Leu Leu Ile Ser Ala Ser
    1               5                  10                  15
   Val Ile Met Ser Arg Gly
               20
<210> 111
   <211> 20
   <212> PRT
   <213> Artificial Sequence
<220>
   <223> Signal peptide in various signle chain fusion proteins (X01xx)
<400> 111
   Met Glu Ala Pro Ala Gln Leu Leu Phe Leu Leu Leu Leu Trp Leu Pro
   1 5 10 15
   Asp Thr Thr Gly
   20
<210> 112
   <211> 107
   <212> PRT
   <213> Homo sapiens
<400> 112
<210> 113
   <211> 106
   <212> PRT
   <213> Homo sapiens
<400> 113
   Gly Gln Pro Lys Ala Asn Pro Thr Val Thr Leu Phe Pro Pro Ser Ser
   1 5 10 15
   Glu Glu Leu Gln Ala Asn Lys Ala Thr Leu Val Cys Leu Ile Ser Asp
   20 25 30
   Phe Tyr Pro Gly Ala Val Thr Val Ala Trp Lys Ala Asp Gly Ser Pro
   35 40 45
   Val Lys Ala Gly Val Glu Thr Thr Lys Pro Ser Lys Gln Ser Asn Asn
   50 55 60
   Lys Tyr Ala Ala Ser Ser Tyr Leu Ser Leu Thr Pro Glu Gln Trp Lys
   65 70 75 80
   Ser His Arg Ser Tyr Ser Cys Gln Val Thr His Glu Gly Ser Thr Val
   85 90 95
   Glu Lys Thr Val Ala Pro Thr Glu Cys Ser
   100 105
<210> 114
   <211> 98
   <212> PRT
   <213> Homo sapiens
<400> 114
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val
<210> 115
   <211> 110
   <212> PRT
   <213> Homo sapiens
<400> 115
   Ala Pro Glu Leu Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 116
   <211> 107
   <212> PRT
   <213> Homo sapiens
<400> 116
   Gly Gln Pro Arg Glu Pro Gln Val Tyr Thr Leu Pro Pro Ser Arg Asp
    1               5                  10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 117
   <211> 125
   <212> PRT
   <213> Artificial Sequence
<220>
   <223> VH of HD37
<400> 117
   Gln Val Gln Leu Gln Gln Ser Gly Ala Glu Leu Val Arg Pro Gly Ser
    1               5                  10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Gln Gly Leu Glu Trp Ile
           35                  40                  45
   Gly Gln Ile Trp Pro Gly Asp Gly Asp Thr Asn Tyr Asn Gly Lys Phe
       50                  55                  60
   Lys Gly Lys Ala Thr Leu Thr Ala Asp Glu Ser Ser Ser Thr Ala Tyr
   65                  70                  75                  80
   Met Gln Leu Ser Ser Leu Ala Ser Glu Asp Ser Ala Val Tyr Phe Cys
                   85                  90                  95
   Ala Arg Arg Glu Thr Thr Thr Val Gly Arg Tyr Tyr Tyr Ala Met Asp
               100                 105                 110
   Tyr Trp Gly Gln Gly Thr Ser Val Thr Val Ser Ser Ser
           115                 120                 125
<210> 118
<400> 118
   000
<210> 119
   <211> 111
   <212> PRT
   <213> Artificial Sequence
<220>
   <223> VL of HD37
<400> 119
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys
               100                 105                 110
<210> 120
   <211> 249
   <212> PRT
   <213> Artificial Sequence
<220>
   <223> M0042 scFv (VL-VH)
<400> 120
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Val
           35                  40                  45
   Tyr Ser Val Ser Ser Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Ala Thr Tyr Tyr Cys Gln Gln Ser Tyr Ser Thr Pro Phe
                   85                  90                  95
   Thr Phe Gly Pro Gly Thr Lys Val Asp Ile Lys Gly Gly Gly Gly Ser
               100                 105                 110
   Gly Gly Gly Gly Ser Gly Gly Gly Gly Ser Gly Gly Gly Gly Ser Glu
           115                 120                 125
   Val Gln Leu Leu Glu Ser Gly Gly Gly Leu Val Gln Pro Gly Gly Ser
       130                 135                 140
   Leu Arg Leu Ser Cys Ala Ala Ser Gly Phe Thr Phe Ser His Tyr Tyr
   145                 150                 155                 160
   Met Ser Trp Val Arg Gln Ala Pro Gly Lys Gly Leu Glu Trp Val Ser
                   165                 170                 175
   Tyr Ile Gly Pro Ser Gly Gly Phe Thr Ala Tyr Ala Asp Ser Val Lys
               180                 185                 190
   Gly Arg Phe Thr Ile Ser Arg Asp Asn Ser Lys Asn Thr Leu Tyr Leu
           195                 200                 205
   Gln Met Asn Ser Leu Arg Ala Glu Asp Thr Ala Val Tyr Tyr Cys Ala
       210                 215                 220
   Arg Asp Gly Trp Val Gly Ser Glu Asn Tyr Ser Gly Met Asp Val Trp
   225                 230                 235                 240
   Gly Gln Gly Thr Thr Val Ser Ser Ser
                   245
<210> 121
   <211> 122
   <212> PRT
   <213> Artificial Sequence
<220>
   <223> VH of M0042
<400> 121
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser His Tyr
               20                  25                  30
   Tyr Met Ser Trp Val Arg Gln Ala Pro Gly Lys Gly Leu Glu Trp Val
           35                  40                  45
   Ser Tyr Ile Gly Pro Ser Gly Gly Phe Thr Ala Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Trp Val Gly Ser Glu Asn Tyr Ser Gly Met Asp Val
               100                 105                 110
   Trp Gly Gln Gly Thr Thr Val Ser Ser Ser
           115                 120
<210> 122
   <211> 107
   <212> PRT
   <213> Artificial Sequence
<220>
   <223> VL of M0042
<400> 122
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Val
           35                  40                  45
   Tyr Ser Val Ser Ser Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Ala Thr Tyr Tyr Cys Gln Gln Ser Tyr Ser Thr Pro Phe
                   85                  90                  95
   Thr Phe Gly Pro Gly Thr Lys Val Asp Ile Lys
               100                 105
<210> 123
<400> 123
   000
<210> 124
<400> 124
   000
<210> 125
   <211> 20
   <212> PRT
   <213> Artificial Sequence
<220>
   <223> NKG2D Linker
<400> 125
   Asn Ser Leu Phe Asn Gln Glu Val Gln Ile Pro Leu Thr Glu Ser Tyr
    1               5                   10                  15
   Ser Pro Asn Ser
               20
<210> 126
<400> 126
   000
<210> 127
<400> 127
   000
<210> 128
<400> 128
   000
<210> 129
<400> 129
   000
<210> 130
<400> 130
   000
<210> 131
<400> 131
   000
<210> 132
<400> 132
   000
<210> 133
<400> 133
   000
<210> 134
<400> 134
   000
<210> 135
<400> 135
   000
<210> 136
<400> 136
   000
<210> 137
<400> 137
   000
<210> 138
<400> 138
   000
<210> 139
<400> 139
   000
<210> 140
   <211> 105
   <212> PRT
   <213> Artificial Sequence
<220>
   <223> Altered human C1 sequence used in constructing Interceptors
<400> 140
   Gln Pro Lys Ala Asn Pro Thr Val Thr Leu Phe Pro Pro Ser Ser Glu
    1               5                  10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Gly Ser Pro Val
           35                  40                  45
   Lys Ala Gly Val Glu Thr Thr Lys Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Ser Ser
               100                 105
<210> 141
   <211> 105
   <212> PRT
   <213> Artificial Sequence
<220>
   <223> Altered human Ck sequence used in constructing Interceptors
<400> 141
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 142
   <211> 105
   <212> PRT
   <213> Artificial Sequence
<220>
   <223> Altered human Ck with N29A substitution
<400> 142
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 143
   <211> 105
   <212> PRT
   <213> Artificial Sequence
<220>
   <223> Altered human Ck with N30A substitution
<400> 143
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Asn Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 144
   <211> 105
   <212> PRT
   <213> Artificial Sequence
<220>
   <223> Altered human Ck with Q52A substitution
<400> 144
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Ala Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 145
   <211> 105
   <212> PRT
   <213> Artificial Sequence
<220>
   <223> Altered human Ck with V55A substitution
<400> 145
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 146
   <211> 105
   <212> PRT
   <213> Artificial Sequence
<220>
   <223> Altered human Ck with T56A substitution
<400> 146
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Ala Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 147
   <211> 105
   <212> PRT
   <213> Artificial Sequence
<220>
   <223> Altered human Ck with S68A substitution
<400> 147
   Thr Val Ala Ala Pro Ser Val Phe Ile Phe Pro Pro Ser Asp Glu Gln
   1 5 10 15
   Leu Lys Ser Gly Thr Ala Ser Val Val Cys Leu Leu Asn Asn Phe Tyr
   20 25 30
   Pro Arg Glu Ala Lys Val Gln Trp Lys Val Asp Asn Ala Leu Gln Ser
   35 40 45
   Gly Asn Ser Gln Glu Ser Val Thr Glu Gln Asp Ser Lys Asp Ser Thr
   50 55 60
   Tyr Ser Leu Ala Ser Thr Leu Thr Leu Ser Lys Ala Asp Tyr Glu Lys
   65 70 75 80
   His Lys Val Tyr Ala Cys Glu Val Thr His Gln Gly Leu Ser Ser Pro
   85 90 95
   Val Thr Lys Ser Phe Asn Arg Gly Glu
   100 105
<210> 148
   <211> 105
   <212> PRT
   <213> Artificial Sequence
<220>
   <223> Altered human Ck with T70A substitution
<400> 148
   Thr Val Ala Ala Pro Ser Val Phe Ile Phe Pro Pro Ser Asp Glu Gln
   1 5 10 15
   Leu Lys Ser Gly Thr Ala Ser Val Val Cys Leu Leu Asn Asn Phe Tyr
   20 25 30
   Pro Arg Glu Ala Lys Val Gln Trp Lys Val Asp Asn Ala Leu Gln Ser
   35 40 45
   Gly Asn Ser Gln Glu Ser Val Thr Glu Gln Asp Ser Lys Asp Ser Thr
   50 55 60
   Tyr Ser Leu Ser Ser Ala Leu Thr Leu Ser Lys Ala Asp Tyr Glu Lys
   65 70 75 80
   His Lys Val Tyr Ala Cys Glu Val Thr His Gln Gly Leu Ser Ser Pro
   85 90 95
   Val Thr Lys Ser Phe Asn Arg Gly Glu
   100 105
<210> 149
   <211> 105
   <212> PRT
   <213> Artificial Sequence
<220>
   <223> Altered human Ck with V55A N29A substitution
<400> 149
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Ala Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 150
   <211> 105
   <212> PRT
   <213> Artificial Sequence
<220>
   <223> Altered human Ck with V55A N30A substitution
<400> 150
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Asn Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 151
   <211> 105
   <212> PRT
   <213> Artificial Sequence
<220>
   <223> Altered human Ck with V55A Q52A substitution
<400> 151
   Thr Val Ala Ala Pro Ser Val Phe Ile Phe Pro Pro Ser Asp Glu Gln
    1              5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Ala Glu Ser Ala Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 152
   <211> 105
   <212> PRT
   <213> Artificial Sequence
<220>
   <223> Altered human Ck with V55A S68A substitution
<400> 152
   Thr Val Ala Ala Pro Ser Val Phe Ile Phe Pro Pro Ser Asp Glu Gln
    1              5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ala Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 153
   <211> 105
   <212> PRT
   <213> Artificial Sequence
<220>
   <223> Altered human Ck with V55A T70A substitution
<400> 153
   Thr Val Ala Ala Pro Ser Val Phe Ile Phe Pro Pro Ser Asp Glu Gln
    1              5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 154
   <211> 105
   <212> PRT
   <213> Artificial Sequence
<220>
   <223> Altered human Ck with V55A N29A N30A substitution
<400> 154
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 155
   <211> 105
   <212> PRT
   <213> Artificial Sequence
<220>
   <223> Altered human Ck with V55A N29A S68A substitution
<400> 155
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ala Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 156
   <211> 105
   <212> PRT
   <213> Artificial Sequence
<220>
   <223> Altered human Ck with V55A S68A T70A substitution
<400> 156
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ala Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 157
   <211> 105
   <212> PRT
   <213> Artificial Sequence
<220>
   <223> Altered human Ck with V55A Q52A S68A substitution
<400> 157
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Ala Glu Ser Ala Thr Glu Gln Asp Ser Lys Asp Ser Thr
       50                  55                  60
   Tyr Ser Leu Ala Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 158
   <211> 105
   <212> PRT
   <213> Artificial Sequence
<220>
   <223> Altered human Ck with N29R V55A T70A substitution
<400> 158
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Arg Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 159
   <211> 105
   <212> PRT
   <213> Artificial Sequence
<220>
   <223> Altered human Ck with N29AK V55A T70A substitution
<400> 159
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Lys Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 160
   <211> 105
   <212> PRT
   <213> Artificial Sequence
<220>
   <223> Altered human Ck with N29W V55A T70A substitution
<400> 160
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Trp Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 161
   <211> 105
   <212> PRT
   <213> Artificial Sequence
<220>
   <223> Altered human Ck with N29Y V55A T70A substitution
<400> 161
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Tyr Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 162
   <211> 105
   <212> PRT
   <213> Artificial Sequence
<220>
   <223> Altered human Ck with S68K V55A substitution
<400> 162
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Lys Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 163
   <211> 105
   <212> PRT
   <213> Artificial Sequence
<220>
   <223> Altered human Ck with S68E V55A substitution
<400> 163
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Glu Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 164
   <211> 105
   <212> PRT
   <213> Artificial Sequence
<220>
   <223> Altered human Ck with S68Q V55A substitution
<400> 164
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Gln Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 165
   <211> 105
   <212> PRT
   <213> Artificial Sequence
<220>
   <223> Altered human Ck with T70E V55A substitution
<400> 165
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 166
   <211> 105
   <212> PRT
   <213> Artificial Sequence
<220>
   <223> Altered human Ck with Q52L N29A N30A substitution
<400> 166
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Leu Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 167
   <211> 105
   <212> PRT
   <213> Artificial Sequence
<220>
   <223> Altered human Ck with N30R V55A T70A substitution
<400> 167
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Arg Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 168
   <211> 105
   <212> PRT
   <213> Artificial Sequence
<220>
   <223> Altered human Ck with N30K V55A T70A substitution
<400> 168
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Lys Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 169
   <211> 105
   <212> PRT
   <213> Artificial Sequence
<220>
   <223> Altered human Ck with N30W V55A T70A substitution
<400> 169
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Trp Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 170
   <211> 105
   <212> PRT
   <213> Artificial Sequence
<220>
   <223> Altered human Ck with N30E V55A T70A substitution
<400> 170
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Glu Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 171
   <211> 105
   <212> PRT
   <213> Artificial Sequence
<220>
   <223> Altered human Ck with N30G V55A T70A substitution
<400> 171
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Gly Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Ala Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 172
   <211> 105
   <212> PRT
   <213> Artificial Sequence
<220>
   <223> Altered human Ck with V55R N29A N30A substitution
<400> 172
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Arg Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 173
   <211> 105
   <212> PRT
   <213> Artificial Sequence
<220>
   <223> Altered human Ck with V55W N29A N30A substitution
<400> 173
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Trp Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 174
   <211> 105
   <212> PRT
   <213> Artificial Sequence
<220>
   <223> Altered human Ck with V55E N29A N30A substitution
<400> 174
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Glu Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 175
   <211> 105
   <212> PRT
   <213> Artificial Sequence
<220>
   <223> Altered human Ck with N29W N30Y V55A T70E (WYAE) substitution
<400> 175
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Trp Tyr Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 176
   <211> 105
   <212> PRT
   <213> Artificial Sequence
<220>
   <223> Altered human Ck with N29Y N30Y V55A T70E (YYAE) substitution
<400> 176
   Thr Val Ala Ala Pro Ser Val Phe Ile Phe Pro Pro Ser Asp Glu Gln
    1               5                   10                  15
   Leu Lys Ser Gly Thr Ala Ser Val Val Cys Leu Leu Tyr Tyr Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 177
   <211> 105
   <212> PRT
   <213> Artificial Sequence
<220>
   <223> Altered human Ck with N30E V55A T70E (EAE) substitution
<400> 177
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Glu Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 178
   <211> 105
   <212> PRT
   <213> Artificial Sequence
<220>
   <223> Altered human Ck with N30Y V55A T70E (YAE) substitution
<400> 178
   Thr Val Ala Ala Pro Ser Val Phe Ile Phe Pro Pro Ser Asp Glu Gln
   1               5                   10                  15
   Leu Lys Ser Gly Thr Ala Ser Val Val Cys Leu Leu Asn Tyr Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 179
<400> 179
   000
<210> 180
<400> 180
   000
<210> 181
   <211> 4
   <212> PRT
   <213> Artificial Sequence
<220>
   <223> Portion of human IgG1 hinge
<400> 181
   Cys Pro Pro Cys
   1
<210> 182
   <211> 112
   <212> PRT
   <213> Artificial Sequence
<220>
   <223> VL of P2C2 scFv
<400> 182
   Asp Ile Val Met Ile Gln Asp Glu Leu Ser Asn Pro Val Ser Ser Gly
   1               5                   10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
               100                 105                 110
<210> 183
   <211> 15
   <212> PRT
   <213> Artificial Sequence
<220>
   <223> Linker of P2C2 scFv
<400> 183
   Gly Gly Gly Gly Ser Gly Gly Gly Gly Ser Gly Gly Gly Gly Ser
   1               5                   10                  15
<210> 184
   <211> 112
   <212> PRT
   <213> Artificial Sequence
<220>
   <223> VH of P2C2 scFv
<400> 184
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Asp Phe
               20                  25                  30
   Ser Met His Trp Val Arg Gln Ala Pro Gly Lys Gly Leu Lys Trp Met
           35                  40                  45
   Gly Trp Ile Asn Thr Glu Thr Gly Glu Pro Thr Tyr Ala Asp Asp Phe
       50                  55                  60
   Arg Gly Arg Phe Ala Phe Ser Leu Glu Thr Ser Ala Ser Thr Ala Tyr
   65                  70                  75                  80
   Leu Gln Ile Asn Asn Leu Lys Asn Glu Asp Thr Ala Thr Tyr Phe Cys
                   85                  90                  95
   Thr Trp Ser Ala Tyr Trp Gly Gln Gly Thr Leu Val Thr Val Ser Ala
               100                 105                 110
<210> 185
   <211> 239
   <212> PRT
   <213> Artificial Sequence
<220>
   <223> full length of P2C2 scFv
<400> 185
   Asp Ile Val Met Ile Gln Asp Glu Leu Ser Asn Pro Val Ser Ser Gly
   1               5                   10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
               100                 105                 110
   Gly Gly Gly Gly Ser Gly Gly Gly Gly Ser Gly Gly Gly Gly Ser Gln
           115                 120                 125
   Ile Gln Leu Val Gln Ser Gly Pro Glu Leu Lys Lys Pro Gly Glu Thr
       130                 135                 140
   Val Lys Ile Ser Cys Lys Ala Ser Gly Tyr Thr Phe Thr Asp Phe Ser
   145                 150                 155                 160
   Met His Trp Val Arg Gln Ala Pro Gly Lys Gly Leu Lys Trp Met Gly
                   165                 170                 175
   Trp Ile Asn Thr Glu Thr Gly Glu Pro Thr Tyr Ala Asp Asp Phe Arg
               180                 185                 190
   Gly Arg Phe Ala Phe Ser Leu Glu Thr Ser Ala Ser Thr Ala Tyr Leu
           195                 200                 205
   Gln Ile Asn Asn Leu Lys Asn Glu Asp Thr Ala Thr Tyr Phe Cys Thr
       210                 215                 220
   Trp Ser Ala Tyr Trp Gly Gln Gly Thr Leu Val Thr Val Ser Ala
   225                 230                 235
<210> 186
   <211> 102
   <212> PRT
   <213> Homo sapiens
<400> 186
   Ala Ser Pro Thr Ser Pro Lys Val Phe Pro Leu Ser Leu Cys Ser Thr
   1               5                   10                  15
   Gln Pro Asp Gly Asn Val Val Ile Ala Cys Leu Val Gln Gly Phe Phe
               20                  25                  30
   Pro Gln Glu Pro Leu Ser Val Thr Trp Ser Glu Ser Gly Gln Gly Val
           35                  40                  45
   Thr Ala Arg Asn Phe Pro Pro Ser Gln Asp Ala Ser Gly Asp Leu Tyr
       50                  55                  60
   Thr Thr Ser Ser Gln Leu Thr Leu Pro Ala Thr Gln Cys Leu Ala Gly
   65                  70                  75                  80
   Lys Ser Val Thr Cys His Val Lys His Tyr Thr Asn Pro Ser Gln Asp
                   85                  90                  95
   Val Thr Val Pro Cys Pro
               100
<210> 187
   <211> 102
   <212> PRT
   <213> Homo sapiens
<400> 187
   Ala Ser Pro Thr Ser Pro Lys Val Phe Pro Leu Ser Leu Asp Ser Thr
   1               5                   10                  15
   Pro Gln Asp Gly Asn Val Val Val Ala Cys Leu Val Gln Gly Phe Phe
               20                  25                  30
   Pro Gln Glu Pro Leu Ser Val Thr Trp Ser Glu Ser Gly Gln Asn Val
           35                  40                  45
   Thr Ala Arg Asn Phe Pro Pro Ser Gln Asp Ala Ser Gly Asp Leu Tyr
       50                  55                  60
   Thr Thr Ser Ser Gln Leu Thr Leu Pro Ala Thr Gln Cys Pro Asp Gly
   65                  70                  75                  80
   Lys Ser Val Thr Cys His Val Lys His Tyr Thr Asn Pro Ser Gln Asp
                   85                  90                  95
   Val Thr Val Pro Cys Pro
               100
<210> 188
   <211> 101
   <212> PRT
   <213> Homo sapiens
<400> 188
   Ala Pro Thr Lys Ala Pro Asp Val Phe Pro Ile Ile Ser Gly Cys Arg
    1               5                  10                  15
   His Pro Lys Asp Asn Ser Pro Val Val Leu Ala Cys Leu Ile Thr Gly
               20                  25                  30
   Tyr His Pro Thr Ser Val Thr Val Thr Trp Tyr Met Gly Thr Gln Ser
           35                  40                  45
   Gln Pro Gln Arg Thr Phe Pro Glu Ile Gln Arg Arg Asp Ser Tyr Tyr
       50                  55                  60
   Met Thr Ser Ser Gln Leu Ser Thr Pro Leu Gln Gln Trp Arg Gln Gly
   65                  70                  75                  80
   Glu Tyr Lys Cys Val Val Gln His Thr Ala Ser Lys Ser Lys Lys Glu
                   85                  90                  95
   Ile Phe Arg Trp Pro
               100
<210> 189
   <211> 103
   <212> PRT
   <213> Homo sapiens
<400> 189
   Ala Ser Thr Gln Ser Pro Ser Val Phe Pro Leu Thr Arg Cys Cys Lys
    1               5                  10                  15
   Asn Ile Pro Ser Asn Ala Thr Ser Val Thr Leu Gly Cys Leu Ala Thr
               20                  25                  30
   Gly Tyr Phe Pro Glu Pro Val Met Val Thr Cys Asp Thr Gly Ser Leu
           35                  40                  45
   Asn Gly Thr Thr Met Thr Leu Pro Ala Thr Thr Leu Thr Leu Ser Gly
       50                  55                  60
   His Tyr Ala Thr Ile Ser Leu Leu Thr Val Ser Gly Ala Trp Ala Lys
   65                  70                  75                  80
   Gln Met Phe Thr Cys Arg Val Ala His Thr Pro Ser Ser Thr Asp Trp
                   85                  90                  95
   Val Asp Asn Lys Thr Phe Ser
               100
<210> 190
   <211> 98
   <212> PRT
   <213> Homo sapiens
<400> 190
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val
<210> 191
   <211> 98
   <212> PRT
   <213> Homo sapiens
<400> 191
   Ala Ser Thr Lys Gly Pro Ser Val Phe Pro Leu Ala Pro Cys Ser Arg
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asn His Lys Pro Ser Asn Thr Lys Val Asp Lys
                   85                  90                  95
   Arg Val
<210> 192
   <211> 98
   <212> PRT
   <213> Homo sapiens
<400> 192
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val
<210> 193
<400> 193
   000
<210> 194
   <211> 104
   <212> PRT
   <213> Homo sapiens
<400> 194
   Gly Ser Ala Ser Ala Pro Thr Leu Phe Pro Leu Val Ser Cys Glu Asn
    1               5                  10                  15
   Ser Pro Ser Asp Thr Ser Ser Val Ala Val Gly Cys Leu Ala Gln Asp
               20                  25                  30
   Phe Leu Pro Asp Ser Ile Thr Leu Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro
               100
<210> 195
   <211> 101
   <212> PRT
   <213> Homo sapiens
<400> 195
   Cys Cys His Pro Arg Leu Ser Leu His Arg Pro Ala Leu Glu Asp Leu
    1               5                  10                  15
   Leu Leu Gly Ser Glu Ala Asn Leu Thr Cys Thr Leu Thr Gly Leu Arg
               20                  25                  30
   Asp Ala Ser Gly Val Thr Phe Thr Trp Thr Pro Ser Ser Gly Lys Ser
           35                  40                  45
   Ala Val Gln Gly Pro Pro Glu Arg Asp Leu Cys Gly Cys Tyr Ser Val
       50                  55                  60
   Ser Ser Val Leu Pro Gly Cys Ala Glu Pro Trp Asn His Gly Lys Thr
   65                  70                  75                  80
   Phe Thr Cys Thr Ala Ala Tyr Pro Glu Ser Lys Thr Pro Leu Thr Ala
                   85                  90                  95
   Thr Leu Ser Lys Ser
               100
<210> 196
   <211> 101
   <212> PRT
   <213> Homo sapiens
<400> 196
   Cys Cys His Pro Arg Leu Ser Leu His Arg Pro Ala Leu Glu Asp Leu
    1               5                  10                  15
   Leu Leu Gly Ser Glu Ala Asn Leu Thr Cys Thr Leu Thr Gly Leu Arg
                           20                  25                  30
   Asp Ala Ser Gly Ala Thr Phe Thr Trp Thr Pro Ser Ser Gly Lys Ser
               35                  40                  45
   Ala Val Gln Gly Pro Pro Glu Arg Asp Leu Cys Gly Cys Tyr Ser Val
           50                  55                  60
   Ser Ser Val Leu Pro Gly Cys Ala Gln Pro Trp Asn His Gly Glu Thr
   65                  70                  75                  80
   Phe Thr Cys Thr Ala Ala His Pro Glu Leu Lys Thr Pro Leu Thr Ala
                   85                  90                  95
   Asn Ile Thr Lys Ser
               100
<210> 197
   <211> 108
   <212> PRT
   <213> Homo sapiens
<400> 197
   Glu Cys Pro Ser His Thr Gln Pro Leu Gly Val Tyr Leu Leu Thr Pro
   1               5                   10                  15
   Ala Val Gln Asp Leu Trp Leu Arg Asp Lys Ala Thr Phe Thr Cys Phe
               20                  25                  30
   Val Val Gly Ser Asp Leu Lys Asp Ala His Leu Thr Trp Glu Val Ala
           35                  40                  45
   Gly Lys Val Pro Thr Gly Gly Val Glu Glu Gly Leu Leu Glu Arg His
       50                  55                  60
   Ser Asn Gly Ser Gln Ser Gln His Ser Arg Leu Thr Leu Pro Arg Ser
   65                  70                  75                  80
   Leu Trp Asn Ala Gly Thr Ser Val Thr Cys Thr Leu Asn His Pro Ser
                   85                  90                  95
   Leu Pro Pro Gln Arg Leu Met Ala Leu Arg Glu Pro
               100                 105
<210> 198
   <211> 107
   <212> PRT
   <213> Homo sapiens
<400> 198
   Val Cys Ser Arg Asp Phe Thr Pro Pro Thr Val Lys Ile Leu Gln Ser
   1               5                   10                  15
   Ser Cys Asp Gly Gly Gly His Phe Pro Pro Thr Ile Gln Leu Leu Cys
               20                  25                  30
   Leu Val Ser Gly Tyr Thr Pro Gly Thr Ile Asn Ile Thr Trp Leu Glu
           35                  40                  45
   Asp Gly Gln Val Met Asp Val Asp Leu Ser Thr Ala Ser Thr Thr Gln
       50                  55                  60
   Glu Gly Glu Leu Ala Ser Thr Gln Ser Glu Leu Thr Leu Ser Gln Lys
   65                  70                  75                  80
   His Trp Leu Ser Asp Arg Thr Tyr Thr Cys Gln Val Thr Tyr Gln Gly
                   85                  90                  95
   His Thr Phe Glu Asp Ser Thr Lys Lys Cys Ala
               100                 105
<210> 199
   <211> 109
   <212> PRT
   <213> Homo sapiens
<400> 199
   Ala Pro Pro Val Ala Gly Pro Ser Val Phe Leu Phe Pro Pro Lys Pro
    1               5                  10                  15
   Lys Asp Thr Leu Met Ile Ser Arg Thr Pro Glu Val Thr Cys Val Val
               20                  25                  30
   Val Asp Val Ser His Glu Asp Pro Glu Val Gln Phe Asn Trp Tyr Val
           35                  40                  45
   Asp Gly Val Glu Val His Asn Ala Lys Thr Lys Pro Arg Glu Glu Gln
       50                  55                  60
   Phe Asn Ser Thr Phe Arg Val Val Ser Val Leu Thr Val Val His Gln
   65                  70                  75                  80
   Asp Trp Leu Asn Gly Lys Glu Tyr Lys Cys Lys Val Ser Asn Lys Gly
                   85                  90                  95
   Leu Pro Ala Pro Ile Glu Lys Thr Ile Ser Lys Thr Lys
               100                 105
<210> 200
   <211> 110
   <212> PRT
   <213> Homo sapiens
<400> 200
   Ala Pro Glu Leu Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Gln Phe Lys Trp Tyr
           35                  40                  45
   Val Asp Gly Val Glu Val His Asn Ala Lys Thr Lys Pro Arg Glu Glu
       50                  55                  60
   Gln Tyr Asn Ser Thr Phe Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Thr Lys
               100                 105                 110
<210> 201
   <211> 110
   <212> PRT
   <213> Homo sapiens
<400> 201
   Ala Pro Glu Phe Leu Gly Gly Pro Ser Val Phe Leu Phe Pro Pro Lys
    1              5                   10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 202
   <211> 105
   <212> PRT
   <213> Artificial Sequence
<220>
   <223> Altered human Ck region + T70E, N29A, N30A, V55A
<400> 202
   Thr Val Ala Ala Pro Ser Val Phe Ile Phe Pro Pro Ser Asp Glu Gln
    1              5                   10                  15
   Leu Lys Ser Gly Thr Ala Ser Val Val Cys Leu Leu Ala Ala Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 203
   <211> 112
   <212> PRT
   <213> Homo sapiens
<400> 203
   Val Ile Ala Glu Leu Pro Pro Lys Val Ser Val Phe Val Pro Pro Arg
    1               5                  10                  15
   Asp Gly Phe Phe Gly Asn Pro Arg Lys Ser Lys Leu Ile Cys Gln Ala
               20                  25                  30
   Thr Gly Phe Ser Pro Arg Gln Ile Gln Val Ser Trp Leu Arg Glu Gly
           35                  40                  45
   Lys Gln Val Gly Ser Gly Val Thr Thr Asp Gln Val Gln Ala Glu Ala
       50                  55                  60
   Lys Glu Ser Gly Pro Thr Thr Tyr Lys Val Thr Ser Thr Leu Thr Ile
   65                  70                  75                  80
   Lys Glu Ser Asp Trp Leu Gly Gln Ser Met Phe Thr Cys Arg Val Asp
                   85                  90                  95
   His Arg Gly Leu Thr Phe Gln Gln Asn Ala Ser Ser Met Cys Val Pro
               100                 105                 110
<210> 204
   <211> 131
   <212> PRT
   <213> Homo sapiens
<400> 204
   Gly Asn Thr Phe Arg Pro Glu Val His Leu Leu Pro Pro Pro Ser Glu
    1               5                  10                  15
   Glu Leu Ala Leu Asn Glu Leu Val Thr Leu Thr Cys Leu Ala Arg Gly
               20                  25                  30
   Phe Ser Pro Lys Asp Val Leu Val Arg Trp Leu Gln Gly Ser Gln Glu
           35                  40                  45
   Leu Pro Arg Glu Lys Tyr Leu Thr Trp Ala Ser Arg Gln Glu Pro Ser
       50                  55                  60
   Gln Gly Thr Thr Thr Phe Ala Val Thr Ser Ile Leu Arg Val Ala Ala
   65                  70                  75                  80
   Glu Asp Trp Lys Lys Gly Asp Thr Phe Ser Cys Met Val Gly His Glu
                   85                  90                  95
   Ala Leu Pro Leu Ala Phe Thr Gln Lys Thr Ile Asp Arg Leu Ala Gly
               100                 105                 110
   Lys Pro Thr His Val Asn Val Ser Val Val Met Ala Glu Val Asp Gly
           115                 120                 125
   Thr Cys Tyr
       130
<210> 205
   <211> 131
   <212> PRT
   <213> Homo sapiens
<400> 205
   Gly Asn Thr Phe Arg Pro Glu Val His Leu Leu Pro Pro Pro Ser Glu
   1 5 10 15
   Glu Leu Ala Leu Asn Glu Leu Val Thr Leu Thr Cys Leu Ala Arg Gly
   20 25 30
   Phe Ser Pro Lys Asp Val Leu Val Arg Trp Leu Gln Gly Ser Gln Glu
   35 40 45
   Leu Pro Arg Glu Lys Tyr Leu Thr Trp Ala Ser Arg Gln Glu Pro Ser
   50 55 60
   Gln Gly Thr Thr Thr Phe Ala Val Thr Ser Ile Leu Arg Val Ala Ala
   65 70 75 80
   Glu Asp Trp Lys Lys Gly Asp Thr Phe Ser Cys Met Val Gly His Glu
   85 90 95
   Ala Leu Pro Leu Ala Phe Thr Gln Lys Thr Ile Asp Arg Leu Ala Gly
   100 105 110
   Lys Pro Thr His Val Asn Val Ser Val Val Met Ala Glu Val Asp Gly
   115 120 125
   Thr Cys Tyr
   130
<210> 206
   <211> 117
   <212> PRT
   <213> Homo sapiens
<400> 206
   Ala Ala Gln Ala Pro Val Lys Leu Ser Leu Asn Leu Leu Ala Ser Ser
   1 5 10 15
   Asp Pro Pro Glu Ala Ala Ser Trp Leu Leu Cys Glu Val Ser Gly Phe
   20 25 30
   Ser Pro Pro Asn Ile Leu Leu Met Trp Leu Glu Asp Gln Arg Glu Val
   35 40 45
   Asn Thr Ser Gly Phe Ala Pro Ala Arg Pro Pro Pro Gln Pro Arg Ser
   50 55 60
   Thr Thr Phe Trp Ala Trp Ser Val Leu Arg Val Pro Ala Pro Pro Ser
   65 70 75 80
   Pro Gln Pro Ala Thr Tyr Thr Cys Val Val Ser His Glu Asp Ser Arg
   85 90 95
   Thr Leu Leu Asn Ala Ser Arg Ser Leu Glu Val Ser Tyr Val Thr Asp
   100 105 110
   His Gly Pro Met Lys
   115
<210> 207
   <211> 108
   <212> PRT
   <213> Homo sapiens
<400> 207
   Asp Ser Asn Pro Arg Gly Val Ser Ala Tyr Leu Ser Arg Pro Ser Pro
   1 5 10 15
   Phe Asp Leu Phe Ile Arg Lys Ser Pro Thr Ile Thr Cys Leu Val Val
   20 25 30
   Asp Leu Ala Pro Ser Lys Gly Thr Val Asn Leu Thr Trp Ser Arg Ala
   35 40 45
   Ser Gly Lys Pro Val Asn His Ser Thr Arg Lys Glu Glu Lys Gln Arg
   50 55 60
   Asn Gly Thr Leu Thr Val Thr Ser Thr Leu Pro Val Gly Thr Arg Asp
   65                  70                  75                  80
   Trp Ile Glu Gly Glu Thr Tyr Gln Cys Arg Val Thr His Pro His Leu
                   85                  90                  95
   Pro Arg Ala Leu Met Arg Ser Thr Thr Lys Thr Ser
               100                 105
<210> 208
   <211> 107
   <212> PRT
   <213> Homo sapiens
<400> 208
   Gly Gln Pro Arg Glu Pro Gln Val Tyr Thr Leu Pro Pro Ser Arg Glu
    1               5                  10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Met Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 209
   <211> 107
   <212> PRT
   <213> Homo sapiens
<400> 209
   Gly Gln Pro Arg Glu Pro Gln Val Tyr Thr Leu Pro Pro Ser Arg Glu
    1               5                  10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Ser Gly Gln Pro Glu
           35                  40                  45
   Asn Asn Tyr Asn Thr Thr Pro Pro Met Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Ile Phe Ser Cys Ser Val Met His Glu Ala Leu His Asn Arg Phe
                   85                  90                  95
   Thr Gln Lys Ser Leu Ser Leu Ser Pro Gly Lys
               100                 105
<210> 210
   <211> 107
   <212> PRT
   <213> Homo sapiens
<400> 210
   Gly Gln Pro Arg Glu Pro Gln Val Tyr Thr Leu Pro Pro Ser Gln Glu
    1               5                  10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Arg Leu Thr Val Asp Lys Ser Arg Trp Gln Glu Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Leu Gly Lys
               100                 105
<210> 211
   <211> 7
   <212> PRT
   <213> Homo sapiens
<400> 211
   Glu Arg Lys Cys Cys Val Glu
    1               5
<210> 212
   <211> 106
   <212> PRT
   <213> Homo sapiens
<400> 212
   Asp Gln Asp Thr Ala Ile Arg Val Phe Ala Ile Pro Pro Ser Phe Ala
    1               5                  10                  15
   Ser Ile Phe Leu Thr Lys Ser Thr Lys Leu Thr Cys Leu Val Thr Asp
               20                  25                  30
   Leu Thr Thr Tyr Asp Ser Val Thr Ile Ser Trp Thr Arg Gln Asn Gly
           35                  40                  45
   Glu Ala Val Lys Thr His Thr Asn Ile Ser Glu Ser His Pro Asn Ala
       50                  55                  60
   Thr Phe Ser Ala Val Gly Glu Ala Ser Ile Cys Glu Asp Asp Trp Asn
   65                  70                  75                  80
   Ser Gly Glu Arg Phe Thr Cys Thr Val Thr His Thr Asp Leu Pro Ser
                   85                  90                  95
   Pro Leu Lys Gln Thr Ile Ser Arg Pro Lys
               100                 105
<210> 213
   <211> 110
   <212> PRT
   <213> Homo sapiens
<400> 213
   Gly Pro Arg Ala Ala Pro Glu Val Tyr Ala Phe Ala Thr Pro Glu Trp
    1               5                  10                  15
   Pro Gly Ser Arg Asp Lys Arg Thr Leu Ala Cys Leu Ile Gln Asn Phe
               20                  25                  30
   Met Pro Glu Asp Ile Ser Val Gln Trp Leu His Asn Glu Val Gln Leu
           35                  40                  45
   Pro Asp Ala Arg His Ser Thr Thr Gln Pro Arg Lys Thr Lys Gly Ser
       50                  55                  60
   Gly Phe Phe Val Phe Ser Arg Leu Glu Val Thr Arg Ala Glu Trp Glu
   65                  70                  75                  80
   Gln Lys Asp Glu Phe Ile Cys Arg Ala Val His Glu Ala Ala Ser Pro
                   85                  90                  95
   Ser Gln Thr Val Gln Arg Ala Val Ser Val Asn Pro Gly Lys
               100                 105                 110
<210> 214
   <211> 131
   <212> PRT
   <213> Homo sapiens
<400> 214
   Gly Val Ala Leu His Arg Pro Asp Val Tyr Leu Leu Pro Pro Ala Arg
    1               5                  10                  15
   Glu Gln Leu Asn Leu Arg Glu Ser Ala Thr Ile Thr Cys Leu Val Thr
               20                  25                  30
   Gly Phe Ser Pro Ala Asp Val Phe Val Gln Trp Met Gln Arg Gly Gln
           35                  40                  45
   Pro Leu Ser Pro Glu Lys Tyr Val Thr Ser Ala Pro Met Pro Glu Pro
       50                  55                  60
   Gln Ala Pro Gly Arg Tyr Phe Ala His Ser Ile Leu Thr Val Ser Glu
   65                  70                  75                  80
   Glu Glu Trp Asn Thr Gly Glu Thr Tyr Thr Cys Val Val Ala His Glu
                   85                  90                  95
   Ala Leu Pro Asn Arg Val Thr Glu Arg Thr Val Asp Lys Ser Thr Gly
               100                 105                 110
   Lys Pro Thr Leu Tyr Asn Val Ser Leu Val Met Ser Asp Thr Ala Gly
           115                 120                 125
   Thr Cys Tyr
       130
<210> 215
   <211> 19
   <212> PRT
   <213> Homo sapiens
<400> 215
   Val Pro Ser Thr Pro Pro Thr Pro Ser Pro Ser Thr Pro Pro Thr Pro
    1               5                  10                  15
   Ser Pro Ser
<210> 216
   <211> 6
   <212> PRT
   <213> Homo sapiens
<400> 216
   Val Pro Pro Pro Pro Pro
   1 5
<210> 217
   <211> 58
   <212> PRT
   <213> Homo sapiens
<400> 217
   Glu Ser Pro Lys Ala Gln Ala Ser Ser Val Pro Thr Ala Gln Pro Gln
   1 5 10 15
   Ala Glu Gly Ser Leu Ala Lys Ala Thr Thr Ala Pro Ala Thr Thr Arg
   20 25 30
   Asn Thr Gly Arg Gly Gly Glu Glu Lys Lys Lys Glu Lys Glu Lys Glu
   35 40 45
   Glu Gln Glu Glu Arg Glu Thr Lys Thr Pro
   50 55
<210> 218
<400> 218
   000
<210> 219
   <211> 12
   <212> PRT
   <213> Homo sapiens
<400> 219
   Glu Arg Lys Cys Cys Val Glu Cys Pro Pro Cys Pro
   1 5 10
<210> 220
   <211> 62
   <212> PRT
   <213> Homo sapiens
<400> 220
   Glu Leu Lys Thr Pro Leu Gly Asp Thr Thr His Thr Cys Pro Arg Cys
   1 5 10 15
   Pro Glu Pro Lys Ser Cys Asp Thr Pro Pro Pro Cys Pro Arg Cys Pro
   20 25 30
   Glu Pro Lys Ser Cys Asp Thr Pro Pro Pro Cys Pro Arg Cys Pro Glu
   35 40 45
   Pro Lys Ser Cys Asp Thr Pro Pro Pro Cys Pro Arg Cys Pro
   50 55 60
<210> 221
   <211> 12
   <212> PRT
   <213> Homo sapiens
<400> 221
   Glu Ser Lys Tyr Gly Pro Pro Cys Pro Ser Cys Pro
    1               5                  10
<210> 222
   <211> 34
   <212> PRT
   <213> Homo sapiens
<400> 222
   Glu Ser Pro Lys Ala Gln Ala Ser Ser Val Pro Thr Ala Gln Pro Gln
    1               5                  10                  15
   Ala Glu Gly Ser Leu Ala Lys Ala Thr Thr Ala Pro Ala Thr Thr Arg
               20                  25                  30
   Asn Thr
<210> 223
   <211> 106
   <212> PRT
   <213> Homo sapiens
<400> 223
   Gly Gln Pro Lys Ala Ala Pro Ser Val Thr Leu Phe Pro Pro Ser Ser
    1               5                  10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Thr Glu Cys Ser
               100                 105
<210> 224
   <211> 106
   <212> PRT
   <213> Homo sapiens
<400> 224
   Gly Gln Pro Lys Ala Ala Pro Ser Val Thr Leu Phe Pro Pro Ser Ser
    1               5                  10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Lys Ser Tyr Ser Cys Gln Val Thr His Glu Gly Ser Thr Val
                   85                  90                  95
   Glu Lys Thr Val Ala Pro Thr Glu Cys Ser
               100                 105
<210> 225
   <211> 106
   <212> PRT
   <213> Homo sapiens
<400> 225
   Gly Gln Pro Lys Ala Ala Pro Ser Val Thr Leu Phe Pro Pro Ser Ser
    1               5                  10                  15
   Glu Glu Leu Gln Ala Asn Lys Ala Thr Leu Val Cys Leu Ile Ser Asp
               20                  25                  30
   Phe Tyr Pro Gly Ala Val Lys Val Ala Trp Lys Ala Asp Gly Ser Pro
           35                  40                  45
   Val Asn Thr Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Ala Glu Cys Ser
               100                 105
<210> 226
   <211> 106
   <212> PRT
   <213> Homo sapiens
<400> 226
   Gly Gln Pro Lys Ala Ala Pro Ser Val Thr Leu Phe Pro Pro Ser Ser
    1               5                  10                  15
   Glu Glu Leu Gln Ala Asn Lys Ala Thr Leu Val Cys Leu Val Ser Asp
               20                  25                  30
   Phe Tyr Pro Gly Ala Val Thr Val Ala Trp Lys Ala Asp Gly Ser Pro
           35                  40                  45
   Val Lys Val Gly Val Glu Thr Thr Lys Pro Ser Lys Gln Ser Asn Asn
   50 55 60
   Lys Tyr Ala Ala Ser Ser Tyr Leu Ser Leu Thr Pro Glu Gln Trp Lys
   65 70 75 80
   Ser His Arg Ser Tyr Ser Cys Arg Val Thr His Glu Gly Ser Thr Val
   85 90 95
   Glu Lys Thr Val Ala Pro Ala Glu Cys Ser
   100 105
<210> 227
   <211> 10
   <212> PRT
   <213> Homo sapiens
<400> 227
   Glu Pro Lys Ser Cys Asp Lys Thr His Thr
   1 5 10
<210> 228
   <211> 5
   <212> PRT
   <213> Homo sapiens
<400> 228
   Cys Pro Pro Cys Pro
   1 5
<210> 229
<400> 229
   000
<210> 230
<400> 230
   000
<210> 231
<400> 231
   000
<210> 232
   <211> 15
   <212> PRT
   <213> Artificial Sequence
<220>
   <223> Altered human IgG1 hinge (CSC-P)
<400> 232
   Glu Pro Lys Ser Cys Asp Lys Thr His Thr Ser Pro Pro Cys Pro
   1 5 10 15
<210> 233
<400> 233
   000
<210> 234
   <211> 15
   <212> PRT
   <213> Artificial Sequence
<220>
   <223> Altered human IgG1 hinge (CCS-P)
<400> 234
   Glu Pro Lys Ser Cys Asp Lys Thr His Thr Cys Pro Pro Ser Pro
    1               5                  10                  15
<210> 235
<400> 235
   000
<210> 236
<400> 236
   000
<210> 237
<400> 237
   000
<210> 238
<400> 238
   000
<210> 239
<400> 239
   000
<210> 240
   <211> 15
   <212> PRT
   <213> Artificial Sequence
<220>
   <223> Altered human IgG1 hinge (CCS-S)
<400> 240
   Glu Pro Lys Ser Cys Asp Lys Thr His Thr Cys Pro Pro Ser Ser
    1               5                  10                  15
<210> 241
   <211> 12
   <212> PRT
   <213> Artificial Sequence
<220>
   <223> Stalk region of CD69
<400> 241
   Gln Tyr Asn Cys Pro Gly Gln Tyr Thr Phe Ser Met
   1               5                   10
<210> 242
   <211> 11
   <212> PRT
   <213> Artificial Sequence
<220>
   <223> Stalk region of CD72
<400> 242
   Pro Phe Phe Thr Cys Gly Ser Ala Asp Thr Cys
   1               5                   10
<210> 243
   <211> 18
   <212> PRT
   <213> Artificial Sequence
<220>
   <223> Stalk region of CD94
<400> 243
   Glu Pro Ala Phe Thr Pro Gly Pro Asn Ile Glu Leu Gln Lys Asp Ser
   1               5                   10                  15
   Asp Cys
<210> 244
   <211> 18
   <212> PRT
   <213> Artificial Sequence
<220>
   <223> Stalk region of NKG2A
<400> 244
   Gln Arg His Asn Asn Ser Ser Leu Asn Thr Arg Thr Gln Lys Ala Arg
   1               5                   10                  15
   His Cys
<210> 245
   <211> 12
   <212> PRT
   <213> Homo sapiens
<400> 245
   Glu Leu Lys Thr Pro Leu Gly Asp Thr Thr His Thr
   1 5 10
<210> 246
   <211> 10
   <212> PRT
   <213> Homo sapiens
<400> 246
   Glu Pro Lys Ser Cys Asp Thr Pro Pro Pro
   1 5 10
<210> 247
   <211> 7
   <212> PRT
   <213> Homo sapiens
<400> 247
   Glu Ser Lys Tyr Gly Pro Pro
   1 5
<210> 248
   <211> 5
   <212> PRT
   <213> Homo sapiens
<400> 248
   Cys Pro Arg Cys Pro
   1 5
<210> 249
   <211> 5
   <212> PRT
   <213> Homo sapiens
<400> 249
   Cys Pro Ser Cys Pro
   1 5
<210> 250
   <211> 17
   <212> PRT
   <213> Homo sapiens
<400> 250
   Glu Leu Lys Thr Pro Leu Gly Asp Thr Thr His Thr Cys Pro Arg Cys
   1 5 10 15
   Pro
<210> 251
   <211> 15
   <212> PRT
   <213> Homo sapiens
<400> 251
   Glu Pro Lys Ser Cys Asp Thr Pro Pro Pro Cys Pro Arg Cys Pro
    1               5                  10                  15
<210> 252
   <211> 24
   <212> PRT
   <213> Homo sapiens
<400> 252
   Gly Arg Gly Gly Glu Glu Lys Lys Lys Glu Lys Glu Lys Glu Glu Gln
    1               5                  10                  15
   Glu Glu Arg Glu Thr Lys Thr Pro
               20
<210> 253
   <211> 107
   <212> PRT
   <213> Homo sapiens
<400> 253
   Val Cys Ser Arg Asp Phe Thr Pro Pro Thr Val Lys Ile Leu Gln Ser
    1               5                  10                  15
   Ser Ser Asp Gly Gly Gly His Phe Pro Pro Thr Ile Gln Leu Leu Cys
               20                  25                  30
   Leu Val Ser Gly Tyr Thr Pro Gly Thr Ile Asn Ile Thr Trp Leu Glu
           35                  40                  45
   Asp Gly Gln Val Met Asp Val Asp Leu Ser Thr Ala Ser Thr Thr Gln
       50                  55                  60
   Glu Gly Glu Leu Ala Ser Thr Gln Ser Glu Leu Thr Leu Ser Gln Lys
   65                  70                  75                  80
   His Trp Leu Ser Asp Arg Thr Tyr Thr Cys Gln Val Thr Tyr Gln Gly
                   85                  90                  95
   His Thr Phe Glu Asp Ser Thr Lys Lys Cys Ala
               100                 105
<210> 254
   <211> 112
   <212> PRT
   <213> Homo sapiens
<400> 254
   Val Ile Ala Glu Leu Pro Pro Lys Val Ser Val Phe Val Pro Pro Arg
    1               5                  10                  15
   Asp Gly Phe Phe Gly Asn Pro Arg Lys Ser Lys Leu Ile Cys Gln Ala
               20                  25                  30
   Thr Gly Phe Ser Pro Arg Gln Ile Gln Val Ser Trp Leu Arg Glu Gly
           35                  40                  45
   Lys Gln Val Gly Ser Gly Val Thr Thr Asp Gln Val Gln Ala Glu Ala
       50                  55                  60
   Lys Glu Ser Gly Pro Thr Thr Tyr Lys Val Thr Ser Thr Leu Thr Ile
   65                  70                  75                  80
   Lys Glu Ser Asp Trp Leu Gly Gln Ser Met Phe Thr Cys Arg Val Asp
                   85                  90                  95
   His Arg Gly Leu Thr Phe Gln Gln Asn Ala Ser Ser Met Cys Val Pro
               100                 105                 110
<210> 255
   <211> 107
   <212> PRT
   <213> Artificial Sequence
<220>
   <223> Altered human CH2 upper hinge like sequence of IgE
<400> 255
   Val Cys Ser Arg Asp Phe Thr Pro Pro Thr Val Lys Ile Leu Gln Ser
    1               5                  10                  15
   Ser Ser Asp Gly Gly Gly His Phe Pro Pro Thr Ile Gln Leu Leu Cys
               20                  25                  30
   Leu Val Ser Gly Tyr Thr Pro Gly Thr Ile Asn Ile Thr Trp Leu Glu
           35                  40                  45
   Asp Gly Gln Val Met Asp Val Asp Leu Ser Thr Ala Ser Thr Thr Gln
       50                  55                  60
   Glu Gly Glu Leu Ala Ser Thr Gln Ser Glu Leu Thr Leu Ser Gln Lys
   65                  70                  75                  80
   His Trp Leu Ser Asp Arg Thr Tyr Thr Cys Gln Val Thr Tyr Gln Gly
                   85                  90                  95
   His Thr Phe Glu Asp Ser Thr Lys Lys Ser Ala
               100                 105
<210> 256
   <211> 268
   <212> PRT
   <213> Artificial Sequence
<220>
   <223> 5D5 scFv (VLVH)
<220>
   <221> SIGNAL
   <222> (10)...(20)
<400> 256
   Met Glu Ala Pro Ala Gln Leu Leu Phe Leu Leu Leu Leu Trp Leu Pro
   -20                 -15                 -10                 -5
   Asp Thr Thr Gly Asp Phe Met Met Ser Gln Ser Pro Ser Ser Leu Thr
                   1               5                   10
   Val Ser Val Gly Glu Lys Val Thr Val Ser Cys Lys Ser Ser Gln Ser
           15                  20                  25
   Leu Leu Tyr Thr Ser Ser Gln Lys Asn Tyr Leu Ala Trp Tyr Gln Gln
       30                  35                  40
   Lys Pro Gly Gln Ser Pro Lys Leu Leu Ile Tyr Trp Ala Ser Thr Arg
   45                  50                  55                  60
   Glu Ser Gly Val Pro Asp Arg Phe Thr Gly Ser Gly Ser Gly Thr Asp
                   65                  70                  75
   Phe Thr Leu Thr Ile Thr Ser Val Lys Ala Asp Asp Leu Ala Val Tyr
               80                  85                  90
   Tyr Cys Gln Gln Tyr Tyr Ala Tyr Pro Trp Thr Phe Gly Gly Gly Thr
           95                  100                 105
   Lys Leu Glu Leu Lys Gly Gly Gly Gly Ser Gly Gly Gly Gly Ser Gly
       110                 115                 120
   Gly Gly Gly Ser Gln Val Gln Leu Gln Gln Ser Gly Pro Glu Leu Val
   125                 130                 135                 140
   Arg Pro Gly Ala Ser Val Lys Met Ser Cys Arg Ala Ser Gly Tyr Thr
                   145                 150                 155
   Phe Thr Ser Tyr Trp Leu His Trp Val Lys Gln Arg Pro Gly Gln Gly
               160                 165                 170
   Leu Glu Trp Ile Gly Met Ile Asp Pro Ser Asn Ser Asp Thr Arg Phe
           175                 180                 185
   Asn Pro Asn Phe Lys Asp Lys Ala Thr Leu Asn Val Asp Arg Ser Ser
       190                 195                 200
   Asn Thr Ala Tyr Met Leu Leu Ser Ser Leu Thr Ser Ala Asp Ser Ala
   205                 210                 215                 220
   Val Tyr Tyr Cys Ala Thr Tyr Gly Ser Tyr Val Ser Pro Leu Asp Tyr
                   225                 230                 235
   Trp Gly Gln Gly Thr Ser Val Thr Val Ser Ser Ser
               240                 245
<210> 257
   <211> 248
   <212> PRT
   <213> Artificial Sequence
<220>
   <223> 5D5 scFv (VLVH) mature protein without the signal peptide
<400> 257
   Asp Phe Met Met Ser Gln Ser Pro Ser Ser Leu Thr Val Ser Val Gly
    1              5                   10                  15
   Glu Lys Val Thr Val Ser Cys Lys Ser Ser Gln Ser Leu Leu Tyr Thr
               20                  25                  30
   Ser Ser Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Thr Ser Val Lys Ala Asp Asp Leu Ala Val Tyr Tyr Cys Gln Gln
                   85                  90                  95
   Tyr Tyr Ala Tyr Pro Trp Thr Phe Gly Gly Gly Thr Lys Leu Glu Leu
               100                 105                 110
   Lys Gly Gly Gly Gly Ser Gly Gly Gly Gly Ser Gly Gly Gly Gly Ser
           115                 120                 125
   Gln Val Gln Leu Gln Gln Ser Gly Pro Glu Leu Val Arg Pro Gly Ala
       130                 135                 140
   Ser Val Lys Met Ser Cys Arg Ala Ser Gly Tyr Thr Phe Thr Ser Tyr
   145                 150                 155                 160
   Trp Leu His Trp Val Lys Gln Arg Pro Gly Gln Gly Leu Glu Trp Ile
                   165                 170                 175
   Gly Met Ile Asp Pro Ser Asn Ser Asp Thr Arg Phe Asn Pro Asn Phe
               180                 185                 190
   Lys Asp Lys Ala Thr Leu Asn Val Asp Arg Ser Ser Asn Thr Ala Tyr
           195                 200                 205
   Met Leu Leu Ser Ser Leu Thr Ser Ala Asp Ser Ala Val Tyr Tyr Cys
       210                 215                 220
   Ala Thr Tyr Gly Ser Tyr Val Ser Pro Leu Asp Tyr Trp Gly Gln Gly
   225                 230                 235                 240
   Thr Ser Val Thr Val Ser Ser Ser
                   245
<210> 258
   <211> 120
   <212> PRT
   <213> Artificial Sequence
<220>
   <223> 5D5 VH
<400> 258
   Gln Val Gln Leu Gln Gln Ser Gly Pro Glu Leu Val Arg Pro Gly Ala
   1               5                   10                  15
   Ser Val Lys Met Ser Cys Arg Ala Ser Gly Tyr Thr Phe Thr Ser Tyr
               20                  25                  30
   Trp Leu His Trp Val Lys Gln Arg Pro Gly Gln Gly Leu Glu Trp Ile
           35                  40                  45
   Gly Met Ile Asp Pro Ser Asn Ser Asp Thr Arg Phe Asn Pro Asn Phe
       50                  55                  60
   Lys Asp Lys Ala Thr Leu Asn Val Asp Arg Ser Ser Asn Thr Ala Tyr
   65                  70                  75                  80
   Met Leu Leu Ser Ser Leu Thr Ser Ala Asp Ser Ala Val Tyr Tyr Cys
                   85                  90                  95
   Ala Thr Tyr Gly Ser Tyr Val Ser Pro Leu Asp Tyr Trp Gly Gln Gly
               100                 105                 110
   Thr Ser Val Thr Val Ser Ser Ser
           115                 120
<210> 259
   <211> 113
   <212> PRT
   <213> Artificial Sequence
<220>
   <223> 5D5 VL
<400> 259
   Asp Phe Met Met Ser Gln Ser Pro Ser Ser Leu Thr Val Ser Val Gly
    1               5                  10                  15
   Glu Lys Val Thr Val Ser Cys Lys Ser Ser Gln Ser Leu Leu Tyr Thr
               20                  25                  30
   Ser Ser Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Thr Ser Val Lys Ala Asp Asp Leu Ala Val Tyr Tyr Cys Gln Gln
                   85                  90                  95
   Tyr Tyr Ala Tyr Pro Trp Thr Phe Gly Gly Gly Thr Lys Leu Glu Leu
               100                 105                 110
   Lys
<210> 260
   <211> 792
   <212> DNA
   <213> Artificial Sequence
<220>
   <223> 4C04 anti-Ron scFv with signal peptide and junction amino acids SSS at carboxy-terminus
<400> 260
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gc                                                     792
<210> 261
   <211> 244
   <212> PRT
   <213> Artificial Sequence
<220>
   <223> 4C04 anti-Ron scFv with signal peptide and junction amino acids SSS at carboxy-terminus
<400> 261
   Asp Ile Gln Met Thr Gln Ser Pro Ala Ser Gln Ser Ala Ser Leu Gly
    1               5                   10                  15
   Glu Ser Val Thr Ile Thr Cys Leu Ala Ser Gln Thr Ile Gly Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Ser Leu Ala Asp Gly Val Pro Ser Arg Phe Ser Ala
       50                  55                  60
   Ser Gly Ser Gly Thr Lys Phe Ser Phe Lys Ile Asp Ser Leu Gln Ser
   65                  70                  75                  80
   Glu Asp Phe Val Ser Tyr Tyr Cys Gln Gln Leu Tyr Asn Thr Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Ile Gln Leu Gln Gln
           115                 120                 125
   Ser Gly Pro Glu Leu Val Lys Pro Gly Thr Ser Val Lys Ile Ser Cys
       130                 135                 140
   Lys Ala Ser Asp Tyr Thr Leu Thr Asp Phe Tyr Met Asn Trp Val Arg
   145                 150                 155                 160
   Gln Lys Pro Gly Gln Gly Leu Glu Trp Ile Gly Arg Ile Tyr Pro Gly
                   165                 170                 175
   Thr Asp Lys Thr Arg Tyr Asn Glu Lys Phe Arg Asp Lys Ala Ile Leu
               180                 185                 190
   Thr Val Asp Thr Ser Ser Ser Thr Ala Tyr Met Gln Leu Ser Ser Leu
           195                 200                 205
   Thr Ser Glu Asp Thr Ala Val Tyr Phe Cys Ala Arg Ser Ala Tyr Tyr
       210                 215                 220
   Gly Asn Tyr Val Ala Met Asp Tyr Trp Gly Gln Gly Thr Ser Val Thr
   225                 230                 235                 240
   Val Ser Ser Ser
<210> 262
   <211> 2115
   <212> DNA
   <213> Artificial Sequence
<220>
   <223> 4C04 anti-Ron single chain polypeptide (4C04 scFv-CH1-CH2-CH3-Ck with signal peptide)
<400> 262
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gcgcctccac caagggccca tcggtcttcc ccctggcacc ctcctccaag 840
   agcacctctg ggggcacagc ggccctgggc tgcctggtca aggactactt ccccgagccg 900
   gtgacggtgt cgtggaactc aggcgccctg accagcggcg tgcacacctt cccggctgtc 960
   ctacagtcct caggactcta ctccctcagc agcgtggtga ccgtgccctc cagcagcttg 1020
   ggcacccaga cctacatctg caacgtgaat cacaagccca gcaacaccaa ggtggacaag 1080
   aaagttcgta cggagcccaa atcttctgac aaaactcaca catgcccacc gtgcccatcc 1140
   ggagcacctg aactcctggg tggaccgtca gtcttcctct tccccccaaa acccaaggac 1200
   accctcatga tctcccggac ccctgaggtc acatgcgtgg tggtggacgt gagccacgaa 1260
   gaccctgagg tcaagttcaa ctggtacgtg gacggcgtgg aggtgcataa tgccaagaca 1320
   aagccgcggg aggagcagta caacagcacg taccgtgtgg tcagcgtcct caccgtcctg 1380
   caccaggact ggctgaatgg caaggagtac aagtgcaagg tctccaacaa agccctccca 1440
   gcccccatcg agaaaaccat ctccaaagcc aaagggcagc cccgagaacc acaggtgtac 1500
   accctgcccc catcccggga tgagctgacc aagaaccagg tcagcctgac ctgcctggtc 1560
   aaaggcttct atccaagcga catcgccgtg gagtgggaga gcaatgggca gccggagaac 1620
   aactacaaga ccacgcctcc cgtgctggac tccgacggct ccttcttcct ctacagcaag 1680
   ctcaccgtgg acaagagcag gtggcagcag gggaacgtct tctcatgctc cgtgatgcat 1740
   gaggctctgc acaaccacta cacgcagaag agcctctccc tgtctccggg taaatctaga 1800
   actgtggctg caccatctgt cttcatcttc ccgccatctg atgagcagtt gaaatctgga 1860
   actgcctctg ttgtgtgcct gctgaataac ttctatccca gagaggccaa agtacagtgg 1920
   aaggtggata acgccctcca atcgggtaac tcccaggaga gtgtcacaga gcaggacagc 1980
   aaggacagca cctacagcct cagcagcacc ctgacgctga gcaaagcaga ctacgagaaa 2040
   cacaaagtct acgcctgcga agtcacccat cagggcctga gctcgcccgt cacaaagagc 2100
   ttcaacaggg gagag                                                  2115
<210> 263
<400> 263
   000
<210> 264
<400> 264
   000
<210> 265
   <211> 240
   <212> PRT
   <213> Artificial Sequence
<220>
   <223> 11H09 anti-RON scFv mature protein without the signal peptide but with junction amino acids SSS at carboxy-terminus
<400> 265
   Asp Ile Gln Met Thr Gln Ser Pro Ser Ser Leu Ser Ala Ser Leu Gly
    1               5                  10                  15
   Glu Arg Val Ser Leu Thr Cys Arg Ala Ser Gln Asp Ile Gly Ser Ser
               20                  25                  30
   Leu Ser Trp Leu Gln Gln Glu Pro Asp Gly Thr Ile Lys Arg Leu Ile
           35                  40                  45
   Tyr Ser Thr Ser Ser Leu Asp Ser Gly Val Pro Lys Arg Phe Ser Gly
       50                  55                  60
   Ser Arg Ser Gly Ser Asp Tyr Ser Leu Thr Ile Ser Ser Leu Glu Ser
   65                  70                  75                  80
   Glu Asp Phe Val Asp Tyr Tyr Cys Leu Gln Tyr Ala Ser Ser Pro Trp
                   85                  90                  95
   Thr Phe Gly Gly Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Gln
           115                 120                 125
   Ser Gly Ala Glu Leu Ala Lys Pro Gly Ala Ser Val Lys Met Ser Cys
       130                 135                 140
   Lys Ser Ser Gly Tyr Thr Phe Thr Ser Tyr Trp Ile His Trp Val Lys
   145                 150                 155                 160
   Glu Arg Pro Gly Gln Gly Leu Glu Trp Ile Gly Tyr Ile Tyr Pro Thr
                   165                 170                 175
   Thr Gly Tyr Thr Glu Ser Asn Gln Lys Phe Lys Asp Lys Ala Thr Leu
               180                 185                 190
   Thr Ala Asp Lys Ser Ser Ser Thr Ala Tyr Met Gln Leu Ser Ser Leu
           195                 200                 205
   Thr Ser Glu Asp Ser Ala Val Tyr Tyr Cys Phe Leu Leu Arg Pro Trp
       210                 215                 220
   Tyr Phe Asp Val Trp Gly Ala Gly Thr Thr Val Thr Val Ser Ser Ser
   225                 230                 235                 240
<210> 266
<400> 266
   000
<210> 267
<400> 267
   000
<210> 268
<400> 268
   000
<210> 269
<400> 269
   000
<210> 270
<400> 270
   000
<210> 271
<400> 271
   000
<210> 272
<400> 272
   000
<210> 273
<400> 273
   000
<210> 274
<400> 274
   000
<210> 275
<400> 275
   000
<210> 276
<400> 276
   000
<210> 277
<400> 277
   000
<210> 278
<400> 278
   000
<210> 279
<400> 279
   000
<210> 280
<400> 280
   000
<210> 281
<400> 281
   000
<210> 282
<400> 282
   000
<210> 283
<400> 283
   000
<210> 284
<400> 284
   000
<210> 285
<400> 285
   000
<210> 286
<400> 286
   000
<210> 287
<400> 287
   000
<210> 288
<400> 288
   000
<210> 289
<400> 289
   000
<210> 290
<400> 290
   000
<210> 291
<400> 291
   000
<210> 292
<400> 292
   000
<210> 293
<400> 293
   000
<210> 294
<400> 294
   000
<210> 295
<400> 295
   000
<210> 296
   <211> 11
   <212> PRT
   <213> Artificial Sequence
<220>
   <223> Anti-c-Met Light Chain Complementarity Determining Region
<400> 296
   Arg Ala Ser Gln Ser Ile Arg Asn Tyr Leu Asn
   1               5                   10
<210> 297
   <211> 7
   <212> PRT
   <213> Artificial Sequence
<220>
   <223> Anti-c-Met Light Chain Complementarity Determining Region
<400> 297
   Ala Ala Ser Ser Leu Gln Ser
   1               5
<210> 298
   <211> 9
   <212> PRT
   <213> Artificial Sequence
<220>
   <223> Anti-c-Met Light Chain Complementarity Determining Region
<400> 298
   Gln Gln Ser Tyr Val Thr Pro Leu Thr
   1               5
<210> 299
<400> 299
   000
<210> 300
<400> 300
   000
<210> 301
   <211> 119
   <212> PRT
   <213> Artificial Sequence
<220>
   <223> OKT3 VH amino acid sequence
<400> 301
   Gln Val Gln Leu Gln Gln Ser Gly Ala Glu Leu Ala Arg Pro Gly Ala
    1               5                   10                  15
   Ser Val Lys Met Ser Cys Lys Ala Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Val Thr Val Ser Ser
           115
<210> 302
   <211> 106
   <212> PRT
   <213> Artificial Sequence
<220>
   <223> OKT3 VL amino acid sequence
<400> 302
<210> 303
   <211> 123
   <212> PRT
   <213> Artificial Sequence
<220>
   <223> BC3 VH amino acid sequence
<400> 303
   Gln Val Gln Leu Gln Gln Ser Ala Ala Glu Leu Ala Arg Pro Gly Ala
    1               5                   10                  15
   Ser Val Lys Met Ser Cys Lys Ala Ser Gly Tyr Thr Phe Thr Arg Tyr
               20                  25                  30
   Thr Met Gln Trp Val Lys Gln Arg Pro Gly Gln Gly Leu Glu Trp Ile
           35                  40                  45
   Gly Tyr Ile Asn Pro Ser Ser Gly Tyr Ile Gly Tyr Ser Gln Lys Phe
       50                  55                  60
   Lys Asp Lys Thr Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Lys Val Tyr Tyr Asp Tyr Asp Val Tyr Ser Met Asp Tyr
               100                 105                 110
   Trp Gly Gln Gly Thr Ser Val Thr Val Ser Ser
           115                 120
<210> 304
   <211> 106
   <212> PRT
   <213> Artificial Sequence
<220>
   <223> BC3 VL amino acid sequence
<400> 304
   Gln Ile Ile Leu Thr Gln Ser Pro Ala Ile Met Ser Ala Ser Pro Gly
    1               5                   10                  15
   Glu Lys Val Thr Met Thr Cys Ser Ala Ser Ser Ser Val Ser Tyr Met
               20                  25                  30
   His Trp Tyr Gln Gln Lys Ser Gly Thr Ser Pro Lys Arg Trp Ile Tyr
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Asn Pro Leu Thr
                   85                  90                  95
   Phe Gly Ala Gly Thr Lys Leu Glu Leu Lys
               100                 105
<210> 305
   <211> 217
   <212> PRT
   <213> Artificial Sequence
<220>
   <223> G1 N297A CH2-CH3 (N297ST - A297ST)
<400> 305
   Ala Pro Glu Leu Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Ala Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
       210                 215
<210> 306
   <211> 216
   <212> PRT
   <213> Artificial Sequence
<220>
   <223> IgG1 AA N297A CH2CH3 (Ala substitutions at 234, 235, 237 and 297; 236 deleted)
<400> 306
   Ala Pro Glu Ala Ala Ala Pro Ser Val Phe Leu Phe Pro Pro Lys Pro
    1               5                  10                  15
   Lys Asp Thr Leu Met Ile Ser Arg Thr Pro Glu Val Thr Cys Val Val
               20                  25                  30
   Val Asp Val Ser His Glu Asp Pro Glu Val Lys Phe Asn Trp Tyr Val
           35                  40                  45
   Asp Gly Val Glu Val His Asn Ala Lys Thr Lys Pro Arg Glu Glu Gln
       50                  55                  60
   Tyr Ala Ser Thr Tyr Arg Val Val Ser Val Leu Thr Val Leu His Gln
   65                  70                  75                  80
   Asp Trp Leu Asn Gly Lys Glu Tyr Lys Cys Lys Val Ser Asn Lys Ala
                   85                  90                  95
   Leu Pro Ala Pro Ile Glu Lys Thr Ile Ser Lys Ala Lys Gly Gln Pro
               100                 105                 110
   Arg Glu Pro Gln Val Tyr Thr Leu Pro Pro Ser Arg Asp Glu Leu Thr
           115                 120                 125
   Lys Asn Gln Val Ser Leu Thr Cys Leu Val Lys Gly Phe Tyr Pro Ser
       130                 135                 140
   Asp Ile Ala Val Glu Trp Glu Ser Asn Gly Gln Pro Glu Asn Asn Tyr
   145                 150                 155                 160
   Lys Thr Thr Pro Pro Val Leu Asp Ser Asp Gly Ser Phe Phe Leu Tyr
                   165                 170                 175
   Ser Lys Leu Thr Val Asp Lys Ser Arg Trp Gln Gln Gly Asn Val Phe
               180                 185                 190
   Ser Cys Ser Val Met His Glu Ala Leu His Asn His Tyr Thr Gln Lys
           195                 200                 205
   Ser Leu Ser Leu Ser Pro Gly Lys
       210                 215
<210> 307
   <211> 216
   <212> PRT
   <213> Artificial Sequence
<220>
   <223> G2 AA N297A CH2CH3 (Ala substitution at 234, 236 and 297)
<400> 307
   Ala Pro Glu Ala Ala Ala Pro Ser Val Phe Leu Phe Pro Pro Lys Pro
    1               5                  10                  15
   Lys Asp Thr Leu Met Ile Ser Arg Thr Pro Glu Val Thr Cys Val Val
               20                  25                  30
   Val Asp Val Ser His Glu Asp Pro Glu Val Gln Phe Asn Trp Tyr Val
           35                  40                  45
   Asp Gly Met Glu Val His Asn Ala Lys Thr Lys Pro Arg Glu Glu Gln
       50                  55                  60
   Phe Ala Ser Thr Phe Arg Val Val Ser Val Leu Thr Val Val His Gln
   65                  70                  75                  80
   Asp Trp Leu Asn Gly Lys Glu Tyr Lys Cys Lys Val Ser Asn Lys Gly
                   85                  90                  95
   Leu Pro Ala Pro Ile Glu Lys Thr Ile Ser Lys Thr Lys Gly Gln Pro
               100                 105                 110
   Arg Glu Pro Gln Val Tyr Thr Leu Pro Pro Ser Arg Glu Glu Met Thr
           115                 120                 125
   Lys Asn Gln Val Ser Leu Thr Cys Leu Val Lys Gly Phe Tyr Pro Ser
       130                 135                 140
   Asp Ile Ala Val Glu Trp Glu Ser Asn Gly Gln Pro Glu Asn Asn Tyr
   145                 150                 155                 160
   Lys Thr Thr Pro Pro Met Leu Asp Ser Asp Gly Ser Phe Phe Leu Tyr
                   165                 170                 175
   Ser Lys Leu Thr Val Asp Lys Ser Arg Trp Gln Gln Gly Asn Val Phe
               180                 185                 190
   Ser Cys Ser Val Met His Glu Ala Leu His Asn His Tyr Thr Gln Lys
           195                 200                 205
   Ser Leu Ser Leu Ser Pro Gly Lys
       210                 215
<210> 308
   <211> 216
   <212> PRT
   <213> Artificial Sequence
<220>
   <223> G4 AA N297A CH2CH3 (Ala substitutions at 234, 235, 237 and 297; 236 deleted)
<400> 308
   Ala Pro Glu Ala Ala Ala Pro Ser Val Phe Leu Phe Pro Pro Lys Pro
   1 5 10 15
   Lys Asp Thr Leu Met Ile Ser Arg Thr Pro Glu Val Thr Cys Val Val
   20 25 30
   Val Asp Val Ser Gln Glu Asp Pro Glu Val Gln Phe Asn Trp Tyr Val
   35 40 45
   Asp Gly Val Glu Val His Asn Ala Lys Thr Lys Pro Arg Glu Glu Gln
   50 55 60
   Phe Ala Ser Thr Tyr Arg Val Val Ser Val Leu Thr Val Leu His Gln
   65 70 75 80
   Asp Trp Leu Asn Gly Lys Glu Tyr Lys Cys Lys Val Ser Asn Lys Gly
   85 90 95
   Leu Pro Ser Ser Ile Glu Lys Thr Ile Ser Lys Ala Lys Gly Gln Pro
   100 105 110
   Arg Glu Pro Gln Val Tyr Thr Leu Pro Pro Ser Gln Glu Glu Met Thr
   115 120 125
   Lys Asn Gln Val Ser Leu Thr Cys Leu Val Lys Gly Phe Tyr Pro Ser
   130 135 140
   Asp Ile Ala Val Glu Trp Glu Ser Asn Gly Gln Pro Glu Asn Asn Tyr
   145 150 155 160
   Lys Thr Thr Pro Pro Val Leu Asp Ser Asp Gly Ser Phe Phe Leu Tyr
   165 170 175
   Ser Arg Leu Thr Val Asp Lys Ser Arg Trp Gln Glu Gly Asn Val Phe
   180 185 190
   Ser Cys Ser Val Met His Glu Ala Leu His Asn His Tyr Thr Gln Lys
   195 200 205
   Ser Leu Ser Leu Ser Pro Gly Lys
   210 215
<210> 309
   <211> 153
   <212> PRT
   <213> Artificial Sequence
<220>
   <223> DeltaCH2 (glCH3 only)
<400> 309
   Gly Gln Pro Arg Glu Pro Gln Val Tyr Thr Leu Pro Pro Ser Arg Asp
   1 5 10 15
   Glu Leu Thr Lys Asn Gln Val Ser Leu Thr Cys Leu Val Lys Gly Phe
   20 25 30
   Tyr Pro Ser Asp Ile Ala Val Glu Trp Glu Ser Asn Gly Gln Pro Glu
   35 40 45
   Asn Asn Tyr Lys Thr Thr Pro Pro Val Leu Asp Ser Asp Gly Ser Phe
   50 55 60
   Phe Leu Tyr Ser Lys Leu Thr Val Asp Lys Ser Arg Trp Gln Gln Gly
   65 70 75 80
   Asn Val Phe Ser Cys Ser Val Met His Glu Ala Leu His Asn His Tyr
   85 90 95
   Thr Gln Lys Ser Leu Ser Leu Ser Pro Gly Lys Thr Gly Leu Asn Asp
   100 105 110
   Ile Phe Glu Ala Gln Lys Ile Glu Trp His Glu Asp Tyr Lys Asp Asp
   115 120 125
   Asp Asp Lys Asp Tyr Lys Asp Asp Asp Asp Lys Asp Tyr Lys Asp Asp
   130 135 140
   Asp Asp Lys His His His His His His
   145 150
<210> 310
   <211> 246
   <212> PRT
   <213> Artificial Sequence
<220>
   <223> BC3-VH-VL amino acid sequence (includes g4s linker)
<400> 310
   Gln Val Gln Leu Gln Gln Ser Ala Ala Glu Leu Ala Arg Pro Gly Ala
    1               5                  10                  15
   Ser Val Lys Met Ser Cys Lys Ala Ser Gly Tyr Thr Phe Thr Arg Tyr
               20                  25                  30
   Thr Met Gln Trp Val Lys Gln Arg Pro Gly Gln Gly Leu Glu Trp Ile
           35                  40                  45
   Gly Tyr Ile Asn Pro Ser Ser Gly Tyr Ile Gly Tyr Ser Gln Lys Phe
       50                  55                  60
   Lys Asp Lys Thr Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Lys Val Tyr Tyr Asp Tyr Asp Val Tyr Ser Met Asp Tyr
               100                 105                 110
   Trp Gly Gln Gly Thr Ser Val Thr Val Ser Ser Gly Gly Gly Gly Ser
           115                 120                 125
   Gly Gly Gly Gly Ser Gly Gly Gly Gly Ser Ala Gln Gln Ile Ile Leu
       130                 135                 140
   Thr Gln Ser Pro Ala Ile Met Ser Ala Ser Pro Gly Glu Lys Val Thr
   145                 150                 155                 160
   Met Thr Cys Ser Ala Ser Ser Ser Val Ser Tyr Met His Trp Tyr Gln
                   165                 170                 175
   Gln Lys Ser Gly Thr Ser Pro Lys Arg Trp Ile Tyr Asp Thr Ser Lys
               180                 185                 190
   Leu Ala Ser Gly Val Pro Ala Arg Phe Ser Gly Ser Gly Ser Gly Thr
           195                 200                 205
   Ser Tyr Ser Leu Thr Ile Ser Ser Met Glu Ala Glu Asp Ala Ala Thr
       210                 215                 220
   Tyr Tyr Cys Gln Gln Trp Ser Ser Asn Pro Leu Thr Phe Gly Ala Gly
   225                 230                 235                 240
   Thr Lys Leu Glu Leu Lys
                   245
<210> 311
   <211> 242
   <212> PRT
   <213> Artificial Sequence
<220>
   <223> OKT3-VH-VL amino acid sequence (includes g4s linker)
<400> 311
   Gln Val Gln Leu Gln Gln Ser Gly Ala Glu Leu Ala Arg Pro Gly Ala
    1               5                  10                  15
   Ser Val Lys Met Ser Cys Lys Ala Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Val Thr Val Ser Ser Gly Gly Gly Gly Ser Gly Gly Gly Gly
           115                 120                 125
   Ser Gly Gly Gly Gly Ser Ala Gln Gln Ile Val Leu Thr Gln Ser Pro
       130                 135                 140
   Ala Ile Met Ser Ala Ser Pro Gly Glu Lys Val Thr Met Thr Cys Ser
   145                 150                 155                 160
   Ala Ser Ser Ser Val Ser Tyr Met Asn Trp Tyr Gln Gln Lys Ser Gly
                   165                 170                 175
   Thr Ser Pro Lys Arg Trp Ile Tyr Asp Thr Ser Lys Leu Ala Ser Gly
               180                 185                 190
   Val Pro Ala His Phe Arg Gly Ser Gly Ser Gly Thr Ser Tyr Ser Leu
           195                 200                 205
   Thr Ile Ser Gly Met Glu Ala Glu Asp Ala Ala Thr Tyr Tyr Cys Gln
       210                 215                 220
   Gln Trp Ser Ser Asn Pro Phe Thr Phe Gly Ser Gly Thr Lys Leu Glu
   225                 230                 235                 240
   Ile Asn
<210> 312
   <211> 244
   <212> PRT
   <213> Artificial Sequence
<220>
   <223> H57 VH-Linker-VL (without leader)
<400> 312
   Glu Val Tyr Leu Val Glu Ser Gly Gly Asp Leu Val Gln Pro Gly Ser
    1               5                  10                  15
   Ser Leu Lys Val Ser Cys Ala Ala Ser Gly Phe Thr Phe Ser Asp Phe
               20                  25                  30
   Trp Met Tyr Trp Val Arg Gln Ala Pro Gly Lys Gly Leu Glu Trp Val
   35 40 45
   Gly Arg Ile Lys Asn Lys Pro Asn Asn Tyr Ala Thr Glu Tyr Ala Asp
   50 55 60
   Ser Val Arg Gly Arg Phe Thr Ile Ser Arg Asp Asp Ser Arg Asn Ser
   65 70 75 80
   Ile Tyr Leu Gln Met Asn Arg Leu Arg Val Asp Asp Thr Ala Ile Tyr
   85 90 95
   Tyr Cys Thr Arg Ala Gly Arg Phe Asp His Phe Asp Tyr Trp Gly Gln
   100 105 110
   Gly Thr Met Val Thr Val Ser Ser Gly Gly Gly Gly Ser Gly Gly Gly
   115 120 125
   Gly Ser Gly Gly Gly Gly Ser Ala Gln Tyr Glu Leu Ile Gln Pro Ser
   130 135 140
   Ser Ala Ser Val Thr Val Gly Glu Thr Val Lys Ile Thr Cys Ser Gly
   145 150 155 160
   Asp Gln Leu Pro Lys Asn Phe Ala Tyr Trp Phe Gln Gln Lys Ser Asp
   165 170 175
   Lys Asn Ile Leu Leu Leu Ile Tyr Met Asp Asn Lys Arg Pro Ser Gly
   180 185 190
   Ile Pro Glu Arg Phe Ser Gly Ser Thr Ser Gly Thr Thr Ala Thr Leu
   195 200 205
   Thr Ile Ser Gly Ala Gln Pro Glu Asp Glu Ala Ala Tyr Tyr Cys Leu
   210 215 220
   Ser Ser Tyr Gly Asp Asn Asn Asp Leu Val Phe Gly Ser Gly Thr Gln
   225 230 235 240
   Leu Thr Val Leu
<210> 313
   <211> 120
   <212> PRT
   <213> Artificial Sequence
<220>
   <223> H57 Null2 SMIP - VH amino acid
<400> 313
   Glu Val Tyr Leu Val Glu Ser Gly Gly Asp Leu Val Gln Pro Gly Ser
   1 5 10 15
   Ser Leu Lys Val Ser Cys Ala Ala Ser Gly Phe Thr Phe Ser Asp Phe
   20 25 30
   Trp Met Tyr Trp Val Arg Gln Ala Pro Gly Lys Gly Leu Glu Trp Val
   35 40 45
   Gly Arg Ile Lys Asn Lys Pro Asn Asn Tyr Ala Thr Glu Tyr Ala Asp
   50 55 60
   Ser Val Arg Gly Arg Phe Thr Ile Ser Arg Asp Asp Ser Arg Asn Ser
   65 70 75 80
   Ile Tyr Leu Gln Met Asn Arg Leu Arg Val Asp Asp Thr Ala Ile Tyr
   85 90 95
   Tyr Cys Thr Arg Ala Gly Arg Phe Asp His Phe Asp Tyr Trp Gly Gln
   100 105 110
   Gly Thr Met Val Thr Val Ser Ser
   115 120
<210> 314
   <211> 110
   <212> PRT
   <213> Artificial Sequence
<220>
   <223> Mouse IGHG2c mutated CH2 (alanine substitutions at positions L234, L235, G237, E318, K320 and K322)
<400> 314
   Ala Pro Asp Ala Ala Gly Ala Pro Ser Val Phe Ile Phe Pro Pro Lys
    1               5                  10                  15
   Ile Lys Asp Val Leu Met Ile Ser Leu Ser Pro Met Val Thr Cys Val
               20                  25                  30
   Val Val Asp Val Ser Glu Asp Asp Pro Asp Val Gln Ile Ser Trp Phe
           35                  40                  45
   Val Asn Asn Val Glu Val His Thr Ala Gln Thr Gln Thr His Arg Glu
       50                  55                  60
   Asp Tyr Asn Ser Thr Leu Arg Val Val Ser Ala Leu Pro Ile Gln His
   65                  70                  75                  80
   Gln Asp Trp Met Ser Gly Lys Ala Phe Ala Cys Ala Val Asn Asn Arg
                   85                  90                  95
   Ala Leu Pro Ser Pro Ile Glu Lys Thr Ile Ser Lys Pro Arg
               100                 105                 110
<210> 315
   <211> 107
   <212> PRT
   <213> Artificial Sequence
<220>
   <223> H57 Null2 SMIP - VL amino acid sequence
<400> 315
   Tyr Glu Leu Ile Gln Pro Ser Ser Ala Ser Val Thr Val Gly Glu Thr
    1               5                  10                  15
   Val Lys Ile Thr Cys Ser Gly Asp Gln Leu Pro Lys Asn Phe Ala Tyr
               20                  25                  30
   Trp Phe Gln Gln Lys Ser Asp Lys Asn Ile Leu Leu Leu Ile Tyr Met
           35                  40                  45
   Asp Asn Lys Arg Pro Ser Gly Ile Pro Glu Arg Phe Ser Gly Ser Thr
       50                  55                  60
   Ser Gly Thr Thr Ala Thr Leu Thr Ile Ser Gly Ala Gln Pro Glu Asp
   65                  70                  75                  80
   Glu Ala Ala Tyr Tyr Cys Leu Ser Ser Tyr Gly Asp Asn Asn Asp Leu
                   85                  90                  95
   Val Phe Gly Ser Gly Thr Gln Leu Thr Val Leu
               100                 105
<210> 316
<400> 316
   000
<210> 317
   <211> 116
   <212> PRT
   <213> Artificial Sequence
<220>
   <223> 2C11 Null2 SMIP - VH amino acid
<400> 317
   Glu Val Gln Leu Val Glu Ser Gly Gly Gly Leu Val Gln Pro Gly Lys
    1               5                  10                  15
   Ser Leu Lys Leu Ser Cys Glu Ala Ser Gly Phe Thr Phe Ser Gly Tyr
               20                  25                  30
   Gly Met His Trp Val Arg Gln Ala Pro Gly Arg Gly Leu Glu Ser Val
           35                  40                  45
   Ala Tyr Ile Thr Ser Ser Ser Ile Asn Ile Lys Tyr Ala Asp Ala Val
       50                  55                  60
   Lys Gly Arg Phe Thr Val Ser Arg Asp Asn Ala Lys Asn Leu Leu Phe
   65                  70                  75                  80
   Leu Gln Met Asn Ile Leu Lys Ser Glu Asp Thr Ala Met Tyr Tyr Cys
                   85                  90                  95
   Ala Arg Phe Asp Trp Asp Lys Asn Tyr Trp Gly Gln Gly Thr Met Val
               100                 105                 110
   Thr Val Ser Ser
           115
<210> 318
   <211> 107
   <212> PRT
   <213> Artificial Sequence
<220>
   <223> 2C11 Null2 SMIP - VL amino acid
<400> 318
   Asp Ile Gln Met Thr Gln Ser Pro Ser Ser Leu Pro Ala Ser Leu Gly
    1               5                  10                  15
   Asp Arg Val Thr Ile Asn Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Asn Lys Leu Ala Asp Gly Val Pro Ser Arg Phe Ser Gly
       50                  55                  60
   Ser Gly Ser Gly Arg Asp Ser Ser Phe Thr Ile Ser Ser Leu Glu Ser
   65                  70                  75                  80
   Glu Asp Ile Gly Ser Tyr Tyr Cys Gln Gln Tyr Tyr Asn Tyr Pro Trp
                   85                  90                  95
   Thr Phe Gly Pro Gly Thr Lys Leu Glu Ile Lys
               100                 105
<210> 319
   <211> 240
   <212> PRT
   <213> Artificial Sequence
<220>
   <223> 2C11 VH-Linker-VL (without leader)
<400> 319
   Glu Val Gln Leu Val Glu Ser Gly Gly Gly Leu Val Gln Pro Gly Lys
    1               5                  10                  15
   Ser Leu Lys Leu Ser Cys Glu Ala Ser Gly Phe Thr Phe Ser Gly Tyr
               20                  25                  30
   Gly Met His Trp Val Arg Gln Ala Pro Gly Arg Gly Leu Glu Ser Val
           35                  40                  45
   Ala Tyr Ile Thr Ser Ser Ser Ile Asn Ile Lys Tyr Ala Asp Ala Val
       50                  55                  60
   Lys Gly Arg Phe Thr Val Ser Arg Asp Asn Ala Lys Asn Leu Leu Phe
   65                  70                  75                  80
   Leu Gln Met Asn Ile Leu Lys Ser Glu Asp Thr Ala Met Tyr Tyr Cys
                   85                  90                  95
   Ala Arg Phe Asp Trp Asp Lys Asn Tyr Trp Gly Gln Gly Thr Met Val
               100                 105                 110
   Thr Val Ser Ser Gly Gly Gly Gly Ser Gly Gly Gly Gly Ser Gly Gly
           115                 120                 125
   Gly Gly Ser Ala Gln Asp Ile Gln Met Thr Gln Ser Pro Ser Ser Leu
       130                 135                 140
   Pro Ala Ser Leu Gly Asp Arg Val Thr Ile Asn Cys Gln Ala Ser Gln
   145                 150                 155                 160
   Asp Ile Ser Asn Tyr Leu Asn Trp Tyr Gln Gln Lys Pro Gly Lys Ala
                   165                 170                 175
   Pro Lys Leu Leu Ile Tyr Tyr Thr Asn Lys Leu Ala Asp Gly Val Pro
               180                 185                 190
   Ser Arg Phe Ser Gly Ser Gly Ser Gly Arg Asp Ser Ser Phe Thr Ile
           195                 200                 205
   Ser Ser Leu Glu Ser Glu Asp Ile Gly Ser Tyr Tyr Cys Gln Gln Tyr
       210                 215                 220
   Tyr Asn Tyr Pro Trp Thr Phe Gly Pro Gly Thr Lys Leu Glu Ile Lys
   225                 230                 235                 240
<210> 320
   <211> 110
   <212> PRT
   <213> Mus musculus
<220>
   <223> Human IGHG2c CH2 domain
<400> 320
   Ala Pro Asp Leu Leu Gly Gly Pro Ser Val Phe Ile Phe Pro Pro Lys
    1               5                  10                  15
   Ile Lys Asp Val Leu Met Ile Ser Leu Ser Pro Met Val Thr Cys Val
               20                  25                  30
   Val Val Asp Val Ser Glu Asp Asp Pro Asp Val Gln Ile Ser Trp Phe
           35                  40                  45
   Val Asn Asn Val Glu Val His Thr Ala Gln Thr Gln Thr His Arg Glu
       50                  55                  60
   Asp Tyr Asn Ser Thr Leu Arg Val Val Ser Ala Leu Pro Ile Gln His
   65                  70                  75                  80
   Gln Asp Trp Met Ser Gly Lys Glu Phe Lys Cys Lys Val Asn Asn Arg
                   85                  90                  95
   Ala Leu Pro Ser Pro Ile Glu Lys Thr Ile Ser Lys Pro Arg
               100                 105                 110
<210> 321
   <211> 213
   <212> PRT
   <213> Artificial Sequence
<220>
   <223> HM1 (IgM CH3:IgG1 CH3) without C-terminal tail
<400> 321
   Asp Gln Asp Thr Ala Ile Arg Val Phe Ala Ile Pro Pro Ser Phe Ala
   1               5                   10                  15
   Ser Ile Phe Leu Thr Lys Ser Thr Lys Leu Thr Cys Leu Val Thr Asp
               20                  25                  30
   Leu Thr Thr Tyr Asp Ser Val Thr Ile Ser Trp Thr Arg Gln Asn Gly
           35                  40                  45
   Glu Ala Val Lys Thr His Thr Asn Ile Ser Glu Ser His Pro Asn Ala
       50                  55                  60
   Thr Phe Ser Ala Val Gly Glu Ala Ser Ile Cys Glu Asp Asp Trp Asn
   65                  70                  75                  80
   Ser Gly Glu Arg Phe Thr Cys Thr Val Thr His Thr Asp Leu Pro Ser
                   85                  90                  95
   Pro Leu Lys Gln Thr Ile Ser Arg Pro Lys Gly Gln Pro Arg Glu Pro
               100                 105                 110
   Gln Val Tyr Thr Leu Pro Pro Ser Arg Asp Glu Leu Thr Lys Asn Gln
           115                 120                 125
   Val Ser Leu Thr Cys Leu Val Lys Gly Phe Tyr Pro Ser Asp Ile Ala
       130                 135                 140
   Val Glu Trp Glu Ser Asn Gly Gln Pro Glu Asn Asn Tyr Lys Thr Thr
   145                 150                 155                 160
   Pro Pro Val Leu Asp Ser Asp Gly Ser Phe Phe Leu Tyr Ser Lys Leu
                   165                 170                 175
   Thr Val Asp Lys Ser Arg Trp Gln Gln Gly Asn Val Phe Ser Cys Ser
               180                 185                 190
   Val Met His Glu Ala Leu His Asn His Tyr Thr Gln Lys Ser Leu Ser
           195                 200                 205
   Leu Ser Pro Gly Lys
       210
<210> 322
   <211> 109
   <212> PRT
   <213> Artificial Sequence
<220>
   <223> IgG4 AA CH2
<400> 322
   Ala Pro Glu Ala Ala Ala Pro Ser Val Phe Leu Phe Pro Pro Lys Pro
    1              5                  10                  15
   Lys Asp Thr Leu Met Ile Ser Arg Thr Pro Glu Val Thr Cys Val Val
               20                  25                  30
   Val Asp Val Ser Gln Glu Asp Pro Glu Val Gln Phe Asn Trp Tyr Val
           35                  40                  45
   Asp Gly Val Glu Val His Asn Ala Lys Thr Lys Pro Arg Glu Glu Gln
       50                  55                  60
   Phe Ala Ser Thr Tyr Arg Val Val Ser Val Leu Thr Val Leu His Gln
   65                  70                  75                  80
   Asp Trp Leu Asn Gly Lys Glu Tyr Lys Cys Lys Val Ser Asn Lys Gly
                   85                  90                  95
   Leu Pro Ser Ser Ile Glu Lys Thr Ile Ser Lys Ala Lys
               100                 105
<210> 323
   <211> 217
   <212> PRT
   <213> Artificial Sequence
<220>
   <223> Human IgG1 CH2 CH3 Null2
<400> 323
   Ala Pro Glu Ala Ala Gly Ala Pro Ser Val Phe Leu Phe Pro Pro Lys
    1              5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Ala Tyr Ala Cys Ala Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
       210                 215
<210> 324
   <211> 110
   <212> PRT
   <213> Artificial Sequence
<220>
   <223> IgG1 N297A CH2
<400> 324
   Ala Pro Glu Leu Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Ala Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 325
   <211> 109
   <212> PRT
   <213> Artificial Sequence
<220>
   <223> IgG1 AA CH2
<400> 325
   Ala Pro Glu Ala Ala Ala Pro Ser Val Phe Leu Phe Pro Pro Lys Pro
    1               5                  10                  15
   Lys Asp Thr Leu Met Ile Ser Arg Thr Pro Glu Val Thr Cys Val Val
               20                  25                  30
   Val Asp Val Ser His Glu Asp Pro Glu Val Lys Phe Asn Trp Tyr Val
           35                  40                  45
   Asp Gly Val Glu Val His Asn Ala Lys Thr Lys Pro Arg Glu Glu Gln
       50                  55                  60
   Tyr Ala Ser Thr Tyr Arg Val Val Ser Val Leu Thr Val Leu His Gln
   65                  70                  75                  80
   Asp Trp Leu Asn Gly Lys Glu Tyr Lys Cys Lys Val Ser Asn Lys Ala
                   85                  90                  95
   Leu Pro Ala Pro Ile Glu Lys Thr Ile Ser Lys Ala Lys
               100                 105
<210> 326
   <211> 108
   <212> PRT
   <213> Artificial Sequence
<220>
   <223> IgG2 AA CH2
<400> 326
   Ala Pro Glu Ala Ala Ala Pro Ser Val Phe Leu Phe Pro Pro Lys Pro
    1               5                  10                  15
   Lys Asp Thr Leu Met Ile Ser Arg Thr Pro Glu Val Thr Cys Val Val
               20                  25                  30
   Val Asp Val Ser His Glu Asp Pro Glu Val Gln Phe Asn Trp Tyr Val
           35                  40                  45
   Asp Gly Met Glu Val His Asn Ala Lys Thr Lys Pro Arg Glu Glu Gln
       50                  55                  60
   Phe Ala Ser Thr Phe Arg Val Val Ser Val Leu Thr Val Val His Gln
   65                  70                  75                  80
   Asp Trp Leu Asn Gly Lys Glu Tyr Lys Cys Lys Val Ser Asn Lys Gly
                   85                  90                  95
   Leu Pro Ala Pro Ile Glu Lys Thr Ile Ser Lys Thr
               100                 105
<210> 327
   <211> 121
   <212> PRT
   <213> Artificial Sequence
<220>
   <223> Cris7-VH murine hybridoma Amino Acid
<220>
   <221> ACT_SITE
   <222> (31)...(35)
   <223> complementarity-determining region
<220>
   <221> ACT_SITE
   <222> (50)...(66)
   <223> complementarity-determining region
<220>
   <221> ACT_SITE
   <222> (100)...(110)
   <223> complementarity-determining region
<400> 327
   Gln Val Gln Leu Gln Gln Ser Gly Ala Glu Leu Ala Arg Pro Gly Ala
    1               5                  10                  15
   Ser Val Lys Met Ser Cys Lys Ala Ser Gly Tyr Thr Phe Thr Arg Ser
               20                  25                  30
   Thr Met His Trp Val Lys Gln Arg Pro Gly Gln Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Ser Pro Gln Val His Tyr Asp Tyr Asn Gly Phe Pro Tyr Trp Gly
               100                 105                 110
   Gln Gly Thr Leu Val Thr Val Ser Ala
           115                 120
<210> 328
   <211> 107
   <212> PRT
   <213> Artificial Sequence
<220>
   <223> Cris7-VL murine hybridoma Amino Acid
<220>
   <221> ACT_SITE
   <222> (24)...(33)
   <223> complementarity-determining region
<220>
   <221> ACT_SITE
   <222> (49)...(55)
   <223> complementarity-determining region
<220>
   <221> ACT_SITE
   <222> (88)...(96)
   <223> complementarity-determining region
<400> 328
   Gln Val Val Leu Thr Gln Ser Pro Ala Ile Met Ser Ala Phe Pro Gly
    1               5                  10                  15
   Glu Lys Val Thr Met Thr Cys Ser Ala Ser Ser Ser Val Ser Tyr Met
               20                  25                  30
   Asn Trp Tyr Gln Gln Lys Ser Gly Thr Ser Pro Lys Arg Trp Ile Tyr
           35                  40                  45
   Asp Ser Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Thr Glu
   65                  70                  75                  80
   Asp Ala Ala Thr Tyr Tyr Cys Gln Gln Trp Ser Arg Asn Pro Pro Thr
                   85                  90                  95
   Phe Gly Gly Gly Thr Lys Leu Gln Ile Thr Arg
               100                 105
<210> 329
   <211> 107
   <212> PRT
   <213> Artificial Sequence
<220>
   <223> HuCris7 VL.1
<220>
   <221> ACT_SITE
   <222> (24)...(33)
   <223> complementarity-determining region
<220>
   <221> ACT_SITE
   <222> (49)...(55)
   <223> complementarity-determining region
<220>
   <221> ACT_SITE
   <222> (88)...(96)
   <223> complementarity-determining region
<400> 329
   Asp Ile Gln Met Thr Gln Ser Pro Ser Ser Leu Ser Ala Ser Val Gly
    1               5                  10                  15
   Asp Arg Val Thr Met Thr Cys Ser Ala Ser Ser Ser Val Ser Tyr Met
               20                  25                  30
   Asn Trp Tyr Gln Gln Lys Pro Gly Lys Ala Pro Lys Arg Trp Ile Tyr
           35                  40                  45
   Asp Ser Ser Lys Leu Ala Ser Gly Val Pro Ala Arg Phe Ser Gly Ser
       50                  55                  60
   Gly Ser Gly Thr Asp Tyr Thr Leu Thr Ile Ser Ser Leu Gln Pro Glu
   65                  70                  75                  80
   Asp Phe Ala Thr Tyr Tyr Cys Gln Gln Trp Ser Arg Asn Pro Pro Thr
                   85                  90                  95
   Phe Gly Gly Gly Thr Lys Leu Gln Ile Thr Arg
               100                 105
<210> 330
   <211> 107
   <212> PRT
   <213> Artificial Sequence
<220>
   <223> HuCris7 VL.2
<400> 330
   Asp Ile Gln Met Thr Gln Ser Pro Ser Ser Leu Ser Ala Ser Val Gly
    1               5                  10                  15
   Asp Arg Val Thr Ile Thr Cys Ser Ala Ser Ser Ser Val Ser Tyr Met
               20                  25                  30
   Asn Trp Tyr Gln Gln Thr Pro Gly Lys Ala Pro Lys Arg Trp Ile Tyr
           35                  40                  45
   Asp Ser Ser Lys Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Ile Ala Thr Tyr Tyr Cys Gln Gln Trp Ser Arg Asn Pro Pro Thr
                   85                  90                  95
   Phe Gly Gln Gly Thr Lys Leu Gln Ile Thr Arg
               100                 105
<210> 331
   <211> 121
   <212> PRT
   <213> Artificial Sequence
<220>
   <223> HuCris7 VH.1
<220>
   <221> ACT_SITE
   <222> (31)...(35)
   <223> complementarity-determining region
<220>
   <221> ACT_SITE
   <222> (50)...(66)
   <223> complementarity-determining region
<220>
   <221> ACT_SITE
   <222> (100)...(110)
   <223> complementarity-determining region
<400> 331
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Arg Ser
               20                  25                  30
   Thr Met His Trp Val Lys Gln Arg Pro Gly Gln Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Arg Ser Glu Asp Thr Ala Val Tyr Tyr Cys
                   85                  90                  95
   Ala Arg Pro Gln Val His Tyr Asp Tyr Asn Gly Phe Pro Tyr Trp Gly
               100                 105                 110
   Gln Gly Thr Leu Val Thr Val Ser Ser
           115                 120
<210> 332
   <211> 121
   <212> PRT
   <213> Artificial Sequence
<220>
   <223> HuCris7 VH.2
<400> 332
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Lys Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Pro Gln Val His Tyr Asp Tyr Asn Gly Phe Pro Tyr Trp Gly
               100                 105                 110
   Gln Gly Thr Leu Val Thr Val Ser Ser
           115                 120
<210> 333
   <211> 121
   <212> PRT
   <213> Artificial Sequence
<220>
   <223> HuCRIS7 VH.3
<400> 333
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser
           115                 120
<210> 334
   <211> 245
   <212> PRT
   <213> Artificial Sequence
<220>
   <223> Cris7-(VH-VL)
<220>
   <221> SITE
   <222> (122)...(138)
   <223> Linker
<400> 334
   Gln Val Gln Leu Gln Gln Ser Gly Ala Glu Leu Ala Arg Pro Gly Ala
    1               5                  10                  15
   Ser Val Lys Met Ser Cys Lys Ala Ser Gly Tyr Thr Phe Thr Arg Ser
               20                  25                  30
   Thr Met His Trp Val Lys Gln Arg Pro Gly Gln Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Ser Pro Gln Val His Tyr Asp Tyr Asn Gly Phe Pro Tyr Trp Gly
               100                 105                 110
   Gln Gly Thr Leu Val Thr Val Ser Ala Gly Gly Gly Gly Ser Gly Gly
           115                 120                 125
   Gly Gly Ser Gly Gly Gly Gly Ser Ala Gln Gln Val Val Leu Thr Gln
       130                 135                 140
   Ser Pro Ala Ile Met Ser Ala Phe Pro Gly Glu Lys Val Thr Met Thr
   145                 150                 155                 160
   Cys Ser Ala Ser Ser Ser Val Ser Tyr Met Asn Trp Tyr Gln Gln Lys
                   165                 170                 175
   Ser Gly Thr Ser Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Ser Tyr
           195                 200                 205
   Ser Leu Thr Ile Ser Ser Met Glu Thr Glu Asp Ala Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg
                   245
<210> 335
   <211> 245
   <212> PRT
   <213> Artificial Sequence
<220>
   <223> HuCris7-(VH1-VL1)
<220>
   <221> SITE
   <222> (122)...(138)
   <223> Linker
<400> 335
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Arg Ser
               20                  25                  30
   Thr Met His Trp Val Lys Gln Ala Pro Gly Gln Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Arg Ser Glu Asp Thr Ala Val Tyr Tyr Cys
                   85                  90                  95
   Ala Arg Pro Gln Val His Tyr Asp Tyr Asn Gly Phe Pro Tyr Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg
                   245
<210> 336
   <211> 245
   <212> PRT
   <213> Artificial Sequence
<220>
   <223> HuCris7-(VH1-VL2)
<220>
   <221> SITE
   <222> (122)...(138)
   <223> Linker
<400> 336
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Arg Ser
               20                  25                  30
   Thr Met His Trp Val Lys Gln Ala Pro Gly Gln Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Arg Ser Glu Asp Thr Ala Val Tyr Tyr Cys
                           85                  90                  95
   Ala Arg Pro Gln Val His Tyr Asp Tyr Asn Gly Phe Pro Tyr Trp Gly
                   100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Ile Thr
   145                 150                 155                 160
   Cys Ser Ala Ser Ser Ser Val Ser Tyr Met Asn Trp Tyr Gln Gln Thr
                   165                 170                 175
   Pro Gly Lys Ala Pro Lys Arg Trp Ile Tyr Asp Ser Ser Lys Leu Ala
               180                 185                 190
   Ser Gly Val Pro Ser Arg Phe Ser Gly Ser Gly Ser Gly Thr Asp Phe
           195                 200                 205
   Thr Leu Thr Ile Ser Ser Leu Gln Pro Glu Asp Ile Ala Thr Tyr Tyr
       210                 215                 220
   Cys Gln Gln Trp Ser Arg Asn Pro Pro Thr Phe Gly Gln Gly Thr Lys
   225                 230                 235                 240
   Leu Gln Ile Thr Arg
                   245
<210> 337
   <211> 245
   <212> PRT
   <213> Artificial Sequence
<220>
   <223> HuCris7-(VH2-VL1)
<220>
   <221> SITE
   <222> (122)...(138)
   <223> Linker
<400> 337
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Lys Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Pro Gln Val His Tyr Asp Tyr Asn Gly Phe Pro Tyr Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg
                   245
<210> 338
   <211> 245
   <212> PRT
   <213> Artificial Sequence
<220>
   <223> HuCris7-(VH2-V1,2)
<220>
   <221> SITE
   <222> (122)...(138)
   <223> Linker
<400> 338
   Gln Val Gln Leu Val Gln Ser Gly Gly Gly Val Val Gln Pro Gly Arg
    1               5                   10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Lys Ala Thr Leu Thr Ala Asp Lys Ser Lys Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Pro Gln Val His Tyr Asp Tyr Asn Gly Phe Pro Tyr Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Ile Thr
   145                 150                 155                 160
   Cys Ser Ala Ser Ser Ser Val Ser Tyr Met Asn Trp Tyr Gln Gln Thr
                   165                 170                 175
   Pro Gly Lys Ala Pro Lys Arg Trp Ile Tyr Asp Ser Ser Lys Leu Ala
               180                 185                 190
   Ser Gly Val Pro Ser Arg Phe Ser Gly Ser Gly Ser Gly Thr Asp Phe
           195                 200                 205
   Thr Leu Thr Ile Ser Ser Leu Gln Pro Glu Asp Ile Ala Thr Tyr Tyr
       210                 215                 220
   Cys Gln Gln Trp Ser Arg Asn Pro Pro Thr Phe Gly Gln Gly Thr Lys
   225                 230                 235                 240
   Leu Gln Ile Thr Arg
                   245
<210> 339
   <211> 245
   <212> PRT
   <213> Artificial Sequence
<220>
   <223> HuCris7-(VH3-VL1)
<220>
   <221> SITE
   <222> (122)...(138)
   <223> Liniker
<400> 339
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg
                   245
<210> 340
   <211> 245
   <212> PRT
   <213> Artificial Sequence
<220>
   <223> HuCris7-(VH3-V1,2)
<220>
   <221> SITE
   <222> (122)...(138)
   <223> Linker
<400> 340
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Ile Thr
   145                 150                 155                 160
   Cys Ser Ala Ser Ser Ser Val Ser Tyr Met Asn Trp Tyr Gln Gln Thr
                   165                 170                 175
   Pro Gly Lys Ala Pro Lys Arg Trp Ile Tyr Asp Ser Ser Lys Leu Ala
               180                 185                 190
   Ser Gly Val Pro Ser Arg Phe Ser Gly Ser Gly Ser Gly Thr Asp Phe
           195                 200                 205
   Thr Leu Thr Ile Ser Ser Leu Gln Pro Glu Asp Ile Ala Thr Tyr Tyr
       210                 215                 220
   Cys Gln Gln Trp Ser Arg Asn Pro Pro Thr Phe Gly Gln Gly Thr Lys
   225                 230                 235                 240
   Leu Gln Ile Thr Arg
                   245
<210> 341
   <211> 216
   <212> PRT
   <213> Artificial Sequence
<220>
   <223> Half null CH2-CH3 regions
<400> 341
   Pro Asp Ala Ala Gly Ala Pro Ser Val Phe Ile Phe Pro Pro Lys Ile
    1               5                  10                  15
   Lys Asp Val Leu Met Ile Ser Leu Ser Pro Met Val Thr Cys Val Val
               20                  25                  30
   Val Asp Val Ser Glu Asp Asp Pro Asp Val Gln Ile Ser Trp Phe Val
           35                  40                  45
   Asn Asn Val Glu Val His Thr Ala Gln Thr Gln Thr His Arg Glu Asp
       50                  55                  60
   Tyr Ala Ser Thr Leu Arg Val Val Ser Ala Leu Pro Ile Gln His Gln
   65                  70                  75                  80
   Asp Trp Met Ser Gly Lys Glu Phe Lys Cys Lys Val Asn Asn Arg Ala
                   85                  90                  95
   Leu Pro Ser Pro Ile Glu Lys Thr Ile Ser Lys Pro Arg Gly Pro Val
               100                 105                 110
   Arg Ala Pro Gln Val Tyr Val Leu Pro Pro Pro Ala Glu Glu Met Thr
           115                 120                 125
   Lys Lys Glu Phe Ser Leu Thr Cys Met Ile Thr Gly Phe Leu Pro Ala
       130                 135                 140
   Glu Ile Ala Val Asp Trp Thr Ser Asn Gly Arg Thr Glu Gln Asn Tyr
   145                 150                 155                 160
   Lys Asn Thr Ala Thr Val Leu Asp Ser Asp Gly Ser Tyr Phe Met Tyr
                   165                 170                 175
   Ser Lys Leu Arg Val Gln Lys Ser Thr Trp Glu Arg Gly Ser Leu Phe
               180                 185                 190
   Ala Cys Ser Val Val His Glu Gly Leu His Asn His Leu Thr Thr Lys
           195                 200                 205
   Thr Ile Ser Arg Ser Leu Gly Lys
       210                 215
<210> 342
   <211> 213
   <212> PRT
   <213> Artificial Sequence
<220>
   <223> HM2 (mouse CH3mu-CH3gamma) without C-termial tail
<400> 342
   Ser Pro Ser Thr Asp Ile Leu Thr Phe Thr Ile Pro Pro Ser Phe Ala
   1               5                   10                  15
   Asp Ile Phe Leu Ser Lys Ser Ala Asn Leu Thr Cys Leu Val Ser Asn
               20                  25                  30
   Leu Ala Thr Tyr Glu Thr Leu Asn Ile Ser Trp Ala Ser Gln Ser Gly
           35                  40                  45
   Glu Pro Leu Glu Thr Lys Ile Lys Ile Met Glu Ser His Pro Asn Gly
       50                  55                  60
   Thr Phe Ser Ala Lys Gly Val Ala Ser Val Cys Val Glu Asp Trp Asn
   65                  70                  75                  80
   Asn Arg Lys Glu Phe Val Cys Thr Val Thr His Arg Asp Leu Pro Ser
                   85                  90                  95
   Pro Gln Lys Lys Phe Ile Ser Lys Pro Asn Gly Pro Val Arg Ala Pro
               100                 105                 110
   Gln Val Tyr Val Leu Pro Pro Pro Ala Glu Glu Met Thr Lys Lys Glu
           115                 120                 125
   Phe Ser Leu Thr Cys Met Ile Thr Gly Phe Leu Pro Ala Glu Ile Ala
       130                 135                 140
   Val Asp Trp Thr Ser Asn Gly Arg Thr Glu Gln Asn Tyr Lys Asn Thr
   145                 150                 155                 160
   Ala Thr Val Leu Asp Ser Asp Gly Ser Tyr Phe Met Tyr Ser Lys Leu
                   165                 170                 175
   Arg Val Gln Lys Ser Thr Trp Glu Arg Gly Ser Leu Phe Ala Cys Ser
               180                 185                 190
   Val Val His Glu Gly Leu His Asn His Leu Thr Thr Lys Thr Ile Ser
           195                 200                 205
   Arg Ser Leu Gly Lys
       210
<210> 343
   <211> 110
   <212> PRT
   <213> Artificial Sequence
<220>
   <223> huamn IgG4 AA CH2 with alanine substitutions at F234 and N297
<400> 343
   Ala Pro Glu Ala Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 344
   <211> 110
   <212> PRT
   <213> Artificial Sequence
<220>
   <223> human IgG4 AA CH2 with alanine substitutions at L235 and N297
<400> 344
   Ala Pro Glu Phe Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 345
   <211> 110
   <212> PRT
   <213> Artificial Sequence
<220>
   <223> human IgG4 AA CH2 with alanine substitutions at G236 and N297
<400> 345
   Ala Pro Glu Phe Leu Ala Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 346
   <211> 110
   <212> PRT
   <213> Artificial Sequence
<220>
   <223> human IgG4 AA CH2 with alanine substitutions at G237 and N297
<400> 346
   Ala Pro Glu Phe Leu Gly Ala Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 347
<400> 347
   000
<210> 348
<400> 348
   000
<210> 349
<400> 349
   000
<210> 350
<400> 350
   000
<210> 351
<400> 351
   000
<210> 352
<400> 352
   000
<210> 353
<400> 353
   000
<210> 354
<400> 354
   000
<210> 355
<400> 355
   000
<210> 356
<400> 356
   000
<210> 357
<400> 357
   000
<210> 358
<400> 358
   000
<210> 359
<400> 359
   000
<210> 360
<400> 360
   000
<210> 361
<400> 361
   000
<210> 362
<400> 362
   000
<210> 363
<400> 363
   000
<210> 364
<400> 364
   000
<210> 365
<400> 365
   000
<210> 366
<400> 366
   000
<210> 367
<400> 367
   000
<210> 368
<400> 368
   000
<210> 369
<400> 369
   000
<210> 370
<400> 370
   000
<210> 371
<400> 371
   000
<210> 372
<400> 372
   000
<210> 373
<400> 373
   000
<210> 374
<400> 374
   000
<210> 375
<400> 375
   000
<210> 376
<400> 376
   000
<210> 377
<400> 377
   000
<210> 378
<400> 378
   000
<210> 379
   <211> 15
   <212> PRT
   <213> Artificial Sequence
<220>
   <223> Linker 134
<400> 379
   Glu Pro Met Ser Cys Asp Lys Thr His Thr Cys Pro Pro Cys Pro
   1 5 10 15
<210> 380
   <211> 15
   <212> PRT
   <213> Artificial Sequence
<220>
   <223> Linker 135
<400> 380
   Glu Pro Met Ser Ser Asp Lys Thr His Thr Cys Pro Pro Cys Pro
   1 5 10 15
<210> 381
   <211> 15
   <212> PRT
   <213> Artificial Sequence
<220>
   <223> Linker 136
<400> 381
   Glu Pro Met Ser Cys Asp Lys Thr His Thr Ser Pro Pro Cys Pro
   1 5 10 15
<210> 382
   <211> 15
   <212> PRT
   <213> Artificial Sequence
<220>
   <223> Linker 137
<400> 382
   Glu Pro Met Ser Cys Asp Lys Thr His Thr Cys Pro Pro Ser Pro
   1 5 10 15
<210> 383
   <211> 15
   <212> PRT
   <213> Artificial Sequence
<220>
   <223> Linker 138
<400> 383
   Glu Pro Met Ser Ser Asp Lys Thr His Thr Ser Pro Pro Cys Pro
   1 5 10 15
<210> 384
   <211> 15
   <212> PRT
   <213> Artificial Sequence
<220>
   <223> Linker 139
<400> 384
   Glu Pro Met Ser Ser Asp Lys Thr His Thr Cys Pro Pro Ser Pro
   1 5 10 15
<210> 385
   <211> 15
   <212> PRT
   <213> Artificial Sequence
<220>
   <223> Linker 140
<400> 385
   Glu Pro Met Ser Cys Asp Lys Thr His Thr Ser Pro Pro Ser Pro
   1 5 10 15
<210> 386
   <211> 15
   <212> PRT
   <213> Artificial Sequence
<220>
   <223> Linker 141
<400> 386
   Glu Pro Met Ser Cys Asp Lys Thr His Thr Cys Pro Pro Cys Ser
   1 5 10 15
<210> 387
   <211> 15
   <212> PRT
   <213> Artificial Sequence
<220>
   <223> Linker 142
<400> 387
   Glu Pro Met Ser Ser Asp Lys Thr His Thr Cys Pro Pro Cys Ser
   1 5 10 15
<210> 388
   <211> 15
   <212> PRT
   <213> Artificial Sequence
<220>
   <223> Linker 143
<400> 388
   Glu Pro Met Ser Cys Asp Lys Thr His Thr Ser Pro Pro Cys Ser
   1 5 10 15
<210> 389
   <211> 15
   <212> PRT
   <213> Artificial Sequence
<220>
   <223> Linker 144
<400> 389
   Glu Pro Met Ser Cys Asp Lys Thr His Thr Cys Pro Pro Ser Ser
   1 5 10 15
<210> 390
   <211> 15
   <212> PRT
   <213> Artificial Sequence
<220>
   <223> Linker 145
<400> 390
   Glu Pro Met Ser Ser Asp Lys Thr His Thr Ser Pro Pro Cys Ser
   1 5 10 15
<210> 391
   <211> 15
   <212> PRT
   <213> Artificial Sequence
<220>
   <223> Linker 146
<400> 391
   Glu Pro Met Ser Ser Asp Lys Thr His Thr Cys Pro Pro Ser Pro
   1 5 10 15
<210> 392
   <211> 15
   <212> PRT
   <213> Artificial Sequence
<220>
   <223> Linker 147
<400> 392
   Glu Pro Met Ser Cys Asp Lys Thr His Thr Ser Pro Pro Ser Ser
   1 5 10 15
<210> 393
   <211> 15
   <212> PRT
   <213> Artificial Sequence
<220>
   <223> Linker 148
<400> 393
   Glu Pro Thr Ser Cys Asp Lys Thr His Thr Cys Pro Pro Cys Pro
   1 5 10 15
<210> 394
   <211> 15
   <212> PRT
   <213> Artificial Sequence
<220>
   <223> Linker 149
<400> 394
   Glu Pro Thr Ser Ser Asp Lys Thr His Thr Cys Pro Pro Cys Pro
   1 5 10 15
<210> 395
   <211> 15
   <212> PRT
   <213> Artificial Sequence
<220>
   <223> Linker 150
<400> 395
   Glu Pro Thr Ser Cys Asp Lys Thr His Thr Ser Pro Pro Cys Pro
   1 5 10 15
<210> 396
   <211> 15
   <212> PRT
   <213> Artificial Sequence
<220>
   <223> Linker 151
<400> 396
   Glu Pro Thr Ser Cys Asp Lys Thr His Thr Cys Pro Pro Ser Pro
   1               5                   10                  15
<210> 397
   <211> 15
   <212> PRT
   <213> Artificial Sequence
<220>
   <223> Linker 152
<400> 397
   Glu Pro Thr Ser Ser Asp Lys Thr His Thr Ser Pro Pro Cys Pro
   1               5                   10                  15
<210> 398
   <211> 15
   <212> PRT
   <213> Artificial Sequence
<220>
   <223> Linker 153
<400> 398
   Glu Pro Thr Ser Ser Asp Lys Thr His Thr Cys Pro Pro Ser Pro
   1               5                   10                  15
<210> 399
   <211> 15
   <212> PRT
   <213> Artificial Sequence
<220>
   <223> Linker 154
<400> 399
   Glu Pro Thr Ser Cys Asp Lys Thr His Thr Ser Pro Pro Ser Pro
   1               5                   10                  15
<210> 400
   <211> 15
   <212> PRT
   <213> Artificial Sequence
<220>
   <223> Linker 155
<400> 400
   Glu Pro Thr Ser Cys Asp Lys Thr His Thr Cys Pro Pro Cys Ser
   1               5                   10                  15
<210> 401
   <211> 15
   <212> PRT
   <213> Artificial Sequence
<220>
   <223> Linker 156
<400> 401
   Glu Pro Thr Ser Ser Asp Lys Thr His Thr Cys Pro Pro Cys Ser
   1               5                   10                  15
<210> 402
   <211> 15
   <212> PRT
   <213> Artificial Sequence
<220>
   <223> Linker 157
<400> 402
   Glu Pro Thr Ser Cys Asp Lys Thr His Thr Ser Pro Pro Cys Ser
   1               5                   10                  15
<210> 403
   <211> 15
   <212> PRT
   <213> Artificial Sequence
<220>
   <223> Linker 158
<400> 403
   Glu Pro Thr Ser Cys Asp Lys Thr His Thr Cys Pro Pro Ser Ser
   1               5                   10                  15
<210> 404
   <211> 15
   <212> PRT
   <213> Artificial Sequence
<220>
   <223> Linker 159
<400> 404
   Glu Pro Thr Ser Ser Asp Lys Thr His Thr Ser Pro Pro Cys Ser
   1               5                   10                  15
<210> 405
   <211> 15
   <212> PRT
   <213> Artificial Sequence
<220>
   <223> Linker 160
<400> 405
   Glu Pro Thr Ser Ser Asp Lys Thr His Thr Cys Pro Pro Ser Ser
   1 5 10 15
<210> 406
   <211> 15
   <212> PRT
   <213> Artificial Sequence
<220>
   <223> Linker 161
<400> 406
   Glu Pro Thr Ser Cys Asp Lys Thr His Thr Ser Pro Pro Ser Ser
   1 5 10 15
<210> 407
   <211> 15
   <212> PRT
   <213> Artificial Sequence
<220>
   <223> Linker 162
<400> 407
   Glu Pro Ala Ser Cys Asp Lys Thr His Thr Cys Pro Pro Cys Pro
   1 5 10 15
<210> 408
   <211> 15
   <212> PRT
   <213> Artificial Sequence
<220>
   <223> Linker 163
<400> 408
   Glu Pro Ala Ser Ser Asp Lys Thr His Thr Cys Pro Pro Cys Pro
   1 5 10 15
<210> 409
   <211> 15
   <212> PRT
   <213> Artificial Sequence
<220>
   <223> Linker 164
<400> 409
   Glu Pro Ala Ser Cys Asp Lys Thr His Thr Ser Pro Pro Cys Pro
   1 5 10 15
<210> 410
   <211> 15
   <212> PRT
   <213> Artificial Sequence
<220>
   <223> Linker 165
<400> 410
   Glu Pro Ala Ser Cys Asp Lys Thr His Thr Cys Pro Pro Ser Pro
   1 5 10 15
<210> 411
   <211> 15
   <212> PRT
   <213> Artificial Sequence
<220>
   <223> Linker 166
<400> 411
   Glu Pro Ala Ser Ser Asp Lys Thr His Thr Ser Pro Pro Cys Pro
   1 5 10 15
<210> 412
   <211> 15
   <212> PRT
   <213> Artificial Sequence
<220>
   <223> Linker 167
<400> 412
   Glu Pro Ala Ser Ser Asp Lys Thr His Thr Cys Pro Pro Ser Pro
   1 5 10 15
<210> 413
   <211> 15
   <212> PRT
   <213> Artificial Sequence
<220>
   <223> Linker 168
<400> 413
   Glu Pro Ala Ser Cys Asp Lys Thr His Thr Ser Pro Pro Ser Pro
   1 5 10 15
<210> 414
   <211> 15
   <212> PRT
   <213> Artificial Sequence
<220>
   <223> Linker 169
<400> 414
   Glu Pro Ala Ser Cys Asp Lys Thr His Thr Cys Pro Pro Cys Ser
   1               5                   10                  15
<210> 415
   <211> 15
   <212> PRT
   <213> Artificial Sequence
<220>
   <223> Linker 170
<400> 415
   Glu Pro Ala Ser Ser Asp Lys Thr His Thr Cys Pro Pro Cys Ser
   1               5                   10                  15
<210> 416
   <211> 15
   <212> PRT
   <213> Artificial Sequence
<220>
   <223> Linker 171
<400> 416
   Glu Pro Ala Ser Cys Asp Lys Thr His Thr Ser Pro Pro Cys Ser
   1               5                   10                  15
<210> 417
   <211> 15
   <212> PRT
   <213> Artificial Sequence
<220>
   <223> Linker 172
<400> 417
   Glu Pro Ala Ser Cys Asp Lys Thr His Thr Cys Pro Pro Ser Ser
   1               5                   10                  15
<210> 418
   <211> 15
   <212> PRT
   <213> Artificial Sequence
<220>
   <223> Linker 173
<400> 418
   Glu Pro Ala Ser Ser Asp Lys Thr His Thr Ser Pro Pro Cys Ser
   1 5 10 15
<210> 419
   <211> 15
   <212> PRT
   <213> Artificial Sequence
<220>
   <223> Linker 174
<400> 419
   Glu Pro Ala Ser Ser Asp Lys Thr His Thr Cys Pro Pro Ser Ser
   1 5 10 15
<210> 420
   <211> 15
   <212> PRT
   <213> Artificial Sequence
<220>
   <223> Linker 175
<400> 420
   Glu Pro Ala Ser Cys Asp Lys Thr His Thr Ser Pro Pro Ser Ser
   1 5 10 15
<210> 421
   <211> 14
   <212> PRT
   <213> Artificial Sequence
<220>
   <223> Linker 176
<400> 421
   Glu Pro Ser Cys Asp Lys Thr His Thr Cys Pro Pro Cys Pro
   1 5 10
<210> 422
   <211> 14
   <212> PRT
   <213> Artificial Sequence
<220>
   <223> Linker 177
<400> 422
   Glu Pro Ser Ser Asp Lys Thr His Thr Cys Pro Pro Cys Pro
   1 5 10
<210> 423
   <211> 14
   <212> PRT
   <213> Artificial Sequence
<220>
   <223> Linker 178
<400> 423
   Glu Pro Ser Cys Asp Lys Thr His Thr Ser Pro Pro Cys Pro
   1               5                   10
<210> 424
   <211> 14
   <212> PRT
   <213> Artificial Sequence
<220>
   <223> Linker 179
<400> 424
   Glu Pro Ser Cys Asp Lys Thr His Thr Cys Pro Pro Ser Pro
   1               5                   10
<210> 425
   <211> 14
   <212> PRT
   <213> Artificial Sequence
<220>
   <223> Linker 180
<400> 425
   Glu Pro Ser Ser Asp Lys Thr His Thr Ser Pro Pro Cys Pro
   1               5                   10
<210> 426
   <211> 14
   <212> PRT
   <213> Artificial Sequence
<220>
   <223> Linker 181
<400> 426
   Glu Pro Ser Ser Asp Lys Thr His Thr Cys Pro Pro Ser Pro
   1               5                   10
<210> 427
   <211> 14
   <212> PRT
   <213> Artificial Sequence
<220>
   <223> Linker 182
<400> 427
   Glu Pro Ser Cys Asp Lys Thr His Thr Ser Pro Pro Ser Pro
   1 5 10
<210> 428
   <211> 14
   <212> PRT
   <213> Artificial Sequence
<220>
   <223> Linker 183
<400> 428
   Glu Pro Ser Cys Asp Lys Thr His Thr Cys Pro Pro Cys Ser
   1 5 10
<210> 429
   <211> 14
   <212> PRT
   <213> Artificial Sequence
<220>
   <223> Linker 184
<400> 429
   Glu Pro Ser Ser Asp Lys Thr His Thr Cys Pro Pro Cys Ser
   1 5 10
<210> 430
   <211> 14
   <212> PRT
   <213> Artificial Sequence
<220>
   <223> Linker 185
<400> 430
   Glu Pro Ser Cys Asp Lys Thr His Thr Ser Pro Pro Cys Ser
   1 5 10
<210> 431
   <211> 14
   <212> PRT
   <213> Artificial Sequence
<220>
   <223> Linker 186
<400> 431
   Glu Pro Ser Cys Asp Lys Thr His Thr Cys Pro Pro Ser Ser
   1 5 10
<210> 432
   <211> 14
   <212> PRT
   <213> Artificial Sequence
<220>
   <223> Linker 187
<400> 432
   Glu Pro Ser Ser Asp Lys Thr His Thr Ser Pro Pro Cys Ser
    1               5                   10
<210> 433
   <211> 14
   <212> PRT
   <213> Artificial Sequence
<220>
   <223> Linker 188
<400> 433
   Glu Pro Ser Ser Asp Lys Thr His Thr Cys Pro Pro Ser Ser
    1               5                   10
<210> 434
   <211> 14
   <212> PRT
   <213> Artificial Sequence
<220>
   <223> Linker 189
<400> 434
   Glu Pro Ser Cys Asp Lys Thr His Thr Ser Pro Pro Ser Ser
    1               5                   10
<210> 435
<400> 435
   000
<210> 436
<400> 436
   000
<210> 437
<400> 437
   000
<210> 438
<400> 438
   000
<210> 439
<400> 439
   000
<210> 440
<400> 440
   000
<210> 441
<400> 441
   000
<210> 442
<400> 442
   000
<210> 443
<400> 443
   000
<210> 444
<400> 444
   000
<210> 445
<400> 445
   000
<210> 446
<400> 446
   000
<210> 447
<400> 447
   000
<210> 448
<400> 448
   000
<210> 449
<400> 449
   000
<210> 450
<400> 450
   000
<210> 451
<400> 451
   000
<210> 452
<400> 452
   000
<210> 453
<400> 453
   000
<210> 454
<400> 454
   000
<210> 455
<400> 455
   000
<210> 456
<400> 456
   000
<210> 457
<400> 457
   000
<210> 458
<400> 458
   000
<210> 459
<400> 459
   000
<210> 460
<400> 460
   000
<210> 461
<400> 461
   000
<210> 462
<400> 462
   000
<210> 463
<400> 463
   000
<210> 464
   <211> 5
   <212> PRT
   <213> Artificial Sequence
<220>
   <223> Anti-c-Met Heavy Chain Complementarity Determining Region
<400> 464
   Lys Tyr Asp Met Leu
   1               5
<210> 465
   <211> 17
   <212> PRT
   <213> Artificial Sequence
<220>
   <223> Anti-c-Met Heavy Chain Complementarity Determining Region
<400> 465
   Tyr Ile Tyr Pro Ser Gly Gly Leu Thr Glu Tyr Ala Asp Ser Val Lys
   1               5                   10                  15
   Gly
<210> 466
   <211> 10
   <212> PRT
   <213> Artificial Sequence
<220>
   <223> Anti-c-Met Heavy Chain Complementarity Determining Region
<400> 466
   Arg Ala Pro Arg Ser Leu Ser Phe Asp Ile
   1               5                   10
<210> 467
<400> 467
   000
<210> 468
<400> 468
   000
<210> 469
<400> 469
   000
<210> 470
<400> 470
   000
<210> 471
<400> 471
   000
<210> 472
<400> 472
   000
<210> 473
<400> 473
   000
<210> 474
<400> 474
   000
<210> 475
<400> 475
   000
<210> 476
<400> 476
   000
<210> 477
<400> 477
   000
<210> 478
<400> 478
   000
<210> 479
<400> 479
   000
<210> 480
<400> 480
   000
<210> 481
<400> 481
   000
<210> 482
<400> 482
   000
<210> 483
<400> 483
   000
<210> 484
<400> 484
   000
<210> 485
<400> 485
   000
<210> 486
<400> 486
   000
<210> 487
<400> 487
   000
<210> 488
<400> 488
   000
<210> 489
<400> 489
   000
<210> 490
<400> 490
   000
<210> 491
<400> 491
   000
<210> 492
<400> 492
   000
<210> 493
<400> 493
   000
<210> 494
<400> 494
   000
<210> 495
<400> 495
   000
<210> 496
<400> 496
   000
<210> 497
<400> 497
   000
<210> 498
<400> 498
   000
<210> 499
<400> 499
   000
<210> 500
<400> 500
   000
<210> 501
<400> 501
   000
<210> 502
<400> 502
   000
<210> 503
<400> 503
   000
<210> 504
<400> 504
   000
<210> 505
<400> 505
   000
<210> 506
<400> 506
   000
<210> 507
<400> 507
   000
<210> 508
<400> 508
   000
<210> 509
<400> 509
   000
<210> 510
<400> 510
   000
<210> 511
<400> 511
   000
<210> 512
<400> 512
   000
<210> 513
<400> 513
   000
<210> 514
<400> 514
   000
<210> 515
<400> 515
   000
<210> 516
<400> 516
   000
<210> 517
<400> 517
   000
<210> 518
<400> 518
   000
<210> 519
<400> 519
   000
<210> 520
<400> 520
   000
<210> 521
<400> 521
   000
<210> 522
<400> 522
   000
<210> 523
<400> 523
   000
<210> 524
<400> 524
   000
<210> 525
<400> 525
   000
<210> 526
<400> 526
   000
<210> 527
<400> 527
   000
<210> 528
<400> 528
   000
<210> 529
<400> 529
   000
<210> 530
<400> 530
   000
<210> 531
<400> 531
   000
<210> 532
<400> 532
   000
<210> 533
<400> 533
   000
<210> 534
<400> 534
   000
<210> 535
<400> 535
   000
<210> 536
<400> 536
   000
<210> 537
<400> 537
   000
<210> 538
<400> 538
   000
<210> 539
<400> 539
   000
<210> 540
<400> 540
   000
<210> 541
<400> 541
   000
<210> 542
<400> 542
   000
<210> 543
<400> 543
   000
<210> 544
<400> 544
   000
<210> 545
<400> 545
   000
<210> 546
<400> 546
   000
<210> 547
<400> 547
   000
<210> 548
<400> 548
   000
<210> 549
<400> 549
   000
<210> 550
<400> 550
   000
<210> 551
<400> 551
   000
<210> 552
<400> 552
   000
<210> 553
<400> 553
   000
<210> 554
<400> 554
   000
<210> 555
<400> 555
   000
<210> 556
<400> 556
   000
<210> 557
<400> 557
   000
<210> 558
<400> 558
   000
<210> 559
<400> 559
   000
<210> 560
<400> 560
   000
<210> 561
<400> 561
   000
<210> 562
<400> 562
   000
<210> 563
<400> 563
   000
<210> 564
<400> 564
   000
<210> 565
<400> 565
   000
<210> 566
<400> 566
   000
<210> 567
<400> 567
   000
<210> 568
<400> 568
   000
<210> 569
<400> 569
   000
<210> 570
<400> 570
   000
<210> 571
<400> 571
   000
<210> 572
<400> 572
   000
<210> 573
<400> 573
   000
<210> 574
<400> 574
   000
<210> 575
<400> 575
   000
<210> 576
<400> 576
   000
<210> 577
<400> 577
   000
<210> 578
<400> 578
   000
<210> 579
<400> 579
   000
<210> 580
<400> 580
   000
<210> 581
<400> 581
   000
<210> 582
<400> 582
   000
<210> 583
<400> 583
   000
<210> 584
<400> 584
   000
<210> 585
<400> 585
   000
<210> 586
<400> 586
   000
<210> 587
<400> 587
   000
<210> 588
<400> 588
   000
<210> 589
<400> 589
   000
<210> 590
<400> 590
   000
<210> 591
<400> 591
   000
<210> 592
<400> 592
   000
<210> 593
<400> 593
   000
<210> 594
<400> 594
   000
<210> 595
   <211> 110
   <212> PRT
   <213> Artificial Sequence
<220>
   <223> Human IgG1 CH2 with L235A, E318A, K320A and K322A substitutions
<400> 595
   Ala Pro Glu Leu Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Ala Tyr Ala Cys Ala Val Ser Asn Lys
                   85                  90                  95
   Ala Leu Pro Ala Pro Ile Glu Lys Thr Ile Ser Lys Ala Lys
               100                 105                 110
<210> 596
   <211> 110
   <212> PRT
   <213> Artificial Sequence
<220>
   <223> Human IgG1 CH2 with L234A, L235A, G237A, E318A, K320A and K322A
<400> 596
   Ala Pro Glu Ala Ala Gly Ala Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Ala Tyr Ala Cys Ala Val Ser Asn Lys
                   85                  90                  95
   Ala Leu Pro Ala Pro Ile Glu Lys Thr Ile Ser Lys Ala Lys
               100                 105                 110
<210> 597
<400> 597
   000
<210> 598
   <211> 3
   <212> PRT
   <213> Artificial Sequence
<220>
   <223> Junction amino acids
<400> 598
   Arg Asp Gln
    1
<210> 599
   <211> 4
   <212> PRT
   <213> Artificial Sequence
<220>
   <223> Junction amino acids
<400> 599
   Ser Ala Ser Ser
   1
<210> 600
   <211> 3
   <212> PRT
   <213> Artificial Sequence
<220>
   <223> Junction amino acids
<400> 600
   Ser Ser Ser
   1
<210> 601
   <211> 17
   <212> PRT
   <213> Artificial Sequence
<220>
   <223> NKG2D Linker
<400> 601
   Asn Ser Leu Phe Asn Gln Glu Val Gln Ile Pro Leu Thr Glu Ser Tyr
   1 5 10 15
   Ser
<210> 602
   <211> 107
   <212> PRT
   <213> Artificial Sequence
<220>
   <223> Anti-RON mAb 4C04 light chain
<400> 602
   Asp Ile Gln Met Thr Gln Ser Pro Ala Ser Gln Ser Ala Ser Leu Gly
    1               5                   10                  15
   Glu Ser Val Thr Ile Thr Cys Leu Ala Ser Gln Thr Ile Gly Thr Trp
               20                  25                  30
   Leu Ala Trp Tyr Gln Gln Lys Pro Gly Lys Ser Pro Gln Leu Leu Ile
           35                  40                  45
   Tyr Ala Ala Thr Ser Leu Ala Asp Gly Val Pro Ser Arg Phe Ser Ala
       50                  55                  60
   Ser Gly Ser Gly Thr Lys Phe Ser Phe Lys Ile Asp Ser Leu Gln Ser
   65                  70                  75                  80
   Glu Asp Phe Val Ser Tyr Tyr Cys Gln Gln Leu Tyr Asn Thr Pro Trp
                   85                  90                  95
   Thr Phe Gly Gly Gly Thr Lys Leu Glu Ile Lys
               100                 105
<210> 603
   <211> 121
   <212> PRT
   <213> Artificial Sequence
<220>
   <223> Anti-RON mAb 4C04 heavy chain
<400> 603
   Gln Ile Gln Leu Gln Gln Ser Gly Pro Glu Leu Val Lys Pro Gly Thr
    1               5                   10                  15
   Ser Val Lys Ile Ser Cys Lys Ala Ser Asp Tyr Thr Leu Thr Asp Phe
               20                  25                  30
   Tyr Met Asn Trp Val Arg Gln Lys Pro Gly Gln Gly Leu Glu Trp Ile
           35                  40                  45
   Gly Arg Ile Tyr Pro Gly Thr Asp Lys Thr Arg Tyr Asn Glu Lys Phe
       50                  55                  60
   Arg Asp Lys Ala Ile Leu Thr Val Asp Thr Ser Ser Ser Thr Ala Tyr
   65                  70                  75                  80
   Met Gln Leu Ser Ser Leu Thr Ser Glu Asp Thr Ala Val Tyr Phe Cys
                   85                  90                  95
   Ala Arg Ser Ala Tyr Tyr Gly Asn Tyr Val Ala Met Asp Tyr Trp Gly
               100                 105                 110
   Gln Gly Thr Ser Val Thr Val Ser Ser
           115                 120
<210> 604
   <211> 6
   <212> PRT
   <213> Artificial Sequence
<220>
   <223> Anti-RON mAb 4C04 light chain CDR1
<400> 604
   Gln Thr Ile Gly Thr Trp
    1               5
<210> 605
   <211> 3
   <212> PRT
   <213> Artificial Sequence
<220>
   <223> Anti-RON mAb 4C04 light chain CDR2
<400> 605
   Ala Ala Thr
   1
<210> 606
   <211> 9
   <212> PRT
   <213> Artificial Sequence
<220>
   <223> Anti-RON mAb 4C04 light chain CDR3
<400> 606
   Gln Gln Leu Tyr Asn Thr Pro Trp Thr
   1 5
<210> 607
   <211> 8
   <212> PRT
   <213> Artificial Sequence
<220>
   <223> Anti-RON mAb 4C04 heavy chain CDR1
<400> 607
   Asp Tyr Thr Leu Thr Asp Phe Tyr
   1 5
<210> 608
   <211> 8
   <212> PRT
   <213> Artificial Sequence
<220>
   <223> Anti-RON mAb 4C04 heavy chain CDR2
<400> 608
   Ile Tyr Pro Gly Thr Asp Lys Thr
   1 5
<210> 609
   <211> 14
   <212> PRT
   <213> Artificial Sequence
<220>
   <223> Anti-RON mAb 4C04 heavy chain CDR3
<400> 609
   Ala Arg Ser Ala Tyr Tyr Gly Asn Tyr Val Ala Met Asp Tyr
   1               5                   10
<210> 610
   <211> 107
   <212> PRT
   <213> Artificial Sequence
<220>
   <223> Anti-RON mAb 11H09 light chain
<400> 610
   Asp Ile Gln Met Thr Gln Ser Pro Ser Ser Leu Ser Ala Ser Leu Gly
   1               5                   10                  15
   Glu Arg Val Ser Leu Thr Cys Arg Ala Ser Gln Asp Ile Gly Ser Ser
               20                  25                  30
   Leu Ser Trp Leu Gln Gln Glu Pro Asp Gly Thr Ile Lys Arg Leu Ile
           35                  40                  45
   Tyr Ser Thr Ser Ser Leu Asp Ser Gly Val Pro Lys Arg Phe Ser Gly
       50                  55                  60
   Ser Arg Ser Gly Ser Asp Tyr Ser Leu Thr Ile Ser Ser Leu Glu Ser
   65                  70                  75                  80
   Glu Asp Phe Val Asp Tyr Tyr Cys Leu Gln Tyr Ala Ser Ser Pro Trp
                   85                  90                  95
   Thr Phe Gly Gly Gly Thr Lys Leu Glu Ile Lys
               100                 105
<210> 611
   <211> 117
   <212> PRT
   <213> Artificial Sequence
<220>
   <223> Anti-RON mAb 11H09 heavy chain
<400> 611
   Gln Val Gln Leu Gln Gln Ser Gly Ala Glu Leu Ala Lys Pro Gly Ala
   1               5                   10                  15
   Ser Val Lys Met Ser Cys Lys Ser Ser Gly Tyr Thr Phe Thr Ser Tyr
               20                  25                  30
   Trp Ile His Trp Val Lys Glu Arg Pro Gly Gln Gly Leu Glu Trp Ile
           35                  40                  45
   Gly Tyr Ile Tyr Pro Thr Thr Gly Tyr Thr Glu Ser Asn Gln Lys Phe
       50                  55                  60
   Lys Asp Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Phe Leu Leu Arg Pro Trp Tyr Phe Asp Val Trp Gly Ala Gly Thr Thr
               100                 105                 110
   Val Thr Val Ser Ser
           115
<210> 612
   <211> 6
   <212> PRT
   <213> Artificial Sequence
<220>
   <223> Anti-RON mAb 11H09 light chain CDR1
<400> 612
   Gln Asp Ile Gly Ser Ser
   1 5
<210> 613
   <211> 3
   <212> PRT
   <213> Artificial Sequence
<220>
   <223> Anti-RON mAb 11H09 light chain CDR2
<400> 613
   Ser Thr Ser
   1
<210> 614
   <211> 9
   <212> PRT
   <213> Artificial Sequence
<220>
   <223> Anti-RON mAb 11H09 light chain CDR3
<400> 614
   Leu Gln Tyr Ala Ser Ser Pro Trp Thr
   1 5
<210> 615
   <211> 8
   <212> PRT
   <213> Artificial Sequence
<220>
   <223> Anti-RON mAb 11H09 heavy chain CDR1
<400> 615
   Gly Tyr Thr Phe Thr Ser Tyr Trp
   1 5
<210> 616
   <211> 8
   <212> PRT
   <213> Artificial Sequence
<220>
   <223> Anti-RON mAb 11H09 heavy chain CDR2
<400> 616
   Ile Tyr Pro Thr Thr Gly Tyr Thr
   1 5
<210> 617
   <211> 10
   <212> PRT
   <213> Artificial Sequence
<220>
   <223> Anti-RON mAb 11H09 heavy chain CDR3
<400> 617
   Phe Leu Leu Arg Pro Trp Tyr Phe Asp Val
   1 5 10
<210> 618
   <211> 2
   <212> PRT
   <213> Artificial Sequence
<220>
   <223> Made in a lab
<400> 618
   Asn Ser
   1
<210> 619
   <211> 6
   <212> PRT
   <213> Artificial Sequence
<220>
   <223> Made in a lab
<400> 619
   Ser Cys Pro Pro Cys Pro
   1 5
<210> 620
   <211> 8
   <212> PRT
   <213> Artificial Sequence
<220>
   <223> Made in a lab
<400> 620
   Gly Gly Gly Gly Ser Gly Asn Ser
   1 5
<210> 621
   <211> 8
   <212> PRT
   <213> Artificial Sequence
<220>
   <223> Made in a lab
<400> 621
   Gly Cys Pro Pro Cys Pro Asn Ser
   1 5
<210> 622
   <211> 8
   <212> PRT
   <213> Artificial Sequence
<220>
   <223> Made in a lab
<400> 622
   Gly Ser Pro Pro Ser Pro Asn Ser
   1 5
<210> 623
   <211> 8
   <212> PRT
   <213> Artificial Sequence
<220>
   <223> Made in a lab
<400> 623
   Gly Ser Pro Pro Ser Pro Asn Ser
   1 5
<210> 624
   <211> 8
   <212> PRT
   <213> Artificial Sequence
<220>
   <223> Made in a lab
<400> 624
   Gly Cys Pro Pro Cys Pro Asn Ser
   1 5
<210> 625
   <211> 8
   <212> PRT
   <213> Artificial Sequence
<220>
   <223> Made in a lab
<400> 625
   Gly Cys Pro Pro Cys Pro Asn Ser
   1 5
<210> 626
   <211> 9
   <212> PRT
   <213> Artificial Sequence
<220>
   <223> Made in a lab
<400> 626
   Gly Cys Pro Pro Cys Pro Gly Asn Ser
   1 5
<210> 627
   <211> 9
   <212> PRT
   <213> Artificial Sequence
<220>
   <223> Made in a lab
<400> 627
   Gly Cys Pro Pro Cys Pro Ala Asn Ser
   1 5
<210> 628
   <211> 9
   <212> PRT
   <213> Artificial Sequence
<220>
   <223> Made in a lab
<400> 628
   Gly Cys Pro Pro Cys Pro Ala Asn Ser
   1 5
<210> 629
   <211> 9
   <212> PRT
   <213> Artificial Sequence
<220>
   <223> Made in a lab
<400> 629
   Glu Glu Glu Glu Asp Glu Gly Asn Ser
   1 5
<210> 630
   <211> 10
   <212> PRT
   <213> Artificial Sequence
<220>
   <223> Made in a lab
<400> 630
   Asn Tyr Gly Gly Gly Gly Ser Gly Asn Ser
   1 5 10
<210> 631
   <211> 10
   <212> PRT
   <213> Artificial Sequence
<220>
   <223> Made in a lab
<400> 631
   Val Ser Glu Arg Pro Phe Pro Pro Asn Ser
   1 5 10
<210> 632
   <211> 11
   <212> PRT
   <213> Artificial Sequence
<220>
   <223> Made in a lab
<400> 632
   Glu Pro Lys Ser Cys Asp Lys Thr Cys Cys Pro
   1 5 10
<210> 633
   <211> 11
   <212> PRT
   <213> Artificial Sequence
<220>
   <223> Made in a lab
<400> 633
   Ser Gln Pro Glu Ile Val Pro Ile Ser Asn Ser
   1 5 10
<210> 634
   <211> 12
   <212> PRT
   <213> Artificial Sequence
<220>
   <223> Made in a lab
<400> 634
   Gly Gly Gly Gly Ser Cys Pro Pro Cys Pro Asn Ser
   1 5 10
<210> 635
   <211> 12
   <212> PRT
   <213> Artificial Sequence
<220>
   <223> Made in a lab
<400> 635
   Lys Ala Asp Phe Leu Thr Pro Ser Ile Gly Asn Ser
   1 5 10
<210> 636
   <211> 12
   <212> PRT
   <213> Artificial Sequence
<220>
   <223> Made in a lab
<400> 636
   Gln Met Asn Ser Glu Leu Ser Val Leu Ala Asn Ser
   1 5 10
<210> 637
   <211> 13
   <212> PRT
   <213> Artificial Sequence
<220>
   <223> Made in a lab
<400> 637
   Glu Pro Lys Ser Cys Asp Lys Thr His Thr Cys Cys Pro
   1 5 10
<210> 638
   <211> 13
   <212> PRT
   <213> Artificial Sequence
<220>
   <223> Made in a lab
<400> 638
   Glu Pro Lys Ser Cys Asp Lys Thr Cys Pro Pro Cys Pro
   1 5 10
<210> 639
   <211> 13
   <212> PRT
   <213> Artificial Sequence
<220>
   <223> Made in a lab
<400> 639
   Gly Gly Gly Gly Ser Gly Gly Gly Gly Ser Gly Asn Ser
   1 5 10
<210> 640
   <211> 13
   <212> PRT
   <213> Artificial Sequence
<220>
   <223> Made in a lab
<400> 640
   Gly Cys Pro Pro Cys Pro Gly Gly Gly Gly Ser Asn Ser
   1 5 10
<210> 641
   <211> 13
   <212> PRT
   <213> Artificial Sequence
<220>
   <223> Made in a lab
<400> 641
   Gly Gly Gly Gly Ser Cys Pro Pro Cys Pro Gly Asn Ser
   1 5 10
<210> 642
   <211> 13
   <212> PRT
   <213> Artificial Sequence
<220>
   <223> Made in a lab
<400> 642
   Gly Cys Pro Pro Cys Pro Gly Gly Gly Gly Ser Asn Ser
   1 5 10
<210> 643
   <211> 13
   <212> PRT
   <213> Artificial Sequence
<220>
   <223> Made in a lab
<400> 643
   Gly Gly Gly Ala Ser Cys Pro Pro Cys Pro Gly Asn Ser
   1 5 10
<210> 644
   <211> 13
   <212> PRT
   <213> Artificial Sequence
<220>
   <223> Made in a lab
<400> 644
   Gly Gly Gly Ala Ser Cys Pro Pro Cys Ala Gly Asn Ser
   1 5 10
<210> 645
   <211> 13
   <212> PRT
   <213> Artificial Sequence
<220>
   <223> Made in a lab
<400> 645
   Gly Gly Gly Ala Ser Cys Pro Pro Cys Ala Gly Asn Ser
   1 5 10
<210> 646
   <211> 15
   <212> PRT
   <213> Artificial Sequence
<220>
   <223> Made in a lab
<400> 646
   Asn Tyr Gly Gly Gly Gly Ser Gly Gly Gly Gly Ser Gly Asn Ser
   1 5 10 15
<210> 647
   <211> 15
   <212> PRT
   <213> Artificial Sequence
<220>
   <223> Made in a lab
<400> 647
   Leu Ser Val Lys Ala Asp Phe Leu Thr Pro Ser Ile Gly Asn Ser
   1 5 10 15
<210> 648
   <211> 15
   <212> PRT
   <213> Artificial Sequence
<220>
   <223> Made in a lab
<400> 648
   Leu Ser Val Leu Ala Asn Phe Ser Gln Pro Glu Ile Gly Asn Ser
   1 5 10 15
<210> 649
   <211> 15
   <212> PRT
   <213> Artificial Sequence
<220>
   <223> Made in a lab
<400> 649
   Leu Lys Ile Gln Glu Arg Val Ser Lys Pro Lys Ile Ser Asn Ser
   1 5 10 15
<210> 650
   <211> 15
   <212> PRT
   <213> Artificial Sequence
<220>
   <223> Made in a lab
<400> 650
   Leu Asp Val Ser Glu Arg Pro Phe Pro Pro His Ile Gln Asn Ser
   1 5 10 15
<210> 651
   <211> 15
   <212> PRT
   <213> Artificial Sequence
<220>
   <223> Made in a lab
<400> 651
   Arg Glu Gln Leu Ala Glu Val Thr Leu Ser Leu Lys Ala Asn Ser
   1 5 10 15
<210> 652
   <211> 15
   <212> PRT
   <213> Artificial Sequence
<220>
   <223> Made in a lab
<400> 652
   Arg Ile His Gln Met Asn Ser Glu Leu Ser Val Leu Ala Asn Ser
   1               5                   10                  15
<210> 653
   <211> 15
   <212> PRT
   <213> Artificial Sequence
<220>
   <223> Made in a lab
<400> 653
   Asp Thr Lys Gly Lys Asn Val Leu Glu Lys Ile Phe Ser Asn Ser
   1               5                   10                  15
<210> 654
   <211> 15
   <212> PRT
   <213> Artificial Sequence
<220>
   <223> Made in a lab
<400> 654
   Leu Pro Pro Glu Thr Gln Glu Ser Gln Glu Val Thr Leu Asn Ser
   1               5                   10                  15
<210> 655
   <211> 15
   <212> PRT
   <213> Artificial Sequence
<220>
   <223> Made in a lab
<400> 655
   Arg Ile His Leu Asn Val Ser Glu Arg Pro Phe Pro Pro Asn Ser
   1               5                   10                  15
<210> 656
   <211> 15
   <212> PRT
   <213> Artificial Sequence
<220>
   <223> Made in a lab
<400> 656
   Leu Ser Val Lys Ala Asp Phe Leu Thr Pro Ser Ile Gly Asn Ser
   1               5                   10                  15
<210> 657
   <211> 15
   <212> PRT
   <213> Artificial Sequence
<220>
   <223> Made in a lab
<400> 657
   Leu Ser Val Leu Ala Asn Phe Ser Gln Pro Glu Ile Gly Asn Ser
   1 5 10 15
<210> 658
   <211> 15
   <212> PRT
   <213> Artificial Sequence
<220>
   <223> Made in a lab
<400> 658
   Leu Ser Val Leu Ala Asn Phe Ser Gln Pro Glu Ile Gly Asn Ser
   1 5 10 15
<210> 659
   <211> 15
   <212> PRT
   <213> Artificial Sequence
<220>
   <223> Made in a lab
<400> 659
   Arg Ile His Gln Met Asn Ser Glu Leu Ser Val Leu Ala Asn Ser
   1 5 10 15
<210> 660
   <211> 15
   <212> PRT
   <213> Artificial Sequence
<220>
   <223> Made in a lab
<400> 660
   Lys Pro Phe Phe Thr Cys Gly Ser Ala Asp Thr Cys Pro Asn Ser
   1 5 10 15
<210> 661
   <211> 15
   <212> PRT
   <213> Artificial Sequence
<220>
   <223> Made in a lab
<400> 661
   Lys Pro Phe Phe Thr Cys Gly Ser Ala Asp Thr Cys Pro Asn Ser
   1 5 10 15
<210> 662
   <211> 15
   <212> PRT
   <213> Artificial Sequence
<220>
   <223> Made in a lab
<400> 662
   Gln Tyr Asn Cys Pro Gly Gln Tyr Thr Phe Ser Met Pro Asn Ser
   1 5 10 15
<210> 663
   <211> 15
   <212> PRT
   <213> Artificial Sequence
<220>
   <223> Made in a lab
<400> 663
   Gly Gly Gly Gly Ser Gly Gly Gly Gly Ser Gly Gly Gly Gly Ser
   1 5 10 15
<210> 664
   <211> 15
   <212> PRT
   <213> Artificial Sequence
<220>
   <223> Made in a lab
<400> 664
   Glu Pro Lys Ser Ser Asp Lys Thr His Thr Cys Pro Pro Cys Pro
   1 5 10 15
<210> 665
   <211> 15
   <212> PRT
   <213> Artificial Sequence
<220>
   <223> Made in a lab
<400> 665
   Glu Pro Lys Ser Ser Asp Lys Thr His Thr Cys Pro Pro Cys Ser
   1 5 10 15
<210> 666
   <211> 15
   <212> PRT
   <213> Artificial Sequence
<220>
   <223> Made in a lab
<400> 666
   Glu Pro Lys Ser Cys Asp Lys Thr His Thr Ser Pro Pro Cys Ser
   1 5 10 15
<210> 667
   <211> 15
   <212> PRT
   <213> Artificial Sequence
<220>
   <223> Made in a lab
<400> 667
   Glu Pro Lys Ser Cys Asp Lys Thr His Thr Cys Pro Pro Cys Pro
   1 5 10 15
<210> 668
   <211> 15
   <212> PRT
   <213> Artificial Sequence
<220>
   <223> Made in a lab
<400> 668
   Glu Pro Lys Ser Cys Asp Lys Thr His Thr Ser Pro Pro Ser Ser
   1 5 10 15
<210> 669
   <211> 15
   <212> PRT
   <213> Artificial Sequence
<220>
   <223> Made in a lab
<400> 669
   Pro Arg Gly Pro Thr Ile Lys Pro Cys Pro Pro Cys Lys Cys Pro
   1 5 10 15
<210> 670
   <211> 15
   <212> PRT
   <213> Artificial Sequence
<220>
   <223> Made in a lab
<400> 670
   Glu Pro Lys Ser Ser Asp Lys Thr His Thr Ser Pro Pro Ser Ser
    1               5                   10                  15
<210> 671
   <211> 15
   <212> PRT
   <213> Artificial Sequence
<220>
   <223> Made in a lab
<400> 671
   Glu Pro Lys Ser Ser Asp Lys Thr His Thr Ser Pro Pro Cys Ser
    1               5                   10                  15
<210> 672
   <211> 15
   <212> PRT
   <213> Artificial Sequence
<220>
   <223> Made in a lab
<400> 672
   Glu Pro Lys Ser Ser Asp Lys Thr His Thr Cys Pro Pro Ser Ser
    1               5                   10                  15
<210> 673
   <211> 15
   <212> PRT
   <213> Artificial Sequence
<220>
   <223> Made in a lab
<400> 673
   Glu Pro Lys Ser Cys Asp Lys Thr His Thr Ser Pro Pro Cys Pro
    1               5                   10                  15
<210> 674
   <211> 15
   <212> PRT
   <213> Artificial Sequence
<220>
   <223> Made in a lab
<400> 674
   Glu Pro Lys Ser Ser Asp Lys Thr His Thr Ser Pro Pro Ser Pro
   1 5 10 15
<210> 675
   <211> 15
   <212> PRT
   <213> Artificial Sequence
<220>
   <223> Made in a lab
<400> 675
   Glu Pro Lys Ser Ser Asp Lys Thr His Thr Ser Pro Pro Cys Pro
   1 5 10 15
<210> 676
   <211> 15
   <212> PRT
   <213> Artificial Sequence
<220>
   <223> Made in a lab
<400> 676
   Glu Pro Lys Ser Cys Asp Lys Thr His Thr Ser Pro Pro Ser Pro
   1 5 10 15
<210> 677
   <211> 15
   <212> PRT
   <213> Artificial Sequence
<220>
   <223> Made in a lab
<400> 677
   Glu Pro Lys Ser Ser Asp Lys Thr His Thr Cys Pro Pro Ser Pro
   1 5 10 15
<210> 678
   <211> 15
   <212> PRT
   <213> Artificial Sequence
<220>
   <223> Made in a lab
<400> 678
   Gly Gly Gly Gly Cys Asp Lys Thr His Thr Cys Pro Pro Cys Pro
   1 5 10 15
<210> 679
   <211> 15
   <212> PRT
   <213> Artificial Sequence
<220>
   <223> Made in a lab
<400> 679
   Glu Pro Lys Ser Cys Gly Gly Gly Gly Gly Cys Pro Pro Cys Pro
   1 5 10 15
<210> 680
   <211> 15
   <212> PRT
   <213> Artificial Sequence
<220>
   <223> Made in a lab
<400> 680
   Glu Pro Lys Ser Cys Asp Lys Thr His Thr Cys Gly Gly Cys Pro
   1 5 10 15
<210> 681
   <211> 15
   <212> PRT
   <213> Artificial Sequence
<220>
   <223> Made in a lab
<400> 681
   Glu Pro Lys Ser Cys Asp Lys Thr His Thr Cys Pro Pro Cys Gly
   1 5 10 15
<210> 682
   <211> 15
   <212> PRT
   <213> Artificial Sequence
<220>
   <223> Made in a lab
<400> 682
   Glu Pro Lys Ser Cys Asp Lys Thr His Thr Cys Pro Pro Ser Pro
   1 5 10 15
<210> 683
   <211> 15
   <212> PRT
   <213> Artificial Sequence
<220>
   <223> Made in a lab
<400> 683
   Glu Pro Lys Ser Cys Asp Lys Cys His Thr Cys Pro Pro Cys Pro
   1 5 10 15
<210> 684
   <211> 15
   <212> PRT
   <213> Artificial Sequence
<220>
   <223> Made in a lab
<400> 684
   Glu Pro Lys Ser Cys Asp Lys Thr Cys Cys Cys Pro Pro Cys Pro
   1 5 10 15
<210> 685
   <211> 15
   <212> PRT
   <213> Artificial Sequence
<220>
   <223> Made in a lab
<400> 685
   Glu Pro Lys Ser Cys Pro Pro Pro Pro Pro Cys Pro Pro Cys Pro
   1 5 10 15
<210> 686
   <211> 15
   <212> PRT
   <213> Artificial Sequence
<220>
   <223> Made in a lab
<400> 686
   Pro Pro Pro Pro Cys Asp Lys Thr His Thr Cys Pro Pro Cys Pro
   1 5 10 15
<210> 687
   <211> 15
   <212> PRT
   <213> Artificial Sequence
<220>
   <223> Made in a lab
<400> 687
   Glu Pro Lys Ser Cys Asp Lys Thr His Thr Cys Trp Trp Cys Pro
   1 5 10 15
<210> 688
   <211> 15
   <212> PRT
   <213> Artificial Sequence
<220>
   <223> Made in a lab
<400> 688
   Glu Pro Lys Ser Cys Asp Trp Trp His Thr Cys Pro Pro Cys Pro
   1 5 10 15
<210> 689
   <211> 15
   <212> PRT
   <213> Artificial Sequence
<220>
   <223> Made in a lab
<400> 689
   Glu Pro Lys Cys Ser Asp Lys Thr His Thr Cys Pro Pro Cys Pro
   1 5 10 15
<210> 690
   <211> 15
   <212> PRT
   <213> Artificial Sequence
<220>
   <223> Made in a lab
<400> 690
   Glu Pro Lys Ser Asp Cys Lys Thr His Thr Cys Pro Pro Cys Pro
   1 5 10 15
<210> 691
   <211> 15
   <212> PRT
   <213> Artificial Sequence
<220>
   <223> Made in a lab
<400> 691
   Glu Pro Lys Ser Asp Cys Trp Trp His Thr Cys Pro Pro Cys Pro
   1 5 10 15
<210> 692
   <211> 15
   <212> PRT
   <213> Artificial Sequence
<220>
   <223> Made in a lab
<400> 692
   Glu Pro Lys Ser Cys Asp Phe Phe His Thr Cys Pro Pro Cys Pro
   1 5 10 15
<210> 693
   <211> 15
   <212> PRT
   <213> Artificial Sequence
<220>
   <223> Made in a lab
<400> 693
   Glu Pro Lys Ser Cys Asp Trp Trp Trp Thr Cys Pro Pro Cys Pro
   1 5 10 15
<210> 694
   <211> 15
   <212> PRT
   <213> Artificial Sequence
<220>
   <223> Made in a lab
<400> 694
   Glu Pro Lys Ser Cys Trp Trp Thr His Thr Cys Pro Pro Cys Pro
   1 5 10 15
<210> 695
   <211> 15
   <212> PRT
   <213> Artificial Sequence
<220>
   <223> Made in a lab
<400> 695
   Glu Pro Trp Trp Cys Asp Lys Thr His Thr Cys Pro Pro Cys Pro
   1 5 10 15
<210> 696
   <211> 16
   <212> PRT
   <213> Artificial Sequence
<220>
   <223> Made in a lab
<400> 696
   Ser Gln Pro Glu Ile Val Pro Ile Ser Cys Pro Pro Cys Pro Asn Ser
   1 5 10 15
<210> 697
   <211> 17
   <212> PRT
   <213> Artificial Sequence
<220>
   <223> Made in a lab
<400> 697
   Thr Gly Glu Pro Lys Ser Ser Asp Lys Thr His Thr Cys Pro Pro Cys
   1 5 10 15
   Pro
<210> 698
   <211> 17
   <212> PRT
   <213> Artificial Sequence
<220>
   <223> Made in a lab
<400> 698
   Glu Pro Lys Ser Thr Asp Lys Thr His Thr Cys Pro Pro Cys Pro Asn
   1 5 10 15
   Ser
<210> 699
   <211> 17
   <212> PRT
   <213> Artificial Sequence
<220>
   <223> Made in a lab
<400> 699
   Glu Pro Lys Ser Thr Asp Lys Thr His Thr Ser Pro Pro Ser Pro Asn
   1 5 10 15
   Ser
<210> 700
   <211> 17
   <212> PRT
   <213> Artificial Sequence
<220>
   <223> Made in a lab
<400> 700
   Glu Pro Lys Ser Thr Asp Lys Thr His Thr Cys Pro Pro Cys Pro Asn
   1               5                   10                  15
   Ser
<210> 701
   <211> 17
   <212> PRT
   <213> Artificial Sequence
<220>
   <223> Made in a lab
<400> 701
   Glu Pro Lys Ser Ser Asp Lys Thr His Thr Cys Pro Pro Cys Pro Asn
   1               5                   10                  15
   Ser
<210> 702
   <211> 18
   <212> PRT
   <213> Artificial Sequence
<220>
   <223> Made in a lab
<400> 702
   Glu Pro Lys Ser Cys Asp Lys Thr His Thr Cys Pro Gly Gly Gly Pro
   1               5                   10                  15
   Cys Pro
<210> 703
   <211> 18
   <212> PRT
   <213> Artificial Sequence
<220>
   <223> Made in a lab
<400> 703
   Glu Pro Lys Ser Cys Asp Gly Gly Gly Lys Thr His Thr Cys Pro Pro
   1               5                   10                  15
   Cys Pro
<210> 704
   <211> 18
   <212> PRT
   <213> Artificial Sequence
<220>
   <223> Made in a lab
<400> 704
   Glu Pro Lys Ser Cys Asp Pro Pro Pro Lys Thr His Thr Cys Pro Pro
   1 5 10 15
   Cys Pro
<210> 705
   <211> 18
   <212> PRT
   <213> Artificial Sequence
<220>
   <223> Made in a lab
<400> 705
   Glu Pro Lys Ser Cys Asp Lys Thr His Thr Cys Pro Pro Pro Pro Pro
   1 5 10 15
   Cys Pro
<210> 706
   <211> 18
   <212> PRT
   <213> Artificial Sequence
<220>
   <223> Made in a lab
<400> 706
   Gly Gly Gly Gly Ser Gly Gly Gly Gly Ser Gly Gly Gly Gly Ser Gly
   1 5 10 15
   Asn Ser
<210> 707
   <211> 19
   <212> PRT
   <213> Artificial Sequence
<220>
   <223> Made in a lab
<400> 707
   Asn Tyr Gly Gly Gly Gly Ser Gly Gly Gly Gly Ser Gly Gly Gly Gly
   1 5 10 15
   Ser Asn Ser
<210> 708
   <211> 19
   <212> PRT
   <213> Artificial Sequence
<220>
   <223> Made in a lab
<400> 708
   Leu Ser Val Lys Ala Asp Phe Leu Thr Pro Ser Ile Ser Pro Pro Cys
   1 5 10 15
   Pro Asn Ser
<210> 709
   <211> 19
   <212> PRT
   <213> Artificial Sequence
<220>
   <223> Made in a lab
<400> 709
   Ser Val Leu Ala Asn Phe Ser Gln Pro Glu Ile Ser Cys Pro Pro Cys
   1 5 10 15
   Pro Asn Ser
<210> 710
   <211> 20
   <212> PRT
   <213> Artificial Sequence
<220>
   <223> Made in a lab
<400> 710
   Gly Gln Arg His Asn Asn Ser Ser Leu Asn Thr Arg Thr Gln Lys Ala
   1 5 10 15
   Arg His Ser Pro
   20
<210> 711
   <211> 20
   <212> PRT
   <213> Artificial Sequence
<220>
   <223> Made in a lab
<400> 711
   Leu Ser Val Leu Ala Asn Phe Ser Gln Pro Glu Ile Ser Cys Pro Pro
   1 5 10 15
   Cys Pro Asn Ser
   20
<210> 712
   <211> 20
   <212> PRT
   <213> Artificial Sequence
<220>
   <223> Made in a lab
<400> 712
   Leu Lys Ile Gln Glu Arg Val Ser Lys Pro Lys Ile Ser Cys Pro Pro
   1 5 10 15
   Cys Pro Asn Ser
   20
<210> 713
   <211> 20
   <212> PRT
   <213> Artificial Sequence
<220>
   <223> Made in a lab
<400> 713
   Arg Glu Gln Leu Ala Glu Val Thr Leu Ser Leu Lys Ala Cys Pro Pro
   1 5 10 15
   Cys Pro Asn Ser
   20
<210> 714
   <211> 20
   <212> PRT
   <213> Artificial Sequence
<220>
   <223> Made in a lab
<400> 714
   Arg Ile His Gln Met Asn Ser Glu Leu Ser Val Leu Ala Cys Pro Pro
   1 5 10 15
   Cys Pro Asn Ser
   20
<210> 715
   <211> 20
   <212> PRT
   <213> Artificial Sequence
<220>
   <223> Made in a lab
<400> 715
   Arg Ile His Leu Asn Val Ser Glu Arg Pro Phe Pro Pro Cys Pro Pro
   1 5 10 15
   Cys Pro Asn Ser
   20
<210> 716
   <211> 20
   <212> PRT
   <213> Artificial Sequence
<220>
   <223> Made in a lab
<400> 716
   Asn Ser Leu Phe Asn Gln Glu Val Gln Ile Pro Leu Thr Glu Ser Tyr
    1               5                  10                  15
   Cys Pro Asn Ser
               20
<210> 717
   <211> 20
   <212> PRT
   <213> Artificial Sequence
<220>
   <223> Made in a lab
<400> 717
   Glu Glu Glu Glu Asp Glu Glu Asp Glu Glu Asp Glu Glu Glu Glu Glu
    1               5                  10                  15
   Asp Gly Asn Ser
               20
<210> 718
   <211> 21
   <212> PRT
   <213> Artificial Sequence
<220>
   <223> Made in a lab
<400> 718
   Leu Asp Val Ser Glu Arg Pro Phe Pro Pro His Ile Gln Ser Cys Pro
    1               5                  10                  15
   Pro Cys Pro Asn Ser
               20
<210> 719
   <211> 21
   <212> PRT
   <213> Artificial Sequence
<220>
   <223> Made in a lab
<400> 719
   Asp Thr Lys Gly Lys Asn Val Leu Glu Lys Ile Phe Asp Ser Cys Pro
    1               5                  10                  15
   Pro Cys Pro Asn Ser
               20
<210> 720
   <211> 21
   <212> PRT
   <213> Artificial Sequence
<220>
   <223> Made in a lab
<400> 720
   Leu Pro Pro Glu Thr Gln Glu Ser Gln Glu Val Thr Leu Ser Cys Pro
    1              5                   10                  15
   Pro Cys Pro Asn Ser
               20
<210> 721
   <211> 21
   <212> PRT
   <213> Artificial Sequence
<220>
   <223> Made in a lab
<400> 721
   Glu Pro Ala Phe Thr Pro Gly Pro Asn Ile Glu Leu Gln Lys Asp Ser
    1              5                   10                  15
   Asp Cys Pro Asn Ser
               20
<210> 722
   <211> 21
   <212> PRT
   <213> Artificial Sequence
<220>
   <223> Made in a lab
<400> 722
   Gln Arg His Asn Asn Ser Ser Leu Asn Thr Arg Thr Gln Lys Ala Arg
    1              5                   10                  15
   His Cys Pro Asn Ser
               20
<210> 723
   <211> 21
   <212> PRT
   <213> Artificial Sequence
<220>
   <223> Made in a lab
<400> 723
   Gln Arg His Asn Asn Ser Ser Leu Asn Thr Arg Thr Gln Lys Ala Arg
    1              5                   10                  15
   His Ser Pro Asn Ser
               20
<210> 724
   <211> 36
   <212> PRT
   <213> Artificial Sequence
<220>
   <223> Made in a lab
<400> 724
   Asn Tyr Gly Gly Gly Gly Ser Gly Gly Gly Gly Ser Gly Gly Gly Gly
    1               5                   10                  15
   Ser Asn Tyr Gly Gly Gly Gly Ser Gly Gly Gly Gly Ser Gly Gly Gly
               20                  25                  30
   Gly Ser Asn Ser
           35
<210> 725
   <211> 122
   <212> PRT
   <213> Artificial Sequence
<220>
   <223> Made in a lab
<400> 725
   Arg Thr Arg Tyr Leu Gln Val Ser Gln Gln Leu Gln Gln Thr Asn Arg
    1               5                   10                  15
   Val Leu Glu Val Thr Asn Ser Ser Leu Arg Gln Gln Leu Arg Leu Lys
               20                  25                  30
   Ile Thr Gln Leu Gly Gln Ser Ala Glu Asp Leu Gln Gly Ser Arg Arg
           35                  40                  45
   Glu Leu Ala Gln Ser Gln Glu Ala Leu Gln Val Glu Gln Arg Ala His
       50                  55                  60
   Gln Ala Ala Glu Gly Gln Leu Gln Ala Cys Gln Ala Asp Arg Gln Lys
   65                  70                  75                  80
   Thr Lys Glu Thr Leu Gln Ser Glu Glu Gln Gln Arg Arg Ala Leu Glu
                   85                  90                  95
   Gln Lys Leu Ser Asn Met Glu Asn Arg Leu Lys Pro Phe Phe Thr Cys
               100                 105                 110
   Gly Ser Ala Asp Thr Cys Cys Pro Asn Ser
           115                 120
<210> 726
   <211> 13
   <212> PRT
   <213> Artificial Sequence
<220>
   <223> Made in a lab
<400> 726
   Gly Gly Gly Ala Ser Cys Pro Pro Cys Ala Gly Asn Ser
    1               5                   10
<210> 727
   <211> 39
   <212> PRT
   <213> Artificial Sequence
<220>
   <223> Made in a lab
<400> 727
   Asn Asn Tyr Gly Gly Gly Gly Ser Gly Gly Gly Gly Ser Gly Gly Gly
    1               5                   10                  15
   Gly Ser Gly Asn Tyr Gly Gly Gly Gly Ser Gly Gly Gly Gly Ser Gly
               20                  25                  30
   Gly Gly Gly Ser Gly Asn Ser
           35
<210> 728
   <211> 20
   <212> PRT
   <213> Artificial Sequence
<220>
   <223> Made in a lab
<400> 728
   Asn Tyr Gly Gly Gly Gly Ser Gly Gly Gly Gly Ser Gly Gly Gly Gly
    1               5                   10                  15
   Ser Gly Asn Ser
               20
<210> 729
   <211> 7
   <212> PRT
   <213> Artificial Sequence
<220>
   <223> Made in a lab
<400> 729
   Ser Pro Pro Ser Pro Asn Ser
    1               5
<210> 730
   <211> 17
   <212> PRT
   <213> Artificial Sequence
<220>
   <223> Made in a lab
<400> 730
   Glu Pro Thr Ser Thr Asp Lys Thr His Thr Ser Pro Pro Ser Pro Asn
    1               5                   10                  15
   Ser
<210> 731
   <211> 17
   <212> PRT
   <213> Artificial Sequence
<220>
   <223> Made in a lab
<400> 731
   Glu Pro Thr Ser Thr Asp Lys Thr His Thr Cys Pro Pro Cys Pro Asn
   1 5 10 15
   Ser
<210> 732
   <211> 19
   <212> PRT
   <213> Artificial Sequence
<220>
   <223> Made in a lab
<400> 732
   Leu Ser Val Lys Ala Asp Phe Leu Thr Pro Ser Ile Ser Pro Pro Cys
   1 5 10 15
   Pro Asn Ser
<210> 733
   <211> 12
   <212> PRT
   <213> Artificial Sequence
<220>
   <223> Made in a lab
<400> 733
   Gly Gly Gly Ala Ser Cys Pro Pro Cys Ala Asn Ser
   1 5 10
<210> 734
   <211> 22
   <212> PRT
   <213> Artificial Sequence
<220>
   <223> Made in a lab
<400> 734
   Arg Thr Gln Arg His Asn Asn Ser Ser Leu Asn Thr Arg Thr Gln Lys
   1 5 10 15
   Ala Arg His Ser Gly His
   20
<210> 735
   <211> 118
   <212> PRT
   <213> Artificial Sequence
<220>
   <223> Made in a lab
<400> 735
   Arg Thr Arg Tyr Leu Gln Val Ser Gln Gln Leu Gln Gln Thr Asn Arg
    1              5                   10                  15
   Val Leu Glu Val Thr Asn Ser Ser Leu Arg Gln Gln Leu Arg Leu Lys
               20                  25                  30
   Ile Thr Gln Leu Gly Gln Ser Ala Glu Asp Leu Gln Gly Ser Arg Arg
           35                  40                  45
   Glu Leu Ala Gln Ser Gln Glu Ala Leu Gln Val Glu Gln Arg Ala His
       50                  55                  60
   Gln Ala Ala Glu Gly Gln Leu Gln Ala Cys Gln Ala Asp Arg Gln Lys
   65                  70                  75                  80
   Thr Lys Glu Thr Leu Gln Ser Glu Glu Gln Gln Arg Arg Ala Leu Glu
                   85                  90                  95
   Gln Lys Leu Ser Asn Met Glu Asn Arg Leu Lys Pro Phe Phe Thr Cys
               100                 105                 110
   Gly Ser Ala Asp Thr Cys
           115
<210> 736
   <211> 35
   <212> PRT
   <213> Artificial Sequence
<220>
   <223> Made in a lab
<400> 736
   Arg Thr Gln Ser Glu Glu Gln Gln Arg Arg Ala Leu Glu Gln Lys Leu
    1              5                   10                  15
   Ser Asn Met Glu Asn Arg Leu Lys Pro Phe Phe Thr Cys Gly Ser Ala
               20                  25                  30
   Asp Thr Cys
           35
<210> 737
   <211> 25
   <212> PRT
   <213> Artificial Sequence
<220>
   <223> Made in a lab
<400> 737
   Arg Thr Glu Gln Lys Leu Ser Asn Met Glu Asn Arg Leu Lys Pro Phe
    1              5                   10                  15
   Phe Thr Cys Gly Ser Ala Asp Thr Cys
               20                  25
<210> 738
   <211> 26
   <212> PRT
   <213> Artificial Sequence
<220>
   <223> Made in a lab
<400> 738
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 739
   <211> 16
   <212> PRT
   <213> Artificial Sequence
<220>
   <223> Made in a lab
<400> 739
   Gly Gly Gly Gly Ser Gly Gly Gly Gly Ser Gly Gly Gly Gly Ala Ser
   1               5                   10                  15
<210> 740
   <211> 16
   <212> PRT
   <213> Artificial Sequence
<220>
   <223> Made in a lab
<400> 740
   Ser Glu Pro Lys Ser Ser Asp Lys Thr His Thr Cys Pro Pro Cys Pro
   1               5                   10                  15
<210> 741
   <211> 18
   <212> PRT
   <213> Artificial Sequence
<220>
   <223> Made in a lab
<400> 741
   Gly Asp Gln Glu Pro Lys Ser Ser Asp Lys Thr His Thr Ser Pro Pro
   1               5                   10                  15
   Cys Pro
<210> 742
   <211> 21
   <212> PRT
   <213> Artificial Sequence
<220>
   <223> Made in a lab
<400> 742
   Gln Arg His Asn Asn Ser Ser Leu Asn Thr Gly Thr Gln Met Ala Gly
   1 5 10 15
   His Ser Pro Asn Ser
   20
<210> 743
   <211> 21
   <212> PRT
   <213> Artificial Sequence
<220>
   <223> Made in a lab
<400> 743
   Gln Arg His Asn Asn Ser Ser Leu Asn Thr Gly Thr Gln Lys Ala Arg
   1 5 10 15
   His Ser Pro Asn Ser
   20
<210> 744
   <211> 21
   <212> PRT
   <213> Artificial Sequence
<220>
   <223> Made in a lab
<400> 744
   Gln Arg His Asn Asn Ser Ser Leu Asn Thr Gly Thr Gln Met Ala Arg
   1 5 10 15
   His Ser Pro Asn Ser
   20
<210> 745
   <211> 21
   <212> PRT
   <213> Artificial Sequence
<220>
   <223> Made in a lab
<400> 745
   Gln Arg His Asn Asn Ser Ser Leu Asn Thr Arg Thr Gln Lys Ala Gly
   1 5 10 15
   His Ser Pro Asn Ser
   20
<210> 746
   <211> 21
   <212> PRT
   <213> Artificial Sequence
<220>
   <223> Made in a lab
<400> 746
   Gln Arg His Asn Asn Ser Ser Leu Asn Thr Arg Thr Gln Met Ala Gly
   1 5 10 15
   His Ser Pro Asn Ser
   20
<210> 747
   <211> 21
   <212> PRT
   <213> Artificial Sequence
<220>
   <223> Made in a lab
<400> 747
   Gln Arg His Asn Asn Ser Ser Leu Asn Thr Arg Thr Gln Met Ala Arg
   1 5 10 15
   His Ser Pro Asn Ser
   20
<210> 748
   <211> 10
   <212> PRT
   <213> Artificial Sequence
<220>
   <223> Hinge sequence
<400> 748
   Pro Pro Pro Pro Pro Cys Pro Pro Cys Pro
   1 5 10
<210> 749
   <211> 16
   <212> PRT
   <213> Artificial Sequence
<220>
   <223> Hinge sequence
<400> 749
   Thr Glu Pro Lys Ser Ser Asp Lys Thr His Thr Cys Pro Pro Cys Pro
   1 5 10 15
<210> 750
   <211> 20
   <212> PRT
   <213> Artificial Sequence
<220>
   <223> Hinge sequence
<400> 750
   Glu Pro Lys Ser Ser Asp Lys Thr His Thr Ser Pro Pro Ser Pro Cys
   1 5 10 15
   Pro Pro Cys Pro
   20
<210> 751
   <211> 20
   <212> PRT
   <213> Artificial Sequence
<220>
   <223> Hinge sequence
<400> 751
   Glu Pro Lys Ser Ser Asp Thr Pro Pro Pro Ser Pro Arg Ser Pro Cys
   1 5 10 15
   Pro Pro Cys Pro
   20
<210> 752
   <211> 23
   <212> PRT
   <213> Artificial Sequence
<220>
   <223> Hinge sequence
<400> 752
   Pro Ser Thr Pro Pro Thr Pro Ser Pro Ser Thr Pro Pro Thr Pro Ser
   1 5 10 15
   Pro Ser Cys Pro Pro Cys Pro
   20
<210> 753
   <211> 23
   <212> PRT
   <213> Artificial Sequence
<220>
   <223> Hinge sequence
<400> 753
   Arg Thr Glu Pro Arg Val Pro Ile Thr Gln Asn Pro Cys Pro Pro Leu
   1 5 10 15
   Lys Glu Cys Pro Pro Cys Ala
   20
<210> 754
   <211> 39
   <212> PRT
   <213> Artificial Sequence
<220>
   <223> Hinge sequence
<400> 754
   Glu Ser Pro Lys Ala Gln Ala Ser Ser Val Pro Thr Ala Gln Pro Gln
    1               5                  10                  15
   Ala Glu Gly Ser Leu Ala Lys Ala Thr Thr Ala Pro Ala Thr Thr Arg
               20                  25                  30
   Asn Thr Cys Pro Pro Cys Pro
           35
<210> 755
   <211> 50
   <212> PRT
   <213> Artificial Sequence
<220>
   <223> Hinge sequence
<400> 755
   Gln Glu Lys Glu Ala Ile Glu Arg Leu Lys Ala Ala Gly Ala Pro Glu
    1               5                  10                  15
   Ser Leu Val Ile Gln Ala Tyr Phe Ala Ser Glu Lys Asn Glu Asn Leu
               20                  25                  30
   Ala Ala Asn Phe Leu Leu Ser Gln Asn Phe Asp Asp Glu Cys Pro Pro
           35                  40                  45
   Cys Pro
       50
<210> 756
   <211> 52
   <212> PRT
   <213> Artificial Sequence
<220>
   <223> Hinge sequence
<400> 756
   Gln Glu Lys Glu Ala Ile Glu Arg Leu Lys Ala Ala Gly Ala Pro Glu
    1               5                  10                  15
   Ser Leu Val Ile Gln Ala Tyr Phe Ala Ser Glu Lys Asn Glu Asn Leu
               20                  25                  30
   Ala Ala Asn Phe Leu Leu Ser Gln Asn Phe Asp Asp Glu Cys Pro Pro
           35                  40                  45
   Cys Pro Ser Gly
       50
<210> 757
   <211> 110
   <212> PRT
   <213> Artificial Sequence
<220>
   <223> Hinge sequence
<400> 757
   Ser Arg Asp Phe Thr Pro Pro Thr Val Lys Ile Leu Gln Ser Ser Ser
    1               5                  10                  15
   Asp Gly Gly Gly His Phe Pro Pro Thr Ile Gln Leu Leu Cys Leu Val
               20                  25                  30
   Ser Gly Tyr Thr Pro Gly Thr Ile Asn Ile Thr Trp Leu Glu Asp Gly
           35                  40                  45
   Gln Val Met Asp Val Asp Leu Ser Thr Ala Ser Thr Thr Gln Glu Gly
       50                  55                  60
   Glu Leu Ala Ser Thr Gln Ser Glu Leu Thr Leu Ser Gln Lys His Trp
   65                  70                  75                  80
   Leu Ser Asp Arg Thr Tyr Thr Cys Gln Val Thr Tyr Gln Gly His Thr
                   85                  90                  95
   Phe Glu Asp Ser Thr Lys Lys Ser Ala Cys Pro Pro Cys Pro
               100                 105                 110
<210> 758
   <211> 111
   <212> PRT
   <213> Artificial Sequence
<220>
   <223> Hinge sequence
<400> 758
   Ser Arg Asp Phe Thr Pro Pro Thr Val Lys Ile Leu Gln Ser Ser Ser
    1               5                  10                  15
   Asp Gly Gly Gly His Phe Pro Pro Thr Ile Gln Leu Leu Cys Leu Val
               20                  25                  30
   Ser Gly Tyr Thr Pro Gly Thr Ile Asn Ile Thr Trp Leu Glu Asp Gly
           35                  40                  45
   Gln Val Met Asp Val Asp Leu Ser Thr Ala Ser Thr Thr Gln Glu Gly
       50                  55                  60
   Glu Leu Ala Ser Thr Gln Ser Glu Leu Thr Leu Ser Gln Lys His Trp
   65                  70                  75                  80
   Leu Ser Asp Arg Thr Tyr Thr Cys Gln Val Thr Tyr Gln Gly His Thr
                   85                  90                  95
   Phe Glu Asp Ser Thr Lys Lys Ser Ala Cys Pro Pro Cys Ser Gly
               100                 105                 110
<210> 759
   <211> 116
   <212> PRT
   <213> Artificial Sequence
<220>
   <223> Hinge sequence
<400> 759
   Ile Ala Glu Leu Pro Pro Lys Val Ser Val Phe Val Pro Pro Arg Asp
   1 5 10 15
   Gly Phe Phe Gly Asn Pro Arg Lys Ser Lys Leu Ile Cys Gln Ala Thr
   20 25 30
   Gly Phe Ser Pro Arg Gln Ile Gln Val Ser Trp Leu Arg Glu Gly Lys
   35 40 45
   Gln Val Gly Ser Gly Val Thr Thr Asp Gln Val Gln Ala Glu Ala Lys
   50 55 60
   Glu Ser Gly Pro Thr Thr Tyr Lys Val Thr Ser Thr Leu Thr Ile Lys
   65 70 75 80
   Glu Ser Asp Trp Leu Gly Gln Ser Met Phe Thr Cys Arg Val Asp His
   85 90 95
   Arg Gly Leu Thr Phe Gln Gln Asn Ala Ser Ser Met Ser Val Pro Cys
   100 105 110
   Pro Pro Cys Pro
   115
<210> 760
   <211> 6
   <212> PRT
   <213> Artificial Sequence
<220>
   <223> linker sequence of monoIL-10
<400> 760
   Gly Gly Gly Ser Gly Gly
   1 5
<210> 761
   <211> 106
   <212> PRT
   <213> Homo sapiens
<400> 761
<210> 762
   <211> 216
   <212> PRT
   <213> Artificial Sequence
<220>
   <223> IgG1 CH2 CH3 Null2 desLys
<400> 762
   Ala Pro Glu Ala Ala Gly Ala Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Ala Tyr Ala Cys Ala Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly
       210                 215
<210> 763
   <211> 7
   <212> PRT
   <213> Artificial Sequence
<220>
   <223> Peptide linker
<400> 763
   Ser Pro Pro Ser Pro Asn Ser
    1               5
<210> 764
   <211> 17
   <212> PRT
   <213> Artificial Sequence
<220>
   <223> Peptide linker
<400> 764
   Glu Pro Thr Ser Thr Asp Lys Thr His Thr Ser Pro Pro Ser Pro Asn
   1 5 10 15
   Ser
<210> 765
   <211> 17
   <212> PRT
   <213> Artificial Sequence
<220>
   <223> Peptide linker
<400> 765
   Glu Pro Thr Ser Thr Asp Lys Thr His Thr Cys Pro Pro Cys Pro Asn
   1 5 10 15
   Ser
<210> 766
   <211> 16
   <212> PRT
   <213> Artificial Sequence
<220>
   <223> Peptide linker
<400> 766
   Asn Gln Glu Val Gln Ile Pro Leu Thr Glu Ser Tyr Ser Pro Asn Ser
   1 5 10 15
<210> 767
   <211> 20
   <212> PRT
   <213> Artificial Sequence
<220>
   <223> Peptide linker
<400> 767
   Asn Ser Gly Ala Asn Gln Glu Val Gln Ile Pro Leu Thr Glu Ser Tyr
   1 5 10 15
   Ser Pro Asn Ser
   20
<210> 768
   <211> 14
   <212> PRT
   <213> Artificial Sequence
<220>
   <223> Peptide linker
<400> 768
   Glu Val Gln Ile Pro Leu Thr Glu Ser Tyr Ser Pro Asn Ser
   1 5 10
<210> 769
   <211> 20
   <212> PRT
   <213> Artificial Sequence
<220>
   <223> Peptide linker
<400> 769
   Asn Ser Gly Phe Asn Gln Glu Val Gln Ile Pro Leu Thr Glu Ser Tyr
    1               5                   10                  15
   Ser Pro Asn Ser
               20
<210> 770
   <211> 20
   <212> PRT
   <213> Artificial Sequence
<220>
   <223> Peptide linker
<400> 770
   Asn Ser Leu Ala Asn Gln Glu Val Gln Ile Pro Leu Thr Glu Ser Tyr
    1               5                   10                  15
   Ser Pro Asn Ser
               20
<210> 771
   <211> 20
   <212> PRT
   <213> Artificial Sequence
<220>
   <223> Peptide linker
<400> 771
   Asn Ser Leu Phe Ser Gln Glu Val Gln Ile Pro Leu Thr Glu Ser Tyr
    1               5                   10                  15
   Ser Pro Asn Ser
               20
<210> 772
   <211> 20
   <212> PRT
   <213> Artificial Sequence
<220>
   <223> Peptide linker
<400> 772
   Asn Ser Leu Ala Ser Gln Glu Val Gln Ile Pro Leu Thr Glu Ser Tyr
   1 5 10 15
   Ser Pro Asn Ser
   20
<210> 773
   <211> 26
   <212> PRT
   <213> Artificial Sequence
<220>
   <223> Peptide linker
<400> 773
   Glu Gln Lys Leu Ser Asn Met Glu Asn Arg Leu Lys Pro Phe Phe Thr
   1 5 10 15
   Ser Gly Ser Ala Asp Thr Ser Pro Asn Ser
   20 25
<210> 774
   <211> 21
   <212> PRT
   <213> Artificial Sequence
<220>
   <223> Peptide linker
<400> 774
   Gln Arg His Ala Asn Ser Ala Leu Asn Thr Gly Thr Gln Met Ala Gly
   1 5 10 15
   His Ser Pro Asn Ser
   20
<210> 775
   <211> 21
   <212> PRT
   <213> Artificial Sequence
<220>
   <223> Peptide linker
<400> 775
   Gln Arg His Asn Ala Ala Ser Leu Asn Thr Gly Thr Gln Met Ala Gly
   1 5 10 15
   His Ser Pro Asn Ser
   20
<210> 776
   <211> 21
   <212> PRT
   <213> Artificial Sequence
<220>
   <223> Peptide linker
<400> 776
   Gln Arg His Ala Ala Ser Ser Leu Asn Thr Gly Thr Gln Met Ala Gly
   1 5 10 15
   His Ser Pro Asn Ser
   20
<210> 777
   <211> 19
   <212> PRT
   <213> Artificial Sequence
<220>
   <223> Peptide linker
<400> 777
   Gln Arg His Ser Ser Leu Asn Thr Gly Thr Gln Met Ala Gly His Ser
   1 5 10 15
   Pro Asn Ser
<210> 778
   <211> 21
   <212> PRT
   <213> Artificial Sequence
<220>
   <223> Peptide linker
<400> 778
   Gln Arg His Gly Gly Ser Ser Leu Asn Thr Gly Thr Gln Met Ala Gly
   1 5 10 15
   His Ser Pro Asn Ser
   20
<210> 779
   <211> 11
   <212> PRT
   <213> Artificial Sequence
<220>
   <223> Peptide linker
<400> 779
   His Asp Gly Ser Ser Leu Asn Thr Pro Asn Ser
   1 5 10
<210> 780
   <211> 16
   <212> PRT
   <213> Artificial Sequence
<220>
   <223> Peptide linker
<400> 780
   Ser Ser Leu Asn Thr Gly Thr Gln Met Ala Gly His Ser Pro Asn Ser
   1 5 10 15
<210> 781
   <211> 16
   <212> PRT
   <213> Artificial Sequence
<220>
   <223> Peptide linker
<400> 781
   Ser Ser Leu Asn Thr Arg Thr Gln Met Ala Gly His Ser Pro Asn Ser
   1 5 10 15
<210> 782
   <211> 16
   <212> PRT
   <213> Artificial Sequence
<220>
   <223> Peptide linker
<400> 782
   Ser Ser Leu Asn Thr Gly Thr Gln Met Ala Arg His Ser Pro Asn Ser
   1 5 10 15
<210> 783
   <211> 16
   <212> PRT
   <213> Artificial Sequence
<220>
   <223> Peptide linker
<400> 783
   Ser Ser Leu Asn Thr Gly Thr Gln Lys Ala Gly His Ser Pro Asn Ser
   1 5 10 15
<210> 784
   <211> 16
   <212> PRT
   <213> Artificial Sequence
<220>
   <223> Peptide linker
<400> 784
   Ser Ser Leu Asn Thr Gly Thr Gln Lys Ala Arg His Ser Pro Asn Ser
   1 5 10 15
<210> 785
   <211> 16
   <212> PRT
   <213> Artificial Sequence
<220>
   <223> Peptide linker
<400> 785
   Ser Ser Leu Asn Thr Arg Thr Gln Met Ala Arg His Ser Pro Asn Ser
   1 5 10 15
<210> 786
   <211> 16
   <212> PRT
   <213> Artificial Sequence
<220>
   <223> Peptide linker
<400> 786
   Ser Ser Leu Asn Thr Arg Thr Gln Lys Ala Gly His Ser Pro Asn Ser
   1 5 10 15
<210> 787
   <211> 17
   <212> PRT
   <213> Artificial Sequence
<220>
   <223> Peptide linker
<400> 787
   Asn Ser Leu Phe Asn Gln Glu Val Gln Ile Pro Leu Thr Glu Pro Asn
   1 5 10 15
   Ser
<210> 788
   <211> 14
   <212> PRT
   <213> Artificial Sequence
<220>
   <223> Peptide linker
<400> 788
   Asn Ser Leu Phe Asn Gln Glu Val Gln Ile Pro Pro Asn Ser
   1 5 10
<210> 789
   <211> 11
   <212> PRT
   <213> Artificial Sequence
<220>
   <223> Peptide linker
<400> 789
   Asn Ser Leu Phe Asn Gln Glu Val Pro Asn Ser
   1 5 10
<210> 790
   <211> 13
   <212> PRT
   <213> Artificial Sequence
<220>
   <223> Peptide linker
<400> 790
   Gln Arg His Asn Asn Ser Ser Leu Asn Thr Pro Asn Ser
   1 5 10
<210> 791
   <211> 19
   <212> PRT
   <213> Artificial Sequence
<220>
   <223> Peptide linker
<400> 791
   Ser Gly Gly Gly Gly Ser Gly Gly Gly Gly Ser Gly Gly Gly Gly Ser
   1 5 10 15
   Pro Asn Ser
<210> 792
   <211> 2730
   <212> DNA
   <213> Artificial Sequence
<220>
   <223> TSC038 comprises: G19-4 (anti-CD3) scFv, human IgG1 SCC-P hinge, human IgG1 CH2, human IgG1 CH3, human Ck(YAE), H75 linker and 3D1 (anti-CD86) scFv
<400> 792
   acgactcact atagggagac ccaagcttgc cgccatggaa gcaccagcgc agcttctctt 60
   cctcctgcta ctctggctcc cagataccac cggtgaggtc cagctgcaac agtctggacc 120
   tgaactggtg aagcctggag cttcaatgaa gatttcctgc aaggcctctg gttactcatt 180
   cactggctac atcgtgaact ggctgaagca gagccatgga aagaaccttg agtggattgg 240
   acttattaat ccatacaaag gtcttactac ctacaaccag aaattcaagg gcaaggccac 300
   attaactgta gacaagtcat ccagcacagc ctacatggag ctcctcagtc tgacatctga 360
   agactctgca gtctattact gtgcaagatc tgggtactat ggtgactcgg actggtactt 420
   cgatgtctgg ggcgcaggga ccacggtcac cgtctcaagc ggtggcggag ggtctggggg 480
   tggcggatcc ggaggtggtg gctctgcaca agacatccag atgacacaga ctacatcctc 540
   cctgtctgcc tctctgggag acagagtcac catcagttgc agggcaagtc aggacattcg 600
   caattattta aactggtatc agcagaaacc agatggaact gttaaactcc tgatctacta 660
   cacatcaaga ttacactcag gagtcccatc aaggttcagt ggcagtgggt ctggaacaga 720
   ttattctctc accattgcca acctgcaacc agaagatatt gccacttact tttgccaaca 780
   gggtaatacg cttccgtgga cgttcggtgg aggcaccaaa ctggtaacca aacgctcgag 840
   cgagcccaaa tcttctgaca aaactcacac atgcccaccg tgcccatccg gagcacctga 900
   actcctgggt ggaccgtcag tcttcctctt ccccccaaaa cccaaggaca ccctcatgat 960
   ctcccggacc cctgaggtca catgcgtggt ggtggacgtg agccacgaag accctgaggt 1020
   caagttcaac tggtacgtgg acggcgtgga ggtgcataat gccaagacaa agccgcggga 1080
   ggagcagtac aacagcacgt accgtgtggt cagcgtcctc accgtcctgc accaggactg 1140
   gctgaatggc aaggagtaca agtgcaaggt ctccaacaaa gccctcccag cccccatcga 1200
   gaaaaccatc tccaaagcca aagggcagcc ccgagaacca caggtgtaca ccctgccccc 1260
   atcccgggat gagctgacca agaaccaggt cagcctgacc tgcctggtca aaggcttcta 1320
   tccaagcgac atcgccgtgg agtgggagag caatgggcag ccggagaaca actacaagac 1380
   cacgcctccc gtgctggact ccgacggctc cttcttcctc tacagcaagc tcaccgtgga 1440
   caagagcagg tggcagcagg ggaacgtctt ctcatgctcc gtgatgcatg aggctctgca 1500
   caaccactac acgcagaaga gcctctccct gtctccgggt aaatctagaa ctgtggctgc 1560
   accatctgtc ttcatcttcc cgccatctga tgagcagttg aaatctggaa ctgcctctgt 1620
   tgtgtgcctg ctgaattact tctatcccag agaggccaaa gtacagtgga aggtggataa 1680
   cgccctccaa tcgggtaact cccaggagag tgccacagag caggacagca aggacagcac 1740
   ctacagcctc agcagcgagc tgacgctgag caaagcagac tacgagaaac acaaagtcta 1800
   cgcctgcgaa gtcacccatc agggcctgag ctcgcccgtc acaaagagct tcaacagggg 1860
   agagcagagg cacaacaatt cttccctgaa tacaggaact cagatggcag gtcattctcc 1920
   gaattctcag gtccagctgc agcagtctgg gcctgagctg gtgaggcctg gggaatcagt 1980
   gaagatttcc tgcaagggtt ccggctacac attcactgat tatgctatac agtgggtgaa 2040
   gcagagtcat gcaaagagtc tagagtggat tggagttatt aatatttact atgataatac 2100
   aaactacaac cagaagttta agggcaaggc cacaatgact gtagacaaat cctccagcac 2160
   agcctatatg gaacttgcca gattgacatc tgaggattct gccatctatt actgtgcaag 2220
   agcggcctgg tatatggact actggggtca aggaacctca gtcaccgtct cctcaggggg 2280
   tggaggctct ggtggcggtg gctctggcgg aggtggatcc ggtggcggcg gatctgacat 2340
   tgtgctgtca cagtctccat cctccctggc tgtgtcagca ggagagaagg tcactatgag 2400
   ctgcaaatcc agtcagagtc tgctcaacag tagaacccga gagaactact tggcttggta 2460
   ccagcagaaa ccagggcagt ctcctaaact gctgatctac tgggcatcca ctagggaatc 2520
   tggggtccct gatcgcttca caggcagtgg atctgggaca gatttcactc tcaccatcag 2580
   cagtgtgcag gctgaagacc tggcagttta ttactgcacg caatcttata atctttacac 2640
   gttcggaggg gggaccaagc tggaaataaa ataatctaga gcggccgctc tagagggccc 2700
   tattctatag tgtcacctaa atgctagagg                                  2730
<210> 793
   <211> 1821
   <212> DNA
   <213> Artificial Sequence
<220>
   <223> TSC039 comprises: BMA031 (anti-TCR) scFv, human IgG1 SCC-P hinge, human IgG1 CH2, human IgG1 CH3, and human Ck(YAE)
<400> 793
   atggaagcac cagcgcagct tctcttcctc ctgctactct ggctcccaga taccaccggt 60
   ggacaggtcc aactagttca gtctggagca gaggttaaga agcctgggag ctcagtgaag 120
   gtttcctgca aggcttccgg atataaattc actagctatg ttatgcactg ggtgaagcag 180
   gcacctggcc aggggctcga gtggattgga tatattaatc cttacaatga tgttactaag 240
   tacaatgaga agttcaaagg caaggccaca cttacggccg acgagtccac taatacagcc 300
   tacatggagc tgagcagcct gagatctgag gacactgcgg tccattactg tgcaagaggg 360
   agctactatg attacgacgg gtttgtttac tggggccaag gtaccctggt caccgtctca 420
   agcggtggcg gagggtctgg gggtggcgga tccggaggtg gtggctctgc acaagatatc 480
   cagatgaccc aatctccatc gaccctgagt gcatctgtcg gggacagagt caccatgacc 540
   tgcagtgcca cctcaagtgt aagttacatg cactggtatc agcagaagcc cgggaaggct 600
   cccaaaagat ggatctatga cacatccaaa ctggcttctg gagtccctgc gcgcttcatt 660
   ggcagtgggt ctgggaccga gttcacgctc acaatcagca gcctgcagcc agatgatttc 720
   gctacgtatt actgccagca gtggagtagt aacccgctca cgttcggtgg aggtaccaag 780
   gtcgagatta aatcctcgag cgagcccaaa tcttctgaca aaactcacac atgcccaccg 840
   tgcccagcac ctgaactcct gggtggaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggag tacaagtgca aggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agaactgtgg ctgcaccatc tgtcttcatc ttcccgccat ctgatgagca gttgaaatct 1560
   ggaactgcct ctgttgtgtg cctgctgaat tacttctatc ccagagaggc caaagtacag 1620
   tggaaggtgg ataacgccct ccaatcgggt aactcccagg agagtgccac agagcaggac 1680
   agcaaggaca gcacctacag cctcagcagc gagctgacgc tgagcaaagc agactacgag 1740
   aaacacaaag tctacgcctg cgaagtcacc catcagggcc tgagctcgcc cgtcacaaag 1800
   agcttcaaca ggggagagtg a                                           1821
<210> 794
   <211> 1821
   <212> DNA
   <213> Artificial Sequence
<220>
   <223> TSC041 comprises: CRIS7 (anti-CD3) scFv, human IgG1 SCC-P hinge, human IgG1 CH2, human IgG1 CH3, and human Ck(YAE)
<400> 794
   atggaagcac cagcgcagct tctcttcctc ctgctactct ggctcccaga taccaccggt 60
   caggtccagc tggtgcagtc tgggggcgga gtggtgcagc ctgggcggtc actgaggctg 120
   tcctgcaagg cttctggcta cacctttact agatctacga tgcactgggt aaggcaggcc 180
   cctggaaagg gtctggaatg gattggatac attaatccta gcagtgctta tactaattac 240
   aatcagaaat tcaaggacag gttcacaatc agcgcagaca aatccaagag cacagccttc 300
   ctgcagatgg acagcctgag gcccgaggac accggcgtct atttctgtgc acggccccaa 360
   gtccactatg attacaacgg gtttccttac tggggccaag ggactcccgt cactgtctct 420
   agcggtggcg gagggtctgg gggtggcgga tccggaggtg gtggctctgc acaagacatc 480
   cagatgaccc agtctccaag cagcctgtct gcaagcgtgg gggacagggt caccatgacc 540
   tgcagtgcca gctcaagtgt aagttacatg aactggtacc agcagaagcc cggcaaggcc 600
   cccaaaagat ggatttatga ctcatccaaa ctggcttctg gagtccctgc tcgcttcagt 660
   ggcagtgggt ctgggaccga ctataccctc acaatcagca gcctgcagcc cgaagatttc 720
   gccacttatt actgccagca gtggagtcgt aacccaccca cgttcggagg ggggaccaag 780
   ctacaaatta cacgctcgag cgagcccaaa tcttctgaca aaactcacac atgcccaccg 840
   tgcccagcac ctgaactcct gggtggaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggag tacaagtgca aggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agaactgtgg ctgcaccatc tgtcttcatc ttcccgccat ctgatgagca gttgaaatct 1560
   ggaactgcct ctgttgtgtg cctgctgaat tacttctatc ccagagaggc caaagtacag 1620
   tggaaggtgg ataacgccct ccaatcgggt aactcccagg agagtgccac agagcaggac 1680
   agcaaggaca gcacctacag cctcagcagc gagctgacgc tgagcaaagc agactacgag 1740
   aaacacaaag tctacgcctg cgaagtcacc catcagggcc tgagctcgcc cgtcacaaag 1800
   agcttcaaca ggggagagtg a                                           1821
<210> 795
   <211> 1773
   <212> DNA
   <213> Artificial Sequence
<220>
   <223> TSC043 comprises: OKT3-M (Micromet variant anti-CD3, see also US 7,635,472) scFv, human IgG1 SCC-P hinge, human IgG1 CH2, human IgG1 CH3, and human Ck(YAE)
<400> 795
   atggaagcac cagcgcagct tctcttcctc ctgctactct ggctcccaga taccaccggt 60
   gatatcaaac tgcagcagtc aggggctgaa ctggcaagac ctggggcctc agtgaagatg 120
   tcctgcaaga cttctggcta cacctttact aggtacacga tgcactgggt aaaacagagg 180
   cctggacagg gtctggaatg gattggatac attaatccta gccgtggtta tactaattac 240
   aatcagaagt tcaaggacaa ggccacattg actacagaca aatcctccag cacagcctac 300
   atgcaactga gcagcctgac atctgaggac tctgcagtct attactgtgc aagatattat 360
   gatgatcatt actgccttga ctactggggc caaggcacca ctctcacagt ctcctcagtc 420
   gaaggtggaa gtggaggttc tggtggaagt ggaggttcag gtggagtcga cgacattcag 480
   ctgacccagt ctccagcaat catgtctgca tctccagggg agaaggtcac catgacctgc 540
   agagccagtt caagtgtaag ttacatgaac tggtaccagc agaagtcagg cacctccccc 600
   aaaagatgga tttatgacac atccaaagtg gcttctggag tcccttatcg cttcagtggc 660
   agtgggtctg ggacctcata ctctctcaca atcagcagca tggaggctga agatgctgcc 720
   acttattact gccaacagtg gagtagctcg agcgagccca aatcttctga caaaactcac 780
   acatgcccac cgtgcccagc acctgaactc ctgggtggac cgtcagtctt cctcttcccc 840
   ccaaaaccca aggacaccct catgatctcc cggacccctg aggtcacatg cgtggtggtg 900
   gacgtgagcc acgaagaccc tgaggtcaag ttcaactggt acgtggacgg cgtggaggtg 960
   cataatgcca agacaaagcc gcgggaggag cagtacaaca gcacgtaccg tgtggtcagc 1020
   gtcctcaccg tcctgcacca ggactggctg aatggcaagg agtacaagtg caaggtctcc 1080
   aacaaagccc tcccagcccc catcgagaaa accatctcca aagccaaagg gcagccccga 1140
   gaaccacagg tgtacaccct gcccccatcc cgggatgagc tgaccaagaa ccaggtcagc 1200
   ctgacctgcc tggtcaaagg cttctatcca agcgacatcg ccgtggagtg ggagagcaat 1260
   gggcagccgg agaacaacta caagaccacg cctcccgtgc tggactccga cggctccttc 1320
   ttcctctaca gcaagctcac cgtggacaag agcaggtggc agcaggggaa cgtcttctca 1380
   tgctccgtga tgcatgaggc tctgcacaac cactacacgc agaagagcct ctccctgtct 1440
   ccgggtaaat ctagaactgt ggctgcacca tctgtcttca tcttcccgcc atctgatgag 1500
   cagttgaaat ctggaactgc ctctgttgtg tgcctgctga attacttcta tcccagagag 1560
   gccaaagtac agtggaaggt ggataacgcc ctccaatcgg gtaactccca ggagagtgcc 1620
   acagagcagg acagcaagga cagcacctac agcctcagca gcgagctgac gctgagcaaa 1680
   gcagactacg agaaacacaa agtctacgcc tgcgaagtca cccatcaggg cctgagctcg 1740
   cccgtcacaa agagcttcaa caggggagag tga                              1773
<210> 796
   <211> 2088
   <212> DNA
   <213> Artificial Sequence
<220>
   <223> TSC049 comprises: HD37 (anti-CD19) scFv, human IgG1 SCC-P hinge, human IgG1 CH2(ADCC/CDC null), human IgG1 CH3, and human IgG1 CH1
<400> 796
   cgaaattaat acgactcact atagggagac ccaagcttgc cgccatggaa gcaccagcgc 60
   agcttctctt cctcctgcta ctctggctcc cagataccac cggtgacatt gtgctgaccc 120
   aatctccagc ttctttggct gtgtctctag ggcagagggc caccatctcc tgcaaggcca 180
   gccaaagtgt tgattatgat ggtgatagtt atttgaactg gtaccaacag attccaggac 240
   agccacccaa actcctcatc tatgatgcat ccaatctagt ttctgggatc ccacccaggt 300
   ttagtggcag tgggtctggg acagacttca ccctcaacat ccatcctgtg gagaaggtgg 360
   atgctgcaac ctatcactgc cagcaaagta ctgaggatcc gtggacgttc ggtggaggca 420
   ccaagctgga aatcaaaggt ggcggtggtt cgggcggtgg tgggtcgggt ggcggcggag 480
   ctagccaggt tcagctgcag cagtctgggg ctgagctggt gaggcctggg tcctcagtga 540
   agatttcctg caaggcttct ggctatgcat tcagtagcta ctggatgaac tgggtgaagc 600
   agaggcctgg acagggtctt gagtggattg gacagatttg gcctggagat ggtgatacta 660
   actacaatgg aaagttcaag ggtaaagcca ctctgactgc agacgaatcc tccagcacag 720
   cctacatgca actcagcagc ctagcatctg aggactctgc ggtctatttc tgtgcaagac 780
   gggagactac gacggtaggc cgttattact atgctatgga ctactggggt caaggaacct 840
   cagtcaccgt ctcctcgagt gagcccaaat cttctgacaa aactcacaca tgcccaccgt 900
   gcccagcacc tgaagccgcg ggtgcaccgt cagtcttcct cttcccccca aaacccaagg 960
   acaccctcat gatctcccgg acccctgagg tcacatgcgt ggtggtggac gtgagccacg 1020
   aagaccctga ggtcaagttc aactggtacg tggacggcgt ggaggtgcat aatgccaaga 1080
   caaagccgcg ggaggagcag tacaacagca cgtaccgtgt ggtcagcgtc ctcaccgtcc 1140
   tgcaccagga ctggctgaat ggcaaggcgt acgcgtgcgc ggtctccaac aaagccctcc 1200
   cagcccccat cgagaaaacc atctccaaag ccaaagggca gccccgagaa ccacaggtgt 1260
   acaccctgcc cccatcccgg gatgagctga ccaagaacca ggtcagcctg acctgcctgg 1320
   tcaaaggctt ctatccaagc gacatcgccg tggagtggga gagcaatggg cagccggaga 1380
   acaactacaa gaccacgcct cccgtgctgg actccgacgg ctccttcttc ctctacagca 1440
   agctcaccgt ggacaagagc aggtggcagc aggggaacgt cttctcatgc tccgtgatgc 1500
   atgaggctct gcacaaccac tacacgcaga agagcctctc cctgtctccg ggtaaatcta 1560
   gagcctccac caagggccca tcggtcttcc ccctggcacc ctcctccaag agcacctctg 1620
   ggggcacagc ggccctgggc tgcctggtca aggactactt ccccgagccg gtgacggtgt 1680
   cgtggaactc aggcgccctg accagcggcg tgcacacctt cccggctgtc ctacagtcct 1740
   caggactcta ctccctcagc agcgtggtga ccgtgccctc cagcagcttg ggcacccaga 1800
   cctacatctg caacgtgaat cacaagccca gcaacaccaa ggtggacaag aaagtttgat 1860
   accactacat atcgcggccg ctctagaggg ccctattcta tagtgtcacc taaatgctag 1920
   agctcgctga tctgcctcga ctgtgccttc tagttgccag ccatctgttg tttgcccctc 1980
   ccccgtgcct tccttgaccc tggaaggtgc cactcccact gtcctttcct aataaaatga 2040
   ggaaattgca tcgcattgtc tgagtaggtg tcattctatt ctgggggg 2088
<210> 797
   <211> 2067
   <212> DNA
   <213> Artificial Sequence
<220>
   <223> TSC050 comprises: 2H7 (anti-CD20) scFv, human IgG1 SCC-P hinge, human IgG1 CH2(ADCC/CDC null), human IgG1 CH3, and human IgG1 CH1
<400> 797
   cgaaattaat acgactcact atagggagac ccaagcttgc cgccatggaa gcaccagcgc 60
   agcttctctt cctcctgcta ctctggctcc cagataccac cggtcaaatt gttctctccc 120
   agtctccagc aatcctgtct gcatctccag gggagaaggt cacaatgact tgcagggcca 180
   gctcaagtgt aagttacatg cactggtacc agcagaagcc aggatcctcc cccaaaccct 240
   ggatttatgc cccatccaac ctggcttctg gagtccctgc tcgcttcagt ggcagtgggt 300
   ctgggacctc ttactctctc acaatcagca gagtggaggc tgaagatgct gccacttatt 360
   actgccagca gtggagtttt aacccaccca cgttcggtgc tgggaccaag ctggagctga 420
   aagatggcgg tggctcgggc ggtggtggat ctggaggagg tggggctagc caggcttatc 480
   tacagcagtc tggggctgag tcggtgaggc ctggggcctc agtgaagatg tcctgcaagg 540
   cttctggcta cacatttacc agttacaata tgcactgggt aaagcagaca cctagacagg 600
   gcctggaatg gattggagct atttatccag gaaatggtga tacttcctac aatcagaagt 660
   tcaagggcaa ggccacactg actgtagaca aatcctccag cacagcctac atgcagctca 720
   gcagcctgac atctgaagac tctgcggtct atttctgtgc aagagtggtg tactatagta 780
   actcttactg gtacttcgat gtctggggca cagggaccac ggtcaccgtc tcctcgagcg 840
   agcccaaatc ttctgacaaa actcacacat gcccaccgtg cccagcacct gaagccgcgg 900
   gtgcaccgtc agtcttcctc ttccccccaa aacccaagga caccctcatg atctcccgga 960
   cccctgaggt cacatgcgtg gtggtggacg tgagccacga agaccctgag gtcaagttca 1020
   actggtacgt ggacggcgtg gaggtgcata atgccaagac aaagccgcgg gaggagcagt 1080
   acaacagcac gtaccgtgtg gtcagcgtcc tcaccgtcct gcaccaggac tggctgaatg 1140
   gcaaggcgta cgcgtgcgcg gtctccaaca aagccctccc agcccccatc gagaaaacca 1200
   tctccaaagc caaagggcag ccccgagaac cacaggtgta caccctgccc ccatcccggg 1260
   atgagctgac caagaaccag gtcagcctga cctgcctggt caaaggcttc tatccaagcg 1320
   acatcgccgt ggagtgggag agcaatgggc agccggagaa caactacaag accacgcctc 1380
   ccgtgctgga ctccgacggc tccttcttcc tctacagcaa gctcaccgtg gacaagagca 1440
   ggtggcagca ggggaacgtc ttctcatgct ccgtgatgca tgaggctctg cacaaccact 1500
   acacgcagaa gagcctctcc ctgtctccgg gtaaatctag agcctccacc aagggcccat 1560
   cggtcttccc cctggcaccc tcctccaaga gcacctctgg gggcacagcg gccctgggct 1620
   gcctggtcaa ggactacttc cccgagccgg tgacggtgtc gtggaactca ggcgccctga 1680
   ccagcggcgt gcacaccttc ccggctgtcc tacagtcctc aggactctac tccctcagca 1740
   gcgtggtgac cgtgccctcc agcagcttgg gcacccagac ctacatctgc aacgtgaatc 1800
   acaagcccag caacaccaag gtggacaaga aagtttgata ccactacata tcgcggccgc 1860
   tctagagggc cctattctat agtgtcacct aaatgctaga gctcgctgat ctgcctcgac 1920
   tgtgccttct agttgccagc catctgttgt ttgcccctcc cccgtgcctt ccttgaccct 1980
   ggaaggtgcc actcccactg tcctttccta ataaaatgag gaaattgcat cgcattgtct 2040
   gagtaggtgt cattctattc tgggggg                                    2067
<210> 798
   <211> 2055
   <212> DNA
   <213> Artificial Sequence
<220>
   <223> TSC051 comprises: P2C2 (anti-CD79) scFv, human IgG1 SCC-P hinge, human IgG1 CH2(ADCC/CDC null), human IgG1 CH3, and human IgG1 CH1
<400> 798
   cgaaattaat acgactcact atagggagac ccaagcttgc cgccatggaa gcaccagcgc 60
   agcttctctt cctcctgcta ctctggctcc cagataccac cggtgatatt gtgatgatcc 120
   aggatgaact ctccaatcct gtcagttctg gagaatcagt ttccatctcc tgtaggtcta 180
   gtaagagtct cctagataag gaagggaaga catacttgaa ttggtttctg cagagaccag 240
   gacaatctcc tcagctcctg atctatctga tgtccatgcg tgaatcagga gtctcagacc 300
   ggtttagtgg cagtgggtca ggaacagatt tcaccctgga aatcagtaga gtgaaggctg 360
   aggatgtggg tgtgtattac tgtcaacaac ttgtagagta tccgtggacg ttcggtggag 420
   gcaccaagct ggaaatcaaa ggtggcggtg gctctggcgg aggtggatcc ggtggcggcg 480
   gctctcagat ccagttggtg cagtctggac ctgagctgaa gaagcctgga gagacagtca 540
   agatctcctg caaggcttct ggttatacct tcacagactt ttcaatgcac tgggtgaggc 600
   aggctccagg aaagggttta aagtggatgg gctggataaa cactgagact ggtgagccaa 660
   catatgcaga tgacttcagg ggacggtttg ccttctcttt ggaaacttct gccagcactg 720
   cctatttgca gatcaacaac ctcaaaaatg aggacacggc tacatatttt tgtacctggt 780
   ctgcttactg gggccaaggg actctggtca ctgtctctgc ctcgagcgag cccaaatctt 840
   ctgacaaaac tcacacatgc ccaccgtgcc cagcacctga agccgcgggt gcaccgtcag 900
   tcttcctctt ccccccaaaa cccaaggaca ccctcatgat ctcccggacc cctgaggtca 960
   catgcgtggt ggtggacgtg agccacgaag accctgaggt caagttcaac tggtacgtgg 1020
   acggcgtgga ggtgcataat gccaagacaa agccgcggga ggagcagtac aacagcacgt 1080
   accgtgtggt cagcgtcctc accgtcctgc accaggactg gctgaatggc aaggcgtacg 1140
   cgtgcgcggt ctccaacaaa gccctcccag cccccatcga gaaaaccatc tccaaagcca 1200
   aagggcagcc ccgagaacca caggtgtaca ccctgccccc atcccgggat gagctgacca 1260
   agaaccaggt cagcctgacc tgcctggtca aaggcttcta tccaagcgac atcgccgtgg 1320
   agtgggagag caatgggcag ccggagaaca actacaagac cacgcctccc gtgctggact 1380
   ccgacggctc cttcttcctc tacagcaagc tcaccgtgga caagagcagg tggcagcagg 1440
   ggaacgtctt ctcatgctcc gtgatgcatg aggctctgca caaccactac acgcagaaga 1500
   gcctctccct gtctccgggt aaatctagag cctccaccaa gggcccatcg gtcttccccc 1560
   tggcaccctc ctccaagagc acctctgggg gcacagcggc cctgggctgc ctggtcaagg 1620
   actacttccc cgagccggtg acggtgtcgt ggaactcagg cgccctgacc agcggcgtgc 1680
   acaccttccc ggctgtccta cagtcctcag gactctactc cctcagcagc gtggtgaccg 1740
   tgccctccag cagcttgggc acccagacct acatctgcaa cgtgaatcac aagcccagca 1800
   acaccaaggt ggacaagaaa gtttgatacc actacatatc gcggccgctc tagagggccc 1860
   tattctatag tgtcacctaa atgctagagc tcgctgatct gcctcgactg tgccttctag 1920
   ttgccagcca tctgttgttt gcccctcccc cgtgccttcc ttgaccctgg aaggtgccac 1980
   tcccactgtc ctttcctaat aaaatgagga aattgcatcg cattgtctga gtaggtgtca 2040
   ttctattctg ggggg                                                  2055
<210> 799
   <211> 1870
   <212> DNA
   <213> Artificial Sequence
<220>
   <223> TSC052 comprises: 5D5 (anti-cMet) scFv, human IgG1 SCC-P hinge, human IgG1 CH2(ADCC/CDC null), human IgG1 CH3, and human IgG1 CH1
<400> 799
   cttatcgaat taatacgact cactataggg agacccaagc ttgccgccat ggaagcacca 60
   gcgcagcttc tcttcctcct gctactctgg ctcccagata ccaccggtga ctttatgatg 120
   tcacagtctc catcctccct aactgtgtca gttggagaga aggttactgt gagctgcaag 180
   tccagtcagt cccttttata tactagcagt cagaagaact acttggcctg gtaccagcag 240
   aaaccaggtc agtctcctaa actgctgatt tactgggcat ccactaggga atctggggtc 300
   cctgatcgct tcacaggcag tggatctggg acagatttca ctctcaccat caccagtgtg 360
   aaggctgacg acctggcagt ttattactgt cagcaatatt atgcctatcc gtggacgttc 420
   ggtggaggca ccaagttgga gctcaaaggt ggcggtggct ctggcggagg tggatccggt 480
   ggcggcggct ctcaggtcca actgcagcag tctgggcctg agctggtgag gcctggggct 540
   tcagtgaaga tgtcctgcag ggcttcgggc tataccttca ccagctactg gttgcactgg 600
   gttaaacaga ggcctggaca aggccttgag tggattggca tgattgatcc ttccaatagt 660
   gacactaggt ttaatccgaa cttcaaggac aaggccacat tgaatgtaga cagatcttcc 720
   aacacagcct acatgctgct cagcagcctg acatctgctg actctgcagt ctattactgt 780
   gccacatatg gtagctacgt ttcccctctg gactactggg gtcaaggaac ctcagtcacc 840
   gtctcctcga gcgagcccaa atcttctgac aaaactcaca catgcccacc gtgcccagca 900
   cctgaagccg cgggtgcacc gtcagtcttc ctcttccccc caaaacccaa ggacaccctc 960
   atgatctccc ggacccctga ggtcacatgc gtggtggtgg acgtgagcca cgaagaccct 1020
   gaggtcaagt tcaactggta cgtggacggc gtggaggtgc ataatgccaa gacaaagccg 1080
   cgggaggagc agtacaacag cacgtaccgt gtggtcagcg tcctcaccgt cctgcaccag 1140
   gactggctga atggcaaggc gtacgcgtgc gcggtctcca acaaagccct cccagccccc 1200
   atcgagaaaa ccatctccaa agccaaaggg cagccccgag aaccacaggt gtacaccctg 1260
   cccccatccc gggatgagct gaccaagaac caggtcagcc tgacctgcct ggtcaaaggc 1320
   ttctatccaa gcgacatcgc cgtggagtgg gagagcaatg ggcagccgga gaacaactac 1380
   aagaccacgc ctcccgtgct ggactccgac ggctccttct tcctctacag caagctcacc 1440
   gtggacaaga gcaggtggca gcaggggaac gtcttctcat gctccgtgat gcatgaggct 1500
   ctgcacaacc actacacgca gaagagcctc tccctgtctc cgggtaaatc tagagcctcc 1560
   accaagggcc catcggtctt ccccctggca ccctcctcca agagcacctc tgggggcaca 1620
   gcggccctgg gctgcctggt caaggactac ttccccgagc cggtgacggt gtcgtggaac 1680
   tcaggcgccc tgaccagcgg cgtgcacacc ttcccggctg tcctacagtc ctcaggactc 1740
   tactccctca gcagcgtggt gaccgtgccc tccagcagct tgggcaccca gacctacatc 1800
   tgcaacgtga atcacaagcc cagcaacacc aaggtggaca agaaagtttg ataccactac 1860
   atatcgcggc                                                        1870
<210> 800
   <211> 1827
   <212> DNA
   <213> Artificial Sequence
<220>
   <223> TSC053 comprises: G19-4 (anti CD3) scFv, human IgG1 SCC-P hinge, human IgG1 CH2(ADCC/CDC null), human IgG1 CH3, and human Ck(YAE)
<400> 800
   atggaagcac cagcgcagct tctcttcctc ctgctactct ggctcccaga taccaccggt 60
   gaggtccagc tgcaacagtc tggacctgaa ctggtgaagc ctggagcttc aatgaagatt 120
   tcctgcaagg cctctggtta ctcattcact ggctacatcg tgaactggct gaagcagagc 180
   catggaaaga accttgagtg gattggactt attaatccat acaaaggtct tactacctac 240
   aaccagaaat tcaagggcaa ggccacatta actgtagaca agtcatccag cacagcctac 300
   atggagctcc tcagtctgac atctgaagac tctgcagtct attactgtgc aagatctggg 360
   tactatggtg actcggactg gtacttcgat gtctggggcg cagggaccac ggtcaccgtc 420
   tcaagcggtg gcggagggtc tgggggtggc ggatccggag gtggtggctc tgcacaagac 480
   atccagatga cacagactac atcctccctg tctgcctctc tgggagacag agtcaccatc 540
   agttgcaggg caagtcagga cattcgcaat tatttaaact ggtatcagca gaaaccagat 600
   ggaactgtta aactcctgat ctactacaca tcaagattac actcaggagt cccatcaagg 660
   ttcagtggca gtgggtctgg aacagattat tctctcacca ttgccaacct gcaaccagaa 720
   gatattgcca cttacttttg ccaacagggt aatacgcttc cgtggacgtt cggtggaggc 780
   accaaactgg taaccaaacg ctcgagcgag cccaaatctt ctgacaaaac tcacacatgc 840
   ccaccgtgcc cagcacctga agccgcgggt gcaccgtcag tcttcctctt ccccccaaaa 900
   cccaaggaca ccctcatgat ctcccggacc cctgaggtca catgcgtggt ggtggacgtg 960
   agccacgaag accctgaggt caagttcaac tggtacgtgg acggcgtgga ggtgcataat 1020
   gccaagacaa agccgcggga ggagcagtac aacagcacgt accgtgtggt cagcgtcctc 1080
   accgtcctgc accaggactg gctgaatggc aaggcgtacg cgtgcgcggt ctccaacaaa 1140
   gccctcccag cccccatcga gaaaaccatc tccaaagcca aagggcagcc ccgagaacca 1200
   caggtgtaca ccctgccccc atcccgggat gagctgacca agaaccaggt cagcctgacc 1260
   tgcctggtca aaggcttcta tccaagcgac atcgccgtgg agtgggagag caatgggcag 1320
   ccggagaaca actacaagac cacgcctccc gtgctggact ccgacggctc cttcttcctc 1380
   tacagcaagc tcaccgtgga caagagcagg tggcagcagg ggaacgtctt ctcatgctcc 1440
   gtgatgcatg aggctctgca caaccactac acgcagaaga gcctctccct gtctccgggt 1500
   aaatctagaa ctgtggctgc accatctgtc ttcatcttcc cgccatctga tgagcagttg 1560
   aaatctggaa ctgcctctgt tgtgtgcctg ctgaattact tctatcccag agaggccaaa 1620
   gtacagtgga aggtggataa cgccctccaa tcgggtaact cccaggagag tgccacagag 1680
   caggacagca aggacagcac ctacagcctc agcagcgagc tgacgctgag caaagcagac 1740
   tacgagaaac acaaagtcta cgcctgcgaa gtcacccatc agggcctgag ctcgcccgtc 1800
   acaaagagct tcaacagggg agagtga 1827
<210> 801
   <211> 2707
   <212> DNA
   <213> Artificial Sequence
<220>
   <223> TSC064 comprises: G19-4 (anti-CD3) scFv, human IgG1 SCC-P hinge, human IgG1 CH2, human IgG1 CH3, human Ck(YAE), H75 linker, and Met021 (anti-c-Met) scFv
<400> 801
   atacgactca ctatagggag acccaagctt gccgccatgg aagcaccagc gcagcttctc 60
   ttcctcctgc tactctggct cccagatacc accggtgagg tccagctgca acagtctgga 120
   cctgaactgg tgaagcctgg agcttcaatg aagatttcct gcaaggcctc tggttactca 180
   ttcactggct acatcgtgaa ctggctgaag cagagccatg gaaagaacct tgagtggatt 240
   ggacttatta atccatacaa aggtcttact acctacaacc agaaattcaa gggcaaggcc 300
   acattaactg tagacaagtc atccagcaca gcctacatgg agctcctcag tctgacatct 360
   gaagactctg cagtctatta ctgtgcaaga tctgggtact atggtgactc ggactggtac 420
   ttcgatgtct ggggcgcagg gaccacggtc accgtctcaa gcggtggcgg agggtctggg 480
   ggtggcggat ccggaggtgg tggctctgca caagacatcc agatgacaca gactacatcc 540
   tccctgtctg cctctctggg agacagagtc accatcagtt gcagggcaag tcaggacatt 600
   cgcaattatt taaactggta tcagcagaaa ccagatggaa ctgttaaact cctgatctac 660
   tacacatcaa gattacactc aggagtccca tcaaggttca gtggcagtgg gtctggaaca 720
   gattattctc tcaccattgc caacctgcaa ccagaagata ttgccactta cttttgccaa 780
   cagggtaata cgcttccgtg gacgttcggt ggaggcacca aactggtaac caaacgctcg 840
   agcgagccca aatcttctga caaaactcac acatgcccac cgtgcccagc acctgaactc 900
   ctgggtggac cgtcagtctt cctcttcccc ccaaaaccca aggacaccct catgatctcc 960
   cggacccctg aggtcacatg cgtggtggtg gacgtgagcc acgaagaccc tgaggtcaag 1020
   ttcaactggt acgtggacgg cgtggaggtg cataatgcca agacaaagcc gcgggaggag 1080
   cagtacaaca gcacgtaccg tgtggtcagc gtcctcaccg tcctgcacca ggactggctg 1140
   aatggcaagg agtacaagtg caaggtctcc aacaaagccc tcccagcccc catcgagaaa 1200
   accatctcca aagccaaagg gcagccccga gaaccacagg tgtacaccct gcccccatcc 1260
   cgggatgagc tgaccaagaa ccaggtcagc ctgacctgcc tggtcaaagg cttctatcca 1320
   agcgacatcg ccgtggagtg ggagagcaat gggcagccgg agaacaacta caagaccacg 1380
   cctcccgtgc tggactccga cggctccttc ttcctctaca gcaagctcac cgtggacaag 1440
   agcaggtggc agcaggggaa cgtcttctca tgctccgtga tgcatgaggc tctgcacaac 1500
   cactacacgc agaagagcct ctccctgtct ccgggtaaat ctagaactgt ggctgcacca 1560
   tctgtcttca tcttcccgcc atctgatgag cagttgaaat ctggaactgc ctctgttgtg 1620
   tgcctgctga attacttcta tcccagagag gccaaagtac agtggaaggt ggataacgcc 1680
   ctccaatcgg gtaactccca ggagagtgcc acagagcagg acagcaagga cagcacctac 1740
   agcctcagca gcgagctgac gctgagcaaa gcagactacg agaaacacaa agtctacgcc 1800
   tgcgaagtca cccatcaggg cctgagctcg cccgtcacaa agagcttcaa caggggagag 1860
   cagaggcaca acaattcttc cctgaataca ggaactcaga tggcaggtca ttctccgaat 1920
   tctgacatcc agatgaccca gtctccatcc tccctgtctg catctgtagg agacagagtc 1980
   accatcactt gtcgggcaag tcagagcatt aggaactatt taaattggta tcagcaggaa 2040
   ccagggaaag cccctaagct cctgatctat gctgcatcca gtttgcaaag tggggtccca 2100
   tcaaggttca gtggcagtgg atctgggaca gacttcattc tcaccatcag cagtctgcag 2160
   cctgaagatt ttgcaactta ctactgtcaa cagagttacg ttaccccgct cactttcggc 2220
   ggagggacca aggtggagat caaagggggt ggaggctctg gtggcggtgg ctctggcgga 2280
   ggtggatccg gtggcggcgg ctctgaagtt caattgttag agtctggtgg cggtcttgtt 2340
   cagcctggtg gttctttacg tctttcttgc gctgcttccg gattcacttt ctctaagtac 2400
   gatatgcttt gggttcgcca agctcctggt aaaggtttgg agtgggtttc ttatatctat 2460
   ccttctggtg gccttactga gtatgctgac tccgttaaag gtcgcttcac tatctctaga 2520
   gacaactcta agaatactct ctacttgcag atgaacagct taagggctga ggacacggcc 2580
   gtgtattact gtgcgagacg ggctccccgg tccctttctt ttgatatctg gggccaaggg 2640
   acaatggtca ccgtctaagc ggccgctcta gagggcccta ttctatagtg tcacctaaat 2700
   gctagag 2707
<210> 802
   <211> 1812
   <212> DNA
   <213> Artificial Sequence
<220>
   <223> TSC076 comprises: OKT3 (anti-CD3) scFv, human IgG1 SCC-P hinge, human IgG1 CH2(ADCC/CDC null), human IgG1 CH3, and human Ck(YAE)
<400> 802
   atggaagcac cagcgcagct tctcttcctc ctgctactct ggctcccaga taccaccggt 60
   caggtccagc tgcagcagtc tggggctgaa ctggcaagac ctggggcctc agtgaagatg 120
   tcctgcaagg cttctggcta cacctttact aggtacacga tgcactgggt aaaacagagg 180
   cctggacagg gtctggaatg gattggatac attaatccta gccgtggtta tactaattac 240
   aatcagaagt tcaaggacaa ggccacattg actacagaca aatcctccag cacagcctac 300
   atgcaactga gcagcctgac atctgaggac tctgcagtct attactgtgc aagatattat 360
   gatgatcatt actgccttga ctactggggc caaggcacca cggtcaccgt ctcaagcggt 420
   ggcggagggt ctgggggtgg cggatccgga ggtggtggct ctgcacaaca aattgttctc 480
   acccagtctc cagcaatcat gtctgcatct ccaggggaga aggtcaccat gacctgcagt 540
   gccagctcaa gtgtaagtta catgaactgg taccagcaga agtcaggcac ctcccccaaa 600
   agatggattt atgacacatc caaactggct tctggagtcc ctgctcactt caggggcagt 660
   gggtctggga cctcttactc tctcacaatc agcggcatgg aggctgaaga tgctgccact 720
   tattactgcc agcagtggag tagtaaccca ttcacgttcg gctcggggac aaagttggaa 780
   ataaactcga gtgagcccaa atcttctgac aaaactcaca catgcccacc gtgcccagca 840
   cctgaagccg cgggtgcacc gtcagtcttc ctcttccccc caaaacccaa ggacaccctc 900
   atgatctccc ggacccctga ggtcacatgc gtggtggtgg acgtgagcca cgaagaccct 960
   gaggtcaagt tcaactggta cgtggacggc gtggaggtgc ataatgccaa gacaaagccg 1020
   cgggaggagc agtacaacag cacgtaccgt gtggtcagcg tcctcaccgt cctgcaccag 1080
   gactggctga atggcaaggc gtacgcgtgc gcggtctcca acaaagccct cccagccccc 1140
   atcgagaaaa ccatctccaa agccaaaggg cagccccgag aaccacaggt gtacaccctg 1200
   cccccatccc gggatgagct gaccaagaac caggtcagcc tgacctgcct ggtcaaaggc 1260
   ttctatccaa gcgacatcgc cgtggagtgg gagagcaatg ggcagccgga gaacaactac 1320
   aagaccacgc ctcccgtgct ggactccgac ggctccttct tcctctacag caagctcacc 1380
   gtggacaaga gcaggtggca gcaggggaac gtcttctcat gctccgtgat gcatgaggct 1440
   ctgcacaacc actacacgca gaagagcctc tccctgtctc cgggtaaatc tagaactgtg 1500
   gctgcaccat ctgtcttcat cttcccgcca tctgatgagc agttgaaatc tggaactgcc 1560
   tctgttgtgt gcctgctgaa ttacttctat cccagagagg ccaaagtaca gtggaaggtg 1620
   gataacgccc tccaatcggg taactcccag gagagtgcca cagagcagga cagcaaggac 1680
   agcacctaca gcctcagcag cgagctgacg ctgagcaaag cagactacga gaaacacaaa 1740
   gtctacgcct gcgaagtcac ccatcagggc ctgagctcgc ccgtcacaaa gagcttcaac 1800
   aggggagagt ga                                                     1812
<210> 803
   <211> 1821
   <212> DNA
   <213> Artificial Sequence
<220>
   <223> TSC077 comprises: HuM291 (anti-CD3) scFv, human IgG1 SCC-P hinge, human IgG1 CH2(ADCC/CDC null), human IgG1 CH3, and human Ck(YAE)
<400> 803
   atggaagcac cagcgcagct tctcttcctc ctgctactct ggctcccaga taccaccggt 60
   caggtccagc ttgtccagtc tggggctgaa gtcaagaaac ctggcgccag cgtgaaggtc 120
   tcctgcaagg cttctggcta cacctttatt agttacacga tgcattgggt aaggcaggcc 180
   cctggacagg gtctggaatg gatgggatat attaatccga gaagtgggta tactcattac 240
   aatcagaagt taaaggacaa ggcaacactt accgcggaca aatccgcgag cacagcctac 300
   atggaactga gcagcctgag atctgaggac accgcagtct attactgtgc aagatcggcc 360
   tactatgatt atgacggctt tgcttactgg ggccaaggaa ccctggtcac cgtctcaagc 420
   ggtggcggag ggtctggggg tggcggatcc ggaggtggtg gctctgcaca agatattcag 480
   atgacccagt ctccatcttc cctctctgct agcgtcgggg atagggtcac cataacctgc 540
   tctgccagct caagtgtaag ttacatgaac tggtaccagc agaagccagg caaagctccc 600
   aagagactaa tttatgacac atccaaactg gcttctggag tcccttctag gttcagtggc 660
   agtggatctg ggaccgattt caccctcaca atcagctctc tgcagccaga agatttcgcc 720
   acttattact gccagcaatg gagtagtaac ccacccacgt tcggtggagg gaccaaggtg 780
   gagatcaaac gaacctcgag tgagcccaaa tcttctgaca aaactcacac atgcccaccg 840
   tgcccagcac ctgaagccgc gggtgcaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggcg tacgcgtgcg cggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agaactgtgg ctgcaccatc tgtcttcatc ttcccgccat ctgatgagca gttgaaatct 1560
   ggaactgcct ctgttgtgtg cctgctgaat tacttctatc ccagagaggc caaagtacag 1620
   tggaaggtgg ataacgccct ccaatcgggt aactcccagg agagtgccac agagcaggac 1680
   agcaaggaca gcacctacag cctcagcagc gagctgacgc tgagcaaagc agactacgag 1740
   aaacacaaag tctacgcctg cgaagtcacc catcagggcc tgagctcgcc cgtcacaaag 1800
   agcttcaaca ggggagagtg a                                          1821
<210> 804
   <211> 1821
   <212> DNA
   <213> Artificial Sequence
<220>
   <223> TSC093 comprises from its amino to carboxy terminus: CRIS7 (anti-CD3) scFv, human IgG1 SCC-P hinge, human IgG1 CH2(ADCC/CDC null), human IgG1 CH3, and human Ck(YAE)
<400> 804
   atggaagcac cagcgcagct tctcttcctc ctgctactct ggctcccaga taccaccggt 60
   caggtccagc tggtgcagtc tgggggcgga gtggtgcagc ctgggcggtc actgaggctg 120
   tcctgcaagg cttctggcta cacctttact agatctacga tgcactgggt aaggcaggcc 180
   cctggaaagg gtctggaatg gattggatac attaatccta gcagtgctta tactaattac 240
   aatcagaaat tcaaggacag gttcacaatc agcgcagaca aatccaagag cacagccttc 300
   ctgcagatgg acagcctgag gcccgaggac accggcgtct atttctgtgc acggccccaa 360
   gtccactatg attacaacgg gtttccttac tggggccaag ggactcccgt cactgtctct 420
   agcggtggcg gagggtctgg gggtggcgga tccggaggtg gtggctctgc acaagacatc 480
   cagatgaccc agtctccaag cagcctgtct gcaagcgtgg gggacagggt caccatgacc 540
   tgcagtgcca gctcaagtgt aagttacatg aactggtacc agcagaagcc cggcaaggcc 600
   cccaaaagat ggatttatga ctcatccaaa ctggcttctg gagtccctgc tcgcttcagt 660
   ggcagtgggt ctgggaccga ctataccctc acaatcagca gcctgcagcc cgaagatttc 720
   gccacttatt actgccagca gtggagtcgt aacccaccca cgttcggagg ggggaccaag 780
   ctacaaatta cacgctcgag tgagcccaaa tcttctgaca aaactcacac atgcccaccg 840
   tgcccagcac ctgaagccgc gggtgcaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggcg tacgcgtgcg cggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agaactgtgg ctgcaccatc tgtcttcatc ttcccgccat ctgatgagca gttgaaatct 1560
   ggaactgcct ctgttgtgtg cctgctgaat tacttctatc ccagagaggc caaagtacag 1620
   tggaaggtgg ataacgccct ccaatcgggt aactcccagg agagtgccac agagcaggac 1680
   agcaaggaca gcacctacag cctcagcagc gagctgacgc tgagcaaagc agactacgag 1740
   aaacacaaag tctacgcctg cgaagtcacc catcagggcc tgagctcgcc cgtcacaaag 1800
   agcttcaaca ggggagagtg a                                           1821
<210> 805
   <211> 1773
   <212> DNA
   <213> Artificial Sequence
<220>
   <223> TSC094 comprises: OKT3-M (Micromet variant anti-CD3) scFv, human IgG1 SCC-P hinge, human IgG1 CH2(ADCC/CDC null), human IgG1 CH3, human Ck(YAE)
<400> 805
   atggaagcac cagcgcagct tctcttcctc ctgctactct ggctcccaga taccaccggt 60
   gatatcaaac tgcagcagtc aggggctgaa ctggcaagac ctggggcctc agtgaagatg 120
   tcctgcaaga cttctggcta cacctttact aggtacacga tgcactgggt aaaacagagg 180
   cctggacagg gtctggaatg gattggatac attaatccta gccgtggtta tactaattac 240
   aatcagaagt tcaaggacaa ggccacattg actacagaca aatcctccag cacagcctac 300
   atgcaactga gcagcctgac atctgaggac tctgcagtct attactgtgc aagatattat 360
   gatgatcatt actgccttga ctactggggc caaggcacca ctctcacagt ctcctcagtc 420
   gaaggtggaa gtggaggttc tggtggaagt ggaggttcag gtggagtcga cgacattcag 480
   ctgacccagt ctccagcaat catgtctgca tctccagggg agaaggtcac catgacctgc 540
   agagccagtt caagtgtaag ttacatgaac tggtaccagc agaagtcagg cacctccccc 600
   aaaagatgga tttatgacac atccaaagtg gcttctggag tcccttatcg cttcagtggc 660
   agtgggtctg ggacctcata ctctctcaca atcagcagca tggaggctga agatgctgcc 720
   acttattact gccaacagtg gagtagctcg agtgagccca aatcttctga caaaactcac 780
   acatgcccac cgtgcccagc acctgaagcc gcgggtgcac cgtcagtctt cctcttcccc 840
   ccaaaaccca aggacaccct catgatctcc cggacccctg aggtcacatg cgtggtggtg 900
   gacgtgagcc acgaagaccc tgaggtcaag ttcaactggt acgtggacgg cgtggaggtg 960
   cataatgcca agacaaagcc gcgggaggag cagtacaaca gcacgtaccg tgtggtcagc 1020
   gtcctcaccg tcctgcacca ggactggctg aatggcaagg cgtacgcgtg cgcggtctcc 1080
   aacaaagccc tcccagcccc catcgagaaa accatctcca aagccaaagg gcagccccga 1140
   gaaccacagg tgtacaccct gcccccatcc cgggatgagc tgaccaagaa ccaggtcagc 1200
   ctgacctgcc tggtcaaagg cttctatcca agcgacatcg ccgtggagtg ggagagcaat 1260
   gggcagccgg agaacaacta caagaccacg cctcccgtgc tggactccga cggctccttc 1320
   ttcctctaca gcaagctcac cgtggacaag agcaggtggc agcaggggaa cgtcttctca 1380
   tgctccgtga tgcatgaggc tctgcacaac cactacacgc agaagagcct ctccctgtct 1440
   ccgggtaaat ctagaactgt ggctgcacca tctgtcttca tcttcccgcc atctgatgag 1500
   cagttgaaat ctggaactgc ctctgttgtg tgcctgctga attacttcta tcccagagag 1560
   gccaaagtac agtggaaggt ggataacgcc ctccaatcgg gtaactccca ggagagtgcc 1620
   acagagcagg acagcaagga cagcacctac agcctcagca gcgagctgac gctgagcaaa 1680
   gcagactacg agaaacacaa agtctacgcc tgcgaagtca cccatcaggg cctgagctcg 1740
   cccgtcacaa agagcttcaa caggggagag tga                              1773
<210> 806
   <211> 1821
   <212> DNA
   <213> Artificial Sequence
<220>
   <223> TSC095 comprises: BMA031 (anti-TCR) scFv, human IgG1 SCC-P hinge, human IgG1 CH2(ADCC/CDC null), human IgG1 CH3, human Ck(YAE)
<400> 806
   atggaagcac cagcgcagct tctcttcctc ctgctactct ggctcccaga taccaccggt 60
   ggacaggtcc aactagttca gtctggagca gaggttaaga agcctgggag ctcagtgaag 120
   gtttcctgca aggcttccgg atataaattc actagctatg ttatgcactg ggtgaagcag 180
   gcacctggcc aggggctcga gtggattgga tatattaatc cttacaatga tgttactaag 240
   tacaatgaga agttcaaagg caaggccaca cttacggccg acgagtccac taatacagcc 300
   tacatggagc tgagcagcct gagatctgag gacactgcgg tccattactg tgcaagaggg 360
   agctactatg attacgacgg gtttgtttac tggggccaag gtaccctggt caccgtctca 420
   agcggtggcg gagggtctgg gggtggcgga tccggaggtg gtggctctgc acaagatatc 480
   cagatgaccc aatctccatc gaccctgagt gcatctgtcg gggacagagt caccatgacc 540
   tgcagtgcca cctcaagtgt aagttacatg cactggtatc agcagaagcc cgggaaggct 600
   cccaaaagat ggatctatga cacatccaaa ctggcttctg gagtccctgc gcgcttcatt 660
   ggcagtgggt ctgggaccga gttcacgctc acaatcagca gcctgcagcc agatgatttc 720
   gctacgtatt actgccagca gtggagtagt aacccgctca cgttcggtgg aggtaccaag 780
   gtcgagatta aatcctcgag tgagcccaaa tcttctgaca aaactcacac atgcccaccg 840
   tgcccagcac ctgaagccgc gggtgcaccg tcagtcttcc tcttcccccc aaaacccaag 900
   gacaccctca tgatctcccg gacccctgag gtcacatgcg tggtggtgga cgtgagccac 960
   gaagaccctg aggtcaagtt caactggtac gtggacggcg tggaggtgca taatgccaag 1020
   acaaagccgc gggaggagca gtacaacagc acgtaccgtg tggtcagcgt cctcaccgtc 1080
   ctgcaccagg actggctgaa tggcaaggcg tacgcgtgcg cggtctccaa caaagccctc 1140
   ccagccccca tcgagaaaac catctccaaa gccaaagggc agccccgaga accacaggtg 1200
   tacaccctgc ccccatcccg ggatgagctg accaagaacc aggtcagcct gacctgcctg 1260
   gtcaaaggct tctatccaag cgacatcgcc gtggagtggg agagcaatgg gcagccggag 1320
   aacaactaca agaccacgcc tcccgtgctg gactccgacg gctccttctt cctctacagc 1380
   aagctcaccg tggacaagag caggtggcag caggggaacg tcttctcatg ctccgtgatg 1440
   catgaggctc tgcacaacca ctacacgcag aagagcctct ccctgtctcc gggtaaatct 1500
   agaactgtgg ctgcaccatc tgtcttcatc ttcccgccat ctgatgagca gttgaaatct 1560
   ggaactgcct ctgttgtgtg cctgctgaat tacttctatc ccagagaggc caaagtacag 1620
   tggaaggtgg ataacgccct ccaatcgggt aactcccagg agagtgccac agagcaggac 1680
   agcaaggaca gcacctacag cctcagcagc gagctgacgc tgagcaaagc agactacgag 1740
   aaacacaaag tctacgcctg cgaagtcacc catcagggcc tgagctcgcc cgtcacaaag 1800
   agcttcaaca ggggagagtg a 1821
<210> 807
   <211> 1836
   <212> DNA
   <213> Artificial Sequence
<220>
   <223> TSC096 comprises: HD37 (anti-CD19) scFv, human IgG1 SCC-P hinge, human IgG1 CH2 (ADCC/CDC null), human IgG1 CH3, human Ck(YAE)
<400> 807
   atggaagcac cagcgcagct tctcttcctc ctgctactct ggctcccaga taccaccggt 60
   gacattgtgc tgacccaatc tccagcttct ttggctgtgt ctctagggca gagggccacc 120
   atctcctgca aggccagcca aagtgttgat tatgatggtg atagttattt gaactggtac 180
   caacagattc caggacagcc acccaaactc ctcatctatg atgcatccaa tctagtttct 240
   gggatcccac ccaggtttag tggcagtggg tctgggacag acttcaccct caacatccat 300
   cctgtggaga aggtggatgc tgcaacctat cactgccagc aaagtactga ggatccgtgg 360
   acgttcggtg gaggcaccaa gctggaaatc aaaggtggcg gtggttcggg cggtggtggg 420
   tcgggtggcg gcggagctag ccaggttcag ctgcagcagt ctggggctga gctggtgagg 480
   cctgggtcct cagtgaagat ttcctgcaag gcttctggct atgcattcag tagctactgg 540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagtgagc ccaaatcttc tgacaaaact 840
   cacacatgcc caccgtgccc agcacctgaa gccgcgggtg caccgtcagt cttcctcttc 900
   cccccaaaac ccaaggacac cctcatgatc tcccggaccc ctgaggtcac atgcgtggtg 960
   gtggacgtga gccacgaaga ccctgaggtc aagttcaact ggtacgtgga cggcgtggag 1020
   gtgcataatg ccaagacaaa gccgcgggag gagcagtaca acagcacgta ccgtgtggtc 1080
   agcgtcctca ccgtcctgca ccaggactgg ctgaatggca aggcgtacgc gtgcgcggtc 1140
   tccaacaaag ccctcccagc ccccatcgag aaaaccatct ccaaagccaa agggcagccc 1200
   cgagaaccac aggtgtacac cctgccccca tcccgggatg agctgaccaa gaaccaggtc 1260
   agcctgacct gcctggtcaa aggcttctat ccaagcgaca tcgccgtgga gtgggagagc 1320
   aatgggcagc cggagaacaa ctacaagacc acgcctcccg tgctggactc cgacggctcc 1380
   ttcttcctct acagcaagct caccgtggac aagagcaggt ggcagcaggg gaacgtcttc 1440
   tcatgctccg tgatgcatga ggctctgcac aaccactaca cgcagaagag cctctccctg 1500
   tctccgggta aatctagaac tgtggctgca ccatctgtct tcatcttccc gccatctgat 1560
   gagcagttga aatctggaac tgcctctgtt gtgtgcctgc tgaattactt ctatcccaga 1620
   gaggccaaag tacagtggaa ggtggataac gccctccaat cgggtaactc ccaggagagt 1680
   gccacagagc aggacagcaa ggacagcacc tacagcctca gcagcgagct gacgctgagc 1740
   aaagcagact acgagaaaca caaagtctac gcctgcgaag tcacccatca gggcctgagc 1800
   tcgcccgtca caaagagctt caacagggga gagtga                           1836
<210> 808
   <211> 1812
   <212> DNA
   <213> Artificial Sequence
<220>
   <223> TSC097 comprises: 4C04 (anti-RON) scFv, human IgG1 SCC-P hinge, human IgG1 CH2(ADCC/CDC null), human IgG1 CH3, and human Ck(YAE)
<400> 808
   atggaagcac cagcgcagct tctcttcctc ctgctactct ggctcccaga taccaccggt 60
   gacattcaga tgacccagtc tcctgcctcc cagtctgcat ctctgggaga aagtgtcacc 120
   atcacatgcc tggcaagtca gaccattggt acatggttag catggtatca gcagaaacca 180
   gggaaatctc ctcagctcct gatttatgct gcaaccagct tggcagatgg ggtcccatca 240
   aggttcagtg ctagtggatc tggcacaaaa ttttctttca agatcgacag cctacagtct 300
   gaagattttg taagttatta ctgtcaacaa ctttacaata ctccgtggac gttcggtgga 360
   ggcaccaagc tggaaatcaa aggtggcggt ggctctggcg gaggtggatc cggtggcggc 420
   ggctctcaga tccagctgca gcagtctgga cctgagctgg tgaagcctgg gacttcagtg 480
   aagatatcct gcaaggcttc tgactacaca ctcactgact tctatatgaa ctgggtgagg 540
   cagaagcctg gacagggact tgagtggatt gggaggattt atcctggaac cgataaaact 600
   agatacaatg agaaattcag ggacaaggcc atactgaccg tggacacgtc ctccagcaca 660
   gcctacatgc agctcagcag cctgacatca gaggacactg ctgtctattt ctgtgcaaga 720
   tccgcctact atggtaacta cgttgctatg gactactggg gtcaaggaac ctcagtcacc 780
   gtctcctcga gtgagcccaa atcttctgac aaaactcaca catgcccacc gtgcccagca 840
   cctgaagccg cgggtgcacc gtcagtcttc ctcttccccc caaaacccaa ggacaccctc 900
   atgatctccc ggacccctga ggtcacatgc gtggtggtgg acgtgagcca cgaagaccct 960
   gaggtcaagt tcaactggta cgtggacggc gtggaggtgc ataatgccaa gacaaagccg 1020
   cgggaggagc agtacaacag cacgtaccgt gtggtcagcg tcctcaccgt cctgcaccag 1080
   gactggctga atggcaaggc gtacgcgtgc gcggtctcca acaaagccct cccagccccc 1140
   atcgagaaaa ccatctccaa agccaaaggg cagccccgag aaccacaggt gtacaccctg 1200
   cccccatccc gggatgagct gaccaagaac caggtcagcc tgacctgcct ggtcaaaggc 1260
   ttctatccaa gcgacatcgc cgtggagtgg gagagcaatg ggcagccgga gaacaactac 1320
   aagaccacgc ctcccgtgct ggactccgac ggctccttct tcctctacag caagctcacc 1380
   gtggacaaga gcaggtggca gcaggggaac gtcttctcat gctccgtgat gcatgaggct 1440
   ctgcacaacc actacacgca gaagagcctc tccctgtctc cgggtaaatc tagaactgtg 1500
   gctgcaccat ctgtcttcat cttcccgcca tctgatgagc agttgaaatc tggaactgcc 1560
   tctgttgtgt gcctgctgaa ttacttctat cccagagagg ccaaagtaca gtggaaggtg 1620
   gataacgccc tccaatcggg taactcccag gagagtgcca cagagcagga cagcaaggac 1680
   agcacctaca gcctcagcag cgagctgacg ctgagcaaag cagactacga gaaacacaaa 1740
   gtctacgcct gcgaagtcac ccatcagggc ctgagctcgc ccgtcacaaa gagcttcaac 1800
   aggggagagt ga                                                     1812
<210> 809
   <211> 1608
   <212> DNA
   <213> Artificial Sequence
<220>
   <223> Bispecific T-Cell Engaging molecule (BiTE) known as bsc19x3
<400> 809
   aagcttgccg ccatgggatg gagctgtatc atcctcttct tggtagcaac agctacaggt 60
   gtccactccg actacaaaga tgatgacgat aaggatatcc agctgaccca gtctccagct 120
   tctttggctg tgtctctagg gcagagggcc accatctcct gcaaggccag ccaaagtgtt 180
   gattatgatg gtgatagtta tttgaactgg taccaacaga ttccaggaca gccacccaaa 240
   ctcctcatct atgatgcatc caatctagtt tctgggatcc cacccaggtt tagtggcagt 300
   gggtctggga cagacttcac cctcaacatc catcctgtgg agaaggtgga tgctgcaacc 360
   tatcactgtc agcaaagtac tgaggatccg tggacgttcg gtggagggac caagctcgag 420
   atcaaaggtg gtggtggttc tggcggcggc ggctccggtg gtggtggttc tcaggtgcag 480
   ctgcagcagt ctggggctga gctggtgagg cctgggtcct cagtgaagat ttcctgcaag 540
   gcttctggct atgcattcag tagctactgg atgaactggg tgaagcagag gcctggacag 600
   ggtcttgagt ggattggaca gatttggcct ggagatggtg atactaacta caatggaaag 660
   ttcaagggta aagccactct gactgcagac gaatcctcca gcacagccta catgcaactc 720
   agcagcctag catctgagga ctctgcggtc tatttctgtg caagacggga gactacgacg 780
   gtaggccgtt attactatgc tatggactac tggggccaag ggaccacggt caccgtctcc 840
   tccggaggtg gtggatccga tatcaaactg cagcagtcag gggctgaact ggcaagacct 900
   ggggcctcag tgaagatgtc ctgcaagact tctggctaca cctttactag gtacacgatg 960
   cactgggtaa aacagaggcc tggacagggt ctggaatgga ttggatacat taatcctagc 1020
   cgtggttata ctaattacaa tcagaagttc aaggacaagg ccacattgac tacagacaaa 1080
   tcctccagca cagcctacat gcaactgagc agcctgacat ctgaggactc tgcagtctat 1140
   tactgtgcaa gatattatga tgatcattac tgccttgact actggggcca aggcaccact 1200
   ctcacagtct cctcagtcga aggtggaagt ggaggttctg gtggaagtgg aggttcaggt 1260
   ggagtcgacg acattcagct gacccagtct ccagcaatca tgtctgcatc tccaggggag 1320
   aaggtcacca tgacctgcag agccagttca agtgtaagtt acatgaactg gtaccagcag 1380
   aagtcaggca cctcccccaa aagatggatt tatgacacat ccaaagtggc ttctggagtc 1440
   ccttatcgct tcagtggcag tgggtctggg acctcatact ctctcacaat cagcagcatg 1500
   gaggctgaag atgctgccac ttattactgc caacagtgga gtagtaaccc gctcacgttc 1560
   ggtgctggga ccaagctgga gctgaaacat catcaccatc atcattag             1608
<210> 810
   <211> 859
   <212> PRT
   <213> Artificial Sequence
<220>
   <223> TSC038 comprises: G19-4 (anti-CD3) scFv, human IgG1 SCC-P hinge, human IgG1 CH2, human IgG1 CH3, human Ck(YAE), H75 linker and 3D1 (anti-CD86) scFv
<400> 810
   Glu Val Gln Leu Gln Gln Ser Gly Pro Glu Leu Val Lys Pro Gly Ala
   1               5                   10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Glu Pro Lys Ser Ser Asp Lys
                   245                 250                 255
   Thr His Thr Cys Pro Pro Cys Pro Ser Gly Ala Pro Glu Leu Leu Gly
               260                 265                 270
   Gly Pro Ser Val Phe Leu Phe Pro Pro Lys Pro Lys Asp Thr Leu Met
           275                 280                 285
   Ile Ser Arg Thr Pro Glu Val Thr Cys Val Val Val Asp Val Ser His
       290                 295                 300
   Glu Asp Pro Glu Val Lys Phe Asn Trp Tyr Val Asp Gly Val Glu Val
   305                 310                 315                 320
   His Asn Ala Lys Thr Lys Pro Arg Glu Glu Gln Tyr Asn Ser Thr Tyr
                   325                 330                 335
   Arg Val Val Ser Val Leu Thr Val Leu His Gln Asp Trp Leu Asn Gly
               340                 345                 350
   Lys Glu Tyr Lys Cys Lys Val Ser Asn Lys Ala Leu Pro Ala Pro Ile
           355                 360                 365
   Glu Lys Thr Ile Ser Lys Ala Lys Gly Gln Pro Arg Glu Pro Gln Val
       370                 375                 380
   Tyr Thr Leu Pro Pro Ser Arg Asp Glu Leu Thr Lys Asn Gln Val Ser
   385                 390                 395                 400
   Leu Thr Cys Leu Val Lys Gly Phe Tyr Pro Ser Asp Ile Ala Val Glu
                   405                 410                 415
   Trp Glu Ser Asn Gly Gln Pro Glu Asn Asn Tyr Lys Thr Thr Pro Pro
   420 425 430
   Val Leu Asp Ser Asp Gly Ser Phe Phe Leu Tyr Ser Lys Leu Thr Val
   435 440 445
   Asp Lys Ser Arg Trp Gln Gln Gly Asn Val Phe Ser Cys Ser Val Met
   450 455 460
   His Glu Ala Leu His Asn His Tyr Thr Gln Lys Ser Leu Ser Leu Ser
   465 470 475 480
   Pro Gly Lys Ser Arg Thr Val Ala Ala Pro Ser Val Phe Ile Phe Pro
   485 490 495
   Pro Ser Asp Glu Gln Leu Lys Ser Gly Thr Ala Ser Val Val Cys Leu
   500 505 510
   Leu Asn Tyr Phe Tyr Pro Arg Glu Ala Lys Val Gln Trp Lys Val Asp
   515 520 525
   Asn Ala Leu Gln Ser Gly Asn Ser Gln Glu Ser Ala Thr Glu Gln Asp
   530 535 540
   Ser Lys Asp Ser Thr Tyr Ser Leu Ser Ser Glu Leu Thr Leu Ser Lys
   545 550 555 560
   Ala Asp Tyr Glu Lys His Lys Val Tyr Ala Cys Glu Val Thr His Gln
   565 570 575
   Gly Leu Ser Ser Pro Val Thr Lys Ser Phe Asn Arg Gly Glu Gln Arg
   580 585 590
   His Asn Asn Ser Ser Leu Asn Thr Gly Thr Gln Met Ala Gly His Ser
   595 600 605
   Pro Asn Ser Gln Val Gln Leu Gln Gln Ser Gly Pro Glu Leu Val Arg
   610 615 620
   Pro Gly Glu Ser Val Lys Ile Ser Cys Lys Gly Ser Gly Tyr Thr Phe
   625 630 635 640
   Thr Asp Tyr Ala Ile Gln Trp Val Lys Gln Ser His Ala Lys Ser Leu
   645 650 655
   Glu Trp Ile Gly Val Ile Asn Ile Tyr Tyr Asp Asn Thr Asn Tyr Asn
   660 665 670
   Gln Lys Phe Lys Gly Lys Ala Thr Met Thr Val Asp Lys Ser Ser Ser
   675 680 685
   Thr Ala Tyr Met Glu Leu Ala Arg Leu Thr Ser Glu Asp Ser Ala Ile
   690 695 700
   Tyr Tyr Cys Ala Arg Ala Ala Trp Tyr Met Asp Tyr Trp Gly Gln Gly
   705 710 715 720
   Thr Ser Val Thr Val Ser Ser Gly Gly Gly Gly Ser Gly Gly Gly Gly
   725 730 735
   Ser Gly Gly Gly Gly Ser Gly Gly Gly Gly Ser Asp Ile Val Leu Ser
   740 745 750
   Gln Ser Pro Ser Ser Leu Ala Val Ser Ala Gly Glu Lys Val Thr Met
   755 760 765
   Ser Cys Lys Ser Ser Gln Ser Leu Leu Asn Ser Arg Thr Arg Glu Asn
   770 775 780
   Tyr Leu Ala Trp Tyr Gln Gln Lys Pro Gly Gln Ser Pro Lys Leu Leu
   785 790 795 800
   Ile Tyr Trp Ala Ser Thr Arg Glu Ser Gly Val Pro Asp Arg Phe Thr
   805 810 815
   Gly Ser Gly Ser Gly Thr Asp Phe Thr Leu Thr Ile Ser Ser Val Gln
   820 825 830
   Ala Glu Asp Leu Ala Val Tyr Tyr Cys Thr Gln Ser Tyr Asn Leu Tyr
   835 840 845
   Thr Phe Gly Gly Gly Thr Lys Leu Glu Ile Lys
   850 855
<210> 811
   <211> 585
   <212> PRT
   <213> Artificial Sequence
<220>
   <223> TSC039 comprises from its amino to carboxyl terminus: BMA031 (anti-TCR) scFv, human IgG1 SCC-P hinge, human IgG1 CH2, human IgG1 CH3, and human Ck(YAE)
<400> 811
   Gln Val Gln Leu Val Gln Ser Gly Ala Glu Val Lys Lys Pro Gly Ser
    1               5                  10                  15
   Ser Val Lys Val Ser Cys Lys Ala Ser Gly Tyr Lys Phe Thr Ser Tyr
               20                  25                  30
   Val Met His Trp Val Lys Gln Ala Pro Gly Gln Gly Leu Glu Trp Ile
           35                  40                  45
   Gly Tyr Ile Asn Pro Tyr Asn Asp Val Thr Lys Tyr Asn Glu Lys Phe
       50                  55                  60
   Lys Gly Lys Ala Thr Leu Thr Ala Asp Glu Ser Thr Asn Thr Ala Tyr
   65                  70                  75                  80
   Met Glu Leu Ser Ser Leu Arg Ser Glu Asp Thr Ala Val His Tyr Cys
                   85                  90                  95
   Ala Arg Gly Ser Tyr Tyr Asp Tyr Asp Gly Phe Val Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Ala Gln Asp Ile Gln Met Thr Gln Ser
       130                 135                 140
   Pro Ser Thr Leu Ser Ala Ser Val Gly Asp Arg Val Thr Met Thr Cys
   145                 150                 155                 160
   Ser Ala Thr Ser Ser Val Ser Tyr Met His Trp Tyr Gln Gln Lys Pro
                   165                 170                 175
   Gly Lys Ala Pro Lys Arg Trp Ile Tyr Asp Thr Ser Lys Leu Ala Ser
               180                 185                 190
   Gly Val Pro Ala Arg Phe Ile Gly Ser Gly Ser Gly Thr Glu Phe Thr
           195                 200                 205
   Leu Thr Ile Ser Ser Leu Gln Pro Asp Asp Phe Ala Thr Tyr Tyr Cys
       210                 215                 220
   Gln Gln Trp Ser Ser Asn Pro Leu Thr Phe Gly Gly Gly Thr Lys Val
   225                 230                 235                 240
   Glu Ile Lys Ser Ser Ser Glu Pro Lys Ser Ser Asp Lys Thr His Thr
                   245                 250                 255
   Cys Pro Pro Cys Pro Ala Pro Glu Leu Leu Gly Gly Pro Ser Val Phe
               260                 265                 270
   Leu Phe Pro Pro Lys Pro Lys Asp Thr Leu Met Ile Ser Arg Thr Pro
           275                 280                 285
   Glu Val Thr Cys Val Val Val Asp Val Ser His Glu Asp Pro Glu Val
       290                 295                 300
   Lys Phe Asn Trp Tyr Val Asp Gly Val Glu Val His Asn Ala Lys Thr
   305                 310                 315                 320
   Lys Pro Arg Glu Glu Gln Tyr Asn Ser Thr Tyr Arg Val Val Ser Val
                   325                 330                 335
   Leu Thr Val Leu His Gln Asp Trp Leu Asn Gly Lys Glu Tyr Lys Cys
               340                 345                 350
   Lys Val Ser Asn Lys Ala Leu Pro Ala Pro Ile Glu Lys Thr Ile Ser
           355                 360                 365
   Lys Ala Lys Gly Gln Pro Arg Glu Pro Gln Val Tyr Thr Leu Pro Pro
       370                 375                 380
   Ser Arg Asp Glu Leu Thr Lys Asn Gln Val Ser Leu Thr Cys Leu Val
   385                 390                 395                 400
   Lys Gly Phe Tyr Pro Ser Asp Ile Ala Val Glu Trp Glu Ser Asn Gly
                   405                 410                 415
   Gln Pro Glu Asn Asn Tyr Lys Thr Thr Pro Pro Val Leu Asp Ser Asp
               420                 425                 430
   Gly Ser Phe Phe Leu Tyr Ser Lys Leu Thr Val Asp Lys Ser Arg Trp
           435                 440                 445
   Gln Gln Gly Asn Val Phe Ser Cys Ser Val Met His Glu Ala Leu His
       450                 455                 460
   Asn His Tyr Thr Gln Lys Ser Leu Ser Leu Ser Pro Gly Lys Ser Arg
   465                 470                 475                 480
   Thr Val Ala Ala Pro Ser Val Phe Ile Phe Pro Pro Ser Asp Glu Gln
                   485                 490                 495
   Leu Lys Ser Gly Thr Ala Ser Val Val Cys Leu Leu Asn Tyr Phe Tyr
               500                 505                 510
   Pro Arg Glu Ala Lys Val Gln Trp Lys Val Asp Asn Ala Leu Gln Ser
           515                 520                 525
   Gly Asn Ser Gln Glu Ser Ala Thr Glu Gln Asp Ser Lys Asp Ser Thr
       530                 535                 540
   Tyr Ser Leu Ser Ser Glu Leu Thr Leu Ser Lys Ala Asp Tyr Glu Lys
   545                 550                 555                 560
   His Lys Val Tyr Ala Cys Glu Val Thr His Gln Gly Leu Ser Ser Pro
                   565                 570                 575
   Val Thr Lys Ser Phe Asn Arg Gly Glu
               580                 585
<210> 812
   <211> 586
   <212> PRT
   <213> Artificial Sequence
<220>
   <223> polypeptide TSC041 comprises from its amino to carboxy terminus: CRIS7 (anti-CD3) scFv, human IgG1 SCC-P hinge, human IgG1 CH2, human IgG1 CH3, and human Ck(YAE)
<400> 812
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Glu Pro Lys Ser Ser Asp Lys Thr His
                   245                 250                 255
   Thr Cys Pro Pro Cys Pro Ala Pro Glu Leu Leu Gly Gly Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Glu Tyr Lys
               340                 345                 350
   Cys Lys Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Thr Val Ala Ala Pro Ser Val Phe Ile Phe Pro Pro Ser Asp Glu
                   485                 490                 495
   Gln Leu Lys Ser Gly Thr Ala Ser Val Val Cys Leu Leu Asn Tyr Phe
               500                 505                 510
   Tyr Pro Arg Glu Ala Lys Val Gln Trp Lys Val Asp Asn Ala Leu Gln
           515                 520                 525
   Ser Gly Asn Ser Gln Glu Ser Ala Thr Glu Gln Asp Ser Lys Asp Ser
       530                 535                 540
   Thr Tyr Ser Leu Ser Ser Glu Leu Thr Leu Ser Lys Ala Asp Tyr Glu
   545                 550                 555                 560
   Lys His Lys Val Tyr Ala Cys Glu Val Thr His Gln Gly Leu Ser Ser
                   565                 570                 575
   Pro Val Thr Lys Ser Phe Asn Arg Gly Glu
               580                 585
<210> 813
   <211> 570
   <212> PRT
   <213> Artificial Sequence
<220>
   <223> TSC043 comprises from its amino to carboxy terminus: OKT3-M (Micromet variant anti-CD3, see also US 7,635,472) scFv, human IgG1 SCC-P hinge, human IgG1 CH2, human IgG1 CH3, and human Ck(YAE)
<400> 813
   Asp Ile Lys Leu Gln Gln Ser Gly Ala Glu Leu Ala Arg Pro Gly Ala
    1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser Val Glu Gly Gly Ser Gly Gly Ser Gly
           115                 120                 125
   Gly Ser Gly Gly Ser Gly Gly Val Asp Asp Ile Gln Leu Thr Gln Ser
       130                 135                 140
   Pro Ala Ile Met Ser Ala Ser Pro Gly Glu Lys Val Thr Met Thr Cys
   145                 150                 155                 160
   Arg Ala Ser Ser Ser Val Ser Tyr Met Asn Trp Tyr Gln Gln Lys Ser
                   165                 170                 175
   Gly Thr Ser Pro Lys Arg Trp Ile Tyr Asp Thr Ser Lys Val Ala Ser
               180                 185                 190
   Gly Val Pro Tyr Arg Phe Ser Gly Ser Gly Ser Gly Thr Ser Tyr Ser
           195                 200                 205
   Leu Thr Ile Ser Ser Met Glu Ala Glu Asp Ala Ala Thr Tyr Tyr Cys
       210                 215                 220
   Gln Gln Trp Ser Ser Ser Ser Glu Pro Lys Ser Ser Asp Lys Thr His
   225                 230                 235                 240
   Thr Cys Pro Pro Cys Pro Ala Pro Glu Leu Leu Gly Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Lys Phe Asn Trp Tyr Val Asp Gly Val Glu Val His Asn Ala Lys
       290                 295                 300
   Thr Lys Pro Arg Glu Glu Gln Tyr Asn Ser Thr Tyr Arg Val Val Ser
   305                 310                 315                 320
   Val Leu Thr Val Leu His Gln Asp Trp Leu Asn Gly Lys Glu Tyr Lys
                   325                 330                 335
   Cys Lys Val Ser Asn Lys Ala Leu Pro Ala Pro Ile Glu Lys Thr Ile
               340                 345                 350
   Ser Lys Ala Lys Gly Gln Pro Arg Glu Pro Gln Val Tyr Thr Leu Pro
           355                 360                 365
   Pro Ser Arg Asp Glu Leu Thr Lys Asn Gln Val Ser Leu Thr Cys Leu
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Val Leu Asp Ser
                   405                 410                 415
   Asp Gly Ser Phe Phe Leu Tyr Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys Ser
       450                 455                 460
   Arg Thr Val Ala Ala Pro Ser Val Phe Ile Phe Pro Pro Ser Asp Glu
   465                 470                 475                 480
   Gln Leu Lys Ser Gly Thr Ala Ser Val Val Cys Leu Leu Asn Tyr Phe
                   485                 490                 495
   Tyr Pro Arg Glu Ala Lys Val Gln Trp Lys Val Asp Asn Ala Leu Gln
               500                 505                 510
   Ser Gly Asn Ser Gln Glu Ser Ala Thr Glu Gln Asp Ser Lys Asp Ser
           515                 520                 525
   Thr Tyr Ser Leu Ser Ser Glu Leu Thr Leu Ser Lys Ala Asp Tyr Glu
       530                 535                 540
   Lys His Lys Val Tyr Ala Cys Glu Val Thr His Gln Gly Leu Ser Ser
   545                 550                 555                 560
   Pro Val Thr Lys Ser Phe Asn Arg Gly Glu
                   565                 570
<210> 814
   <211> 584
   <212> PRT
   <213> Artificial Sequence
<220>
   <223> TSC049 comprises from its amino to carboxy terminus: HD37 (anti-CD19) scFv, human IgG1 SCC-P hinge, human IgG1 CH2(ADCC/CDC null), human IgG1 CH3, and human IgG1 CH1
<400> 814
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Cys Pro Pro Cys Pro Ala Pro Glu Ala Ala
               260                 265                 270
   Gly Ala Pro Ser Val Phe Leu Phe Pro Pro Lys Pro Lys Asp Thr Leu
           275                 280                 285
   Met Ile Ser Arg Thr Pro Glu Val Thr Cys Val Val Val Asp Val Ser
       290                 295                 300
   His Glu Asp Pro Glu Val Lys Phe Asn Trp Tyr Val Asp Gly Val Glu
   305                 310                 315                 320
   Val His Asn Ala Lys Thr Lys Pro Arg Glu Glu Gln Tyr Asn Ser Thr
                   325                 330                 335
   Tyr Arg Val Val Ser Val Leu Thr Val Leu His Gln Asp Trp Leu Asn
               340                 345                 350
   Gly Lys Ala Tyr Ala Cys Ala Val Ser Asn Lys Ala Leu Pro Ala Pro
           355                 360                 365
   Ile Glu Lys Thr Ile Ser Lys Ala Lys Gly Gln Pro Arg Glu Pro Gln
       370                 375                 380
   Val Tyr Thr Leu Pro Pro Ser Arg Asp Glu Leu Thr Lys Asn Gln Val
   385                 390                 395                 400
   Ser Leu Thr Cys Leu Val Lys Gly Phe Tyr Pro Ser Asp Ile Ala Val
                   405                 410                 415
   Glu Trp Glu Ser Asn Gly Gln Pro Glu Asn Asn Tyr Lys Thr Thr Pro
               420                 425                 430
   Pro Val Leu Asp Ser Asp Gly Ser Phe Phe Leu Tyr Ser Lys Leu Thr
           435                 440                 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465                 470                 475                 480
   Ser Pro Gly Lys Ser Arg Ala Ser Thr Lys Gly Pro Ser Val Phe Pro
                   485                 490                 495
   Leu Ala Pro Ser Ser Lys Ser Thr Ser Gly Gly Thr Ala Ala Leu Gly
               500                 505                 510
   Cys Leu Val Lys Asp Tyr Phe Pro Glu Pro Val Thr Val Ser Trp Asn
           515                 520                 525
   Ser Gly Ala Leu Thr Ser Gly Val His Thr Phe Pro Ala Val Leu Gln
       530                 535                 540
   Ser Ser Gly Leu Tyr Ser Leu Ser Ser Val Val Thr Val Pro Ser Ser
   545                 550                 555                 560
   Ser Leu Gly Thr Gln Thr Tyr Ile Cys Asn Val Asn His Lys Pro Ser
                   565                 570                 575
   Asn Thr Lys Val Asp Lys Lys Val
               580
<210> 815
   <211> 577
   <212> PRT
   <213> Artificial Sequence
<220>
   <223> TSC050 comprises from its amino to carboxy terminus: 2H7 (anti-CD20) scFv, human IgG1 SCC-P hinge, human IgG1 CH2(ADCC/CDC null), human IgG1 CH3, and human IgG1 CH1
<400> 815
   Gln Ile Val Leu Ser Gln Ser Pro Ala Ile Leu Ser Ala Ser Pro Gly
    1               5                  10                  15
   Glu Lys Val Thr Met Thr Cys Arg Ala Ser Ser Ser Val Ser Tyr Met
               20                  25                  30
   His Trp Tyr Gln Gln Lys Pro Gly Ser Ser Pro Lys Pro Trp Ile Tyr
           35                  40                  45
   Ala Pro Ser Asn Leu Ala Ser Gly Val Pro Ala Arg Phe Ser Gly Ser
       50                  55                  60
   Gly Ser Gly Thr Ser Tyr Ser Leu Thr Ile Ser Arg Val Glu Ala Glu
   65                  70                  75                  80
   Asp Ala Ala Thr Tyr Tyr Cys Gln Gln Trp Ser Phe Asn Pro Pro Thr
                   85                  90                  95
   Phe Gly Ala Gly Thr Lys Leu Glu Leu Lys Asp Gly Gly Gly Ser Gly
               100                 105                 110
   Gly Gly Gly Ser Gly Gly Gly Gly Ala Ser Gln Ala Tyr Leu Gln Gln
           115                 120                 125
   Ser Gly Ala Glu Ser Val Arg Pro Gly Ala Ser Val Lys Met Ser Cys
       130                 135                 140
   Lys Ala Ser Gly Tyr Thr Phe Thr Ser Tyr Asn Met His Trp Val Lys
   145                 150                 155                 160
   Gln Thr Pro Arg Gln Gly Leu Glu Trp Ile Gly Ala Ile Tyr Pro Gly
                   165                 170                 175
   Asn Gly Asp Thr Ser Tyr Asn Gln Lys Phe Lys Gly Lys Ala Thr Leu
               180                 185                 190
   Thr Val Asp Lys Ser Ser Ser Thr Ala Tyr Met Gln Leu Ser Ser Leu
           195                 200                 205
   Thr Ser Glu Asp Ser Ala Val Tyr Phe Cys Ala Arg Val Val Tyr Tyr
       210                 215                 220
   Ser Asn Ser Tyr Trp Tyr Phe Asp Val Trp Gly Thr Gly Thr Thr Val
   225                 230                 235                 240
   Thr Val Ser Ser Ser Glu Pro Lys Ser Ser Asp Lys Thr His Thr Cys
                   245                 250                 255
   Pro Pro Cys Pro Ala Pro Glu Ala Ala Gly Ala Pro Ser Val Phe Leu
               260                 265                 270
   Phe Pro Pro Lys Pro Lys Asp Thr Leu Met Ile Ser Arg Thr Pro Glu
           275                 280                 285
   Val Thr Cys Val Val Val Asp Val Ser His Glu Asp Pro Glu Val Lys
       290                 295                 300
   Phe Asn Trp Tyr Val Asp Gly Val Glu Val His Asn Ala Lys Thr Lys
   305                 310                 315                 320
   Pro Arg Glu Glu Gln Tyr Asn Ser Thr Tyr Arg Val Val Ser Val Leu
                   325                 330                 335
   Thr Val Leu His Gln Asp Trp Leu Asn Gly Lys Ala Tyr Ala Cys Ala
               340                 345                 350
   Val Ser Asn Lys Ala Leu Pro Ala Pro Ile Glu Lys Thr Ile Ser Lys
           355                 360                 365
   Ala Lys Gly Gln Pro Arg Glu Pro Gln Val Tyr Thr Leu Pro Pro Ser
       370                 375                 380
   Arg Asp Glu Leu Thr Lys Asn Gln Val Ser Leu Thr Cys Leu Val Lys
   385                 390                 395                 400
   Gly Phe Tyr Pro Ser Asp Ile Ala Val Glu Trp Glu Ser Asn Gly Gln
                   405                 410                 415
   Pro Glu Asn Asn Tyr Lys Thr Thr Pro Pro Val Leu Asp Ser Asp Gly
               420                 425                 430
   Ser Phe Phe Leu Tyr Ser Lys Leu Thr Val Asp Lys Ser Arg Trp Gln
           435                 440                 445
   Gln Gly Asn Val Phe Ser Cys Ser Val Met His Glu Ala Leu His Asn
       450                 455                 460
   His Tyr Thr Gln Lys Ser Leu Ser Leu Ser Pro Gly Lys Ser Arg Ala
   465                 470                 475                 480
   Ser Thr Lys Gly Pro Ser Val Phe Pro Leu Ala Pro Ser Ser Lys Ser
                   485                 490                 495
   Thr Ser Gly Gly Thr Ala Ala Leu Gly Cys Leu Val Lys Asp Tyr Phe
               500                 505                 510
   Pro Glu Pro Val Thr Val Ser Trp Asn Ser Gly Ala Leu Thr Ser Gly
           515                 520                 525
   Val His Thr Phe Pro Ala Val Leu Gln Ser Ser Gly Leu Tyr Ser Leu
       530                 535                 540
   Ser Ser Val Val Thr Val Pro Ser Ser Ser Leu Gly Thr Gln Thr Tyr
   545                 550                 555                 560
   Ile Cys Asn Val Asn His Lys Pro Ser Asn Thr Lys Val Asp Lys Lys
                   565                 570                 575
   Val
<210> 816
   <211> 573
   <212> PRT
   <213> Artificial Sequence
<220>
   <223> TSC051 comprises from its amino to carboxy terminus: P2C2 (anti-CD79) scFv, human IgG1 SCC-P hinge, human IgG1 CH2(ADCC/CDC null), human IgG1 CH3, and human IgG1 CH1
<400> 816
   Asp Ile Val Met Ile Gln Asp Glu Leu Ser Asn Pro Val Ser Ser Gly
   1               5                   10                  15
   Glu Ser Val Ser Ile Ser Cys Arg Ser Ser Lys Ser Leu Leu Asp Lys
               20                  25                  30
   Glu Gly Lys Thr Tyr Leu Asn Trp Phe Leu Gln Arg Pro Gly Gln Ser
           35                  40                  45
   Pro Gln Leu Leu Ile Tyr Leu Met Ser Met Arg Glu Ser Gly Val Ser
       50                  55                  60
   Asp Arg Phe Ser Gly Ser Gly Ser Gly Thr Asp Phe Thr Leu Glu Ile
   65                  70                  75                  80
   Ser Arg Val Lys Ala Glu Asp Val Gly Val Tyr Tyr Cys Gln Gln Leu
                   85                  90                  95
   Val Glu Tyr Pro Trp Thr Phe Gly Gly Gly Thr Lys Leu Glu Ile Lys
                   100                 105                 110
   Gly Gly Gly Gly Ser Gly Gly Gly Gly Ser Gly Gly Gly Gly Ser Gln
               115                 120                 125
   Ile Gln Leu Val Gln Ser Gly Pro Glu Leu Lys Lys Pro Gly Glu Thr
       130                 135                 140
   Val Lys Ile Ser Cys Lys Ala Ser Gly Tyr Thr Phe Thr Asp Phe Ser
   145                 150                 155                 160
   Met His Trp Val Arg Gln Ala Pro Gly Lys Gly Leu Lys Trp Met Gly
                   165                 170                 175
   Trp Ile Asn Thr Glu Thr Gly Glu Pro Thr Tyr Ala Asp Asp Phe Arg
                   180                 185                 190
   Gly Arg Phe Ala Phe Ser Leu Glu Thr Ser Ala Ser Thr Ala Tyr Leu
               195                 200                 205
   Gln Ile Asn Asn Leu Lys Asn Glu Asp Thr Ala Thr Tyr Phe Cys Thr
       210                 215                 220
   Trp Ser Ala Tyr Trp Gly Gln Gly Thr Leu Val Thr Val Ser Ala Ser
   225                 230                 235                 240
   Ser Glu Pro Lys Ser Ser Asp Lys Thr His Thr Cys Pro Pro Cys Pro
                   245                 250                 255
   Ala Pro Glu Ala Ala Gly Ala Pro Ser Val Phe Leu Phe Pro Pro Lys
                   260                 265                 270
   Pro Lys Asp Thr Leu Met Ile Ser Arg Thr Pro Glu Val Thr Cys Val
               275                 280                 285
   Val Val Asp Val Ser His Glu Asp Pro Glu Val Lys Phe Asn Trp Tyr
       290                 295                 300
   Val Asp Gly Val Glu Val His Asn Ala Lys Thr Lys Pro Arg Glu Glu
   305                 310                 315                 320
   Gln Tyr Asn Ser Thr Tyr Arg Val Val Ser Val Leu Thr Val Leu His
                   325                 330                 335
   Gln Asp Trp Leu Asn Gly Lys Ala Tyr Ala Cys Ala Val Ser Asn Lys
                   340                 345                 350
   Ala Leu Pro Ala Pro Ile Glu Lys Thr Ile Ser Lys Ala Lys Gly Gln
               355                 360                 365
   Pro Arg Glu Pro Gln Val Tyr Thr Leu Pro Pro Ser Arg Asp Glu Leu
       370                 375                 380
   Thr Lys Asn Gln Val Ser Leu Thr Cys Leu Val Lys Gly Phe Tyr Pro
   385                 390                 395                 400
   Ser Asp Ile Ala Val Glu Trp Glu Ser Asn Gly Gln Pro Glu Asn Asn
                   405                 410                 415
   Tyr Lys Thr Thr Pro Pro Val Leu Asp Ser Asp Gly Ser Phe Phe Leu
                   420                 425                 430
   Tyr Ser Lys Leu Thr Val Asp Lys Ser Arg Trp Gln Gln Gly Asn Val
               435                 440                 445
   Phe Ser Cys Ser Val Met His Glu Ala Leu His Asn His Tyr Thr Gln
       450                 455                 460
   Lys Ser Leu Ser Leu Ser Pro Gly Lys Ser Arg Ala Ser Thr Lys Gly
   465                 470                 475                 480
   Pro Ser Val Phe Pro Leu Ala Pro Ser Ser Lys Ser Thr Ser Gly Gly
                   485                 490                 495
   Thr Ala Ala Leu Gly Cys Leu Val Lys Asp Tyr Phe Pro Glu Pro Val
                   500                 505                 510
   Thr Val Ser Trp Asn Ser Gly Ala Leu Thr Ser Gly Val His Thr Phe
               515                 520                 525
   Pro Ala Val Leu Gln Ser Ser Gly Leu Tyr Ser Leu Ser Ser Val Val
       530                 535                 540
   Thr Val Pro Ser Ser Ser Leu Gly Thr Gln Thr Tyr Ile Cys Asn Val
   545                 550                 555                 560
   Asn His Lys Pro Ser Asn Thr Lys Val Asp Lys Lys Val
                   565                 570
<210> 817
   <211> 580
   <212> PRT
   <213> Artificial Sequence
<220>
   <223> TSC052 comprises from its amino to carboxy terminus: 5D5 (anti-cMet) scFv, human IgG1 SCC-P hinge, human IgG1 CH2(ADCC/CDC null), human IgG1 CH3, and human IgG1 CH1
<400> 817
   Asp Phe Met Met Ser Gln Ser Pro Ser Ser Leu Thr Val Ser Val Gly
    1               5                  10                  15
   Glu Lys Val Thr Val Ser Cys Lys Ser Ser Gln Ser Leu Leu Tyr Thr
               20                  25                  30
   Ser Ser Gln Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Thr Ser Val Lys Ala Asp Asp Leu Ala Val Tyr Tyr Cys Gln Gln
                   85                  90                  95
   Tyr Tyr Ala Tyr Pro Trp Thr Phe Gly Gly Gly Thr Lys Leu Glu Leu
               100                 105                 110
   Lys Gly Gly Gly Gly Ser Gly Gly Gly Gly Ser Gly Gly Gly Gly Ser
           115                 120                 125
   Gln Val Gln Leu Gln Gln Ser Gly Pro Glu Leu Val Arg Pro Gly Ala
       130                 135                 140
   Ser Val Lys Met Ser Cys Arg Ala Ser Gly Tyr Thr Phe Thr Ser Tyr
   145                 150                 155                 160
   Trp Leu His Trp Val Lys Gln Arg Pro Gly Gln Gly Leu Glu Trp Ile
                   165                 170                 175
   Gly Met Ile Asp Pro Ser Asn Ser Asp Thr Arg Phe Asn Pro Asn Phe
               180                 185                 190
   Lys Asp Lys Ala Thr Leu Asn Val Asp Arg Ser Ser Asn Thr Ala Tyr
           195                 200                 205
   Met Leu Leu Ser Ser Leu Thr Ser Ala Asp Ser Ala Val Tyr Tyr Cys
       210                 215                 220
   Ala Thr Tyr Gly Ser Tyr Val Ser Pro Leu Asp Tyr Trp Gly Gln Gly
   225                 230                 235                 240
   Thr Ser Val Thr Val Ser Ser Ser Glu Pro Lys Ser Ser Asp Lys Thr
                   245                 250                 255
   His Thr Cys Pro Pro Cys Pro Ala Pro Glu Ala Ala Gly Ala Pro Ser
               260                 265                 270
   Val Phe Leu Phe Pro Pro Lys Pro Lys Asp Thr Leu Met Ile Ser Arg
           275                 280                 285
   Thr Pro Glu Val Thr Cys Val Val Val Asp Val Ser His Glu Asp Pro
       290                 295                 300
   Glu Val Lys Phe Asn Trp Tyr Val Asp Gly Val Glu Val His Asn Ala
   305                 310                 315                 320
   Lys Thr Lys Pro Arg Glu Glu Gln Tyr Asn Ser Thr Tyr Arg Val Val
                   325                 330                 335
   Ser Val Leu Thr Val Leu His Gln Asp Trp Leu Asn Gly Lys Ala Tyr
               340                 345                 350
   Ala Cys Ala Val Ser Asn Lys Ala Leu Pro Ala Pro Ile Glu Lys Thr
           355                 360                 365
   Ile Ser Lys Ala Lys Gly Gln Pro Arg Glu Pro Gln Val Tyr Thr Leu
       370                 375                 380
   Pro Pro Ser Arg Asp Glu Leu Thr Lys Asn Gln Val Ser Leu Thr Cys
   385                 390                 395                 400
   Leu Val Lys Gly Phe Tyr Pro Ser Asp Ile Ala Val Glu Trp Glu Ser
                   405                 410                 415
   Asn Gly Gln Pro Glu Asn Asn Tyr Lys Thr Thr Pro Pro Val Leu Asp
               420                 425                 430
   Ser Asp Gly Ser Phe Phe Leu Tyr Ser Lys Leu Thr Val Asp Lys Ser
           435                 440                 445
   Arg Trp Gln Gln Gly Asn Val Phe Ser Cys Ser Val Met His Glu Ala
       450                 455                 460
   Leu His Asn His Tyr Thr Gln Lys Ser Leu Ser Leu Ser Pro Gly Lys
   465                 470                 475                 480
   Ser Arg Ala Ser Thr Lys Gly Pro Ser Val Phe Pro Leu Ala Pro Ser
                   485                 490                 495
   Ser Lys Ser Thr Ser Gly Gly Thr Ala Ala Leu Gly Cys Leu Val Lys
               500                 505                 510
   Asp Tyr Phe Pro Glu Pro Val Thr Val Ser Trp Asn Ser Gly Ala Leu
           515                 520                 525
   Thr Ser Gly Val His Thr Phe Pro Ala Val Leu Gln Ser Ser Gly Leu
       530                 535                 540
   Tyr Ser Leu Ser Ser Val Val Thr Val Pro Ser Ser Ser Leu Gly Thr
   545                 550                 555                 560
   Gln Thr Tyr Ile Cys Asn Val Asn His Lys Pro Ser Asn Thr Lys Val
                   565                 570                 575
   Asp Lys Lys Val
               580
<210> 818
   <211> 588
   <212> PRT
   <213> Artificial Sequence
<220>
   <223> TSC053 comprises from its amino to carboxy terminus: G19-4 (anti CD3) scFv, human IgG1 SCC-P hinge, human IgG1 CH2(ADCC/CDC null), human IgG1 CH3, and human Ck(YAE)
<400> 818
   Glu Val Gln Leu Gln Gln Ser Gly Pro Glu Leu Val Lys Pro Gly Ala
    1               5                  10                  15
   Ser Met Lys Ile Ser Cys Lys Ala Ser Gly Tyr Ser Phe Thr Gly Tyr
               20                  25                  30
   Ile Val Asn Trp Leu Lys Gln Ser His Gly Lys Asn Leu Glu Trp Ile
           35                  40                  45
   Gly Leu Ile Asn Pro Tyr Lys Gly Leu Thr Thr Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Ala Gly Thr Thr Val Thr Val Ser Ser Gly Gly Gly Gly Ser Gly
           115                 120                 125
   Gly Gly Gly Ser Gly Gly Gly Gly Ser Ala Gln Asp Ile Gln Met Thr
       130                 135                 140
   Gln Thr Thr Ser Ser Leu Ser Ala Ser Leu Gly Asp Arg Val Thr Ile
   145                 150                 155                 160
   Ser Cys Arg Ala Ser Gln Asp Ile Arg Asn Tyr Leu Asn Trp Tyr Gln
                   165                 170                 175
   Gln Lys Pro Asp Gly Thr Val Lys Leu Leu Ile Tyr Tyr Thr Ser Arg
               180                 185                 190
   Leu His Ser Gly Val Pro Ser Arg Phe Ser Gly Ser Gly Ser Gly Thr
           195                 200                 205
   Asp Tyr Ser Leu Thr Ile Ala Asn Leu Gln Pro Glu Asp Ile Ala Thr
       210                 215                 220
   Tyr Phe Cys Gln Gln Gly Asn Thr Leu Pro Trp Thr Phe Gly Gly Gly
   225                 230                 235                 240
   Thr Lys Leu Val Thr Lys Arg Ser Ser Glu Pro Lys Ser Ser Asp Lys
                   245                 250                 255
   Thr His Thr Cys Pro Pro Cys Pro Ala Pro Glu Ala Ala Gly Ala Pro
                   260                 265                 270
   Ser Val Phe Leu Phe Pro Pro Lys Pro Lys Asp Thr Leu Met Ile Ser
           275                 280                 285
   Arg Thr Pro Glu Val Thr Cys Val Val Val Asp Val Ser His Glu Asp
       290                 295                 300
   Pro Glu Val Lys Phe Asn Trp Tyr Val Asp Gly Val Glu Val His Asn
   305                 310                 315                 320
   Ala Lys Thr Lys Pro Arg Glu Glu Gln Tyr Asn Ser Thr Tyr Arg Val
                   325                 330                 335
   Val Ser Val Leu Thr Val Leu His Gln Asp Trp Leu Asn Gly Lys Ala
               340                 345                 350
   Tyr Ala Cys Ala Val Ser Asn Lys Ala Leu Pro Ala Pro Ile Glu Lys
           355                 360                 365
   Thr Ile Ser Lys Ala Lys Gly Gln Pro Arg Glu Pro Gln Val Tyr Thr
       370                 375                 380
   Leu Pro Pro Ser Arg Asp Glu Leu Thr Lys Asn Gln Val Ser Leu Thr
   385                 390                 395                 400
   Cys Leu Val Lys Gly Phe Tyr Pro Ser Asp Ile Ala Val Glu Trp Glu
                   405                 410                 415
   Ser Asn Gly Gln Pro Glu Asn Asn Tyr Lys Thr Thr Pro Pro Val Leu
               420                 425                 430
   Asp Ser Asp Gly Ser Phe Phe Leu Tyr Ser Lys Leu Thr Val Asp Lys
           435                 440                 445
   Ser Arg Trp Gln Gln Gly Asn Val Phe Ser Cys Ser Val Met His Glu
       450                 455                 460
   Ala Leu His Asn His Tyr Thr Gln Lys Ser Leu Ser Leu Ser Pro Gly
   465                 470                 475                 480
   Lys Ser Arg Thr Val Ala Ala Pro Ser Val Phe Ile Phe Pro Pro Ser
                   485                 490                 495
   Asp Glu Gln Leu Lys Ser Gly Thr Ala Ser Val Val Cys Leu Leu Asn
               500                 505                 510
   Tyr Phe Tyr Pro Arg Glu Ala Lys Val Gln Trp Lys Val Asp Asn Ala
           515                 520                 525
   Leu Gln Ser Gly Asn Ser Gln Glu Ser Ala Thr Glu Gln Asp Ser Lys
       530                 535                 540
   Asp Ser Thr Tyr Ser Leu Ser Ser Glu Leu Thr Leu Ser Lys Ala Asp
   545                 550                 555                 560
   Tyr Glu Lys His Lys Val Tyr Ala Cys Glu Val Thr His Gln Gly Leu
                   565                 570                 575
   Ser Ser Pro Val Thr Lys Ser Phe Asn Arg Gly Glu
               580                 585
<210> 819
   <211> 853
   <212> PRT
   <213> Artificial Sequence
<220>
   <223> TSC064 comprises from its amino to carboxy terminus: G19-4 (anti-CD3) scFv, human IgG1 SCC-P hinge, human IgG1 CH2, human IgG1 CH3, human Ck(YAE), H75 linker, and Met021 (anti-c-Met) scFv
<400> 819
<210> 820
   <211> 583
   <212> PRT
   <213> Artificial Sequence
<220>
   <223> TSC076 comprises from its amino to carboxy terminus: OKT3 (anti-CD3) scFv, human IgG1 SCC-P hinge, human IgG1 CH2(ADCC/CDC null), human IgG1 CH3, and human Ck(YAE)
<400> 820
   Gln Val Gln Leu Gln Gln Ser Gly Ala Glu Leu Ala Arg Pro Gly Ala
    1               5                   10                  15
   Ser Val Lys Met Ser Cys Lys Ala Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Val Thr Val Ser Ser Gly Gly Gly Gly Ser Gly Gly Gly Gly
           115                 120                 125
   Ser Gly Gly Gly Gly Ser Ala Gln Gln Ile Val Leu Thr Gln Ser Pro
       130                 135                 140
   Ala Ile Met Ser Ala Ser Pro Gly Glu Lys Val Thr Met Thr Cys Ser
   145                 150                 155                 160
   Ala Ser Ser Ser Val Ser Tyr Met Asn Trp Tyr Gln Gln Lys Ser Gly
                   165                 170                 175
   Thr Ser Pro Lys Arg Trp Ile Tyr Asp Thr Ser Lys Leu Ala Ser Gly
               180                 185                 190
   Val Pro Ala His Phe Arg Gly Ser Gly Ser Gly Thr Ser Tyr Ser Leu
           195                 200                 205
   Thr Ile Ser Gly Met Glu Ala Glu Asp Ala Ala Thr Tyr Tyr Cys Gln
       210                 215                 220
   Gln Trp Ser Ser Asn Pro Phe Thr Phe Gly Ser Gly Thr Lys Leu Glu
   225                 230                 235                 240
   Ile Asn Ser Ser Glu Pro Lys Ser Ser Asp Lys Thr His Thr Cys Pro
                   245                 250                 255
   Pro Cys Pro Ala Pro Glu Ala Ala Gly Ala Pro Ser Val Phe Leu Phe
               260                 265                 270
   Pro Pro Lys Pro Lys Asp Thr Leu Met Ile Ser Arg Thr Pro Glu Val
           275                 280                 285
   Thr Cys Val Val Val Asp Val Ser His Glu Asp Pro Glu Val Lys Phe
       290                 295                 300
   Asn Trp Tyr Val Asp Gly Val Glu Val His Asn Ala Lys Thr Lys Pro
   305                 310                 315                 320
   Arg Glu Glu Gln Tyr Asn Ser Thr Tyr Arg Val Val Ser Val Leu Thr
                   325                 330                 335
   Val Leu His Gln Asp Trp Leu Asn Gly Lys Ala Tyr Ala Cys Ala Val
               340                 345                 350
   Ser Asn Lys Ala Leu Pro Ala Pro Ile Glu Lys Thr Ile Ser Lys Ala
           355                 360                 365
   Lys Gly Gln Pro Arg Glu Pro Gln Val Tyr Thr Leu Pro Pro Ser Arg
       370                 375                 380
   Asp Glu Leu Thr Lys Asn Gln Val Ser Leu Thr Cys Leu Val Lys Gly
   385                 390                 395                 400
   Phe Tyr Pro Ser Asp Ile Ala Val Glu Trp Glu Ser Asn Gly Gln Pro
                   405                 410                 415
   Glu Asn Asn Tyr Lys Thr Thr Pro Pro Val Leu Asp Ser Asp Gly Ser
               420                 425                 430
   Phe Phe Leu Tyr Ser Lys Leu Thr Val Asp Lys Ser Arg Trp Gln Gln
           435                 440                 445
   Gly Asn Val Phe Ser Cys Ser Val Met His Glu Ala Leu His Asn His
       450                 455                 460
   Tyr Thr Gln Lys Ser Leu Ser Leu Ser Pro Gly Lys Ser Arg Thr Val
   465                 470                 475                 480
   Ala Ala Pro Ser Val Phe Ile Phe Pro Pro Ser Asp Glu Gln Leu Lys
                   485                 490                 495
   Ser Gly Thr Ala Ser Val Val Cys Leu Leu Asn Tyr Phe Tyr Pro Arg
               500                 505                 510
   Glu Ala Lys Val Gln Trp Lys Val Asp Asn Ala Leu Gln Ser Gly Asn
           515                 520                 525
   Ser Gln Glu Ser Ala Thr Glu Gln Asp Ser Lys Asp Ser Thr Tyr Ser
       530                 535                 540
   Leu Ser Ser Glu Leu Thr Leu Ser Lys Ala Asp Tyr Glu Lys His Lys
   545                 550                 555                 560
   Val Tyr Ala Cys Glu Val Thr His Gln Gly Leu Ser Ser Pro Val Thr
                   565                 570                 575
   Lys Ser Phe Asn Arg Gly Glu
               580
<210> 821
   <211> 586
   <212> PRT
   <213> Artificial Sequence
<220>
   <223> TSC077 comprises: HuM291 (anti-CD3) scFv, human IgG1 SCC-P hinge, human IgG1 CH2(ADCC/CDC null), human IgG1 CH3, and human Ck(YAE)
<400> 821
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Ile Ser Tyr
               20                  25                  30
   Thr Met His Trp Val Arg Gln Ala Pro Gly Gln Gly Leu Glu Trp Met
           35                  40                  45
   Gly Tyr Ile Asn Pro Arg Ser Gly Tyr Thr His Tyr Asn Gln Lys Leu
       50                  55                  60
   Lys Asp Lys Ala Thr Leu Thr Ala Asp Lys Ser Ala Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ala Tyr Tyr Asp Tyr Asp Gly Phe Ala Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Ala Gln Asp Ile Gln Met Thr Gln Ser
       130                 135                 140
   Pro Ser Ser Leu Ser Ala Ser Val Gly Asp Arg Val Thr Ile Thr Cys
   145                 150                 155                 160
   Ser Ala Ser Ser Ser Val Ser Tyr Met Asn Trp Tyr Gln Gln Lys Pro
                   165                 170                 175
   Gly Lys Ala Pro Lys Arg Leu Ile Tyr Asp Thr Ser Lys Leu Ala Ser
               180                 185                 190
   Gly Val Pro Ser Arg Phe Ser Gly Ser Gly Ser Gly Thr Asp Phe Thr
           195                 200                 205
   Leu Thr Ile Ser Ser Leu Gln Pro Glu Asp Phe Ala Thr Tyr Tyr Cys
       210                 215                 220
   Gln Gln Trp Ser Ser Asn Pro Pro Thr Phe Gly Gly Gly Thr Lys Val
   225                 230                 235                 240
   Glu Ile Lys Arg Thr Ser Ser Glu Pro Lys Ser Ser Asp Lys Thr His
                   245                 250                 255
   Thr Cys Pro Pro Cys Pro Ala Pro Glu Ala Ala Gly Ala Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Thr Val Ala Ala Pro Ser Val Phe Ile Phe Pro Pro Ser Asp Glu
                       485                 490                 495
   Gln Leu Lys Ser Gly Thr Ala Ser Val Val Cys Leu Leu Asn Tyr Phe
                   500                 505                 510
   Tyr Pro Arg Glu Ala Lys Val Gln Trp Lys Val Asp Asn Ala Leu Gln
               515                 520                 525
   Ser Gly Asn Ser Gln Glu Ser Ala Thr Glu Gln Asp Ser Lys Asp Ser
           530                 535                 540
   Thr Tyr Ser Leu Ser Ser Glu Leu Thr Leu Ser Lys Ala Asp Tyr Glu
   545                 550                 555                 560
   Lys His Lys Val Tyr Ala Cys Glu Val Thr His Gln Gly Leu Ser Ser
                       565                 570                 575
   Pro Val Thr Lys Ser Phe Asn Arg Gly Glu
                   580                 585
<210> 822
   <211> 586
   <212> PRT
   <213> Artificial Sequence
<220>
   <223> TSC093 comprises from its amino to carboxy terminus: CRIS7 (anti-CD3) scFv, human IgG1 SCC-P hinge, human IgG1 CH2(ADCC/CDC null), human IgG1 CH3, and human Ck(YAE)
<400> 822
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Glu Pro Lys Ser Ser Asp Lys Thr His
                   245                 250                 255
   Thr Cys Pro Pro Cys Pro Ala Pro Glu Ala Ala Gly Ala Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Thr Val Ala Ala Pro Ser Val Phe Ile Phe Pro Pro Ser Asp Glu
                   485                 490                 495
   Gln Leu Lys Ser Gly Thr Ala Ser Val Val Cys Leu Leu Asn Tyr Phe
               500                 505                 510
   Tyr Pro Arg Glu Ala Lys Val Gln Trp Lys Val Asp Asn Ala Leu Gln
           515                 520                 525
   Ser Gly Asn Ser Gln Glu Ser Ala Thr Glu Gln Asp Ser Lys Asp Ser
       530                 535                 540
   Thr Tyr Ser Leu Ser Ser Glu Leu Thr Leu Ser Lys Ala Asp Tyr Glu
   545                 550                 555                 560
   Lys His Lys Val Tyr Ala Cys Glu Val Thr His Gln Gly Leu Ser Ser
                   565                 570                 575
   Pro Val Thr Lys Ser Phe Asn Arg Gly Glu
               580                 585
<210> 823
   <211> 570
   <212> PRT
   <213> Artificial Sequence
<220>
   <223> TSC094 comprises from its amino to carboxy terminus: OKT3-M (Micromet variant anti-CD3) scFv, human IgG1 SCC-P hinge, human IgG1 CH2(ADCC/CDC null), human IgG1 CH3, human Ck(YAE)
<400> 823
   Asp Ile Lys Leu Gln Gln Ser Gly Ala Glu Leu Ala Arg Pro Gly Ala
    1               5                  10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser Val Glu Gly Gly Ser Gly Gly Ser Gly
           115                 120                 125
   Gly Ser Gly Gly Ser Gly Gly Val Asp Asp Ile Gln Leu Thr Gln Ser
       130                 135                 140
   Pro Ala Ile Met Ser Ala Ser Pro Gly Glu Lys Val Thr Met Thr Cys
   145                 150                 155                 160
   Arg Ala Ser Ser Ser Val Ser Tyr Met Asn Trp Tyr Gln Gln Lys Ser
                   165                 170                 175
   Gly Thr Ser Pro Lys Arg Trp Ile Tyr Asp Thr Ser Lys Val Ala Ser
               180                 185                 190
   Gly Val Pro Tyr Arg Phe Ser Gly Ser Gly Ser Gly Thr Ser Tyr Ser
           195                 200                 205
   Leu Thr Ile Ser Ser Met Glu Ala Glu Asp Ala Ala Thr Tyr Tyr Cys
       210                 215                 220
   Gln Gln Trp Ser Ser Ser Ser Glu Pro Lys Ser Ser Asp Lys Thr His
   225                 230                 235                 240
   Thr Cys Pro Pro Cys Pro Ala Pro Glu Ala Ala Gly Ala Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Lys Phe Asn Trp Tyr Val Asp Gly Val Glu Val His Asn Ala Lys
       290                 295                 300
   Thr Lys Pro Arg Glu Glu Gln Tyr Asn Ser Thr Tyr Arg Val Val Ser
   305                 310                 315                 320
   Val Leu Thr Val Leu His Gln Asp Trp Leu Asn Gly Lys Ala Tyr Ala
                   325                 330                 335
   Cys Ala Val Ser Asn Lys Ala Leu Pro Ala Pro Ile Glu Lys Thr Ile
               340                 345                 350
   Ser Lys Ala Lys Gly Gln Pro Arg Glu Pro Gln Val Tyr Thr Leu Pro
           355                 360                 365
   Pro Ser Arg Asp Glu Leu Thr Lys Asn Gln Val Ser Leu Thr Cys Leu
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Val Leu Asp Ser
                   405                 410                 415
   Asp Gly Ser Phe Phe Leu Tyr Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys Ser
       450                 455                 460
   Arg Thr Val Ala Ala Pro Ser Val Phe Ile Phe Pro Pro Ser Asp Glu
   465                 470                 475                 480
   Gln Leu Lys Ser Gly Thr Ala Ser Val Val Cys Leu Leu Asn Tyr Phe
                   485                 490                 495
   Tyr Pro Arg Glu Ala Lys Val Gln Trp Lys Val Asp Asn Ala Leu Gln
               500                 505                 510
   Ser Gly Asn Ser Gln Glu Ser Ala Thr Glu Gln Asp Ser Lys Asp Ser
           515                 520                 525
   Thr Tyr Ser Leu Ser Ser Glu Leu Thr Leu Ser Lys Ala Asp Tyr Glu
       530                 535                 540
   Lys His Lys Val Tyr Ala Cys Glu Val Thr His Gln Gly Leu Ser Ser
   545                 550                 555                 560
   Pro Val Thr Lys Ser Phe Asn Arg Gly Glu
                   565                 570
<210> 824
   <211> 586
   <212> PRT
   <213> Artificial Sequence
<220>
   <223> TSC095 comprises from its amino to carboxy terminus: BMA031 (anti-TCR) scFv, human **I**gG1 SCC-P hinge, human IgG1 CH2(ADCC/CDC null), human IgG1 CH3, human Ck(YAE)
<400> 824
   Gly Gln Val Gln Leu Val Gln Ser Gly Ala Glu Val Lys Lys Pro Gly
    1               5                  10                  15
   Ser Ser Val Lys Val Ser Cys Lys Ala Ser Gly Tyr Lys Phe Thr Ser
               20                  25                  30
   Tyr Val Met His Trp Val Lys Gln Ala Pro Gly Gln Gly Leu Glu Trp
           35                  40                  45
   Ile Gly Tyr Ile Asn Pro Tyr Asn Asp Val Thr Lys Tyr Asn Glu Lys
       50                  55                  60
   Phe Lys Gly Lys Ala Thr Leu Thr Ala Asp Glu Ser Thr Asn Thr Ala
   65                  70                  75                  80
   Tyr Met Glu Leu Ser Ser Leu Arg Ser Glu Asp Thr Ala Val His Tyr
                   85                  90                  95
   Cys Ala Arg Gly Ser Tyr Tyr Asp Tyr Asp Gly Phe Val Tyr Trp Gly
               100                 105                 110
   Gln Gly Thr Leu Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Thr Leu Ser Ala Ser Val Gly Asp Arg Val Thr Met Thr
   145                 150                 155                 160
   Cys Ser Ala Thr Ser Ser Val Ser Tyr Met His Trp Tyr Gln Gln Lys
                   165                 170                 175
   Pro Gly Lys Ala Pro Lys Arg Trp Ile Tyr Asp Thr Ser Lys Leu Ala
               180                 185                 190
   Ser Gly Val Pro Ala Arg Phe Ile Gly Ser Gly Ser Gly Thr Glu Phe
           195                 200                 205
   Thr Leu Thr Ile Ser Ser Leu Gln Pro Asp Asp Phe Ala Thr Tyr Tyr
       210                 215                 220
   Cys Gln Gln Trp Ser Ser Asn Pro Leu Thr Phe Gly Gly Gly Thr Lys
   225                 230                 235                 240
   Val Glu Ile Lys Ser Ser Ser Glu Pro Lys Ser Ser Asp Lys Thr His
                   245                 250                 255
   Thr Cys Pro Pro Cys Pro Ala Pro Glu Ala Ala Gly Ala Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys Ser
   465                 470                 475                 480
   Arg Thr Val Ala Ala Pro Ser Val Phe Ile Phe Pro Pro Ser Asp Glu
                   485                 490                 495
   Gln Leu Lys Ser Gly Thr Ala Ser Val Val Cys Leu Leu Asn Tyr Phe
               500                 505                 510
   Tyr Pro Arg Glu Ala Lys Val Gln Trp Lys Val Asp Asn Ala Leu Gln
           515                 520                 525
   Ser Gly Asn Ser Gln Glu Ser Ala Thr Glu Gln Asp Ser Lys Asp Ser
       530                 535                 540
   Thr Tyr Ser Leu Ser Ser Glu Leu Thr Leu Ser Lys Ala Asp Tyr Glu
   545                 550                 555                 560
   Lys His Lys Val Tyr Ala Cys Glu Val Thr His Gln Gly Leu Ser Ser
                   565                 570                 575
   Pro Val Thr Lys Ser Phe Asn Arg Gly Glu
               580                 585
<210> 825
   <211> 591
   <212> PRT
   <213> Artificial Sequence
<220>
   <223> TSC096 comprises: HD37 (anti-CD19) scFv, human IgG1 SCC-P hinge, human IgG1 CH2 (ADCC/CDC null), human IgG1 CH3, human Ck(YAE)
<400> 825
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Cys Pro Pro Cys Pro Ala Pro Glu Ala Ala
               260                 265                 270
   Gly Ala Pro Ser Val Phe Leu Phe Pro Pro Lys Pro Lys Asp Thr Leu
           275                 280                 285
   Met Ile Ser Arg Thr Pro Glu Val Thr Cys Val Val Val Asp Val Ser
       290                 295                 300
   His Glu Asp Pro Glu Val Lys Phe Asn Trp Tyr Val Asp Gly Val Glu
   305                 310                 315                 320
   Val His Asn Ala Lys Thr Lys Pro Arg Glu Glu Gln Tyr Asn Ser Thr
                   325                 330                 335
   Tyr Arg Val Val Ser Val Leu Thr Val Leu His Gln Asp Trp Leu Asn
               340                 345                 350
   Gly Lys Ala Tyr Ala Cys Ala Val Ser Asn Lys Ala Leu Pro Ala Pro
           355                 360                 365
   Ile Glu Lys Thr Ile Ser Lys Ala Lys Gly Gln Pro Arg Glu Pro Gln
       370                 375                 380
   Val Tyr Thr Leu Pro Pro Ser Arg Asp Glu Leu Thr Lys Asn Gln Val
   385                 390                 395                 400
   Ser Leu Thr Cys Leu Val Lys Gly Phe Tyr Pro Ser Asp Ile Ala Val
                   405                 410                 415
   Glu Trp Glu Ser Asn Gly Gln Pro Glu Asn Asn Tyr Lys Thr Thr Pro
               420                 425                 430
   Pro Val Leu Asp Ser Asp Gly Ser Phe Phe Leu Tyr Ser Lys Leu Thr
           435                 440                 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465                 470                 475                 480
   Ser Pro Gly Lys Ser Arg Thr Val Ala Ala Pro Ser Val Phe Ile Phe
                   485                 490                 495
   Pro Pro Ser Asp Glu Gln Leu Lys Ser Gly Thr Ala Ser Val Val Cys
               500                 505                 510
   Leu Leu Asn Tyr Phe Tyr Pro Arg Glu Ala Lys Val Gln Trp Lys Val
           515                 520                 525
   Asp Asn Ala Leu Gln Ser Gly Asn Ser Gln Glu Ser Ala Thr Glu Gln
       530                 535                 540
   Asp Ser Lys Asp Ser Thr Tyr Ser Leu Ser Ser Glu Leu Thr Leu Ser
   545                 550                 555                 560
   Lys Ala Asp Tyr Glu Lys His Lys Val Tyr Ala Cys Glu Val Thr His
                   565                 570                 575
   Gln Gly Leu Ser Ser Pro Val Thr Lys Ser Phe Asn Arg Gly Glu
               580                 585                 590
<210> 826
   <211> 583
   <212> PRT
   <213> Artificial Sequence
<220>
   <223> TSC097 comprises from its amino to carboxy terminus: 4C04 (anti-RON) scFv, human IgG1 SCC-P hinge, human IgG1 CH2(ADCC/CDC null), human IgG1 CH3, and human Ck(YAE)
<400> 826
<210> 827
   <211> 512
   <212> PRT
   <213> Artificial Sequence
<220>
   <223> Bispecific T-Cell Engaging molecule (BiTE) known as bsc19x3
<400> 827
   Asp Tyr Lys Asp Asp Asp Asp Lys Asp Ile Gln Leu Thr Gln Ser Pro
    1               5                   10                  15
   Ala Ser Leu Ala Val Ser Leu Gly Gln Arg Ala Thr Ile Ser Cys Lys
               20                  25                  30
   Ala Ser Gln Ser Val Asp Tyr Asp Gly Asp Ser Tyr Leu Asn Trp Tyr
           35                  40                  45
   Gln Gln Ile Pro Gly Gln Pro Pro Lys Leu Leu Ile Tyr Asp Ala Ser
       50                  55                  60
   Asn Leu Val Ser Gly Ile Pro Pro Arg Phe Ser Gly Ser Gly Ser Gly
   65                  70                  75                  80
   Thr Asp Phe Thr Leu Asn Ile His Pro Val Glu Lys Val Asp Ala Ala
                   85                  90                  95
   Thr Tyr His Cys Gln Gln Ser Thr Glu Asp Pro Trp Thr Phe Gly Gly
               100                 105                 110
   Gly Thr Lys Leu Glu Ile Lys Gly Gly Gly Gly Ser Gly Gly Gly Gly
           115                 120                 125
   Ser Gly Gly Gly Gly Ser Gln Val Gln Leu Gln Gln Ser Gly Ala Glu
       130                 135                 140
   Leu Val Arg Pro Gly Ser Ser Val Lys Ile Ser Cys Lys Ala Ser Gly
   145                 150                 155                 160
   Tyr Ala Phe Ser Ser Tyr Trp Met Asn Trp Val Lys Gln Arg Pro Gly
                   165                 170                 175
   Gln Gly Leu Glu Trp Ile Gly Gln Ile Trp Pro Gly Asp Gly Asp Thr
               180                 185                 190
   Asn Tyr Asn Gly Lys Phe Lys Gly Lys Ala Thr Leu Thr Ala Asp Glu
           195                 200                 205
   Ser Ser Ser Thr Ala Tyr Met Gln Leu Ser Ser Leu Ala Ser Glu Asp
       210                 215                 220
   Ser Ala Val Tyr Phe Cys Ala Arg Arg Glu Thr Thr Thr Val Gly Arg
   225                 230                 235                 240
   Tyr Tyr Tyr Ala Met Asp Tyr Trp Gly Gln Gly Thr Thr Val Thr Val
                   245                 250                 255
   Ser Ser Gly Gly Gly Gly Ser Asp Ile Lys Leu Gln Gln Ser Gly Ala
               260                 265                 270
   Glu Leu Ala Arg Pro Gly Ala Ser Val Lys Met Ser Cys Lys Thr Ser
           275                 280                 285
   Gly Tyr Thr Phe Thr Arg Tyr Thr Met His Trp Val Lys Gln Arg Pro
       290                 295                 300
   Gly Gln Gly Leu Glu Trp Ile Gly Tyr Ile Asn Pro Ser Arg Gly Tyr
   305                 310                 315                 320
   Thr Asn Tyr Asn Gln Lys Phe Lys Asp Lys Ala Thr Leu Thr Thr Asp
                   325                 330                 335
   Lys Ser Ser Ser Thr Ala Tyr Met Gln Leu Ser Ser Leu Thr Ser Glu
               340                 345                 350
   Asp Ser Ala Val Tyr Tyr Cys Ala Arg Tyr Tyr Asp Asp His Tyr Cys
           355                 360                 365
   Leu Asp Tyr Trp Gly Gln Gly Thr Thr Leu Thr Val Ser Ser Val Glu
       370                 375                 380
   Gly Gly Ser Gly Gly Ser Gly Gly Ser Gly Gly Ser Gly Gly Val Asp
   385                 390                 395                 400
   Asp Ile Gln Leu Thr Gln Ser Pro Ala Ile Met Ser Ala Ser Pro Gly
                   405                 410                 415
   Glu Lys Val Thr Met Thr Cys Arg Ala Ser Ser Ser Val Ser Tyr Met
               420                 425                 430
   Asn Trp Tyr Gln Gln Lys Ser Gly Thr Ser Pro Lys Arg Trp Ile Tyr
           435                 440                 445
   Asp Thr Ser Lys Val Ala Ser Gly Val Pro Tyr Arg Phe Ser Gly Ser
       450                 455                 460
   Gly Ser Gly Thr Ser Tyr Ser Leu Thr Ile Ser Ser Met Glu Ala Glu
   465                 470                 475                 480
   Asp Ala Ala Thr Tyr Tyr Cys Gln Gln Trp Ser Ser Asn Pro Leu Thr
                   485                 490                 495
   Phe Gly Ala Gly Thr Lys Leu Glu Leu Lys His His His His His His
               500                 505                 510
<210> 828
   <211> 120
   <212> PRT
   <213> Homo sapiens
<220>
   <223> VH region of BMA031 (anti-TCR) scFv
<400> 828
   Gln Val Gln Leu Val Gln Ser Gly Ala Glu Val Lys Lys Pro Gly Ser
    1               5                  10                  15
   Ser Val Lys Val Ser Cys Lys Ala Ser Gly Tyr Lys Phe Thr Ser Tyr
               20                  25                  30
   Val Met His Trp Val Lys Gln Ala Pro Gly Gln Gly Leu Glu Trp Ile
           35                  40                  45
   Gly Tyr Ile Asn Pro Tyr Asn Asp Val Thr Lys Tyr Asn Glu Lys Phe
       50                  55                  60
   Lys Gly Lys Ala Thr Leu Thr Ala Asp Glu Ser Thr Asn Thr Ala Tyr
   65                  70                  75                  80
   Met Glu Leu Ser Ser Leu Arg Ser Glu Asp Thr Ala Val His Tyr Cys
                   85                  90                  95
   Ala Arg Gly Ser Tyr Tyr Asp Tyr Asp Gly Phe Val Tyr Trp Gly Gln
               100                 105                 110
   Gly Thr Leu Val Thr Val Ser Ser
           115                 120
<210> 829
   <211> 111
   <212> PRT
   <213> Homo sapiens
<220>
   <223> VL region of BMA031 (anti-TCR) scFv
<400> 829
   Ala Gln Asp Ile Gln Met Thr Gln Ser Pro Ser Thr Leu Ser Ala Ser
    1               5                  10                  15
   Val Gly Asp Arg Val Thr Met Thr Cys Ser Ala Thr Ser Ser Val Ser
               20                  25                  30
   Tyr Met His Trp Tyr Gln Gln Lys Pro Gly Lys Ala Pro Lys Arg Trp
           35                  40                  45
   Ile Tyr Asp Thr Ser Lys Leu Ala Ser Gly Val Pro Ala Arg Phe Ile
       50                  55                  60
   Gly Ser Gly Ser Gly Thr Glu Phe Thr Leu Thr Ile Ser Ser Leu Gln
   65                  70                  75                  80
   Pro Asp Asp Phe Ala Thr Tyr Tyr Cys Gln Gln Trp Ser Ser Asn Pro
                   85                  90                  95
   Leu Thr Phe Gly Gly Gly Thr Lys Val Glu Ile Lys Ser Ser Ser
               100                 105                 110
<210> 830
   <211> 246
   <212> PRT
   <213> Homo sapiens
<220>
   <223> BMA031 scFv
<400> 830
   Gln Val Gln Leu Val Gln Ser Gly Ala Glu Val Lys Lys Pro Gly Ser
    1               5                  10                  15
   Ser Val Lys Val Ser Cys Lys Ala Ser Gly Tyr Lys Phe Thr Ser Tyr
               20                  25                  30
   Val Met His Trp Val Lys Gln Ala Pro Gly Gln Gly Leu Glu Trp Ile
           35                  40                  45
   Gly Tyr Ile Asn Pro Tyr Asn Asp Val Thr Lys Tyr Asn Glu Lys Phe
       50                  55                  60
   Lys Gly Lys Ala Thr Leu Thr Ala Asp Glu Ser Thr Asn Thr Ala Tyr
   65                  70                  75                  80
   Met Glu Leu Ser Ser Leu Arg Ser Glu Asp Thr Ala Val His Tyr Cys
                   85                  90                  95
   Ala Arg Gly Ser Tyr Tyr Asp Tyr Asp Gly Phe Val Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Ala Gln Asp Ile Gln Met Thr Gln Ser
       130                 135                 140
   Pro Ser Thr Leu Ser Ala Ser Val Gly Asp Arg Val Thr Met Thr Cys
   145                 150                 155                 160
   Ser Ala Thr Ser Ser Val Ser Tyr Met His Trp Tyr Gln Gln Lys Pro
                   165                 170                 175
   Gly Lys Ala Pro Lys Arg Trp Ile Tyr Asp Thr Ser Lys Leu Ala Ser
               180                 185                 190
   Gly Val Pro Ala Arg Phe Ile Gly Ser Gly Ser Gly Thr Glu Phe Thr
           195                 200                 205
   Leu Thr Ile Ser Ser Leu Gln Pro Asp Asp Phe Ala Thr Tyr Tyr Cys
       210                 215                 220
   Gln Gln Trp Ser Ser Asn Pro Leu Thr Phe Gly Gly Gly Thr Lys Val
   225                 230                 235                 240
   Glu Ile Lys Ser Ser Ser
                   245
<210> 831
   <211> 119
   <212> PRT
   <213> Homo sapiens
<220>
   <223> VH region of OKT3-M (anti-CD3) scFv
<400> 831
   Asp Ile Lys Leu Gln Gln Ser Gly Ala Glu Leu Ala Arg Pro Gly Ala
   1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser
           115
<210> 832
   <211> 18
   <212> PRT
   <213> Artificial Sequence
<220>
   <223> OKT3-M Linker
<400> 832
   Val Glu Gly Gly Ser Gly Gly Ser Gly Gly Ser Gly Gly Ser Gly Gly
   1               5                   10                  15
   Val Asp
<210> 833
   <211> 94
   <212> PRT
   <213> Homo sapiens
<220>
   <223> VL region of OKT3-M (anti-CD3) scFv
<400> 833
   Asp Ile Gln Leu Thr Gln Ser Pro Ala Ile Met Ser Ala Ser Pro Gly
   1               5                   10                  15
   Glu Lys Val Thr Met Thr Cys Arg Ala Ser Ser Ser Val Ser Tyr Met
               20                  25                  30
   Asn Trp Tyr Gln Gln Lys Ser Gly Thr Ser Pro Lys Arg Trp Ile Tyr
           35                  40                  45
   Asp Thr Ser Lys Val Ala Ser Gly Val Pro Tyr Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Ser Ser
                   85                  90
<210> 834
   <211> 231
   <212> PRT
   <213> Homo sapiens
<220>
   <223> OKT3-M scFv
<400> 834
   Asp Ile Lys Leu Gln Gln Ser Gly Ala Glu Leu Ala Arg Pro Gly Ala
   1               5                   10                  15
   Ser Val Lys Met Ser Cys Lys Thr Ser Gly Tyr Thr Phe Thr Arg Tyr
               20                  25                  30
   Thr Met His Trp Val Lys Gln Arg Pro Gly Gln Gly Leu Glu Trp Ile
           35                  40                  45
   Gly Tyr Ile Asn Pro Ser Arg Gly Tyr Thr Asn Tyr Asn Gln Lys Phe
       50                  55                  60
   Lys Asp Lys Ala Thr Leu Thr Thr Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Asp Asp His Tyr Cys Leu Asp Tyr Trp Gly Gln Gly
               100                 105                 110
   Thr Thr Leu Thr Val Ser Ser Val Glu Gly Gly Ser Gly Gly Ser Gly
           115                 120                 125
   Gly Ser Gly Gly Ser Gly Gly Val Asp Asp Ile Gln Leu Thr Gln Ser
       130                 135                 140
   Pro Ala Ile Met Ser Ala Ser Pro Gly Glu Lys Val Thr Met Thr Cys
   145                 150                 155                 160
   Arg Ala Ser Ser Ser Val Ser Tyr Met Asn Trp Tyr Gln Gln Lys Ser
                   165                 170                 175
   Gly Thr Ser Pro Lys Arg Trp Ile Tyr Asp Thr Ser Lys Val Ala Ser
               180                 185                 190
   Gly Val Pro Tyr Arg Phe Ser Gly Ser Gly Ser Gly Thr Ser Tyr Ser
           195                 200                 205
   Leu Thr Ile Ser Ser Met Glu Ala Glu Asp Ala Ala Thr Tyr Tyr Cys
       210                 215                 220
   Gln Gln Trp Ser Ser Ser Ser
   225                 230
<210> 835
   <211> 120
   <212> PRT
   <213> Homo sapiens
<220>
   <223> VH region of HuM291 (anti-CD3) scFv
<400> 835
<210> 836
   <211> 112
   <212> PRT
   <213> Homo sapiens
<220>
   <223> VL region of HuM291 (anti-CD3) scFv
<400> 836
   Ala Gln Asp Ile Gln Met Thr Gln Ser Pro Ser Ser Leu Ser Ala Ser
    1               5                  10                  15
   Val Gly Asp Arg Val Thr Ile Thr Cys Ser Ala Ser Ser Ser Val Ser
               20                  25                  30
   Tyr Met Asn Trp Tyr Gln Gln Lys Pro Gly Lys Ala Pro Lys Arg Leu
           35                  40                  45
   Ile Tyr Asp Thr Ser Lys Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gln Gln Trp Ser Ser Asn Pro
                   85                  90                  95
   Pro Thr Phe Gly Gly Gly Thr Lys Val Glu Ile Lys Arg Thr Ser Ser
               100                 105                 110
<210> 837
   <211> 247
   <212> PRT
   <213> Homo sapiens
<220>
   <223> HuM291 scFv
<400> 837
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Ile Ser Tyr
               20                  25                  30
   Thr Met His Trp Val Arg Gln Ala Pro Gly Gln Gly Leu Glu Trp Met
           35                  40                  45
   Gly Tyr Ile Asn Pro Arg Ser Gly Tyr Thr His Tyr Asn Gln Lys Leu
       50                  55                  60
   Lys Asp Lys Ala Thr Leu Thr Ala Asp Lys Ser Ala Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ala Tyr Tyr Asp Tyr Asp Gly Phe Ala Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Ala Gln Asp Ile Gln Met Thr Gln Ser
       130                 135                 140
   Pro Ser Ser Leu Ser Ala Ser Val Gly Asp Arg Val Thr Ile Thr Cys
   145                 150                 155                 160
   Ser Ala Ser Ser Ser Val Ser Tyr Met Asn Trp Tyr Gln Gln Lys Pro
                   165                 170                 175
   Gly Lys Ala Pro Lys Arg Leu Ile Tyr Asp Thr Ser Lys Leu Ala Ser
               180                 185                 190
   Gly Val Pro Ser Arg Phe Ser Gly Ser Gly Ser Gly Thr Asp Phe Thr
           195                 200                 205
   Leu Thr Ile Ser Ser Leu Gln Pro Glu Asp Phe Ala Thr Tyr Tyr Cys
       210                 215                 220
   Gln Gln Trp Ser Ser Asn Pro Pro Thr Phe Gly Gly Gly Thr Lys Val
   225                 230                 235                 240
   Glu Ile Lys Arg Thr Ser Ser
                   245
<210> 838
   <211> 105
   <212> PRT
   <213> Artificial Sequence
<220>
   <223> mutated human Ck DAE
<400> 838
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asp Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
                       100                 105
<210> 839
   <211> 105
   <212> PRT
   <213> Artificial Sequence
<220>
   <223> mutated human Ck MAE
<400> 839
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Met Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 840
   <211> 105
   <212> PRT
   <213> Artificial Sequence
<220>
   <223> mutated human Ck SAE
<400> 840
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Ser Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 841
   <211> 105
   <212> PRT
   <213> Artificial Sequence
<220>
   <223> mutated human FAE
<400> 841
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Phe Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Ala Thr Glu Gln Asp Ser Lys Asp Ser Thr
       50                  55                  60
   Tyr Ser Leu Ser Ser Glu Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 842
   <211> 105
   <212> PRT
   <213> Artificial Sequence
<220>
   <223> mutated Ck L29E
<400> 842
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Glu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 843
   <211> 105
   <212> PRT
   <213> Artificial Sequence
<220>
   <223> mutated Ck L29K
<400> 843
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Lys Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu
               100                 105
<210> 844
   <211> 98
   <212> PRT
   <213> Artificial Sequence
<220>
   <223> mutated CH1 V68E
<400> 844
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Glu Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val
<210> 845
   <211> 98
   <212> PRT
   <213> Artificial Sequence
<220>
   <223> mutated CH1 V68K
<400> 845
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Lys Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val
<210> 846
   <211> 3
   <212> PRT
   <213> Artificial Sequence
<220>
   <223> CH3-CH1 linker or CH3-Ck linker
<400> 846
   Lys Ser Arg
   1
<210> 847
   <211> 5
   <212> PRT
   <213> Artificial Sequence
<220>
   <223> CH3-CH1 linker
<400> 847
   Gly Gly Gly Ser Ser
   1 5
<210> 848
   <211> 6
   <212> PRT
   <213> Artificial Sequence
<220>
   <223> CH3-CH1 linker
<400> 848
   Ser Tyr Ser Pro Asn Ser
   1 5
<210> 849
   <211> 8
   <212> PRT
   <213> Artificial Sequence
<220>
   <223> CH3-CH1 linker
<400> 849
   Ser Ser Leu Asn Thr Pro Asn Ser
   1 5
<210> 850
   <211> 5
   <212> PRT
   <213> Artificial Sequence
<220>
   <223> CH3-Ck linker
<400> 850
   Gly Gly Gly Ser Arg
   1 5
<210> 851
   <211> 7
   <212> PRT
   <213> Artificial Sequence
<220>
   <223> CH3-Ck linker
<400> 851
   Ser Tyr Ser Pro Asn Ser Arg
   1 5
<210> 852
   <211> 9
   <212> PRT
   <213> Artificial Sequence
<220>
   <223> CH3-Ck linker
<400> 852
   Ser Ser Leu Asn Thr Pro Asn Ser Arg
   1 5
<210> 853
   <211> 1806
   <212> DNA
   <213> Artificial Sequence
<220>
   <223> TSC145 (Cris7 scFv-hinge-CH2-CH3-GGGSS linker-CH1)
<400> 853
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag tgagcccaaa tcttctgaca aaactcacac atgcccaccg     840
   tgcccagcac ctgaagccgc gggtgcaccg tcagtcttcc tcttcccccc aaaacccaag     900
   gacaccctca tgatctcccg gacccctgag gtcacatgcg tggtggtgga cgtgagccac     960
   gaagaccctg aggtcaagtt caactggtac gtggacggcg tggaggtgca taatgccaag    1020
   acaaagccgc gggaggagca gtacaacagc acgtaccgtg tggtcagcgt cctcaccgtc    1080
   ctgcaccagg actggctgaa tggcaaggcg tacgcgtgcg cggtctccaa caaagccctc    1140
   ccagccccca tcgagaaaac catctccaaa gccaaagggc agccccgaga accacaggtg    1200
   tacaccctgc ccccatcccg ggatgagctg accaagaacc aggtcagcct gacctgcctg    1260
   gtcaaaggct tctatccaag cgacatcgcc gtggagtggg agagcaatgg gcagccggag    1320
   aacaactaca agaccacgcc tcccgtgctg gactccgacg gctccttctt cctctacagc    1380
   aagctcaccg tggacaagag caggtggcag caggggaacg tcttctcatg ctccgtgatg    1440
   catgaggctc tgcacaacca ctacacgcag aagagcctct ccctgtctcc gggtggcgga    1500
   ggctcctcag cctccaccaa gggcccatcg gtcttccccc tggcaccctc ctccaagagc    1560
   acctctgggg gcacagcggc cctgggctgc ctggtcaagg actacttccc cgagccggtg    1620
   acggtgtcgt ggaactcagg cgccctgacc agcggcgtgc acaccttccc ggctgtccta    1680
   cagtcctcag gactctactc cctcagcagc gtggtgaccg tgccctccag cagcttgggc    1740
   acccagacct acatctgcaa cgtgaatcac aagcccagca acaccaaggt ggacaagaaa    1800
   gtttga                                                               1806
<210> 854
   <211> 1809
   <212> DNA
   <213> Artificial Sequence
<220>
   <223> TSC146 (Cris7 scFv-hinge-CH2-CH3-SYSPNS linker-CH1)
<400> 854
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag tgagcccaaa tcttctgaca aaactcacac atgcccaccg     840
   tgcccagcac ctgaagccgc gggtgcaccg tcagtcttcc tcttcccccc aaaacccaag     900
   gacaccctca tgatctcccg gacccctgag gtcacatgcg tggtggtgga cgtgagccac     960
   gaagaccctg aggtcaagtt caactggtac gtggacggcg tggaggtgca taatgccaag    1020
   acaaagccgc gggaggagca gtacaacagc acgtaccgtg tggtcagcgt cctcaccgtc    1080
   ctgcaccagg actggctgaa tggcaaggcg tacgcgtgcg cggtctccaa caaagccctc    1140
   ccagccccca tcgagaaaac catctccaaa gccaaagggc agccccgaga accacaggtg    1200
   tacaccctgc ccccatcccg ggatgagctg accaagaacc aggtcagcct gacctgcctg    1260
   gtcaaaggct tctatccaag cgacatcgcc gtggagtggg agagcaatgg gcagccggag    1320
   aacaactaca agaccacgcc tcccgtgctg gactccgacg gctccttctt cctctacagc    1380
   aagctcaccg tggacaagag caggtggcag caggggaacg tcttctcatg ctccgtgatg    1440
   catgaggctc tgcacaacca ctacacgcag aagagcctct ccctgtctcc gggtagttac    1500
   agcccgaact ctgcctccac caagggccca tcggtcttcc ccctggcacc ctcctccaag    1560
   agcacctctg ggggcacagc ggccctgggc tgcctggtca aggactactt ccccgagccg    1620
   gtgacggtgt cgtggaactc aggcgccctg accagcggcg tgcacacctt cccggctgtc    1680
   ctacagtcct caggactcta ctccctcagc agcgtggtga ccgtgccctc cagcagcttg    1740
   ggcacccaga cctacatctg caacgtgaat cacaagccca gcaacaccaa ggtggacaag    1800
   aaagtttga                                                             1809
<210> 855
   <211> 1815
   <212> DNA
   <213> Artificial Sequence
<220>
   <223> TSC147 (Cris7 scFv-hinge-CH2-CH3-SSLNTPNS linker-CH1)
<400> 855
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag tgagcccaaa tcttctgaca aaactcacac atgcccaccg     840
   tgcccagcac ctgaagccgc gggtgcaccg tcagtcttcc tcttcccccc aaaacccaag     900
   gacaccctca tgatctcccg gacccctgag gtcacatgcg tggtggtgga cgtgagccac     960
   gaagaccctg aggtcaagtt caactggtac gtggacggcg tggaggtgca taatgccaag    1020
   acaaagccgc gggaggagca gtacaacagc acgtaccgtg tggtcagcgt cctcaccgtc    1080
   ctgcaccagg actggctgaa tggcaaggcg tacgcgtgcg cggtctccaa caaagccctc    1140
   ccagccccca tcgagaaaac catctccaaa gccaaagggc agccccgaga accacaggtg    1200
   tacaccctgc ccccatcccg ggatgagctg accaagaacc aggtcagcct gacctgcctg    1260
   gtcaaaggct tctatccaag cgacatcgcc gtggagtggg agagcaatgg gcagccggag    1320
   aacaactaca agaccacgcc tcccgtgctg gactccgacg gctccttctt cctctacagc    1380
   aagctcaccg tggacaagag caggtggcag caggggaacg tcttctcatg ctccgtgatg    1440
   catgaggctc tgcacaacca ctacacgcag aagagcctct ccctgtctcc gggttcttcc    1500
   ctgaatacac cgaactctgc ctccaccaag ggcccatcgg tcttccccct ggcaccctcc    1560
   tccaagagca cctctggggg cacagcggcc ctgggctgcc tggtcaagga ctacttcccc    1620
   gagccggtga cggtgtcgtg gaactcaggc gccctgacca gcggcgtgca caccttcccg    1680
   gctgtcctac agtcctcagg actctactcc ctcagcagcg tggtgaccgt gccctccagc    1740
   agcttgggca cccagaccta catctgcaac gtgaatcaca agcccagcaa caccaaggtg    1800
   gacaagaaag tttga                                                     1815
<210> 856
   <211> 1842
   <212> DNA
   <213> Artificial Sequence
<220>
   <223> TSC148 (HD37 scFv-hinge-CH2-CH3-GGGSR linker-Ck)
<400> 856
   atggaagcac cagcgcagct tctcttcctc ctgctactct ggctcccaga taccaccggt      60
   gacattgtgc tgacccaatc tccagcttct ttggctgtgt ctctagggca gagggccacc     120
   atctcctgca aggccagcca aagtgttgat tatgatggtg atagttattt gaactggtac     180
   caacagattc caggacagcc acccaaactc ctcatctatg atgcatccaa tctagtttct     240
   gggatcccac ccaggtttag tggcagtggg tctgggacag acttcaccct caacatccat     300
   cctgtggaga aggtggatgc tgcaacctat cactgccagc aaagtactga ggatccgtgg     360
   acgttcggtg gaggcaccaa gctggaaatc aaaggtggcg gtggttcggg cggtggtggg     420
   tcgggtggcg gcggagctag ccaggttcag ctgcagcagt ctggggctga gctggtgagg     480
   cctgggtcct cagtgaagat ttcctgcaag gcttctggct atgcattcag tagctactgg     540
   atgaactggg tgaagcagag gcctggacag ggtcttgagt ggattggaca gatttggcct     600
   ggagatggtg atactaacta caatggaaag ttcaagggta aagccactct gactgcagac     660
   gaatcctcca gcacagccta catgcaactc agcagcctag catctgagga ctctgcggtc     720
   tatttctgtg caagacggga gactacgacg gtaggccgtt attactatgc tatggactac     780
   tggggtcaag gaacctcagt caccgtctcc tcgagtgagc ccaaatcttc tgacaaaact     840
   cacacatgcc caccgtgccc agcacctgaa gccgcgggtg caccgtcagt cttcctcttc     900
   cccccaaaac ccaaggacac cctcatgatc tcccggaccc ctgaggtcac atgcgtggtg     960
   gtggacgtga gccacgaaga ccctgaggtc aagttcaact ggtacgtgga cggcgtggag    1020
   gtgcataatg ccaagacaaa gccgcgggag gagcagtaca acagcacgta ccgtgtggtc    1080
   agcgtcctca ccgtcctgca ccaggactgg ctgaatggca aggcgtacgc gtgcgcggtc    1140
   tccaacaaag ccctcccagc ccccatcgag aaaaccatct ccaaagccaa agggcagccc    1200
   cgagaaccac aggtgtacac cctgccccca tcccgggatg agctgaccaa gaaccaggtc    1260
   agcctgacct gcctggtcaa aggcttctat ccaagcgaca tcgccgtgga gtgggagagc    1320
   aatgggcagc cggagaacaa ctacaagacc acgcctcccg tgctggactc cgacggctcc    1380
   ttcttcctct acagcaagct caccgtggac aagagcaggt ggcagcaggg gaacgtcttc    1440
   tcatgctccg tgatgcatga ggctctgcac aaccactaca cgcagaagag cctctccctg    1500
   tctccgggtg gcggaggctc caggactgtg gctgcaccat ctgtcttcat cttcccgcca    1560
   tctgatgagc agttgaaatc tggaactgcc tctgttgtgt gcctgctgaa ttacttctat    1620
   cccagagagg ccaaagtaca gtggaaggtg gataacgccc tccaatcggg taactcccag    1680
   gagagtgcca cagagcagga cagcaaggac agcacctaca gcctcagcag cgagctgacg    1740
   ctgagcaaag cagactacga gaaacacaaa gtctacgcct gcgaagtcac ccatcagggc    1800
   ctgagctcgc ccgtcacaaa gagcttcaac aggggagagt ga                       1842
<210> 857
   <211> 1848
   <212> DNA
   <213> Artificial Sequence
<220>
   <223> TSC149 (HD37 scFv-hinge-CH2-CH3-SYSPNSR linker-Ck)
<400> 857
   atggaagcac cagcgcagct tctcttcctc ctgctactct ggctcccaga taccaccggt      60
   gacattgtgc tgacccaatc tccagcttct ttggctgtgt ctctagggca gagggccacc     120
   atctcctgca aggccagcca aagtgttgat tatgatggtg atagttattt gaactggtac     180
   caacagattc caggacagcc acccaaactc ctcatctatg atgcatccaa tctagtttct     240
   gggatcccac ccaggtttag tggcagtggg tctgggacag acttcaccct caacatccat     300
   cctgtggaga aggtggatgc tgcaacctat cactgccagc aaagtactga ggatccgtgg     360
   acgttcggtg gaggcaccaa gctggaaatc aaaggtggcg gtggttcggg cggtggtggg     420
   tcgggtggcg gcggagctag ccaggttcag ctgcagcagt ctggggctga gctggtgagg     480
   cctgggtcct cagtgaagat ttcctgcaag gcttctggct atgcattcag tagctactgg     540
   atgaactggg tgaagcagag gcctggacag ggtcttgagt ggattggaca gatttggcct     600
   ggagatggtg atactaacta caatggaaag ttcaagggta aagccactct gactgcagac     660
   gaatcctcca gcacagccta catgcaactc agcagcctag catctgagga ctctgcggtc     720
   tatttctgtg caagacggga gactacgacg gtaggccgtt attactatgc tatggactac     780
   tggggtcaag gaacctcagt caccgtctcc tcgagtgagc ccaaatcttc tgacaaaact     840
   cacacatgcc caccgtgccc agcacctgaa gccgcgggtg caccgtcagt cttcctcttc     900
   cccccaaaac ccaaggacac cctcatgatc tcccggaccc ctgaggtcac atgcgtggtg     960
   gtggacgtga gccacgaaga ccctgaggtc aagttcaact ggtacgtgga cggcgtggag    1020
   gtgcataatg ccaagacaaa gccgcgggag gagcagtaca acagcacgta ccgtgtggtc    1080
   agcgtcctca ccgtcctgca ccaggactgg ctgaatggca aggcgtacgc gtgcgcggtc    1140
   tccaacaaag ccctcccagc ccccatcgag aaaaccatct ccaaagccaa agggcagccc    1200
   cgagaaccac aggtgtacac cctgccccca tcccgggatg agctgaccaa gaaccaggtc    1260
   agcctgacct gcctggtcaa aggcttctat ccaagcgaca tcgccgtgga gtgggagagc    1320
   aatgggcagc cggagaacaa ctacaagacc acgcctcccg tgctggactc cgacggctcc    1380
   ttcttcctct acagcaagct caccgtggac aagagcaggt ggcagcaggg gaacgtcttc    1440
   tcatgctccg tgatgcatga ggctctgcac aaccactaca cgcagaagag cctctccctg    1500
   tctccgggta gttacagccc gaactctagg actgtggctg caccatctgt cttcatcttc    1560
   ccgccatctg atgagcagtt gaaatctgga actgcctctg ttgtgtgcct gctgaattac    1620
   ttctatccca gagaggccaa agtacagtgg aaggtggata acgccctcca atcgggtaac    1680
   tcccaggaga gtgccacaga gcaggacagc aaggacagca cctacagcct cagcagcgag    1740
   ctgacgctga gcaaagcaga ctacgagaaa cacaaagtct acgcctgcga agtcacccat    1800
   cagggcctga gctcgcccgt cacaaagagc ttcaacaggg gagagtga                 1848
<210> 858
   <211> 1854
   <212> DNA
   <213> Artificial Sequence
<220>
   <223> TSC150 (HD37 scFv-hinge-CH2-CH3-SSLNTPNSR linker-Ck)
<400> 858
   atggaagcac cagcgcagct tctcttcctc ctgctactct ggctcccaga taccaccggt      60
   gacattgtgc tgacccaatc tccagcttct ttggctgtgt ctctagggca gagggccacc     120
   atctcctgca aggccagcca aagtgttgat tatgatggtg atagttattt gaactggtac     180
   caacagattc caggacagcc acccaaactc ctcatctatg atgcatccaa tctagtttct     240
   gggatcccac ccaggtttag tggcagtggg tctgggacag acttcaccct caacatccat     300
   cctgtggaga aggtggatgc tgcaacctat cactgccagc aaagtactga ggatccgtgg     360
   acgttcggtg gaggcaccaa gctggaaatc aaaggtggcg gtggttcggg cggtggtggg     420
   tcgggtggcg gcggagctag ccaggttcag ctgcagcagt ctggggctga gctggtgagg     480
   cctgggtcct cagtgaagat ttcctgcaag gcttctggct atgcattcag tagctactgg     540
   atgaactggg tgaagcagag gcctggacag ggtcttgagt ggattggaca gatttggcct     600
   ggagatggtg atactaacta caatggaaag ttcaagggta aagccactct gactgcagac     660
   gaatcctcca gcacagccta catgcaactc agcagcctag catctgagga ctctgcggtc     720
   tatttctgtg caagacggga gactacgacg gtaggccgtt attactatgc tatggactac     780
   tggggtcaag gaacctcagt caccgtctcc tcgagtgagc ccaaatcttc tgacaaaact     840
   cacacatgcc caccgtgccc agcacctgaa gccgcgggtg caccgtcagt cttcctcttc     900
   cccccaaaac ccaaggacac cctcatgatc tcccggaccc ctgaggtcac atgcgtggtg     960
   gtggacgtga gccacgaaga ccctgaggtc aagttcaact ggtacgtgga cggcgtggag    1020
   gtgcataatg ccaagacaaa gccgcgggag gagcagtaca acagcacgta ccgtgtggtc    1080
   agcgtcctca ccgtcctgca ccaggactgg ctgaatggca aggcgtacgc gtgcgcggtc    1140
   tccaacaaag ccctcccagc ccccatcgag aaaaccatct ccaaagccaa agggcagccc    1200
   cgagaaccac aggtgtacac cctgccccca tcccgggatg agctgaccaa gaaccaggtc    1260
   agcctgacct gcctggtcaa aggcttctat ccaagcgaca tcgccgtgga gtgggagagc    1320
   aatgggcagc cggagaacaa ctacaagacc acgcctcccg tgctggactc cgacggctcc    1380
   ttcttcctct acagcaagct caccgtggac aagagcaggt ggcagcaggg gaacgtcttc    1440
   tcatgctccg tgatgcatga ggctctgcac aaccactaca cgcagaagag cctctccctg    1500
   tctccgggtt cttccctgaa tacaccgaac tctaggactg tggctgcacc atctgtcttc    1560
   atcttcccgc catctgatga gcagttgaaa tctggaactg cctctgttgt gtgcctgctg    1620
   aattacttct atcccagaga ggccaaagta cagtggaagg tggataacgc cctccaatcg    1680
   ggtaactccc aggagagtgc cacagagcag gacagcaagg acagcaccta cagcctcagc    1740
   agcgagctga cgctgagcaa agcagactac gagaaacaca aagtctacgc ctgcgaagtc    1800
   acccatcagg gcctgagctc gcccgtcaca aagagcttca acaggggaga gtga          1854
<210> 859
   <211> 581
   <212> PRT
   <213> Artificial Sequence
<220>
   <223> TSC145 (Cris7 scFv-hinge-CH2-CH3-GGGSS linker-CH1)
<400> 859
   Gln Val Gln Leu Val Gln Ser Gly Gly Gly Val Val Gln Pro Gly Arg
   1               5                   10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65 70 75 80
   Leu Gln Met Asp Ser Leu Arg Pro Glu Asp Thr Gly Val Tyr Phe Cys
   85 90 95
   Ala Arg Pro Gln Val His Tyr Asp Tyr Asn Gly Phe Pro Tyr Trp Gly
   100 105 110
   Gln Gly Thr Pro Val Thr Val Ser Ser Gly Gly Gly Gly Ser Gly Gly
   115 120 125
   Gly Gly Ser Gly Gly Gly Gly Ser Ala Gln Asp Ile Gln Met Thr Gln
   130 135 140
   Ser Pro Ser Ser Leu Ser Ala Ser Val Gly Asp Arg Val Thr Met Thr
   145 150 155 160
   Cys Ser Ala Ser Ser Ser Val Ser Tyr Met Asn Trp Tyr Gln Gln Lys
   165 170 175
   Pro Gly Lys Ala Pro Lys Arg Trp Ile Tyr Asp Ser Ser Lys Leu Ala
   180 185 190
   Ser Gly Val Pro Ala Arg Phe Ser Gly Ser Gly Ser Gly Thr Asp Tyr
   195 200 205
   Thr Leu Thr Ile Ser Ser Leu Gln Pro Glu Asp Phe Ala Thr Tyr Tyr
   210 215 220
   Cys Gln Gln Trp Ser Arg Asn Pro Pro Thr Phe Gly Gly Gly Thr Lys
   225 230 235 240
   Leu Gln Ile Thr Arg Ser Ser Glu Pro Lys Ser Ser Asp Lys Thr His
   245 250 255
   Thr Cys Pro Pro Cys Pro Ala Pro Glu Ala Ala Gly Ala Pro Ser Val
   260 265 270
   Phe Leu Phe Pro Pro Lys Pro Lys Asp Thr Leu Met Ile Ser Arg Thr
   275 280 285
   Pro Glu Val Thr Cys Val Val Val Asp Val Ser His Glu Asp Pro Glu
   290 295 300
   Val Lys Phe Asn Trp Tyr Val Asp Gly Val Glu Val His Asn Ala Lys
   305 310 315 320
   Thr Lys Pro Arg Glu Glu Gln Tyr Asn Ser Thr Tyr Arg Val Val Ser
   325 330 335
   Val Leu Thr Val Leu His Gln Asp Trp Leu Asn Gly Lys Ala Tyr Ala
   340 345 350
   Cys Ala Val Ser Asn Lys Ala Leu Pro Ala Pro Ile Glu Lys Thr Ile
   355 360 365
   Ser Lys Ala Lys Gly Gln Pro Arg Glu Pro Gln Val Tyr Thr Leu Pro
   370 375 380
   Pro Ser Arg Asp Glu Leu Thr Lys Asn Gln Val Ser Leu Thr Cys Leu
   385 390 395 400
   Val Lys Gly Phe Tyr Pro Ser Asp Ile Ala Val Glu Trp Glu Ser Asn
   405 410 415
   Gly Gln Pro Glu Asn Asn Tyr Lys Thr Thr Pro Pro Val Leu Asp Ser
   420 425 430
   Asp Gly Ser Phe Phe Leu Tyr Ser Lys Leu Thr Val Asp Lys Ser Arg
   435 440 445
   Trp Gln Gln Gly Asn Val Phe Ser Cys Ser Val Met His Glu Ala Leu
   450 455 460
   His Asn His Tyr Thr Gln Lys Ser Leu Ser Leu Ser Pro Gly Gly Gly
   465 470 475 480
   Gly Ser Ser Ala Ser Thr Lys Gly Pro Ser Val Phe Pro Leu Ala Pro
   485 490 495
   Ser Ser Lys Ser Thr Ser Gly Gly Thr Ala Ala Leu Gly Cys Leu Val
   500 505 510
   Lys Asp Tyr Phe Pro Glu Pro Val Thr Val Ser Trp Asn Ser Gly Ala
   515 520 525
   Leu Thr Ser Gly Val His Thr Phe Pro Ala Val Leu Gln Ser Ser Gly
   530 535 540
   Leu Tyr Ser Leu Ser Ser Val Val Thr Val Pro Ser Ser Ser Leu Gly
   545 550 555 560
   Thr Gln Thr Tyr Ile Cys Asn Val Asn His Lys Pro Ser Asn Thr Lys
   565 570 575
   Val Asp Lys Lys Val
   580
<210> 860
   <211> 582
   <212> PRT
   <213> Artificial Sequence
<220>
   <223> TSC146 (Cris7 scFv-hinge-CH2-CH3-SYSPNS linker-CH1)
<400> 860
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Glu Pro Lys Ser Ser Asp Lys Thr His
                   245                 250                 255
   Thr Cys Pro Pro Cys Pro Ala Pro Glu Ala Ala Gly Ala Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
                   420                   425                   430
   Asp Gly Ser Phe Phe Leu Tyr Ser Lys Leu Thr Val Asp Lys Ser Arg
               435                   440                   445
   Trp Gln Gln Gly Asn Val Phe Ser Cys Ser Val Met His Glu Ala Leu
       450                   455                   460
   His Asn His Tyr Thr Gln Lys Ser Leu Ser Leu Ser Pro Gly Ser Tyr
   465                   470                   475                   480
   Ser Pro Asn Ser Ala Ser Thr Lys Gly Pro Ser Val Phe Pro Leu Ala
                   485                   490                   495
   Pro Ser Ser Lys Ser Thr Ser Gly Gly Thr Ala Ala Leu Gly Cys Leu
               500                   505                   510
   Val Lys Asp Tyr Phe Pro Glu Pro Val Thr Val Ser Trp Asn Ser Gly
           515                   520                   525
   Ala Leu Thr Ser Gly Val His Thr Phe Pro Ala Val Leu Gln Ser Ser
       530                   535                   540
   Gly Leu Tyr Ser Leu Ser Ser Val Val Thr Val Pro Ser Ser Ser Leu
   545                   550                   555                   560
   Gly Thr Gln Thr Tyr Ile Cys Asn Val Asn His Lys Pro Ser Asn Thr
                   565                   570                   575
   Lys Val Asp Lys Lys Val
               580
<210> 861
   <211> 584
   <212> PRT
   <213> Artificial Sequence
<220>
   <223> TSC147 (Cris7 scFv-hinge-CH2-CH3-SSLNTPNS linker-CH1)
<400> 861
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Glu Pro Lys Ser Ser Asp Lys Thr His
                   245                 250                 255
   Thr Cys Pro Pro Cys Pro Ala Pro Glu Ala Ala Gly Ala Pro Ser Val
                   260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Ser Ser
   465                 470                 475                 480
   Leu Asn Thr Pro Asn Ser Ala Ser Thr Lys Gly Pro Ser Val Phe Pro
                   485                 490                 495
   Leu Ala Pro Ser Ser Lys Ser Thr Ser Gly Gly Thr Ala Ala Leu Gly
               500                 505                 510
   Cys Leu Val Lys Asp Tyr Phe Pro Glu Pro Val Thr Val Ser Trp Asn
           515                 520                 525
   Ser Gly Ala Leu Thr Ser Gly Val His Thr Phe Pro Ala Val Leu Gln
       530                 535                 540
   Ser Ser Gly Leu Tyr Ser Leu Ser Ser Val Val Thr Val Pro Ser Ser
   545                 550                 555                 560
   Ser Leu Gly Thr Gln Thr Tyr Ile Cys Asn Val Asn His Lys Pro Ser
                   565                 570                 575
   Asn Thr Lys Val Asp Lys Lys Val
               580
<210> 862
   <211> 593
   <212> PRT
   <213> Artificial Sequence
<220>
   <223> TSC148 (HD37 scFv-hinge-CH2-CH3-GGGSR linker-Ck)
<400> 862
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
   115 120 125
   Val Gln Leu Gln Gln Ser Gly Ala Glu Leu Val Arg Pro Gly Ser Ser
   130 135 140
   Val Lys Ile Ser Cys Lys Ala Ser Gly Tyr Ala Phe Ser Ser Tyr Trp
   145 150 155 160
   Met Asn Trp Val Lys Gln Arg Pro Gly Gln Gly Leu Glu Trp Ile Gly
   165 170 175
   Gln Ile Trp Pro Gly Asp Gly Asp Thr Asn Tyr Asn Gly Lys Phe Lys
   180 185 190
   Gly Lys Ala Thr Leu Thr Ala Asp Glu Ser Ser Ser Thr Ala Tyr Met
   195 200 205
   Gln Leu Ser Ser Leu Ala Ser Glu Asp Ser Ala Val Tyr Phe Cys Ala
   210 215 220
   Arg Arg Glu Thr Thr Thr Val Gly Arg Tyr Tyr Tyr Ala Met Asp Tyr
   225 230 235 240
   Trp Gly Gln Gly Thr Ser Val Thr Val Ser Ser Ser Glu Pro Lys Ser
   245 250 255
   Ser Asp Lys Thr His Thr Cys Pro Pro Cys Pro Ala Pro Glu Ala Ala
   260 265 270
   Gly Ala Pro Ser Val Phe Leu Phe Pro Pro Lys Pro Lys Asp Thr Leu
   275 280 285
   Met Ile Ser Arg Thr Pro Glu Val Thr Cys Val Val Val Asp Val Ser
   290 295 300
   His Glu Asp Pro Glu Val Lys Phe Asn Trp Tyr Val Asp Gly Val Glu
   305 310 315 320
   Val His Asn Ala Lys Thr Lys Pro Arg Glu Glu Gln Tyr Asn Ser Thr
   325 330 335
   Tyr Arg Val Val Ser Val Leu Thr Val Leu His Gln Asp Trp Leu Asn
   340 345 350
   Gly Lys Ala Tyr Ala Cys Ala Val Ser Asn Lys Ala Leu Pro Ala Pro
   355 360 365
   Ile Glu Lys Thr Ile Ser Lys Ala Lys Gly Gln Pro Arg Glu Pro Gln
   370 375 380
   Val Tyr Thr Leu Pro Pro Ser Arg Asp Glu Leu Thr Lys Asn Gln Val
   385 390 395 400
   Ser Leu Thr Cys Leu Val Lys Gly Phe Tyr Pro Ser Asp Ile Ala Val
   405 410 415
   Glu Trp Glu Ser Asn Gly Gln Pro Glu Asn Asn Tyr Lys Thr Thr Pro
   420 425 430
   Pro Val Leu Asp Ser Asp Gly Ser Phe Phe Leu Tyr Ser Lys Leu Thr
   435 440 445
   Val Asp Lys Ser Arg Trp Gln Gln Gly Asn Val Phe Ser Cys Ser Val
   450 455 460
   Met His Glu Ala Leu His Asn His Tyr Thr Gln Lys Ser Leu Ser Leu
   465 470 475 480
   Ser Pro Gly Gly Gly Gly Ser Arg Thr Val Ala Ala Pro Ser Val Phe
   485 490 495
   Ile Phe Pro Pro Ser Asp Glu Gln Leu Lys Ser Gly Thr Ala Ser Val
   500 505 510
   Val Cys Leu Leu Asn Tyr Phe Tyr Pro Arg Glu Ala Lys Val Gln Trp
   515 520 525
   Lys Val Asp Asn Ala Leu Gln Ser Gly Asn Ser Gln Glu Ser Ala Thr
   530 535 540
   Glu Gln Asp Ser Lys Asp Ser Thr Tyr Ser Leu Ser Ser Glu Leu Thr
   545 550 555 560
   Leu Ser Lys Ala Asp Tyr Glu Lys His Lys Val Tyr Ala Cys Glu Val
   565 570 575
   Thr His Gln Gly Leu Ser Ser Pro Val Thr Lys Ser Phe Asn Arg Gly
   580 585 590
   Glu
<210> 863
   <211> 595
   <212> PRT
   <213> Artificial Sequence
<220>
   <223> TSC149 (HD37 scFv-hinge-CH2-CH3-SYSPNSR linker-Ck)
<400> 863
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Cys Pro Pro Cys Pro Ala Pro Glu Ala Ala
               260                 265                 270
   Gly Ala Pro Ser Val Phe Leu Phe Pro Pro Lys Pro Lys Asp Thr Leu
           275                 280                 285
   Met Ile Ser Arg Thr Pro Glu Val Thr Cys Val Val Val Asp Val Ser
       290                 295                 300
   His Glu Asp Pro Glu Val Lys Phe Asn Trp Tyr Val Asp Gly Val Glu
   305                 310                 315                 320
   Val His Asn Ala Lys Thr Lys Pro Arg Glu Glu Gln Tyr Asn Ser Thr
                   325                 330                 335
   Tyr Arg Val Val Ser Val Leu Thr Val Leu His Gln Asp Trp Leu Asn
               340                 345                 350
   Gly Lys Ala Tyr Ala Cys Ala Val Ser Asn Lys Ala Leu Pro Ala Pro
           355                 360                 365
   Ile Glu Lys Thr Ile Ser Lys Ala Lys Gly Gln Pro Arg Glu Pro Gln
       370                 375                 380
   Val Tyr Thr Leu Pro Pro Ser Arg Asp Glu Leu Thr Lys Asn Gln Val
   385                 390                 395                 400
   Ser Leu Thr Cys Leu Val Lys Gly Phe Tyr Pro Ser Asp Ile Ala Val
                   405                 410                 415
   Glu Trp Glu Ser Asn Gly Gln Pro Glu Asn Asn Tyr Lys Thr Thr Pro
               420                 425                 430
   Pro Val Leu Asp Ser Asp Gly Ser Phe Phe Leu Tyr Ser Lys Leu Thr
           435                 440                 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465 470 475 480
   Ser Pro Gly Ser Tyr Ser Pro Asn Ser Arg Thr Val Ala Ala Pro Ser
   485 490 495
   Val Phe Ile Phe Pro Pro Ser Asp Glu Gln Leu Lys Ser Gly Thr Ala
   500 505 510
   Ser Val Val Cys Leu Leu Asn Tyr Phe Tyr Pro Arg Glu Ala Lys Val
   515 520 525
   Gln Trp Lys Val Asp Asn Ala Leu Gln Ser Gly Asn Ser Gln Glu Ser
   530 535 540
   Ala Thr Glu Gln Asp Ser Lys Asp Ser Thr Tyr Ser Leu Ser Ser Glu
   545 550 555 560
   Leu Thr Leu Ser Lys Ala Asp Tyr Glu Lys His Lys Val Tyr Ala Cys
   565 570 575
   Glu Val Thr His Gln Gly Leu Ser Ser Pro Val Thr Lys Ser Phe Asn
   580 585 590
   Arg Gly Glu
   595
<210> 864
   <211> 597
   <212> PRT
   <213> Artificial Sequence
<220>
   <223> TSC150 (HD37 scFv-hinge-CH2-CH3-SSLNTPNSR linker-Ck)
<400> 864
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Cys Pro Pro Cys Pro Ala Pro Glu Ala Ala
               260                 265                 270
   Gly Ala Pro Ser Val Phe Leu Phe Pro Pro Lys Pro Lys Asp Thr Leu
           275                 280                 285
   Met Ile Ser Arg Thr Pro Glu Val Thr Cys Val Val Val Asp Val Ser
       290                 295                 300
   His Glu Asp Pro Glu Val Lys Phe Asn Trp Tyr Val Asp Gly Val Glu
   305                 310                 315                 320
   Val His Asn Ala Lys Thr Lys Pro Arg Glu Glu Gln Tyr Asn Ser Thr
                   325                 330                 335
   Tyr Arg Val Val Ser Val Leu Thr Val Leu His Gln Asp Trp Leu Asn
               340                 345                 350
   Gly Lys Ala Tyr Ala Cys Ala Val Ser Asn Lys Ala Leu Pro Ala Pro
           355                 360                 365
   Ile Glu Lys Thr Ile Ser Lys Ala Lys Gly Gln Pro Arg Glu Pro Gln
       370                 375                 380
   Val Tyr Thr Leu Pro Pro Ser Arg Asp Glu Leu Thr Lys Asn Gln Val
   385                 390                 395                 400
   Ser Leu Thr Cys Leu Val Lys Gly Phe Tyr Pro Ser Asp Ile Ala Val
                   405                 410                 415
   Glu Trp Glu Ser Asn Gly Gln Pro Glu Asn Asn Tyr Lys Thr Thr Pro
               420                 425                 430
   Pro Val Leu Asp Ser Asp Gly Ser Phe Phe Leu Tyr Ser Lys Leu Thr
           435                 440                 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465                 470                 475                 480
   Ser Pro Gly Ser Ser Leu Asn Thr Pro Asn Ser Arg Thr Val Ala Ala
                   485                 490                 495
   Pro Ser Val Phe Ile Phe Pro Pro Ser Asp Glu Gln Leu Lys Ser Gly
               500                 505                 510
   Thr Ala Ser Val Val Cys Leu Leu Asn Tyr Phe Tyr Pro Arg Glu Ala
           515                 520                 525
   Lys Val Gln Trp Lys Val Asp Asn Ala Leu Gln Ser Gly Asn Ser Gln
       530                 535                 540
   Glu Ser Ala Thr Glu Gln Asp Ser Lys Asp Ser Thr Tyr Ser Leu Ser
   545                 550                 555                 560
   Ser Glu Leu Thr Leu Ser Lys Ala Asp Tyr Glu Lys His Lys Val Tyr
                   565                 570                 575
   Ala Cys Glu Val Thr His Gln Gly Leu Ser Ser Pro Val Thr Lys Ser
               580                 585                 590
   Phe Asn Arg Gly Glu
           595
<210> 865
   <211> 1800
   <212> DNA
   <213> Artificial Sequence
<220>
   <223> TSC125 (Cris7 scFv-CH2-CH3-CH1)
<400> 865
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag tgagcccaaa tcttctgaca aaactcacac atgcccaccg     840
   tgcccagcac ctgaagccgc gggtgcaccg tcagtcttcc tcttcccccc aaaacccaag     900
   gacaccctca tgatctcccg gacccctgag gtcacatgcg tggtggtgga cgtgagccac     960
   gaagaccctg aggtcaagtt caactggtac gtggacggcg tggaggtgca taatgccaag    1020
   acaaagccgc gggaggagca gtacaacagc acgtaccgtg tggtcagcgt cctcaccgtc    1080
   ctgcaccagg actggctgaa tggcaaggcg tacgcgtgcg cggtctccaa caaagccctc    1140
   ccagccccca tcgagaaaac catctccaaa gccaaagggc agccccgaga accacaggtg    1200
   tacaccctgc ccccatcccg ggatgagctg accaagaacc aggtcagcct gacctgcctg    1260
   gtcaaaggct tctatccaag cgacatcgcc gtggagtggg agagcaatgg gcagccggag    1320
   aacaactaca agaccacgcc tcccgtgctg gactccgacg gctccttctt cctctacagc    1380
   aagctcaccg tggacaagag caggtggcag caggggaacg tcttctcatg ctccgtgatg    1440
   catgaggctc tgcacaacca ctacacgcag aagagcctct ccctgtctcc gggtaaatct    1500
   agagcctcca ccaagggccc atcggtcttc cccctggcac cctcctccaa gagcacctct    1560
   gggggcacag cggccctggg ctgcctggtc aaggactact tccccgagcc ggtgacggtg    1620
   tcgtggaact caggcgccct gaccagcggc gtgcacacct tcccggctgt cctacagtcc    1680
   tcaggactct actccctcag cagcgtggtg accgtgccct ccagcagctt gggcacccag    1740
   acctacatct gcaacgtgaa tcacaagccc agcaacacca aggtggacaa gaaagtttga    1800
<210> 866
   <211> 1785
   <212> DNA
   <213> Artificial Sequence
<220>
   <223> TSC157 (Cris7 scFv-IgA2UH-CH2-CH3-CH1)
<400> 866
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag ccccccacct cccccatgcc caccgtgccc agcacctgaa     840
   gccgcgggtg caccgtcagt cttcctcttc cccccaaaac ccaaggacac cctcatgatc     900
   tcccggaccc ctgaggtcac atgcgtggtg gtggacgtga gccacgaaga ccctgaggtc     960
   aagttcaact ggtacgtgga cggcgtggag gtgcataatg ccaagacaaa gccgcgggag    1020
   gagcagtaca acagcacgta ccgtgtggtc agcgtcctca ccgtcctgca ccaggactgg    1080
   ctgaatggca aggcgtacgc gtgcgcggtc tccaacaaag ccctcccagc ccccatcgag    1140
   aaaaccatct ccaaagccaa agggcagccc cgagaaccac aggtgtacac cctgccccca    1200
   tcccgggatg agctgaccaa gaaccaggtc agcctgacct gcctggtcaa aggcttctat    1260
   ccaagcgaca tcgccgtgga gtgggagagc aatgggcagc cggagaacaa ctacaagacc    1320
   acgcctcccg tgctggactc cgacggctcc ttcttcctct acagcaagct caccgtggac    1380
   aagagcaggt ggcagcaggg gaacgtcttc tcatgctccg tgatgcatga ggctctgcac    1440
   aaccactaca cgcagaagag cctctccctg tctccgggta aatctagagc ctccaccaag    1500
   ggcccatcgg tcttccccct ggcaccctcc tccaagagca cctctggggg cacagcggcc    1560
   ctgggctgcc tggtcaagga ctacttcccc gagccggtga cggtgtcgtg gaactcaggc    1620
   gccctgacca gcggcgtgca caccttcccg gctgtcctac agtcctcagg actctactcc    1680
   ctcagcagcg tggtgaccgt gccctccagc agcttgggca cccagaccta catctgcaac    1740
   gtgaatcaca agcccagcaa caccaaggtg gacaagaaag tttga                    1785
<210> 867
   <211> 1815
   <212> DNA
   <213> Artificial Sequence
<220>
   <223> TSC158 (Cris7 scFv-IgG1miniUH-CH2-CH3-CH1)
<400> 867
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag cgagcccaaa tcttctgaca aaactcacac aagcccaccg     840
   agcccatgcc caccgtgccc agcacctgaa gccgcgggtg caccgtcagt cttcctcttc     900
   cccccaaaac ccaaggacac cctcatgatc tcccggaccc ctgaggtcac atgcgtggtg     960
   gtggacgtga gccacgaaga ccctgaggtc aagttcaact ggtacgtgga cggcgtggag    1020
   gtgcataatg ccaagacaaa gccgcgggag gagcagtaca acagcacgta ccgtgtggtc    1080
   agcgtcctca ccgtcctgca ccaggactgg ctgaatggca aggcgtacgc gtgcgcggtc    1140
   tccaacaaag ccctcccagc ccccatcgag aaaaccatct ccaaagccaa agggcagccc    1200
   cgagaaccac aggtgtacac cctgccccca tcccgggatg agctgaccaa gaaccaggtc    1260
   agcctgacct gcctggtcaa aggcttctat ccaagcgaca tcgccgtgga gtgggagagc    1320
   aatgggcagc cggagaacaa ctacaagacc acgcctcccg tgctggactc cgacggctcc    1380
   ttcttcctct acagcaagct caccgtggac aagagcaggt ggcagcaggg gaacgtcttc    1440
   tcatgctccg tgatgcatga ggctctgcac aaccactaca cgcagaagag cctctccctg    1500
   tctccgggta aatctagagc ctccaccaag ggcccatcgg tcttccccct ggcaccctcc    1560
   tccaagagca cctctggggg cacagcggcc ctgggctgcc tggtcaagga ctacttcccc    1620
   gagccggtga cggtgtcgtg gaactcaggc gccctgacca gcggcgtgca caccttcccg    1680
   gctgtcctac agtcctcagg actctactcc ctcagcagcg tggtgaccgt gccctccagc    1740
   agcttgggca cccagaccta catctgcaac gtgaatcaca agcccagcaa caccaaggtg    1800
   gacaagaaag tttga                                                     1815
<210> 868
   <211> 1815
   <212> DNA
   <213> Artificial Sequence
<220>
   <223> TSC159 (Cris7 scFv-IgG3UH-CH2-CH3-CH1)
<400> 868
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag cgagcccaaa tctagcgaca cacctccccc aagcccacgg     840
   tccccatgcc caccgtgccc agcacctgaa gccgcgggtg caccgtcagt cttcctcttc     900
   cccccaaaac ccaaggacac cctcatgatc tcccggaccc ctgaggtcac atgcgtggtg     960
   gtggacgtga gccacgaaga ccctgaggtc aagttcaact ggtacgtgga cggcgtggag    1020
   gtgcataatg ccaagacaaa gccgcgggag gagcagtaca acagcacgta ccgtgtggtc    1080
   agcgtcctca ccgtcctgca ccaggactgg ctgaatggca aggcgtacgc gtgcgcggtc    1140
   tccaacaaag ccctcccagc ccccatcgag aaaaccatct ccaaagccaa agggcagccc    1200
   cgagaaccac aggtgtacac cctgccccca tcccgggatg agctgaccaa gaaccaggtc    1260
   agcctgacct gcctggtcaa aggcttctat ccaagcgaca tcgccgtgga gtgggagagc    1320
   aatgggcagc cggagaacaa ctacaagacc acgcctcccg tgctggactc cgacggctcc    1380
   ttcttcctct acagcaagct caccgtggac aagagcaggt ggcagcaggg gaacgtcttc    1440
   tcatgctccg tgatgcatga ggctctgcac aaccactaca cgcagaagag cctctccctg    1500
   tctccgggta aatctagagc ctccaccaag ggcccatcgg tcttccccct ggcaccctcc     1560
   tccaagagca cctctggggg cacagcggcc ctgggctgcc tggtcaagga ctacttcccc    1620
   gagccggtga cggtgtcgtg gaactcaggc gccctgacca gcggcgtgca caccttcccg    1680
   gctgtcctac agtcctcagg actctactcc ctcagcagcg tggtgaccgt gccctccagc    1740
   agcttgggca cccagaccta catctgcaac gtgaatcaca agcccagcaa caccaaggtg    1800
   gacaagaaag tttga                                                     1815
<210> 869
   <211> 2103
   <212> DNA
   <213> Artificial Sequence
<220>
   <223> TSC160 (Cris7 scFv-IgMCH2UH-CH2-CH3-CH1)
<400> 869
   atggaagcac cagcgcagct tctcttcctc ctgctactct ggctcccaga taccaccggt      60
   caggtccagc tggtgcagtc tgggggcgga gtggtgcagc ctgggcggtc actgaggctg     120
   tcctgcaagg cttctggcta cacctttact agatctacga tgcactgggt aaggcaggcc     180
   cctggaaagg gtctggaatg gattggatac attaatccta gcagtgctta tactaattac     240
   aatcagaaat tcaaggacag gttcacaatc agcgcagaca aatccaagag cacagccttc     300
   ctgcagatgg acagcctgag gcccgaggac accggcgtct atttctgtgc acggccccaa     360
   gtccactatg attacaacgg gtttccttac tggggccaag ggactcccgt cactgtctct     420
   agcggtggcg gagggtctgg gggtggcgga tccggaggtg gtggctctgc acaagacatc     480
   cagatgaccc agtctccaag cagcctgtct gcaagcgtgg gggacagggt caccatgacc     540
   tgcagtgcca gctcaagtgt aagttacatg aactggtacc agcagaagcc cggcaaggcc     600
   cccaaaagat ggatttatga ctcatccaaa ctggcttctg gagtccctgc tcgcttcagt     660
   ggcagtgggt ctgggaccga ctataccctc acaatcagca gcctgcagcc cgaagatttc     720
   gccacttatt actgccagca gtggagtcgt aacccaccca cgttcggagg ggggaccaag     780
   ctacaaatta cacgctcgag cattgccgag ctgcctccca aagtgagcgt cttcgtccca     840
   ccccgcgacg gcttcttcgg caacccccgc aagtccaagc tcatctgcca ggccacgggt     900
   ttcagtcccc ggcagattca ggtgtcctgg ctgcgcgagg ggaagcaggt ggggtctggc     960
   gtcaccacgg accaggtgca ggctgaggcc aaagagtctg ggcccacgac ctacaaggtg    1020
   accagcacac tgaccatcaa agagagcgac tggctcagcc agagcatgtt cacctgccgc    1080
   gtggatcaca ggggcctgac cttccagcag aatgcgtcct ccatgagtgt cccctgccca    1140
   ccgtgcccag cacctgaagc cgcgggtgca ccgtcagtct tcctcttccc cccaaaaccc    1200
   aaggacaccc tcatgatctc ccggacccct gaggtcacat gcgtggtggt ggacgtgagc    1260
   cacgaagacc ctgaggtcaa gttcaactgg tacgtggacg gcgtggaggt gcataatgcc    1320
   aagacaaagc cgcgggagga gcagtacaac agcacgtacc gtgtggtcag cgtcctcacc    1380
   gtcctgcacc aggactggct gaatggcaag gcgtacgcgt gcgcggtctc caacaaagcc    1440
   ctcccagccc ccatcgagaa aaccatctcc aaagccaaag ggcagccccg agaaccacag    1500
   gtgtacaccc tgcccccatc ccgggatgag ctgaccaaga accaggtcag cctgacctgc    1560
   ctggtcaaag gcttctatcc aagcgacatc gccgtggagt gggagagcaa tgggcagccg    1620
   gagaacaact acaagaccac gcctcccgtg ctggactccg acggctcctt cttcctctac    1680
   agcaagctca ccgtggacaa gagcaggtgg cagcagggga acgtcttctc atgctccgtg    1740
   atgcatgagg ctctgcacaa ccactacacg cagaagagcc tctccctgtc tccgggtaaa    1800
   tctagagcct ccaccaaggg cccatcggtc ttccccctgg caccctcctc caagagcacc    1860
   tctgggggca cagcggccct gggctgcctg gtcaaggact acttccccga gccggtgacg    1920
   gtgtcgtgga actcaggcgc cctgaccagc ggcgtgcaca ccttcccggc tgtcctacag    1980
   tcctcaggac tctactccct cagcagcgtg gtgaccgtgc cctccagcag cttgggcacc    2040
   cagacctaca tctgcaacgt gaatcacaag cccagcaaca ccaaggtgga caagaaagtt    2100
   tga                                                                  2103
<210> 870
   <211> 1821
   <212> DNA
   <213> Artificial Sequence
<220>
   <223> TSC161 (HD37 scFv-IgA2UH-CH2-CH3-Ck)
<400> 870
   atggaagcac cagcgcagct tctcttcctc ctgctactct ggctcccaga taccaccggt      60
   gacattgtgc tgacccaatc tccagcttct ttggctgtgt ctctagggca gagggccacc     120
   atctcctgca aggccagcca aagtgttgat tatgatggtg atagttattt gaactggtac      180
   caacagattc caggacagcc acccaaactc ctcatctatg atgcatccaa tctagtttct      240
   gggatcccac ccaggtttag tggcagtggg tctgggacag acttcaccct caacatccat      300
   cctgtggaga aggtggatgc tgcaacctat cactgccagc aaagtactga ggatccgtgg      360
   acgttcggtg gaggcaccaa gctggaaatc aaaggtggcg gtggttcggg cggtggtggg      420
   tcgggtggcg gcggagctag ccaggttcag ctgcagcagt ctggggctga gctggtgagg      480
   cctgggtcct cagtgaagat ttcctgcaag gcttctggct atgcattcag tagctactgg      540
   atgaactggg tgaagcagag gcctggacag ggtcttgagt ggattggaca gatttggcct      600
   ggagatggtg atactaacta caatggaaag ttcaagggta aagccactct gactgcagac      660
   gaatcctcca gcacagccta catgcaactc agcagcctag catctgagga ctctgcggtc      720
   tatttctgtg caagacggga gactacgacg gtaggccgtt attactatgc tatggactac      780
   tggggtcaag gaacctcagt caccgtctcc tcgagccccc cacctccccc atgcccaccg      840
   tgcccagcac ctgaagccgc gggtgcaccg tcagtcttcc tcttcccccc aaaacccaag      900
   gacaccctca tgatctcccg gacccctgag gtcacatgcg tggtggtgga cgtgagccac      960
   gaagaccctg aggtcaagtt caactggtac gtggacggcg tggaggtgca taatgccaag     1020
   acaaagccgc gggaggagca gtacaacagc acgtaccgtg tggtcagcgt cctcaccgtc     1080
   ctgcaccagg actggctgaa tggcaaggcg tacgcgtgcg cggtctccaa caaagccctc     1140
   ccagccccca tcgagaaaac catctccaaa gccaaagggc agccccgaga accacaggtg     1200
   tacaccctgc ccccatcccg ggatgagctg accaagaacc aggtcagcct gacctgcctg     1260
   gtcaaaggct tctatccaag cgacatcgcc gtggagtggg agagcaatgg gcagccggag     1320
   aacaactaca agaccacgcc tcccgtgctg gactccgacg gctccttctt cctctacagc     1380
   aagctcaccg tggacaagag caggtggcag caggggaacg tcttctcatg ctccgtgatg     1440
   catgaggctc tgcacaacca ctacacgcag aagagcctct ccctgtctcc gggtaaatct     1500
   agaactgtgg ctgcaccatc tgtcttcatc ttcccgccat ctgatgagca gttgaaatct     1560
   ggaactgcct ctgttgtgtg cctgctgaat tacttctatc ccagagaggc caaagtacag     1620
   tggaaggtgg ataacgccct ccaatcgggt aactcccagg agagtgccac agagcaggac     1680
   agcaaggaca gcacctacag cctcagcagc gagctgacgc tgagcaaagc agactacgag     1740
   aaacacaaag tctacgcctg cgaagtcacc catcagggcc tgagctcgcc cgtcacaaag     1800
   agcttcaaca ggggagagtg a                                               1821
<210> 871
   <211> 1851
   <212> DNA
   <213> Artificial Sequence
<220>
   <223> TSC162 (HD37 scFv-IgG1miniUH-CH2-CH3-Ck)
<400> 871
   atggaagcac cagcgcagct tctcttcctc ctgctactct ggctcccaga taccaccggt      60
   gacattgtgc tgacccaatc tccagcttct ttggctgtgt ctctagggca gagggccacc     120
   atctcctgca aggccagcca aagtgttgat tatgatggtg atagttattt gaactggtac     180
   caacagattc caggacagcc acccaaactc ctcatctatg atgcatccaa tctagtttct     240
   gggatcccac ccaggtttag tggcagtggg tctgggacag acttcaccct caacatccat     300
   cctgtggaga aggtggatgc tgcaacctat cactgccagc aaagtactga ggatccgtgg     360
   acgttcggtg gaggcaccaa gctggaaatc aaaggtggcg gtggttcggg cggtggtggg     420
   tcgggtggcg gcggagctag ccaggttcag ctgcagcagt ctggggctga gctggtgagg     480
   cctgggtcct cagtgaagat ttcctgcaag gcttctggct atgcattcag tagctactgg     540
   atgaactggg tgaagcagag gcctggacag ggtcttgagt ggattggaca gatttggcct     600
   ggagatggtg atactaacta caatggaaag ttcaagggta aagccactct gactgcagac     660
   gaatcctcca gcacagccta catgcaactc agcagcctag catctgagga ctctgcggtc     720
   tatttctgtg caagacggga gactacgacg gtaggccgtt attactatgc tatggactac     780
   tggggtcaag gaacctcagt caccgtctcc tcgagcgagc ccaaatcttc tgacaaaact     840
   cacacaagcc caccgagccc atgcccaccg tgcccagcac ctgaagccgc gggtgcaccg     900
   tcagtcttcc tcttcccccc aaaacccaag gacaccctca tgatctcccg gacccctgag     960
   gtcacatgcg tggtggtgga cgtgagccac gaagaccctg aggtcaagtt caactggtac    1020
   gtggacggcg tggaggtgca taatgccaag acaaagccgc gggaggagca gtacaacagc    1080
   acgtaccgtg tggtcagcgt cctcaccgtc ctgcaccagg actggctgaa tggcaaggcg    1140
   tacgcgtgcg cggtctccaa caaagccctc ccagccccca tcgagaaaac catctccaaa    1200
   gccaaagggc agccccgaga accacaggtg tacaccctgc ccccatcccg ggatgagctg    1260
   accaagaacc aggtcagcct gacctgcctg gtcaaaggct tctatccaag cgacatcgcc    1320
   gtggagtggg agagcaatgg gcagccggag aacaactaca agaccacgcc tcccgtgctg    1380
   gactccgacg gctccttctt cctctacagc aagctcaccg tggacaagag caggtggcag    1440
   caggggaacg tcttctcatg ctccgtgatg catgaggctc tgcacaacca ctacacgcag    1500
   aagagcctct ccctgtctcc gggtaaatct agaactgtgg ctgcaccatc tgtcttcatc    1560
   ttcccgccat ctgatgagca gttgaaatct ggaactgcct ctgttgtgtg cctgctgaat    1620
   tacttctatc ccagagaggc caaagtacag tggaaggtgg ataacgccct ccaatcgggt    1680
   aactcccagg agagtgccac agagcaggac agcaaggaca gcacctacag cctcagcagc    1740
   gagctgacgc tgagcaaagc agactacgag aaacacaaag tctacgcctg cgaagtcacc    1800
   catcagggcc tgagctcgcc cgtcacaaag agcttcaaca ggggagagtg a             1851
<210> 872
   <211> 1851
   <212> DNA
   <213> Artificial Sequence
<220>
   <223> TSC163 (HD37 scFv-IgG3UH-CH2-CH3-Ck)
<400> 872
   atggaagcac cagcgcagct tctcttcctc ctgctactct ggctcccaga taccaccggt     60
   gacattgtgc tgacccaatc tccagcttct ttggctgtgt ctctagggca gagggccacc    120
   atctcctgca aggccagcca aagtgttgat tatgatggtg atagttattt gaactggtac    180
   caacagattc caggacagcc acccaaactc ctcatctatg atgcatccaa tctagtttct    240
   gggatcccac ccaggtttag tggcagtggg tctgggacag acttcaccct caacatccat    300
   cctgtggaga aggtggatgc tgcaacctat cactgccagc aaagtactga ggatccgtgg    360
   acgttcggtg gaggcaccaa gctggaaatc aaaggtggcg gtggttcggg cggtggtggg    420
   tcgggtggcg gcggagctag ccaggttcag ctgcagcagt ctggggctga gctggtgagg    480
   cctgggtcct cagtgaagat ttcctgcaag gcttctggct atgcattcag tagctactgg    540
   atgaactggg tgaagcagag gcctggacag ggtcttgagt ggattggaca gatttggcct    600
   ggagatggtg atactaacta caatggaaag ttcaagggta aagccactct gactgcagac    660
   gaatcctcca gcacagccta catgcaactc agcagcctag catctgagga ctctgcggtc    720
   tatttctgtg caagacggga gactacgacg gtaggccgtt attactatgc tatggactac    780
   tggggtcaag gaacctcagt caccgtctcc tcgagcgagc ccaaatctag cgacacacct    840
   cccccaagcc cacggtcccc atgcccaccg tgcccagcac ctgaagccgc gggtgcaccg    900
   tcagtcttcc tcttcccccc aaaacccaag gacaccctca tgatctcccg gacccctgag    960
   gtcacatgcg tggtggtgga cgtgagccac gaagaccctg aggtcaagtt caactggtac   1020
   gtggacggcg tggaggtgca taatgccaag acaaagccgc gggaggagca gtacaacagc   1080
   acgtaccgtg tggtcagcgt cctcaccgtc ctgcaccagg actggctgaa tggcaaggcg   1140
   tacgcgtgcg cggtctccaa caaagccctc ccagccccca tcgagaaaac catctccaaa   1200
   gccaaagggc agccccgaga accacaggtg tacaccctgc ccccatcccg ggatgagctg   1260
   accaagaacc aggtcagcct gacctgcctg gtcaaaggct tctatccaag cgacatcgcc   1320
   gtggagtggg agagcaatgg gcagccggag aacaactaca agaccacgcc tcccgtgctg   1380
   gactccgacg gctccttctt cctctacagc aagctcaccg tggacaagag caggtggcag   1440
   caggggaacg tcttctcatg ctccgtgatg catgaggctc tgcacaacca ctacacgcag   1500
   aagagcctct ccctgtctcc gggtaaatct agaactgtgg ctgcaccatc tgtcttcatc   1560
   ttcccgccat ctgatgagca gttgaaatct ggaactgcct ctgttgtgtg cctgctgaat   1620
   tacttctatc ccagagaggc caaagtacag tggaaggtgg ataacgccct ccaatcgggt   1680
   aactcccagg agagtgccac agagcaggac agcaaggaca gcacctacag cctcagcagc   1740
   gagctgacgc tgagcaaagc agactacgag aaacacaaag tctacgcctg cgaagtcacc   1800
   catcagggcc tgagctcgcc cgtcacaaag agcttcaaca ggggagagtg a             1851
<210> 873
   <211> 2139
   <212> DNA
   <213> Artificial Sequence
<220>
   <223> TSC164 (HD37 scFv-IgMCH2UH-CH2-CH3-Ck)
<400> 873
   atggaagcac cagcgcagct tctcttcctc ctgctactct ggctcccaga taccaccggt     60
   gacattgtgc tgacccaatc tccagcttct ttggctgtgt ctctagggca gagggccacc    120
   atctcctgca aggccagcca aagtgttgat tatgatggtg atagttattt gaactggtac    180
   caacagattc caggacagcc acccaaactc ctcatctatg atgcatccaa tctagtttct    240
   gggatcccac ccaggtttag tggcagtggg tctgggacag acttcaccct caacatccat    300
   cctgtggaga aggtggatgc tgcaacctat cactgccagc aaagtactga ggatccgtgg    360
   acgttcggtg gaggcaccaa gctggaaatc aaaggtggcg gtggttcggg cggtggtggg    420
   tcgggtggcg gcggagctag ccaggttcag ctgcagcagt ctggggctga gctggtgagg    480
   cctgggtcct cagtgaagat ttcctgcaag gcttctggct atgcattcag tagctactgg    540
   atgaactggg tgaagcagag gcctggacag ggtcttgagt ggattggaca gatttggcct 600
   ggagatggtg atactaacta caatggaaag ttcaagggta aagccactct gactgcagac 660
   gaatcctcca gcacagccta catgcaactc agcagcctag catctgagga ctctgcggtc 720
   tatttctgtg caagacggga gactacgacg gtaggccgtt attactatgc tatggactac 780
   tggggtcaag gaacctcagt caccgtctcc tcgagcattg ccgagctgcc tcccaaagtg 840
   agcgtcttcg tcccaccccg cgacggcttc ttcggcaacc cccgcaagtc caagctcatc 900
   tgccaggcca cgggtttcag tccccggcag attcaggtgt cctggctgcg cgaggggaag 960
   caggtggggt ctggcgtcac cacggaccag gtgcaggctg aggccaaaga gtctgggccc 1020
   acgacctaca aggtgaccag cacactgacc atcaaagaga gcgactggct cagccagagc 1080
   atgttcacct gccgcgtgga tcacaggggc ctgaccttcc agcagaatgc gtcctccatg 1140
   agtgtcccct gcccaccgtg cccagcacct gaagccgcgg gtgcaccgtc agtcttcctc 1200
   ttccccccaa aacccaagga caccctcatg atctcccgga cccctgaggt cacatgcgtg 1260
   gtggtggacg tgagccacga agaccctgag gtcaagttca actggtacgt ggacggcgtg 1320
   gaggtgcata atgccaagac aaagccgcgg gaggagcagt acaacagcac gtaccgtgtg 1380
   gtcagcgtcc tcaccgtcct gcaccaggac tggctgaatg gcaaggcgta cgcgtgcgcg 1440
   gtctccaaca aagccctccc agcccccatc gagaaaacca tctccaaagc caaagggcag 1500
   ccccgagaac cacaggtgta caccctgccc ccatcccggg atgagctgac caagaaccag 1560
   gtcagcctga cctgcctggt caaaggcttc tatccaagcg acatcgccgt ggagtgggag 1620
   agcaatgggc agccggagaa caactacaag accacgcctc ccgtgctgga ctccgacggc 1680
   tccttcttcc tctacagcaa gctcaccgtg gacaagagca ggtggcagca ggggaacgtc 1740
   ttctcatgct ccgtgatgca tgaggctctg cacaaccact acacgcagaa gagcctctcc 1800
   ctgtctccgg gtaaatctag aactgtggct gcaccatctg tcttcatctt cccgccatct 1860
   gatgagcagt tgaaatctgg aactgcctct gttgtgtgcc tgctgaatta cttctatccc 1920
   agagaggcca aagtacagtg gaaggtggat aacgccctcc aatcgggtaa ctcccaggag 1980
   agtgccacag agcaggacag caaggacagc acctacagcc tcagcagcga gctgacgctg 2040
   agcaaagcag actacgagaa acacaaagtc tacgcctgcg aagtcaccca tcagggcctg 2100
   agctcgcccg tcacaaagag cttcaacagg ggagagtga 2139
<210> 874
   <211> 579
   <212> PRT
   <213> Artificial Sequence
<220>
   <223> TSC125 (Cris7 scFv-CH2-CH3-CH1)
<400> 874
   Gln Val Gln Leu Val Gln Ser Gly Gly Gly Val Val Gln Pro Gly Arg
    1               5                   10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225 230 235 240
   Leu Gln Ile Thr Arg Ser Ser Glu Pro Lys Ser Ser Asp Lys Thr His
   245 250 255
   Thr Cys Pro Pro Cys Pro Ala Pro Glu Ala Ala Gly Ala Pro Ser Val
   260 265 270
   Phe Leu Phe Pro Pro Lys Pro Lys Asp Thr Leu Met Ile Ser Arg Thr
   275 280 285
   Pro Glu Val Thr Cys Val Val Val Asp Val Ser His Glu Asp Pro Glu
   290 295 300
   Val Lys Phe Asn Trp Tyr Val Asp Gly Val Glu Val His Asn Ala Lys
   305 310 315 320
   Thr Lys Pro Arg Glu Glu Gln Tyr Asn Ser Thr Tyr Arg Val Val Ser
   325 330 335
   Val Leu Thr Val Leu His Gln Asp Trp Leu Asn Gly Lys Ala Tyr Ala
   340 345 350
   Cys Ala Val Ser Asn Lys Ala Leu Pro Ala Pro Ile Glu Lys Thr Ile
   355 360 365
   Ser Lys Ala Lys Gly Gln Pro Arg Glu Pro Gln Val Tyr Thr Leu Pro
   370 375 380
   Pro Ser Arg Asp Glu Leu Thr Lys Asn Gln Val Ser Leu Thr Cys Leu
   385 390 395 400
   Val Lys Gly Phe Tyr Pro Ser Asp Ile Ala Val Glu Trp Glu Ser Asn
   405 410 415
   Gly Gln Pro Glu Asn Asn Tyr Lys Thr Thr Pro Pro Val Leu Asp Ser
   420 425 430
   Asp Gly Ser Phe Phe Leu Tyr Ser Lys Leu Thr Val Asp Lys Ser Arg
   435 440 445
   Trp Gln Gln Gly Asn Val Phe Ser Cys Ser Val Met His Glu Ala Leu
   450 455 460
   His Asn His Tyr Thr Gln Lys Ser Leu Ser Leu Ser Pro Gly Lys Ser
   465 470 475 480
   Arg Ala Ser Thr Lys Gly Pro Ser Val Phe Pro Leu Ala Pro Ser Ser
   485 490 495
   Lys Ser Thr Ser Gly Gly Thr Ala Ala Leu Gly Cys Leu Val Lys Asp
   500 505 510
   Tyr Phe Pro Glu Pro Val Thr Val Ser Trp Asn Ser Gly Ala Leu Thr
   515 520 525
   Ser Gly Val His Thr Phe Pro Ala Val Leu Gln Ser Ser Gly Leu Tyr
   530 535 540
   Ser Leu Ser Ser Val Val Thr Val Pro Ser Ser Ser Leu Gly Thr Gln
   545 550 555 560
   Thr Tyr Ile Cys Asn Val Asn His Lys Pro Ser Asn Thr Lys Val Asp
   565 570 575
   Lys Lys Val
<210> 875
   <211> 574
   <212> PRT
   <213> Artificial Sequence
<220>
   <223> TSC157 (Cris7 scFv-IgA2UH-CH2-CH3-CH1)
<400> 875
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Pro Pro Pro Pro Pro Cys Pro Pro Cys
                   245                 250                 255
   Pro Ala Pro Glu Ala Ala Gly Ala Pro Ser Val Phe Leu Phe Pro Pro
               260                 265                 270
   Lys Pro Lys Asp Thr Leu Met Ile Ser Arg Thr Pro Glu Val Thr Cys
           275                 280                 285
   Val Val Val Asp Val Ser His Glu Asp Pro Glu Val Lys Phe Asn Trp
       290                 295                 300
   Tyr Val Asp Gly Val Glu Val His Asn Ala Lys Thr Lys Pro Arg Glu
   305                 310                 315                 320
   Glu Gln Tyr Asn Ser Thr Tyr Arg Val Val Ser Val Leu Thr Val Leu
                   325                 330                 335
   His Gln Asp Trp Leu Asn Gly Lys Ala Tyr Ala Cys Ala Val Ser Asn
               340                 345                 350
   Lys Ala Leu Pro Ala Pro Ile Glu Lys Thr Ile Ser Lys Ala Lys Gly
           355                 360                 365
   Gln Pro Arg Glu Pro Gln Val Tyr Thr Leu Pro Pro Ser Arg Asp Glu
       370                 375                 380
   Leu Thr Lys Asn Gln Val Ser Leu Thr Cys Leu Val Lys Gly Phe Tyr
   385                 390                 395                 400
   Pro Ser Asp Ile Ala Val Glu Trp Glu Ser Asn Gly Gln Pro Glu Asn
                   405                 410                 415
   Asn Tyr Lys Thr Thr Pro Pro Val Leu Asp Ser Asp Gly Ser Phe Phe
               420                 425                 430
   Leu Tyr Ser Lys Leu Thr Val Asp Lys Ser Arg Trp Gln Gln Gly Asn
           435                 440                 445
   Val Phe Ser Cys Ser Val Met His Glu Ala Leu His Asn His Tyr Thr
       450                 455                 460
   Gln Lys Ser Leu Ser Leu Ser Pro Gly Lys Ser Arg Ala Ser Thr Lys
   465                 470                 475                 480
   Gly Pro Ser Val Phe Pro Leu Ala Pro Ser Ser Lys Ser Thr Ser Gly
                   485                 490                 495
   Gly Thr Ala Ala Leu Gly Cys Leu Val Lys Asp Tyr Phe Pro Glu Pro
               500                 505                 510
   Val Thr Val Ser Trp Asn Ser Gly Ala Leu Thr Ser Gly Val His Thr
           515                 520                 525
   Phe Pro Ala Val Leu Gln Ser Ser Gly Leu Tyr Ser Leu Ser Ser Val
       530                 535                 540
   Val Thr Val Pro Ser Ser Ser Leu Gly Thr Gln Thr Tyr Ile Cys Asn
   545                 550                 555                 560
   Val Asn His Lys Pro Ser Asn Thr Lys Val Asp Lys Lys Val
                   565                 570
<210> 876
   <211> 584
   <212> PRT
   <213> Artificial Sequence
<220>
   <223> TSC158 (Cris7 scFv-IgG1miniUH-CH2-CH3-CH1)
<400> 876
   Gln Val Gln Leu Val Gln Ser Gly Gly Gly Val Val Gln Pro Gly Arg
   1 5 10 15
   Ser Leu Arg Leu Ser Cys Lys Ala Ser Gly Tyr Thr Phe Thr Arg Ser
   20 25 30
   Thr Met His Trp Val Arg Gln Ala Pro Gly Lys Gly Leu Glu Trp Ile
   35 40 45
   Gly Tyr Ile Asn Pro Ser Ser Ala Tyr Thr Asn Tyr Asn Gln Lys Phe
   50 55 60
   Lys Asp Arg Phe Thr Ile Ser Ala Asp Lys Ser Lys Ser Thr Ala Phe
   65 70 75 80
   Leu Gln Met Asp Ser Leu Arg Pro Glu Asp Thr Gly Val Tyr Phe Cys
   85 90 95
   Ala Arg Pro Gln Val His Tyr Asp Tyr Asn Gly Phe Pro Tyr Trp Gly
   100 105 110
   Gln Gly Thr Pro Val Thr Val Ser Ser Gly Gly Gly Gly Ser Gly Gly
   115 120 125
   Gly Gly Ser Gly Gly Gly Gly Ser Ala Gln Asp Ile Gln Met Thr Gln
   130 135 140
   Ser Pro Ser Ser Leu Ser Ala Ser Val Gly Asp Arg Val Thr Met Thr
   145 150 155 160
   Cys Ser Ala Ser Ser Ser Val Ser Tyr Met Asn Trp Tyr Gln Gln Lys
   165 170 175
   Pro Gly Lys Ala Pro Lys Arg Trp Ile Tyr Asp Ser Ser Lys Leu Ala
   180 185 190
   Ser Gly Val Pro Ala Arg Phe Ser Gly Ser Gly Ser Gly Thr Asp Tyr
   195 200 205
   Thr Leu Thr Ile Ser Ser Leu Gln Pro Glu Asp Phe Ala Thr Tyr Tyr
   210 215 220
   Cys Gln Gln Trp Ser Arg Asn Pro Pro Thr Phe Gly Gly Gly Thr Lys
   225 230 235 240
   Leu Gln Ile Thr Arg Ser Ser Glu Pro Lys Ser Ser Asp Lys Thr His
   245 250 255
   Thr Ser Pro Pro Ser Pro Cys Pro Pro Cys Pro Ala Pro Glu Ala Ala
   260 265 270
   Gly Ala Pro Ser Val Phe Leu Phe Pro Pro Lys Pro Lys Asp Thr Leu
   275 280 285
   Met Ile Ser Arg Thr Pro Glu Val Thr Cys Val Val Val Asp Val Ser
   290 295 300
   His Glu Asp Pro Glu Val Lys Phe Asn Trp Tyr Val Asp Gly Val Glu
   305 310 315 320
   Val His Asn Ala Lys Thr Lys Pro Arg Glu Glu Gln Tyr Asn Ser Thr
   325 330 335
   Tyr Arg Val Val Ser Val Leu Thr Val Leu His Gln Asp Trp Leu Asn
   340 345 350
   Gly Lys Ala Tyr Ala Cys Ala Val Ser Asn Lys Ala Leu Pro Ala Pro
   355 360 365
   Ile Glu Lys Thr Ile Ser Lys Ala Lys Gly Gln Pro Arg Glu Pro Gln
   370 375 380
   Val Tyr Thr Leu Pro Pro Ser Arg Asp Glu Leu Thr Lys Asn Gln Val
   385 390 395 400
   Ser Leu Thr Cys Leu Val Lys Gly Phe Tyr Pro Ser Asp Ile Ala Val
   405 410 415
   Glu Trp Glu Ser Asn Gly Gln Pro Glu Asn Asn Tyr Lys Thr Thr Pro
   420 425 430
   Pro Val Leu Asp Ser Asp Gly Ser Phe Phe Leu Tyr Ser Lys Leu Thr
   435 440 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465                 470                 475                 480
   Ser Pro Gly Lys Ser Arg Ala Ser Thr Lys Gly Pro Ser Val Phe Pro
                   485                 490                 495
   Leu Ala Pro Ser Ser Lys Ser Thr Ser Gly Gly Thr Ala Ala Leu Gly
               500                 505                 510
   Cys Leu Val Lys Asp Tyr Phe Pro Glu Pro Val Thr Val Ser Trp Asn
           515                 520                 525
   Ser Gly Ala Leu Thr Ser Gly Val His Thr Phe Pro Ala Val Leu Gln
       530                 535                 540
   Ser Ser Gly Leu Tyr Ser Leu Ser Ser Val Val Thr Val Pro Ser Ser
   545                 550                 555                 560
   Ser Leu Gly Thr Gln Thr Tyr Ile Cys Asn Val Asn His Lys Pro Ser
                   565                 570                 575
   Asn Thr Lys Val Asp Lys Lys Val
               580
<210> 877
   <211> 584
   <212> PRT
   <213> Artificial Sequence
<220>
   <223> TSC159 (Cris7 scFv-IgG3UH-CH2-CH3-CH1)
<400> 877
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Glu Pro Lys Ser Ser Asp Thr Pro Pro
                   245                 250                 255
   Pro Ser Pro Arg Ser Pro Cys Pro Pro Cys Pro Ala Pro Glu Ala Ala
               260                 265                 270
   Gly Ala Pro Ser Val Phe Leu Phe Pro Pro Lys Pro Lys Asp Thr Leu
           275                 280                 285
   Met Ile Ser Arg Thr Pro Glu Val Thr Cys Val Val Val Asp Val Ser
       290                 295                 300
   His Glu Asp Pro Glu Val Lys Phe Asn Trp Tyr Val Asp Gly Val Glu
   305                 310                 315                 320
   Val His Asn Ala Lys Thr Lys Pro Arg Glu Glu Gln Tyr Asn Ser Thr
                   325                 330                 335
   Tyr Arg Val Val Ser Val Leu Thr Val Leu His Gln Asp Trp Leu Asn
               340                 345                 350
   Gly Lys Ala Tyr Ala Cys Ala Val Ser Asn Lys Ala Leu Pro Ala Pro
           355                 360                 365
   Ile Glu Lys Thr Ile Ser Lys Ala Lys Gly Gln Pro Arg Glu Pro Gln
       370                 375                 380
   Val Tyr Thr Leu Pro Pro Ser Arg Asp Glu Leu Thr Lys Asn Gln Val
   385                 390                 395                 400
   Ser Leu Thr Cys Leu Val Lys Gly Phe Tyr Pro Ser Asp Ile Ala Val
                   405                 410                 415
   Glu Trp Glu Ser Asn Gly Gln Pro Glu Asn Asn Tyr Lys Thr Thr Pro
               420                 425                 430
   Pro Val Leu Asp Ser Asp Gly Ser Phe Phe Leu Tyr Ser Lys Leu Thr
           435                 440                 445
   Val Asp Lys Ser Arg Trp Gln Gln Gly Asn Val Phe Ser Cys Ser Val
       450                 455                 460
   Met His Glu Ala Leu His Asn His Tyr Thr Gln Lys Ser Leu Ser Leu
   465                 470                 475                 480
   Ser Pro Gly Lys Ser Arg Ala Ser Thr Lys Gly Pro Ser Val Phe Pro
                   485                 490                 495
   Leu Ala Pro Ser Ser Lys Ser Thr Ser Gly Gly Thr Ala Ala Leu Gly
               500                 505                 510
   Cys Leu Val Lys Asp Tyr Phe Pro Glu Pro Val Thr Val Ser Trp Asn
           515                 520                 525
   Ser Gly Ala Leu Thr Ser Gly Val His Thr Phe Pro Ala Val Leu Gln
       530                 535                 540
   Ser Ser Gly Leu Tyr Ser Leu Ser Ser Val Val Thr Val Pro Ser Ser
   545                 550                 555                 560
   Ser Leu Gly Thr Gln Thr Tyr Ile Cys Asn Val Asn His Lys Pro Ser
                   565                 570                 575
   Asn Thr Lys Val Asp Lys Lys Val
               580
<210> 878
   <211> 680
   <212> PRT
   <213> Artificial Sequence
<220>
   <223> TSC160 (Cris7 scFv-IgMCH2UH-CH2-CH3-CH1)
<400> 878
   Gln Val Gln Leu Val Gln Ser Gly Gly Gly Val Val Gln Pro Gly Arg
    1               5                  10                  15
   Ser Leu Arg Leu Ser Cys Lys Ala Ser Gly Tyr Thr Phe Thr Arg Ser
               20                  25                  30
   Thr Met His Trp Val Arg Gln Ala Pro Gly Lys Gly Leu Glu Trp Ile
           35                  40                  45
   Gly Tyr Ile Asn Pro Ser Ser Ala Tyr Thr Asn Tyr Asn Gln Lys Phe
       50                  55                  60
   Lys Asp Arg Phe Thr Ile Ser Ala Asp Lys Ser Lys Ser Thr Ala Phe
   65                  70                  75                  80
   Leu Gln Met Asp Ser Leu Arg Pro Glu Asp Thr Gly Val Tyr Phe Cys
                   85                  90                  95
   Ala Arg Pro Gln Val His Tyr Asp Tyr Asn Gly Phe Pro Tyr Trp Gly
               100                 105                 110
   Gln Gly Thr Pro Val Thr Val Ser Ser Gly Gly Gly Gly Ser Gly Gly
           115                 120                 125
   Gly Gly Ser Gly Gly Gly Gly Ser Ala Gln Asp Ile Gln Met Thr Gln
       130                 135                 140
   Ser Pro Ser Ser Leu Ser Ala Ser Val Gly Asp Arg Val Thr Met Thr
   145                 150                 155                 160
   Cys Ser Ala Ser Ser Ser Val Ser Tyr Met Asn Trp Tyr Gln Gln Lys
                   165                 170                 175
   Pro Gly Lys Ala Pro Lys Arg Trp Ile Tyr Asp Ser Ser Lys Leu Ala
               180                 185                 190
   Ser Gly Val Pro Ala Arg Phe Ser Gly Ser Gly Ser Gly Thr Asp Tyr
           195                 200                 205
   Thr Leu Thr Ile Ser Ser Leu Gln Pro Glu Asp Phe Ala Thr Tyr Tyr
       210                 215                 220
   Cys Gln Gln Trp Ser Arg Asn Pro Pro Thr Phe Gly Gly Gly Thr Lys
   225                 230                 235                 240
   Leu Gln Ile Thr Arg Ser Ser Ile Ala Glu Leu Pro Pro Lys Val Ser
                   245                 250                 255
   Val Phe Val Pro Pro Arg Asp Gly Phe Phe Gly Asn Pro Arg Lys Ser
               260                 265                 270
   Lys Leu Ile Cys Gln Ala Thr Gly Phe Ser Pro Arg Gln Ile Gln Val
           275                 280                 285
   Ser Trp Leu Arg Glu Gly Lys Gln Val Gly Ser Gly Val Thr Thr Asp
       290                 295                 300
   Gln Val Gln Ala Glu Ala Lys Glu Ser Gly Pro Thr Thr Tyr Lys Val
   305                 310                 315                 320
   Thr Ser Thr Leu Thr Ile Lys Glu Ser Asp Trp Leu Ser Gln Ser Met
                   325                 330                 335
   Phe Thr Cys Arg Val Asp His Arg Gly Leu Thr Phe Gln Gln Asn Ala
               340                 345                 350
   Ser Ser Met Ser Val Pro Cys Pro Pro Cys Pro Ala Pro Glu Ala Ala
           355                 360                 365
   Gly Ala Pro Ser Val Phe Leu Phe Pro Pro Lys Pro Lys Asp Thr Leu
       370                 375                 380
   Met Ile Ser Arg Thr Pro Glu Val Thr Cys Val Val Val Asp Val Ser
   385                 390                 395                 400
   His Glu Asp Pro Glu Val Lys Phe Asn Trp Tyr Val Asp Gly Val Glu
                   405                 410                 415
   Val His Asn Ala Lys Thr Lys Pro Arg Glu Glu Gln Tyr Asn Ser Thr
               420                 425                 430
   Tyr Arg Val Val Ser Val Leu Thr Val Leu His Gln Asp Trp Leu Asn
           435                 440                 445
   Gly Lys Ala Tyr Ala Cys Ala Val Ser Asn Lys Ala Leu Pro Ala Pro
       450                 455                 460
   Ile Glu Lys Thr Ile Ser Lys Ala Lys Gly Gln Pro Arg Glu Pro Gln
   465                 470                 475                 480
   Val Tyr Thr Leu Pro Pro Ser Arg Asp Glu Leu Thr Lys Asn Gln Val
                   485                 490                 495
   Ser Leu Thr Cys Leu Val Lys Gly Phe Tyr Pro Ser Asp Ile Ala Val
               500                 505                 510
   Glu Trp Glu Ser Asn Gly Gln Pro Glu Asn Asn Tyr Lys Thr Thr Pro
           515                 520                 525
   Pro Val Leu Asp Ser Asp Gly Ser Phe Phe Leu Tyr Ser Lys Leu Thr
       530                 535                 540
   Val Asp Lys Ser Arg Trp Gln Gln Gly Asn Val Phe Ser Cys Ser Val
   545                 550                 555                 560
   Met His Glu Ala Leu His Asn His Tyr Thr Gln Lys Ser Leu Ser Leu
                   565                 570                 575
   Ser Pro Gly Lys Ser Arg Ala Ser Thr Lys Gly Pro Ser Val Phe Pro
               580                 585                 590
   Leu Ala Pro Ser Ser Lys Ser Thr Ser Gly Gly Thr Ala Ala Leu Gly
           595                 600                 605
   Cys Leu Val Lys Asp Tyr Phe Pro Glu Pro Val Thr Val Ser Trp Asn
       610                 615                 620
   Ser Gly Ala Leu Thr Ser Gly Val His Thr Phe Pro Ala Val Leu Gln
   625                 630                 635                 640
   Ser Ser Gly Leu Tyr Ser Leu Ser Ser Val Val Thr Val Pro Ser Ser
                   645                 650                 655
   Ser Leu Gly Thr Gln Thr Tyr Ile Cys Asn Val Asn His Lys Pro Ser
               660                 665                 670
   Asn Thr Lys Val Asp Lys Lys Val
   675 680
<210> 879
   <211> 586
   <212> PRT
   <213> Artificial Sequence
<220>
   <223> TSC161 (HD37 scFv-IgA2UH-CH2-CH3-Ck)
<400> 879
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Pro Pro Pro Pro
                   245                 250                 255
   Pro Cys Pro Pro Cys Pro Ala Pro Glu Ala Ala Gly Ala Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Ala Tyr Ala
               340                 345                 350
   Cys Ala Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Asp Glu Leu Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
                   420                     425                     430
   Asp Gly Ser Phe Phe Leu Tyr Ser Lys Leu Thr Val Asp Lys Ser Arg
               435                     440                     445
   Trp Gln Gln Gly Asn Val Phe Ser Cys Ser Val Met His Glu Ala Leu
       450                     455                     460
   His Asn His Tyr Thr Gln Lys Ser Leu Ser Leu Ser Pro Gly Lys Ser
   465                     470                     475                 480
   Arg Thr Val Ala Ala Pro Ser Val Phe Ile Phe Pro Pro Ser Asp Glu
                       485                     490                 495
   Gln Leu Lys Ser Gly Thr Ala Ser Val Val Cys Leu Leu Asn Tyr Phe
                   500                     505                     510
   Tyr Pro Arg Glu Ala Lys Val Gln Trp Lys Val Asp Asn Ala Leu Gln
               515                     520                     525
   Ser Gly Asn Ser Gln Glu Ser Ala Thr Glu Gln Asp Ser Lys Asp Ser
       530                     535                     540
   Thr Tyr Ser Leu Ser Ser Glu Leu Thr Leu Ser Lys Ala Asp Tyr Glu
   545                     550                     555                 560
   Lys His Lys Val Tyr Ala Cys Glu Val Thr His Gln Gly Leu Ser Ser
                       565                     570                 575
   Pro Val Thr Lys Ser Phe Asn Arg Gly Glu
                   580                     585
<210> 880
   <211> 596
   <212> PRT
   <213> Artificial Sequence
<220>
   <223> TSC162 (HD37 scFv-IgG1miniUH-CH2-CH3-Ck)
<400> 880
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Lys Thr His Thr Ser Pro Pro Ser Pro Cys Pro Pro Cys Pro
               260                 265                 270
   Ala Pro Glu Ala Ala Gly Ala Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Asp Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Ala Tyr Ala Cys Ala Val Ser Asn Lys
           355                 360                 365
   Ala Leu Pro Ala Pro Ile Glu Lys Thr Ile Ser Lys Ala Lys Gly Gln
       370                 375                 380
   Pro Arg Glu Pro Gln Val Tyr Thr Leu Pro Pro Ser Arg Asp Glu Leu
   385                 390                 395                 400
   Thr Lys Asn Gln Val Ser Leu Thr Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys Ser Arg Thr Val Ala Ala Pro
                   485                 490                 495
   Ser Val Phe Ile Phe Pro Pro Ser Asp Glu Gln Leu Lys Ser Gly Thr
               500                 505                 510
   Ala Ser Val Val Cys Leu Leu Asn Tyr Phe Tyr Pro Arg Glu Ala Lys
           515                 520                 525
   Val Gln Trp Lys Val Asp Asn Ala Leu Gln Ser Gly Asn Ser Gln Glu
       530                 535                 540
   Ser Ala Thr Glu Gln Asp Ser Lys Asp Ser Thr Tyr Ser Leu Ser Ser
   545                 550                 555                 560
   Glu Leu Thr Leu Ser Lys Ala Asp Tyr Glu Lys His Lys Val Tyr Ala
                   565                 570                 575
   Cys Glu Val Thr His Gln Gly Leu Ser Ser Pro Val Thr Lys Ser Phe
               580                 585                 590
   Asn Arg Gly Glu
           595
<210> 881
   <211> 596
   <212> PRT
   <213> Artificial Sequence
<220>
   <223> TSC163 (HD37 scFv-IgG3UH-CH2-CH3-Ck)
<400> 881
   Asp Ile Val Leu Thr Gln Ser Pro Ala Ser Leu Ala Val Ser Leu Gly
    1               5                  10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Gly
               100                 105                 110
   Gly Gly Gly Ser Gly Gly Gly Gly Ser Gly Gly Gly Gly Ala Ser Gln
           115                 120                 125
   Val Gln Leu Gln Gln Ser Gly Ala Glu Leu Val Arg Pro Gly Ser Ser
       130                 135                 140
   Val Lys Ile Ser Cys Lys Ala Ser Gly Tyr Ala Phe Ser Ser Tyr Trp
   145                 150                 155                 160
   Met Asn Trp Val Lys Gln Arg Pro Gly Gln Gly Leu Glu Trp Ile Gly
                   165                 170                 175
   Gln Ile Trp Pro Gly Asp Gly Asp Thr Asn Tyr Asn Gly Lys Phe Lys
               180                 185                 190
   Gly Lys Ala Thr Leu Thr Ala Asp Glu Ser Ser Ser Thr Ala Tyr Met
           195                 200                 205
   Gln Leu Ser Ser Leu Ala Ser Glu Asp Ser Ala Val Tyr Phe Cys Ala
       210                 215                 220
   Arg Arg Glu Thr Thr Thr Val Gly Arg Tyr Tyr Tyr Ala Met Asp Tyr
   225                 230                 235                 240
   Trp Gly Gln Gly Thr Ser Val Thr Val Ser Ser Ser Glu Pro Lys Ser
                   245                 250                 255
   Ser Asp Thr Pro Pro Pro Ser Pro Arg Ser Pro Cys Pro Pro Cys Pro
               260                 265                 270
   Ala Pro Glu Ala Ala Gly Ala Pro Ser Val Phe Leu Phe Pro Pro Lys
           275                 280                 285
   Pro Lys Asp Thr Leu Met Ile Ser Arg Thr Pro Glu Val Thr Cys Val
       290                 295                 300
   Val Val Asp Val Ser His Glu Asp Pro Glu Val Lys Phe Asn Trp Tyr
   305                 310                 315                 320
   Val Asp Gly Val Glu Val His Asn Ala Lys Thr Lys Pro Arg Glu Glu
                   325                 330                 335
   Gln Tyr Asn Ser Thr Tyr Arg Val Val Ser Val Leu Thr Val Leu His
               340                 345                 350
   Gln Asp Trp Leu Asn Gly Lys Ala Tyr Ala Cys Ala Val Ser Asn Lys
           355                 360                 365
   Ala Leu Pro Ala Pro Ile Glu Lys Thr Ile Ser Lys Ala Lys Gly Gln
       370                 375                 380
   Pro Arg Glu Pro Gln Val Tyr Thr Leu Pro Pro Ser Arg Asp Glu Leu
   385                 390                 395                 400
   Thr Lys Asn Gln Val Ser Leu Thr Cys Leu Val Lys Gly Phe Tyr Pro
                   405                 410                 415
   Ser Asp Ile Ala Val Glu Trp Glu Ser Asn Gly Gln Pro Glu Asn Asn
               420                 425                 430
   Tyr Lys Thr Thr Pro Pro Val Leu Asp Ser Asp Gly Ser Phe Phe Leu
           435                 440                 445
   Tyr Ser Lys Leu Thr Val Asp Lys Ser Arg Trp Gln Gln Gly Asn Val
       450                 455                 460
   Phe Ser Cys Ser Val Met His Glu Ala Leu His Asn His Tyr Thr Gln
   465                 470                 475                 480
   Lys Ser Leu Ser Leu Ser Pro Gly Lys Ser Arg Thr Val Ala Ala Pro
                   485                 490                 495
   Ser Val Phe Ile Phe Pro Pro Ser Asp Glu Gln Leu Lys Ser Gly Thr
               500                 505                 510
   Ala Ser Val Val Cys Leu Leu Asn Tyr Phe Tyr Pro Arg Glu Ala Lys
           515                 520                 525
   Val Gln Trp Lys Val Asp Asn Ala Leu Gln Ser Gly Asn Ser Gln Glu
       530                 535                 540
   Ser Ala Thr Glu Gln Asp Ser Lys Asp Ser Thr Tyr Ser Leu Ser Ser
   545                 550                 555                 560
   Glu Leu Thr Leu Ser Lys Ala Asp Tyr Glu Lys His Lys Val Tyr Ala
                   565                 570                 575
   Cys Glu Val Thr His Gln Gly Leu Ser Ser Pro Val Thr Lys Ser Phe
               580                 585                 590
   Asn Arg Gly Glu
           595
<210> 882
   <211> 692
   <212> PRT
   <213> Artificial Sequence
<220>
   <223> TSC164 (HD37 scFv-IgMCH2UH-CH2-CH3-Ck)
<400> 882
   Asp Ile Val Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Tyr Leu Asn Trp Tyr Gln Gln Ile Pro Gly Gln Pro Pro
   35 40 45
   Lys Leu Leu Ile Tyr Asp Ala Ser Asn Leu Val Ser Gly Ile Pro Pro
   50 55 60
   Arg Phe Ser Gly Ser Gly Ser Gly Thr Asp Phe Thr Leu Asn Ile His
   65 70 75 80
   Pro Val Glu Lys Val Asp Ala Ala Thr Tyr His Cys Gln Gln Ser Thr
   85 90 95
   Glu Asp Pro Trp Thr Phe Gly Gly Gly Thr Lys Leu Glu Ile Lys Gly
   100 105 110
   Gly Gly Gly Ser Gly Gly Gly Gly Ser Gly Gly Gly Gly Ala Ser Gln
   115 120 125
   Val Gln Leu Gln Gln Ser Gly Ala Glu Leu Val Arg Pro Gly Ser Ser
   130 135 140
   Val Lys Ile Ser Cys Lys Ala Ser Gly Tyr Ala Phe Ser Ser Tyr Trp
   145 150 155 160
   Met Asn Trp Val Lys Gln Arg Pro Gly Gln Gly Leu Glu Trp Ile Gly
   165 170 175
   Gln Ile Trp Pro Gly Asp Gly Asp Thr Asn Tyr Asn Gly Lys Phe Lys
   180 185 190
   Gly Lys Ala Thr Leu Thr Ala Asp Glu Ser Ser Ser Thr Ala Tyr Met
   195 200 205
   Gln Leu Ser Ser Leu Ala Ser Glu Asp Ser Ala Val Tyr Phe Cys Ala
   210 215 220
   Arg Arg Glu Thr Thr Thr Val Gly Arg Tyr Tyr Tyr Ala Met Asp Tyr
   225 230 235 240
   Trp Gly Gln Gly Thr Ser Val Thr Val Ser Ser Ser Ile Ala Glu Leu
   245 250 255
   Pro Pro Lys Val Ser Val Phe Val Pro Pro Arg Asp Gly Phe Phe Gly
   260 265 270
   Asn Pro Arg Lys Ser Lys Leu Ile Cys Gln Ala Thr Gly Phe Ser Pro
   275 280 285
   Arg Gln Ile Gln Val Ser Trp Leu Arg Glu Gly Lys Gln Val Gly Ser
   290 295 300
   Gly Val Thr Thr Asp Gln Val Gln Ala Glu Ala Lys Glu Ser Gly Pro
   305 310 315 320
   Thr Thr Tyr Lys Val Thr Ser Thr Leu Thr Ile Lys Glu Ser Asp Trp
   325 330 335
   Leu Ser Gln Ser Met Phe Thr Cys Arg Val Asp His Arg Gly Leu Thr
   340 345 350
   Phe Gln Gln Asn Ala Ser Ser Met Ser Val Pro Cys Pro Pro Cys Pro
   355 360 365
   Ala Pro Glu Ala Ala Gly Ala Pro Ser Val Phe Leu Phe Pro Pro Lys
   370 375 380
   Pro Lys Asp Thr Leu Met Ile Ser Arg Thr Pro Glu Val Thr Cys Val
   385 390 395 400
   Val Val Asp Val Ser His Glu Asp Pro Glu Val Lys Phe Asn Trp Tyr
   405 410 415
   Val Asp Gly Val Glu Val His Asn Ala Lys Thr Lys Pro Arg Glu Glu
   420 425 430
   Gln Tyr Asn Ser Thr Tyr Arg Val Val Ser Val Leu Thr Val Leu His
   435 440 445
   Gln Asp Trp Leu Asn Gly Lys Ala Tyr Ala Cys Ala Val Ser Asn Lys
       450                 455                 460
   Ala Leu Pro Ala Pro Ile Glu Lys Thr Ile Ser Lys Ala Lys Gly Gln
   465                 470                 475                 480
   Pro Arg Glu Pro Gln Val Tyr Thr Leu Pro Pro Ser Arg Asp Glu Leu
                   485                 490                 495
   Thr Lys Asn Gln Val Ser Leu Thr Cys Leu Val Lys Gly Phe Tyr Pro
               500                 505                 510
   Ser Asp Ile Ala Val Glu Trp Glu Ser Asn Gly Gln Pro Glu Asn Asn
           515                 520                 525
   Tyr Lys Thr Thr Pro Pro Val Leu Asp Ser Asp Gly Ser Phe Phe Leu
       530                 535                 540
   Tyr Ser Lys Leu Thr Val Asp Lys Ser Arg Trp Gln Gln Gly Asn Val
   545                 550                 555                 560
   Phe Ser Cys Ser Val Met His Glu Ala Leu His Asn His Tyr Thr Gln
                   565                 570                 575
   Lys Ser Leu Ser Leu Ser Pro Gly Lys Ser Arg Thr Val Ala Ala Pro
               580                 585                 590
   Ser Val Phe Ile Phe Pro Pro Ser Asp Glu Gln Leu Lys Ser Gly Thr
           595                 600                 605
   Ala Ser Val Val Cys Leu Leu Asn Tyr Phe Tyr Pro Arg Glu Ala Lys
       610                 615                 620
   Val Gln Trp Lys Val Asp Asn Ala Leu Gln Ser Gly Asn Ser Gln Glu
   625                 630                 635                 640
   Ser Ala Thr Glu Gln Asp Ser Lys Asp Ser Thr Tyr Ser Leu Ser Ser
                   645                 650                 655
   Glu Leu Thr Leu Ser Lys Ala Asp Tyr Glu Lys His Lys Val Tyr Ala
               660                 665                 670
   Cys Glu Val Thr His Gln Gly Leu Ser Ser Pro Val Thr Lys Ser Phe
           675                 680                 685
   Asn Arg Gly Glu
       690

## Description

The Sequence Listing associated with this application is provided in text format in lieu of a paper copy, and is hereby incorporated by reference into the specification. The name of the text file containing the Sequence Listing is 910180_422PC_SEQUENCE_LISTING.txt. The text file is 839 KB, was created on December 29, 2010, and is being submitted electronically via EFS-Web, concurrent with the filing of the specification.

### BACKGROUND

### Technical Field

The present disclosure generally provides polypeptide heterodimers, compositions thereof, and methods for making and using such polypeptide heterodimers. More specifically, the polypeptide heterodimers provided herein are formed, in part, via natural heterodimerization between an immunoglobulin CHl region and an immunoglobulin light chain constant region (CL). In addition, the polypeptide heterodimers provided herein comprise two or more binding domains that specifically bind one or more targets. Furthermore, both single chain polypeptides of the polypeptide heterodimers provided herein each comprise an Fc region portion (e.g., immunoglobulin CH2 and CH3 domains).

### Description of the Related Art

The process of signal transduction often involves receptor proteins that have extracellular domains, transmembrane domains, and intracellular domains. During ligand binding, cell surface receptor molecules often oligomerize or multimerize (also referred to as "cross-link") to effectively transmit a signal to the intracellular compartment of the cell. The stimulation or blockade of this interaction between a receptor and a ligand or of the subsequent oligomerization or multimerization of receptors has important therapeutic implications for a wide variety of diseases.

Exemplary molecules useful in modulating receptor and ligand interactions include antibodies or molecules derived from antibodies. For instance, an antibody or its derivative may function as a receptor antagonist that binds to a cell surface receptor and inactivates it by blocking the binding site of an activating ligand or preventing receptor dimerization or multimerization required for activation. In certain other cases, an antibody or its derivative may function as an agonist by binding to and cross-linking multiple membrane receptors, mimicking the function of a natural ligand. Another example is a bispecific antibody derivative that may be used to direct cytotoxic agents or immune effector cells to target sites, such as tumors.

Bispecific antibodies are antibody-based molecules that can simultaneously bind two separate and distinct antigens (or different epitopes of the same antigen). One use of bispecific antibodies has been to redirect cytotoxic immune effector cells for enhanced killing of tumor cells by antibody dependent cellular cytotoxicity (ADCC). In this context, one arm of the bispecific antibody binds an antigen on the tumor cell, and the other binds a determinant expressed on effector cells. By cross-linking tumor and effector cells, the bispecific antibody not only brings the effector cells within the proximity of the tumor cells but also simultaneously triggers their activation, leading to effective tumor cell-killing. Bispecific antibodies have also been used to enrich chemo- or radiotherapeutic agents in tumor tissues to minimize detrimental effects to normal tissue. In this setting, one arm of the bispecific antibody binds an antigen expressed on the cell targeted for destruction, and the other arm delivers a chemotherapeutic drug, radioisotope, or toxin.

A major obstacle in the general development of bispecific antibodies has been the difficulty of producing materials of sufficient quality and quantity for both preclinical and clinical studies. Initially, the main route to the production of bispecific antibodies was by co-expression of both the light chains and both the heavy chains of two parent antibodies of different specificities in a single cell. However, the desired binding-competent bispecific antibodies are a minor product, and purification from the other products is very difficult. Another traditional method for bispecific antibody production is chemical conjugation of two antibodies or their fragments having different specificities. However, this method is also complicated, and the chemical modification process may inactivate the antibody or promote aggregation. Because purification from undesired products remains difficult, the resulting low yield and poor quality of bispecific antibody make this process unsuitable for the large scale production required for clinical development.

Recently, various heterodimerization techniques have been used to improve the production of bispecific antibodies. However, fusion of simple heterodimerization domains like the Jun/Fos coiled-coil to scFv domains lead to a mixture of homo- and heterodimers and need to be assembled by refolding (de Kruif and Logtenberg, J. Biol. Chem. 271 : 7630-4, 1996). Fusion of scFv fragments to whole antibodies was also used as a dimerization device (Coloma and Morrison, Nat. Biotechnol. 15 : 159-63, 1997). However, such fusion results in a large molecule with poor solid tissue penetration capabilities. Fusion of two scFv fragments together has also been used to generate bispecific proteins (e.g., BITE® antibodies by Micromet Inc., Bethesda, MD, U.S. Patent No. 7,635,472). However, such proteins do not contain Fc regions, and thus do not allow manipulation of their activities via Fc regions. In addition, these proteins are small (-55 kDa) and thus have relatively short half lives in serum.

WO 2006/074399 A2 discloses multispecific binding molecules comprising synthetic connecting peptides.

WO 02/02781 A1 relates to the production of bispecific or multispecific, bi- or tetravalent antibodies using recombinant DNA methods and recombinant production methods. The resulting antibody consists of one or two diabody molecules that are heterodimerized by creating a fusion protein with the CL and CH1 immunoglobulin constant domains.

US 6 809 185 B1 discloses a class of molecules specified as novel multipurpose antibody derivatives.

US 2007/014794 A1 discloses a method of preparing heteromultimeric polypeptides such as bispecific antibodies, bispecific immunoadhesins and antibody-immunoadhesin chimeras.

To date, immunoglobulin fusion technology has not provided commercially viable heterodimeric proteins or methods for making them. Thus, there remains a need in the art for alternative multispecific heterodimeric proteins as well as efficient methods for producing the same.

In a first aspect of the present invention there is provided a polypeptide heterodimer, comprising:
(a) a first single chain polypeptide (SCP-I) comprising from one to four binding domains that specifically bind from one to four targets, a hinge (H-I), an immunoglobulin heterodimerization domain (HD-I), and an immunoglobulin CH2 and CH3 domain of IgG1, IgG2, IgG3, IgG4, IgA1, IgA2, IgD, or any combination thereof (CH2CH3-I); and
(b) a second single chain polypeptide (SCP-II) comprising from zero to four binding domains that specifically bind from zero to four targets, a hinge (H-II), an immunoglobulin 3 heterodimerization domain (HD-II), and an immunoglobulin CH2 and CH3 domain of IgG1, IgG2, IgG3, IgG4, IgA1, IgA2, IgD, or any combination thereof (CH2CH3-II);
wherein
(i) the immunoglobulin heterodimerization domain of the first single chain polypeptide (HD-I) and the immunoglobulin heterodimerization domain of the second single chain polypeptide (HD-11) preferentially associate with each other to form a polypeptide heterodimer comprised of the first single chain polypeptide (SCP-I) and the second single chain polypeptide (SCP-II), and
   (1) the immunoglobulin heterodimerization domain of the first single chain polypeptide (HD-I) comprises a first immunoglobulin CH1 region, and the immunoglobulin heterodimerization domain of the second single chain polypeptide (HD-II) comprises a first immunoglobulin CL region, or
   (2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region;
(ii) wherein the first immunoglobulin CL region is an altered human immunoglobulin Cκ region with one or more amino acids of a wild type human Cκ region substituted at N29, N30, Q52, V55, T56, S68, or T70,
provided that the polypeptide heterodimer comprises at least two binding domains that specifically bind at least two different targets.

In a further aspect of the invention there is also provided a composition comprising the polypeptide heterodimer of the present invention and a pharmaceutically acceptable excipient.

In another aspect of the present invention there is also provided the polypeptide heterodimer of the present invention for use in a) a method for directing T cell activation, wherein the polypeptide heterodimer comprises a binding domain that specifically binds TCRα, TCRβ, CD3γ, CD3δ, CD3ε or a combination thereof, and a second binding domain that specifically binds a different target; or
b) inhibiting growth, metastasis or metastatic growth of a malignancy, wherein the polypeptide heterodimer comprises a binding domain that specifically binds TCRα, TCRβ, CD3γ, CD3δ, CD3ε, c-Met or Ron.
c) treating an autoimmune or inflammatory condition, wherein the polypeptide heterodimer comprises a binding domain that specifically binids TCRα, TCRβ, CD3γ, CD3δ, CD3ε, or CD28.

In another aspect of the present invention there is provided an expression effector capable of expressing the polypeptide heterodimer of the present invention comprising an expression vector capable of expressing the polypeptide heterodimer of the present invention, comprising a first polynucleotide encoding the first single chain polypeptide and a second polynucleotide encoding the second single chain polypeptide.

The present invention also provides a host cell comprising the expression vector of the present invention and a host cell comprising first and second expression vectors capable of expressing the first and second single chain polypeptides, respectively, of the polypeptide heterodimer of the present invention.

In another aspect of the present invention there is provided a method for making a polypeptide heterodimer, comprising
(a) culturing a host cell in accordance with the present invention under conditions suitable to express first and second single chain polypeptides, and
(b) optionally isolating or purifying the heterodimers formed from the first and second single chain polypeptides from the culture.

The present disclosure provides polypeptide heterodimers formed between two different single chain polypeptides via natural heterodimerization of an immunoglobulin CHI region and an immunoglobulin light chain constant region (CL). The present disclosure also provides nucleic acids, vectors, host cells and methods for making polypeptide heterodimers as well as methods for using such polypeptide heterodimers, such as in directed T cell activation, inhibiting solid malignancy growth, treating autoimmune or inflammatory conditions, or treating B-cell associated disorders or diseases.

In one aspect, the present disclosure provides a polypeptide heterodimer that comprises (a) a first single chain polypeptide (SCP-I) comprising from one to four binding domains that specifically bind from one to four targets, a hinge (H-I), an immunoglobulin heterodimerization domain (HD-I), and an Fc region portion (FRP-I); and (b) a second single chain polypeptide (SCP-II) comprising from zero to four binding domains that specifically bind from zero to four targets, a hinge (H-II), an immunoglobulin heterodimerization domain (HD-II), and an Fc region portion (FRP-II); wherein (i) the immunoglobulin HD-I and the immunoglobulin HD-II preferentially associate with each other to form a polypeptide heterodimer comprised of SCP-I and SCP-II, and (1) the immunoglobulin heterodimerization domain of the first single chain polypeptide (HD-I) comprises a first immunoglobulin CHI region, and the immunoglobulin heterodimerization domain of the second single chain polypeptide (HD-II) comprises a first immunoglobulin CL region, or (2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region; and (ii) the Fc region portion of SCP-I and the Fc region portion of SCP-II comprise an immunoglobulin CH2 and CH3 domain of IgG1, IgG2, IgG3, IgG4, IgA1, IgA2, IgD, or any combination thereof; one or two immunoglobulin CH3 domains of IgG1, IgG2, IgG3, IgG4, IgA1, IgA2, IgD, IgE, IgM, or any combination thereof; or an immunoglobulin CH3 and CH4 domain of IgE, IgM, or any combination thereof, provided that the polypeptide heterodimer comprises at least two binding domains that specifically bind a target, for instance, at least two different targets.

In certain embodiments, the binding domains of the polypeptide heterodimers are single chain Fv (scFv) polypeptides.

In certain embodiments, the polypeptide heterodimer comprises two binding domains (BD1 and BD2). In one embodiment, the two binding domains (BD1 and BD2) are both on the first single chain polypeptide (SCP-I) and wherein the HD-I and FRP-I are disposed between BD1 and BD2. In another embodiment, the first binding domain (BD1) is on the first single chain polypeptide (SCP-I) and the second binding domain (BD2) is on the second single chain polypeptide (SCP-II). For example, the first binding domain (BD1) may be amino terminal to the Fc region portion of the first single chain polypeptide (FRP-I), and the second binding domain (BD2) may amino terminal to the Fc region portion of the second single chain polypeptide (FRP-II). Alternatively, the first binding domain (BD1) may be amino terminal to the Fc region portion of the first single chain polypeptide (FRP-I), and the second binding domain (BD2) may be carboxyl terminal to the Fc region portion of the second single chain polypeptide (FRP-II). Also alternatively, the first binding domain (BD1) may be carboxyl terminal to the Fc region portion of the first single chain polypeptide (FRP-I), and the second binding domain (BD2) may be carboxyl terminal to the Fc region portion of the second single chain polypeptide (FRP-II).

In certain embodiments, the polypeptide heterodimer comprises three binding domains (BD1, BD2 and BD3). In one embodiment, the HD-I and FRP-I are disposed between BD1 and BD2, and the third binding domain (BD3) is amino terminal to the Fc region portion of the second single chain polypeptide (FRP-II). In an alternative embodiment, the HD-I and FRP-I are disposed between BD1 and BD2, and the third binding domain (BD3) is carboxyl terminal to the Fc region portion of the second single chain polypeptide (FRP-II).

In certain embodiments, the polypeptide heterodimer comprises four binding domains (BD1, BD2, BD3, and BD4). For instance, the HD-I and FRP-I may be disposed between BD1 and BD2, and the HD-II and FRP-II may be disposed between BD3 and BD4.

In certain embodiments, the polypeptide heterodimer comprises five to eight binding domains (*e.g.,* 5, 6, 7 or 8 binding domains).

In certain embodiments, at least one of the binding domains of the polypeptide heterodimers provided herein specifically binds to, or is an antagonist of, TCRα, TCRβ, CD3γ, CD3δ, CD3ε, CD28, CD79b, hyperIL-6, monoIL-10 , CD86, CD20, PSMA, CD 19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACI, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, EGFβ, IL10, IL17A/F, CSF2, IGF1, IGF2, BLyS/APRIL, , HGF, MSP, EGF (including epiregulin, herregulin, β-regulin, neuregulin), HIF-1α, VEGFA, VEGFB, VEGFC, VEGFD, TNFα, Wnt, sHH, EGFβ, PDGF, TWEAK, EpCAM, CEA, PCTA-1, STEAP-1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, ganglioside GD3, 9-O-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD137, LIFERβ, TLR7 or TLR9.

In certain other embodiments, at least one of the binding domains of the polypeptide heterodimer is an agonist of IL-10, HLA-G, HGF, IL-35, PD-1, BTLA, TNFR1, TNFR2, DR4, DR5, TWEAKR, or FAS.

In certain polypeptide heterodimers provided herein, at least one binding domain specifically binds a TCR complex or a component thereof, and at least another binding domain specifically binds to PSMA, CD79b, CD19, HLA-DR, CD20, RON, c-Met, or CEACAM-6.

In certain other polypeptide heterodimers provided herein, at least one binding domain specifically binds to CD28, and at least another binding domain specifically binds to, or is an antagonist of, CD79b, hyperIL-6, PDL2, monoIL-10, CD86, LIGHT, GITRL, CD40, PDL1, HVEM, or LTBR.

In certain other polypeptide heterodimers provided herein, at least one binding domain specifically binds to CD28, and at least another binding domain is an agonist of IL-10, HLA-G, HGF, IL-35, PD-1, or BTLA.

In certain embodiments, the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region. In one embodiment, the first CH1 region is amino terminal to the Fc region portion of the first single chain polypeptide, and the first CL region is amino terminal to the Fc region portion of the second single chain polypeptide. In another embodiment, the first CH1 region is carboxyl terminal to the Fc region portion of the first single chain polypeptide, and the first CL region is carboxyl terminal to the Fc region portion of the second single chain polypeptide.

In certain embodiments in which the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region, the first single chain polypeptide further comprises a second CH1 region and the second single chain polypeptide further comprises a second CL region, and the second CH1 region of the first single chain polypeptide and the second CL region of the second single chain polypeptide associate with each other in the polypeptide heterodimer. For instance, the Fc region portion of the first single chain polypeptide may be disposed between the first and second CH1 regions, and the Fc region portion of the second single chain polypeptide may be disposed between the first and second CL regions. Alternatively, both the first and second CH1 regions may be amino terminal to the Fc region portion of the first single chain polypeptide, and both the first and second CL regions may be amino terminal to the Fc region portion of the second single chain polypeptide. Also alternatively, both the first and second CH1 regions may be carboxyl terminal to the Fc region portion of the first single chain polypeptide, and both the first and second CL regions may be carboxyl terminal to the Fc region portion of the second single chain polypeptide.

In certain other embodiments in which the immunoglobulin heterodimerization domain of the first single chain polypeptide (HD-I) comprises the first immunoglobulin CH1 region and the immunoglobulin heterodimerization domain of the second single chain polypeptide (HD-II) comprises the first immunoglobulin CL region, the first single chain polypeptide further comprises a second CL region and the second single chain polypeptide further comprises a second CH1 region, and the second CL region of the first single chain polypeptide and the second CH1 region of the second single chain polypeptide associate with each other in the polypeptide heterodimer. For instance, in one embodiment, in the first single chain polypeptide, the first CH1 region is amino terminal to the Fc region portion and the second CL region is carboxyl terminal to the Fc region portion; and in the second single chain polypeptide, the first CL region is amino terminal to the Fc region portion, and the second CH1 region is carboxyl terminal to the Fc region portion. In another embodiment, in the first single chain polypeptide, the first CH1 region is carboxyl terminal to the Fc region portion, and the second CL region is amino terminal to the Fc region portion; and in the second single chain polypeptide, the first CL region is carboxyl terminal to the Fc region portion, and the second CH1 region is amino terminal to the Fc region portion. In yet another embodiment, in the first single chain polypeptide, both the first CH1 region and the second CL regions are amino terminal to the Fc region portion, and the first CH1 region is amino terminal to the second CL region; and in the second single chain polypeptide, both the first CL region and the second CH1 region are amino terminal to the Fc region portion, and the first CL region is amino terminal to the second CH1 region. In yet another embodiment, in the first single chain polypeptide, both the first CH1 region and the second CL regions are amino terminal to the Fc region portion, and the second CL region is amino terminal to the first CH1 region; and in the second single chain polypeptide, both the first CL region and the second CH1 region are amino terminal to the Fc region portion, and the second CH1 region is amino terminal to the first CL region. In a further embodiment, in the first single chain polypeptide, both the first CH1 region and the second CL regions are carboxyl terminal to the Fc region portion, and the first CH1 region is amino terminal to the second CL region; and in the second single chain polypeptide, both the first CL region and the second CH1 region are carboxyl terminal to the Fc region portion, and the first CL region is amino terminal to the second CH1 region. In another embodiment, in the first single chain polypeptide, both the first CH1 region and the second CL regions are carboxyl terminal to the Fc region portion, and the second CL region is amino terminal to the first CH1 region; and in the second single chain polypeptide, both the first CL region and the second CH1 region are carboxyl terminal to the Fc region portion, and the second CH1 region is amino terminal to the first CL region.

In certain embodiments, the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region. In one embodiment, the first CL region is amino terminal to the Fc region portion of the first single chain polypeptide, and the first CH1 region is amino terminal to the Fc region portion of the second single chain polypeptide. In another embodiment, the first CL region is carboxyl terminal to the Fc region portion of the first single chain polypeptide, and the first CH1 region is carboxyl terminal to the Fc region portion of the second single chain polypeptide. In yet another embodiment, the first single chain polypeptide further comprises a second CL region and the second single chain polypeptide further comprises a second CH1 region, and the second CL region of the first single chain polypeptide and the second CH1 region of the second single chain polypeptide associate with each other in the polypeptide heterodimer. For instance, the Fc region portion of the first single chain polypeptide may be disposed between the first and second CL regions, and the Fc region portion of the second single chain polypeptide may be disposed between the first and second CH1 regions. Alternatively, both the first and second CL regions may be amino terminal to the Fc region portion of the first single chain polypeptide, and both the first and second CH1 regions may be amino terminal to the Fc region portion of the second single chain polypeptide. Also alternatively, both the first and second CL regions may be carboxyl terminal to the Fc region portion of the first single chain polypeptide, and both the first and second CH1 regions may be carboxyl terminal to the Fc region portion of the second single chain polypeptide.

In certain embodiments, the first CL region is a Cκ region. In other embodiments, the first CL region is a Cλ region.

In certain embodiments, the second CL region is a Cκ region. In other embodiments, the second CL region is a Cλ region.

In certain embodiments, the Cκ region is a wild type human immunoglobulin Cκ region. In certain other embodiments, the Cκ region is an altered human immunoglobulin Cκ region with one or more amino acids of a wild type human Cκ region substituted at N29, N30, Q52, V55, T56, T56, S68, or T70. For example, the one or more amino acid substitutions may be selected from Ala (A), Arg (R), Trp (W), Tyr (Y), Glu (E), Gln (Q), Lys (K), Asp (D), Met (M), Ser (S), and Phe (F).

In certain embodiments, the CH1 region is an altered human immunoglobulin CH1 region comprising an amino acid substitution by which Val (V) at position 68 is substituted by Lys (K), Arg (R) or His (H), and wherein the Ck region is an altered human immunoglobulin Ck region comprising an amino acid substitution by which Leu (L) at position 29 is substituted by Asp (D) or Glu (E). In certain other embodiments, the CH1 region is an altered human immunoglobulin CH1 region comprising an amino acid substitution by which Val (V) at position 68 is changed to Asp (D) or Glu (E), and wherein the Ck region is an altered human immunoglobulin Ck region comprising an amino acid substitution by which Leu (L) at position 29 is changed to Lys (K), Arg (R) or His (H).

In certain embodiments, the Cλ region is a wild type human immunoglobulin Cλ region.

In certain embodiments, the first CH1 region or the second CH1 region when present is a wild type human immunoglobulin CH1 region, such as a wild type human IgG1 CH1 region.

In certain embodiments, the first CH1 region or the second CH1 region when present is an altered human immunoglobulin CH1 region, such as an altered human IgG1 CH1 region, with the cysteine of a wild type human immunoglobulin CH1 region that is involved in forming a disulfide bond with a wild type human immunoglobulin CL region deleted or substituted.

In certain embodiments, the Cκ region is an altered human immunoglobulin Ck region, such as an with the cysteine residue of a wild type human Cκ region that is involved in forming a disulfide bond with a wild type human immunoglobulin CH1 region deleted or substituted.

In certain embodiments, the Cλ region is an altered human immunoglobulin Cλ region with the cysteine residue of a wild type human Cλ region that is involved in forming a disulfide bond with a wild type human immunoglobulin CH1 region deleted or substituted.

In certain embodiments, the first CH1 region and the second CH1 region when present is a polypeptide comprising SEQ ID NO:114, 844 or 845.

In certain embodiments, the Ck region when present is selected from any one of the polypeptides comprising SEQ ID NOS:141-178, 202, and 838-843.

In certain embodiments, the Cλ region when present is a polypeptide comprising SEQ ID NO:140.

In certain embodiments, the Fc region portion of the first single chain polypeptide (FRP-I) and the Fc region portion of the second single chain polypeptide (FRP-II) each comprise an immunoglobulin CH2 domain, such as an IgG1 CH2 domain or an IgG2, IgG3, IgG4, IgA1, IgA2, or IgD CH2 domain.

In certain embodiments, the Fc region portion of the first single chain polypeptide (FRP-I) and the Fc region portion of the second single chain polypeptide (FRP-II) each comprise an immunoglobulin CH3 domain, such as an IgG1 CH3 domain or an IgG2, IgG3, IgG4, IgA1, IgA2, IgD, IgE or IgM CH3 domain.

In certain embodiments, the Fc region portion of the first single chain polypeptide (FRP-I) and the Fc region portion of the second single chain polypeptide (FRP-II) each comprise an immunoglobulin CH2 domain and an immunoglobulin CH3 domain, such as are IgG1, IgG2, IgG3, IgG4, IgA1, IgA2, or IgD CH2 and CH3 domains.

In some embodiments in which the Fc region portion comprises an immunoglobulin CH3 domain that is immediately amino terminal to an immunoglobulin heterodimerization domain *(e.g.,* a CH1 domain or a Ck domain), the immunoglobulin CH3 domain is linked to the CH1 domain immediately carboxyl terminal to the immunoglobulin CH3 domain in one single chain polypeptide via a peptide comprising SEQ ID NOS:846, 847, 848, or 849; and the immunoglobulin CH3 domain is linked to the Ck domain immediately carboxyl terminal to the immunoglobulin CH3 domain in the other single chain polypeptide via a peptide comprising SEQ ID NOs:846, 850, 951, or 852.

In certain embodiments, the Fc region portion of the first single chain polypeptide (FRP-I) and the Fc region portion of the second chain polypeptide (FRP-II) comprise IgM or IgE CH3 and CH4 domains.

In certain embodiments in which the Fc region portion comprises an immunoglobulin CH2 domain, the CH2 domain may be an altered human IgG1, IgG2, IgG3, or IgG4 CH2 domain. Exemplary altered human IgG1, IgG2, IgG3 or IgG4 CH2 domain include those that comprise (a) an amino acid substitution at position 297 and at least one additional substitution or deletion at positions 234 to 238; (b) one or more amino acid mutations at positions 234-238 and at least one substitution at position 253, 310, 318, 320, 322, or 331; or (c) an amino acid substitution at the asparagine of position 297, one or more substitutions or deletions at positions 234 to 238, and at least one substitution at position 253, 310, 318, 320, 322, or 331. Another exemplary CH2 domain is an altered human IgG1 CH2 domain that comprises amino acid substitutions at positions L234, L235, G237, E318, K320 and K322.

In certain embodiments, the CH3 domain of the first single chain polypeptide is an altered human IgG1, IgG2, IgG3, or IgG4 CH3 domain that comprises a T366W, and the CH3 domain of the second single chain polypeptide is an altered human IgG1, IgG2, IgG3 or IgG4 CH3 domain that comprises a Y407A substitution. In certain other embodiments, the CH3 domain of the first single chain polypeptide is an altered human IgG1, IgG2, IgG3, or IgG4 CH3 domain that comprises a T366Y substitution, and the CH3 domain of the second single chain polypeptide is an altered human IgG1, IgG2, IgG3 or IgG4 CH3 domain that comprises a Y407T substitution. In certain other embodiments, the CH3 domain of the first single chain polypeptide is an altered human IgG1, IgG2, IgG3, or IgG4 CH3 domain that comprises a T366W substitution, and the CH3 domain of the second single chain polypeptide is an altered human IgG1, IgG2, IgG3 or IgG4 CH3 domain that comprises T366S, L368A and Y407V substitutions. In certain other embodiments, the CH3 domain of the first single chain polypeptide is an altered human IgG1, IgG2, IgG3, or IgG4 CH3 domain that comprises a Y407A substitution, and the CH3 domain of the second single chain polypeptide is an altered human IgG1, IgG2, IgG3 or IgG4 CH3 domain that comprises a T366W substitution. In certain other embodiments, the CH3 domain of the first single chain polypeptide is an altered human IgG1, IgG2, IgG3, or IgG4 CH3 domain that comprises a Y407T substitution, and the CH3 domain of the second single chain polypeptide is an altered human IgG1, IgG2, IgG3 or IgG4 CH3 domain that comprises a T366Y substitution. In certain other embodiments, the CH3 domain of the first single chain polypeptide is an altered human IgG1, IgG2, IgG3, or IgG4 CH3 domain that comprises T366S, L368A and Y407W substitutions, and the CH3 domain of the second single chain polypeptide is an altered human IgG1, IgG2, IgG3 or IgG4 CH3 domain that comprises a T366W substitution.

In certain embodiments, the hinge of both the first and second single chain polypeptides is an immunoglobulin hinge region, such as an IgG1, IgG2, IgG3, IgG4, IgA1, IgA2, IgD, or IgE hinge. In certain embodiments, the immunoglobulin hinge is a wild type immunoglobulin hinge. In certain other embodiments, the immunoglobulin hinge is an altered immunoglobulin hinge selected from SEQ ID NOS:232, 234, 240, 664-673, 675 and 676.

In certain embodiments, the hinge region is (a) at the amino terminal to the Fc region portion, (b) disposed between the binding domain and the immunoglobulin heterodimerization domain, (c) disposed between the immunoglobulin heterodimerization domain and the Fc region portion, (d) at the amino terminus of the first or second single chain polypeptide, (e) disposed between the Fc region portion and a binding domain, or (f) at the carboxyl terminus of the first or second single chain polypeptide.

In certain embodiments, at least one of the first and second single chain polypeptide hinges is a C-type lectin hinge region, such as an NKg2A or NKg2D peptide, or a derivative thereof.

In certain embodiments, the hinges of the first and second single chain polypeptides are identical. In certain other embodiments, the hinges of the first and second single chain polypeptides are different.

In certain embodiments, the first single chain polypeptide comprises a binding domain that specifically binds a TCR complex or a component thereof, and the second single chain polypeptide comprises a binding domain that specifically binds CD 19, CD79b, HLA-DR or CD20.

In certain embodiments, the first single chain polypeptide comprises a binding domain that specifically binds CD28, and the second single chain polypeptide comprises a binding domain (a) that specifically binds CD79b, hyperIL-6, or CD86 or (b) that comprises a PDL ectodomain or a monoIL-10.

In certain embodiments, the first single chain polypeptide comprises a binding domain that specifically binds c-Met, and the second single chain polypeptide comprises a binding domain that specifically binds RON.

In certain embodiments, the first and second single chain polypeptides comprise SEQ ID NOS: SEQ ID NOS:2 and 4, SEQ ID NOS:6 and 8, SEQ ID NOS:10 and 12, SEQ ID NOS: 14 and 16, SEQ ID NOS:18 and 20, SEQ ID NOS:20 and 22, SEQ ID NOS:20 and 24, SEQ ID NOS:30 and 32, SEQ ID NOS:29 and 31, SEQ ID NOS:29 and 32, SEQ ID NOS:30 and 72, SEQ ID NOS:53 and 72, SEQ ID NOS:54 and 72, SEQ ID NOS:55 and 72, SEQ ID NOS:70 and 72, SEQ ID NOS:71 and 72, SEQ ID NOS:63 and 56, SEQ ID NOS:64 and 57, SEQ ID NOS:65 and 60, SEQ ID NOS:66 and 58, SEQ ID NOS:67 and 59, SEQ ID NOS:68 and 61, SEQ ID NOS:69 and 62, SEQ ID NOS:54 and 811, SEQ ID NOS:54 and 812, SEQ ID NOS:54 and 813, SEQ ID NOS:814 and 818, SEQ ID NOS:815 and 818, SEQ ID NOS:816 and 818, SEQ ID NOS:817 and 818, SEQ ID NOS:814 and 820, SEQ ID NOS:814 and 821, SEQ ID NOS:54 and 819, SEQ ID NOS:814 and 826, SEQ ID NOS:814 and 822, SEQ ID NOS:814 and 823, SEQ ID NOS:814 and 824, SEQ ID NOS:859 and 862, SEQ ID NOS:860 and 863, SEQ ID NOS:861 and 864, SEQ ID NOS:874 and 825, SEQ ID NOS:875 and 879, SEQ ID NOS:876 and 880, SEQ ID NOS:877 and 881, or SEQ ID NOS:878 and 882.

In another aspect, the present disclosure provides a composition comprising a polypeptide heterodimer provided herein and a pharmaceutically acceptable excipient.

In another aspect, the present disclosure provides an expression vector capable of expressing a polypeptide heterodimer provided herein, comprising a first polynucleotide encoding the first single chain polypeptide and a second polynucleotide encoding the second single chain polypeptide.

In another aspect, the present disclosure provides a host cell comprising the above expression vector.

In another aspect, the present disclosure provides a host cell comprising first and second expression vectors capable of expressing the first and second single chain polypeptides, respectively, of the polypeptide heterodimer provided herein.

In another aspect, the present disclosure provides a method for making a polypeptide heterodimer, comprising (a) culturing a host cell provided herein under conditions suitable to express first and second single chain polypeptides, and (b) optionally isolating or purifying the heterodimers formed from the first and second single chain polypeptides from the culture.

In another aspect, the present disclosure provides a method for directing T cell activation, comprising administering to a patient in need thereof an effective amount of a polypeptide heterodimer that comprises a binding domain that specifically binds TCRα, TCRβ, CD3γ, CD3δ, CD3ε, or a combination thereof, and a second binding domain that specifically binds a different target, for instance, a tumor-specific antigen or other antigen of choice at a site or cell where T cell activation is desired.

In another aspect, the present disclosure provides a method for inhibiting growth, metastatis, or metastatic growth of a malignancy, comprising administering to a patient in need thereof an effective amount of a polypeptide heterodimer that comprises a binding domain that specifically binds TCRα, TCRβ, CD3γ, CD3δ, CD3ε, c-Met, RON, or a combination thereof. In certain embodiments, the method further comprises administering to a patient in need thereof a chemotherapeutic agent or ionizing radiation.

In another aspect, the present disclosure provides a method for treating an autoimmune or inflammatory condition, comprising administering to a patient in need thereof an effective amount of a polypeptide heterodimer that comprises a binding domain that specifically binds TCRα, TCRβ, CD3γ, CD3δ, CD3ε, or CD28.

In another aspect, the present disclosure provides a method for treating a B-cell associated disorder or disease comprising administering to a patient in need thereof an effective amount of a polypeptide heterodimer that comprises a binding domain that specifically binds TCRα, TCRβ, CD3γ, CD3δ, or CD3ε, and a second binding domain that specifically binds to CD 19, CD20, CD79b or HLA-DR.

In certain embodiments, the methods for using the polypeptide heterodimers provided herein may further comprise administering to a patient in need thereof a second active agent, such as a second polypeptide heterodimer, a monoclonal antibody, or an immunoglobulin-derived fusion protein.

### BRIEF DESCRIPTION OF THE DRAWINGS

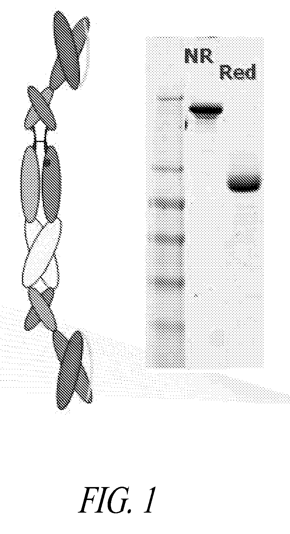

Figure 1 is a schematic representation of bivalent anti-CD28 polypeptide heterodimer X0172 (left) and SDS-PAGE analysis of X0172 (right). "NR" stands for "Non-Reduced," and "Red" stands for "Reduced."
Figure 2 shows that both a monovalent (X0124) and a bivalent (X0172) anti-CD28 polypeptide heterodimer synergize with a suboptimal concentration of PMA in stimulating purified human T cells when compared to an anti-CD28 scFv (2E12 scFv) but less than a bivalent anti-CD28 SMIP protein (2E12 SMIP).
Figure 3 shows that bivalent polypeptide heterodimer X0172 binds to CD4⁺ T cells better than 2E12 scFv and monovalent polypeptide heterodimer X0124.
Figure 4 shows cation exchange chromatography of polypeptide heterodimers X0251, X0252 and X0253 (left) and SDS-PAGE electrophoresis analysis of the same polypeptide heterodimers under non-reduced (NR) and reduced (Red) conditions (right).
Figure 5 shows the mass spectra of polypeptide heterodimer X0252, which demonstrates that the heterodimer is the predominant species.
Figure 6 shows SDS-PAGE electrophoresis analysis of polypeptide heterodimers X0283 and X0284 under non-reduced (NR) and reduced (Red) conditions (left) and cation exchange chromatography analysis of polypeptide heterodimer X0283.
Figure 7 shows direct binding to CD86 by polypeptide heterodimer X0283 as examined by BIACORE® analysis, with response units (Ru) plotted against time (left) and a schematic representation of X0283.
Figures 8A and 8B show binding of bispecific anti-RON and anti-CD3 constructs (polypeptide heterodimer S0268 and Scorpion protein S0266) to MDA-MB-453 cells (A) and to isolated T cells (B).
Figures 9A and 9B show specificity of binding to (A) Rec1 (CD19⁺, CD20⁺) cells or (B) Jurkat (CD3⁺) cells by bispecific heterodimers having either anti-CD19 and anti-CD3 binding domains (TSC020) or having anti-CD20 and anti-CD3 binding domains (TSC021).
Figures 10A-10D show proliferation of CD4+ and CD8+ T-cells in response to bispecific polypeptide heterodimers TSC054, TSC078, TSC079, and bsc19x3 (TSC036) with (A and B) Daudi (CD19⁺) cells or (C and D) MDA-MB-453 (CD19⁻) cells.
Figures 11A and 11B shows T-cell directed cytotoxicity induced by bispecific polypeptide heterodimers TSC054, TSC078, TSC079, and S0268 in a chromium (⁵¹Cr) release assay with (A) Daudi (RON⁻, CD19⁺) cells or (B) BxPC-3 (RON⁺, CD19⁻) cells.
Figure 12 shows target-dependent T-cell proliferation induced by a CD 19+ cell line (Daudi) using bispecific polypeptide heterodimers TSC165, TSC166, TSC167, TSC168 and TSC100 at concentrations from 0.1 pM to 10000 pM.
Figure 13 shows target-dependent T-cell proliferation induced by a CD19+ cell line (Daudi) using bispecific polypeptide heterodimers TSC127 and TS165 with bsc19x3 BiTE as a control at concentrations from 0.001 pM to 1000 pM.
Figure 14 shows target-dependent redirected T-cell cytotoxicity on a CD 19+ cell line (Daudi) using bispecific polypeptide heterodimers TSC100, TSC165, TC166, TSC167, and TSC168.

### DETAILED DESCRIPTION

The present disclosure provides polypeptide heterodimers formed between two different single chain polypeptides via natural heterodimerization of an immunoglobulin CH1 region and an immunoglobulin light chain constant region (CL). The polypeptide heterodimer has two or more binding domains that specifically bind one or more targets (*e.g*., an antigen, a receptor, or a ligand). In addition, both chains of a heterodimer each further comprise an Fc region portion (*e.g*., immunoglobulin CH2 and/or CH3 domains). The present disclosure also provides nucleic acids, vectors, host cells and methods for making polypeptide heterodimers.

The heterodimerization technology described herein has one or more of the following advantages: (1) potential for minimal immunogenicity of the polypeptide heterodimers because the dimers are formed via natural heterodimerization of an immunoglobulin CH1 region and an immunoglobulin CL region; (2) efficient production and purification of polypeptide heterodimers of the present disclosure is possible by co-expressing the two different single chain polypeptides, as shown in the examples; (3) the ability to mediate Fc effector functions (*e.g*., CDC, ADCC, ADCP), which can be modulated up or down by mutagenesis, and a longer serum half life because each chain of a polypeptide heterodimer according to the present disclosure has an Fc region portion (*e.g*., immunoglobulin CH2 and CH3 domains); and (4) polypeptide heterodimers of the present disclosure have a size that is typically smaller than an antibody molecule, which can allow for better tissue penetration, such as into a solid malignancy.

The polypeptide heterodimers provided herein are useful to direct therapeutic agents or immune effector cells to target cells. For instance, in certain embodiments, the polypeptide heterodimers may comprise a binding domain that specifically binds a TCR complex or a component thereof (*e.g.,* TCRα, TCRβ, CD3γ, CD3δ, and CD3ε) and another binding domain that specifically binds a second different target, such as an oncology target (*e.g*., c-Met, RON, CEACAM-6, and PSMA) or a B-cell target (*e.g.,* CD19, CD79b, HLA-DR and CD20). The binding domain specific for the second different target may have a higher affinity for its target than the affinity of the binding domain for the TCR complex or component thereof, such as CD3. Such polypeptide heterodimers will preferably bind to the oncology target or the B-cell target first and subsequently recruit T cells to the tumor or cancer cells expressing the oncology target or B-cell target and are useful in inhibiting growth, metastasis or metastatic growth of a malignancy (including B-cell cancers). Additional uses of polypeptide heterodimers provided herein include directed T cell activation and treating autoimmune or inflammatory conditions.

The section headings used herein are for organizational purposes only and are not to be construed as limiting the subject matter described. All documents, or portions of documents, cited herein, including but not limited to patents, patent applications, articles, books, and treatises, are hereby expressly incorporated by reference in their entirety for any purpose. In the event that one or more of the incorporated documents or portions of documents defines a term that contradicts that term's definition in the application, the definition that appears in this application controls.

In the present description, any concentration range, percentage range, ratio range, or integer range is to be understood to include the value of any integer within the recited range and, when appropriate, fractions thereof (such as one tenth and one hundredth of an integer), unless otherwise indicated. As used herein, "about" means ± 20% of the indicated range, value, sequence, or structure, unless otherwise indicated. It should be understood that the terms "a" and "an" as used herein refer to "one or more" of the enumerated components unless otherwise indicated or dictated by its context. The use of the alternative (e.g., "or") should be understood to mean either one, both, or any combination thereof of the alternatives. As used herein, the terms "include" and "comprise" are used synonymously. In addition, it should be understood that the individual single chain polypeptides or heterodimers derived from various combinations of the structures and substituents (e.g., domains, regions, hinges, and linkers) described herein are disclosed by the present application to the same extent as if each single chain polypeptide or heterodimer were set forth individually. Thus, selection of particular components to form individual single chain polypeptides or heterodimers is within the scope of the present disclosure.

As used herein, a protein "consists essentially of" several domains (*e.g*., a binding domain that specifically binds a target, a hinge, an immunoglobulin heterodimerization domain, and an Fc region portion) if the other portions of the protein (*e.g*., amino acids at the amino- or carboxy-terminus or between two domains), in combination, contribute to at most 20% *(e.g.,* at most 15%, 10%, 8%, 6%, 5%, 4%, 3%, 2% or 1 %) of the length of the protein and do not substantially affect (*i.e.*, do not reduce the activity by more than 50%, such as more than 40%, 30%, 25%, 20%, 15%, 10%, or 5%) the activities of various domains (*e.g*., the target binding affinity of the binding domain, the activities of the Fc region portion, and the capability of the immunoglobulin heterodimerization domain in facilitating heterodimerization). In certain embodiments, a protein (*e.g.,* a single chain polypeptide) consists essentially of a binding domain that specifically binds a target, an immunoglobulin heterodimerization domain, a hinge, and an Fc region portion may comprise junction amino acids at the amino- and/or carboxy-terminus of the protein and/or between two different domains (*e.g*., between the binding domain and the immunoglobulin heterodimerization domain, between the immunoglobulin heterodimerization domain and the hinge, and/or between the hinge and the Fc region portion).

A "polypeptide heterodimer" or "heterodimer," as used herein, refers to a dimer formed from two different single chain polypeptides. This term does not include an antibody formed from four single chain polypeptides *(i.e.,* two light chains and two heavy chains). A "dimer" refers to a biological entity that consists of two subunits associated with each other via one or more forms of intramolecular forces, including covalent bonds *(e.g.,* disulfide bonds) and other interactions *(e.g.,* electrostatic interactions, salt bridges, hydrogen bonding, and hydrophobic interactions), and is stable under appropriate conditions (*e.g*., under physiological conditions, in an aqueous solution suitable for expressing, purifying, and/or storing recombinant proteins, or under conditions for non-denaturing and/or non-reducing electrophoresis).

A "single chain polypeptide" is a single, linear and contiguous arrangement of covalently linked amino acids. It does not include two polypeptide chains that link together in a non-linear fashion, such as via an interchain disulfide bond *(e.g.,* a half immunoglobulin molecule in which a light chain links with a heavy chain via a disulfide bond). In certain embodiments, a single chain polypeptide may have or form one or more intrachain disulfide bonds.

An "immunoglobulin heterodimerization domain," as used herein, refers to an immunoglobulin domain of a single chain polypeptide that preferentially interacts or associates with a different immunoglobulin domain of another single chain polypeptide wherein the interaction of the different heterodimerization domains substantially contributes to or efficiently promotes heterodimerization of the first and second single chain polypeptides *(i.e.,* the formation of a dimer between two different single chain polypeptides, which is also referred to as a "heterodimer"). The interaction(s) between heterodimerization domains "substantially contributes to or efficiently promotes" the heterodimerization of first and second single chain polypeptides if there is a statistically significant reduction in the dimerization between the first and second single chain polypeptides in the absence of the heterodimerization domain of the first single chain polypeptide (HD-I) and/or the heterodimerization domain of the second single chain polypeptide (HD-II). In certain embodiments, when the first and second single chain polypeptides are co-expressed, at least 60%, at least about 60% to about 70%, at least about 70% to about 80%, at least about 80% to about 90%, 91%, 92%, 93%, 94%, 95%, 96%, 97%, 98%, 99%, or 100%, and at least about 90% to about 92%, 93%, 94%, 95%, 96%, 97%, 98%, or 99% of the first and second single chain polypeptides form heterodimers with each other. Representative immunoglobulin heterodimerization domains of the present disclosure include an immunoglobulin CH1 region, an immunoglobulin CL region (*e.g.,* Cκ or Cλ isotypes), or derivatives thereof, including wild type immunoglobulin CH1 and CL regions and altered (or mutated) immunoglobulin CH1 and CL regions, as provided herein.

A "binding domain" or "binding region," as used herein, refers to a protein, polypeptide, oligopeptide, or peptide that possesses the ability to specifically recognize and bind to a target *(e.g.,* CD3, TCR, CD28, c-Met, RON). A binding domain includes any naturally occurring, synthetic, semi-synthetic, or recombinantly produced binding partner for a biological molecule or another target of interest. Exemplary binding domains include single chain antibody variable regions (*e.g*., domain antibodies, sFv, scFv, Fab), receptor ectodomains (*e.g*., c-Met, RON), or ligands (*e.g*., cytokines, chemokines). A variety of assays are known for identifying binding domains of the present disclosure that specifically bind a particular target, including Western blot, ELISA, and Biacore analysis.

A binding domain and a fusion protein thereof "specifically binds" a target if it binds the target with an affinity or Ka *(i.e.,* an equilibrium association constant of a particular binding interaction with units of 1/M) equal to or greater than 10⁵ M⁻¹, while not significantly binding other components present in a test sample. Binding domains (or fusion proteins thereof) may be classified as "high affinity" binding domains (or fusion proteins thereof) and "low affinity" binding domains (or fusion proteins thereof). "High affinity" binding domains refer to those binding domains with a Kₐ of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. "Low affinity" binding domains refer to those binding domains with a Kₐ of up to 10⁷ M⁻¹, up to 10⁶ M⁻¹, up to 10⁵ M⁻¹. Alternatively, affinity may be defined as an equilibrium dissociation constant (K_{d}) of a particular binding interaction with units of M (*e.g.,* 10⁻⁵ M to 10⁻¹³ M). Affinities of binding domain polypeptides and single chain polyepeptides according to the present disclosure can be readily determined using conventional techniques *(see, e.g.,* Scatchard et al. (1949) Ann. N.Y. Acad. Sci. 51:660; and U.S. Patent Nos. 5,283,173, 5,468,614, or the equivalent).

"T cell receptor" (TCR) is a molecule found on the surface of T cells that, along with CD3, is generally responsible for recognizing antigens bound to major histocompatibility complex (MHC) molecules. It consists of a disulfide-linked heterodimer of the highly variable α and β chains in most T cells. In other T cells, an alternative receptor made up of variable γ and δ chains is expressed. Each chain of the TCR is a member of the immunoglobulin superfamily and possesses one N-terminal immunoglobulin variable domain, one immunoglobulin constant domain, a transmembrane region, and a short cytoplasmic tail at the C-terminal end *(see,* Abbas and Lichtman, Cellular and Molecular Immunology (5th Ed.), Editor: Saunders, Philadelphia, 2003; Janeway et al., Immunobiology: The Immune System in Health and Disease, 4th Ed., Current Biology Publications, p148, 149, and 172, 1999). TCR as used in the present disclosure may be from various animal species, including human, mouse, rat, or other mammals.

"CD3" is known in the art as a multi-protein complex of six chains *(see,* Abbas and Lichtman, 2003; Janeway *et al.,* p. 172 and 178, 1999). In mammals, the complex comprises a CD3γ chain, a CD3δ chain, two CD3ε chains, and a homodimer of CD3ζ chains. The CD3γ, CD3δ, and CD3ε chains are highly related cell surface proteins of the immunoglobulin superfamily containing a single immunoglobulin domain. The transmembrane regions of the CD3γ, CD3δ, and CD3ε chains are negatively charged, which is a characteristic that allows these chains to associate with the positively charged T cell receptor chains. The intracellular tails of the CD3γ, CD3δ, and CD3ε chains each contain a single conserved motif known as an immunoreceptor tyrosine-based activation motif or ITAM, whereas each CD3ζ chain has three. It is believed the ITAMs are important for the signaling capacity of a TCR complex. CD3 as used in the present disclosure may be from various animal species, including human, mouse, rat, or other mammals.

"TCR complex," as used herein, refers to a complex formed by the association of CD3 with TCR. For example, a TCR complex can be composed of a CD3γ chain, a CD3δ chain, two CD3ε chains, a homodimer of CD3ζ chains, a TCRα chain, and a TCRβ chain. Alternatively, a TCR complex can be composed of a CD3γ chain, a CD3δ chain, two CD3ε chains, a homodimer of CD3ζ chains, a TCRγ chain, and a TCRδ chain.

"A component of a TCR complex," as used herein, refers to a TCR chain *(i.e.,* TCRα, TCRβ, TCRγ or TCRδ), a CD3 chain (*i.e.,* CD3γ, CD3δ, CD3ε or CD3ζ), or a complex formed by two or more TCR chains or CD3 chains (*e.g.,* a complex of TCRα and TCRβ, a complex of TCRγ and TCRδ, a complex of CD3ε and CD3δ, a complex of CD3γ and CD3ε, or a sub-TCR complex of TCRα, TCRβ, CD3γ, CD3δ, and two CD3ε chains).

Terms understood by those in the art of antibody technology are each given the meaning acquired in the art, unless expressly defined differently herein. Antibodies are known to have variable regions, a hinge region, and constant domains. Immunoglobulin structure and function are reviewed, for example, in Harlow et al., Eds., Antibodies: A Laboratory Manual, Chapter 14 (Cold Spring Harbor Laboratory, Cold Spring Harbor, 1988).

For example, the terms "VL" and "VH" refer to the variable binding region from an antibody light and heavy chain, respectively. The variable binding regions are made up of discrete, well-defined sub-regions known as "complementarity determining regions" (CDRs) and "framework regions" (FRs). The term "CL" refers to an "immunoglobulin light chain constant region" or a "light chain constant region," *i.e.,* a constant region from an antibody light heavy chain. The term "CH" refers to an "immunoglobulin heavy chain constant region" or a "heavy chain constant region," which is further divisible, depending on the antibody isotype into CH1, CH2, and CH3 (IgA, IgD, IgG), or CH1, CH2, CH3, and CH4 domains (IgE, IgM). A "Fab" (fragment antigen binding) is the part of an antibody that binds to antigens and includes the variable region and CH1 of the heavy chain linked to the light chain via an inter-chain disulfide bond.

As used herein, "an Fc region constant domain portion" or "Fc region portion" refers to the heavy chain constant region segment of the Fc fragment (the "fragment crystallizable" region or Fc region) from an antibody, which can include one or more constant domains, such as CH2, CH3, CH4, or any combination thereof. By way of background, the Fc region is responsible for the effector functions of an immunoglobulin, such as ADCC (antibody-dependent cell-mediated cytotoxicity), ADCP (antibody-dependent cellular phagocytosis), CDC (complement-dependent cytotoxicity) and complement fixation, binding to Fc receptors (e.g., CD16, CD32, FcRn), greater half-life *in vivo* relative to a polypeptide lacking an Fc region, protein A binding, and perhaps even placental transfer (*see* Capon et al., Nature, 337:525 (1989)).

In addition, antibodies have a hinge sequence that is typically situated between the Fab and Fc region (but a lower section of the hinge may include an amino-terminal portion of the Fc region). By way of background, an immunoglobulin hinge acts as a flexible spacer to allow the Fab portion to move freely in space. In contrast to the constant regions, hinges are structurally diverse, varying in both sequence and length between immunoglobulin classes and even among subclasses. For example, a human IgG1 hinge region is freely flexible, which allows the Fab fragments to rotate about their axes of symmetry and move within a sphere centered at the first of two inter-heavy chain disulfide bridges. By comparison, a human IgG2 hinge is relatively short and contains a rigid poly-proline double helix stabilized by four inter-heavy chain disulfide bridges, which restricts the flexibility. A human IgG3 hinge differs from the other subclasses by its unique extended hinge region (about four times as long as the IgG1 hinge), containing 62 amino acids (including 21 prolines and 11 cysteines), forming an inflexible poly-proline double helix and providing greater flexibility because the Fab fragments are relatively far away from the Fc fragment. A human IgG4 hinge is shorter than IgG1 but has the same length as IgG2, and its flexibility is intermediate between that of IgG1 and IgG2.

According to crystallographic studies, an IgG hinge domain can be functionally and structurally subdivided into three regions: the upper, the core or middle, and the lower hinge regions (Shin et al., Immunological Reviews 130:87 (1992)). Exemplary upper hinge regions include EPKSCDKTHT (SEQ ID NO:227) as found in IgG1, ERKCCVE (SEQ ID NO:211) as found in IgG2, ELKTPLGDTT HT (SEQ ID NO:245) or EPKSCDTPPP (SEQ ID NO:246) as found in IgG3, and ESKYGPP (SEQ ID NO:247) as found in IgG4. Exemplary middle or core hinge regions include CPPCP (SEQ ID NO:228) as found in IgG1 and IgG2, CPRCP (SEQ ID NO:248) as found in IgG3, and CPSCP (SEQ ID NO:249) as found in IgG4. While IgG1, IgG2, and IgG4 antibodies each appear to have a single upper and middle hinge, IgG3 has four in tandem - one being ELKTPLGDTTHTCPRCP (SEQ ID NO:250) and three being EPKSCDTPPP CPRCP (SEQ ID NO:251).

IgA and IgD antibodies appear to lack an IgG-like core region, and IgD appears to have two upper hinge regions in tandem *(see* SEQ ID NOS:222 and 252). Exemplary wild type upper hinge regions found in IgA1 and IgA2 antibodies are set forth in SEQ ID NOS:215 and 216.

IgE and IgM antibodies, in contrast, lack a typical hinge region and instead have a CH2 domain with hinge-like properties. Exemplary wild-type CH2 upper hinge-like sequences of IgE and IgM are set forth in SEQ ID NO:253 (VCSRDFTPPTVKILQSSSDGGGHFPPTIQLLCLVSGYTPGTINITWLEDG QVMDVDLSTASTTQEGELASTQSELTLSQKHWLSDRTYTCQVTYQGHTFE DSTKKCA) and SEQ ID NO:254 (VIAELPPKVSVFVPPRDGFFGNPRKSKLIC QATGFSPRQIQVSWLREGKQVGSGVTTDQVQAEAKESGPTTYKVTSTLTI KESDWLGQSMFTCRVDHRGLTFQQNASSMCVP), respectively.

As used herein, a "hinge region" or a "hinge" refers to (a) an immunoglobulin hinge region (made up of, for example, upper and core regions) or a functional variant thereof, including wild type and altered immunoglobulin hinges, (b) a lectin interdomain region or a functional variant thereof, (c) a cluster of differentiation (CD) molecule stalk region or a functional variant thereof, or (d) a portion of a cell surface receptor (interdomain region) that connects immunoglobulin V-like or immunoglobulin C-like domains.

As used herein, a "wild type immunoglobulin hinge region" refers to a naturally occurring upper and middle hinge amino acid sequences interposed between and connecting the CH1 and CH2 domains (for IgG, IgA, and IgD) or interposed between and connecting the CH1 and CH3 domains (for IgE and IgM) found in the heavy chain of an antibody. In certain embodiments, a wild type immunoglobulin hinge region sequence is human, and may comprises a human IgG hinge region. Exemplary human wild type immunoglobulin hinge regions are set forth in SEQ ID NOS:215 (IgA1 hinge), 216 (IgA2 hinge), 217 (IgD hinge), 667 (IgG1 hinge), 219 (IgG2 hinge), 220 (IgG3 hinge) and 221 (IgG4 hinge).

An "altered wild type immunoglobulin hinge region" or "altered immunoglobulin hinge region" refers to (a) a wild type immunoglobulin hinge region with up to 30% amino acid changes (*e.g*., up to 25%, 20%, 15%, 10%, or 5% amino acid substitutions or deletions), or (b) a portion of a wild type immunoglobulin hinge region that has a length of about 5 amino acids (*e.g*., about 5, 6, 7, 8, 9, 10, 11, 12, 13, 14, 15, 16, 17, 18, 19, or 20 amino acids) up to about 120 amino acids (for instance, having a length of about 10 to about 40 amino acids or about 15 to about 30 amino acids or about 15 to about 20 amino acids or about 20 to about 25 amino acids), has up to about 30% amino acid changes (*e.g*., up to about 25%, 20%, 15%, 10%, 5%, 4%, 3%, 2%, or 1% amino acid substitutions or deletions or a combination thereof), and has an IgG core hinge region as set forth in SEQ ID NOS:228, 248, or 249.

A "peptide linker" refers to an amino acid sequence that connects a heavy chain variable region to a light chain variable region and provides a spacer function compatible with interaction of the two sub-binding domains so that the resulting polypeptide retains a specific binding affinity to the same target molecule as an antibody that comprises the same light and heavy chain variable regions. In certain embodiments, a linker is comprised of five to about 35 amino acids, for instance, about 15 to about 25 amino acids.

"Junction amino acids" or "junction amino acid residues" refer to one or more (*e.g*., about 2-10) amino acid residues between two adjacent regions or domains of a single chain polypeptide, such as between a hinge and an adjacent Fc region portion or between a hinge and an adjacent binding domain or between a peptide linker that links two immunoglobulin variable domains and an adjacent immunoglobulin variable domain. Junction amino acids may result from the construct design of a single chain polypeptide (*e.g*., amino acid residues resulting from the use of a restriction enzyme site during the construction of a nucleic acid molecule encoding a single chain polypeptide).

A "linker between CH3 and CH1 or CL" refers to one or more (*e.g.,* about 2-12) amino acid residues between the C- terminus of a CH3 domain *(e.g.,* a wild type CH3 or a mutated CH3) and the N-terminus of a CH1 domain or CL domain (*e.g*., Ck).

A "wild type immunoglobulin region" or "wild type immunoglobulin domain" refers to a naturally occurring immunoglobulin region or domain *(e.g.,* a naturally occurring VL, VH, hinge, CL, CH1, CH2, CH3, or CH4) from various immunoglobulin classes or subclasses (including, for example, IgG1, IgG2, IgG3, IgG4, IgA1, IgA2, IgD, IgE, and IgM) and from various species (including, for example, human, sheep, mouse, rat, and other mammals). Exemplary wild type human CH1 regions are set forth in SEQ ID NOS:114, 186-192 and 194, wild type human Cκ region in SEQ ID NO:112, wild type human Cλ regions in SEQ ID NO:113 and 224-226, wild type human CH2 domains in SEQ ID NOS:115, 195-201 and 203, wild type human CH3 domains in SEQ ID NOS:116, 204-210 and 212, and wild type human CH4 domains in SEQ ID NO:213 and 214.

An "altered immunoglobulin region," "altered immunoglobulin domain," "mutated immunoglobulin domain," or the like, refers to an immunoglobulin region with a sequence identity to a wild type immunoglobulin region or domain *(e.g.,* a wild type VL, VH, hinge, CL, CH1, CH2, CH3, or CH4) of at least 75% *(e.g.,* 80%, 82%, 84%, 86%, 88%, 90%, 91%, 92%, 93%, 94%, 95%, 96%, 97%, 98%, 99%, or 99.5%). For example, an "altered immunoglobulin CH1 region" or "altered CH1 region" refers to a CH1 region with a sequence identity to a wild type immunoglobulin CH1 region *(e.g.,* a human CH1) of at least 75% *(e.g.,* 80%, 82%, 84%, 86%, 88%, 90%, 91%, 92%, 93%, 94%, 95%, 96%, 97%, 98%, 99%, or 99.5%). Similarly, an "altered immunoglobulin CH2 domain" or "altered CH2 domain" refers to a CH2 domain with a sequence identity to a wild type immunoglobulin CH2 region *(e.g.,* a human CH2) of at least 75% *(e.g.,* 80%, 82%, 84%, 86%, 88%, 90%, 91%, 92%, 93%, 94%, 95%, 96%, 97%, 98%, 99%, or 99.5%).

"Sequence identity," as used herein, refers to the percentage of amino acid residues in one sequence that are identical with the amino acid residues in another reference polypeptide sequence after aligning the sequences and introducing gaps, if necessary, to achieve the maximum percent sequence identity, and not considering any conservative substitutions as part of the sequence identity. The percentage sequence identity values are generated by the NCBI BLAST2.0 software as defined by Altschul et al. (1997) "Gapped BLAST and PSI-BLAST: a new generation of protein database search programs," Nucleic Acids Res. 25:3389-3402, with the parameters set to default values.

In certain embodiments, an altered immunoglobulin domain only contains conservative amino acid substitutions of a wild type immunoglobulin domain. In certain other embodiments, an altered immunoglobulin domain only contains non-conservative amino acid substitutions of a wild type immunoglobulin domain. In yet other embodiments, an altered immunoglobulin domain contains both conservative and non-conservative amino acid substitutions.

A "conservative substitution" is recognized in the art as a substitution of one amino acid for another amino acid that has similar properties. Exemplary conservative substitutions are well known in the art *(see, e.g.,* WO 97/09433, page 10, published March 13, 1997; Lehninger, Biochemistry, Second Edition; Worth Publishers, Inc. NY:NY (1975), pp.71-77; Lewin, Genes IV, Oxford University Press, NY and Cell Press, Cambridge, MA (1990), p. 8). In certain embodiments, a conservative substitution includes a leucine to serine substitution.

As used herein, the term "derivative" refers to a modification of one or more amino acid residues of a peptide by chemical or biological means, either with or without an enzyme, *e.g*., by glycosylation, alkylation, acylation, ester formation, or amide formation. Generally, a "derivative" differs from an "analogue" in that a parent polypeptide may be the starting material to generate a "derivative," whereas the parent polypeptide may not necessarily be used as the starting material to generate an "analogue." A derivative may have different chemical, biological or physical properties of the parent polypeptide. For example, a derivative may be more hydrophilic or it may have altered reactivity (e.g., a CDR having an amino acid change that alters its affinity for a target) as compared to the parent polypeptide.

As used herein, unless otherwise provided, a position of an amino acid residue in a variable region of an immunoglobulin molecule is numbered according to the Kabat numbering convention (Kabat, Sequence of Proteins of Immunological Interest, 5th ed. Bethesda, MD: Public Health Service, National Institutes of Health (1991)), and a position of an amino acid residue in a constant region of an immunoglobulin molecule is numbered according to EU nomenclature (Ward et al., 1995 Therap. Immunol. 2:77-94).

A "receptor" is a protein molecule present in the plasma membrane or in the cytoplasm of a cell to which a signal molecule *(i.e.,* a ligand, such as a hormone, a neurotransmitter, a toxin, a cytokine) may attach. The binding of the signal molecule to the receptor results in a conformational change of the receptor, which ordinarily initiates a cellular response. However, some ligands merely block receptors without inducing any response (e.g., antagonists). Some receptor proteins are peripheral membrane proteins. Many hormone and neurotransmitter receptors are transmembrane proteins that are embedded in the phospholipid bilayer of cell membranes, and another major class of receptors are intracellular proteins such as those for steroid and intracrine peptide hormone receptors.

"B-cell associated disorder or disease" or "a disease or disorder associated with aberrant B-cell activity" refers to a disease or disorder associated with (e.g., causing or resulting from) aberrant B-cell activity or activity that deviates from the normal, proper, or expected course. For example, a B-cell associated disorder or disease may include inappropriate proliferation of B-cells that have damaged or defective DNA or other cellular components. Aberrant B-cell activity may include cell proliferation characterized by inappropriately high levels of B-cell division, inappropriately low levels of B-cell apoptosis, or both. Such diseases may have, for example, single or multiple local abnormal proliferations of B-cells, groups of B-cells or tissue(s), whether cancerous or non-cancerous, benign or malignant. A B-cell associated disorder or disease may also include aberrant antibody production, such as production of autoantibodies, or overproduction of antibodies more desirable when produced at normal levels. It is also contemplated herein that aberrant B-cell activity may occur in certain subpopulations of B-cells and not in other subpopulations, or may include inappropriate stimulation of T-cells, such as by inappropriate antigen presentation to T-cells or by other B-cell pathways. Examples of B-cell associated disorders or diseases include a B-cell malignancy or B-cell cancer *(e.g.,* B-cell lymphoma, B-cell leukemia or B-cell myeloma), a disease characterized by autoantibody production (*e.g*., autoimmune diseases) or inflammation or a disease characterized by inappropriate T-cell stimulation caused by inappropriate B-cell antigen presentation to T-cells or caused by other pathways involving B-cells.

"Treatment," "treating" or "ameliorating" refers to either a therapeutic treatment or prophylactic/preventative treatment. A treatment is therapeutic if at least one symptom of disease in an individual receiving treatment improves or a treatment may delay worsening of a progressive disease in an individual, or prevent onset of additional associated diseases.

A "therapeutically effective amount (or dose)" or "effective amount (or dose)" of a specific binding molecule or compound refers to that amount of the compound sufficient to result in amelioration of one or more symptoms of the disease being treated in a statistically significant manner. When referring to an individual active ingredient, administered alone, a therapeutically effective dose refers to that ingredient alone. When referring to a combination, a therapeutically effective dose refers to combined amounts of the active ingredients that result in the therapeutic effect, whether administered serially or simultaneously (in the same formulation or concurrently in separate formulations).

The term "pharmaceutically acceptable" refers to molecular entities and compositions that do not produce allergic or other serious adverse reactions when administered using routes well known in the art.

A "patient in need" refers to a patient at risk of, or suffering from, a disease, disorder or condition that is amenable to treatment or amelioration with a polypeptide heterodimer or a composition thereof provided herein.

The term "immunoglobulin-derived fusion protein," as used herein, refers to a fusion protein that comprises at least one immunoglobulin region, such as a VL, VH, CL, CH1, CH2, CH3, and CH4 domain. The immunoglobulin region may be a wild type immunoglobulin region or an altered immunoglobulin region. Exemplary immunoglobulin-derived fusion proteins include single chain variable antibody fragment (scFv) (*see, e.g.,* Huston et al., Proc. Natl. Acad. Sci. USA 85: 5879-83, 1988), SMIP™ proteins *(see,* U.S. Patent Publication Nos. 2003/0133939, 2003/0118592, and 2005/0136049), PIMS proteins (*see,* PCT Application Publication No. WO 2009/023386), and multi-functional binding proteins (such as SCORPION™ and Xceptor proteins) *(see,* PCT Application Publication No. WO 2007/146968, U.S. Patent Application Publication No. 2006/0051844, and U.S. Patent No. 7,166,707).

Additional definitions are provided throughout the present disclosure.

### Polypeptide Heterodimers

In one aspect, the present disclosure provides a polypeptide heterodimer formed by the association of two different single chain polypeptides. The first single chain polypeptide (SCP-I) comprises, consists essentially of, or consists of from one to four binding domains that specifically bind from one to four targets, a hinge (H-I), an immunoglobulin heterodimerization domain (HD-I), and an Fc region portion (FRP-I), whereas the second single chain polypeptide (SCP-II) comprises, consists essentially of, or consists of from zero to four binding domains that specifically bind from zero to four targets, a hinge (H-II), an immunoglobulin heterodimerization domain (HD-II), and an Fc region portion (FRP-II), provided that the polypeptide heterodimer comprises at least two binding domains that specifically bind to one or more targets, for instance, at least two different targets. The H-I and H-II may have the same sequence, but may be different. The HD-I may comprise an immunoglobulin CH1 region, and the HD-II may comprise an immunoglobulin CL region. Alternatively, the HD-I may comprise an immunoglobulin CL region, and the HD-II may comprise an immunoglobulin CH1 region. The FRP-I and FRP-II may have the same sequence, but may be different. The individual components of the polypeptide heterodimers of the present disclosure are described in detail herein.

### Binding Domains

As indicated above, the polypeptide heterodimer of the present disclosure comprises two single chain polypeptides: One single chain polypeptide comprises from one to four binding domains that specifically bind from one to four targets, and the other single chain polypeptide of the polypeptide heterodimer comprises from zero to four binding domains that bind from zero to four targets. The total number of binding domains of the polypeptide heterodimer ranges from about two to eight, and the total number of different targets that the binding domains bind ranges from about one to eight, for instance, from two to eight, two to four, two to three or two targets.

If a single chain polypeptide of a polypeptide heterodimer comprises a single binding domain, the binding domain may be located either amino or carboxyl terminal to the Fc region portion of the single chain polypeptide. For example, a single chain polypeptide comprising two binding domains may have one binding domain located amino terminal and the other carboxyl terminal to the Fc region portion of the single chain polypeptide, or both binding domains may be amino terminal or both carboxyl terminal to the Fc region portion. In another example, a single chain polypeptide may comprise three binding domains wherein (a) two binding domains are amino terminal on different single chain proteins and the third binding domain is carboxyl terminal to the Fc region portion on either SCP-I or SCP-II, (b) two binding domains are carboxyl terminal on different single chain proteins and the third binding domain is amino terminal to the Fc region portion on either SCP-I or SCP-II. In still a further example, a polypeptide heterodimer may comprise four binding domains, wherein two binding domains are located amino terminal to the Fc region portion on different chains and the other two binding domains are located carboxyl terminal to the Fc region portion on different chains. Alternatively, in any of these embodiments, two binding domains may be linked to each other in tandem and located on either SCP-I or SCP-II or both, depending on the number of binding domains present - the tandem stacking is used when five to eight binding domains combined are present in SCP-I and SCP-II.

Binding of a target by a binding domain modulates the interaction between the target *(e.g.,* a receptor or a ligand) and another molecule. In certain embodiments, the binding of a target *(e.g.,* a receptor) by a binding domain stimulates certain functions of the target (e.g., signal transduction) or brings different targets closer together for a biological effect (e.g., directing T cells to a tumor which in turn activates the T cells). In certain other embodiments, the binding of a target by a binding domain blocks the interaction between the target and another molecule and thus interferes, reduces or eliminates certain functions of the target.

A target molecule, which is specifically bound by a binding domain contained in a polypeptide heterodimer of the present disclosure, may be found on or in association with a cell of interest ("target cell"). Exemplary target cells include cancer cells, cells associated with an autoimmune disease or disorder or with an inflammatory disease or disorder, B-cells and T-cells. A target molecule may also not be associated with a cell. Exemplary target molecules not associated with a cell include soluble proteins, secreted proteins, deposited proteins, and extracellular structural (matrix) proteins.

In certain embodiments, binding domains of polypeptide heterodimers of the present disclosure specifically bind a target selected from T-cell targets, tumor antigens, B-cell targets, pro-inflammatory cytokines or chemokines, pro-oncogenic cytokines or growth factors, angiogenic agents, Fc receptors, transferrin receptor, receptor tyrosine kinases (RTKs), TNFSFRs, or any combination thereof. For instance, polypeptide heterodimers of the present disclosure specifically bind a T-cell target and a tumor target.

In certain embodiments, a binding domain of a polypeptide heterodimer of the present disclosure specifically binds a T-cell target, such as a TCR complex or a component thereof (e.g., TCRα, TCRβ, CD3γ, CD3δ, and CD3ε), CD28, PD-1, HVEM, BTLA, CD80, CD86, GITR, and TGFBR1.

In certain embodiments, a binding domain of polypeptide heterodimer of the present disclosure specifically binds a TCR complex or a component thereof. For example, in certain embodiments, a binding domain specifically binds to an individual human CD3 chain (e.g., human CD3γ chain, human CD3δ chain, and human CD3ε chain) or a combination of two or more of the individual human CD3 chains *(e.g.,* a complex of human CD3γ and human CD3ε or a complex of human CD3δ and human CD3ε). In certain embodiments, a binding domain specifically binds to a human CD3ε chain. In certain other embodiments, a binding domain specifically binds to one or more of human TCRα, human TCRβ, or a heterodimer formed from human TCRα and human TCRβ. In certain other embodiments, a binding domain of the present disclosure binds to a complex formed from one or more human CD3 chains with one or more human TCR chains, such as a complex formed from a human CD3γ chain, a human CD3δ chain, or a human CD3ε chain with a human TCRα chain or a human TCRβ chain. In any of these embodiments, a polypeptide heterodimer of the present disclosure may also bind a tumor target.

In certain embodiments, one or more binding domains of a polypeptide heterodimer of the present disclosure specifically bind a tumor target, including RON, c-Met, CEACAM-6, PSMA, EpCAM, CEA, PCTA-1, STEAP-1, PSCA, ALCAM (CD166), EphA2, CD151, CA-125, MUC-1, MAGE-1, TROP2, IGF1R, CD44v6, CD151, TGFBR2, GHRHR, GHR, IL-6R, gp130, TNFR2, OSMRβ, Patched-1, Frizzled, Robo1, LTβT, CD80, CD81, CD86, CCR5, HER-3, HER-4, EGFR, CEA, MUC2, MUC3, MUC4, MUC5_{AC}, MUC5_{b}, MUC7, βhCG, Lewis-Y, CD33, CD30, ganglioside GD3, 9-O-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP-2, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, B7-H3, or any combination thereof.

In certain embodiments, one or more binding domains of a polypeptide heterodimer of the present disclosure specifically bind a B-cell target, such as CD 19, CD20, CD21, CD22, CD30, CD33, CD37, CD38, CD70, CD79b, HLA-DR, or any combination thereof.

In certain embodiments, one or more binding domains of a polypeptide heterodimer of the present disclosure specifically bind a pro-inflammatory cytokine or chemokine, such as TNFα, IL-6, hyperIL-6, IL-2, IL-1, IL-8, IP-10, IFNγ, IFNα, RANKL, FASL, EGFβ, IL7, IL10, IL17A/F, TWEAK, CSF2, IGF1, IGF2 or BLyS/APRIL, or any combination thereof.

In certain embodiments, one or more binding domains of a polypeptide heterodimer of the present disclosure specifically bind a pro-oncogenic cytokine or growth factor, such as HGF, MSP, EGF (including epiregulin, herregulin, β-regulin, neuregulin), HIF-1α, VEGFA, VEGFB, VEGFC, VEGFD, TNFα, IL-6, hyperIL-6, IL-8, Wnt, sHH, TGFβ, PDGF, or any combination thereof.

In certain embodiments, one or more binding domains of a polypeptide heterodimer of the present disclosure specifically bind an angiogenic agent, such as PDGFR, VEGFR1-4, NRP1, Angiopoietin 2, c-Met or any combination thereof.

In certain embodiments, one or more binding domains of a polypeptide heterodimer of the present disclosure specifically bind an Fc receptor, such as CD64, CD32A, CD32B, CD16, FcRn, or any combination thereof.

In certain embodiments, a binding domain of a polypeptide heterodimer of the present disclosure specifically binds a transferrin receptor, such as CD71.

In certain embodiments, one or more binding domains of a polypeptide heterodimer of the present disclosure specifically binds a receptor tyrosine kinase, such as EGFR, EGFRvIII, ErbB2, ErbB3, ErbB4, IGF1R, EphA2, c-Met, RON, or any combination thereof.

In certain embodiments, one or more binding domains of a polypeptide heterodimer of the present disclosure specifically binds a TNFSFR, such as TNFR1, TNFR2, TWEAKR, TACI, BAFF-R, BCMA, FAS, OX40, GITR, 4-1-BB, LTbetaR, HVEM, RANK, or any combination thereof.

In certain embodiments, one or more binding domains of a polypeptide heterodimer of the present disclosure specifically bind hyperIL-6, IL-10, LIGHT, CD40, PDL1, PDL2, or any combination thereof.

In certain embodiments, one or more binding domains of a polypeptide heterodimer of the present disclosure are an agonist of one of the following molecules: IL-10, HLA-G, HGF, IL-35, PD-1, BTLA, TNFR1, TNFR2, DR4, DR5, TWEAKR, FAS, or any combination thereof.

A binding domain may be any peptide that specifically binds a target of interest. Sources of binding domains include antibody variable regions from various species (which can be formatted as antibodies, sFvs, scFvs, Fabs, or soluble VH domain or domain antibodies), including human, rodent, avian, and ovine. Additional sources of binding domains include variable regions of antibodies from other species, such as camelid (from camels, dromedaries, or llamas; Ghahroudi et al. (1997) FEBS Letters 414(3):521-526; Vincke et al. (2009) Journal of Biological Chemistry (2009) 284:3273-3284; Hamers-Casterman et al. (1993) Nature, 363:446 and Nguyen et al. (1998) J. Mol. Biol., 275:413), nurse sharks (Roux et al. (1998) Proc. Nat'1. Acad. Sci. (USA) 95:11804), spotted ratfish (Nguyen et al. (2002) Immunogenetics, 54:39), or lamprey (Herrin et al., (2008) Proc. Nat'1. Acad. Sci. (USA) 105:2040-2045 and Alder et al. (2008) Nature Immunology 9:319-327). These antibodies can apparently form antigen-binding regions using only heavy chain variable region, *i.e.,* these functional antibodies are homodimers of heavy chains only (referred to as "heavy chain antibodies") (Jespers et al. (2004) Nature Biotechnology 22:1161-1165; Cortez-Retamozo et al. (2004) Cancer Research 64:2853-2857; Baral et al. (2006) Nature Medicine 12:580-584, and Barthelemy et al. (2008) Journal of Biological Chemistry 283:3639-3654).

Exemplary anti-CD3 antibodies from which the binding domain of this disclosure may be derived include Cris-7 monoclonal antibody (Reinherz, E. L. et al. (eds.), Leukocyte typing II., Springer Verlag, New York, (1986)), BC3 monoclonal antibody (Anasetti et al. (1990) J. Exp. Med. 172:1691), OKT3 (Ortho multicenter Transplant Study Group (1985) N. Engl. J. Med. 313:337) and derivatives thereof such as OKT3 ala-ala (Herold et al. (2003) J. Clin. Invest. 11:409), visilizumab (Carpenter et al. (2002) Blood 99:2712), and 145-2C11 monoclonal antibody (Hirsch et al. (1988) J. Immunol. 140: 3766). An exemplary anti-TCR antibody is H57 monoclonal antibody (Lavasani et al. (2007) Scandinavian Journal of Immunology 65:39-47).

An alternative source of binding domains of this disclosure includes sequences that encode random peptide libraries or sequences that encode an engineered diversity of amino acids in loop regions of alternative non-antibody scaffolds, such as fibrinogen domains (*see*, *e.g.,* Weisel et al. (1985) Science 230:1388), Kunitz domains *(see, e.g.,* US Patent No. 6,423,498), ankyrin repeat proteins (Binz et al. (2003) Journal of Molecular Biology 332:489-503 and Binz et al. (2004) Nature Biotechnology 22(5):575-582), fibronectin binding domains (Richards et al. (2003) Journal of Molecular Biology 326:1475-1488; Parker et al. (2005) Protein Engineering Design and Selection 18(9):435-444 and Hackel et al. (2008) Journal of Molecular Biology 381:1238-1252), cysteine-knot miniproteins (Vita et al. (1995) Proc. Nat'1. Acad. Sci. (USA) 92:6404-6408; Martin et al. (2002) Nature Biotechnology 21:71-76 and Huang et al. (2005) Structure 13:755-768), tetratricopeptide repeat domains (Main et al. (2003) Structure 11:497-508 and Cortajarena et al. (2008) ACS Chemical Biology 3:161-166), leucine-rich repeat domains (Stumpp et al. (2003) Journal of Molecular Biology 332:471-487), lipocalin domains (*see, e.g.,* WO 2006/095164, Beste et al. (1999) Proc. Nat'1. Acad. Sci. (USA) 96:1898-1903 and Schönfeld et al. (2009) Proc. Nat'1. Acad. Sci. (USA) 106:8198-8203), V-like domains *(see, e.g.,* US Patent Application Publication No. 2007/0065431), C-type lectin domains (Zelensky and Gready (2005) FEBS J. 272:6179; Beavil et al. (1992) Proc. Nat'1. Acad. Sci. (USA) 89:753-757 and Sato et al. (2003) Proc. Nat'1. Acad. Sci. (USA) 100:7779-7784), mAb² or FCAB™ *(see, e.g.,* PCT Patent Application Publication Nos. WO 2007/098934; WO 2006/072620), or the like (Nord et al. (1995) Protein Engineering 8(6):601-608; Nord et al. (1997) Nature Biotechnology 15:772-777; Nord et al. (2001) European Journal of Biochemistry 268(15):4269-4277 and Binz et al. (2005) Nature Biotechnology 23:1257-1268).

Binding domains of this disclosure can be generated as described herein or by a variety of methods known in the art *(see, e.g.,* U.S. Patent Nos. 6,291,161 and 6,291,158). For example, binding domains of this disclosure may be identified by screening a Fab phage library for Fab fragments that specifically bind to a target of interest (*see* Hoet et al. (2005) Nature Biotechnol. 23:344). Additionally, traditional strategies for hybridoma development using a target of interest as an immunogen in convenient systems (e.g., mice, HUMAB MOUSE®, TC MOUSE™, KM-MOUSE^{®}, llamas, chicken, rats, hamsters, rabbits, *etc.*) can be used to develop binding domains of this disclosure.

In certain embodiments, a polypeptide heterodimer comprises a CD86 binding domain, such as a CTLA4 ectodomain, a CD28 ectodomain, or an immunoglobulin variable region binding domain (such as a scFv) specific for CD86 *(e.g.,* from monoclonal antibodies 3D1 or FUN1). In some embodiments, less than an entire ectodomain is used. For example, domains within the CTLA4 ectodomain that bind CD86 and prevent binding of CD86 to CD28 can be used. The CD86 binding domain can block binding of CD86 to CD28 and thereby downregulate T-cell activation.

In certain embodiments, a polypeptide heterodimer comprises an IL-10 agonist, such as IL-10, monoIL-10, or a functional region thereof. "MonoIL-10" refers to an IL-10 molecule having a short linker (GGGSGG, SEQ ID NO:760) that separates the two subdomains of the molecule (amino and carboxyl end domains) so that these subdomains can form an intramolecular dimer.

In certain embodiments, a polypeptide heterodimer comprises an HLA-G agonist, such as an HLA-G5, an HLA-G1, an HLA-G mutein, or a functional region thereof; an ectodomain of an HLA-G5, an HLA-G1 or an HLA-G mutein; or an immunoglobulin variable region binding domain (such as an scFv) specific for ILT2, ILT4 or KIR2DL4.

In certain embodiments, a polypeptide heterodimer comprises an HGF agonist, such as an HGF or a sub-domain thereof.

In certain embodiments, a polypeptide heterodimer comprises an IL35 agonist, such as an immunoglobulin variable region binding domain (such as an scFv) specific for IL35R or IL35, or a functional region thereof.

In certain embodiments, a polypeptide heterodimer comprises a LIGHT antagonist, such as an immunoglobulin variable region binding domain (such as an scFv) specific for LIGHT, or a HVEM ectodomain or a functional region thereof.

In certain embodiments, a polypeptide heterodimer comprises a PD-1 agonist, such as an immunoglobulin variable region binding domain (such as an scFv) specific for PD-1, or a PD-1 ligand (e.g. PD-L1 or PD-L2) or a functional region thereof.

In certain embodiments, a polypeptide heterodimer comprises a BTLA agonist, such as an immunoglobulin-like variable region binding domain (such as an scFv) specific for BTLA, or a HVEM ectodomain or a functional region thereof.

In certain embodiments, a polypeptide heterodimer comprises a GITRL antagonist, such as an immunoglobulin-like variable region binding domain (such as an scFv) specific for GITRL, or a GITR ectodomain, soluble GITR, or a functional region thereof.

In certain embodiments, a polypeptide heterodimer comprises a CD40 antagonist, such as an immunoglobulin-like variable region binding domain (such as an scFv) specific for CD40.

In some embodiments, a binding domain is a single chain Fv fragment (scFv) that comprises VH and VL regions specific for a target of interest. In certain embodiments, the V_{H} and V_{L} domains are human. Exemplary VH regions include the VH region of 2E12 (anti-CD28) scFv as set forth in SEQ ID NO:106, the VH region of P2C2 (anti-CD79b) scFv as set forth in SEQ ID NO:184, the VH region of 5D5 (anti-c-Met) scFv as set forth in SEQ ID NO:258, the VH region of A2 (anti-hyperIL-6) scFv as set forth in SEQ ID NO:80, the VH region of 3D1 (anti-CD86) scFv as set forth in SEQ ID NO:92, the VH region of MET021 (anti-c-Met) scFv as set forth in SEQ ID NO:100, the VH region of G19-4 (anti-CD3) scFv as set forth in SEQ ID NO:103, the VH region of HD37 (anti-CD 19) scFv as set forth in SEQ ID NO:117, the VH region of M0042 (anti-HLA-DR) scFv as set forth in SEQ ID NO:121, the VH region of BMA031 (anti-TCR) scFv as set forth in SEQ ID NO:828, the VH region of OKT3-M (anti-CD3) scFv as set forth in SEQ ID NO:831, and the VH region of HuM291 (anti-CD3) scFv as set forth in SEQ ID NO:835. The nucleotide sequences encoding the VH regions of the A2 (anti-hyperIL-6) and 3D1 (anti-CD86) scFv's are set forth in SEQ ID NOS:79 and 91, respectively.

Exemplary VL domains are the VL region of 2E12 (anti-CD28) scFv as set forth in SEQ ID NO:107, the VL region of P2C2 (anti-CD79b) scFv as set forth in SEQ ID NO:182, the VL region of 5D5 (anti-c-Met) scFv as set forth in SEQ ID NO:259, the VL region of A2 (anti-hyper IL-6) scFv as set forth in SEQ ID NO:84, the VL region of 3D1 (anti-CD86) scFv as set forth in SEQ ID NO:96, the VL region of MET021 (anti-c-Met) scFv as set forth in SEQ ID NO:101, the VL region of G19-4 (anti-CD3) scFv as set forth in SEQ ID NO:104, the VL region of HD37 (anti-CD19) scFv as set forth in SEQ ID NO:119, the VL region of M0042 (anti-HLA-DR) scFv as set forth in SEQ ID NO:122, the VL region of BMA031 (anti-TCR) scFv as set forth in SEQ ID NO:829, the VL region of OKT3-M (anti-CD3) scFv as set forth in SEQ ID NO:833, and the VL region of HuM291 (anti-CD3) scFv as set forth in SEQ ID NO:836. The nucleotide sequences encoding the VL regions of the A2 (anti-hyperIL-6) and 3D1 (anti-CD86) scFv's are set forth in SEQ ID NOS:83 and 95, respectively.

In some embodiments, a binding domain is a single chain Fv fragment (scFv) that comprises V_{H} and V_{L} domains specific for a TCR complex or a component thereof. In certain embodiments, the V_{H} and V_{L} domains are human or humanized V_{H} and V_{L} domains. Exemplary V_{H} domains include BC3 (anti-CD3) V_{H}, OKT3 (anti-CD3) V_{H}, H57 (anti-TCR) V_{H}, and 2C11 (anti-CD3) V_{H} domains as set forth in SEQ ID NOS:301, 303, 313, and 317, respectively. Further exemplary V_{H} domains include Cris-7 (anti-CD3) V_{H} domains, such as those set forth in SEQ ID NOS:327 and 331-333. Exemplary V_{L} domains are BC3 (anti-CD3) V_{L}, OKT3 (anti-CD3) V_{L}, H57 (anti-TCR) V_{L}, and 2C11 (anti-CD3) V_{L} domains as set forth in SEQ ID NOS:302, 304, 315 and 318, respectively. Further exemplary V_{L} domains include Cris-7 (anti-CD3) V_{L} domains, such as those set forth in SEQ ID NOS:328, 329 and 330.

In certain embodiments, a binding domain comprises or is a sequence that is at least 90%, at least 91%, at least 92%, at least 93%, at least 94%, at least 95%, at least 96%, at least 97%, at least 98%, at least 99%, at least 99.5%, or 100% identical to an amino acid sequence of a light chain variable region (V_{L}) or to a heavy chain variable region (V_{H}), or both, wherein each CDR comprises zero changes or at most one, two, or three changes, from a monoclonal antibody or fragment or derivative thereof that specifically binds to a target of interest (e.g., c-Met, RON, CD28, CD79b, CD3ε, TCRα, TCRβ, hyper IL-6, CD86, CD19, and HLA-DR) and such mutant or derivative still binds its target.

In certain embodiments, a binding domain VH or VL region of the present disclosure can be derived from or based on a VH or VL of a known monoclonal antibody and contains one or more *(e.g.,* 2, 3, 4, 5, 6, 7, 8, 9, 10) insertions, one or more *(e.g.,* 2, 3, 4, 5, 6, 7, 8, 9, 10) deletions, one or more *(e.g.,* 2, 3, 4, 5, 6, 7, 8, 9, 10) amino acid substitutions (*e.g*., conservative amino acid substitutions or non-conservative amino acid substitutions), or a combination of the above-noted changes, when compared with the VH or VL of a known monoclonal antibody. The insertion(s), deletion(s) or substitution(s) may be anywhere in the VH region, including at the amino- or carboxy-terminus or both ends of this region, provided that each CDR comprises zero changes or at most one, two, or three changes and provided that a binding domain containing the modified VH or VL region can still specifically bind its target with an affinity about the same as the wild type binding domain.

The VH and VL domains may be arranged in either orientation *(i.e.,* from amino-terminus to carboxyl terminus, VH-VL or VL-VH) and may be joined by an amino acid sequence (*e.g*., having a length of about five to about 35 amino acids) capable of providing a spacer function such that the two sub-binding domains can interact to form a functional binding domain. In certain embodiments, an amino acid sequence that joins the VH and VL domains (also referred to herein as a "linker") includes those belonging to the (GlyₙSer) family, such as (Gly₃Ser)ₙ(Gly₄Ser)₁, (Gly₃Ser)₁(Gly₄Ser)ₙ, (Gly₃Ser)ₙ(Gly₄Ser)ₙ, or (Gly₄Ser)ₙ, wherein n is an integer of 1 to 5. In certain embodiments, the linker is GGGGSGGGGS GGGGS (SEQ ID NO:183) or GGGGSGGGGSGGGGSGGGGS (SEQ ID NO:108). An additional exemplary linker is GGGGSGGGGSGGGGAS (SEQ ID NO:739). In certain embodiments, these (GlyₙSer)-based linkers are used to link the VH and VL domains in a binding domain, but are not used to link a binding domain to an immunoglobulin heterodimerization domain or to an Fc region portion.

Exemplary binding domains specific for CD28 include a 2E12 scFv as set forth in SEQ ID NO:109, binding domains specific for CD79b include a P2C2 scFv as set forth in SEQ ID NO:185; binding domains specific for c-Met include a 5D5 scFv as set forth in SEQ ID NO:257; binding domains specific for RON include a 4C04 scFv as set forth in SEQ ID NO:261 and a 11H09 scFv as set forth in SEQ ID NO:265; binding domains specific for hyperIL-6 include an A2 scFv as set forth in SEQ ID NO:86; binding domains specific for CD86 include a 3D1 scFv as set forth in SEQ ID NO:98; binding domains specific for HLA-DR include an M0042 scFv as set forth in SEQ ID NO:120; binding domains specific for CD3 include a G19-4 scFv as set forth in SEQ ID NO:102, an OKT3-M scFv as set forth in SEQ ID NO:834, and a HuM291 scFv as set forth in SEQ ID NO:837; binding domain specific for CD19 include a H37 scFv as set forth in SEQ ID NO:105; and binding domains specific for c-Met include an MET021 scFv as set forth in SEQ ID NO:120 (with the light chain CDR1, CDR2, CDR3 and heavy chain CDR1, CDR2 and CDR3 as set forth in SEQ ID NOS:296-298 and 464-466, respectively). The nucleotide sequences encoding the A2 (anti-hyperIL6) and 3D1 (anti-CD86) scFv's are set forth in SEQ ID NOS:85 and 97, respectively. Exemplary binding domains that bind a TCR complex or a component thereof include a BMA031 scFv as set forth in SEQ ID NO:830 and other scFv's as set forth in SEQ ID NOS:310, 311, 312, 319, and 334-340.

Additional exemplary binding domains include a PDL2 ectodomain as set forth in SEQ ID NO:88 and monoIL-10 as set forth in SEQ ID NO:90. The nucleotide sequences encoding the PDL2 ectodomain and monoIL-10 are set forth in SEQ ID NOS:87 and 89, respectively.

The light chain amino acid sequence of the 4C04 (anti-RON) scFv is set forth in SEQ ID NO:602, and its CDR1, CDR2, and CDR3 are set forth in SEQ ID NOS:604-606, respectively. The heavy chain amino acid sequence of the 4C04 (anti-RON) scFv is set forth in SEQ ID NO:603, and its CDR1, CDR2, and CDR3 are set forth in SEQ ID NOS:607-609, respectively.

The light chain amino acid sequence of the 11H09 (anti-RON) scFv is set forth in SEQ ID NO:610, and its CDR1, CDR2, and CDR3 are set forth in SEQ ID NOS:612-614, respectively. The heavy chain amino acid sequence of the 11H09 (anti-RON) scFv is set forth in SEQ ID NO:611, and its CDR1, CDR2, and CDR3 are set forth in SEQ ID NOS:615-617, respectively.

A binding domain may be located either at amino terminal or carboxyl terminal to the Fc region portion of a single chain polypeptide of the present disclosure. In certain embodiments, the binding domain is located at the amino terminus of a single chain polypeptide. In certain other embodiments, the binding domain is located at the carboxyl terminus of a single chain polypeptide. In certain other embodiments, a binding domain is located at both the amino and carboxyl terminus of a single chain polypeptide.

### Heterodimerization Domains

As indicated above, a polypeptide heterodimer of the present disclosure comprises an immunoglobulin heterodimerization domain in each polypeptide chain. The immunoglobulin heterodimerization domains in the two single chain polypeptides of a polypeptide heterodimer are different from each other and thus may be differentially modified to facilitate heterodimerization of both chains and to minimize homodimerization of either chain. As shown in the examples, immunoglobulin heterodimerization domains provided herein allow for efficient heterodimerization between different polypeptides and facilitate purification of the resulting polypeptide heterodimers.

As provided herein, immunoglobulin heterodimerization domains useful for promoting heterodimerization of two different single chain polypeptides (e.g., one short and one long) according to the present disclosure include immunoglobulin CH1 and CL domains, for instance, human CH1 and CL domains. In certain embodiments, an immunoglobulin heterodimerization domain is a wild type CH1 region, such as a wild type IgG1, IgG2, IgG3, IgG4, IgA1, IgA2, IgD, IgE, or IgM CH1 region. In further embodiments, an immunoglobulin heterodimerization domain is a wild type human IgG1, IgG2, IgG3, IgG4, IgA1, IgA2, IgD, IgE, or IgM CH1 region as set forth in SEQ ID NOS:114, 186-192 and 194, respectively. In certain embodiments, an immunoglobulin heterodimerization domain is a wild type human IgG1 CH1 region as set forth in SEQ ID NO:114.

In further embodiments, an immunoglobulin heterodimerization domain is an altered immunoglobulin CH1 region, such as an altered IgG1, IgG2, IgG3, IgG4, IgA1, IgA2 IgD, IgE, or IgM CH1 region. In certain embodiments, an immunoglobulin heterodimerization domain is an altered human IgG1, IgG2, IgG3, IgG4, IgA1, IgA2, IgD, IgE, or IgM CH1 region. In still further embodiments, a cysteine residue of a wild type CH1 region *(e.g.,* a human CH1) involved in forming a disulfide bond with a wild type immunoglobulin CL domain *(e.g.,* a human CL) is deleted or substituted in the altered immunoglobulin CH1 region such that a disulfide bond is not formed between the altered CH1 region and the wild type CL domain.

In certain embodiments, an immunoglobulin heterodimerization domain is a wild type CL domain, such as a wild type Cκ domain or a wild type Cλ domain. In particular embodiments, an immunoglobulin heterodimerization domain is a wild type human Cκ or human Cλ domain as set forth in SEQ ID NOS:112 and 113, respectively. In further embodiments, an immunoglobulin heterodimerization domain is an altered immunoglobulin CL domain, such as an altered Cκ or Cλ domain, for instance, an altered human Cκ or human Cλ domain.

In certain embodiments, a cysteine residue of a wild type CL domain *(e.g.,* a human CL) involved in forming a disulfide bond with a wild type immunoglobulin CH1 region *(e.g.,* a human CH1) is deleted or substituted in the altered immunoglobulin CL domain. Such altered CL domains may further comprise an amino acid deletion at their amino termini. An exemplary Cκ domain is set forth in SEQ ID NO:141, in which the first arginine and the last cysteine of the wild type human Ck domain are both deleted. In certain embodiments, only the last cysteine of the wild type human Ck domain is deleted in the altered Ck domain because the first arginine deleted from the wild type human Ck domain may be provided by a linker that has an arginine at its carboxyl terminus and links the amino terminus of the altered Ck domain with another domain (e.g., an Fc region portion). An exemplary Cλ domain is set forth in SEQ ID NO:140, in which the first arginine of a wild type human Cλ domain is deleted and the cysteine involved in forming a disulfide bond with a cysteine in a CH1 region is substituted by a serine.

In further embodiments, an immunoglobulin heterodimerization domain is an altered Cκ domain that contains one or more amino acid substitutions, as compared to a wild type Cκ domain, at positions that may be involved in forming the interchain-hydrogen bond network at a Cκ-Cκ interface. For example, in certain embodiments, an immunoglobulin heterodimerization domain is an altered human Cκ domain having one or more amino acids at positions N29, N30, Q52, V55, T56, S68 or T70 that are substituted with a different amino acid. The numbering of the amino acids is based on their positions in the altered human Cκ sequence as set forth in SEQ ID NO:141. In certain embodiments, an immunoglobulin heterodimerization domain is an altered human Cκ domain having one, two, three or four amino acid substitutions at positions N29, N30, V55, or T70. The amino acid used as a substitute at the above-noted positions may be an alanine, or an amino acid residue with a bulk side chain moiety such as arginine, tryptophan, tyrosine, glutamate, glutamine, or lysine. Additional amino acid residues that may be used to substitute amino acid residues of the wild type human Ck sequence at the above noted positions (*e.g.,* N30) include aspartate, methionine, serine and phenyalanine. Exemplary altered human Cκ domains are set forth in SEQ ID NOS: 142-178. Altered human Cκ domains are those that facilitate heterodimerization with a CH1 region, but minimize homodimerization with another Cκ domain. Representative altered human Cκ domains are set forth in SEQ ID NOS:160 (N29W V55A T70A), 161 (N29Y V55A T70A), 202 (T70E N29A N30A V55A), 167 (N30R V55A T70A), 168 (N30K V55A T70A), 170 (N30E V55A T70A), 172 (V55R N29A N30A), 175 (N29W N30Y V55A T70E), 176 (N29Y N30Y V55A T70E), 177 (N30E V55A T70E), 178 (N30Y V55A T70E), 838 (N30D V55A T70E), 839 (N30M V55A T70E), 840 (N30S V55A T70E), and 841 (N30F V55A T70E).

In certain embodiments, in addition to or alternative to the mutations in Ck domains described herein, both the immunoglobulin heterodimerization domains (*i.e*., immunoglobulin CH1 and CL domains) of a polypeptide heterodimer have mutations so that the resulting immunoglobulin heterodimerization domains form salt bridges (*i.e*., ionic interactions) between the amino acid residues at the mutated sites. For example, the immunoglobulin heterodimerization domains of a polypeptide heterodimer may be a mutated CH1 domain in combination with a mutated Ck domain. In the mutated CH1 domain, valine at position 68 (V68) of the wild type human CH1 domain is substituted by an amino acid residue having a negative charge (*e.g.,* aspartate or glutamate), whereas leucine at position 29 (L29) of a mutated human Ck domain in which the first arginine and the last cysteine have been deleted is substituted by an amino acid residue having a positive charge (*e.g.,* lysine, arginine or histidine). The charge-charge interaction between the amino acid residue having a negative charge of the resulting mutated CH1 domain and the amino acid residue having a positive charge of the resulting mutated Ck domain forms a salt bridge, which stabilizes the heterodimeric interface between the mutated CH1 and Ck domains. Alternatively, V68 of the wild type CH1 may be substituted by an amino acid residue having a positive charge, whereas L29 of a mutated human Ck domain in which the first arginine and the last cysteine have been deleted may be substituted by an amino acid residue having a negative charge. Exemplary mutated CH1 sequences in which V68 is substituted by an amino acid with either a negative or positive charge are set forth in SEQ ID NOS:844 and 845. Exemplary mutated Ck sequences in which L29 is substituted by an amino acid with either a negative or positive charge are set forth in SEQ ID NOS:842 and 843.

Positions other than V68 of human CH1 domain and L29 of human Ck domain may be substituted with amino acids having opposite charges to produce ionic interactions between the amino acids in addition or alternative to the mutations in V68 of CH1 domain and L29 of Ck domain. Such positions can be identified by any suitable method, including random mutagenesis, analysis of the crystal structure of the CH1-Ck pair to identify amino acid residues at the CH1-Ck interface, and further identifying suitable positions among the amino acid residues at the CH1-Ck interface using a set of criteria (*e.g.,* propensity to engage in ionic interactions, proximity to a potential partner residue, *etc*.).

In certain embodiments, polypeptide heterodimers of the present disclosure contain only one pair of immunoglobulin heterodimerization domains. For example, a first chain of a polypeptide heterodimer may comprise a CH1 region as an immunoglobulin heterodimerization domain, while a second chain may comprise a CL domain *(e.g.,* a Cκ or Cλ) as an immunoglobulin heterodimerization domain. Alternatively, a first chain may comprise a CL region *(e.g.,* a Cκ or Cλ) as an immunoglobulin heterodimerization domain, while a second chain may comprise a CH1 region as an immunoglobulin heterodimerization domain. As set forth herein, the immunoglobulin heterodimerization domains of the first and second chains are capable of associating to form a polypeptide heterodimer of this disclosure.

In certain other embodiments, polypeptide heterodimers of the present disclosure may have two pairs of immunoglobulin heterodimerization domains. For example, a first chain of a polypeptide heterodimer may comprise two CH1 regions, while a second chain may have two CL domains that associate with the two CH1 regions in the first chain. Alternatively, a first chain may comprise two CL domains, while a second chain may have two CH1 regions that associate with the two CL domains in the first chain. In certain embodiments, a first chain polypeptide comprises a CH1 region and a CL domain, while a second chain polypeptide comprises a CL domain and a CH1 region that associate with the CH1 region and the CL domain, respectively, of the first chain polypeptide.

In the embodiments where a polypeptide heterodimer comprises only one heterodimerization pair (*i*.*e*., one immunoglobulin heterodimerization domain in each chain), the immunoglobulin heterodimerization domain of each chain may be located amino terminal to the Fc region portion of that chain. Alternatively, the immunoglobulin heterodimerization domain in each chain may be located carboxyl terminal to the Fc region portion of that chain.

In the embodiments where a polypeptide heterodimer comprises two heterodimerization pairs (*i*.*e*., two immunoglobulin heterodimerization domains in each chain), both immunoglobulin heterodimerization domains in each chain may be located amino terminal to the Fc region portion of that chain. Alternatively, both immunoglobulin heterodimerization domains in each chain may be located carboxyl terminal to the Fc region portion of that chain. In further embodiments, one immunoglobulin heterodimerization domain in each chain may be located amino terminal to the Fc region portion of that chain, while the other immunoglobulin heterodimerization domain of each chain may be located carboxyl terminal to the Fc region portion of that chain. In other words, in those embodiments, the Fc region portion is interposed between the two immunoglobulin heterodimerization domains of each chain.

### Fc region portion

As indicated herein, polypeptide heterodimers of the present disclosure comprise an Fc region constant domain portion (also referred to as an Fc region portion) in each polypeptide chain. The inclusion of an Fc region portion slows clearance of the heterodimers from circulation after administration to a subject. By mutations or other alterations, the Fc region portion further enables relatively easy modulation of heterodimer polypeptide effector functions (*e.g.,* ADCC, ADCP, CDC, complement fixation and binding to Fc receptors), which can either be increased or decreased depending on the disease being treated, as known in the art and described herein. In certain embodiments, an Fc region portion of polypeptide heterodimers of the present disclosure will be capable of mediating one or more of these effector functions.

An Fc region portion present in single chain polypeptides that form part of the polypeptide heterodimers of the present disclosure may comprise a CH2 domain, a CH3 domain, a CH4 domain or any combination thereof. For example, an Fc region portion may comprise a CH2 domain, a CH3 domain, both CH2 and CH3 domains, both CH3 and CH4 domains, two CH3 domains, a CH4 domain, or two CH4 domains.

A CH2 domain that may form an Fc region portion of a single chain polypeptide of a heterodimer of the present disclosure may be a wild type immunoglobulin CH2 domain or an altered immunoglobulin CH2 domain thereof from certain immunoglobulin classes or subclasses (*e.g.,* IgG1, IgG2, IgG3, IgG4, IgA1, IgA2, or IgD) and from various species (including human, mouse, rat, and other mammals).

In certain embodiments, a CH2 domain is a wild type human immunoglobulin CH2 domain, such as wild type CH2 domains of human IgG1, IgG2, IgG3, IgG4, IgA1, IgA2, or IgD, as set forth in SEQ ID NOS:115, 199-201 and 195-197, respectively. In certain embodiments, the CH2 domain is a wild type human IgG1 CH2 domain as set forth in SEQ ID NO:115.

In certain embodiments, a CH2 domain is an altered immunoglobulin CH2 region *(e.g.,* an altered human IgG1 CH2 domain) that comprises an amino acid substitution at the asparagine of position 297 (*e.g.,* asparagine to alanine). Such an amino acid substitution reduces or eliminates glycosylation at this site and abrogates efficient Fc binding to FcγR and C1q. The sequence of an altered human IgG1 CH2 domain with an Asn to Ala substitution at position 297 is set forth in SEQ ID NO:324.

In certain embodiments, a CH2 domain is an altered immunoglobulin CH2 region *(e.g.,* an altered human IgG1 CH2 domain) that comprises at least one substitution or deletion at positions 234 to 238. For example, an immunoglobulin CH2 region can comprise a substitution at position 234, 235, 236, 237 or 238, positions 234 and 235, positions 234 and 236, positions 234 and 237, positions 234 and 238, positions 234-236, positions 234, 235 and 237, positions 234, 236 and 238, positions 234, 235, 237, and 238, positions 236-238, or any other combination of two, three, four, or five amino acids at positions 234-238. In addition or alternatively, an altered CH2 region may comprise one or more (*e.g.,* two, three, four or five) amino acid deletions at positions 234-238, for instance, at one of position 236 or position 237 while the other position is substituted. The above-noted mutation(s) decrease or eliminate the antibody-dependent cell-mediated cytotoxicity (ADCC) activity or Fc receptor-binding capability of a polypeptide heterodimer that comprises the altered CH2 domain. In certain embodiments, the amino acid residues at one or more of positions 234-238 has been replaced with one or more alanine residues. In further embodiments, only one of the amino acid residues at positions 234-238 have been deleted while one or more of the remaining amino acids at positions 234-238 can be substituted with another amino acid (*e.g.,* alanine or serine).

In certain other embodiments, a CH2 domain is an altered immunoglobulin CH2 region (*e.g.,* an altered human IgG1 CH2 domain) that comprises one or more amino acid substitutions at positions 253, 310, 318, 320, 322, and 331. For example, an immunoglobulin CH2 region can comprise a substitution at position 253, 310, 318, 320, 322, or 331, positions 318 and 320, positions 318 and 322, positions 318, 320 and 322, or any other combination of two, three, four, five or six amino acids at positions 253, 310, 318, 320, 322, and 331. The above-noted mutation(s) decrease or eliminate the complement-dependent cytotoxicity (CDC) of a polypeptide heterodimer that comprises the altered CH2 domain.

In certain other embodiments, in addition to the amino acid substitution at position 297, an altered CH2 region (*e.g.,* an altered human IgG1 CH2 domain) can further comprise one or more (*e.g.,* two, three, four, or five) additional substitutions at positions 234-238. For example, an immunoglobulin CH2 region can comprise a substitution at positions 234 and 297, positions 234, 235, and 297, positions 234, 236 and 297, positions 234-236 and 297, positions 234, 235, 237 and 297, positions 234, 236, 238 and 297, positions 234, 235, 237, 238 and 297, positions 236-238 and 297, or any combination of two, three, four, or five amino acids at positions 234-238 in addition to position 297. In addition or alternatively, an altered CH2 region may comprise one or more (*e.g.,* two, three, four or five) amino acid deletions at positions 234-238, such as at position 236 or position 237. The additional mutation(s) decreases or eliminates the antibody-dependent cell-mediated cytotoxicity (ADCC) activity or Fc receptor-binding capability of a polypeptide heterodimer that comprises the altered CH2 domain. In certain embodiments, the amino acid residues at one or more of positions 234-238 have been replaced with one or more alanine residues. In further embodiments, only one of the amino acid residues at positions 234-238 has been deleted while one or more of the remaining amino acids at positions 234-238 can be substituted with another amino acid (*e.g.,* alanine or serine).

In certain embodiments, in addition to one or more (*e.g.,* 2, 3, 4, or 5) amino acid substitutions at positions 234-238, a mutated CH2 region (*e.g.,* an altered human IgG1 CH2 domain) in a fusion protein of the present disclosure may contain one or more *(e.g.,* 2, 3, 4, 5, or 6) additional amino acid substitutions (*e.g.,* substituted with alanine) at one or more positions involved in complement fixation (*e.g.,* at positions I253, H310, E318, K320, K322, or P331). Examples of mutated immunoglobulin CH2 regions include human IgG1, IgG2, IgG4 and mouse IgG2a CH2 regions with alanine substitutions at positions 234, 235, 237 (if present), 318, 320 and 322. An exemplary mutated immunoglobulin CH2 region is mouse IGHG2c CH2 region with alanine substitutions at L234, L235, G237, E318, K320, and K322 (SEQ ID NO:314).

In still further embodiments, in addition to the amino acid substitution at position 297 and the additional deletion(s) or substitution(s) at positions 234-238, an altered CH2 region *(e.g.,* an altered human IgG1 CH2 domain) can further comprise one or more (*e.g.,* two, three, four, five, or six) additional substitutions at positions 253, 310, 318, 320, 322, and 331. For example, an immunoglobulin CH2 region can comprise a (1) substitution at position 297, (2) one or more substitutions or deletions or a combination thereof at positions 234-238, and one or more *(e.g.,* 2, 3, 4, 5, or 6) amino acid substitutions at positions I253, H310, E318, K320, K322, and P331, such as one, two, three substitutions at positions E318, K320 and K322. The amino acids at the above-noted positions may be substituted by alanine or serine.

In certain embodiments, an immunoglobulin CH2 region polypeptide comprises: (i) an amino acid substitution at the asparagines of position 297 and one amino acid substitution at position 234, 235, 236 or 237; (ii) an amino acid substitution at the asparagine of position 297 and amino acid substitutions at two of positions 234-237; (iii) an amino acid substitution at the asparagine of position 297 and amino acid substitutions at three of positions 234-237; (iv) an amino acid substitution at the asparagine of position 297, amino acid substitutions at positions 234, 235 and 237, and an amino acid deletion at position 236; (v) amino acid substitutions at three of positions 234-237 and amino acid substitutions at positions 318, 320 and 322; or (vi) amino acid substitutions at three of positions 234-237, an amino acid deletion at position 236, and amino acid substitutions at positions 318, 320 and 322.

Exemplary altered immunoglobulin CH2 regions with amino acid substitutions at the asparagine of position 297 include: human IgG1 CH2 region with alanine substitutions at L234, L235, G237 and N297 and a deletion at G236 (SEQ ID NO:325), human IgG2 CH2 region with alanine substitutions at V234, G236, and N297 (SEQ ID NO:326), human IgG4 CH2 region with alanine substitutions at F234, L235, G237 and N297 and a deletion of G236 (SEQ ID NO:322), human IgG4 CH2 region with alanine substitutions at F234 and N297 (SEQ ID NO:343), human IgG4 CH2 region with alanine substitutions at L235 and N297 (SEQ ID NO:344), human IgG4 CH2 region with alanine substitutions at G236 and N297 (SEQ ID NO:345), and human IgG4 CH2 region with alanine substitutions at G237 and N297 (SEQ ID NO:346).

In certain embodiments, in addition to the amino acid substitutions described above, an altered CH2 region *(e.g.,* an altered human IgG1 CH2 domain) may contain one or more additional amino acid substitutions at one or more positions other than the above-noted positions. Such amino acid substitutions may be conservative or non-conservative amino acid substitutions. For example, in certain embodiments, P233 may be changed to E233 in an altered IgG2 CH2 region (*see*, *e.g.,* SEQ ID NO:326). In addition or alternatively, in certain embodiments, the altered CH2 region may contain one or more amino acid insertions, deletions, or both. The insertion(s), deletion(s) or substitution(s) may anywhere in an immunoglobulin CH2 region, such as at the N- or C-terminus of a wild type immunoglobulin CH2 region resulting from linking the CH2 region with another region *(e.g.,* a binding domain or an immunoglobulin heterodimerization domain) via a hinge.

In certain embodiments, an altered CH2 region in a polypeptide heterodimer of the present disclosure comprises or is a sequence that is at least 90%, at least 91%, at least 92%, at least 93%, at least 94%, at least 95%, at least 96%, at least 97%, at least 98%, at least 99% identical to a wild type immunoglobulin CH2 region, such as the CH2 region of wild type human IgG1, IgG2, or IgG4, or mouse IgG2a *(e.g.,* IGHG2c).

An altered immunoglobulin CH2 region in a polypeptide heterodimer of the present disclosure may be derived from a CH2 region of various immunoglobulin isotypes, such as IgG1, IgG2, IgG3, IgG4, IgA1, IgA2, and IgD, from various species (including human, mouse, rat, and other mammals). In certain embodiments, an altered immunoglobulin CH2 region in a fusion protein of the present disclosure may be derived from a CH2 region of human IgG1, IgG2 or IgG4, or mouse IgG2a *(e.g.,* IGHG2c), whose sequences are set forth in SEQ ID NOS:115, 199, 201 and 320.

In certain embodiments, an altered CH2 domain is a human IgG1 CH2 domain with alanine substitutions at positions 235, 318, 320, and 322 (*i.e*., a human IgG1 CH2 domain with L235A, E318A, K320A and K322A substitutions) (SEQ ID NO:595), and optionally an N297 mutation (e.g., to alanine). In certain other embodiments, an altered CH2 domain is a human IgG1 CH2 domain with alanine substitutions at positions 234, 235, 237, 318, 320 and 322 (*i.e*., a human IgG1 CH2 domain with L234A, L235A, G237A, E318A, K320A and K322A substitutions) (SEQ ID NO:596), and optionally an N297 mutation (*e.g.,* to alanine).

In certain embodiments, an altered CH2 domain is an altered human IgG1 CH2 domain with mutations known in the art that enhance immunological activities such as ADCC, ADCP, CDC, complement fixation, Fc receptor binding, or any combination thereof.

The CH3 domain that may form an Fc region portion of a single chain polypeptide of a heterodimer of the present disclosure may be a wild type immunoglobulin CH3 domain or an altered immunoglobulin CH3 domain thereof from certain immunoglobulin classes or subclasses (*e.g.,* IgG1, IgG2, IgG3, IgG4, IgA1, IgA2, IgD, IgE, IgM) of various species (including human, mouse, rat, and other mammals). In certain embodiments, a CH3 domain is a wild type human immunoglobulin CH3 domain, such as wild type CH3 domains of human IgG1, IgG2, IgG3, IgG4, IgA1, IgA2, IgD, IgE, or IgM as set forth in SEQ ID NOS:116, 208-210, 204-207, and 212, respectively. In certain embodiments, the CH3 domain is a wild type human IgG1 CH3 domain as set forth in SEQ ID NO:116. In certain embodiments, a CH3 domain is an altered human immunoglobulin CH3 domain, such as an altered CH3 domain based on or derived from a wild-type CH3 domain of human IgG1, IgG2, IgG3, IgG4, IgA1, IgA2, IgD, IgE, or IgM antibodies. For example, an altered CH3 domain may be a human IgG1 CH3 domain with one or two mutations at positions H433 and N434 (positions are numbered according to EU numbering). The mutations in such positions may be involved in complement fixation. In certain other embodiments, an altered CH3 domain may be a human IgG1 CH3 domain but with one or two amino acid substitutions at position F405 or Y407. The amino acids at such positions are involved in interacting with another CH3 domain. In certain embodiments, an altered CH3 domain may be an altered human IgG1 CH3 domain with its last lysine deleted. The sequence of this altered CH3 domain is set forth in SEQ ID NO:761.

In certain embodiments, a polypeptide heterodimer comprises a CH3 pair that comprises so called "knobs-into-holes" mutations (*see,* Marvin and Zhu, Acta Pharmacologica Sinica 26:649-58, 2005; Ridgway et al., Protein Engineering 9:617-21, 1966). More specifically, mutations may be introduced into each of the two CH3 domains so that the steric complementarity required for CH3/CH3 association obligates these two CH3 domains to pair with each other. For example, a CH3 domain in one single chain polypeptide of a polypeptide heterodimer may contain a T366W mutation (a "knob" mutation, which substitutes a small amino acid with a larger one), and a CH3 domain in the other single chain polypeptide of the polypeptide heterodimer may contain a Y407A mutation (a "hole" mutation, which substitutes a large amino acid with a smaller one). Other exemplary knobs-into-holes mutations include (1) a T366Y mutation in one CH3 domain and a Y407T in the other CH3 domain, and (2) a T366W mutation in one CH3 domain and T366S, L368A and Y407V mutations in the other CH3 domain.

The CH4 domain that may form an Fc region portion of a single chain polypeptide of a heterodimer of the present disclosure may be a wild type immunoglobulin CH4 domain or an altered immunoglobulin CH4 domain thereof from IgE or IgM molecules. In certain embodiments, the CH4 domain is a wild type human immunoglobulin CH4 domain, such as wild type CH4 domains of human IgE and IgM molecules as set forth in SEQ ID NOS:213 and 214, respectively. In certain embodiments, a CH4 domain is an altered human immunoglobulin CH4 domain, such as an altered CH4 domain based on or derived from a CH4 domain of human IgE or IgM molecules, which have mutations that increase or decrease an immunological activity known to be associated with an IgE or IgM Fc region.

In certain embodiments, an Fc region constant domain portion in heterodimers of the present disclosure comprises a combination of CH2, CH3 or CH4 domains (*i.e*., more than one constant sub-domain selected from CH2, CH3 and CH4). For example, the Fc region portion may comprise CH2 and CH3 domains or CH3 and CH4 domains. In certain other embodiments, the Fc region portion may comprise two CH3 domains and no CH2 or CH4 domains (*i.e*., only two or more CH3). The multiple constant sub-domains that form an Fc region portion may be based on or derived from the same immunoglobulin molecule, or the same class or subclass immunoglobulin molecules. In certain embodiments, the Fc region portion is an IgG CH2CH3 (*e.g.,* IgG1 CH2CH3, IgG2 CH2CH3, and IgG4 CH2CH3) and may be a human (e.g., human IgG1, IgG2, and IgG4) CH2CH3. For example, in certain embodiments, the Fc region portion comprises (1) wild type human IgG1 CH2 and CH3 domains, (2) human IgG1 CH2 with N297A substitution (*i.e*., CH2(N297A)) and wild type human IgG1 CH3, or (3) human IgG1 CH2(N297A) and an altered human IgG1 CH3 with the last lysine deleted.

Alternatively, the multiple constant sub-domains may be based on or derived from different immunoglobulin molecules, or different classes or subclasses immunoglobulin molecules. For example, in certain embodiments, an Fc region portion comprises both human IgM CH3 domain and human IgG1 CH3 domain. The multiple constant sub-domains that form an Fc region portion may be directly linked together or may be linked to each other via one or more (*e.g.,* about 2-10) amino acids.

Exemplary Fc region portions are set forth in SEQ ID NOS:305-309, 321, 323, 341, 342, and 762.

In certain embodiments, the Fc region portions of both single chain polypeptides of a polypeptide heterodimer are identical to each other. In certain other embodiments, the Fc region portion of one single chain polypeptide of a polypeptide heterodimer is different from the Fc region portion of the other single chain polypeptide of the heterodimer. For example, one Fc region portion may contain a CH3 domain with a "knob" mutation, whereas the other Fc region portion may contain a CH3 domain with a "hole" mutation.

### Hinge

A hinge region contained in a single chain polypeptide of a polypeptide heterodimer according to the present disclosure may be located (a) immediately amino terminal to an Fc region portion (*e.g.,* depending on the isotype, amino terminal to a CH2 domain wherein the Fc region portion is a CH2CH3, or amino terminal to a CH3 domain wherein the Fc region portion is a CH3CH4), (b) interposed between and connecting a binding domain (*e.g.,* scFv) and an immunoglobulin heterodimerization domain, (c) interposed between and connecting an immunoglobulin heterodimerization domain and an Fc region portion (*e.g.,* wherein the Fc region portion is a CH2CH3 or a CH3CH4, depending on the isotype or isotypes), (d) interposed between and connecting an Fc region portion and a binding domain, (e) at the amino terminus of the single chain polypeptide, or (f) at the carboxyl terminus of the single chain polypeptide. The single chain polypeptide comprising a hinge region as described herein will be capable of associating with a different single chain fusion polypeptide to form a polypeptide heterodimer provided herein, and the polypeptide heterodimer formed will contain a binding domain that retains its target specificity or its specific target binding affinity.

In certain embodiments, a hinge present in a single chain polypeptide that forms a polypeptide heterodimer with another single chain polypeptide may be an immunoglobulin hinge region, such as a wild type immunoglobulin hinge region or an altered immunoglobulin hinge region thereof.

In certain embodiments, a hinge is a wild type human immunoglobulin hinge region (*e.g.,* human immunoglobulin hinge regions as set forth in SEQ ID NOS:215-221). In certain other embodiments, one or more amino acid residues may be added at the amino- or carboxy- terminus of a wild type immunoglobulin hinge region as part of a fusion protein construct design. For example, additional junction amino acid residues at the hinge amino-terminus can be "RT," "RSS," "TG," or "T", or at the hinge carboxy-terminus can be "SG", or a hinge deletion can be combined with an addition, such as ΔP with "SG" added at the carboxyl terminus.

In certain embodiments, a hinge is an altered immunoglobulin hinge in which one or more cysteine residues in a wild type immunoglobulin hinge region is substituted with one or more other amino acid residues (*e.g.,* serine or alanine). For example, a hinge may be an altered immunoglobulin hinge based on or derived from a wild type human IgG1 hinge as set forth in SEQ ID NO:667, which from amino terminus to carboxyl terminus comprises the upper hinge region (EPKSCDKTHT, SEQ ID NO:227) and the core hinge region (CPPCP, SEQ ID NO:228). Exemplary altered immunoglobulin hinges include an immunoglobulin human IgG1 hinge region having one, two or three cysteine residues found in a wild type human IgG1 hinge substituted by one, two or three different amino acid residues (e.g., serine or alanine). An altered immunoglobulin hinge may additionally have a proline substituted with another amino acid *(e.g.,* serine or alanine). For example, the above-described altered human IgG1 hinge may additionally have a proline located carboxyl terminal to the three cysteines of wild type human IgG1 hinge region substituted by another amino acid residue (*e.g.,* serine, alanine). In one embodiment, the prolines of the core hinge region are not substituted. Exemplary altered immunoglobulin hinges are set forth in SEQ ID NOS: 229-240, 255, 664-677, and 748-759. An example of an altered IgG1 hinge is an altered human IgG1 hinge in which the first cysteine is substituted by serine. The sequence of this altered IgG1 hinge is set forth in SEQ ID NO:664, and is referred to as the " human IgG1 SCC-P hinge" or "SCC-P hinge." In certain embodiments, one or more amino acid residues (*e.g.,* "RT," "RSS," or "T") may be added at the amino-or carboxy-terminus of a mutated immunoglobulin hinge region as part of a fusion protein construct design.

In certain embodiments, a hinge polypeptide comprises or is a sequence that is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% identical to a wild type immunoglobulin hinge region, such as a wild type human IgG1 hinge, a wild type human IgG2 hinge, or a wild type human IgG4 hinge.

In further embodiments, a hinge present in a single chain polypeptide that forms a polypeptide heterodimer with another single chain polypeptide may be a hinge that is not based on or derived from an immunoglobulin hinge (*i*.*e*., not a wild type immunoglobulin hinge or an altered immunoglobulin hinge). These types of non-immunoglobulin based hinges may be used on or near the carboxyl end *(e.g.,* located carboxyl terminal to Fc region portions) of the single chain polypeptides that form the polypeptide heterodimers. Examples for such hinges include peptides of about five to about 150 amino acids of the interdomain or stalk region of type II C-lectins or CD molecules, for instance, peptides of about eight to 25 amino acids and peptides of about seven to 18 amino acids, and derivatives thereof.

The "interdomain or stalk region" of a type II C-lectin or CD molecule refers to the portion of the extracellular domain of the type II C-lectin or CD molecule that is located between the C-type lectin-like domain (CTLD; *e.g.,* similar to CTLD of natural killer cell receptors) and the transmembrane domain. For example, in the human CD94 molecule (GenBank Accession No. AAC50291.1, PRI November 30, 1995), the extracellular domain corresponds to amino acid residues 34-179, whereas the CTLD corresponds to amino acid residues 61-176. Accordingly, the interdomain or stalk region of the human CD94 molecule includes amino acid residues 34-60, which is found between the membrane and the CTLD (*see* Boyington *et al*., Immunity 10:75, 1999; for descriptions of other stalk regions, *see also* Beavil et al., Proc. Nat'l. Acad. Sci. USA 89:753, 1992; and Figdor et al., Nature Rev. Immunol. 2:77, 2002). These type II C-lectin or CD molecules may also have from six to 10 junction amino acids between the stalk region and the transmembrane region or the CTLD. In another example, the 233 amino acid human NKG2A protein (GenBank Accession No. P26715.1, PRI June 15, 2010) has a transmembrane domain ranging from amino acids 71-93 and an extracellular domain ranging from amino acids 94-233. The CTLD is comprised of amino acids 119-231, and the stalk region comprises amino acids 99-116, which is flanked by junctions of five and two amino acids. Other type II C-lectin or CD molecules, as well as their extracellular ligand-bind domains, interdomain or stalk regions, and CTLDs are known in the art (*see, e.g.,* GenBank Accession Nos. NP_001993.2; AAH07037.1, PRI July 15, 2006; NP_001773.1, PRI June 20, 1010; AAL65234.1, PRI January 17, 2002, and CAA04925.1, PRI November 14, 2006, for the sequences of human CD23, CD69, CD72, NKG2A and NKG2D and their descriptions, respectively).

A "derivative" of an interdomain or stalk region, or fragment thereof, of a type II C-lectin or CD molecule includes an about an eight to about 150 amino acid sequence in which one, two, or three amino acids of the stalk region of a wild type type II C-lectin or CD molecule have a deletion, insertion, substitution, or any combination thereof, for instance, the one or more changes are substitutions or the one or more mutations include only one deletion. In further embodiments, a derivative of an interdomain or stalk region is more resistant to proteolytic cleavage as compared to the wild-type interdomain or stalk region sequence, such as those derived from about eight to about 20 amino acids of NKG2A, NKG2D, CD23, CD64, CD72, or CD94.

In certain embodiments, interdomain or stalk region hinges have seven to 18 amino acids and can form an α-helical coiled coil structure. In certain embodiments, interdomain or stalk region hinges contain 0, 1, 2, 3, or 4 cysteines. Exemplary interdomain or stalk region hinges are peptide fragments of the interdomain or stalk regions, such as ten to 150 amino acid fragments from the stalk regions of CD69, CD72, CD94, NKG2A and NKG2D, as set forth in SEQ ID NOS:241-244, 716 and 601. Additional exemplary stalk region or interdomain hinges include those as set forth in SEQ ID NOS:78, 734-737, 742-747, and 766-790.

Alternative hinges that can be used in single chain polypeptides of polypeptide heterodimers are from portions of cell surface receptors (interdomain regions) that connect immunoglobulin V-like or immunoglobulin C-like domains. Regions between Ig V-like domains where the cell surface receptor contains multiple Ig V-like domains in tandem and between Ig C-like domains where the cell surface receptor contains multiple tandem Ig C-like regions are also contemplated as hinges useful in single chain polypeptides of polypeptide heterodimers. In certain embodiments, hinge sequences comprised of cell surface receptor interdomain regions may further contain a naturally occurring or added motif, such as an IgG core hinge sequence that confers one or more disulfide bonds to stabilize the polypeptide heterodimer formation. Examples of hinges include interdomain regions between the Ig V-like and Ig C-like regions of CD2, CD4, CD22, CD33, CD48, CD58, CD66, CD80, CD86, CD150, CD166, and CD244.

In certain embodiments, hinge sequences have about 5 to 150 amino acids, 5 to 10 amino acids, 10 to 20 amino acids, 20 to 30 amino acids, 30 to 40 amino acids, 40 to 50 amino acids, 50 to 60 amino acids, 5 to 60 amino acids, 5 to 40 amino acids, 8 to 20 amino acids, or 10 to 15 amino acids. The hinge may be primarily flexible, but may also provide more rigid characteristics or may contain primarily α-helical structure with minimal β-sheet structure. The lengths or the sequences of the hinges may affect the binding affinities of the binding domains to which the hinges are directly or indirectly (via another region or domain, such as an immunoglobulin heterodimerization domain) connected as well as one or more activities of the Fc region portions to which the hinges are directly or indirectly connected (*see,* Examples 9 and 10).

In certain embodiments, hinge sequences are stable in plasma and serum and are resistant to proteolytic cleavage. The first lysine in the IgG1 upper hinge region may be mutated to minimize proteolytic cleavage, for instance, the lysine may be substituted with methionine, threonine, alanine or glycine, or is deleted (*see*, *e.g.,* SEQ ID NOS:379-434, which may include junction amino acids at the amino terminus such as RT).

In some embodiments, hinge sequences may contain a naturally occurring or added motif such as an immunoglobulin hinge core structure CPPCP (SEQ ID NO:228) that confers the capacity to form a disulfide bond or multiple disulfide bonds to stabilize the carboxy-terminus of a molecule. In other embodiments, hinge sequences may contain one or more glycosylation sites.

Exemplary hinges, including altered immunoglobulin hinges, are set forth in SEQ ID NOS:379-434, 618-749, and 763-791.

In certain embodiments, a single chain polypeptide of a polypeptide heterodimer according to the present disclosure comprises more than one hinge. For example, a single chain polypeptide having two binding domains, one of which at the amino terminus and the other at the carboxyl terminus, may have two hinges. One hinge may be directly or indirectly (*e.g.,* via an immunoglobulin heterodimerization domain) connected to the binding domain at or near the amino terminus, and the other hinge may be connected (*e.g.,* directly connected) to the other binding domain at or near the carboxyl terminus. In certain embodiments, even if a single chain polypeptide has only one binding domain, it may have more than one hinge, for example, at its amino or carboxyl terminus. Such a hinge may interact with a corresponding hinge in the other chain of the heterodimer, such as forming one or more interchain disulfide bonds, to facilitate or enhance the heterodimerization of the two chains. A hinge (H-I) of a SCP-I of a polypeptide heterodimer "corresponds to" a hinge (H-II) of a SCP-II of the heterodimer when H-I and H-II are located on the same end of the Fc region portion of their respective single chain polypeptide. For example, a polypeptide heterodimer may comprise the following two single chain polypeptides: A first chain polypeptide from amino to carboxyl terminus comprises a first binding domain, CH1, hinge, CH2, and CH3, and a second chain polypeptide from amino to carboxyl terminus comprises a CK, first hinge, CH2, CH3, second hinge, and a second binding domain. The hinge in the first chain would be regarded as "corresponding" to the first hinge of the second chain because both are amino terminal to the Fc region portions to which they are connected.

In certain embodiments where a single chain polypeptide of a polypeptide heterodimer comprises a binding domain at or near its carboxyl terminus, a hinge may be present to link the binding domain with another portion of the single chain polypeptide (*e.g.,* an Fc region portion or an immunoglobulin heterodimerization domain). In one embodiment, such a hinge is a non-immunoglobulin hinge (*i.e*., a hinge not based on or derived from a wild type immunoglobulin hinge) and may be a stalk region of a type II C-lectin or CD molecule, an interdomain region that connect IgV-like or IgC-like domains of a cell surface receptor, or a derivative or functional variant thereof. Exemplary carboxyl terminal hinges, sometimes referred to as "back-end" hinges, includes those set forth in SEQ ID NOS:78, 734-737, 742-747, and 766-790.

In certain embodiments, a hinge of one single chain polypeptide of a polypeptide heterodimer is identical to a corresponding hinge of the other single chain polypeptide of the heterodimer. In certain other embodiments, a hinge of one chain is different from that of the other chain (in their length or sequence). The different hinges in the different chains allow different manipulation of the binding affinities of the binding domains to which the hinges are connected, so that the heterodimer is able to preferentially bind to the target of one binding domain over the target of the other binding domain. For example, in certain embodiments, a polypeptide heterodimer has a CD3 or TCR binding domain in one chain and a tumor antigen binding domain in another chain. Having two different hinges in the two chains may allow the heterodimer to bind to the tumor antigen first, and then to a CD3 or TCR molecule second. Thus, the heterodimer may recruit CD3⁺ T cells to tumor cells carrying the tumor antigen, which in turn may damage or destroy the tumor cells.

### Other components or modifications

In certain embodiments, a single chain polypeptide that forms a heterodimer with another single chain polypeptide may contain one or more additional domains or regions. Such additional regions may be a leader sequence (also referred to as "signal peptide") at the amino-terminus for secretion of an expressed single chain polypeptide. Exemplary leader peptides of this disclosure include natural leader sequences or others, such as those as set forth in SEQ ID NOS:110 and 111.

Additional regions may also be sequences at the carboxy-terminus for identifying or purifying single chain polypeptides (*e.g.,* epitope tags for detection or purification, such as a histidine tag, biotin, a FLAG® epitope, or any combination thereof).

Further optional regions may be additional amino acid residues (referred to as "junction amino acids" or "junction amino acid residues") having a length of 1 to about 10 amino acids (*e.g.,* about 2 to 5 amino acids), which may be resulted from use of specific expression systems or construct design for the single chain polypeptides of the present disclosure. Such additional amino acid residues (for instance, one, two, three, four or five additional amino acids) may be present at the amino or carboxyl terminus or between various regions or domains of a single chain polypeptide, such as between a binding domain and an immunoglobulin heterodimerization domain, between an immunoglobulin heterodimerization domain and a hinge, between a hinge and an Fc region portion, between domains of an Fc region portion (*e.g.,* between CH2 and CH3 domains or between two CH3 domains), between a binding domain and a hinge, between an Fc region portion and an immunoglobulin heterodimerization domain, or between a variable domain and a linker. Exemplary junction amino acids amino-terminal to a hinge include RDQ (SEQ ID NO:598), RT, SS, SASS (SEQ ID NO:599) and SSS (SEQ ID NO:600). Exemplary junction amino acids carboxy-terminal to a hinge include amino acids SG. Additional exemplary junction amino acids include SR.

In certain embodiments, junction amino acids are present between an Fc region portion that comprises CH2 and CH3 domains and an immunoglobulin heterodimerization domain (CH1 or CL). These junction amino acids are also referred to as a "linker between CH3 and CH1 or CL" if they are present between the C-terminus of CH3 and the N-terminus of CH1 or CL. Such a linker may be about 2-12 amino acids in length. In certain embodiments, the Fc region portion comprises human IgG1 CH2 and CH3 domains in which the C-terminal lysine residue of human IgG1 CH3 is deleted. Exemplary linkers between CH3 and CH1 include those set forth in SEQ ID NO:847-849. Exemplary linkers between CH3 and Ck include those set forth in SEQ ID NOS:850-852 (in which the carboxyl terminal arginine in the linkers may alternatively be regarded as the first arginine of Ck). In certain embodiments, the presence of such linkers or linker pairs *(e.g.,* SEQ ID NO:847 as a CH3-CH1 linker in one single chain polypeptide of a heterodimer and SEQ ID NO:850 as a CH3-Ck linker in the other single chain polypeptide of the heterodimer; SEQ ID NO:848 as a CH3-CH1 linker and SEQ ID NO:851 as a CH3-Ck linker; and SEQ ID NO:849 as a CH3-CH1 linker and SEQ ID NO:852 as a CH3-Ck linker) improves the production of heterodimer compared the presence of a reference linker as set forth in SEQ ID NO:846 (in which the last lysine of CH3 is included as part of the linker) in both single chain polypeptides of a heterodimer.

In certain embodiments, an immunoglobulin Fc region *(e.g.,* CH2, CH3, and/or CH4 regions) of a polypeptide heterodimer of the present disclosure may have an altered glycosylation pattern relative to an immunoglobulin reference sequence. For example, any of a variety of genetic techniques may be employed to alter one or more particular amino acid residues that form a glycosylation site (*see* Co et al. (1993) Mol. Immunol. 30:1361; Jacquemon et al. (2006) J. Thromb. Haemost. 4:1047; Schuster et al. (2005) Cancer Res. 65:7934; Warnock et al. (2005) Biotechnol. Bioeng. 92:831), such as N297 of the CH2 domain (EU numbering). Alternatively, the host cells producing polypeptide heterodimers of this disclosure may be engineered to produce an altered glycosylation pattern. One method known in the art, for example, provides altered glycosylation in the form of bisected, non-fucosylated variants that increase ADCC. The variants result from expression in a host cell containing an oligosaccharide-modifying enzyme. Alternatively, the POTELLIGENT® technology of BioWa/Kyowa Hakko is contemplated to reduce the fucose content of glycosylated molecules according to this disclosure. In one known method, a CHO host cell for recombinant immunoglobulin production is provided that modifies the glycosylation pattern of the immunoglobulin Fc region, through production of GDP-fucose.

Alternatively, chemical techniques are used to alter the glycosylation pattern of polypeptide heterodimers of this disclosure. For example, a variety of glycosidase and/or mannosidase inhibitors provide one or more of desired effects of increasing ADCC activity, increasing Fc receptor binding, and altering glycosylation pattern. In certain embodiments, cells expressing polypeptide heterodimers of the instant disclosure are grown in a culture medium comprising a carbohydrate modifier at a concentration that increases the ADCC of immunoglycoprotein molecules produced by said host cell, wherein said carbohydrate modifier is at a concentration of less than 800 µM. In one embodiment, the cells expressing these polypeptide heterodimers are grown in a culture medium comprising castanospermine or kifunensine, for instance, castanospermine at a concentration of 100-800 µM, such as 100 µM, 200 µM, 300 µM, 400 µM, 500 µM, 600 µM, 700 µM, or 800 µM. Methods for altering glycosylation with a carbohydrate modifier such as castanospermine are provided in U.S. Patent No. 7,846,434 or PCT Publication No. WO 2008/052030.

### Structural Arrangements and Exemplary Heterodimers

To form a polypeptide heterodimer according to the present invention, two single chain polypeptides are designed so that the immunoglobulin heterodimerization domain of the first single chain polypeptide is properly aligned and interacts with the immunoglobulin heterodimerization domain of the second single chain polypeptide. In certain embodiments, the heterodimer may comprise a second immunoglobulin heterodimerization domain pair to facilitate or enhance the heterodimerization of the two chains. In certain embodiments, in addition to the interaction between the two immunoglobulin heterodimerization domains, an Fc region portion *(e.g.,* a CH3 domain) in the first chain may interact with an Fc region in the second chain to enhance heterodimerization (*e.g.,* via interaction between two wild type CH3 domains or between a CH3 domain pair with "knobs-into-holes" mutations). Moreover, in certain embodiments, the hinge in the first chain *(e.g.,* an altered human IgG1 hinge with two cysteine residues as set forth in SEQ ID NO:664) may interact with the hinge in the second chain *(e.g.,* the same altered human IgG1 hinge as set forth in SEQ ID NO:664) to form, for example, disulfide bonds, which may further strengthen the interaction between the first and second single chain polypeptides to form a polypeptide heterodimer of the present disclosure. Furthermore, in certain embodiments, the first and second chain may comprise a second hinge pair *(e.g.,* at the carboxyl termini of the two chains) to further enhance the interactions between the two chains.

In certain embodiments, a polypeptide heterodimer of the present disclosure comprises two binding domains (BD1 and BD2), wherein the binding domains bind to two different target molecules. In certain embodiments, the two binding domains (BD1 and BD2) are both on the SCP-I with the immunoglobulin heterodimerization domain (HD-I) and the Fc region portion (FRP-1) disposed between BD1 and BD2. In certain other embodiments, the first binding domain (BD1) is on the SCP-I and the second binding domain (BD2) is on the SCP-II. In certain embodiments, both BD1 and BD2 are amino terminal to the Fc region portion of the SCP-I and SCP-II, respectively. In certain other embodiments, BD1 is amino terminal to the Fc region portion of the SCP-I and BD2 is carboxyl terminal to the Fc region portion of the SCP-II. In certain other embodiments, BD1 is carboxyl terminal to the Fc region portion of the SCP-I and BD2 is amino terminal to the Fc region portion of the SCP-II. In certain other embodiments, both BD1 and BD2 are carboxyl terminal to the Fc region portion of the SCP-I and SCP-II, respectively.

In certain embodiments, a polypeptide heterodimer comprises three binding domains (BD1, BD2 and BD3), wherein the binding domains bind to two or three different target molecules. For example, BD1, BD2, and BD3 each bind a different target, or BD1 and BD2 bind a first target while BD3 binds a second target, or BD1 and BD3 bind a first target while BD2 binds a second target, or BD2 and BD3 bind a first target while BD1 binds a second target. In certain embodiments, the immunoglobulin heterodimerization domain and the Fc region portion are disposed between BD1 and BD2 on SCP-I, and BD3 is amino terminal to the Fc region portion of the SCP-II. In further embodiments, the immunoglobulin heterodimerization domain and the Fc region portion are disposed between BD1 and BD2 on SCP-II, and BD3 is carboxyl terminal to the Fc region portion of the SCP-I.

In certain embodiments, a polypeptide heterodimer comprises four binding domains (BD1, BD2, BD3 and BD4), wherein the binding domains bind to two to four different target molecules. In certain embodiments, the immunoglobulin heterodimerization domain and Fc region portion of the SCP-I are disposed between BD1 and BD2, and the immunoglobulin heterodimerization domain and Fc region portion of SCP-II are disposed between BD3 and BD4.

In certain embodiments, a polypeptide heterodimer comprises five binding domains (BD1, BD2, BD3, BD4 and BD5), wherein the binding domains bind to two to four different target molecules. In certain embodiments, SCP-I comprises three binding domains (BD1, BD2 and BD3), and SCP-II comprises two binding domains (BD4 and BD5). In further embodiments, BD1 and BD2 may be, for example, linked in tandem to each other (either directly or via a peptide linker of about two to eight amino acids) at the amino (or carboxyl) terminus of SCP-I with BD3 located at the carboxyl (or amino) terminus of SCP-I or SCP-II, wherein BD4 and BD5 are on SCP-II if BD3 is on SCP-I, or BD4 or BD5 are on SCP-I if BD3 is on SCP-II.

In still further embodiments, the polypeptide heterodimer comprises six binding domains (BD1-BD6). In certain embodiments, SCP-I and SCP-II each comprise three binding domains (e.g., BD1-BD3 on SCP-I, and BD4-BD6 on SCP-II). In such embodiments, for example, BD1 and BD2 may be linked in tandem and located at the SCP-I amino (or carboxyl) terminus, and BD3 may be located at the SCP-I carboxyl (or amino) terminus. Similarly, BD4 and BD5 may be linked in tandem and located at the SCP-II amino (or carboxyl) terminus, and BD6 may be located at the SCP-II carboxyl (or amino) terminus. In certain other embodiments, the first single chain polypeptide (SCP-I) comprises four binding domains (BD1-BD4) and the second single chain polypeptide (SCP-II) comprises two binding domains (BD5 and BD6). In such embodiments, BD1 and BD2 may be linked in tandem and located at or near amino terminus of SCP-I, BD3 and BD4 may be linked in tandem and located at or near carboxyl terminus of SCP-I, and BD5 and BD6 may be located at or near the amino and carboxyl termini of SCP-II, respectively.

In certain embodiments, the polypeptide heterodimer comprises seven binding domains (BD1-BD7). In such embodiments, SCP-I may comprise four binding domains (BD1-BD4), and SCP-II may comprise the other three binding domains (BD5-BD7). For example, BD1 and BD2 may be linked in tandem and located at or near the amino terminus of SCP-I, and BD3 and BD4 may be linked in tandem and located at or near the carboxyl terminus of SCP-II. BD5 and BD6 may be linked in tandem and located at or near the amino (or carboxyl) terminus of SCP-II, and BD7 may be located at or near the carboxyl (or amino) terminus of SCP-II.

In certain embodiments, the polypeptide heterodimer comprises eight binding domains (BD1-BD8). In such embodiments, the first and second single chain polypeptides may each comprise four binding domains. In each chain, two binding domains may be located at or near the amino terminus, and the other two binding domains located at or near the carboxyl terminus.

To simplify the description of how various components can be arranged to make first and second single chain polypeptides that form polypeptide heterodimers of the present disclosure, arrangements in exemplary embodiments (1) to (50) are provided below in which only two binding domains are included in each heterodimer.

In embodiment (1), a polypeptide heterodimer comprises the following two single chain polypeptides: from amino terminus to carboxyl terminus, a first single chain polypeptide comprising a first binding domain, a CH1 region, a hinge, and an Fc region portion; and a second single chain polypeptide comprising a second binding domain, a CL region *(e.g.,* Cκ, Cλ), a hinge, and an Fc region portion.

In embodiment (2), a polypeptide heterodimer comprises the following two single chain polypeptides: from amino terminus to carboxyl terminus, a first single chain polypeptide comprising a first binding domain, a hinge, an Fc region portion, and a CH1 region; and a second single chain polypeptide comprising a second binding domain, a hinge, an Fc region portion, and a CL region.

In embodiment (3), a polypeptide heterodimer comprises the following two single chain polypeptides: from amino terminus to carboxyl terminus, a first single chain polypeptide comprising a first binding domain, a CH1 region, a hinge, an Fc region portion, and a second CH1 region; and a second single chain polypeptide comprising a second binding domain, a CL region, a hinge, an Fc region portion, and a second CL region.

In embodiment (4), a polypeptide heterodimer comprises the following two single chain polypeptides: from amino terminus to carboxyl terminus, a first single chain polypeptide comprising a first binding domain, a CH1 region, a second CH1 region, a hinge, and an Fc region portion; and a second single chain polypeptide comprising a second binding domain, a CL region, a second CL region, a hinge and an Fc region portion.

In embodiment (5), a polypeptide heterodimer comprises the following two single chain polypeptides: from amino terminus to carboxyl terminus, a first single chain polypeptide comprising a first binding domain, a hinge, an Fc region portion, a CH1 region, and a second CH1 region; and a second single chain polypeptide comprising a second binding domain, a hinge, an Fc region portion, a CL region and a second CL region.

In embodiment (6), a polypeptide heterodimer comprises the following two single chain polypeptides: from amino terminus to carboxyl terminus, a first single chain polypeptide comprising a CH1 region, a hinge, an Fc region portion, a second hinge, and a first binding domain; and a second single chain polypeptide comprising a CL region, a hinge, an Fc region portion, a second hinge, and a second binding domain.

In embodiment (7), a polypeptide heterodimer is formed from the following two single chain polypeptides: from amino terminus to carboxyl terminus, a first single chain polypeptide comprising a hinge, an Fc region portion, a CH1 region, a second hinge, and a first binding domain; and a second single chain polypeptide comprising a hinge, an Fc region portion, a CL region, a second hinge, and a second binding domain.

In embodiment (8), a polypeptide heterodimer comprises the following two single chain polypeptides: from amino terminus to carboxyl terminus, a first single chain polypeptide comprising a CH1 region, a hinge, an Fc region portion, a second CH1 region, a second hinge, and a first binding domain; and a second single chain polypeptide comprising a CL region, a hinge, an Fc region portion, a second CL region, a second hinge, and a second binding domain.

In embodiment (9), a polypeptide heterodimer comprises the following two single chain polypeptides: from amino terminus to carboxyl terminus, a first single chain polypeptide comprising a CH1 region, a second CH1 region, a hinge, an Fc region portion, a second hinge, and a first binding domain; and a second single chain polypeptide comprising a CL region, a second CL region, a hinge, an Fc region portion, a second hinge, and a second binding domain.

In embodiment (10), a polypeptide heterodimer comprises the following two single chain polypeptides: from amino terminus to carboxyl terminus, a first single chain polypeptide comprising a hinge, an Fc region portion, a CH1 region, a second CH1 region, a second hinge, and a first binding domain; and a second single chain polypeptide comprising a hinge, an Fc region portion, a CL region, a second CL region, a second hinge, and a second binding domain.

In embodiment (11), a polypeptide heterodimer comprises the following two single chain polypeptides: from amino terminus to carboxyl terminus, a first single chain polypeptide comprising a first binding domain, a CL region, a hinge, and an Fc region portion; and a second single chain polypeptide comprising a second binding domain, a CH1 region, a hinge, and an Fc region portion.

In embodiment (12), a polypeptide heterodimer comprises the following two single chain polypeptides: from amino terminus to carboxyl terminus, a first single chain polypeptide comprising a first binding domain, a hinge, an Fc region portion, and a CL region; and a second single chain polypeptide comprising a second binding domain, a hinge, an Fc region portion, and a CH1 region.

In embodiment (13), a polypeptide heterodimer comprises the following two single chain polypeptides: from amino terminus to carboxyl terminus, a first single chain polypeptide comprising a first binding domain, a CL region, a hinge, an Fc region portion, and a second CL region; and a second single chain polypeptide comprising a second binding domain, a CH1 region, a hinge, an Fc region portion, and a second CH1 region.

In embodiment (14), a polypeptide heterodimer comprises the following two single chain polypeptides: from amino terminus to carboxyl terminus, a first single chain polypeptide comprising a first binding domain, a CL region, a second CL region, a hinge, and an Fc region portion; and a second single chain polypeptide comprising a second binding domain, a CH1 region, a second CH1 region, a hinge, and an Fc region portion.

In embodiment (15), a polypeptide heterodimer comprises the following two single chain polypeptides: from amino terminus to carboxyl terminus, a first single chain polypeptide comprising a first binding domain, a hinge, an Fc region portion, a CL region, and a second CL region; and a second single chain polypeptide comprising a second binding domain, a hinge, an Fc region portion, a CH1 region, and a second CH1 region.

In embodiment (16), a polypeptide heterodimer comprises the following two single chain polypeptides: from amino terminus to carboxyl terminus, a first single chain polypeptide comprising a CL region, a hinge, an Fc region portion, a second hinge, and a first binding domain; and a second single chain polypeptide comprising a CH1 region, a hinge, an Fc region portion, a second hinge, and a second binding domain.

In embodiment (17), a polypeptide heterodimer comprises the following two single chain polypeptides: from amino terminus to carboxyl terminus, a first single chain polypeptide comprising a hinge, an Fc region portion, a CL region, a second hinge, and a first binding domain; and a second single chain polypeptide comprising a hinge, an Fc region portion, a CH1 region, a second hinge, and a second binding domain.

In embodiment (18), a polypeptide heterodimer comprises the following two single chain polypeptides: from amino terminus to carboxyl terminus, a first single chain polypeptide comprising a CL region, a hinge, an Fc region portion, a second CL region, a second hinge, and a first binding domain; and a second single chain polypeptide comprising a CH1 region, a hinge, an Fc region portion, a second CH1 region, a second hinge, and a second binding domain.

In embodiment (19), a polypeptide heterodimer comprises the following two single chain polypeptides: from amino terminus to carboxyl terminus, a first single chain polypeptide comprising a CL region, a second CL region, a hinge, an Fc region portion, a second hinge, and a first binding domain; and a second single chain polypeptide comprising a CH1 region, a second CH1 region, a hinge, an Fc region portion, a second hinge, and a second binding domain.

In embodiment (20), a polypeptide heterodimer comprises the following two single chain polypeptides: from amino terminus to carboxyl terminus, a first single chain polypeptide comprising a hinge, an Fc region portion, a CL region, a second CL region, a second hinge, and a first binding domain; and a second single chain polypeptide comprising a hinge, an Fc region portion, a CH1 region, a second CH1 region, a second hinge, and a second binding domain.

In embodiment (21), a polypeptide heterodimer comprises the following two single chain polypeptides: from amino terminus to carboxyl terminus, a first single chain polypeptide comprising a first binding domain, a CH1 region, a hinge, an Fc region portion, and a CL region; and a second single chain polypeptide comprising a second binding domain, a CL region, a hinge, an Fc region portion, and a CH1 region.

In embodiment (22), a polypeptide heterodimer comprises the following two single chain polypeptides: from amino terminus to carboxyl terminus, a first single chain polypeptide comprising a first binding domain, a CL region, a hinge, an Fc region portion, and a CH1 region; and a second single chain polypeptide comprising a second binding domain, a CH1 region, a hinge, an Fc region portion, and a CL region.

In embodiment (23), a polypeptide heterodimer comprises the following two single chain polypeptides: from amino terminus to carboxyl terminus, a first single chain polypeptide comprising a first binding domain, a CH1 region, a CL region, a hinge, and an Fc region portion; and a second single chain polypeptide comprising a second binding domain, a CL region, a CH1 region, a hinge and an Fc region portion.

In embodiment (24), a polypeptide heterodimer comprises the following two single chain polypeptides: from amino terminus to carboxyl terminus, a first single chain polypeptide comprising a first binding domain, a CL region, a CH1 region, a hinge, and an Fc region portion; and a second single chain polypeptide comprising a second binding domain, a CH1 region, a CL region, a hinge and an Fc region portion.

In embodiment (25), a polypeptide heterodimer comprises the following two single chain polypeptides: from amino terminus to carboxyl terminus, a first single chain polypeptide comprising a first binding domain, a hinge, an Fc region portion, a CH1 region and a CL region; and a second single chain polypeptide comprising a second binding domain, a hinge, an Fc region portion, a CL region and a CH1 region.

In embodiment (26), a polypeptide heterodimer comprises the following two single chain polypeptides: from amino terminus to carboxyl terminus, a first single chain polypeptide comprising a first binding domain, a hinge, an Fc region portion, a CL region and a CH1 region; and a second single chain polypeptide comprising a second binding domain, a hinge, an Fc region portion, a CH1 region and a CL region.

In embodiment (27), a polypeptide heterodimer comprises the following two single chain polypeptides: from amino terminus to carboxyl terminus, a first single chain polypeptide comprising a CH1 region, a hinge, an Fc region portion, a CL region, a second hinge, and a first binding domain; and a second single chain polypeptide comprising a CL region, a hinge, an Fc region portion, a CH1 region, a second hinge, and a second binding domain.

In embodiment (28), a polypeptide heterodimer comprises the following two single chain polypeptides: from amino terminus to carboxyl terminus, a first single chain polypeptide comprising a CL region, a hinge, an Fc region portion, a CH1 region, a second hinge, and a first binding domain; and a second single chain polypeptide comprising a CH1 region, a hinge, an Fc region portion, a CL region, a second hinge, and a second binding domain.

In embodiment (29), a polypeptide heterodimer comprises the following two single chain polypeptides: from amino terminus to carboxyl terminus, a first single chain polypeptide comprising a CH1 region, a CL region, a hinge, an Fc region portion, a second hinge, and a first binding domain; and a second single chain polypeptide comprising a CL region, a CH1 region, a hinge, an Fc region portion, a second hinge, and a second binding domain.

In embodiment (30), a polypeptide heterodimer comprises the following two single chain polypeptides: from amino terminus to carboxyl terminus, a first single chain polypeptide comprising a CL region, a CH1 region, a hinge, an Fc region portion, a second hinge, and a first binding domain; and a second single chain polypeptide comprising a CH1 region, a CL region, a hinge, an Fc region portion, a second hinge, and a second binding domain.

In embodiment (31), a polypeptide heterodimer comprises the following two single chain polypeptides: from amino terminus to carboxyl terminus, a first single chain polypeptide comprising a hinge, an Fc region portion, a CH1 region, a CL region, a second hinge, and a first binding domain; and a second single chain polypeptide comprising a hinge, an Fc region portion, a CL region, a CH1 region, a second hinge, and a second binding domain.

In embodiment (32), a polypeptide heterodimer comprises the following two single chain polypeptides: from amino terminus to carboxyl terminus, a first single chain polypeptide comprising a hinge, an Fc region portion, a CL region, a CH1 region, a second hinge, and a first binding domain; and a second single chain polypeptide comprising a hinge, an Fc region portion, a CH1 region, a CL region, a second hinge, and a second binding domain.

In embodiment (33), a polypeptide heterodimer comprises the following two single chain polypeptides: from amino to carboxyl terminus, a first single chain polypeptide comprising a first binding domain, a CH1 region, a hinge, an Fc region portion, a second hinge, and a second binding domain; and a second single chain polypeptide comprising a CL region, a hinge, and an Fc region.

In embodiment (34), a polypeptide heterodimer comprises the following two single chain polypeptides: from amino to carboxyl terminus, a first single chain polypeptide comprising a first binding domain, a CL region, a hinge, an Fc region portion, a second hinge, and a second binding domain; and a second single chain polypeptide comprising a CH1 region, a hinge and an Fc region portion.

In embodiment (35), a polypeptide heterodimer comprises the following two single chain polypeptides: from amino to carboxyl terminus, a first single chain polypeptide comprising a first binding domain, a CH1 region, a hinge and an Fc region portion; and a second single chain polypeptide comprising a CL region, a hinge, an Fc region portion, a second hinge and a second binding domain.

In embodiment (36), a polypeptide heterodimer comprises the following two single chain polypeptides: from amino to carboxyl terminus, a first single chain polypeptide comprising a CH1 region, a hinge, an Fc region portion, a second hinge and a first binding domain; and a second single chain polypeptide comprising a second binding domain, a CL region, a hinge and an Fc region portion.

In embodiment (37), a polypeptide heterodimer comprises the following two single chain polypeptides: from amino to carboxyl terminus, a first single chain polypeptide comprising a first binding domain, a CH1 region, a hinge, an Fc region portion, and a second hinge; and a second single chain polypeptide comprising a second binding domain, a CL region, a hinge, an Fc region portion, and a second hinge.

In embodiment (38), a polypeptide heterodimer comprises the following two single chain polypeptides: from amino to carboxyl terminus, a first single chain polypeptide comprising a first binding domain, a hinge, an Fc region portion, a CH1 region, a second hinge, and a second binding domain; and the second single chain polypeptide comprising a hinge, an Fc region portion, and a CL region.

In embodiment (39), a polypeptide heterodimer comprises the following two single chain polypeptides: from amino to carboxyl terminus, a first single chain polypeptide comprising a hinge, an Fc region portion, and a CH1 region; and a second single chain polypeptide comprising a first binding domain, a hinge, an Fc region portion, a CL region, a second hinge, and a second binding domain.

In embodiment (40), a polypeptide heterodimer comprises the following two single chain polypeptides: from amino to carboxyl terminus, a first single chain polypeptide comprising a first binding domain, a hinge, an Fc region portion, and a CH1 region; and a second single chain polypeptide comprising a hinge, an Fc region portion, a CL region, a second hinge and a second binding domain.

In embodiment (41), a polypeptide heterodimer comprises the following two single chain polypeptides: from amino to carboxyl terminus, a first single chain polypeptide comprising a hinge, an Fc region portion, a CH1 region, a second hinge and a first binding domain; and the second single chain polypeptide comprising a second binding domain, a hinge, an Fc region portion, and a CL region.

In embodiment (42), a polypeptide heterodimer comprises the following two single chain polypeptides: from amino to carboxyl terminus, a first single chain polypeptide comprising a first binding domain, a hinge, an Fc region portion, a CH1 region, and a second hinge; and a second single chain polypeptide comprising a second binding domain, a hinge, an Fc region portion, a CL region and a second hinge.

In embodiment (43), a polypeptide heterodimer comprises the following two single chain polypeptides: from amino to carboxyl terminus, a first single chain polypeptide comprising a first binding domain, a CH1 region, a hinge an Fc region portion, a CL region, a second hinge and a second binding domain; and the second single chain polypeptide comprising a CL region, a hinge, an Fc region portion and a CH1 region.

In embodiment (44), a polypeptide heterodimer comprises the following two single chain polypeptides: from amino to carboxyl terminus, a first single chain polypeptide comprising a CH1 region, a hinge, an Fc region portion, and a CL region; and a second single chain polypeptide comprising a first binding domain, a CL region, a hinge, an Fc region, a CH1 region, a second hinge and a second binding domain.

In embodiment (45), a polypeptide heterodimer comprises the following two single chain polypeptides: from amino to carboxyl terminus, a first single chain polypeptide comprising a first binding domain, a CH1 region, a hinge, an Fc region portion, and a CL region; and a second single chain polypeptide comprising a CL region, a hinge, an Fc region portion, a CH1 region, a second hinge, and a second binding domain.

In embodiment (46), a polypeptide heterodimer comprises the following two single chain polypeptides: from amino to carboxyl terminus, a first single chain polypeptide comprising a CH1 region, a hinge, an Fc region portion, a CL, a second hinge and a first binding domain; and a second single chain polypeptide comprising a second binding domain, a CL region, a hinge, an Fc region portion, and a CH1 region.

In embodiment (47), a polypeptide heterodimer comprises the following two single chain polypeptides: from amino to carboxyl terminus, a first single chain polypeptide comprising a first binding domain, a CH1 region, a hinge, an Fc region portion, a second CH1 region, a second hinge, and a second binding domain; and a second single chain polypeptide comprising a CL region, a hinge, an Fc region portion, and a second CL region.

In embodiment (48), a polypeptide heterodimer comprises the following two single chain polypeptides: from amino to carboxyl terminus, a first single chain polypeptide comprising a CH1 region, a hinge, an Fc region portion, and a second CH1 region; and a second single chain polypeptide comprising a first binding domain, a CL region, a hinge, an Fc region portion, a second CL region, a second hinge, and a second binding domain.

In embodiment (49), a polypeptide heterodimer comprises the following two single chain polypeptides: from amino to carboxyl terminus, a first single chain polypeptide comprising a first binding domain, a CH1 region, a hinge, an Fc region portion, and a second CH1 region; and a second single chain polypeptide comprising a CL region, a hinge, an Fc region portion, a second CL region, a second hinge and a second binding domain.

In embodiment (50), a polypeptide heterodimer comprises the following two single chain polypeptides: from amino to carboxyl terminus, a first single chain polypeptide comprising a CH1 region, a hinge, an Fc region portion, a CH1 region, a second hinge, and a first binding domain; and a second single chain polypeptide comprising a second binding domain, a CL region, a hinge, an Fc region portion, and a second CL region.

Exemplary embodiments (51) to (60) are also provided below in which three or four binding domains are included in each heterodimer. Additional binding domains may be included to make polypeptide heterodimers that comprise five to eight binding domains according to the present disclosure in view of the general description provided herein.

In embodiment (51), a polypeptide heterodimer comprises the following two single chain polypeptides: from amino to carboxyl terminus, a first single chain polypeptide comprising a first binding domain, a CH1 region, a hinge, an Fc region portion, a second hinge and a second binding domain; and a second single chain polypeptide comprising a third binding domain, a CL region, a hinge and an Fc region portion.

In embodiment (52), a polypeptide heterodimer comprises the following two single chain polypeptides: from amino to carboxyl terminus, a first single chain polypeptide comprising a first binding domain, a CH1 region, a hinge, an Fc region portion, a second hinge, and a second binding domain; and a second single chain polypeptide comprising a CL region, a hinge, an Fc region portion, a second hinge and a third binding domain.

In embodiment (53), a polypeptide heterodimer comprises the following two single chain polypeptides: from amino to carboxyl terminus, a first single chain polypeptide comprising a first binding domain, a CL region, a hinge, an Fc region portion, a second hinge and a second binding domain; and the second single chain polypeptide comprising a third binding domain, a CH1 region, a hinge and an Fc region portion.

In embodiment (54), a polypeptide heterodimer comprises the following two single chain polypeptides: from amino to carboxyl terminus, a first single chain polypeptide comprising a first binding domain, a CL region, a hinge, an Fc region portion, a second hinge and a second binding domain; and a second single chain polypeptide comprising a CH1 region, a hinge, an Fc region portion, a second hinge and a third binding domain.

In embodiment (55), a polypeptide heterodimer comprises the following two single chain polypeptides: from amino to carboxyl terminus, a first single chain polypeptide comprising a first binding domain, a hinge, an Fc region portion, a CH1 region, a second hinge and a second binding domain; and the second single chain polypeptide comprising a third binding domain, a hinge, an Fc region portion and a CL region.

In embodiment (56), a polypeptide heterodimer comprises the following two single chain polypeptides: from amino to carboxyl terminus, a first single chain polypeptide comprising a first binding domain, a hinge, an Fc region portion, a CH1 region, a second hinge, and a second binding domain; and a second single chain polypeptide comprising a hinge, an Fc region portion, a CL region, a second hinge, and a third binding domain.

In embodiment (57), a polypeptide heterodimer comprises the following two single chain polypeptides: from amino to carboxyl terminus, a first single chain polypeptide comprising a first binding domain, a hinge, an Fc region portion, a CL region, a second hinge, and a second binding domain; and a second single chain polypeptide comprising a third binding domain, a hinge, an Fc region portion and a CH1 region.

In embodiment (58), a polypeptide heterodimer comprises the following two single chain polypeptides: from amino to carboxyl terminus, a first single chain polypeptide comprising a first binding domain, a hinge, an Fc region portion, a CL region, a second hinge, and a second binding domain; and a second single chain polypeptide comprising a hinge, an Fc region portion, a CH1 region, a second hinge, and a third binding domain.

In embodiment (59), a polypeptide heterodimer comprises the following two single chain polypeptides: from amino to carboxyl terminus: a first binding domain, a CH1 region, a hinge, an Fc region portion, a second hinge, and a second binding domain; and a second single chain polypeptide comprising a third binding domain, a CL region, a hinge, an Fc region portion, a second hinge and a fourth binding domain.

In embodiment (60), a polypeptide heterodimer comprises the following two single chain polypeptides: from amino to carboxyl terminus, a first single chain polypeptide comprising a first binding domain, a hinge, an Fc region portion, a CH1 region, a second hinge and a second binding domain; and a second single chain polypeptide comprising a third binding domain, a hinge, an Fc region portion, a CL region, a second hinge and a fourth binding domain.

In embodiments (1) to (32), a polypeptide heterodimer of the present disclosure comprises the following two single chain polypeptides: a first single chain polypeptide comprising a first binding domain (BD1) that specifically binds to a T cell target *(e.g.,* a TCR complex or a component thereof, including TCRα, TCRβ, CD3γ, CD3δ, and CD3ε), a hinge that is an SCC-P IgG1 hinge, an Fc region portion that is a wild type or altered human IgG1 CH2CH3, and a CL region that is wild type or an altered human Ck region with N30Y V55A T70E (YAE) substitutions; and a second single chain polypeptide comprising binding domain (BD2) that specifically binds to a B-cell target *(e.g.,* CD19, CD79b, HLA-DR, CD37, CD20) or a tumor or cancer antigen *(e.g.,* RON, c-Met, EpCAM, CEACAM-6, PSMA), a hinge that is also an SCC-P IgG1 hinge, an Fc region portion that is a wild type or altered human IgG1 CH2CH3, and a CH1 region that is human CH1 region.

In further embodiments, a polypeptide heterodimer of embodiments (1) to (32) may further comprise a third binding domain (BD3) that is identical to BD1 or BD2, and is linked to a single chain polypeptide via a second hinge *(e.g.,* linker H75 or H68 as set forth in SEQ ID NOS:742 and 78, respectively). In still further embodiments, a polypeptide heterodimer of embodiment of embodiments (1) to (32) may further comprise third and fourth binding domains (BD3) and (BD4) that each are identical or different to BD1 or BD2 and linked to the single chain polypeptide(s) via a second hinge *(e.g.,* linker H75 or H68 as set forth in SEQ ID NOS:742 and 78).

In certain embodiments, the polypeptide heterodimers of the present disclosure can be engineered to have binding domains with different affinities to target specific cell types. For example, it may be desirable for a polypeptide heterodimer with a binding domain for a TCR complex or a component thereof and another binding domain for a tumor antigen (or a B-cell target) to preferentially bind tumor cells having the tumor antigen (or B-cells carrying the B-cell target) with a higher affinity so that the polypeptide heterodimer will bind the tumor cells (or the B-cells) first and then recruit T cells via its TCR/CD3 binding domain to the tumor site or cells. Differential binding affinities may be achieved by, for example, choosing a binding domain for one target with a higher binding affinity than the other binding domain has for its target or by including multiple binding domains for one target on a polypeptide heterodimer and a single binding domain or fewer binding domains for the second or other targets. In addition, using different hinges (e.g., using hinges of different lengths) may affect binding of one domain more than another or using different hinges for different binding domains in order to alter the binding activity of the binding domains.

In certain embodiments, multiple binding domains may need to be located at an appropriate distance from each other so that their interactions with their targets will produce a desirable effect. For example, in certain embodiments, a polypeptide heterodimer comprising a binding domain for a TCR complex or a component thereof in a single chain polypeptide and another binding domain for a tumor antigen or a B-cell target in a second single chain polypeptide may have both binding domains at the amino or carboxyl termini of their corresponding chains so that they are in physical proximity to each other in the polypeptide heterodimer.

Exemplary heterodimers may be formed from single chain polypeptide pairs described herein. If sequence identification numbers noted herein contain signal peptide sequences (*e.g.,* the first 20 amino acids), such signal peptide sequences are not part of the mature single chain polypeptides that form the exemplary polypeptide heterodimers and thus should be considered excluded.

Exemplary single chain polypeptides are set forth in SEQ ID NOS:2, 4, 6, 8, 10, 12, 14, 16, 18, 20, 22, 24, 29-32, 53-72, 74, 810-826, 859-864, and 874-882.

Exemplary heterodimers may be formed from the following single chain polypeptide pairs: SEQ ID NOS:2 and 4, SEQ ID NOS:6 and 8, SEQ ID NOS:10 and 12, SEQ ID NOS:14 and 16, SEQ ID NOS:18 and 20, SEQ ID NOS:20 and 22, SEQ ID NOS:20 and 24, SEQ ID NOS:30 and 32, SEQ ID NOS:29 and 31, SEQ ID NOS:29 and 32, SEQ ID NOS:30 and 72, SEQ ID NOS:53 and 72, SEQ ID NOS:54 and 72, SEQ ID NOS:55 and 72, SEQ ID NOS:70 and 72, SEQ ID NOS:71 and 72, SEQ ID NOS:63 and 56, SEQ ID NOS:64 and 57, SEQ ID NOS:65 and 60, SEQ ID NOS:66 and 58, SEQ ID NOS:67 and 59, SEQ ID NOS:68 and 61, SEQ ID NOS:69 and 62, SEQ ID NOS:54 and 811, SEQ ID NOS:54 and 812, SEQ ID NOS:54 and 813, SEQ ID NOS:814 and 818, SEQ ID NOS:815 and 818, SEQ ID NOS:816 and 818, SEQ ID NOS:817 and 818, SEQ ID NOS:814 and 820, SEQ ID NOS:814 and 821, SEQ ID NOS:54 and 819, SEQ ID NOS:814 and 826, SEQ ID NOS:814 and 822, SEQ ID NOS:814 and 823, SEQ ID NOS:814 and 824, SEQ ID NOS:859 and 862, SEQ ID NOS:860 and 863, SEQ ID NOS:861 and 864, SEQ ID NOS:874 and 825, SEQ ID NOS:875 and 879, SEQ ID NOS:876 and 880, SEQ ID NOS:877 and 881, or SEQ ID NOS:878 and 882.

### Nucleic Acids, Vectors, Host Cells, and Methods for Making Heterodimers

In a related aspect, the present disclosure also provides isolated nucleic acid (used interchangeably with "polynucleotide") molecules that encode single chain polypeptides provided herein. Exemplary nucleic acid molecules (either with or without a nucleotide sequence encoding a signal peptide sequence) are set forth in SEQ ID NOS:1, 3, 5, 7, 9, 11, 13, 15,17, 19, 21, 23, 25-28, 33-52 and 792-808.

The present disclosure also provides vectors that comprise nucleic acid sequence encoding single chain polypeptides provided herein. As used herein, "vector" refers to a nucleic acid molecule capable of transporting another nucleic acid to which it has been linked. Exemplary vectors include plasmids, yeast artificial chromosomes, and viral genomes. Certain vectors can autonomously replicate in a host cell, while other vectors can be integrated into the genome of a host cell and thereby are replicated with the host genome.

In certain embodiments, the vectors may be recombinant expression vectors. "Recombinant expression vectors" or "expression vectors" refer to vectors that contain nucleic acid sequences that are operatively linked to an expression control sequence (*e.g.,* a promoter) and are thus capable of directing the expression of those sequences.

Promoter sequences useful in expression vectors provided herein can be selected from any desired gene using CAT (chloramphenicol transferase) vectors or other vectors with selectable markers. Eukaryotic promoters include CMV immediate early, HSV thymidine kinase, early and late SV40, LTRs from retrovirus, and mouse metallothionein-I. In certain embodiments, the promoters are inducible promoters.

In certain embodiments, a vector is an expression vector that comprises a nucleic acid sequence encoding a first single chain polypeptide of a polypeptide heterodimer provided herein. In certain other embodiments, a vector is an expression vector that comprises a nucleic acid sequence encoding a second single chain polypeptide of a polypeptide heterodimer provided herein.

In certain embodiments, a vector is an expression vector that comprises nucleic acid sequences encoding both first and second single chain polypeptides of a polypeptide heterodimer. The promoter for the nucleic acid sequence encoding the first single chain polypeptide may be the same as the promoter for the nucleic acid encoding the second single chain polypeptide. Alternatively, the promoter for the nucleic acid sequence encoding the first single chain polypeptide may be different from the promoter for the nucleic acid encoding the second single chain polypeptide so that the expression level of the first and second single chain polypeptides may be differentially modulated to maximum heterodimerization of the first and second single chain polypeptides. In certain embodiments, one or both the promoters for the nucleic acid encoding the first and second single chain polypeptides are inducible promoters.

The present disclosure also provides a host cell transformed or transfected with, or otherwise containing, any of the nucleic acids or vectors provided herein. Exemplary host cells include VERO cells, HeLa cells, Chinese hamster ovary (CHO) cell lines (including modified CHO cells capable of modifying the glycosylation pattern of expressed multivalent binding molecules, *see* US Patent Application Publication No. 2003/0115614), COS cells (such as COS-7), W138, BHK, HepG2, 3T3, RIN, MDCK, A549, PC 12, K562, HEK293 cells, HepG2 cells, N cells, 3T3 cells, *Spodoptera frugiperda* cells *(e.g.,* Sf9 cells), *Saccharomyces cerevisiae* cells, *Escherichia coli, Bacillus subtilis, Salmonella typhimurium,* or members of the *Streptomycete* family.

In certain embodiments, a host cell comprises a first expression vector containing a nucleic acid encoding a first single chain polypeptide and a second expression vector containing a nucleic acid encoding a second single chain polypeptide.

In certain other embodiments, a host cell comprises an expression vector containing a nucleic acid encoding both first and second single chain polypeptides.

The disclosure also includes a method of producing polypeptide heterodimers described herein. In certain embodiments, the method comprises culturing a host cell that comprises nucleic acids encoding both the first and second single chain polypeptides under conditions suitable to express the polypeptides, and optionally isolating or purifying the heterodimers formed from the first and second single chain polypeptides from the culture. The nucleic acid encoding the first single chain polypeptide and the nucleic acid encoding the second single chain polypeptide may be present in a single expression vector in the host cell or in two different expression vectors in the host cells. In the latter case, the ratio between the two expression vectors may be controlled to maximize heterodimerization of the first and second single chain polypeptides.

The present disclosure provides purified polypeptide heterodimers as described herein. The term "purified," as used herein, refers to a composition, isolatable from other components, wherein the polypeptide heterodimer is enriched to any degree relative to its naturally obtainable state. In certain embodiments, the present disclosure provides substantially purified polypeptide heterodimers as described herein. "Substantially purified" refers to a polypeptide heterodimer composition in which the polypeptide heterodimer forms the major component of the composition, such as constituting at least about 50%, such as at least about 60%, about 70%, about 80%, about 90%, about 95%, about 99%, of the polypeptides, by weight, in the composition.

Protein purification techniques are well known to those of skill in the art. These techniques involve, at one level, the crude fractionation of the polypeptide and non-polypeptide fractions. Further purification using chromatographic and electrophoretic techniques to achieve partial or complete purification (or purification to homogeneity) is frequently desired. Analytical methods particularly suited to the preparation of a pure fusion protein are ion-exchange chromatography, size exclusion chromatography; polyacrylamide gel electrophoresis; and isoelectric focusing. Particularly efficient methods of purifying peptides are fast protein liquid chromatography and HPLC.

Various methods for quantifying the degree of purification are known to those of skill in the art in light of the present disclosure. These include, for example, assessing the amount of polypeptide heterodimers in a fraction by SDS/PAGE analysis and HPLC as illustrated in the examples provided herein.

The method for making polypeptide heterodimers provided herein is advantageous over a method for first expressing and purifying separately individual single chain polypeptides and then incubating purified individual single chain polypeptides together to form polypeptide heterodimers. For example, certain single chain polypeptides (*e.g.,* certain polypeptides containing only CH1 regions as their immunoglobulin heterodimerization domains) are unstable when expressed alone. In addition, separate expression and purification of individual single chain polypeptides followed by combining the purified individual single chain polypeptides involve more steps than coexpressing both single chain polypeptides followed by purifying resulting polypeptide heterodimers and generally less efficient.

### Compositions and Methods for Using Heterodimers

In addition to polypeptide heterodimers, the present disclosure also provides pharmaceutical compositions and unit dose forms that comprise the polypeptide heterodimers as well as methods for using the polypeptide heterodimers, the pharmaceutical compositions and unit dose forms.

Compositions of polypeptide heterodimers of this disclosure generally comprise a polypeptide heterodimer provided herein in combination with a pharmaceutically acceptable excipient, including pharmaceutically acceptable carriers and diluents. Pharmaceutical acceptable excipients will be nontoxic to recipients at the dosages and concentrations employed. They are well known in the pharmaceutical art and described, for example, in Rowe et al., Handbook of Pharmaceutical Excipients: A Comprehensive Guide to Uses, Properties, and Safety, 5th Ed., 2006.

Pharmaceutically acceptable carriers for therapeutic use are also well known in the pharmaceutical art, and are described, for example, in Remington's Pharmaceutical Sciences, Mack Publishing Co. (A.R. Gennaro (Ed.) 1985). Exemplary pharmaceutically acceptable carriers include sterile saline and phosphate buffered saline at physiological pH. Preservatives, stabilizers, dyes and the like may be provided in the pharmaceutical composition. In addition, antioxidants and suspending agents may also be used.

Pharmaceutical compositions may also contain diluents such as buffers, antioxidants such as ascorbic acid, low molecular weight (less than about 10 residues) polypeptides, proteins, amino acids, carbohydrates (*e.g.,* glucose, sucrose, dextrins), chelating agents (*e.g.,* EDTA), glutathione and other stabilizers and excipients. Neutral buffered saline or saline mixed with nonspecific serum albumin are exemplary diluents. For instance, the product may be formulated as a lyophilizate using appropriate excipient solutions (*e.g.,* sucrose) as diluents.

The present disclosure also provides a method for treating a disease or disorder associated with, for example, excessive receptor-mediated signal transduction, comprising administering to a patient in need thereof an effective amount of a polypeptide heterodimer comprising a binding domain that specifically binds a receptor, such as a receptor tyrosine kinase, including EGFR, EGFRvIII, ErbB2, ErbB3, ErbB4, IGF1R, c-Met, RON, and EphA2.

Exemplary diseases or disorders associated with excess receptor-mediated signal transduction include cancer *(e.g.,* solid malignancy and hematologic malignancy), autoimmune or inflammatory diseases or conditions, sepsis resulting from bacterial infection, and viral infection.

In one aspect, the present disclosure provides a method for directing T cell activation, comprising administering to a patient in need thereof an effective amount of a polypeptide heterodimer that comprises a binding domain that specifically binds TCRα, TCRβ, CD3γ, CD3δ, CD3ε or a combination thereof, and a second binding domain that specifically binds a different target, for instance, a tumor-specific antigen or other antigen of choice at a site or cell where T cell activation is desired.

In another aspect, the present disclosure provides a method for inhibiting growth, metastasis or metastatic growth of a malignancy (*e.g.,* a solid malignancy or a hematologic malignancy), comprising administering to a patient in need thereof an effective amount of a polypeptide heterodimer provided herein or a composition thereof.

A wide variety of cancers, including solid malignancy and hematologic malignancy, are amenable to the compositions and methods disclosed herein. Types of cancer that may be treated include, but are not limited to: adenocarcinoma of the breast, prostate, pancreas, colon and rectum; all forms of bronchogenic carcinoma of the lung (including squamous cell carcinoma, adenocarcinoma, small cell lung cancer and non-small cell lung cancer); myeloid; melanoma; hepatoma; neuroblastoma; papilloma; apudoma; choristoma; branchioma; malignant carcinoid syndrome; carcinoid heart disease; and carcinoma (*e.g.,* Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell). Additional types of cancers that may be treated include: histiocytic disorders; leukemia; histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin; and glioblastoma multiforme. The types of cancers that may be treated also include, but are not limited to, angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

Additional exemplary cancers that are also amenable to the compositions and methods disclosed herein are B-cell cancers, including B-cell lymphomas [such as various forms of Hodgkin's disease, non-Hodgkins lymphoma (NHL) or central nervous system lymphomas], leukemias [such as acute lymphoblastic leukemia (ALL), chronic lymphocytic leukemia (CLL), Hairy cell leukemia and chronic myoblastic leukemia] and myelomas (such as multiple myeloma). Additional B cell cancers include small lymphocytic lymphoma, B-cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B-cell lymphoma of mucosa-associated (MALT) lymphoid tissue, nodal marginal zone B-cell lymphoma, follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma/leukemia, B-cell proliferations of uncertain malignant potential, lymphomatoid granulomatosis, and post-transplant lymphoproliferative disorder.

In certain embodiments, polypeptide heterodimers useful for inhibiting growth of a solid malignancy or metastasis or metastatic growth of a solid malignancy or a hematologic malignancy include those that specifically bind to a tumor or cancer antigen and a T cell target. Such heterodimers are typically designed to have a higher binding affinity to the tumor or cancer antigen (*e.g.,* at least about 5, 10, 15, 20, 25, 50, 75 or 100 folds higher) than to the T cell target so that they preferentially bind to the tumor or cancer antigen first and subsequently bind to the T cell target, which in turn recruit and activate T cells to damage or destroy the tumor or cancer cells carrying the tumor or cancer antigen on their surface. Exemplary tumor or cancer antigens and T cell targets include those provided above in the section describing binding domains of polypeptide heterodimers. For instance, a T cell target can be CD3 or PSMA.

In certain embodiments, polypeptide heterodimers useful for inhibiting growth, metastasis, or metastatic growth of a malignancy, or by directed T cell activation, comprise at least one binding domain that specifically binds an oncology target (including a tumor or cell antigen, such as RON, c-Met, CEACAM-6, or PSMA) and another binding domain that specifically binds a TCR complex or a component thereof (*e.g*., TCRα, TCR, CD3γ, CD3δ, and CD3ε).

In certain other embodiments, polypeptide heterodimers useful for inhibiting growth, metastasis, or metastatic growth of a malignancy comprise at least one binding domain that specifically binds CD28 and another binding domain that specifically binds CD79b. Such polypeptide heterodimers may further comprise a binding domain that comprises a PDL2 ectodomain, a binding domain that specifically binds hyperIL-6, or both binding domains.

In certain other embodiments, polypeptide heterodimers useful for inhibiting growth, metastasis, or metastatic growth of a malignancy comprise at least one binding domain that specifically binds RON and another binding domain that specifically binds c-Met.

In certain other embodiments, polypeptide heterodimers useful for inhibiting growth, metastasis, or metastatic growth of a malignancy comprise binding domains that specifically bind one or more of the following receptor tyrosine kinases: c-Met, RON, EGFR, EGFRvIII, Her2, ErbB3, ErbB4, and IGF1R, EphA2. In certain embodiments, the heterodimers may further comprise one or more binding domains that specifically bind to one or more of the following antiangiogenic agents: PDGFR, VEGFR1-4, and angiopoietin 2. In certain embodiments, the above heterodimers may further comprise one or more binding domains that specifically bind to one or more the following Fc receptors to increase targeting of cytotoxic effector function: CD64, CD32A and CD16. In certain embodiments, the above heterodimers may further comprise a binding domain that specifically binds the transferrin receptor (CD71) to enable degradation of the receptors.

In certain other embodiments, polypeptide heterodimers useful for inhibiting growth, metastasis, or metastatic growth of a malignancy comprise binding domains that are agonists of two or more of the following TNFSFRs: TNFR1, TNFR2, DR4, DR5, TWEAKR, and FAS.

In certain other embodiments, polypeptide heterodimers useful for inhibiting growth, metastasis, or metastatic growth of a malignancy comprise binding domains that specifically bind two or more of the following pro-oncogenic cytokines or growth factors: HGF, MSP, EGF (including epiregulin, herregulin, β-regulin, neuregulin), HIF-1α, VEGFA, VEGFB, VEGFC, VEGFD, TNFα, IL-6, hyperIL-6, IL-8, Wnt, sHH, TGFβ, or PDGF.

In certain embodiments, polypeptide heterodimers useful for inhibiting growth of a solid malignancy or metastasis or metastatic growth of a solid malignancy include those that specifically bind to, for example, EGFR, ErbB3, ErbB4, c-Met, RON, EphA2, IGF1R, VEGFR1, VEGFR2, VEGFR3, CD44v6, CD151, EpCAM, CEACAM6, TGFBR2, , GHRHR, GHR, IL-6R, gp130, TNFR2, PD1, TWEAK-R, OSMRβ, Patched-1, Frizzled, or Robol.

Polypeptide heterodimers useful for inhibiting metastasis or metastatic growth of a hematologic malignancy include those that specifically bind to, for example, EGFR, ErbB3, c-Met, RON, EphA2, IGF1R, TGFBR2, IL-6R, gp130, TNFR2, PD1, OSMRβ, LTβR, CD19, CD80, CD81, or CD86.

In another aspect, the present disclosure provides a method for treating an autoimmune or inflammatory disease, disorder or condition, comprising administering to a patient in need thereof an effective amount of a polypeptide heterodimer provided herein or a composition thereof.

Exemplary autoimmune or inflammatory diseases, disorders or conditions that may be treated by the fusion proteins and compositions and unit dose forms thereof include, and are not limited to, inflammatory bowel disease (*e.g.,* Crohn's disease or ulcerative colitis), diabetes mellitus (*e.g.,* type I diabetes), dermatomyositis, polymyositis, pernicious anaemia, primary biliary cirrhosis, acute disseminated encephalomyelitis (ADEM), Addison's disease, ankylosing spondylitis, antiphospholipid antibody syndrome (APS), autoimmune hepatitis, Goodpasture's syndrome, Graves' disease, Guillain-Barré syndrome (GBS), Hashimoto's disease, idiopathic thrombocytopenic purpura, systemic lupus erythematosus, lupus nephritis, neuropsychiatric lupus, multiple sclerosis (MS), myasthenia gravis, pemphigus vulgaris, asthma, psoriatic arthritis, rheumatoid arthritis, Sjögren's syndrome, temporal arteritis (also known as "giant cell arteritis"), autoimmune hemolytic anemia, Bullous pemphigoid, vasculitis, coeliac disease, chronic obstructive pulmonary disease, endometriosis, Hidradenitis suppurativa, interstitial cystitis, morphea, scleroderma, narcolepsy, neuromyotonia, vitiligo, and autoimmune inner ear disease.

Exemplary polypeptide heterodimers useful for treating an autoimmune or inflammatory disease, disorder or condition may comprises one or more binding domain that specifically binds CD28, and one, two, or three of the following additional binding domains: a binding domain that comprises PDL2 ectodomain, a binding domain that comprises monoIL-10, and a binding domain that specifically binds CD86. For example, a polypeptide heterodimer may comprise a binding domain that specifically binds CD28 and one to three binding domains that specifically bind CD86.

Additional exemplary polypeptide heterodimers useful for treating an autoimmune or inflammatory disease, disorder or condition may comprise one or more binding domains that specifically bind CD28 and one or more binding domains that are an IL-10 agonist (*e.g.,* monoIL-10), an HLA-G agonist, an HGF agonist, an IL-35 agonist, a PD-1 agonist, a BTLA agonist, a LIGHT antagonist, a GITRL antagonist or a CD40 antagonist. Alternatively, polypeptide heterodimers may comprise two or more binding domains selected from an IL-10 agonist (e.g., monoIL-10), an HLA-G agonist, an HGF agonist, an IL-35 agonist, a PD-1 agonist, a BTLA agonist, a LIGHT antagonist, a GITRL antagonist or a CD40 antagonist.

Additional exemplary polypeptide heterodimers useful for treating an autoimmune or inflammatory disease, disorder or condition may comprise one or more binding domains that specifically bind CD32B and one or more binding domains that are an IL-10 agonist (*e.g.,* monoIL-10), an HLA-G agonist, an HGF agonist, an IL-35 agonist, a PD-1 agonist, a BTLA agonist, a LIGHT antagonist, a GITRL antagonist or a CD40 antagonist.

Additional exemplary polypeptide heterodimers useful for treating an autoimmune or inflammatory disease, disorder or condition may comprise binding domains that specifically bind one or more of the following TNFSFRs: TNFR1, TNFR2, HVEW, LTβR, TWEAKR, TACI, BAFF-R, BCMA, or FAS.

Additional exemplary polypeptide heterodimers useful for treating an autoimmune or inflammatory disease, disorder or condition may comprise binding domains that specifically bind two or more of the following pro-inflammatory cytokines/chemokines, such as TNFα, IL-6, IL-2, IL-1, IL-8, IP-10, IFNγ, IFNα, RANKL, FASL, TGFβ, IL7, IL10, IL17A/F, TWEAK, CSF2, IGF1, IGF2 or BLyS/APRIL.

Polypeptide heterodimers useful for treating an autoimmune or inflammatory disease, disorder or condition include those that specifically bind to, for example, TGFBR2, IL-6R, gp130, TNFR1, TNFR2, PD1, HVEM, OX40, CD40, CD137, TWEAK-R, LTβR, LIFERβ, OSMRβ, CD3, TCRα, TCRβ CD19, CD28, CD80, CD81, CD86, TLR7, TLR9, or any combination thereof.

In another aspect, the present disclosure provides a method for treating a B-cell associated disorder or disease that comprises administering to a patient in need thereof *(e.g.,* a patient having or suspected of having a B-cell associated disorder or disease) an effective amount of a polypeptide heterodimer provided herein, such as those that specifically bind a B cell target.

Polypeptide heterodimers useful in treating a B-cell associated disorder or disease may comprise binding domains that specifically bind one or more B-cell targets, such as CD79b, CD19, HLA-DR, CD20, CD21, CD22, CD30, CD33, CD37, CD38, or CD70. The polypeptide heterodimers may further comprise a binding domain that specifically binds a T-cell target, such as a TCR complex or a component thereof, including TCRα, TCRβ, CD3γ, CD3δ, or CD3ε.

Polypeptide heterodimers useful in treating a B-cell associated disorder or disease may comprise a binding domain that specifically bind a B-cell target, such as CD79b, CD19, HLA-DR, CD20, CD21, CD22, CD30, CD33, CD37, CD38, and CD70, and a binding domain that specifically binds CD64, CD32A, or CD16 to increase targeting of cytotoxic effector function.

The polypeptide heterodimers or compositions thereof of the present disclosure may be administered orally, topically, transdermally, parenterally, by inhalation spray, vaginally, rectally, or by intracranial injection, or any combination thereof. In one embodiment, the polypeptide heterodimers or compositions thereof are administered parenterally. The term "parenteral," as used herein, includes subcutaneous injections, intravenous, intramuscular, intracisternal injection, or infusion techniques. Administration by intravenous, intradermal, intramusclar, intramammary, intraperitoneal, intrathecal, retrobulbar, intrapulmonary injection and/or surgical implantation at a particular site is contemplated as well. For instance, the invention includes administering polypeptide heterodimers or compositions thereof by intravenous injection.

The pharmaceutically effective dose depends on the type of disease, the composition used, the route of administration, the type of subject being treated, the physical characteristics of the specific subject under consideration for treatment, concurrent medication, and other factors that those skilled in the medical arts will recognize. For example, an amount between 0.01 mg/kg and 1000 mg/kg (*e.g.,* about 0.1 to 1 mg/kg, about 1 to 10 mg/kg, about 10-50 mg/kg, about 50-100 mg/kg, about 100-500 mg/kg, or about 500-1000 mg/kg) body weight (which can be administered as a single dose, daily, weekly, monthly, or at any appropriate interval) of active ingredient may be administered depending on the potency of a polypeptide heterodimer of this disclosure.

Also contemplated is the administration of polypeptide heterodimers or compositions thereof in combination with a second agent. A second agent may be one accepted in the art as a standard treatment for a particular disease state or disorder, such as in cancer, inflammation, autoimmunity, and infection. Exemplary second agents contemplated include polypeptide heterodimers that bind to targets different from those that primary polypeptide heterodimers bind, polyclonal antibodies, monoclonal antibodies, immunoglobulin-derived fusion proteins, chemotherapeutics, ionizing radiation, steroids, NSAIDs, anti-infective agents, or other active and ancillary agents, or any combination thereof.

In certain embodiments, a polypeptide heterodimer and a second agent act synergistically. In other words, these two compounds interact such that the combined effect of the compounds is greater than the sum of the individual effects of each compound when administered alone (*see, e.g.,* Berenbaum, Pharmacol. Rev. 41:93, 1989).

In certain other embodiments, a polypeptide heterodimer and a second agent act additively. In other words, these two compounds interact such that the combined effect of the compounds is the same as the sum of the individual effects of each compound when administered alone.

Second agents useful in combination with polypeptide heterodimers or compositions thereof provided herein may be steroids, NSAIDs, mTOR inhibitors (*e.g.,* rapamycin (sirolimus), temsirolimus, deforolimus, everolimus, zotarolimus, curcumin, farnesylthiosalicylic acid), calcineurin inhibitors (*e.g.,* cyclosporine, tacrolimus), antimetabolites (*e.g.,* mycophenolic acid, mycophenolate mofetil), polyclonal antibodies *(e.g.,* anti-thymocyte globulin), monoclonal antibodies *(e.g.,* daclizumab, basiliximab), and CTLA4-Ig fusion proteins (*e.g.,* abatacept or belatacept).

Second agents useful for inhibiting growth of a solid malignancy, inhibiting metastasis or metastatic growth of a solid malignancy, or treating or ameliorating a hematologic malignancy include chemotherapeutic agents, ionizing radiation, and other anti-cancer drugs. Examples of chemotherapeutic agents contemplated as further therapeutic agents include alkylating agents, such as nitrogen mustards (e.g., mechlorethamine, cyclophosphamide, ifosfamide, melphalan, and chlorambucil); bifunctional chemotherapeutics (*e.g.,* bendamustine); nitrosoureas (e.g., carmustine (BCNU), lomustine (CCNU), and semustine (methyl-CCNU)); ethyleneimines and methyl-melamines (e.g., triethylenemelamine (TEM), triethylene thiophosphoramide (thiotepa), and hexamethylmelamine (HMM, altretamine)); alkyl sulfonates (e.g., buslfan); and triazines (e.g., dacabazine (DTIC)); antimetabolites, such as folic acid analogues (e.g., methotrexate, trimetrexate, and pemetrexed (multi-targeted antifolate)); pyrimidine analogues (such as 5-fluorouracil (5-FU), fluorodeoxyuridine, gemcitabine, cytosine arabinoside (AraC, cytarabine), 5-azacytidine, and 2,2'-difluorodeoxycytidine); and purine analogues (e.g., 6-mercaptopurine, 6-thioguanine, azathioprine, 2'-deoxycoformycin (pentostatin), erythrohydroxynonyladenine (EHNA), fludarabine phosphate, 2-chlorodeoxyadenosine (cladribine, 2-CdA)); Type I topoisomerase inhibitors such as camptothecin (CPT), topotecan, and irinotecan; natural products, such as epipodophylotoxins (e.g., etoposide and teniposide); and vinca alkaloids (e.g., vinblastine, vincristine, and vinorelbine); anti-tumor antibiotics such as actinomycin D, doxorubicin, and bleomycin; radiosensitizers such as 5-bromodeozyuridine, 5-iododeoxyuridine, and bromodeoxycytidine; platinum coordination complexes such as cisplatin, carboplatin, and oxaliplatin; substituted ureas, such as hydroxyurea; and methylhydrazine derivatives such as N-methylhydrazine (MIH) and procarbazine.

In certain embodiments, second agents useful for inhibiting growth metastasis or metastatic growth of a malignancy include polypeptide heterodimers according to the present disclosure that bind to cancer cell targets other than the target that the first polypeptide heterodimer binds. In certain other embodiments, second agents useful for such treatments include polyclonal antibodies, monoclonal antibodies, and immunoglobulin-derived fusion proteins that bind to cancer cell targets. Exemplary cancer cell targets are provided above in the context of describing targets of polypeptide heterodimers useful for the above-noted treatment.

Further therapeutic agents contemplated by this disclosure for treatment of autoimmune diseases are referred to as immunosuppressive agents, which act to suppress or mask the immune system of the individual being treated. Immunosuppressive agents include, for example, non-steroidal anti-inflammatory drugs (NSAIDs), analgesics, glucocorticoids, disease-modifying antirheumatic drugs (DMARDs) for the treatment of arthritis, or biologic response modifiers. Compositions in the DMARD description are also useful in the treatment of many other autoimmune diseases aside from rheumatoid arthritis.

Exemplary NSAIDs are chosen from the group consisting of ibuprofen, naproxen, naproxen sodium, Cox-2 inhibitors such as Vioxx and Celebrex, and sialylates. Exemplary analgesics are chosen from the group consisting of acetaminophen, oxycodone, tramadol of proporxyphene hydrochloride. Exemplary glucocorticoids are chosen from the group consisting of cortisone, dexamethasone, hydrocortisone, methylprednisolone, prednisolone, or prednisone. Exemplary biological response modifiers include molecules directed against cell surface markers (e.g., CD4, CD5, etc.), cytokine inhibitors, such as the TNF antagonists (e.g. etanercept (Enbrel), adalimumab (Humira) and infliximab (Remicade)), chemokine inhibitors and adhesion molecule inhibitors. The biological response modifiers include monoclonal antibodies as well as recombinant forms of molecules. Exemplary DMARDs include azathioprine, cyclophosphamide, cyclosporine, methotrexate, penicillamine, leflunomide, sulfasalazine, hydroxychloroquine, Gold (oral (auranofin) and intramuscular) and minocycline.

Additional second agents useful for treating an autoimmune or inflammatory disease, disorder or condition may be a polyclonal or monoclonal antibody, an immunoglobulin-derived fusion protein (e.g., scFv, SMIP™, PIMS, SCORPION™ fusion proteins), or a polypeptide heterodimer according to the present disclosure that specifically bind a target associated with such a disease, disorder or condition. Examples of such targets are provided above in the context of targets of polypeptide heterodimers of the present disclosure useful in the above-noted treatment.

It is contemplated the binding molecule composition and the second active agent may be given simultaneously in the same formulation. Alternatively, the second agents may be administered in a separate formulation but concurrently (*i.e*., given within less than one hour of each other).

In certain embodiments, the second active agent may be administered prior to administration of a polypeptide heterodimer or a composition thereof. Prior administration refers to administration of the second active agent at least one hour prior to treatment with the polypeptide heterodimer or the composition thereof. It is further contemplated that the active agent may be administered subsequent to administration of the binding molecule composition. Subsequent administration is meant to describe administration at least one hour after the administration of the polypeptide heterodimer or the composition thereof.

This disclosure contemplates a dosage unit comprising a pharmaceutical composition of this disclosure. Such dosage units include, for example, a single-dose or a multi-dose vial or syringe, including a two-compartment vial or syringe, one comprising the pharmaceutical composition of this disclosure in lyophilized form and the other a diluent for reconstitution. A multi-dose dosage unit can also be, *e.g.,* a bag or tube for connection to an intravenous infusion device.

This disclosure also contemplates a kit comprising a pharmaceutical composition of this disclosure in unit dose, or multi-dose, container, *e.g.,* a vial, and a set of instructions for administering the composition to patients suffering a disorder such as a disorder described above.

### EXAMPLES

### EXAMPLE 1

### BIVALENT POLYPEPTIDE HETERODIMER WITH TWO PAIRS OF HETERODIMERIZATION DOMAINS

Polypeptide heterodimer X0172 was made by co-expressing single chain polypeptides X0130 (2E12 CH1 CH2 CH3 Ck) and X0168 (Ck CH2 CH3 CH1 H68 2E12). Single chain polypeptide X0130 comprises, from its amino to carboxyl terminus: 2E12 (anti-CD28) scFv, human IgG1 CH1, human IgG1 SCC-P hinge, human IgG1 CH2, human IgG1 CH3, and altered human Ck (without the first Arg or the last Cys). The nucleic acid and amino acid sequences of X130 are set forth in SEQ ID NOS:1 and 2, respectively. Single chain polypeptide X0168 comprises, from its amino to carboxyl terminus: altered human Ck (without the first Arg or the last Cys), human IgG1 SCC-P hinge, human IgG1 CH2, human IgG1 CH3, human IgG1 CH1, H68 linker, and 2E12 (anti-CD28) scFv. The nucleic acid and amino acid sequences of X0168 are set forth in SEQ ID NOS:3 and 4, respectively. The amino acid sequence of H68 linker is set forth in SEQ ID NO:78.

For comparison, polypeptide heterodimer X0124 was made by co-expressing single chain polypeptide X0112 (2E12 CH1 CH2 CH3) and X0113 (Ck CH2 CH3). Single chain polypeptide X0112 comprises, from its amino to carboxyl terminus: 2E12 (anti-CD28) scFv, human IgG1 CH1, human IgG1 SCC-P hinge, human IgG1 CH2, and human IgG1 CH3. The nucleic acid and amino acid sequences of X0112 are set forth in SEQ ID NOS:5 and 6, respectively. Single chain polypeptide X0113 comprises, from its amino to carboxyl terminus: altered human Ck (without the first Arg or the last Cys), human IgG1 SCC-P hinge, human IgG1 CH2, and human IgG1 CH3. The nucleic acid and amino acid sequence of X0113 are set forth in SEQ ID NOS:7 and 8, respectively.

### Expression

The day before transfection, HEK293 cells were suspended at a cell concentration of 0.5x10⁶ cells/ml in Freestyle 293 expression medium (Gibco). For a large transfection, 250 ml of cells were used, but for a small transfection, 60 ml of cells were used. On the transfection day, 320 ul of 293fectin reagent (Invitrogen) was mixed with 8 ml of media. At the same time, 250 ug of DNA for each of the two chains were also mixed with 8 ml of media and incubated for 5 minutes. After 15 minutes of incubation, the DNA-293fectin mixture was added to the 250ml of 293 cells and returned to the shaker at 37°C and shaken at a speed of 120 RPM. For the smaller transfection using 60 ml of cells, a fourth of the DNA, 293fectin and media were used.

### Purification

Protein A affinity chromatography was used to purify the proteins. 2 mL of packed protein A agarose (Repligen) was added to a Biorad column (Econo-column chromatography column, size 2.5 x 10 cm), washed extensively with PBS (10x column volume) and the supernatants were loaded, washed with PBS again and eluted with 3 column volumes of Pierce IgG elution buffer. Proteins were then dialyzed extensively against PBS. Proteins were then concentrated using Amicon centrifugal filter devices to a final volume of around 0.5 mL.

For second step purification, Protein L affinity chromatography or cation exchange chromatography were used. For Protein L purification, protein A purified Polypeptide heterodimer was passed over a Protein L agarose column that had been pre-equilibrated with PBS, washed with PBS (10x column volume) and then eluted with Pierce IgG elution buffer. Proteins were then dialyzed against PBS extensively and concentrated using Amicon centrifugal filter devices to a final volume of around 0.5 mL.

Samples (200-300 ug) of previously affinity purified (Protein A or Protein L) Polypeptide heterodimer constructs were dialyzed into 20 mM MES, pH 6.0 (Buffer A) and loaded onto a MonoS 5/50 GL cation exchange column (GE Healthcare) at a flow rate of 2 mL/min, using an AKTA Explorer FPLC. The column was allowed to equilibrate for 5 column volumes (CV) and then run in a gradient format to a mixture of 50%:50% buffer A:buffer B (buffer B being 20 mM MES, 1 M NaCl, pH 6.0) over 20 CV. A following mixture of 100% buffer B was run for 5 CV to clean the column, and the system was run for another 5 CV at 100% buffer A to re-equilibrate prior to the next injection. Peaks were collected and analyzed by SDS-PAGE and electrospray mass spectrometry.

### SDS-PAGE Analysis

Proteins purified were analyzed on a 10 % SDS-PAGE gel using Invitrogen's X-cell Surelock gel box.

### Synergy with Suboptimal Concentration of PMA

Peripheral blood mononuclear cells (PBMC) from in-house donors were isolated from heparinized whole blood via centrifugation over Lymphocyte Separation Media (MP Biomedicals, Aurora, OH) and washed two times with RPMI media (Gibco-Invitrogen, Carlsbad, CA). CD4+ T-cells were then enriched from the PBMC using negative selection with a MACS CD4+ T-cell Isolation Kit (Miltenyi Biotec, Auburn, CA). The enriched (>95%) CD4+ T-cells were then resuspended at a concentration of 1x10⁶ cell/ml in complete RPMI/10% FCS. Test reagents were prepared at 40 ug/ml (yielding a final concentration of 10 µg/ml) in complete RPMI/10% FCS and added in 50 µl/well to flat-bottom 96-well plates (BD Falcon, San Jose, CA). PMA (Phorbol 12 myristate 13-acetate; A.G. Scientific, Inc., San Diego, CA) in complete RPMI/10% FCS was added in 50 ul/well at 4 µg/ml (final concentration of 1 µg/ml). Then T-cells in complete RPMI/10% FCS were added at a concentration of 5 x 10⁴ cells/well in a 50 µl volume, and finally an appropriate amount of complete RPMI/10% FCS was added to each well (typically 50 ul) to bring the final volume to 200 ul/well. The cells were treated with the test samples +/- PMA and incubated for 72 hours at 37°C in 5% CO₂. One microliter of tritiated thymidine (Amersham Biosciences, Pisctaway, NJ) in a 1:50 dilution of complete RPMI/10% FCS (50 ul/well) was added to the wells for the last 6 hours of culture. Plates were harvested onto a Unifilter-96, GF/C microplate (Perkin Elmer, Boston, MA) with a Packard Filtermate Harvester (Perkin Elmer, Boston, MA). Numbers are expressed as cpm and are the mean of replicate samples.

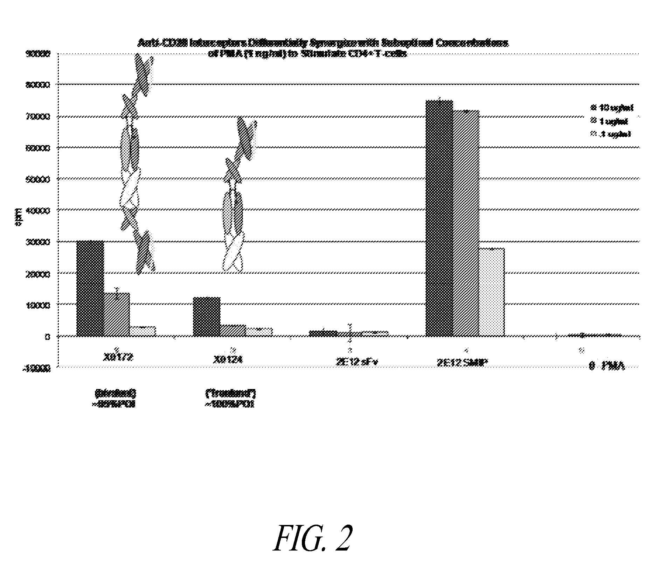

Figure 2 shows that Polypeptide heterodimer X0172 has a differential property compared to 2E12 SMIP M0039 (SEQ ID NO:77). It did not synergize with PMA as well as the SMIP. This bivalent polypeptide heterodimer has a property closer to the monovalent polypeptide heterodimer, X0124, rather than the bivalent SMIP.

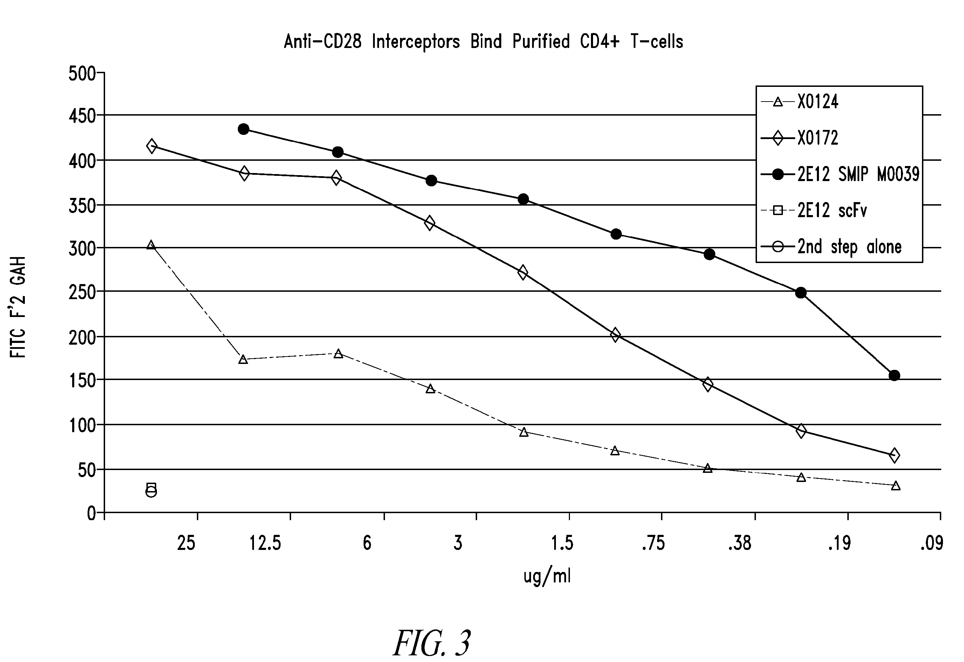

Figure 3 shows that bivalent polypeptide heterodimer X0172 bound to CD4⁺ cells better than 2E12 scFv (SEQ ID NO:109) and monovalent polypeptide heterodimer X0124.

### EXAMPLE 2

### BIVALENT, TRIVALENT, TETRAVALENT POLYPEPTIDE HETERODIMER USING SINGLE HETERODIMERIZATION DOMAIN PAIR

Bivalent polypeptide heterodimer X0251 was made by co-expressing single chain polypeptides X0244 (Ck(YAE) CH2(N297A) CH3 H68 2E12) and X0245 (2E12 CH1 CH2(N297A) CH3). Single chain polypeptide X0244 comprises, from its amino to carboxyl terminus: human Ck(YAE) (*i.e*., human Ck without the first Arg or last Cys but with N30Y, V55A, and T70E substitutions), human IgG1 SCC-P hinge, human IgG1 CH2 (N297A) (*i.e*., human IgG1 CH2 with a N297A substitution), human IgG1 CH3, H68 linker, and 2E12 (anti-CD28) scFv. The nucleotide and amino acid sequences of X0244 are set forth in SEQ ID NOS:9 and 10, respectively. Single chain polypeptide X0245 comprises, from its amino to carboxyl terminus: 2E12 (anti-CD28) scFv, human IgG1 CH1, human IgG1 SCC-P hinge, human IgG1 CH2 (N297A), and human IgG1 CH3. The nucleotide and amino acid sequences of X0245 are set forth in SEQ ID NOS:11 and 12, respectively.

Trivalent, bispecific polypeptide heterodimer X0252 was made by co-expressing single chain polypeptides X0246 (P2C2 Ck(YAE) CH2(N297A) CH3) and X0247 (2E12 CH1 CH2(N297A) CH3 H68 2E12). Single chain polypeptide X0246 comprises, from its amino to carboxyl terminus: P2C2 (anti-CD79b) scFv, human Ck (YAE), human IgG1 SCC-P hinge, human IgG1 CH2 (N297A), and human IgG1 CH3. The nucleotide and amino acid sequences of X0246 are set forth in SEQ ID NOS:13 and 14, respectively. Single chain polypeptide X0247 comprises, from its amino to carboxyl terminus: 2E12 (anti-CD28) scFv, human IgG1 CH1, human IgG1 SCC-P hinge, human IgG1 CH2 (N297A), human IgG1 CH3, H68 linker, and 2E12 (anti-CD28) scFv. The nucleotide and amino acid sequences of X0247 are set forth in SEQ IDNOS:15 and 16, respectively.

Tetravalent, trispecific polypeptide heterodimer X0253 was made by co-expressing single chain polypeptides X0248 (P2C2 CK(YAE) CH2(N297A) CH3 H68 A2) and X0249 (PDL2 ECD CH1 CH2(N297A) CH3 H68 2E12). Single chain polypeptide X0248 comprises, from its amino to carboxyl terminus: P2C2 (anti-CD79b) scFv, human Ck (YAE), human IgG1 SCC-P hinge, human IgG1 CH2 (N297A), human IgG1 CH3, H68 linker, and A2 (anti-hyperIL-6) scFv. The nucleotide and amino acid sequences of X0248 are set forth in SEQ ID NOS:17 and 18, respectively. Single chain polypeptide X0249 comprises, from its amino to carboxyl terminus: PDL2 ECD (*i.e*., PDL2 ectodomain), human IgG1 CH1, human IgG1 SCC-P hinge, human IgG1 CH2 (N297A), human IgG1 CH3, H68 linker, and 2E12 (anti-CD28) scFv. The nucleotide and amino acid sequences of X0249 are set forth in SEQ ID NOS:19 and 20, respectively.

Another tetravalent, tetraspecific polypeptide heterodimer X0283, was made by co-expressing single chain polypeptides X0249 (PDL2 ECD CH1 CH2(N297A) CH3 H68 2E12) and X0281 (monoIL-10 Ck(YAE) CH2(N297A) CH3 H68 3D1). Single chain polypeptide X0281 comprises, from its amino to carboxyl terminus: monoIL-10, human Ck(YAE), human IgG1 SCC-P hinge, human IgG1 CH2 (N297A), human IgG1 CH3, H68 linker, and 3D1(anti-CD86) scFv. The nucleotide and amino acid sequences of X0281 are set forth in SEQ ID NOS:21 and 22.

As a control for polypeptide heterodimer X0283, tetravalent heterodimer, polypeptide heterodimer X0284, was made by co-expressing single chain polypeptides X0249 (PDL2 ECD CH1 CH2(N297A) CH3 H68 2E12) and X0282 (monoIL-10 Ck CH2(N297A) CH3 H68 3D1). Single chain polypeptide X0282 is identical to X0281 except that it does not contain N30Y V55A T70E substitutions in the human Ck sequence. Thus, single chain polypeptide X0282 comprises, from its amino to carboxyl terminus: monoIL-10, altered human Ck (without the first Arg or the last Cys), human IgG1 SCC-P hinge, human IgG1 CH2 (N297A), human IgG1 CH3, H68 linker, and 3D1(anti-CD86) scFv. The nucleotide and amino acid sequences of X0282 are set forth in SEQ ID NOS:23 and 24, respectively.

The polypeptide heterodimer molecules were expressed and purified according to Example 1. Purified proteins were analyzed using SDS-PAGE electrophoresis under reduced and non-reduced conditions. Size exclusion chromatography was performed on an AKTA Explorer FPLC (Pharmacia Biotech) using a Superdex200 10/300 GL column. Some proteins were analyzed by electrospray mass spectrometry using an Agilent 6120 TOF ES/MS.

Surface plasmon resonance (SPR) measurements were performed on a Biacore T100 SPR using HBS-P+ (GE Healthcare) as a running buffer. Target was directly immobilized onto a CM5 chip using standard amine coupling chemistry (BIACORE® Amine Coupling Kit, GE Healthcare), with final immobilization levels between 800 and 1900 Ru (resonance units). Polypeptide heterodimer X0283 was injected at 25°C or 37°C for 150 seconds at a flow rate of 30 µl/min in a series of concentrations from 10 nM to 1 µM. Dissociation was monitored for 1200 seconds, and the surface was regenerated by injecting 50 mM NaOH for 60 seconds. Binding interactions with the surface were stable through at least 60 regeneration cycles. Data were analyzed using BiaEvaluation for the T100 software (version 2.0, GE Healthcare).

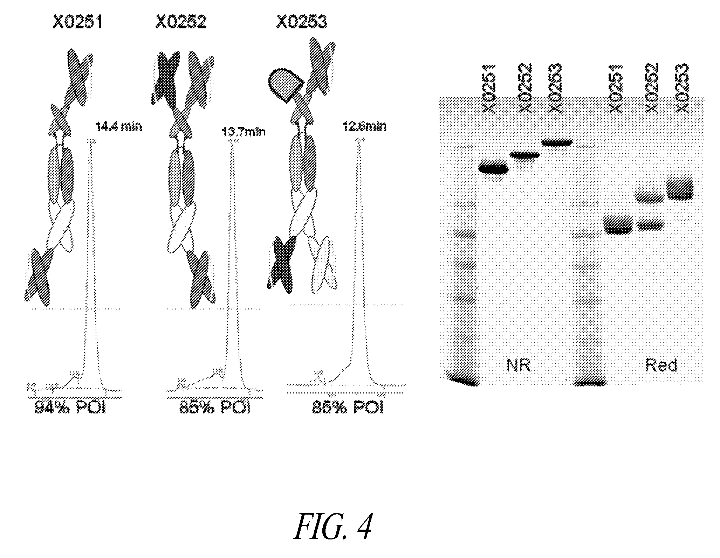

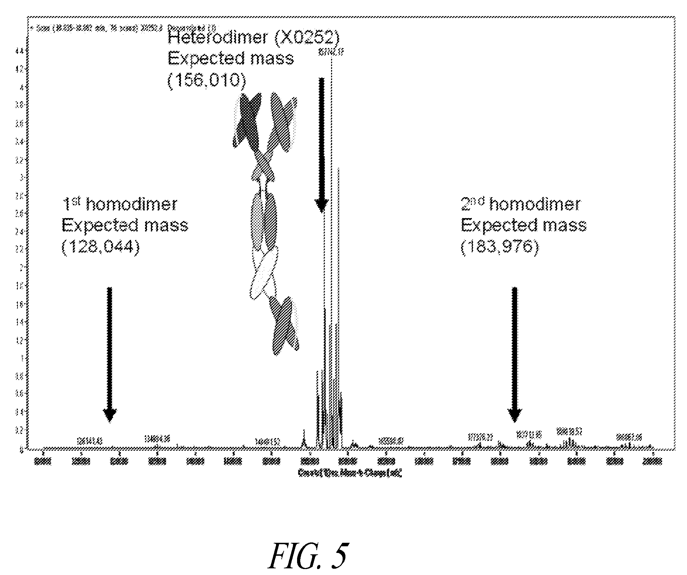

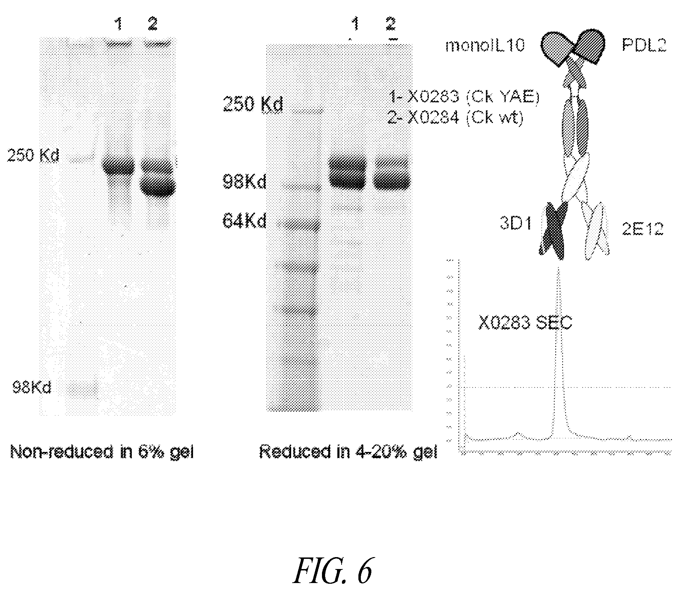

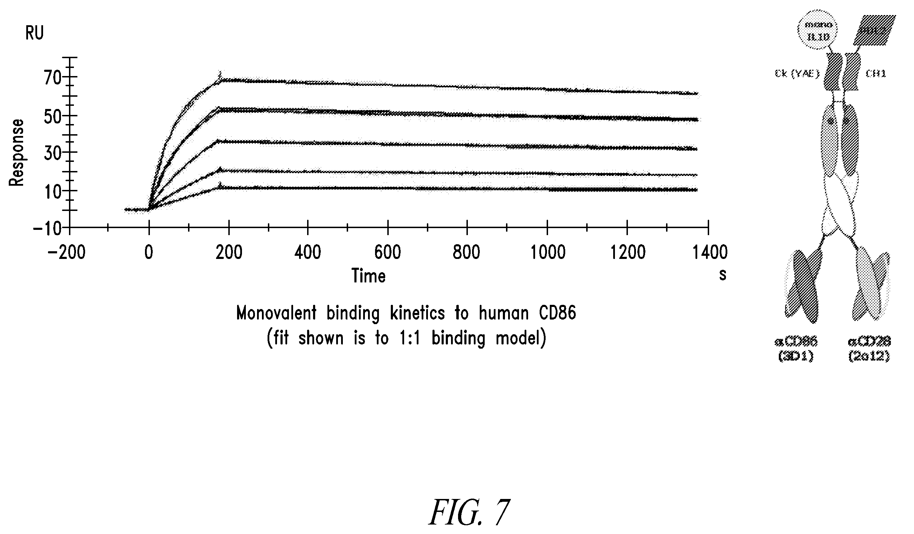

The SDS-PAGE electrophoresis and cation exchange chromatography analyses show that polypeptide heterodimers X0251, X0252 and X0253 were reasonably well expressed and purified (Figure 4). Mass spectrometry analysis of X0252 shows that the protein is mostly homogeneous with an undetectable amount of the homodimers (Figure 5). In addition, the SDS-PAGE electrophoresis and cation exchange chromatography analyses of polypeptide heterodimers X0283 and X0284 show that polypeptide heterodimer X0283 having the CH1/Ck(YAE) heterodimerization domains efficiently assembled into a heterodimer compared to control polypeptide heterodimer X0284 having the CH1/Ck heterodimerization domains, which formed homodimers as well (Figure 6). The Biocore analysis further shows that the 3D1 (anti-CD86) binding domain at the carboxyl terminus of single chain polypeptide X0281 monovalently bound to CD86 (Figure 7).

### EXAMPLE 3

### POLYPEPTIDE HETERODIMERS WITH ANTI-RON AND ANTI-C-MET BINDING DOMAINS

A bivalent polypeptide heterodimer with anti-RON binding domains (ORN151) and two bispecific polypeptide heterodimers comprising anti-RON and anti-cMet binding domains (ORN152 and ORN153) were made.

Bivalent polypeptide heterodimer ORN151 comprises single chain polypeptides ORN145 (4C04 CH2 CH3 CH1) and ORN148 (11H09 CH2 CH3 Ck(YAE)). Single chain polypeptide ORN145 comprises from its amino to carboxyl terminus: 4C04 (anti-RON) scFv, human IgG1 SCC-P hinge, human IgG1 CH2, human IgG1 CH3 and human IgG1 CH1. The nucleotide and amino acid sequences of ORN145 are set forth in SEQ ID NOS:26 and 30, respectively. Single chain polypeptide ORN148 comprises from its amino to carboxyl terminus: 11H09 (anti-RON) scFv, human IgG1 SCC-P hinge, human CH2, human CH3, and human Ck(YAE). The nucleotide and amino acid sequences of ORN148 are set forth in SEQ ID NOS:28 and 32, respectively.

Bispecific (c-Met, RON) polypeptide heterodimer ORN152 comprises single chain polypeptides ORN116 (MET021 CH2 CH3 CH1) and ORN146 (4C04 CH2 CH3 Ck(YAE)). Single chain polypeptide ORN116 comprises from its amino to carboxyl terminus: MET021 (anti-c-Met) scFv, human IgG1 SCC-P hinge, human IgG1 CH2, human IgG1 CH3 and human IgG1 CH1. The nucleotide and amino acid sequences of ORN116 are set forth in SEQ ID NOS:25 and 29, respectively. Single chain polypeptide ORN146 comprises from its amino to carboxyl terminus: 4C04 (anti-RON) scFv, human IgG1 SCC-P hinge, human CH2, human CH3, and human Ck(YAE). The nucleotide and amino acid sequences of ORN146 are set forth in SEQ ID NOS:27 and 31, respectively.

Bispecific (c-Met, RON) polypeptide heterodimer ORN153 comprises single chain polypeptides ORN116 (MET021 CH2 CH3 CH1) and ORN148 (11H09 CH2 CH3 Ck(YAE)).

Polypeptide heterodimers ORN151, ORN152 and ORN153 were expressed according to Example 1. The following expression levels were obtained: 1.9 µg protein / mL of culture for ORN151, 3.1 µg / mL for ORN152, and 4.9 µg / mL for ORN153.

### EXAMPLE 4

### POLYPEPTIDE HETERODIMERS WITH ANTI-CD3 BINDING DOMAINS

Several bispecific polypeptide heterodimers with an anti-CD3 binding domain were made: polypeptide heterodimers S0268, S0269, and TSC020 to TSC030. Polypeptide heterodimer S0268 comprises single chain polypeptides ORN145 (4C04 CH2 CH3 CH1) and TSC019 (G19-4 CH2 CH3 Ck(YAE)). Single chain polypeptide TSC019 comprises from its amino to carboxyl terminus: G19-4 (anti-CD3) scFv, human IgG1 SCC-P hinge, human CH2, human CH3, and human Ck(YAE). The nucleotide and amino acid sequences of TSC019 are set forth in SEQ ID NOS:52 and 72, respectively.

Bispecific (CD3, c-Met) Polypeptide heterodimer S0269 comprises single chain polypeptides ORN160 (5D5 CH2 CH3 CH1) and TSC019 (G19-4 CH2 CH3 Ck(YAE)). Single chain polypeptide ORN160 comprises from its amino to carboxyl terminus: 5D5 (anti-c-Met) scFv, human IgG1 SCC-P hinge, human IgG1 CH2, human IgG1 CH3, and human IgG1 CH1. The nucleotide and amino acid sequences of ORN160 are set forth in SEQ ID NOS:33 and 53, respectively.

Bivalent (CD19) polypeptide heterodimer TSC020 comprises single chain polypeptides TSC001 (HD37 CH2 CH3 CH1) and TSC019 (G19-4 CH2 CH3 Ck(YAE)). Single chain polypeptide TSC001 comprises from its amino to carboxyl terminus: HD37 (anti-CD19) scFv, human IgG1 SCC-P hinge, human IgG1 CH2, human IgG1 CH3 and human IgG1 CH1. The nucleotide and amino acid sequences of TSC001 are set forth in SEQ ID NOS:34 and 54, respectively.

Bispecific (CD20, CD3) polypeptide heterodimer TSC021 comprises single chain polypeptides TSC002 (2H7 CH2 CH3 CH1) and TSC019 (G19-4 CH2 CH3 Ck(YAE)). Single chain polypeptide TSC002 comprises from its amino to carboxyl terminus: 2H7 (anti-CD20) scFv, human IgG1 SCC-P hinge, human IgG1 CH2, human IgG1 CH3 and human IgG1 CH1. The nucleotide and amino acid sequences of TSC002 are set forth in SEQ ID NOS:35 and 55, respectively.

Bispecific (CD79b, CD3) polypeptide heterodimer TSC022 comprises single chain polypeptides TSC017 (P2C2 H2 CH3 CH1) and TSC019 (G19-4 CH2 CH3 Ck(YAE)). Single chain polypeptide TSC017 comprises from its amino to carboxyl terminus: P2C2 (anti-CD79b) scFv, human IgG1 SCC-P hinge, human IgG1 CH2, human IgG1 CH3 and human IgG1 CH1. The nucleotide and amino acid sequences of TSC017 are set forth in SEQ ID NOS:50 and 70, respectively.

Bispecific (HLA-DR, CD3) polypeptide heterodimer TSC023 comprises single chain polypeptides TSC018 (M0042 CH2 CH3 CH1) and TSC019 (G19-4 CH2 CH3 Ck(YAE)). Single chain polypeptide TSC018 comprises from its amino to carboxyl terminus: M0042 (anti-HLA-DR) scFv, human IgG1 SCC-P hinge, human IgG1 CH2, human IgG1 CH3, and human IgG1 CH1. The nucleotide and amino acid sequences of TSC018 are set forth in SEQ ID NOS:51 and 71, respectively.

Bivalent polypeptide heterodimer TSC024 comprises single chain polypeptides TSC010 (HD37-"IgA1 hinge"-CH2 CH3 CH1) and TSC003 (G19-4-"IgA1 hinge"-CH2 CH3 Ck(YAE)). Single chain polypeptide TSC010 comprises from its amino to carboxyl terminus: HD37 (anti-CD19) scFv, altered human IgA1 hinge (PSTPPTPSPSTPPTPSPSCPPCP, SEQ ID NO:752), human IgG1 CH2, human IgG1 CH3, and human IgG1 CH1. The nucleotide and amino acid sequences of TSC010 are set forth in SEQ ID NOS:43 and 63, respectively. Single chain polypeptide TSC003 comprises from its amino to carboxyl terminus: G19-4 (anti-CD3) scFv, altered human IgA1 hinge (SEQ ID NO:752), human IgG1 CH2, human IgG1 CH3, and human Ck(YAE). The nucleotide and amino acid sequences of TSC003 are set forth in SEQ ID NOS:36 and 56, respectively.

Bivalent polypeptide heterodimer TSC025 comprises single chain polypeptides TSC011 (HD37-"IgA2 hinge"-CH2 CH3 CH1) and TSC004 (G19-4-"IgA2 hinge"-CH2 CH3 Ck(YAE)). Single chain polypeptide TSC011 comprises from its amino to carboxyl terminus: HD37 (anti-CD19) scFv, altered human IgA2 hinge (PPPPPCPPCP, SEQ ID NO:748), human IgG1 CH2, human IgG1 CH3, and human IgG1 CH1. The nucleotide and amino acid sequences of TSC011 are set forth in SEQ ID NOS:44 and 64, respectively. Single chain polypeptide TSC004 comprises from its amino to carboxyl terminus: G19-4 (anti-CD3) scFv, altered human IgA2 hinge (SEQ ID NO:748), human IgG1 CH2, human IgG1 CH3, and human Ck(YAE). The nucleotide and amino acid sequences of TSC004 are set forth in SEQ ID NOS:37 and 57, respectively.

Bivalent polypeptide heterodimer TSC026 comprises single chain polypeptides TSC012 (HD37-"IgG1 hinge"-CH2 CH3 CH1) and TSC007 (G19-4-"IgG1 hinge"-CH2 CH3 Ck(YAE)). Single chain polypeptide TSC012 comprises from its amino to carboxyl terminus: HD37 (anti-CD19) scFv, altered human IgG1 hinge (EPKSSDKTHTSPPSPCPPCP, SEQ ID NO:750), human IgG1 CH2, human IgG1 CH3, and human IgG1 CH1. The nucleotide and amino acid sequences of TSC012 are set forth in SEQ ID NOS:45 and 65, respectively. Single chain polypeptide TSC007 comprises from its amino to carboxyl terminus: G19-4 (anti-CD3) scFv, altered human IgG1 hinge (SEQ ID NO:750), human IgG1 CH2, human IgG1 CH3, and human Ck(YAE). The nucleotide and amino acid sequences of TSC007 are set forth in SEQ ID NOS:40 and 60, respectively.

Bivalent polypeptide heterodimer TSC027 comprises single chain polypeptides TSC013 (HD37-"IgG3 hinge"-CH2 CH3 CH1) and TSC005 (G19-4-"IgG3 hinge"-CH2 CH3 Ck(YAE)). Single chain polypeptide TSC013 comprises from its amino to carboxyl terminus: HD37 (anti-CD19) scFv, altered human IgG3 hinge (EPKSSDTPPPSPRSPCPPCP, SEQ ID NO:751), human IgG1 CH2, human IgG1 CH3, and human IgG1 CH1. The nucleotide and amino acid sequences of TSC013 are set forth in SEQ ID NOS:46 and 66, respectively. Single chain polypeptide TSC005 comprises from its amino to carboxyl terminus: G19-4 (anti-CD3) scFv, altered human IgG3 hinge (SEQ ID NO:751), human IgG1 CH2, human IgG1 CH3, and human Ck(YAE). The nucleotide and amino acid sequences of TSC005 are set forth in SEQ ID NOS:38 and 58, respectively.

Bivalent polypeptide heterodimer TSC028 comprises single chain polypeptides TSC014 (HD37-"IgD hinge"-CH2 CH3 CH1) and TSC006 (G19-4-"IgD hinge"-CH2 CH3 Ck(YAE)). Single chain polypeptide TSC014 comprises from its amino to carboxyl terminus: HD37 (anti-CD19) scFv, altered human IgD hinge (ESPKAQASSVPTAQPQAEGSLAKATTAPATTRNTCPPCP, SEQ ID NO:754), human IgG1 CH2, human IgG1 CH3, and human IgG1 CH1. The nucleotide and amino acid sequences of TSC014 are set forth in SEQ ID NOS:47 and 67, respectively. Single chain polypeptide TSC006 comprises from its amino to carboxyl terminus: G19-4 (anti-CD3) scFv, altered human IgD hinge (SEQ ID NO:754), human IgG1 CH2, human IgG1 CH3, and human Ck(YAE). The nucleotide and amino acid sequences of TSC006 are set forth in SEQ ID NOS:39 and 59, respectively.

Bivalent polypeptide heterodimer TSC029 comprises single chain polypeptides TSC015 (HD37-"IgE CH2 hinge"-CH2 CH3 CH1) and TSC008 (G19-4-"IgE CH2 hinge"-CH2 CH3 Ck(YAE)). Single chain polypeptide TSC015 comprises from its amino to carboxyl terminus: HD37 (anti-CD19) scFv, altered human IgE CH2 hinge (SEQ ID NO:757), human IgG1 CH2, human IgG1 CH3, and human IgG1 CH1. The nucleotide and amino acid sequences of TSC014 are set forth in SEQ ID NOS:48 and 68, respectively. Single chain polypeptide TSC008 comprises from its amino to carboxyl terminus: G19-4 (anti-CD3) scFv, altered human IgE CH2 hinge (SEQ ID NO:757), human IgG1 CH2, human IgG1 CH3, and human Ck(YAE). The nucleotide and amino acid sequences of TSC008 are set forth in SEQ ID NOS:41 and 61, respectively.

Bivalent polypeptide heterodimer TSC030 comprises single chain polypeptides TSC016 (HD37-"IgM CH2 hinge"-CH2 CH3 CH1) and TSC009 (G19-4-"IgM CH2 hinge"-CH2 CH3 Ck(YAE)). Single chain polypeptide TSC016 comprises from its amino to carboxyl terminus: HD37 (anti-CD19) scFv, altered human IgM CH2 hinge (SEQ ID NO:759), human IgG1 CH2, human IgG1 CH3, and human IgG1 CH1. The nucleotide and amino acid sequences of TSC015 are set forth in SEQ ID NOS:49 and 69, respectively. Single chain polypeptide TSC009 comprises from its amino to carboxyl terminus: G19-4 (anti-CD3) scFv, altered human IgM CH2 hinge (SEQ ID NO:759), human IgG1 CH2, human IgG1 CH3, and human Ck(YAE). The nucleotide and amino acid sequences of TSC009 are set forth in SEQ ID NOS:42 and 62, respectively.

Bispecific polypeptide heterodimers S0268 (RON, CD3) and S0269 (CD3, c-Met) were expressed according to Example 1. The following expression levels were obtained: 2.2 µg protein / mL of culture for S0268 and 2.7 µg / mL for S0269.

### EXAMPLE 5

### CELL BINDING OF BISPECIFIC POLYPEPTIDE HETERODIMERS

To compare the effectiveness of bispecific polypeptide heterodimer molecules at targeting a tumor cell antigen and T-cells, the on-cell binding characteristics of an anti-RON (4C04 binding domain) x anti-CD3 (G19-4 binding domain) polypeptide heterodimer, S0268 (as described in Example 4), with a different bispecific scaffold (SCORPION™ protein) containing the same binding domains, S0266, were compared. Additionally, the on-cell binding characteristics of two bispecific polypeptide heterodimers, TSC020 and TSC021 (as described in Example 4), targeting different B-cell antigens (CD19 for TSC020 and CD20 for TSC021) as well as a T-cell antigen (CD3) were compared. The nucleotide and amino acid sequences of SCORPION protein S0266 are set forth in SEQ ID NOS:73 and 74, respectively. Nucleotide and amino acid sequences of the single chain polypeptides making up the bispecific polypeptide heterodimers S0268, TSC020, and TSC021 are set forth in SEQ ID NOS:26 and 52 (nucleotide), 30 and 72 (amino acid); 34 and 52 (nucleotide), 54 and 72 (amino acid); and 35 and 52 (nucleotide), 55 and 72 (amino acid), respectively (see also Example 4). Transient transfection in human 293 cells produced 6.9 µg protein / mL of culture for S0266; 2.3 µg / mL of culture for S0268; 3.0 µg / mL of culture for TSC020; and 3.2 µg / mL of culture for TSC021.

MDA-MB-453 (RON+) breast carcinoma cells, Rec1 (CD19⁺, CD20⁺) mantle cell lymphoma cells, and Jurkat (CD3⁺) T cell leukemia cells were obtained from ATCC (Manassas, VA), and cultured according to the provided protocol. T-cells were isolated from donor PBMCs using a Pan T-cell Isolation Kit II from Miltenyi Biotec (Bergisch Gladbach, Germany). Non T-cells were separated from PBMCs by being indirectly magnetically labeled with biotin-conjugated monoclonal antibodies and anti-biotin magnetic microbeads. These cells were then depleted by retaining them in a column surrounded by a magnetic field. The T-cells were not retained in the column and were collected in the flow through.

Binding was assessed by incubating 5×10⁵ T cells or target (MDA-MB-453, Rec1, Jurkat) cells for 30 minutes at 4°C with serially diluted bispecific molecules S0266 (αRON × αCD3 SCORPION™ protein) or S0268 (αRON × αCD3 polypeptide heterodimer) (for MDA-MB-453 cells and isolated T cells); or TSC020 (αCD19 x αCD3) or TSC021 (αCD20 x αCD3) for Rec1 and Jurkat cells, in concentrations from 100 nM to 0.1 nM. The cells were washed three times and then incubated with goat anti-human IgG-FITC (1:200 dilution) for another 30 minutes at 4°C. The cells were then washed again three times, fixed in 1% paraformaldehyde and read on a FACS-Calibur instrument.

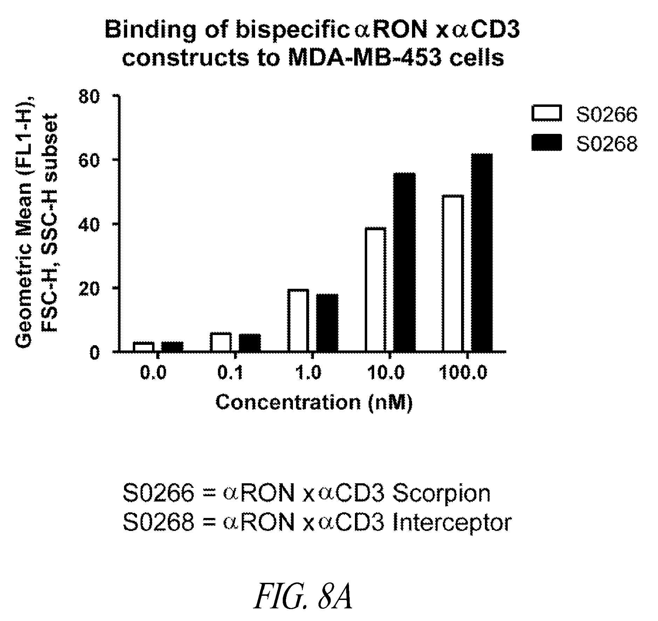

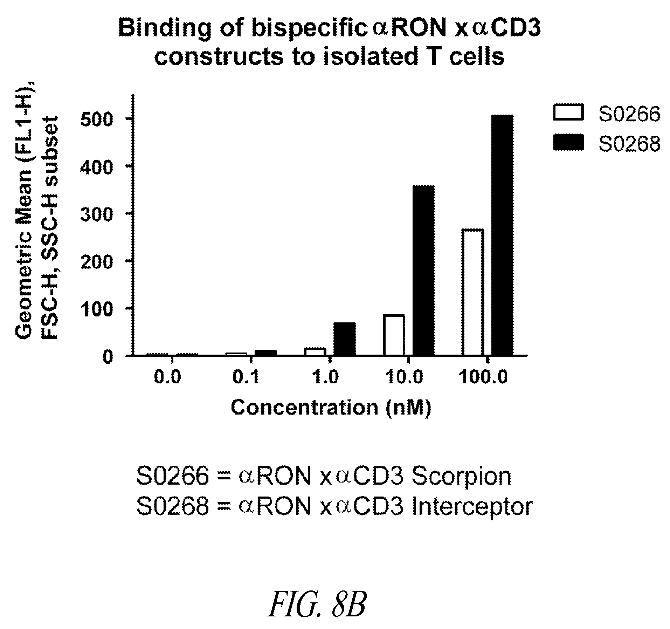

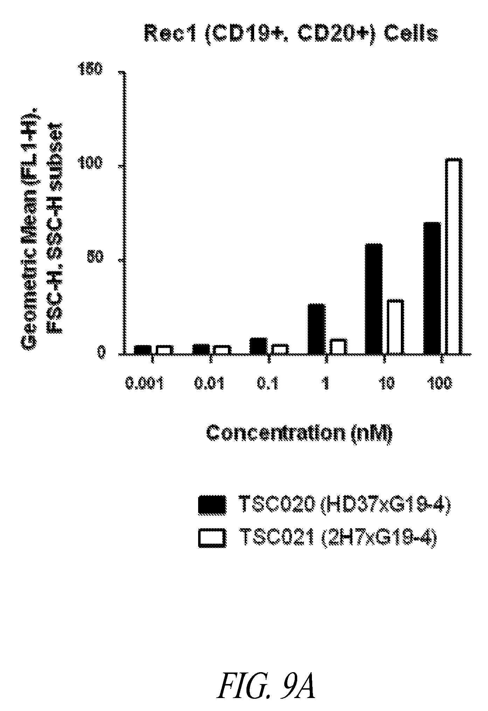

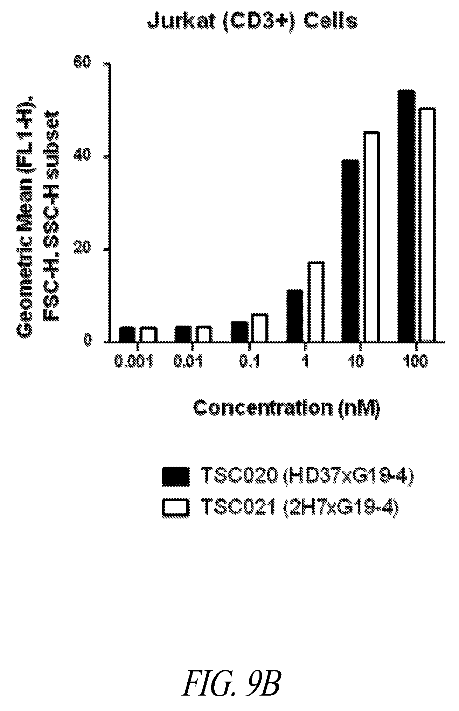

Analysis of the FSC high, SSC high subset in FlowJo v7.5 (Tree Star, Inc, Ashland, OR) showed dose-dependent binding of bispecific molecules S0266 and S0268 to both MDA-MB-453 and isolated T-cells (Figures 8A and 8B). Unexpectedly, the S0268 polypeptide heterodimer bound with similar affinity to the comparable SCORPION™ molecule (S0266) on both MDA-MB-453 cells and T-cells, although it lacked the potential for any avidity. Higher saturation on both target cell types was also observed with the polypeptide heterodimer, which would be the case if the polypeptide heterodimer was binding at a higher stoichometry (1:1 binding of polypeptide heterodimer to surface antigen) than the equivalent SCORPION™ (potential 1:2 binding of the bivalent Scorpion to surface antigens). Comparison of the CD 19-targeting bispecific heterodimer (TSC020; HD37 and G19-4 binding domains) and the CD20-targeting bispecific heterodimer (TSC021; 2H7 and G19-4 binding domains) on Rec1 and Jurkat cells revealed that the TSC020 heterodimer had higher affinity for the Rec1 cells than TSC021 heterodimer (Figure 9A). However, both TSC020 and TSC021 heterodimers showed similar binding to CD3⁺ Jurkat cells, which is expected since both heterodimers possess the same anti-CD3 binding domain (G19-4) (Figure 9B).

### EXAMPLE 6

### ADDITIONAL BIVALENT & TRIVALENT POLYPEPTIDE HETERODIMERS USING SINGLE HETERODIMERIZATION DOMAIN PAIR

Additional multispecific heterodimers were made: TSC046, TSC047, TSC048, TSC054, TSC055, TSC056, TSC057, TSC078, TSC079, TSC080, TSC099, TSC100, TSC101, and TSC102.

Bivalent polypeptide heterodimer TSC046 comprises single chain polypeptides TSC001 (HD37 CH2 CH3 CH1) and TSC039 (BMA031 CH2 CH3 Ck(YAE)). Single chain polypeptide TSC001 comprises from its amino to carboxyl terminus: HD37 (anti-CD19) scFv, human IgG1 SCC-P hinge, human IgG1 CH2, human IgG1 CH3, and human IgG1 CH1. The nucleotide and amino acid sequences of TSC001 are set forth in SEQ ID NOS:34 and 54, respectively. Single chain polypeptide TSC039 comprises from its amino to carboxyl terminus: BMA031 (anti-TCR) scFv, human IgG1 SCC-P hinge, human IgG1 CH2, human IgG1 CH3, and human Ck(YAE). The nucleotide and amino acid sequences of TSC039 are set forth in SEQ ID NOS:793 and 811, respectively.

Bivalent polypeptide heterodimer TSC047 comprises single chain polypeptides TSC001 (HD37 CH2 CH3 CH1) and TSC041 (CRIS7 CH2 CH3 Ck(YAE)). Single chain polypeptide TSC041 comprises from its amino to carboxyl terminus: CRIS7 (anti-CD3) scFv, human IgG1 SCC-P hinge, human IgG1 CH2, human IgG1 CH3, and human Ck(YAE). The nucleotide and amino acid sequences of TSC041 are set forth in SEQ ID NOS:794 and 812, respectively.

Bivalent polypeptide heterodimer TSC048 comprises single chain polypeptides TSC001 (HD37 CH2 CH3 CH1) and TSC043 (OKT3-M CH2 CH3 Ck(YAE)). Single chain polypeptide TSC043 comprises from its amino to carboxyl terminus: OKT3-M (Micromet variant anti-CD3, see also US 7,635,472) scFv, human IgG1 SCC-P hinge, human IgG1 CH2, human IgG1 CH3, and human Ck(YAE). The nucleotide and amino acid sequences of TSC043 are set forth in SEQ ID NOs:795 and 813, respectively.

Bivalent polypeptide heterodimer TSC054 comprises single chian polypeptides TSC049 (HD37 CH2(ADCC/CDC null) CH3 CH1) and TSC053 (G19-4 CH2(ADCC/CDC null) CH3 Ck(YAE)). Single chain polypeptide TSC049 comprises from its amino to carboxyl terminus: HD37 (anti-CD19) scFv, human IgG1 SCC-P hinge, human IgG1 CH2(ADCC/CDC null) (*i.e.*, human IgG1 CH2 with L234A, L235A, G237A, E318A, K320A, and K322A substitutions), human IgG1 CH3, and human IgG1 CH1. The nucleotide and amino acid sequences of TSC049 are set forth in SEQ ID NOS:796 and 814, respectively. Single chain polypeptide TSC053 comprises from its amino to carboxyl terminus: G19-4 (anti-CD3) scFv, human IgG1 SCC-P hinge, human IgG1 CH2(ADCC/CDC null) (*i.e.*, human IgG1 CH2 with L234A, L235A, G237A, E318A, K320A, and K322A substitutions), human IgG1 CH3, and human Ck(YAE). The nucleotide and amino acid sequences of TSC053 are set forth in SEQ ID NOS:800 and 818, respectively.

Bivalent polypeptide heterodimer TSC055 comprises single chain polypeptides TSC050 (2H7 CH2(ADCC/CDC null) CH3 CH1) and TSC053 (G19-4 CH2(ADCC/CDC null) CH3 Ck(YAE)). Single chain polypeptide TSC050 comprises from its amino to carboxyl terminus: 2H7 (anti-CD20) scFv, human IgG1 SCC-P hinge, human IgG1 CH2(ADCC/CDC null), human IgG1 CH3, and human IgG1 CH1. The nucleotide and amino acid sequences of TSCO50 are set forth in SEQ ID NOS:797 and 815, respectively.

Bivalent polypeptide heterodimer TSC056 comprises single chain polypeptides TSC051 (P2C2 CH2(ADCC/CDC null) CH3 CH1) and TSC053 (G19-4 CH2(ADCC/CDC null) CH3 Ck(YAE)). Single chain polypeptide TSC051 comprises from its amino to carboxyl terminus: P2C2 (anti-CD79) scFv, human IgG1 SCC-P hinge, human IgG1 CH2(ADCC/CDC null), human IgG1 CH3, and human IgG1 CH1. The nucleotide and amino acid sequences of TSC051 are set forth in SEQ ID NOS:798 and 816, respectively.

Bivalent polypeptide heterodimer TSC057 comprises single chain polypeptides TSC052 (5D5 CH2(ADCC/CDC null) CH3 CH1) and TSC053 (G19-4 CH2(ADCC/CDC null) CH3 Ck(YAE)). Single chain polypeptide TSC052 comprises from its amino to carboxyl terminus: 5D5 (anti-cMet) scFv, human IgG1 SCC-P hinge, human IgG1 CH2(ADCC/CDC null), human IgG1 CH3, and human IgG1 CH1. The nucleotide and amino acid sequences of TSC052 are set forth in SEQ ID NOS:799 and 817, respectively.

Bivalent polypeptide heterodimer TSC078 comprises single chain polypeptides TSC049 (HD37 CH2(ADCC/CDC null) CH3 CH1) and TSC076 (OKT3 CH2(ADCC/CDC null) CH3 Ck(YAE)). Single chain polypeptide TSC076 comprises from its amino to carboxyl terminus: OKT3 (anti-CD3) scFv, human IgG1 SCC-P hinge, human IgG1 CH2(ADCC/CDC null), human IgG1 CH3, and human Ck(YAE). The nucleotide and amino acid sequences of TSC076 are set forth in SEQ ID NOS:802 and 820, respectively.

Bivalent polypeptide heterodimer TSC079 comprises single chain polypeptides TSC049 (HD37 CH2(ADCC/CDC null) CH3 CH1) and TSC077 (Nuvion CH2(ADCC/CDC null) CH3 Ck(YAE)). Single chain polypeptide TSC077 comprises from its amino to carboxyl terminus: Nuvion (anti-CD3) scFv, human IgG1 SCC-P hinge, human IgG1 CH2(ADCC/CDC null), human IgG1 CH3, and human Ck(YAE). The nucleotide and amino acid sequences of TSC077 are set forth in SEQ ID NOS:803 and 821, respectively.

Trivalent polypeptide heterodimer TSC080 comprises single chain polypeptides TSC001 (HD37 CH2 CH3 CH1) and TSC064 (G19-4 CH2 CH3 Ck(YAE) H75 Met021). Single chain polypeptide TSC064 comprises from its amino to carboxyl terminus: G19-4 (anti-CD3) scFv, human IgG1 SCC-P hinge, human IgG1 CH2, human IgG1 CH3, human Ck(YAE), H75 linker, and Met021 (anti-c-Met) scFv (with the three serine residues at the C-temrinus deleted). The nucleotide and amino acid sequences of TSC064 are set forth in SEQ ID NOS:801 and 819, respectively.

Bivalent polypeptide heterodimer TSC099 comprises single chain polypeptides TSC049 (HD37 CH2(ADCC/CDC null) CH3 CH1) and TSC097 (4C04 CH2(ADCC/CDC null) CH3 Ck(YAE)). Single chain polypeptide TSC097 comprises from its amino to carboxyl terminus: 4C04 (anti-RON) scFv, human IgG1 SCC-P hinge, human IgG1 CH2(ADCC/CDC null), human IgG1 CH3, and human Ck(YAE). The nucleotide and amino acid sequences of TSC097 are set forth in SEQ ID NOS:808 and 826, respectively.

Bivalent polypeptide heterodimer TSC100 comprises single chain polypeptides TSC049 (HD37 CH2(ADCC/CDC null) CH3 CH1) and TSC093 (CRIS7 CH2(ADCC/CDC null) CH3 Ck(YAE)). Single chain polypeptide TSC093 comprises from its amino to carboxyl terminus: CRIS7 (anti-CD3) scFv, human IgG1 SCC-P hinge, human IgG1 CH2(ADCC/CDC null), human IgG1 CH3, and human Ck(YAE). The nucleotide and amino acid sequences of TSC093 are set forth in SEQ ID NOS:804 and 822, respectively.

Bivalent polypeptide heterodimer TSC101 comprises single chain polypeptides TSC049 (HD37 CH2(ADCC/CDC null) CH3 CH1) and TSC094 (OKT3-M CH2(ADCC/CDC null) CH3 Ck(YAE)). Single chain polypeptide TSC094 comprises from its amino to carboxyl terminus: OKT3-M (Micromet variant anti-CD3) scFv, human IgG1 SCC-P hinge, human IgG1 CH2(ADCC/CDC null), human IgG1 CH3, human Ck(YAE). The nucleotide and amino acid sequences of TSC094 are set forth in SEQ ID NOS:805 and 823, respectively.

Bivalent polypeptide heterodimer TSC102 comprises single chain polypeptides TSC049 (HD37 CH2(ADCC/CDC null) CH3 CH1) and TSC095 (BMA031 CH2(ADCC/CDC null) CH3 Ck(YAE)). Single chain polypeptide TSC095 comprises from its amino to carboxyl terminus: BMA031 (anti-TCR) scFv, human IgG1 SCC-P hinge, human IgG1 CH2(ADCC/CDC null), human IgG1 CH3, human Ck(YAE). The nucleotide and amino acid sequences of TSC095 are set forth in SEQ ID NOS:806 and 824, respectively.

The polypeptide heterodimer molecules were expressed and purified according to Example 1.

### EXAMPLE 7

### TARGET-DEPENDENT T-CELL PROLIFERATION BY POLYPEPTIDE HETERODIMERS

To compare the effectiveness of different bispecific polypeptide heterodimer molecules at inducing target-dependent T-cell activation and proliferation, three different bispecific molecules (TSC054, TSC078, and TSC079 as described in Example 6) with a common anti-CD 19 binding domain (HD37) and three different anti-CD3 binding domains (G19-4 for TSC054, OKT3 for TSC078, HuM291 for TSC079) were compared. As a positive control, a Bispecific T-Cell Engaging molecule (BiTE) known as bsc19x3 was also prepared (see U.S. Patent 7,635,472). The nucleotide and amino acid sequences for bsc19x3 are set forth in SEQ ID NOS:809 and 827, respectively. Transient transfection in human 293 cells produced 2.33 ug/mL protein for TSC054, 0.67 ug/mL protein for TSC078, and 3.5 ug/mL for TSC079.

Daudi Burkitt's lymphoma cells (CD19⁺) and MDA-MB-453 (CD19⁻) breast carcinoma cells were obtained from ATCC (Manassas, VA) and cultured according to the provided protocol. Peripheral blood mononuclear cells (PBMC) were isolated from human blood using standard ficoll gradients. The isolated cells were washed in saline buffer. T cells were additionally isolated using a Pan T-cell Isolation Kit II from Miltenyi Biotec (Bergisch Gladbach, Germany) using the manufacturer's protocol (see also Example 5 for more information).

Proliferation was assessed by labeling isolated PBMC or T cell populations with carboxyfluorescein diacetate succinimidyl ester (CFSE). CFSE-labeled PBMC or T cells were plated in U-bottom 96-well plates at 150,000 or 100,000 cells/well, respectively, with various numbers of tumor cells, to achieve T cell to tumor cell ratios of 10:1 to 3:1. Concentrations of test molecules ranging from 8 nM to 0.08 pM were added to the cell mixtures in a total of 200ul/well in RPMI 1640 media supplemented with 10% human or bovine serum, sodium pyruvate and non-essential amino acids. Plates were incubated at 37°C, 5% CO₂ in humidified incubators. After 3 days, cells were labeled with antibodies for flow cytometric analysis. Cells were labeled and washed in their original plates to minimize cell losses during transfers, and all labeling was done in saline buffer with 0.2% bovine serum albumin. First, cells were pre-incubated with 100ug/ml human IgG at room temperature for 15 min. Subsequently, cells were incubated with a mixture (total volume 50 ul) of the following dye-labeled antibodies: CD5-PE, CD4-APC, CD8-Pacific Blue, CD25-PE-Cy7, as well as 7-Amino Actinomycin D (7AAD hereafter) for 40 min. Plates were washed twice, resuspended in 80 to 120u1 volumes and ran immediately in a BD LSRII flow cytometer to acquire 80% of the contents of each well. The sample files were analyzed using FlowJo software to calculate the percentages and numbers of cells that had undergone at least one cell division, according to their CFSE profile, by gating sequentially on activated, live CD4+ or CD8+ T cells (7AAD-, CD5+ CD25+ CD4+ or 7AAD- CD5+ CD25+ CD8+, respectively). Mean values and standard deviations were calculated using Microsoft Excel software. Graphs were plotted using Microsoft Excel or Graphpad Prism.

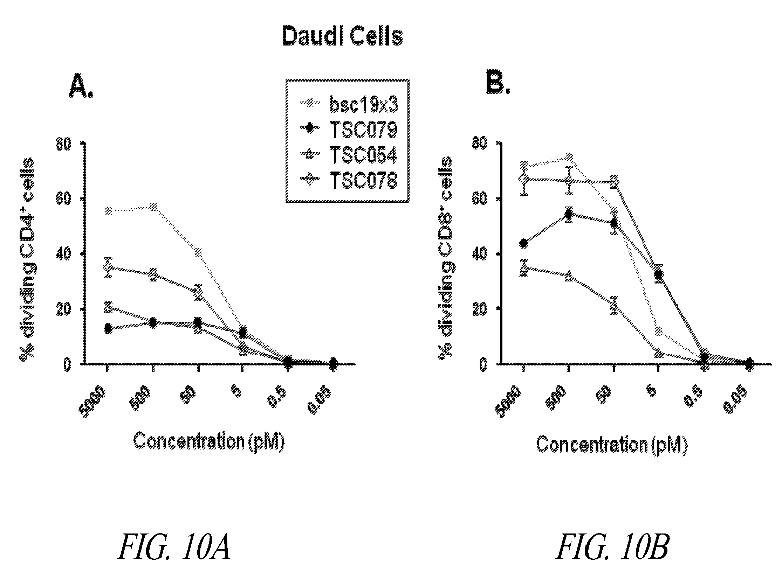

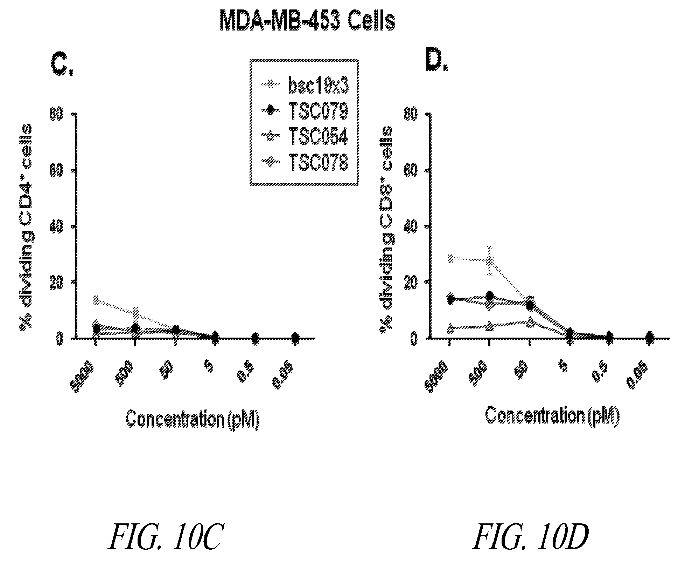

Analysis of live CD4+ and CD8+ populations from Daudi cells or MDA-MB-453 cells treated with whole PBMCs (Figures 10A-10D) revealed a significant increase in both the total number of cells and percent proliferating cells in the presence of Daudi cells displaying the target CD 19 antigen (Figures 10A, 10B), but a lack of significant proliferation in the presence of MDA-MB-453 cells that lacked the CD19 antigen (Figures 10C, 10D). Some proliferation was observed at a lower level with the MDA-MB-453 cells and whole PBMCs, since B-cells (CD19+) were present in the PBMCs, but overall proliferation was greatly reduced in comparison. This selectivity was observed with isolated T-cells as well. Proliferation was higher for CD8+ T-cells than CD4+ T-cells in the presence of Daudi cells or MDA-MB-453 cells treated with PBMCs (Figure 10A -10D), and the proliferation induced by TSC078 (HD37xOKT3) was generally higher at all concentrations than the response induced by TSC054 (HD37xG19-4) or TSC079 (HD37xHuM291)(Figures 10A-10D). Induced proliferation of CD4+ T-cells was lower in all cases for TSC054, TSC078, and TSC079 than the BiTE bsc19x3 (Figure 10A), although TSC078 and TSC079 showed superior induction of proliferation of CD8+ cells at lower concentrations (e.g. 5 pM) than the BiTE molecule (Figure 10B).

### EXAMPLE 8

### REDIRECTED T-CELL CYTOTOXICITY BY POLYPEPTIDE HETERODIMERS

To compare the effectiveness of different bispecific polypeptide heterodimer molecules at inducing target-dependent T-cell cytotoxicity, four different bispecific molecules were compared in a chromium (⁵¹Cr) release assay. Three different bispecific molecules (TSC054, TSC078, TSC079, as described in Example 6) with a common anti-CD19 binding domain (HD37) and three different anti-CD3 binding domains (G19-4 for TSC054, OKT3 for TSC078, HuM291 for TSC079) were tested alongside a fourth bispecific molecule (S0268, described in Example 4) with an anti-RON binding domain (4C04) and an anti-CD3 binding domain (G19-4). Transient transfection in human 293 cells produced about 2.33 µg/mL protein for TSC054, about 0.67 µg/mL protein for TSC078, and about 3.5 µg/mL protein for TSC079.

Daudi Burkitt's lymphoma cells (CD19+, RON-) and BxPC-3 cells (CD 19-, RON+) were obtained from ATCC (Manassas, VA) and cultured according to the provided protocol. Peripheral blood mononuclear cells (PBMC) were isolated from human blood using standard ficoll gradients. The isolated cells were washed in saline buffer. T cells were additionally isolated using a Pan T-cell Isolation Kit II from Miltenyi Biotec (Bergisch Gladbach, Germany) using the manufacturer's protocol (see also Example 5 for more information).

Cytotoxicity was assessed by a ⁵¹Cr release assay. Approximately 5×10⁶ Daudi or BxPC-3 cells were treated with 0.3 mCi of ⁵¹Cr and incubated for 75 minutes at 37°C. After 75 minutes, cells were washed 3 times with media (RPMI + 10% FBS) and resuspended in 11.5 mL of media. From this suspension, 50 µL was dispensed per well into 96 well U-bottom plates (approximately 20,000 cells/well). Concentrations of bispecific molecules ranging from 10 nM to 0.1 pM were added to the target (Daudi, BxPC-3) cells, bringing the total volume to 100 µL/well. Target cells were incubated at room temperature for 15 minutes. Then 100 µL of isolated T-cells (approximately 200,000) were added to bring the T-cell to target cell ratio to 10:1. 50 µL of 0.8% NP-40 was added to a control well containing target cells, left for 15 minutes, then 100 µL of media was added to provide a total lysis control.

Plates were incubated for 4 hours, spun at 1500 rpm for 3 minutes, and 25 µL of supernatant was transferred from each well to the corresponding well of a 96-well Luma sample plate. Sample plates were allowed to air dry in a chemical safety hood for 18 hours, and then radioactivity was read on a Topcount scintillation counter using a standard protocol.

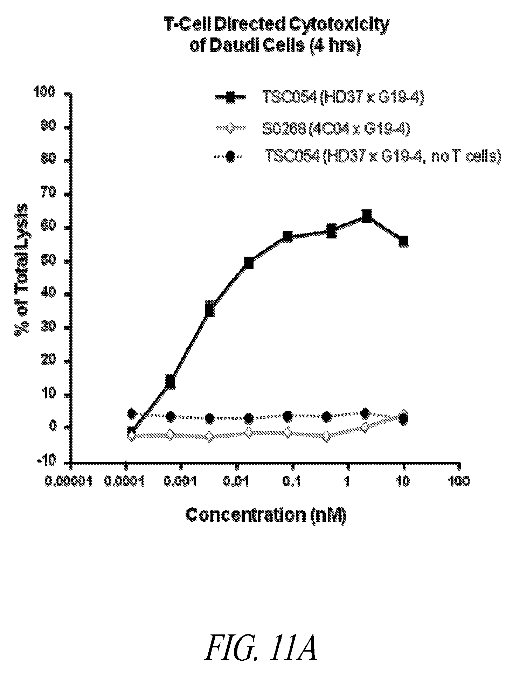

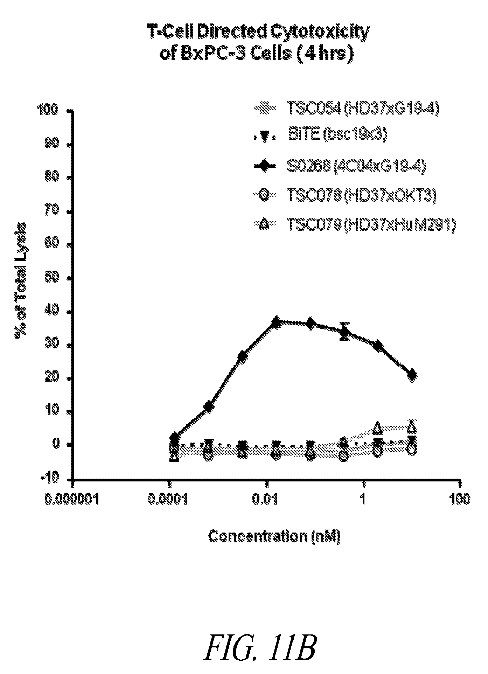

Analysis of cytotoxicity data showed a lack of off-target cytotoxicity on the Daudi (RON-) cells from the anti-RON directed bispecific molecule S0268 (Figure 11A). Similarly, there was a lack of direct cytotoxicity observed from treating Daudi cells with TSC054 in the absence of T-cells (Figure 11A). However, strong T-cell directed cytotoxicity was observed with the Daudi cells in the presence of T-cells and an anti-CD19 directed bispecific molecule (TSC054), reaching maximal lysis at a concentration between 10 and 100 pM (Figure 11A). Similarly, using a second T-cell donor (Figure 11B), no off-target cytotoxicity of the BxPC-3 (CD19-) cells was observed from the CD19-directed bispecifics TSC054, TSC078, or TSC079, or the CD19-directed BiTE bsc19x3. The anti-RON directed S0268 bispecific molecule induced cytotoxicity in BxPC-3 (RON+) cells, reaching a maximum between 10 and 100 pM (Figure 11B).

### EXAMPLE 9

### MODULATION OF TARGET-DEPENDENT T-CELL PROLIFERATION BY POLYPEPTIDE HETERODIMERS WITH ALTERED HINGE SEQUENCES

To compare the effectiveness of different bispecific polypeptide heterodimer molecules with altered hinge sequences at inducing target-dependent T-cell activation and proliferation, six different bispecific heterodimers (TSC100, TSC127, TSC165, TSC166, TSC167 and TSC168 with a common anti-CD19 binding domain (HD37), a common anti-CD3 binding domain (Cris7) and five different hinge constructs (IgG1 SCC-P hinge for TSC100 and TSC127, IgA2 hinge without the first Val residue linked to a human IgG1 core hinge for TSC165, IgG1 SSS-P hinge linked to a human IgG1 core hinge for TSC166, a portion of mutated IgG3 hinge linked to a IgG1 core hinge for TSC167, and the IgM CH2 domain without the first Val linked to a IgG1 core hinge for TSC168) were compared.

More specifically, bispecific heterodimer TSC100 is as described in Example 6.

Bispecific heterodimer TSC127 comprises single chain polypeptides TSC125 (Cris7 CH2(ADCC/CDC null) CH3 CH1) and TSC096 (HD37 CH2(ADCC/CDC null) CH3 Ck(YAE). Single chain polypeptide TSC125 comprises from its amino to carboxyl terminus: humanized Cris7 (anti-CD3) scFv, human IgG1 SCC-P hinge, human IgG1 CH2 (ADCC/CDC null), human IgG1 CH3, and human IgG1 CH1. The nucleotide and amino acid sequences of TSC125 are set forth in SEQ ID NOS:865 and 874, respectively. Single chain polypeptide TSC096 comprises from its amino to carboxyl terminus: HD37 (anti-CD19) scFv, human IgG1 SCC-P hinge, human IgG1 CH2 (ADCC/CDC null), human IgG1 CH3, and human Ck (YAE). The nucleotide and amino acid sequences of TSC096 are set forth in SEQ ID NOS:807 and 825, respectively.

Bispecific heterodimer TSC 165 comprises single chain polypeptides TSC157 (Cris7 IgA2UH CH2 (ADCC/CDC null) CH3 CH1) and TSC161 (HD37 IgA2UH CH2 (ADCC/CDC null) CH3 Ck(YAE)). Single chain polypeptide TSC157 comprises from its amino to carboxyl terminus: humanized Cris7(anti-CD3) scFv, human IgA2 hinge without the first Val linked with a human IgG1core hinge, human IgG1 CH2 (ADCC/CDC null), human IgG1 CH3, and human IgG1 CH1. The nucleotide and amino acid sequences of TSC157 are set forth in SEQ ID NOS:866 and 875, respectively. Single chain polypeptide TSC161 comprises from its amino to carboxyl terminus: HD37 (anti-CD19) scFv, human IgA2 hinge without the first Val linked with a human IgG1 core hinge, human IgG1 CH2 (ADCC/CDC null), human IgG1 CH3, and human Ck (YAE). The nucleotide and amino acid sequences of TSC161 are set forth in SEQ ID NOS:870 and 879, respectively. The amino acid sequence of the hinge used in TSC157 and TSC161 is set forth in SEQ ID NO:748.

Bispecific heterodimer TSC166 comprises single chain polypeptide TSC158 (Cris7 IgG1miniUH CH2 (ADCC/CDC null) CH3 CH1) and TSC162 (HD37 IgG1miniUH CH2 (ADCC/CDC null) CH3 Ck(YAE)). Single chain polypeptide TSC158 comprises from its amino to carboxyl terminus: humanized Cris7 (anti-CD3) scFv, human IgG1 SSC-P hinge linked with a human IgG1 core hinge, human IgG1 CH2 (ADCC/CDC null), human IgG1 CH3, and human IgG1 CH1. The nucleotide and amino acid sequences of TSC158 are set forth in SEQ ID NOS:867 and 876, respectively. Single chain polypeptide TSC162 comprises from its amino to carboxyl terminus: HD37 (anti-CD19) scFv, human IgG1 SSC-P hinge linked with a human IgG1 core hinge, human IgG1 CH2 (ADCC/CDC null), human IgG1 CH3, and human Ck (YAE). The nucleotide and amino acid sequences of TSC162 are set forth in SEQ ID NOS:871 and 880. The amino acid sequence of the hinge used in TSC158 and TSC162 is set forth in SEQ ID NO:750.

Bispecific heterodimer TSC167 comprises single chain polypeptide TSC159 (Cris7 IgG3UH CH2 (ADCC/CDC null) CH3 CH1) and TSC163 (HD37 IgG3UH CH2 (ADCC/CDC null) CH3 Ck(YAE)). Single chain polypeptide TSC159 comprises from its amino to carboxyl terminus: humanized Cris7 (anti-CD3) scFv, a portion of mutated human IgG3 hinge linked with a human IgG1 core hinge, human IgG1 CH2 (ADCC/CDC null), human IgG1 CH3, and human IgG1 CH1. The nucleotide and amino acid sequences of TSC159 are set forth in SEQ ID NOS:868 and 877, respectively. Single chain polypeptide TSC163 comprises from its amino to carboxyl terminus: HD37 (anti-CD19) scFv, a portion of mutated human IgG3 hinge linked with a human IgG1 core hinge, human IgG1 CH2 (ADCC/CDC null), human IgG1 CH3, and human Ck (YAE). The nucleotide and amino acid sequences of TSC163 are set forth in SEQ ID NOS:872 and 881. The amino acid sequence of the hinge used in TSC159 and TSC163 is set forth in SEQ ID NO:751.

Bispecific heterodimer TSC168 comprises single chain polypeptide TSC160 (Cris7 IgMCH2UH CH2 (ADCC/CDC null) CH3 CH1) and TSC164 (HD37 IgMCH2UH CH2 (ADCC/CDC null) CH3 Ck(YAE)). Single chain polypeptide TSC160 comprises from its amino to carboxyl terminus: humanized Cris7(anti-CD3) scFv, human IgM CH2 without the first Val linked with a human IgG1 core hinge, human IgG1 CH2 (ADCC/CDC null), human IgG1 CH3, and human IgG1 CH1. The nucleotide and amino acid sequences of TSC160 are set forth in SEQ ID NOS:869 and 878, respectively. Single chain polypeptide TSC163 comprises from its amino to carboxyl terminus: HD37 (anti-CD19) scFv, human IgM CH2 without the first Val linked with a human IgG1core hinge, human IgG1 CH2 (ADCC/CDC null), human IgG1 CH3, and human Ck (YAE). The nucleotide and amino acid sequences of TSC164 are set forth in SEQ ID NOS:873 and 882. The amino acid sequence of the hinge used in TSC160 and TSC164 is set forth in SEQ ID NO:759.

As a positive control, a Bispecific T-Cell Engaging molecule (BiTE) known as bsc19x3 was also prepared (see U.S. Patent 7,635,472). The nucleotide and amino acid sequences for bsc19x3 are set forth in SEQ ID NOS:809 and 827, respectively.

Transient transfection in human 293 cells produced 3.2 ug protein / mL of culture for TSC100, 6.1 ug protein / mL of culture for TSC127, 4.8 ug protein / mL of culture for TSC165, 6.2 ug protein / mL of culture for TSC166, 6.4 ug protein / mL of culture for TSC167, and 6.4 ug/mL protein for TSC168.

Daudi Burkitt's lymphoma cells (CD19⁺) and C4-2 (CD19⁻) prostate carcinoma cells were obtained from ATCC (Manassas, VA) and MD Anderson Cancer Center (Houston, TX) and cultured according to the provided protocol. T cells were isolated using a Pan T-cell Isolation Kit II from Miltenyi Biotec (Bergisch Gladbach, Germany) using the manufacturer's protocol (see also Example 5 for more information).

Proliferation was assessed by labeling isolated T cell populations with carboxyfluorescein diacetate succinimidyl ester (CFSE). CFSE-labeled T cells were plated in U-bottom 96-well plates at 100,000 cells/well, with 10,000 tumor cells/well, to achieve a T cell to tumor cell ratio of 10:1. Concentrations of test molecules ranging from 5 nM to 0.005 pM were added to the cell mixtures in a total of 200 ul/well in RPMI 1640 media supplemented with 10% human or bovine serum, sodium pyruvate and non-essential amino acids. Plates were incubated at 37°C, 5% CO₂ in humidified incubators. After 3 days, cells were labeled with antibodies for flow cytometric analysis. Cells were labeled and washed in their original plates to minimize cell losses during transfers, and all labeling was done in saline buffer with 0.2% bovine serum albumin. First, cells were pre-incubated with 100 ug/ml human IgG at room temperature for 15 min. Subsequently, cells were incubated with a mixture (total volume 50 ul) of the following dye-labeled antibodies: CD5-PE, CD4-APC, CD8-Pacific Blue, CD25-PE-Cy7, as well as 7-Amino Actinomycin D (7AAD hereafter) for 40 minutes. Plates were washed twice, resuspended in 80 to 120 ul volumes and ran immediately in a BD LSRII flow cytometer to acquire 80% of the contents of each well. The sample files were analyzed using FlowJo software to calculate the percentages and numbers of cells that had undergone at least one cell division, according to their CFSE profile, by gating sequentially on activated, live CD4+ or CD8+ T cells (7AAD-, CD5+ CD25+ CD4+ or 7AAD- CD5+ CD25+ CD8+, respectively). Mean values and standard deviations were calculated using Microsoft Excel software. Graphs were plotted using Microsoft Excel or Graphpad Prism.

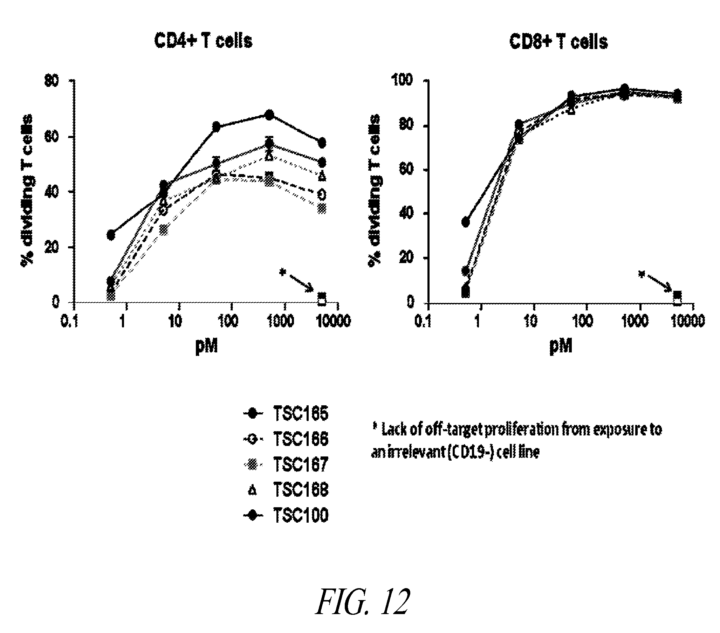

Analysis of live CD4+ and CD8+ populations from Daudi cells or C4-2 cells treated with isolated T-cells revealed a significant increase in both the total number of cells and percent proliferating cells in the presence of Daudi cells displaying the target CD 19 antigen, but a lack of significant proliferation in the presence of C4-2 cells that lacked the CD19 antigen (Figure 12). Proliferation of CD8+ cells was very similar for bispecific molecules with the default IgG1 hinge (TSC100) as well as those with longer hinges (TSC166, TSC167, TSC168). Slightly higher CD8+ proliferation at low concentrations was observed with the bispecific with the shorter IgA2 upper hinge (TSC165). Similar, but more marked differences were observed with proliferation of CD4+ cells, wherein the molecule containing the shorter IgA2 hinge (TSC165) showed higher proliferation at most concentrations than the standard IgG1 hinge (TSC100), and molecules with longer hinges (TSC166, TSC167, TSC168) showed lower proliferation.

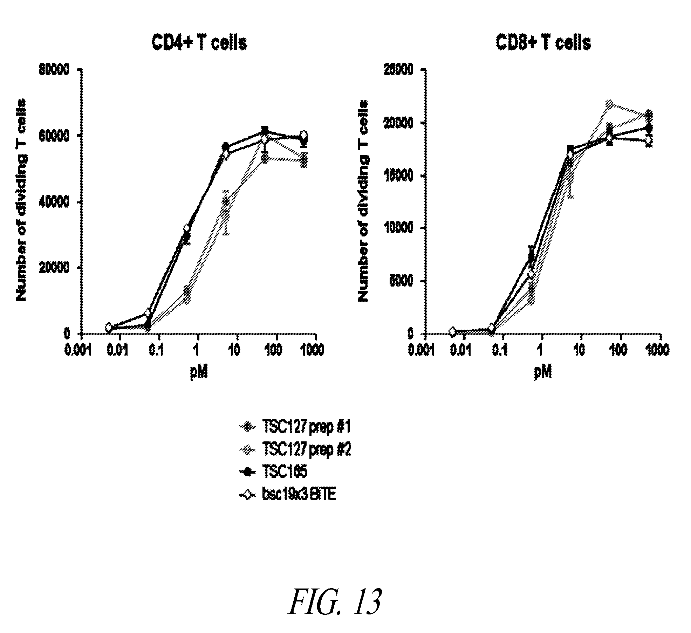

To confirm the differential activity of the IgA2 hinge, a second proliferation experiment was carried out with a titration to lower protein concentrations (Figure 13), comparing the bispecific featuring the IgA2 hinge (TSC165) to two different production lots of a bispecific featuring the default IgG1 hinge (TSC127) and the bsc19x3 BiTE molecule. Similar to the previous experiment, less variation was observed with proliferation of CD8+ cells, with TSC165 showing comparable or greater induction of proliferation to bsc19x3, which in turn showed slightly higher or comparable proliferation to TSC127. Again, similar to the previous experiment, these trends repeated in a magnified fashion with the CD4+ cell proliferation, with TSC165 and bsc19x3 showing comparable proliferation, which in turn was noticeably greater than that of TSC127.

### EXAMPLE 10

### MODULATION OF REDIRECTED T-CELL CYTOTOXICITY BY POLYPEPTIDE HETERODIMERS WITH ALTERED HINGE SEQUENCES

To compare the effectiveness of changing hinge composition in bispecific polypeptide heterodimer molecules on inducing target-dependent T-cell cytotoxicity, five different bispecific molecules were compared in a chromium (⁵¹Cr) release assay. Five different bispecific molecules (TSC100, TSC165, TSC166, TSC167 and TSC168, as described in Example 9) with a common anti-CD 19 binding domain (HD37), a common anti-CD3 binding domain (Cris7) and five different hinge constructs (IgG1 SCC-P hinge for TSC100 and TSC127, IgA2 hinge without the first Val linked to a human IgG1 core hinge for TSC165, IgG1 SSS-P hinge linked to a human IgG1 core hinge for TSC166, a portion of mutated IgG3 hinge linked to a human IgG1 core hinge for TSC167, and the IgM CH2 domain without the first Val linked to a human IgG1 core hinge for TSC168) were compared.

Daudi Burkitt's lymphoma cells (CD19+, RON-) were obtained from ATCC (Manassas, VA) and cultured according to the provided protocol. T cells were isolated using a Pan T-cell Isolation Kit II from Miltenyi Biotec (Bergisch Gladbach, Germany) using the manufacturer's protocol (see also Example 5 for more information).

Cytotoxicity was assessed by a ⁵¹Cr release assay. Approximately 5×10⁶ Daudi cells were treated with 0.3 mCi of ⁵¹Cr and incubated for 75 minutes at 37°C. After 75 minutes, cells were washed 3 times with media (RPMI + 10% FBS) and resuspended in 11.5 mL of media. From this suspension, 50 µL was dispensed per well into 96 well U-bottom plates (approximately 20,000 cells/well). Concentrations of bispecific molecules ranging from 10 nM to 0.1 pM were added to the target (Daudi) cells, bringing the total volume to 100 µL/well. Target cells were incubated at room temperature for 15 minutes. Then 100 µL of isolated T-cells (approximately 200,000) were added to bring the T-cell to target cell ratio to 10:1. 50 µL of 0.8% NP-40 was added to a control well containing target cells, left for 15 minutes, then 100 µL of media was added to provide a total lysis control.

Plates were incubated for 4 hours, spun at 1500 rpm for 3 minutes, and 25 µL of supernatant was transferred from each well to the corresponding well of a 96-well Luma sample plate. Sample plates were allowed to air dry in a chemical safety hood for 18 hours, and then radioactivity was read on a Topcount scintillation counter using a standard protocol.

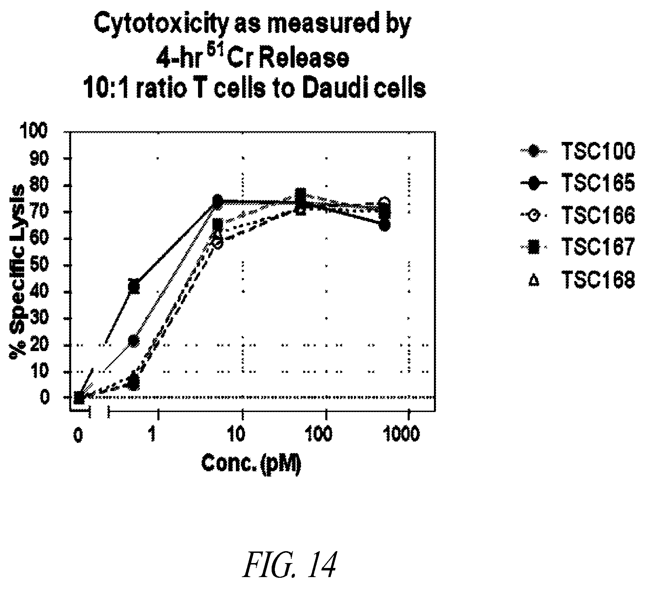

Analysis of cytotoxicity data showed strong T-cell directed cytotoxicity with the Daudi cells in the presence of T-cells and the anti-CD 19 directed bispecific molecules (TSC100 - TSC168), reaching maximal lysis at a concentration between 5 and 50 pM (Figure 14). Similar to the trends observed in Example 9, the bispecific molecule with a shorter IgA2 upper hinge region (TSC165) showed comparable or greater cytotoxicity to the molecule with the standard IgG1 upper hinge region (TSC100), whereas the molecules with longer upper hinge regions (TSC166, TSC167, TSC168) were less potent at inducing cytotoxicity.

### EXAMPLE 11

### BISPECIFIC HETERODIMERS WITH CH3-CH1 AND CH3-CK LINKER VARIATIONS

The following bispecific heterodimers with CH3-CH1 and CH3-Ck linker variations were made:
Bispecific heterodimer TSC151 comprises single chain polypeptides TSC145 and TSC148. Single chain polypeptide TSC145 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence GGGSS (SEQ ID NO:847). The nucleotide and amino acid sequences of TSC145 are set forth in SEQ ID NOS:853 and 859, respectively. Single chain polypeptide TSC148 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence GGGSR (SEQ ID NO:850) in which R may alternatively be regarded as the first arginine of human Ck (YAE). The nucleotide and amino acid sequences of TSC148 are set forth in SEQ ID NOS:856 and 862, respectively.
Bispecific heterodimer TSC152 comprises single chain polypeptides TSC146 and TSC149. Single chain polypeptide TSC146 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence SYSPNS (SEQ ID NO:848). The nucleotide and amino acid sequences of TSC146 are set forth in SEQ ID NOS:854 and 860, respectively. Single chain polypeptide TSC149 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence SYSPNSR (SEQ ID NO:851) in which R may alternatively be regarded as the first arginine of human Ck (YAE). The nucleotide and amino acid sequences of TSC149 are set forth in SEQ ID NOS:857 and 863, respectively.
Bispecific heterodimer TSC153 comprises single chain polypeptides TSC147 and TSC150. Single chain polypeptide TSC147 comprises from its amino to carboxyl terminus: humanized Cris7 (anti-CD3) scFv, human IgG1 SCC-P hinge, human CH2 (ADCC/CDC null), human CH3, and human IgG1 CH1. The human CH3 and human IgG1 CH1 are linked by a linker having the sequence SSLNTPNS (SEQ ID NO:849). The nucleotide and amino acid sequences of TSC147 are set forth in SEQ ID NOS:855 and 861, respectively. Single chain polypeptide TSC150 comprises from its amino to carboxyl terminus: HD37 (anti-CD19) scFv, human IgG1 SCC-P hinge, human CH2 (ADCC/CDC null), human CH3, and human Ck (YAE). The human CH3 and human Ck (YAE) are linked with a linker having the sequence SSLNTPNSR (SEQ ID NO:852) in which R may alternatively be regarded as the first arginine of human Ck (YAE). The nucleotide and amino acid sequences of TSC150 are set forth in SEQ ID NOS:858 and 864, respectively.

The above bispecific heterodimers were expressed according to Example 1. The following expression levels were obtained: 9.2 µg protein / ml of culture for TSC151, 11.2 µg protein / ml of culture for TSC152, and 14.7 µg protein / ml of culture for TSC153. In comparison, about 6 µg protein / ml of culture was obtained for heterodimers with a CH3-CH1 and CH3-Ck linker having the amino acid sequence KSR (SEQ ID NO:846).

The various embodiments described above can be combined to provide further embodiments. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. A polypeptide heterodimer, comprising:
(a) a first single chain polypeptide (SCP-I) comprising from one to four binding domains that specifically bind from one to four targets, a hinge (H-I), an immunoglobulin heterodimerization domain (HD-I), and an immunoglobulin CH2 and CH3 domain of IgG1, IgG2, IgG3, IgG4, IgA1, IgA2, IgD, or any combination thereof (CH2CH3-I); and
(b) a second single chain polypeptide (SCP-II) comprising from zero to four binding domains that specifically bind from zero to four targets, a hinge (H-II), an immunoglobulin heterodimerization domain (HD-II), and an immunoglobulin CH2 and CH3 domain of IgG1, IgG2, IgG3, IgG4, IgA1, IgA2, IgD, or any combination thereof (CH2CH3-II);
wherein
(i) the immunoglobulin heterodimerization domain of the first single chain polypeptide (HD-I) and the immunoglobulin heterodimerization domain of the second single chain polypeptide (HD-II) preferentially associate with each other to form a polypeptide heterodimer comprised of the first single chain polypeptide (SCP-I) and the second single chain polypeptide (SCP-II), and
(1) the immunoglobulin heterodimerization domain of the first single chain polypeptide (HD-I) comprises a first immunoglobulin CH1 region, and the immunoglobulin heterodimerization domain of the second single chain polypeptide (HD-II) comprises a first immunoglobulin CL region, or
(2) the immunoglobulin heterodimerization domain of the first single chain polypeptide (HD-I) comprises a first immunoglobulin CL region, and the immunoglobulin heterodimerization domain of the second single chain polypeptide (HD-II) comprises a first immunoglobulin CH1 region;
(ii) wherein the first immunoglobulin CL region is an altered human immunoglobulin Cκ region with one or more amino acids of a wild type human Cκ region substituted at N29, N30, Q52, V55, T56, S68, or T70,
provided that the polypeptide heterodimer comprises at least two binding domains that specifically bind at least two different targets.

2. The polypeptide heterodimer of claim 1, wherein the binding domains are single chain Fv (scFv) polypeptides.

3. The polypeptide heterodimer of claim 1, wherein the polypeptide heterodimer comprises two binding domains (BD1 and BD2), wherein preferably the first binding domain (BD1) is on the first single chain polypeptide (SCP-I) and the second binding domain (BD2) is on the second single chain polypeptide (SCP-II).

4. The polypeptide heterodimer of claim 3, wherein the first binding domain (BD1) is amino terminal to the CH2CH3-I of the first single chain polypeptide, and the second binding domain (BD2) is amino terminal to the CH2CH3-II of the second single chain polypeptide.

5. The polypeptide heterodimer of claim 1 or 2, wherein the polypeptide heterodimer comprises three binding domains (BD1, BD2 and BD3).

6. The polypeptide heterodimer of claim 1, wherein the polypeptide heterodimer comprises four binding domains (BD1, BD2, BD3, and BD4), wherein preferably the HD-I and CH2CH3-I are disposed between BD1 and BD2, and the HD-II and CH2CH3-II are disposed between BD3 and BD4.

7. The polypeptide heterodimer of claim 1 or 2, wherein the polypeptide heterodimer comprises five to eight binding domains.

8. The polypeptide heterodimer of any one of claims 1 to 7, wherein at least one of the binding domains specifically binds to, or is an antagonist of, a TCR complex, TCRα, TCRβ, CD3γ, CD3δ, CD3ε, CD28, CD79b, hyperIL-6, monoIL-10, CD86, CD20, PSMA, CD19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACI, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL, HGF, MSP, EGF (including epiregulin, herregulin, β-regulin, neuregulin), HIF-1α, VEGFA, VEGFB, VEGFC, VEGFD, TNFα, Wnt, sHH, TGFβ, PDGF, TWEAK, EpCAM, CEA, PCTA-1, STEAP-1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, ganglioside GD3, 9-O-Acetyl-GD3, GM2, Globo H, fucosyl GM1, Poly SA, GD2, Carboanhydrase IX (MN/CA IX), CD44v6, Sonic Hedgehog (Shh), Wue-1, Plasma Cell Antigen, (membrane-bound) IgE, Melanoma Chondroitin Sulfate Proteoglycan (MCSP), CCR8, TNF-alpha precursor, STEAP, mesothelin, A33 Antigen, Prostate Stem Cell Antigen (PSCA), Ly-6; desmoglein 4, E-cadherin neoepitope, Fetal Acetylcholine Receptor, CD25, CA19-9 marker, CA-125 marker and Muellerian Inhibitory Substance (MIS) Receptor type II, sTn (sialylated Tn antigen; TAG-72), FAP (fibroblast activation antigen), endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD137, LIFRβ, TLR7 or TLR9.

9. The polypeptide heterodimer of any one of the preceding claims, wherein the one or more amino acids of a wild type human Cκ region are substituted with one or more amino acid substitutions selected from Ala (A), Arg (R), Trp (W), Tyr (Y), Glu (E), Gln (Q), Lys (K), Asp (D), Met (M), Ser (S), and Phe (F).

10. The polypeptide heterodimer of any one of the preceding claims, wherein the altered human immunoglobulin Cκ region comprises the following substitutions: (i) N30Y V55A T70E; or (ii) N30M, V55A T70E.

11. The polypeptide heterodimer of any one of the preceding claims, wherein the CH1 region is an altered human immunoglobulin CH1 region comprising an amino acid substitution by which Val (V) at position 68 is substituted by Lys (K), Arg (R) or His (H), and wherein the Cκ region is an altered human immunoglobulin Cκ region comprising an amino acid substitution by which Leu (L) at position 27 is substituted by Asp (D) or Glu (E) or wherein the CH1 region is an altered human immunoglobulin CH1 region comprising an amino acid substitution by which Val (V) at position 68 is substituted by Asp (D) or Glu (E), and wherein the Cκ region is an altered human immunoglobulin Cκ region comprising an amino acid substitution by which Leu (L) at position 27 is changed to Lys (K), Arg (R) or His (H).

12. The polypeptide heterodimer of any one of claims 1 to 11, wherein the first CH1 region is an altered human immunoglobulin CH1 region with the cysteine of a wild type human immunoglobulin CH1 region that is involved in forming a disulfide bond with a wild type human immunoglobulin CL region deleted or substituted.

13. The polypeptide heterodimer of any one of the preceding claims, wherein the Cκ region is an altered human immunoglobulin Cκ region with the cysteine residue of a wild type human Cκ region that is involved in forming a disulfide bond with a wild type human immunoglobulin CH1 region deleted or substituted.

14. The polypeptide heterodimer of any one of claims 1 to 13 wherein the hinge of both the first and second single chain polypeptides is an immunoglobulin hinge region.

15. The polypeptide heterodimer of any one of claims 1 to 14 wherein the hinge region is
(a) at the amino terminal to CH2CH3-I or CH2CH3-II,
(b) disposed between the binding domain and the immunoglobulin heterodimerization domain of the first single chain polypeptide (HD-I) and/or the immunoglobulin heterodimerization domain of the second single chain polypeptide (HD-II),
(c) disposed between a immunoglobulin heterodimerization domain and CH2CH3-I or CH2CH3-II,
(d) at the amino terminus of the first or second single chain polypeptide,
(e) a C-type lectin hinge region,
(f) an NKg2A or NKg2D peptide, or a derivative thereof,
(g) disposed between CH2CH3-I and a binding domain, or
(h) at the carboxyl terminus of the first or second single chain polypeptide.

16. The heterodimer of any one of the preceding claims, wherein the first single chain polypeptide comprises a binding domain that specifically binds a TCR complex or a component thereof, and the second single chain polypeptide comprises a binding domain that specifically binds a tumor-specific antigen.

17. A composition comprising a polypeptide heterodimer of any one of claims 1 to 16 and a pharmaceutically acceptable excipient.

18. The polypeptide heterodimer according to any one of claims 1 to 17 for use in:
a) a method for directing T cell activation, wherein the polypeptide heterodimer comprises a binding domain that specifically binds TCRα, TCRβ, CD3γ, CD3δ, CD3ε or a combination thereof, and a second binding domain that specifically binds a different target; or
b) inhibiting growth, metastasis or metastatic growth of a malignancy, wherein the polypeptide heterodimer comprises a binding domain that specifically binds TCRα, TCRβ, CD3γ, CD3δ, CD3ε, c-Met or Ron.
c) treating an autoimmune or inflammatory condition, wherein the polypeptide heterodimer comprises a binding domain that specifically binids TCRα, TCRβ, CD3γ, CD3δ, CD3ε, or CD28.
d) treating a B-cell associated disorder or disease, wherein the polypeptide heterodimer comprises a binding domain that specifically binds TCRα, TCRβ, CD3γ, CD3δ, CD3ε, and a second binding domain that specifically binds to CD19, CD20, CD79b or HLA-DR.

19. An expression vector capable of expressing the polypeptide heterodimer of any one of claims 1 to 16, comprising a first polynucleotide encoding the first single chain polypeptide and a second polynucleotide encoding the second single chain polypeptide.

20. A host cell comprising (i) the expression vector of claim 19 or (ii) first and second expression vectors capable of expressing the first and second single chain polypeptides, respectively, of the polypeptide heterodimer of any one of claims 1 to 16.

21. A method for making a polypeptide heterodimer, comprising
(a) culturing a host cell of claim 20 under conditions suitable to express first and second single chain polypeptides, and
(b) optionally isolating or purifying the heterodimers formed from the first and second single chain polypeptides from the culture.

## Patentansprüche

1. Polypeptid-Heterodimer, umfassend:
(a) ein erstes Einzelkettenpolypeptid (SCP-I) umfassend eine bis vier Bindungsdomänen, die ein bis vier Targets spezifisch binden, ein Scharnier (H-1), eine Immunglobulin-Heterodimerisierungsdomäne (HD-I) und eine Immunglobulin-CH2- und - CH3-Domäne von IgG1, IgG2, IgG3, IgG4, IgA1, IgA2, IgD oder eine beliebige Kombination davon (CH2CH3-I); und
(b) ein zweites Einzelkettenpolypeptid (SCP-II) umfassend null bis vier Bindungsdomänen, die null bis vier Targets spezifisch binden, ein Scharnier (H-II), eine Immunglobulin-Heterodimerisierungsdomäne (HD-II) und eine Immunglobulin-CH2- und - CH3-Domäne von IgG1, IgG2, IgG3, IgG4, IgA1, IgA2, IgD oder eine beliebige Kombination davon (CH2CH3-II);
worin
(i) die Immunglobulin-Heterodimerisierungsdomäne des ersten Einzelkettenpolypeptids (HD-I) und die Immunglobulin-Heterodimerisierungsdomäne des zweiten Einzelkettenpolypeptids (HD-II) sich bevorzugterweise miteinander assoziieren, um ein Polypeptid-Heterodimer bestehend aus dem ersten Einzelkettenpolypeptid (SCP-I) und dem zweiten Einzelkettenpolypeptid (SCP-II) zu bilden, und
(1) die Immunglobulin-Heterodimerisierungsdomäne des ersten Einzelkettenpolypeptids (HD-I) eine erste Immunglobulin-CH1-Region umfasst und die Immunglobulin-Heterodimerisierungsdomäne des zweiten Einzelkettenpolypeptids (HD-II) eine erste Immunglobulin-CL-Region umfasst, oder
(2) die Immunglobulin-Heterodimerisierungsdomäne des ersten Einzelkettenpolypeptids (HD-I) eine erste Immunglobulin-CL-Region umfasst und die Immunglobulin-Heterodimerisierungsdomäne des zweiten Einzelkettenpolypeptids (HD-II) eine erste Immunglobulin-CH1-Region umfasst;
(ii) worin die erste Immunglobulin-CL-Region eine veränderte humane Immunglobulin-Cκ-Region mit einer oder mehreren an N29, N30, Q52, V55, T56, S68 oder T70 substituierten Aminosäuren einer humanen Cκ-Region der Wildform ist,
vorausgesetzt, dass das Polypeptid-Heterodimer mindestens zwei Bindungsdomänen umfasst, die mindestens zwei verschiedene Targets spezifisch binden.

2. Polypeptid-Heterodimer nach Anspruch 1, worin die Bindungsdomänen Einzelketten-Fv- (scFv-) Polypeptide sind.

3. Polypeptid-Heterodimer nach Anspruch 1, worin das Polypeptid-Heterodimer zwei Bindungsdomänen (BD1 und BD2) umfasst, worin vorzugsweise die erste Bindungsdomäne (BD1) auf dem ersten Einzelkettenpolypeptid (SCP-I) ist und die zweite Bindungsdomäne (BD2) auf dem zweiten Einzelkettenpolypeptid (SCP-II) ist.

4. Polypeptid-Heterodimer nach Anspruch 3, worin die erste Bindungsdomäne (BD1) aminoterminal zur CH2CH3-I des ersten Einzelkettenpolypeptids ist und die zweite Bindungsdomäne (BD2) aminoterminal zur CH2CH3-II des zweiten Einzelkettenpolypeptids ist.

5. Polypeptid-Heterodimer nach Anspruch 1 oder 2, worin das Polypeptid-Heterodimer drei Bindungsdomänen (BD1, BD2 und BD3) umfasst.

6. Polypeptid-Heterodimer nach Anspruch 1, worin das Polypeptid-Heterodimer vier Bindungsdomänen (BD1, BD2, BD3 und BD4) umfasst, worin vorzugsweise HD-I und CH2CH3-I zwischen BD1 und BD2 angeordnet sind und HD-II und CH2CH3-II zwischen BD3 und BD4 angeordnet sind.

7. Polypeptid-Heterodimer nach Anspruch 1 oder 2, worin das Polypeptid-Heterodimer fünf bis acht Bindungsdomänen umfasst.

8. Polypeptid-Heterodimer nach irgendeinem der Ansprüche 1 bis 7, worin mindestens eine der Bindungsdomänen sich an Folgendes spezifisch bindet oder ein Antagonist davon ist: einen/einem TCR-Komplex, TCRα, TCRβ, CD3γ, CD3δ, CD3ε, CD28, CD79b, hyperIL-6, monoIL-10, CD86, CD20, PSMA, CD19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, Angiopoietin 2, CD64, CD32A, CD16, CD71, TNFR1, TNFR2, TWEAKR, TACI, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL, HGF, MSP, EGF (einschließlich Epiregulin, Herregulin, β-Regulin, Neuregulin), HIF-1α, VEGFA, VEGFB, VEGFC, VEGFD, TNFα, Wnt, sHH, TGFβ, PDGF, TWEAK, EpCAM, CEA, PCTA-1, STEAP-1, PSCA, ALCAM (CD166), EphA2, CD151, CA-125, MUC-1, MAGE-1, TROP2, CCR5, HER-3, HER-4, EGFR, CEA, MUC2, MUC3, MUC4, MUC5_{AC}, MUC5_{b}, MUC7, βhCG, Lewis-Y, Gangliosid GD3, 9-O-Acetyl-GD3, GM2, Globo H, Fucosyl GM1, Poly SA, GD2, Carboanhydrase IX (MN/CA IX), CD44v6, Sonic Hedgehog (Shh), Wue-1, Plasmazellen-Antigen, (membrangebundenes) IgE, Melanom-Chondroitinsulfat-Proteoglycan (MCSP), CCR8, TNF-alpha-Vorläufer, STEAP, Mesothelin, A33-Antigen, Prostatastammzellantigen (PSCA), Ly-6; Desmoglein 4, E-Cadherin-Neoepitop, fötaler Acetylcholin-Rezeptor, CD25, CA19-9-Marker, CA-125-Marker und MIS- (Müllersche Inhibitorische Substanz) Rezeptor Typ II, sTn (sialyliertes Tn-Antigen; TAG-72), FAP (Fibroblastenaktivierungsantigen), Endosialin, EGFRvIII, LG, SAS, CD63, IGF1R, CD151, TGFBR2, GHRHR, GHR, IL-6R, gp130, TNFR2, OSMRβ, Patched-1, Frizzled, Robo1, CD80, CD81, CD86, OX40, CD40, CD137, LIFRβ, TLR7 oder TLR9.

9. Polypeptid-Heterodimer nach irgendeinem der vorhergehenden Ansprüche, worin die eine oder mehrere Aminosäuren einer humanen Cκ-Region der Wildform mit einer oder mehreren Aminosäuresubstitutionen ausgewählt aus Ala (A), Arg (R), Trp (W), Tyr (Y), Glu (E), Gin (Q), Lys (K), Asp (D), Met (M), Ser (S) und Phe (F) substituiert sind.

10. Polypeptid-Heterodimer nach irgendeinem der vorhergehenden Ansprüche, worin die veränderte humane Immunglobulin-CK-Region die folgenden Substitutionen umfasst: (i) N30Y V55A T70E; oder (ii) N30M, V55A T70E.

11. Polypeptid-Heterodimer nach irgendeinem der vorhergehenden Ansprüche, worin die CH1-Region eine veränderte humane Immunglobulin-CH1-Region umfassend eine Aminosäuresubstitution ist, durch die Val (V) in Position 68 durch Lys (K), Arg (R) oder His (H) substituiert wird, und worin die Cκ-Region eine veränderte humane Immunglobulin-CK-Region umfassend eine Aminosäuresubstitution ist, durch die Leu (L) in Position 27 durch Asp (D) oder Glu (E) substituiert wird, oder worin die CH1-Region eine veränderte humane Immunglobulin-CH1-Region umfassend eine Aminosäuresubstitution ist, durch die Val (V) in Position 68 durch Asp (D) oder Glu (E) substituiert wird, und worin die Cκ-Region eine veränderte humane Immunglobulin-CK-Region umfassend eine Aminosäuresubstitution ist, durch die Leu (L) in Position 27 in Lys (K), Arg (R) oder His (H) abgeändert wird.

12. Polypeptid-Heterodimer nach irgendeinem der Ansprüche 1 bis 11, worin die erste CH1-Region eine veränderte humane Immunglobulin-CH1-Region mit dem Cystein einer humanen CH1-Region der Wildform ist, das an der Bildung einer Disulfidbindung mit einer gelöschten oder substituierten humanen Immunglobulin-CL-Region der Wildform beteiligt ist.

13. Polypeptid-Heterodimer nach irgendeinem der vorhergehenden Ansprüche, worin die Cκ-Region eine veränderte humane Immunglobulin-CK-Region mit dem Cysteinrest einer humanen Cκ-Region der Wildform ist, der an der Bildung einer Disulfidbindung mit einer gelöschten oder substituierten humanen Immunglobulin-CH1-Region der Wildform beteiligt ist.

14. Polypeptid-Heterodimer nach irgendeinem der Ansprüche 1 bis 13, worin das Scharnier sowohl der ersten als auch zweiten Einzelkettenpolypeptide eine Immunglobulin-Scharnierregion ist.

15. Polypeptid-Heterodimer nach irgendeinem der Ansprüche 1 bis 14, worin die Scharnierregion
(a) am Aminoterminal zu CH2CH3-1 oder CH2CH3-II ist,
(b) zwischen der Bindungsdomäne und der Immunglobulin-Heterodimerisierungsdomäne des ersten Einzelkettenpolypeptids (HD-I) und/oder der Immunglobulin-Heterodimerisierungsdomäne des zweiten Einzelkettenpolypeptids (HD-II) angeordnet ist,
(c) zwischen einer Immunglobulin-Heterodimerisierungsdomäne und CH2CH3-I oder CH2CH3-II angeordnet ist,
(d) am Aminoterminus des ersten oder zweiten Einzelkettenpolypeptids ist,
(e) eine C-Typ-Lectin-Scharnierregion ist,
(f) ein NKg2A- oder NKg2D-Peptid oder ein Derivat davon ist,
(g) zwischen CH2CH3-I und einer Bindungsdomäne angeordnet ist, oder
(h) am Carboxylterminus des ersten oder zweiten Einzelkettenpolypeptids ist.

16. Heterodimer nach irgendeinem der vorhergehenden Ansprüche, worin das erste Einzelkettenpolypeptid eine Bindungsdomäne umfasst, die einen TCR-Komplex oder eine Komponente davon spezifisch bindet, und das zweite Einzelkettenpolypeptid eine Bindungsdomäne umfasst, die ein tumorspezifisches Antigen spezifisch bindet.

17. Zusammensetzung umfassend ein Polypeptid-Heterodimer nach irgendeinem der Ansprüche 1 bis 16 und einen pharmazeutisch annehmbaren Hilfsstoff.

18. Polypeptid-Heterodimer nach irgendeinem der Ansprüche 1 bis 17 zur Verwendung:
(a) in einem Verfahren zum Lenken der T-Zell-Aktivierung, worin das Polypeptid-Heterodimer eine Bindungsdomäne umfasst, die TCRα, TCRβ, CD3γ, CD3δ, CD3ε oder eine Kombination davon spezifisch bindet, und eine zweite Bindungsdomäne, die ein anderes Target spezifisch bindet; oder
(b) beim Inhibieren von Wachstum, Metastasen oder metastatischem Wachstum einer Malignität, worin das Polypeptid-Heterodimer eine Bindungsdomäne umfasst, die TCRα, TCRβ, CD3γ, CD3δ, CD3ε, c-Met oder Ron spezifisch bindet.
(c) bei der Behandlung einer autoimmunen oder entzündlichen Erkrankung, worin das Polypeptid-Heterodimer eine Bindungsdomäne umfasst, die TCRα, TCRβ, CD3γ, CD3δ, Cd3ε oder CD28 spezifisch bindet.
(d) bei der Behandlung einer B-Zell-assozüerten Störung oder Krankheit, worin das Polypeptid-Heterodimer eine Bindungsdomäne umfasst, die TCRα, TCRβ, CD3γ, CD3δ, CD3ε spezifisch bindet, und eine zweite Bindungsdomäne, die spezifisch an CD19, CD20, CD79b oder HLA-DR bindet.

19. Expressionsvektor, der das Polypeptid-Heterodimer nach irgendeinem der Ansprüche 1 bis 16 exprimieren kann, umfassend ein erstes Polynukleotid, das das erste Einzelkettenpolypeptid codiert, und ein zweites Polynukleotid, das das zweite Einzelkettenpolypeptid codiert.

20. Wirtszelle umfassend (i) den Expressionsvektor nach Anspruch 19 oder (ii) erste und zweite Expressionsvektoren, die die ersten bzw. zweiten Einzelkettenpolypeptide des Polypeptid-Heterodimers nach irgendeinem der Ansprüche 1 bis 16 exprimieren können.

21. Verfahren zur Herstellung eines Polypeptid-Heterodimers, umfassend
(a) Kultivieren einer Wirtszelle nach Anspruch 20 unter Bedingungen, die zum Exprimieren erster und zweiter Einzelkettenpolypeptide geeignet sind, und
(b) wahlweises Isolieren oder Reinigen der aus den ersten und zweiten Einzelkettenpolypeptiden aus der Kultur hergestellten Heterodimere.

## Revendications

1. Hétérodimère polypeptidique comprenant :
(a) un premier polypeptide simple chaîne (SCP-I) comprenant de un à quatre domaines de liaison qui se lient de manière spécifique à une à quatre cibles, une charnière (H-I), un domaine d'hétérodimérisation d'immunoglobuline (HD-I) et un domaine CH2 et CH3 d'immunoglobuline d'IgGI, IgG2, IgG3, IgG4, IgAI, IgA2, IgD ou d'une combinaison quelconque correspondante (CH2CH3-II) ; et
(b) un second polypeptide simple chaîne (SCP-II) comprenant de zéro à quatre domaines de liaison qui se lient de manière spécifique à zéro à quatre cibles, une charnière (H-II), un domaine d'hétérodimérisation d'immunoglobuline (HD-II) et un domaine CH2 et CH3 d'immunoglobuline d'IgGI, IgG2, IgG3, IgG4, IgAI, IgA2, IgD ou d'une combinaison quelconque correspondante (CH2CH3-II) ;
dans lequel
(i) le domaine d'hétérodimérisation d'immunoglobuline du premier polypeptide simple chaîne (HD-I) et le domaine d'hétérodimérisation d'immunoglobuline du second polypeptide simple chaîne (HD-II) s'associent de préférence l'un à l'autre pour former un hétérodimère polypeptidique constitué du premier polypeptide simple chaîne (SCP-I) et du second polypeptide simple chaîne (SCP-II), et
(1) le domaine d'hétérodimérisation d'immunoglobuline du premier polypeptide simple chaîne (HD-I) comprend une première région CH1 d'immunoglobuline, et le domaine d'hétérodimérisation d'immunoglobuline du second polypeptide simple chaîne (HD-II) comprend une première région CL d'immunoglobuline, ou
(2) le domaine d'hétérodimérisation d'immunoglobuline du premier polypeptide simple chaîne (HD-I) comprend une première région CL d'immunoglobuline, et le domaine d'hétérodimérisation d'immunoglobuline du second polypeptide simple chaîne (HD-II) comprend une première région CH1 d'immunoglobuline ;
(ii) la première région CL d'immunoglobuline étant une région Ck d'immunoglobuline humaine modifiée avec un ou plusieurs acides aminés d'une région Ck humaine de type sauvage substituée à N29, N30, Q52, V55, T56, S68 ou T70,
à condition que l'hétérodimère polypeptidique comprenne au moins deux domaines de liaison qui se lient de manière spécifique à au moins deux cibles différentes.

2. Hétérodimère polypeptidique selon la revendication 1, dans lequel les domaines de liaison sont des polypeptides Fv simple chaîne (scFv).

3. Hétérodimère polypeptidique selon la revendication 1, l'hétérodimère polypeptidique comprenant deux domaines de liaison (BD1 et BD2), le premier domaine de liaison (BD1) étant de préférence sur le premier polypeptide simple chaîne (SCP-I), et le second domaine de liaison (BD2) étant de préférence sur le second polypeptide simple chaîne (SCP-II).

4. Hétérodimère polypeptidique selon la revendication 3, dans lequel le premier domaine de liaison (BD1) est amino terminal par rapport au CH2CH3-I du premier polypeptide simple chaîne, et dans lequel le second domaine de liaison (BD2) est amino terminal par rapport au CH2CH3-II du second polypeptide simple chaîne.

5. Hétérodimère polypeptidique selon la revendication 1 ou 2, l'hétérodimère polypeptidique comprenant trois domaines de liaison (BD1, BD2 et BD3).

6. Hétérodimère polypeptidique selon la revendication 1, l'hétérodimère polypeptidique comprenant quatre domaines de liaison (BD1, BD2, BD3 et BD4), dans lequel le HD-1 et le CH2CH3T sont de préférence disposés entre BD1 et BD2, et le HD-II et CH2CH3-II sont de préférence disposés entre BD3 et BD4.

7. Hétérodimère polypeptidique selon la revendication 1 ou 2, l'hétérodimère polypeptidique comprenant de cinq à huit domaines de liaison.

8. Hétérodimère polypeptidique selon l'une quelconque des revendications 1 à 7, dans lequel au moins un des domaines de liaison se lie de manière spécifique à, ou est un antagoniste de : un complexe TCR, à TCRα, TCRβ, CD3γ, CD3δ, CD3ε, CD28, CD79b, hyperIL-6, monoIL-10, CD86, CD20, PSMA, CD19, HLA-DR, Ron, c-Met, CEACAM-6, LIGHT, GITRL, CD40, PDL1, PDL2, HVEM, LTBR, EGFR, EGFRvIII, ErbB2, ErbB3, ErbB4, IGF1R, EphA2, PDGFR, VEGFR1-4, l'angiopoiétine 2, CD64, CD32A, CD16, CD71, TNFR1, TNFR2, TWEAKR, TACI, BAFF-R, BCMA, FAS, CD32B, CD21, CD22, CD30, CD33, CD37, CD38, CD70, TNFα, IL-6, hyperIL-6, IL-2, IL-1, IL-7, IL-8, IL-17A/C, IP-10, IFNγ, IFNα, RANKL, FASL, TGFβ, IL10, IL17A/F, CSF2, IGF1, IGF2, BLyS/APRIL, HGF, MSP, EGF (notamment l'épiréguline, l'héréguline, la β-réguline, la neuréguline), HIF-1α, VEGFA, VEGFB, VEGFC, VEGFD, TNFα, Wnt, sHH, TGFβ, PDGF, TWEAK, EpCAM, CEA, PCTA-1, STEAP-1, PSCA, ALCAM (CD 166), EphA2, CD151, CA-125, MUC-1, MAGE-1, TROP2, CCR5, HER-3, HER-4, EGFR, CEA, MUC2, MUC3, MUC4, MUC5_{A}C, MUC5_{b}, MUC7, βhCG, Lewis-Y, le ganglioside GD3, le 9-O-acétyl-GD3, GM2, Globo H, le fucosyle GM1, la poly SA, GD2, la carboanhydrase IX (MN/CA IX), CD44v6, Sonic Hedgehog (Shh), Wue-1, l'antigène des cellules de plasma, IgE (liée à la membrane), un protéoglycane de sulfate de chondroïtine associé à un mélanome (MCSP), CCR8, un précurseur alpha-TNF, STEAP, la mésothéline, l'antigène de A33, l'antigène des cellules souches de la prostate (PSCA), Ly-6, la desmogléine 4, le néoépitope de l'E-cadhérine, le récepteur de l'acétylcholine foetale, CD25, le marqueur CA19-9, le marqueur CA-125, le récepteur du type II de la substance inhibitrice Müllerienne (MIS), sTn (antigène Tn sialylé ; TAG-72), FAP (antigène d'activation fibroblastique), l'endosialine, EGFRvIII, LG, SAS, CD63, IGF1R, CD 151, TGFBR2, GHRHR, GHR, IL-6R, gpl30, TNFR2, OSMRβ, Patched-1, Frizzled, Robol, CD80, CD81, CD86, 0X40, CD40, CD 137, LIFRβ, TLR7 ou TLR9.

9. Hétérodimère polypeptidique selon l'une quelconque des revendications précédentes, dans lequel le ou les acides aminés d'une région Ck humaine de type sauvage sont substitués par une ou plusieurs substitutions d'acide aminé choisies parmi Ala (A), Arg (R), Tip (W), Tyr (Y), Glu (E), Gin (Q), Lys (K), Asp (D), Met (M), Ser (S) et Phe (F).

10. Hétérodimère polypeptidique selon l'une quelconque des revendications précédentes, dans lequel la région Ck d'immunoglobuline humaine modifiée comprend les substitutions suivantes : (i) N30Y V55A T70E ; ou (ii) N30M, V55A T70E.

11. Hétérodimère polypeptidique selon l'une quelconque des revendications précédentes, dans lequel la région CH1 est une région CH1 d'immunoglobuline humaine modifiée comprenant une substitution d'acide aminé par laquelle Val (V) en position 68 est substitué par Lys (K), Arg (R) ou His (H), et dans lequel la région Ck est une région Ck d'immunoglobuline humaine modifiée comprenant une substitution d'acide aminé par laquelle Leu (L) en position 27 est substitué par Asp (D) ou Glu (E), ou dans lequel la région CH1 est une région CH1 d'immunoglobuline humaine modifiée comprenant une substitution d'acide aminé par laquelle Yal (V) en position 68 est substitué par Asp (D) ou Glu (E), et dans lequel la région Ck est une région Ck d'immunoglobuline humaine modifiée comprenant une substitution d'acide aminé par laquelle Leu (L) en position 27 est substitué par Lys (K), Arg (R) ou His (H).

12. Hétérodimère polypeptidique selon l'une quelconque des revendications 1 à 11, dans lequel la première région CH1 est une région CH1 d'immunoglobuline humaine modifiée avec la cystéine d'une région CH1 d'immunoglobuline humaine de type sauvage impliquée dans la formation d'une liaison disulfure avec une région CL d'immunoglobuline humaine de type sauvage supprimée ou substituée.

13. Hétérodimère polypeptidique selon l'une quelconque des revendications précédentes, dans lequel la région Ck est une région Ck d'immunoglobuline humaine modifiée avec le résidu de cystéine d'une région Ck humaine de type sauvage impliquée dans la formation d'une liaison disulfure avec une région CH1 d'immunoglobuline humaine de type sauvage supprimée ou substituée.

14. Hétérodimère polypeptidique selon l'une quelconque des revendications 1à 13, dans lequel la charnière des premier et second polypeptides simple chaîne est une région charnière d'immunoglobuline.

15. Hétérodimère polypeptidique selon l'une quelconque des revendications 1 à 14, dans lequel la région charnière est
(a) à la terminaison amino par rapport à CH2CH3-I ou CH2CH3-II,
(b) disposée entre le domaine de liaison et le domaine d'hétérodimérisation d'immunoglobuline du premier polypeptide simple chaîne (HD-I) et/ou le domaine d'hétérodimérisation d'immunoglobuline du second polypeptide simple chaîne (HD-II),
(c) disposée entre un domaine d'hétérodimérisation d'immunoglobuline et CH2CH3-I ou CH2CH3-II,
(d) à la terminaison amino du premier ou du second polypeptide simple chaîne,
(e) une région charnière de la lectine de type C,
(f) un peptide NKg2A ou NKg2D, ou un dérivé correspondant,
(g) disposée entre CH2CH3-I et un domaine de liaison, ou
(h) à la terminaison carboxyle du premier ou du second polypeptide simple chaîne.

16. Hétérodimère polypeptidique selon l'une quelconque des revendications précédentes, dans lequel le premier polypeptide simple chaîne comprend un domaine de liaison qui se lie de manière spécifique à un complexe TCR ou à un composant correspondant, et le second polypeptide simple chaîne comprend un domaine de liaison qui se lie de manière spécifique à un antigène spécifique d'une tumeur.

17. Composition comprenant un hétérodimère polypeptidique selon l'une quelconque des revendications 1 à 16 et un excipient pharmaceutiquement acceptable.

18. Hétérodimère polypeptidique selon l'une quelconque des revendications 1 à 17, destiné à être utilisé dans un procédé consistant à :
(a) diriger une activation des cellules T, l'hétérodimère polypeptidique comprenant un domaine de liaison qui se lie de manière spécifique à TCRα, TCRβ, CD3γ, CD3δ, CD3ε ou une combinaison correspondante, et un second domaine de liaison qui se lie de manière spécifique à une cible différente ; ou
(b) inhiber la croissance, la métastase ou la croissance métastatique d'une malignité, l'hétérodimère polypeptidique comprenant un domaine de liaison qui se lie de manière spécifique à TCRα, TCRβ, CD3γ, CD3δ, CD3ε, c-Met ou Ron ;
(c) traiter une condition auto-immune ou inflammatoire, l'hétérodimère polypeptidique comprenant un domaine de liaison qui se lie de manière spécifique à TCRα, TCRβ, CD3γ, CD3δ, CD3ε ou CD28 ;
(d) traiter une maladie ou un trouble associé à une cellule B, l'hétérodimère polypeptidique comprenant un domaine de liaison qui se lie de manière spécifique à TCRα, TCRβ, CD3γ, CD3δ, CD3ε, et un second domaine de liaison qui se lie de manière spécifique à CD19, CD20, CD79b ou HLA-DR.

19. Vecteur d'expression capable d'exprimer l'hétérodimère polypeptidique selon l'une quelconque des revendications 1 à 16, comprenant un premier polynucléotide codant le premier polypeptide simple chaîne, et un second polynucléotide codant le second polypeptide simple chaîne.

20. Cellule hôte comprenant (i) le vecteur d'expression selon la revendication 19, ou (ii) des premier et second vecteurs d'expression capables d'exprimer respectivement les premier et second polypeptides simple chaîne de l'hétérodimère polypeptidique selon l'une quelconque des revendications 1 à 16.

21. Procédé d'élaboration d'un hétérodimère polypeptidique, consistant à :
(a) faire une culture d'une cellule hôte selon la revendication 20 dans des conditions appropriées pour exprimer les premier et second polypeptides simple chaîne, et
(b) éventuellement isoler ou purifier les hétérodimères formés à partir des premier et second polypeptides simple chaîne obtenus de la culture.
